(19) Europäisches Patentamt
European Patent Office
Office européen des brevets

(11) EP 3 617 215 A1

(12) # EUROPEAN PATENT APPLICATION

(43) Date of publication:
**04.03.2020 Bulletin 2020/10**

(21) Application number: **19193722.6**

(22) Date of filing: **27.08.2019**

(51) Int Cl.:
***C07F 15/00*** *(2006.01)* ***C09K 11/06*** *(2006.01)*
***H01L 51/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2018 KR 20180104028**

(71) Applicant: **Samsung Electronics Co., Ltd.**
**Gyeonggi-do 16677 (KR)**

(72) Inventors:
- **YI, Jeoungin**
  **16678 Gyeonggi-do (KR)**
- **KIM, Juhyun**
  **16678 Gyeonggi-do (KR)**
- **PARK, Sangho**
  **16678 Gyeonggi-do (KR)**
- **LEE, Sunyoung**
  **16678 Gyeonggi-do (KR)**
- **HONG, Seokhwan**
  **16678 Gyeonggi-do (KR)**
- **HWANG, Kyuyoung**
  **16678 Gyeonggi-do (KR)**
- **KWAK, Yoonhyun**
  **16678 Gyeonggi-do (KR)**
- **LEE, Sunghun**
  **16678 Gyeonggi-do (KR)**
- **CHOI, Byoungki**
  **16678 Gyeonggi-do (KR)**
- **CHOI, Hyeonho**
  **16678 Gyeonggi-do (KR)**

(74) Representative: **Elkington and Fife LLP**
**Prospect House**
**8 Pembroke Road**
**Sevenoaks, Kent TN13 1XR (GB)**

(54) **ORGANOMETALLIC COMPOUND, ORGANIC LIGHT-EMITTING DEVICE INCLUDING THE ORGANOMETALLIC COMPOUND, AND DIAGNOSTIC COMPOSITION INCLUDING THE ORGANOMETALLIC COMPOUND**

(57) The organometallic compound represented by Formula 1:

EP 3 617 215 A1

Formula 1

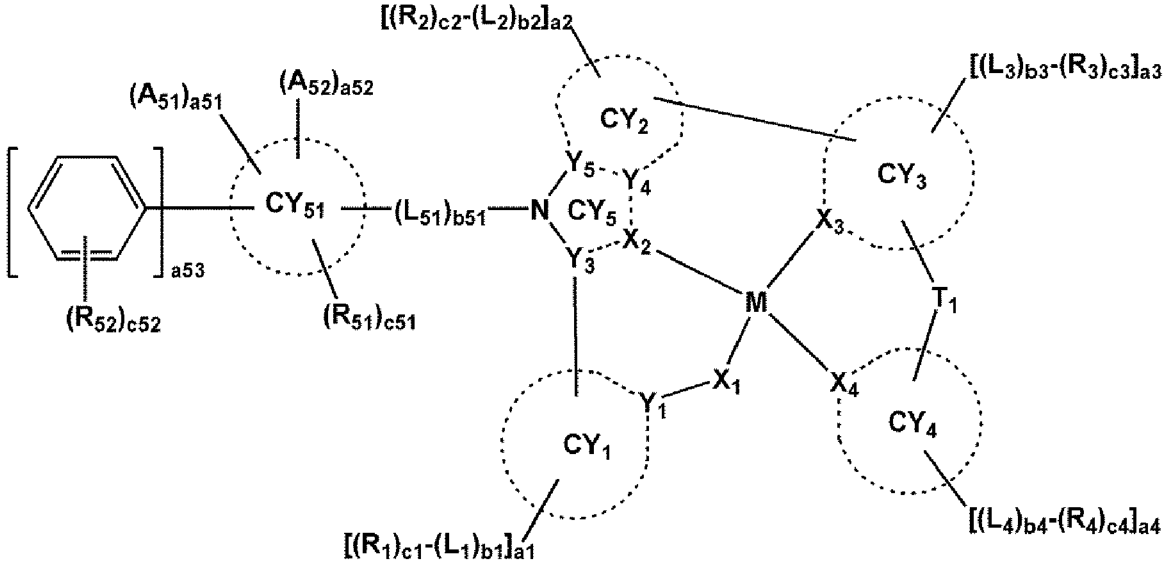

,

wherein, in Formula 1, groups and variables are the same as described in the specification.

## Description

FIELD OF THE INVENTION

One or more embodiments relate to an organometallic compound, an organic light-emitting device including the same, and a diagnostic composition including the organometallic compound.

BACKGROUND OF THE INVENTION

Organic light-emitting devices (OLEDs) are self-emission devices, which have superior characteristics in terms of a viewing angle, a response time, a brightness, a driving voltage, and a response speed, and which produce full-color images.

In an example, an organic light-emitting device includes an anode, a cathode, and an organic layer disposed between the anode and the cathode, wherein the organic layer includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

Meanwhile, luminescent compounds may be used to monitor, sense, or detect a variety of biological materials including cells and proteins. An example of the luminescent compounds includes a phosphorescent luminescent compound.

Various types of organic light emitting devices are known. However, there still remains a need in OLEDs having low driving voltage, high efficiency, high brightness, and long lifespan.

SUMMARY OF THE INVENTION

Aspects of the present disclosure provide an organometallic compound, an organic light-emitting device including the same, and a diagnostic composition including the organometallic compound.

Additional aspects will be set forth in part in the description which follows and, in part, will be apparent from the description, or may be learned by practice of the presented embodiments.

An aspect of the present disclosure provides an organometallic compound represented by Formula 1:

Formula 1

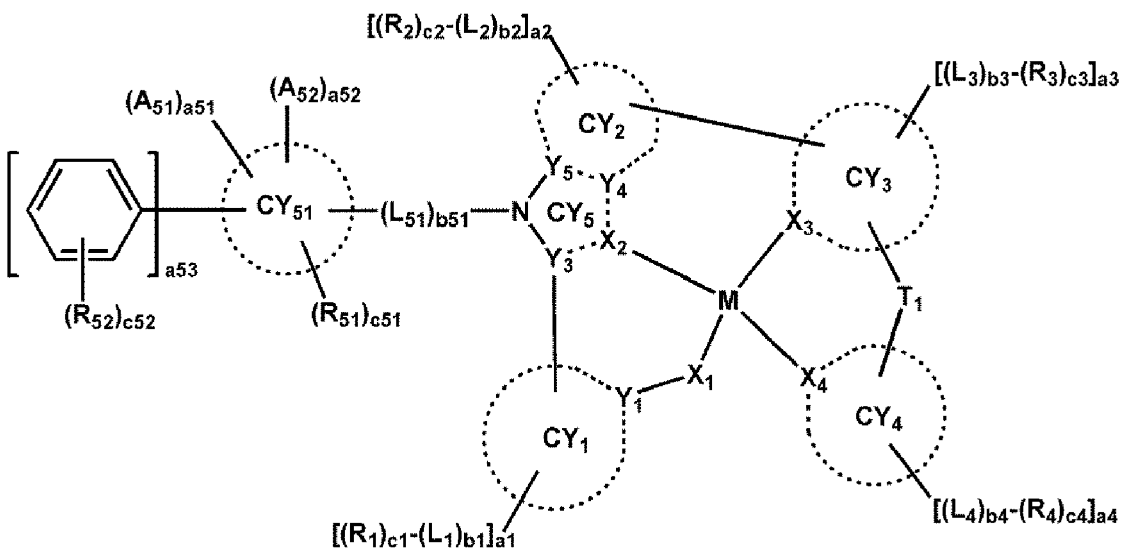

In Formula 1,

M may be a transition metal,

$X_1$ may be O or S, wherein a bond between $X_1$ and M may be a covalent bond,
$X_2$ to $X_4$ may each independently be C or N,
one bond selected from a bond between $X_2$ and M, a bond between $X_3$ and M, and a bond between $X_4$ and M may be a covalent bond, and the other bonds selected from a bond between $X_2$ and M, a bond between $X_3$ and M, and a bond between $X_4$ and M may be coordinate bonds,
$Y_1$ and $Y_3$ to $Y_5$ may each independently be C or N,
a bond between $X_2$ and $Y_3$, a bond between $X_2$ and $Y_4$, and a bond between $Y_4$ and $Y_5$ may be a chemical bond,
ring $CY_1$ to ring $CY_4$ and ring $CY_{51}$ may each independently be a $C_5$-$C_{30}$ carbocyclic group or a $C_1$-$C_{30}$ heterocyclic group,
a cyclometalated ring formed by ring $CY_5$, ring $CY_2$, ring $CY_3$, and M may be a 6-membered ring,
$T_1$ may be a single bond, a double bond, *-N($R_5$)-*', *-B($R_5$)-*', *-P($R_5$)-*', *-C($R_5$)($R_6$)-*', *-Si($R_5$)($R_6$)-*', *-Ge($R_5$)($R_6$)-*', *-S-*', *-Se-*', *-O-*', *-C(=O)-*', *-S(=O)-*', *-S(=O)$_2$-*', *-C($R_5$)=*', *=C($R_5$)-*', *-C($R_5$)=C($R_6$)-*', *-C(=S)-*', or *-C≡C-*', wherein * and *' each indicate a binding site to a neighboring atom,
$L_1$ to $L_4$ and $L_{51}$ may each independently be a single bond, a $C_5$-$C_{30}$ carbocyclic group that is unsubstituted or substituted with at least one $R_{10a}$, or a $C_1$-$C_{30}$ heterocyclic group that is unsubstituted or substituted with at least one $R_{10a}$,
b1 to b4 and b51 may each independently be an integer from 1 to 5,
$R_1$ to $R_6$, $R_{51}$, and $R_{52}$ may each independently be hydrogen, deuterium, -F, -Cl, -Br, -I, -$SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group, -N($Q_1$)($Q_2$),-Si($Q_3$)($Q_4$)($Q_5$), -Ge($Q_3$)($Q_4$)($Q_5$), -B($Q_6$)($Q_7$), -P(=O)($Q_8$)($Q_9$), or -P($Q_8$)($Q_9$),
c1 to c4, c51, and c52 may each independently be an integer from 1 to 5,
$A_{51}$ may be a $C_4$-$C_{60}$ alkyl group,
$A_{52}$ may be deuterium or a deuterium-containing $C_1$-$C_{60}$ alkyl group unsubstituted or substituted with at least one $C_3$-$C_{10}$ cycloalkyl group,
a1 to a4, a51, and a52 may each independently be an integer from 0 to 10, provided that the sum of a51 and a52 may be 1 or more,
a53 may be an integer from 1 to 10,
two or more groups selected from a plurality of groups $R_1$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,
two or more groups selected from a plurality of groups $R_2$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,
two or more groups selected from a plurality of groups $R_3$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,
two or more groups selected from a plurality of groups $R_4$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,
two or more groups selected from $R_1$ to $R_6$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,
$R_{10a}$ may be the same as described in connection with $R_1$,
a substituent(s) of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_7$-$C_{60}$ arylalkyl group,

the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted monovalent non-aromatic condensed polycyclic group, and the substituted monovalent non-aromatic condensed heteropolycyclic group is(are):

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a C1-$C_{60}$ alkoxy group, each substituted with deuterium, -F, -Cl, -Br, -I, $-CD_3$, $-CD_2H$,$-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{11})(Q_{12})$,$-Si(Q_{13})(Q_{14})(Q_{15})$, $-Ge(Q_{13})(Q_{14})(Q_{15})$, $-B(Q_{16})(Q_{17})$, $-P(=O)(Q_{18})(Q_{19})$, $-P(Q_{18})(Q_{19})$, or any combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{21})(Q_{22})$, $-Si(Q_{23})(Q_{24})(Q_{25})$,$-Ge(Q_{23})(Q_{24})(Q_{25})$, $-B(Q_{26})(Q_{27})$, $-P(=O)(Q_{28})(Q_{29})$, $-P(Q_{28})(Q_{29})$, or any combination thereof;

$-N(Q_{31})(Q_{32})$, $-Si(Q_{33})(Q_{34})(Q_{35})$, $-Ge(Q_{33})(Q_{34})(Q_{35})$, $-B(Q_{36})(Q_{37})$,$-P(=O)(Q_{38})(Q_{39})$, or $-P(Q_{38})(Q_{39})$; or any combination thereof; and

$Q_1$ to $Q_9$, $Q_{11}$ to $Q_{19}$, $Q_{21}$ to $Q_{29}$, and $Q_{31}$ to $Q_{39}$ are each independently hydrogen; deuterium; -F; -Cl -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a $C_1$-$C_{60}$ alkyl group unsubstituted or substituted with deuterium, a $C_1$-C60 alkyl group, a $C_6$-$C_{60}$ aryl group, or any combination thereof; a $C_2$-$C_{60}$ alkenyl group; a $C_2$-$C_{60}$ alkynyl group; a $C_1$-$C_{60}$ alkoxy group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group unsubstituted or substituted with deuterium, a $C_1$-$C_{60}$ alkyl group, a $C_6$-$C_{60}$ aryl group, or any combination thereof; a C6-C60 aryloxy group; a $C_6$-$C_{60}$ arylthio group; a $C_7$-$C_{60}$ arylalkyl group; a $C_1$-$C_{60}$ heteroaryl group; a $C_1$-$C_{60}$ heteroaryloxy group; a $C_1$-$C_{60}$ heteroarylthio group; a $C_2$-$C_{60}$ heteroarylalkyl group; a monovalent non-aromatic condensed polycyclic group; or a monovalent non-aromatic condensed heteropolycyclic group.

Another aspect of the present disclosure provides an organic light-emitting device including:

a first electrode;
a second electrode; and
an organic layer disposed between the first electrode and the second electrode,
wherein the organic layer includes an emission layer and at least one organometallic compound represented by Formula 1.

The organometallic compound included in the emission layer in the organic layer may act as a dopant.

Another aspect of the present disclosure provides a diagnostic composition including at least one organometallic compound represented by Formula 1.

BRIEF DESCRIPTION OF THE DRAWING

These and/or other aspects will become apparent and more readily appreciated from the following description of the embodiments, taken in conjunction with the FIGURE which is a schematic view of an organic light-emitting device according to an embodiment.

DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

It will be understood that when an element is referred to as being "on" another element, it can be directly in contact with the other element or intervening elements may be present therebetween. In contrast, when an element is referred to as being "directly on" another element, there are no intervening elements present.

It will be understood that, although the terms first, second, third etc. may be used herein to describe various elements, components, regions, layers, and/or sections, these elements, components, regions, layers, and/or sections should not be limited by these terms. These terms are only used to distinguish one element, component, region, layer, or section from another element, component, region, layer, or section. Thus, a first element, component, region, layer, or section discussed below could be termed a second element, component, region, layer, or section without departing from the teachings of the present embodiments.

The terminology used herein is for the purpose of describing particular embodiments only and is not intended to be limiting. As used herein, the singular forms "a," "an," and "the" are intended to include the plural forms as well, unless the context clearly indicates otherwise.

The term "or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art to which this general inventive concept belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

"About" or "approximately" as used herein is inclusive of the stated value and means within an acceptable range of deviation for the particular value as determined by one of ordinary skill in the art, considering the measurement in question and the error associated with measurement of the particular quantity (*i.e.,* the limitations of the measurement system). For example, "about" can mean within one or more standard deviations, or within $\pm$ 30%, 20%, 10%, 5% of the stated value.

In an embodiment, an organometallic compound is provided. The organometallic compound according to an embodiment may be represented by Formula 1:

Formula 1

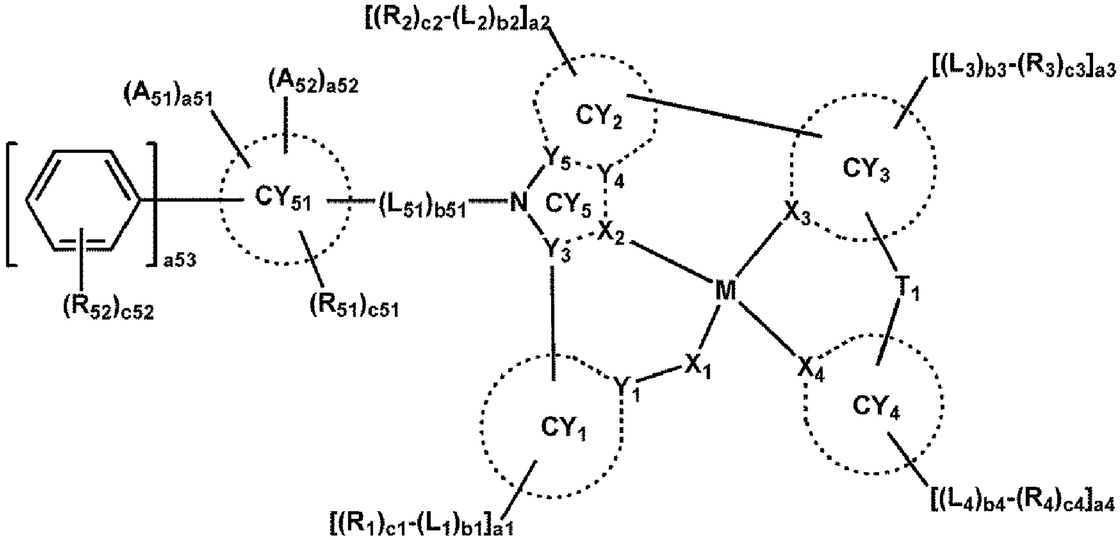

.

In Formula 1, M may be a transition metal. Alternatively, M may be beryllium (Be), magnesium (Mg), aluminum (Al), calcium (Ca), titanium (Ti), manganese (Mn), cobalt (Co), copper (Cu), zinc (Zn), gallium (Ga), germanium (Ge), zirconium (Zr), ruthenium (Ru), rhodium (Rh), palladium (Pd), silver (Ag), rhenium (Re), platinum (Pt), or gold (Au). For example, M may be Pt, Pd, or Au.

In Formula 1, $X_1$ may be O or S, wherein a bond between $X_1$ and M may be a covalent bond. For example, $X_1$ may be O.

In Formula 1, $X_2$ to $X_4$ may each independently be C or N. For example, $X_2$ and $X_3$ may each independently be C or N, and $X_4$ may be N.

In an embodiment, in Formula 1, $X_2$ and $X_4$ may each be N, and $X_3$ may be C, but embodiments of the present disclosure are not limited thereto.

In Formula 1, one bond selected from a bond between $X_2$ and M, a bond between $X_3$ and M, and a bond between $X_4$ and M may be a covalent bond, and the other bonds selected from a bond between $X_2$ and M, a bond between $X_3$ and M, and a bond between $X_4$ and M may be coordinate bonds. Therefore, the organometallic compound represented by Formula 1 may be electrically neutral.

In an embodiment, a bond between $X_2$ and M and a bond between $X_4$ and M may be a coordinate bond, and a bond between $X_3$ and M may be a covalent bond, but embodiments of the present disclosure are not limited thereto.

In Formula 1, $Y_1$ and $Y_3$ to $Y_5$ may each independently be C or N. For example, $Y_1$ and $Y_3$ to $Y_5$ may be C.

In Formula 1, a bond between $X_2$ and $Y_3$, a bond between $X_2$ and $Y_4$, and a bond between $Y_4$ and $Y_5$ may be a chemical bond (for example, a single bond, a double bond, or the like).

In Formula 1, ring $CY_1$ to ring $CY_4$ and ring $CY_{51}$ may each independently be a $C_5$-$C_{30}$ carbocyclic group or a $C_1$-$C_{30}$ heterocyclic group.

For example, ring $CY_1$ to ring $CY_4$ and ring $CY_{51}$ may each independently be i) a first ring, ii) a second ring, iii) a condensed ring in which at least two first rings are condensed, iv) a condensed ring in which at least two second rings are condensed, or v) a condensed ring in which at least one first ring and at least one second ring are condensed.

In an embodiment, the first ring may be a cyclopentane group, a cyclopentadiene group, a furan group, a thiophene group, a pyrrole group, a silole group, an indene group, a benzofuran group, a benzothiophene group, an indole group, a benzosilole group, an oxazole group, an isoxazole group, an oxadiazole group, an isozadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group, and

the second ring may be an adamantane group, a norbornane group, a norbornene group, a bicyclo[1.1.1]pentane group, a bicyclo[2.1.1]hexane group, a bicyclo[2.2.1]heptane group, a bicyclo[2.2.2]octane group, a cyclohexane group, a cyclohexene group, a benzene group, a pyridine group, a pyrimidine group, a pyrazine group, a pyridazine group, or a triazine group.

In an embodiment, in Formula 1, ring $CY_1$ to ring $CY_4$ and ring $CY_{51}$ may each independently be a cyclopentane group, a cyclohexane group, a cycloheptane group, a cyclopentene group, a cyclohexene group, a cycloheptene group, a benzene group, a naphthalene group, an anthracene group, a phenanthrene group, a triphenylene group, a pyrene

group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In one or more embodiments, in Formula 1, ring $CY_4$ may not be a benzimidazole group.

In Formula 1, a cyclometalated ring formed by ring $CY_5$, ring $CY_2$, ring $CY_3$, and M may each independently be a 6-membered ring.

In Formula 1, $T_1$ may be a single bond, a double bond, *-N($R_5$)-*', *-B($R_5$)-*', *-P($R_5$)-*', *-C($R_5$)($R_6$)-*', *-Si($R_5$)($R_6$)-*', *-Ge($R_5$)($R_6$)-*', *-S-*', *-Se-*', *-O-*', *-C(=O)-*', *-S(=O)-*', *-S(=O)$_2$-*', *-C($R_5$)=*', *=C($R_5$)-*', *-C($R_5$)=C($R_6$)-*', *-C(=S)-*', or *-C≡C-*', wherein * and *' each indicate a binding site to a neighboring atom. Here, $R_5$ and $R_6$ are the same as described above, and may optionally be linked to each other via a single bond, a double bond, *-N(R')-*', *-B(R')-*', *-P(R')-*', *-C(R')(R")-*', *-Si(R')(R")-*', *-Ge(R')(R")-*', *-S-*', *-Se-*', *-O-*', *-C(=O)-*', *-S(=O)-*', *-S(=O)$_2$-*', *-C(R')=*', *=C(R')-*', *-C(R')=C(R")-*', *-C(=S)-*', or *-C≡C-*' to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$. R', R", and $R_{10a}$ are each independently defined the same as $R_1$.

For example, in Formula 1, $T_1$ may be a single bond, but embodiments of the present disclosure are not limited thereto.

In Formula 1, $L_1$ to $L_4$ and $L_{51}$ may each independently be a single bond, a $C_5$-$C_{30}$ carbocyclic group that is unsubstituted or substituted with at least one $R_{10a}$, or a $C_1$-$C_{30}$ heterocyclic group that is unsubstituted or substituted with at least one $R_{10a}$. Here, $R_{10a}$ is defined the same as $R_1$.

For example, in Formula 1, $L_1$ to $L_4$ and $L_{51}$ may each independently be:

a single bond; or

a benzene group, a naphthalene group, an anthracene group, a phenanthrene group, a triphenylene group, a pyrene group, a chrysene group, a cyclopentadiene group, a furan group, a thiophene group, a silole group, an indene group, a fluorene group, an indole group, a carbazole group, a benzofuran group, a dibenzofuran group, a benzothiophene group, a dibenzothiophene group, a benzosilole group, a dibenzosilole group, an azafluorene group, an azacarbazole group, an azadibenzofuran group, an azadibenzothiophene group, an azadibenzosilole group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, or a benzothiadiazole group, each unsubstituted or substituted with at least one $R_{10a}$.

In an embodiment, in Formula 1, $L_1$ to $L_4$ and $L_{51}$ may each independently be:

a single bond; or

a benzene group unsubstituted or substituted with at least one $R_{10a}$, but embodiments of the present disclosure are not limited thereto.

In an embodiment, in Formula 1,

$Y_1$ and $X_3$ may be C, and $X_2$ and $X_4$ may be N,

ring $CY_1$ to ring $CY_3$ and ring $CY_{51}$ may be a benzene group, and ring $CY_4$ may be a pyridine group, and

$L_4$ and $L_{51}$ may each independently be:

a single bond; or
a benzene group unsubstituted or substituted with at least one $R_{10a}$.

In Formula 1, b1 to b4 and b51 each indicate the number of $L_1$ to $L_4$ and $L_{51}$, respectively, and may each independently be an integer from 1 to 5. When b1 is two or more, two or more groups $L_1$ may be identical to or different from each other, when b2 is two or more, two or more groups $L_2$ may be identical to or different from each other, when b3 is two or more, two or more groups $L_3$ may be identical to or different from each other, when b4 is two or more, two or more groups $L_4$ may be identical to or different from each other, and when b51 is two or more, two or more groups $L_{51}$ may be identical to or different from each other.

For example, in Formula 1, b1 to b4 and b51 may each independently be 1, 2 or 3, but embodiments of the present disclosure are not limited thereto.

In Formula 1, $R_1$ to $R_6$, $R_{51}$, and $R_{52}$ may each independently be hydrogen, deuterium, -F, -Cl, -Br, -I, $-SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group, $-N(Q_1)(Q_2)$, $-Si(Q_3)(Q_4)(Q_5)$, $-Ge(Q_3)(Q_4)(Q_5)$,$-B(Q_6)(Q_7)$, $-P(=O)(Q_8)(Q_9)$, or $-P(Q_8)(Q_9)$, wherein $Q_1$ to $Q_9$ are each independently the same as described above.

For example, $R_1$ to $R_6$, $R_{51}$, and $R_{52}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, $-SF_5$, $C_1$-$C_{20}$ alkyl group, or a $C_1$-$C_{20}$ alkoxy group;
a $C_1$-$C_{20}$ alkyl group and a $C_1$-$C_{20}$ alkoxy group, each substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a cyclopentyl group, a cyclohexyl group, a cycloheptyl group, a cyclooctyl group, an adamantanyl (adamantyl) group, a norbornanyl (norbornyl) group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl (adamantyl) group, a norbornanyl (norbornyl) group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group,

a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a deuterium-containing $C_2$-$C_{20}$ alkyl group (for example, *-$C(CD_3)_3$), a $C_1$-$C_{20}$ alkoxy group, a cyclopentyl group, a cyclohexyl group, a cycloheptyl group, a cyclooctyl group, an adamantanyl (adamantyl) group, a norbornanyl (norbornyl) group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-C20 alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or

-$N(Q_1)(Q_2)$, -$Si(Q_3)(Q_4)(Q_5)$, -$Ge(Q_3)(Q_4)(Q_5)$, -$B(Q_6)(Q_7)$,-$P(=O)(Q_8)(Q_9)$, or -$P(Q_8)(Q_9)$, and

$Q_1$ to $Q_9$ may each independently be:

-$CH_3$, -$CD_3$, -$CD_2H$, -$CDH_2$, -$CH_2CH_3$, -$CH_2CD_3$, -$CH_2CD_2H$,-$CH_2CDH_2$, -$CHDCH_3$, -$CHDCD_2H$, -$CHDCDH_2$, -$CHDCD_3$, -$CD_2CD_3$,-$CD_2CD_2H$, or -$CD_2CDH_2$; or

an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, a sec-iso-pentyl group, a phenyl group, a biphenyl group or a naphthyl group, each unsubstituted or substituted with deuterium, a $C_1$-$C_{10}$ alkyl group, a phenyl group, or any combination thereof,

but embodiments of the present disclosure are not limited thereto.

In Formula 1, c1 to c4, c51, and c52 each indicate the number of $R_1$ to $R_4$, $R_{51}$, and $R_{52}$, respectively, and may each independently be an integer from 1 to 5. When c1 is two or more, two or more groups $R_1$ may be identical to or different from each other, when c2 is two or more, two or more groups $R_2$ may be identical to or different from each other, when c3 is two or more, two or more groups $R_3$ may be identical to or different from each other, when c4 is two or more, two or more groups $R_4$ may be identical to or different from each other, when c51 is two or more, two or more groups $R_{51}$ may be identical to or different from each other, and when c52 is two or more, two or more groups $R_{52}$ may be identical to or different from each other. For example, c1 to c4, c51, and c52 may each independently be 1 or 2, but embodiments of the present disclosure are not limited thereto.

In Formula 1, $A_{51}$ may be a $C_4$-$C_{60}$ alkyl group, and $A_{52}$ may be deuterium or a deuterium-containing $C_1$-$C_{60}$ alkyl group unsubstituted or substituted with at least one $C_3$-$C_{10}$ cycloalkyl group.

In an embodiment, in Formula 1, $A_{51}$ may be a linear or branched $C_4$-$C_{10}$ alkyl group, and $A_{52}$ may be deuterium or a deuterium-containing linear or branched $C_1$-$C_{20}$ alkyl group unsubstituted or substituted with at least one $C_3$-$C_{10}$ cycloalkyl group.

In an embodiment, in Formula 1, $A_{51}$ may be an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, or a sec-iso-pentyl group, unsubstituted or substituted with a methyl group, an ethyl group, an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, a sec-iso-pentyl group, or any combination thereof. For example, Formula 9-33 may be a branched $C_6$ alkyl group and a tert-butyl group substituted with two methyl groups.

In an embodiment, in Formula 1, $A_{52}$ may be a deuterium-containing linear or branched $C_1$-$C_{20}$ alkyl group unsubstituted or substituted with at least one $C_3$-$C_{10}$ cycloalkyl group, in which the linear or branced $C_1$-$C_{20}$ alkyl group may be a methyl group, an ethyl group, an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, or a sec-iso-pentyl group, unsubstituted or substituted with a methyl group, an ethyl

group, an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, a sec-iso-pentyl group, or any combination thereof.

The deuterium-containing $C_1$-$C_{60}$ alkyl group (or, the deuterium-containing C1-C20 alkyl group, the deuterium-containing $C_2$-$C_{20}$ alkyl group, etc.) means a $C_1$-$C_{60}$ alkyl group substituted with at least one deuterium (or, a $C_1$-$C_{20}$ alkyl group substituted with at least one deuterium, a $C_2$-$C_{20}$ alkyl group substituted with at least one deuterium, etc.). For example, a deuterium-containing $C_1$ alkyl group (that is, a deuterium-containing methyl group) includes $-CD_3$, $-CD_2H$ and $-CDH_2$.

The deuterium-containing $C_1$-$C_{60}$ alkyl group (or, the deuterium-containing C1-C20 alkyl group, the deuterium-containing $C_2$-$C_{20}$ alkyl group, etc.) may be additionally substituted with at least one $C_3$-$C_{10}$ cycloalkyl group. For example, Formula 9-619 is a group corresponding to $-CD_2H$, in which "-H" is substituted with a cyclopentyl group.

The organometallic compound represented by Formula 1 may include at least one selected from deuterium and a deuterium-containing $C_1$-$C_{60}$ alkyl group unsubstituted or substituted with at least one $C_3$-$C_{10}$ cycloalkyl group. The number of deuterium atoms in the organometallic compound represented by Formula 1 may be from 1 to 20, for example, from 1 to 15, from 1 to 10, or from 1 to 5.

In one or more embodiments, regarding Formula 1,

$R_1$ to $R_6$, $R_{51}$, and $R_{52}$ may each independently be:

hydrogen, deuterium, -F, a cyano group, a nitro group, $-SF_5$, $-CH_3$,$-CD_3$, $-CD_2H$, $-CDH_2$, $-CF_3$, $-CF_2H$, $-CFH_2$, a group represented by one of Formulae 9-1 to 9-66, a group represented by one of Formulae 9-1 to 9-66 in which at least one hydrogen is substituted with deuterium, a group represented by one of Formulae 10-1 to 10-118, a group represented by one of Formulae 10-1 to 10-118 in which at least one hydrogen is substituted with deuterium, a group represented by one of Formulae 10-201 to 10-342, or a group represented by one of Formulae 10-201 to 10-342 in which at least one hydrogen is substituted with deuterium, (and/or)

$A_{51}$ may be a group represented by one Formulae 9-4 to 9-36, (and/or)

$A_{52}$ may be a group represented by one of Formulae 9-1 to 9-63 in which at least one hydrogen is substituted with deuterium, (and/or)

a group represented by

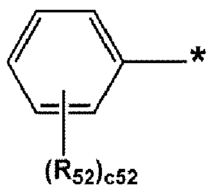

in Formula 1 may be a group represented by one of Formulae 10-10 to 10-118, or a group represented by one of Formulae 10-10 to 10-118 in which at least one hydrogen is substituted with deuterium:

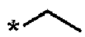
9-1
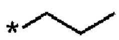
9-2
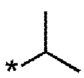
9-3
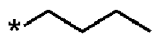
9-4
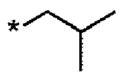
9-5
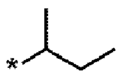
9-6
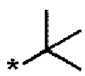
9-7
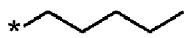
9-8

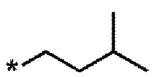
9-9
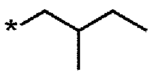
9-10
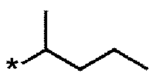
9-11
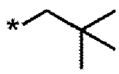
9-12
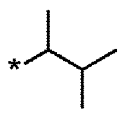
9-13
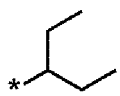
9-14
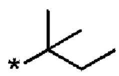
9-15

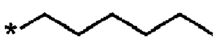
9-16
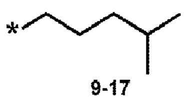
9-17
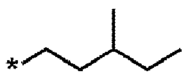
9-18
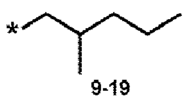
9-19
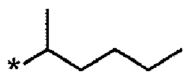
9-20
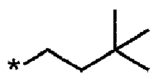
9-21

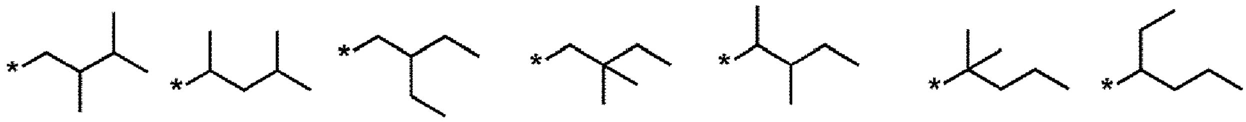

9-22 9-23 9-24 9-25 9-26 9-27 9-28

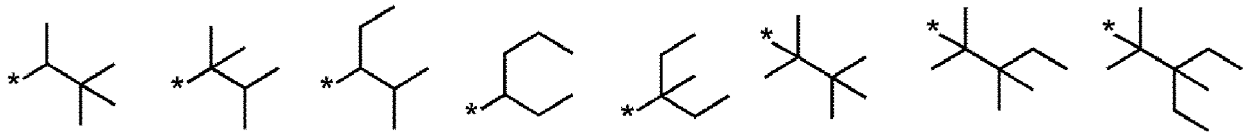

9-29 9-30 9-31 9-32 9-33 9-34 9-35 9-36

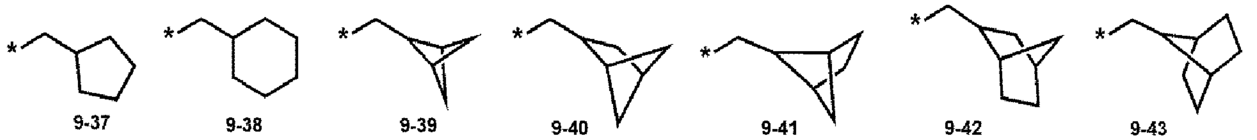

9-37 9-38 9-39 9-40 9-41 9-42 9-43

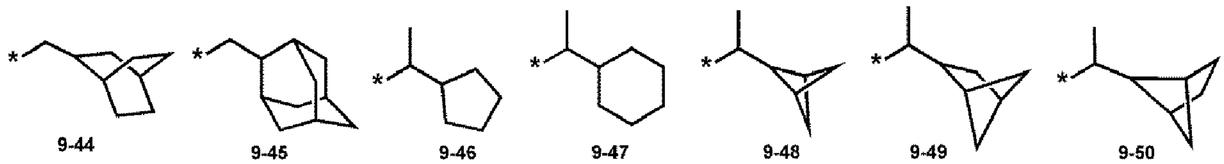

9-44 9-45 9-46 9-47 9-48 9-49 9-50

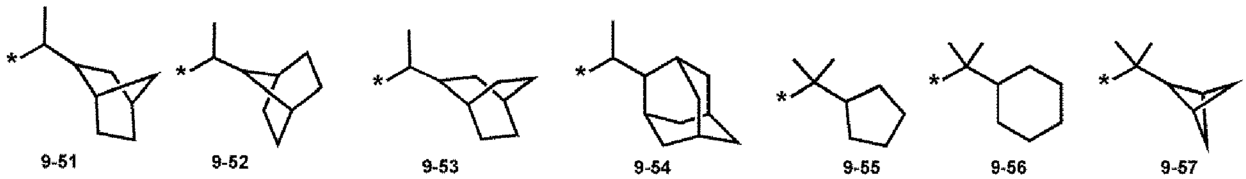

9-51 9-52 9-53 9-54 9-55 9-56 9-57

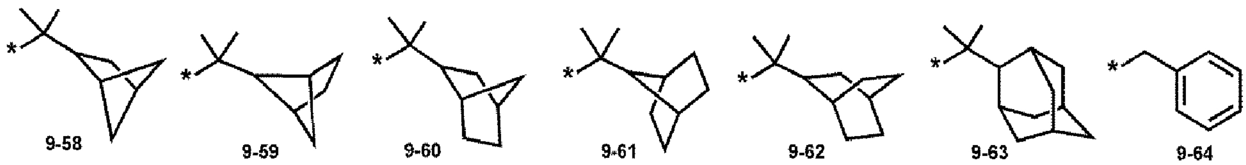

9-58 9-59 9-60 9-61 9-62 9-63 9-64

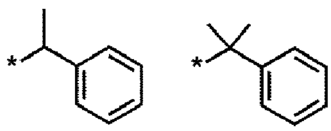

9-65 9-66

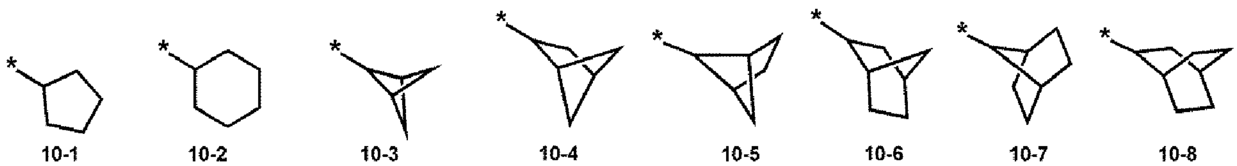

10-1 10-2 10-3 10-4 10-5 10-6 10-7 10-8

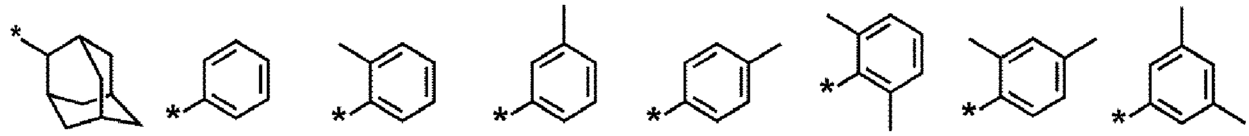
10-9 10-10 10-11 10-12 10-13 10-14 10-15 10-16

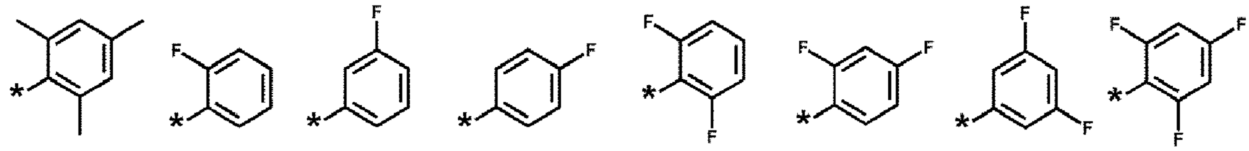
10-17 10-18 10-19 10-20 10-21 10-22 10-23 10-24

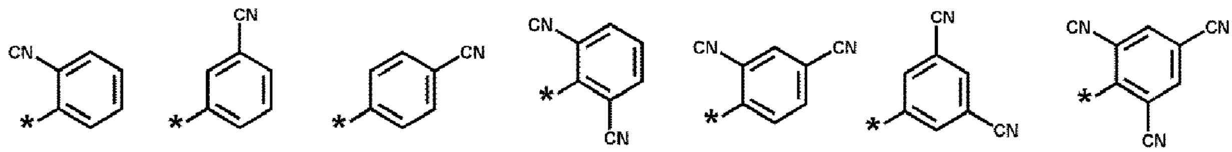
10-25 10-26 10-27 10-28 10-29 10-30 10-31

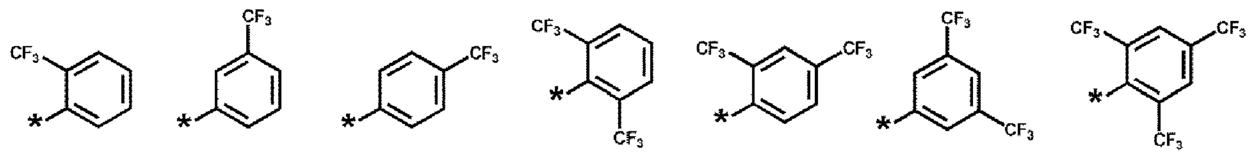
10-32 10-33 10-34 10-35 10-36 10-37 10-38

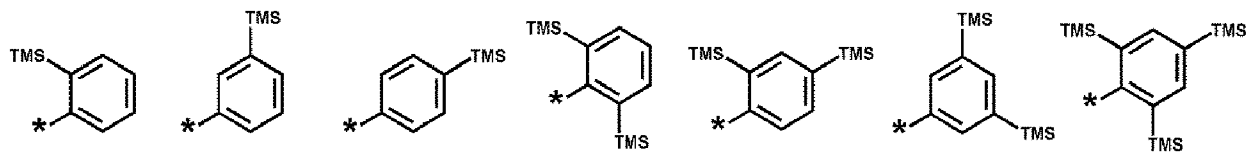
10-39 10-40 10-41 10-42 10-43 10-44 10-45

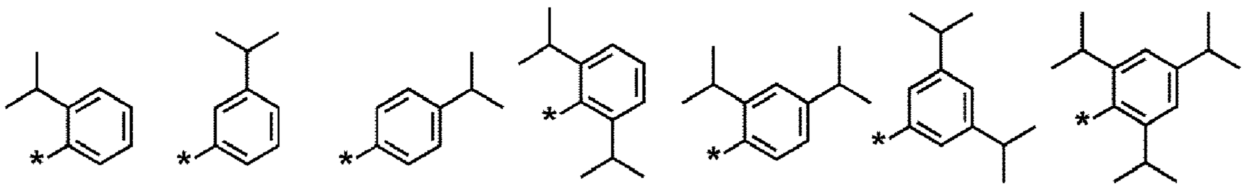
10-46 10-47 10-48 10-49 10-50 10-51 10-52

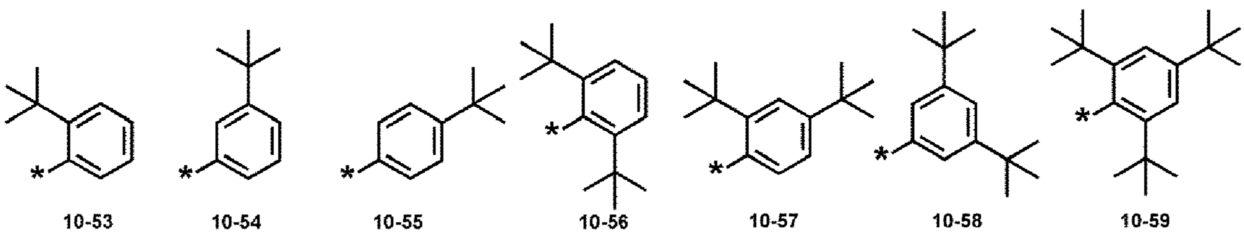
10-53 10-54 10-55 10-56 10-57 10-58 10-59

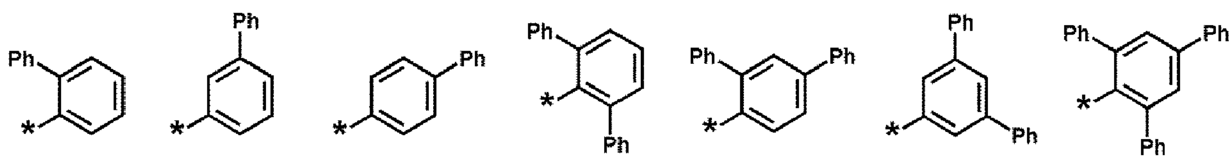
10-60 10-61 10-62 10-63 10-64 10-65 10-66

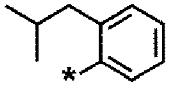
10-67

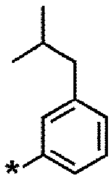
10-68

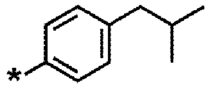
10-69

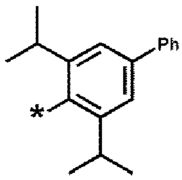
10-70

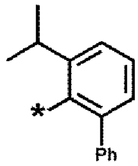
10-71

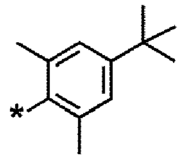
10-72

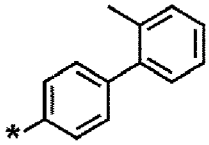
10-73

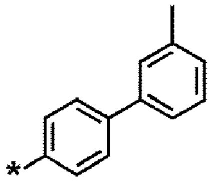
10-74

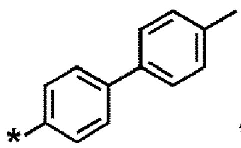
10-75

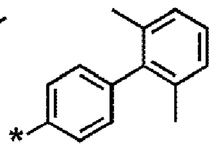
10-76

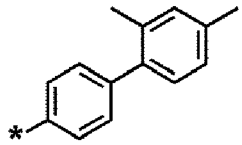
10-77

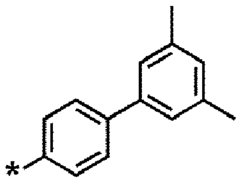
10-78

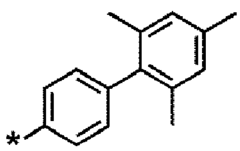
10-79

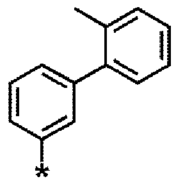
10-80

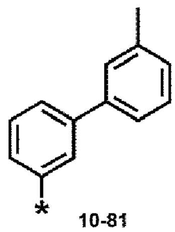
10-81

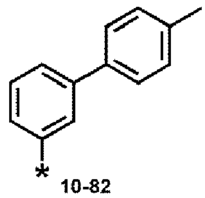
10-82

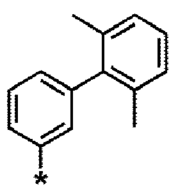
10-83

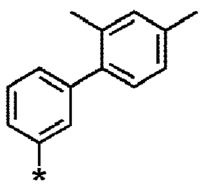
10-84

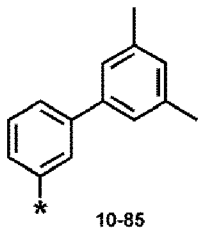
10-85

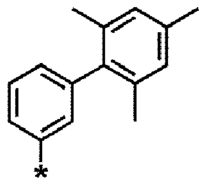
10-86

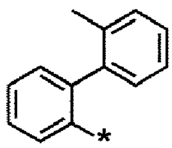
10-87

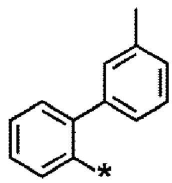
10-88

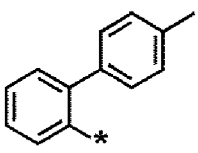
10-89

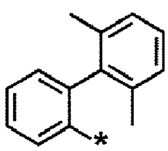
10-90

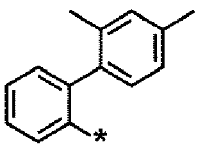
10-91

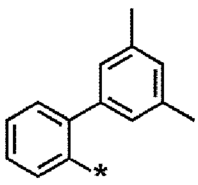
10-92

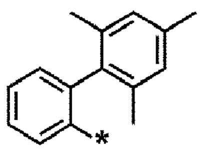
10-93

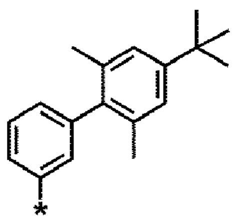
10-94

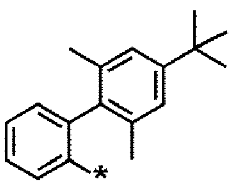
10-95

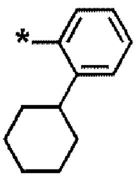
10-96

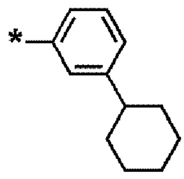
10-97

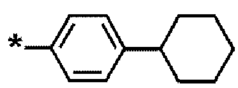
10-98

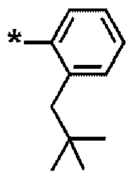
10-99

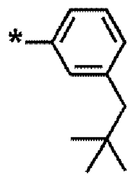
10-100

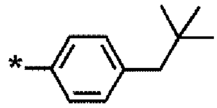 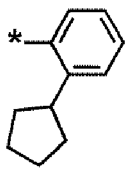 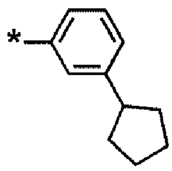 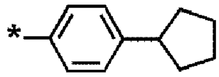 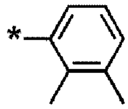 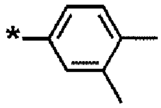

10-101 10-102 10-103 10-104 10-105 10-106

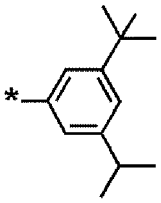 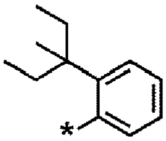 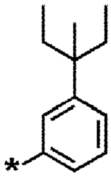 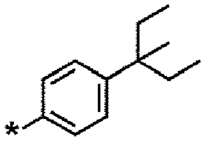 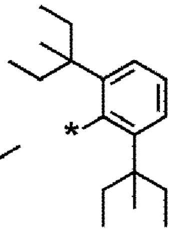 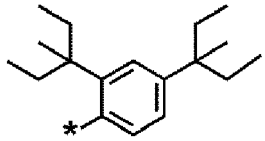

10-107 10-108 10-109 10-110 10-111 10-112

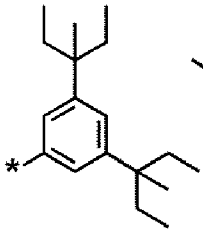 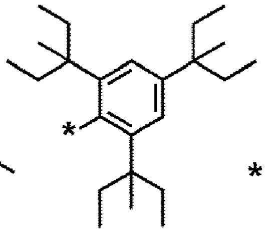 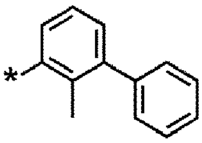 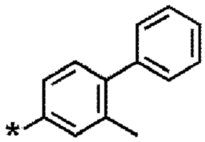 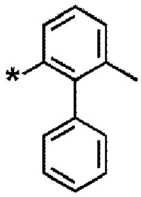 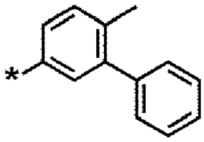

10-113 10-114 10-115 10-116 10-117 10-118

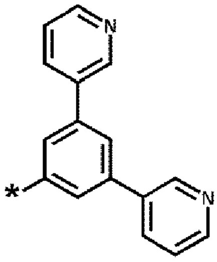 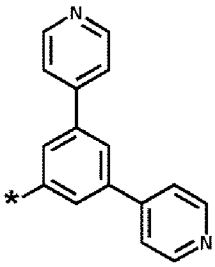 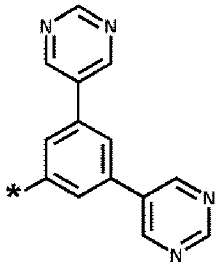 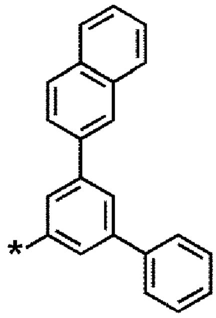 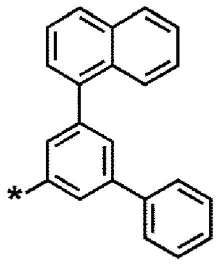

10-201 10-202 10-203 10-204 10-205

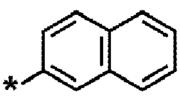 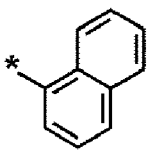 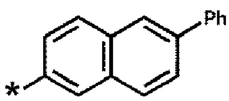 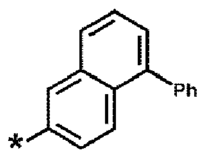 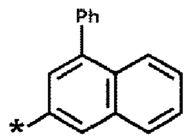

10-206 10-207 10-208 10-209 10-210

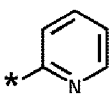 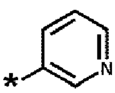 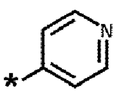 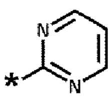 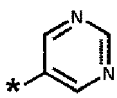 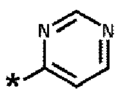 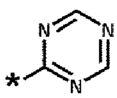

10-211 10-212 10-213 10-214 10-215 10-216 10-217

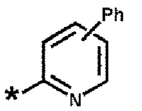
10-218

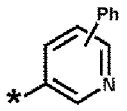
10-219

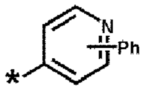
10-220

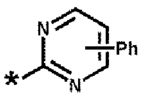
10-221

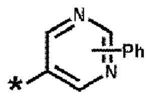
10-222

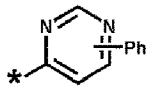
10-223

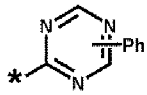
10-224

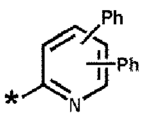
10-225

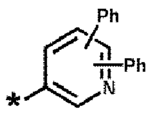
10-226

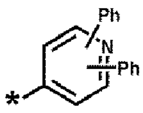
10-227

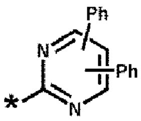
10-228

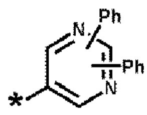
10-229

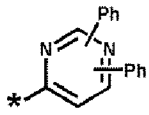
10-230

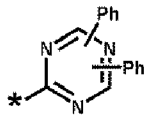
10-231

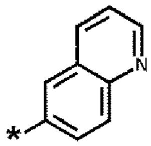
10-232

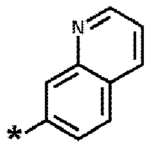
10-233

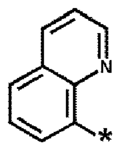
10-234

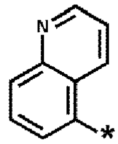
10-235

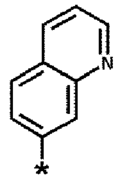
10-236

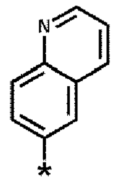
10-237

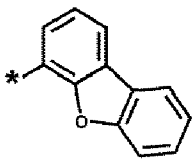
10-238

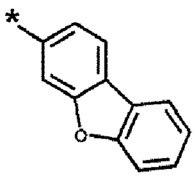
10-239

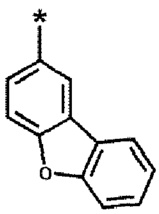
10-240

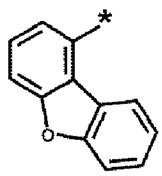
10-241

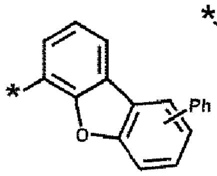
10-242

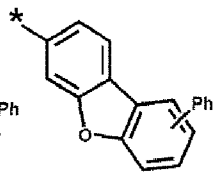
10-243

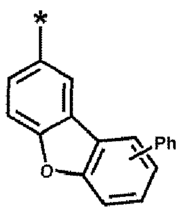
10-244

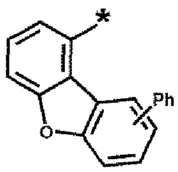
10-245

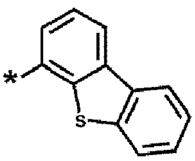
10-246

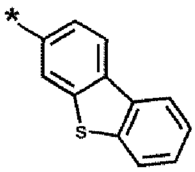
10-247

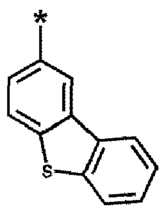
10-248

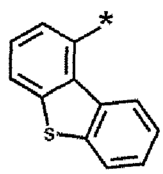
10-249

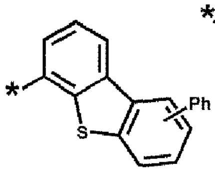
10-250

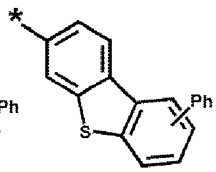
10-251

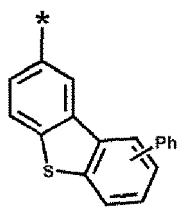
10-252

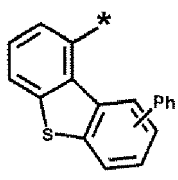
10-253

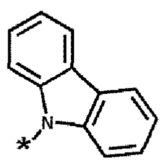
10-254

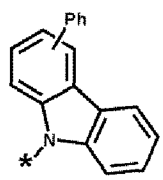
10-255

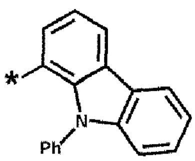
10-256

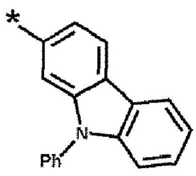
10-257

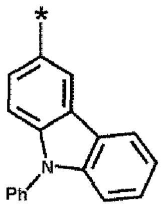
10-258

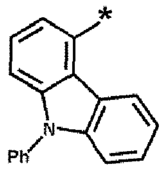
10-259

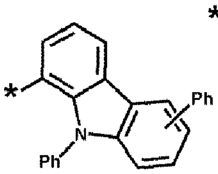
10-260

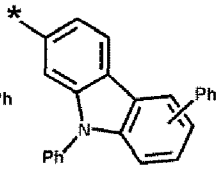
10-261

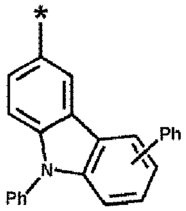
10-262

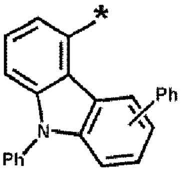
10-263

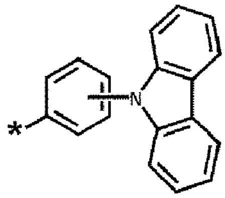
10-264

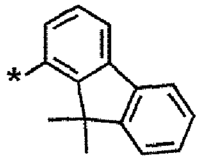
10-265

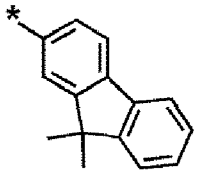
10-266

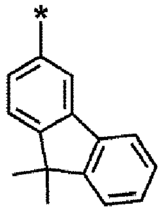
10-267

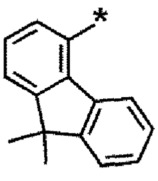
10-268

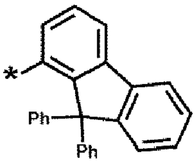
10-269

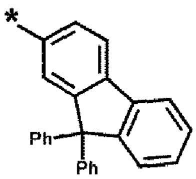
10-270

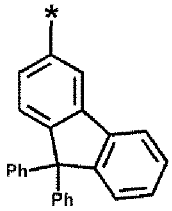
10-271

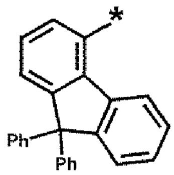
10-272

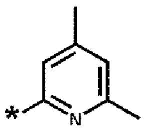
10-273

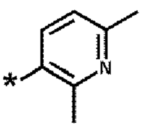
10-274

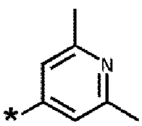
10-275

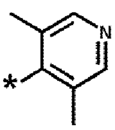
10-276

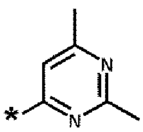
10-277

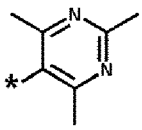
10-278

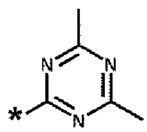
10-279

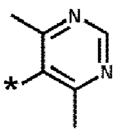
10-280

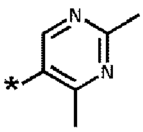
10-281

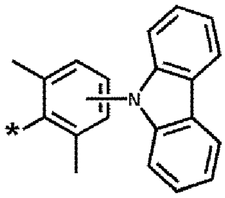
10-282

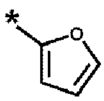
10-283

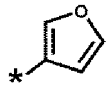
10-284

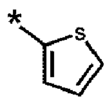
10-285

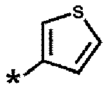
10-286

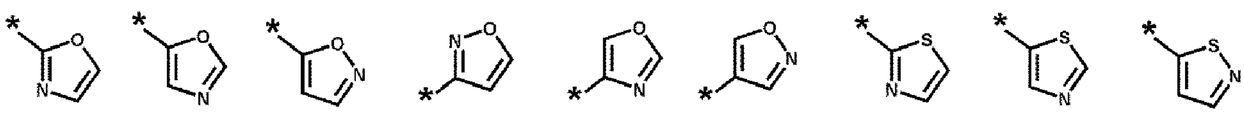
10-287 10-288 10-289 10-290 10-291 10-292 10-293 10-294 10-295

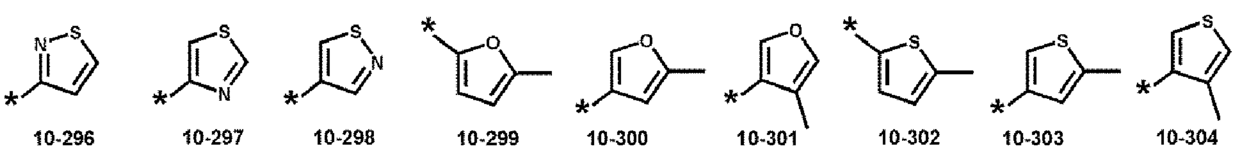
10-296 10-297 10-298 10-299 10-300 10-301 10-302 10-303 10-304

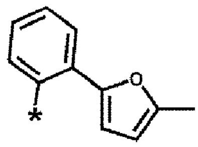
10-305

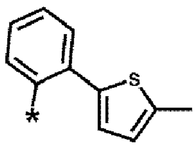
10-306

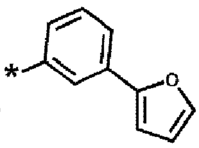
10-307

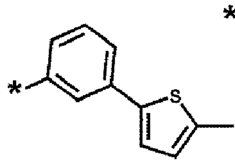
10-308

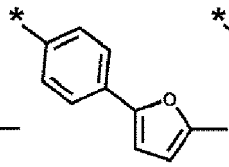
10-309

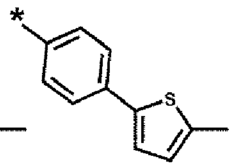
10-310

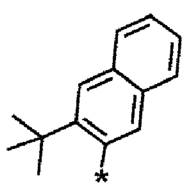
10-311

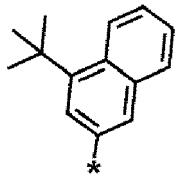
10-312

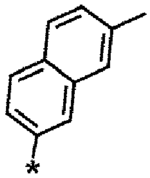
10-313

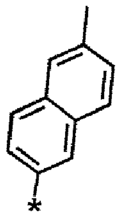
10-314

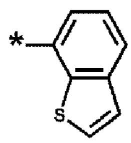
10-315

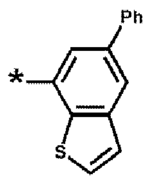
10-316

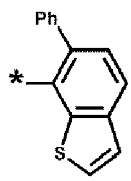
10-317

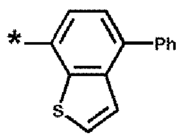
10-318

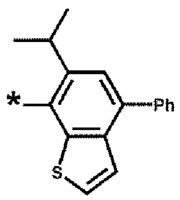
10-319

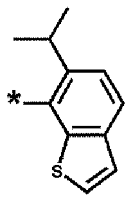
10-320

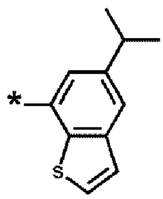
10-321

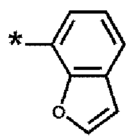
10-322

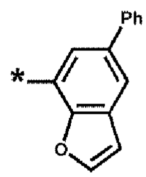
10-323

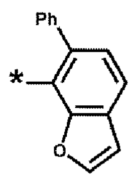
10-324

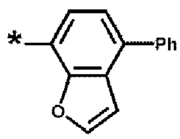
10-325

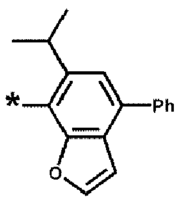
10-326

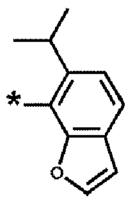
10-327

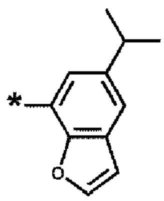
10-328

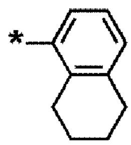
10-329

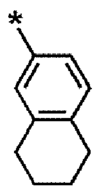
10-330

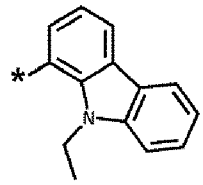
10-331

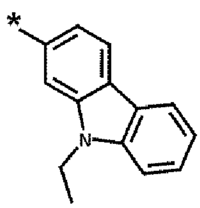
10-332

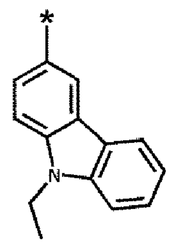
10-333

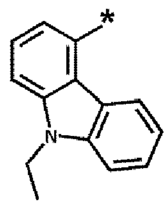
10-334

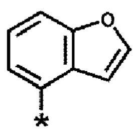
10-335

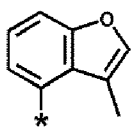
10-336

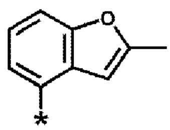
10-337

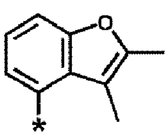
10-338

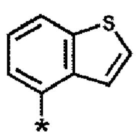
10-339

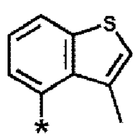
10-340

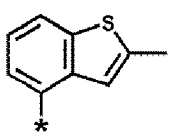
10-341

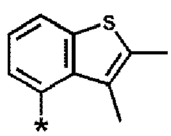
10-342

.

In Formulae 9-1 to 9-66, 10-1 to 10-118 and 10-201 to 10-342, * indicates a binding site to a neighboring atom, Ph indicates a phenyl group, and TMS indicates a trimethylsilyl group.

The "group represented by one of Formulae 9-1 to 9-66 in which at least one hydrogen is substituted with deuterium" may be, for example, a group represented by one of Formulae 9-501 to 9-514 and 9-601 to 9-638.

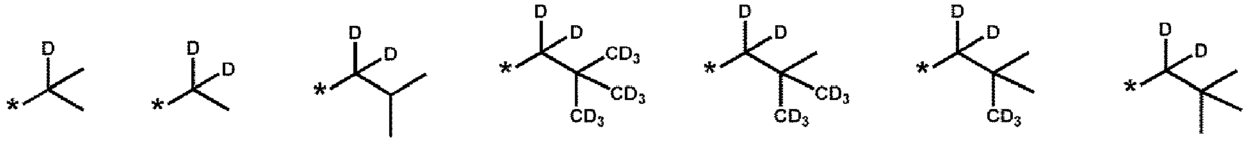
9-501 9-502 9-503 9-504 9-505 9-506 9-507

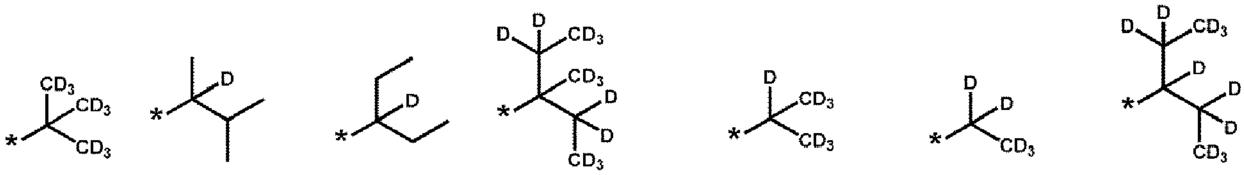
9-508 9-509 9-510 9-511 9-512 9-513 9-514

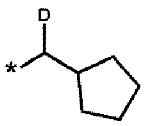
9-601

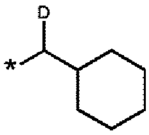
9-602

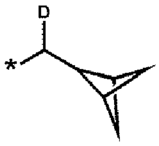
9-603

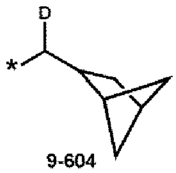
9-604

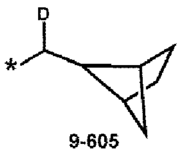
9-605

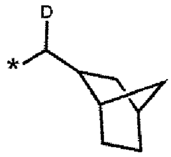
9-606

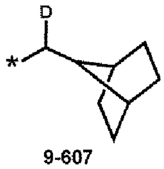
9-607

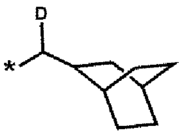
9-608

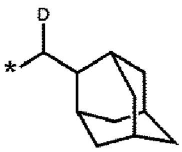
9-609

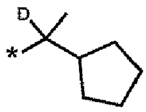
9-610

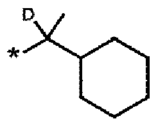
9-611

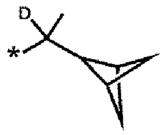
9-612

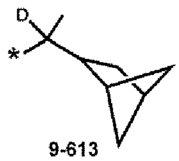
9-613

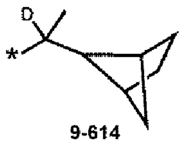
9-614

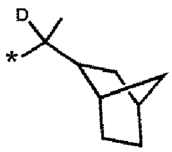
9-615

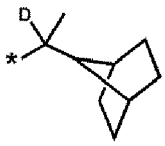
9-616

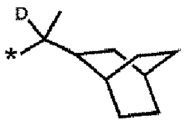
9-617

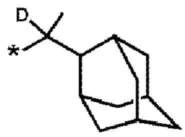
9-618

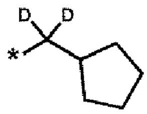
9-619

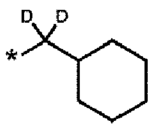
9-620

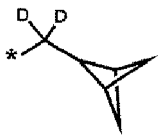
9-621

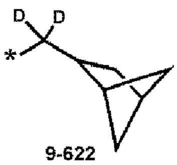
9-622

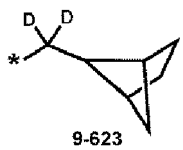
9-623

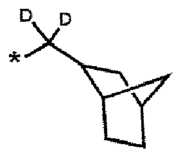
9-624

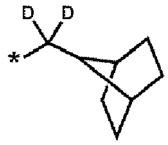
9-625

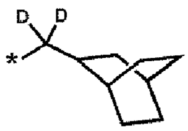
9-626

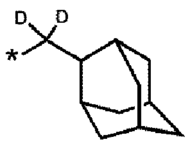
9-627

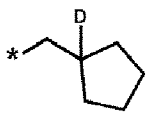
9-628

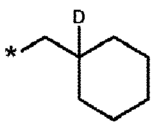
9-629

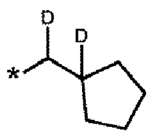
9-630

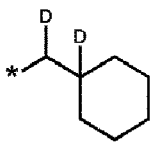
9-631

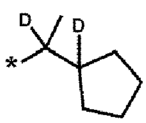
9-632

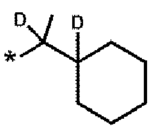
9-633

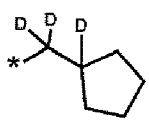
9-634

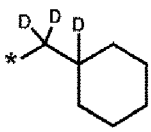
9-635

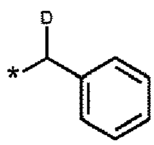
9-636

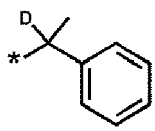
9-637

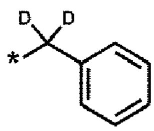
9-638

.

The "group represented by one of Formulae 10-1 to 10-118 in which at least one hydrogen is substituted with deuterium" may be, for example, a group represented by one of Formulae 10-501 to 10-552:

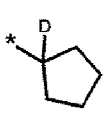
10-501
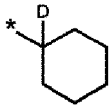
10-502
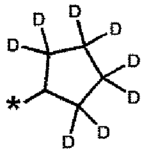
10-503
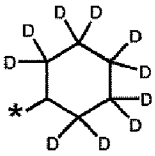
10-504
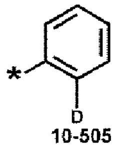
10-505
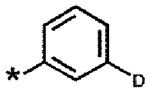
10-506
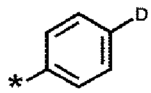
10-507
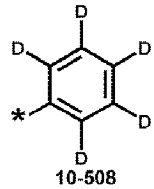
10-508

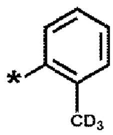
10-509
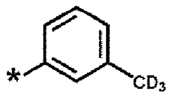
10-510
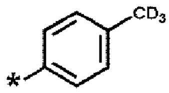
10-511
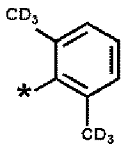
10-512
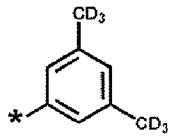
10-513
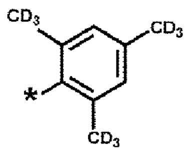
10-514
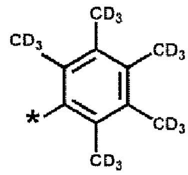
10-515

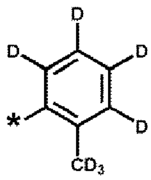
10-516
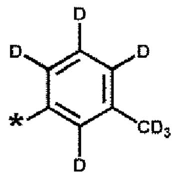
10-517
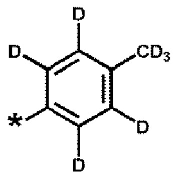
10-518
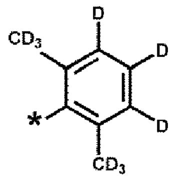
10-519
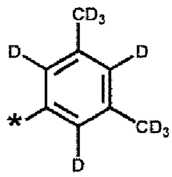
10-520
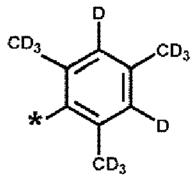
10-521

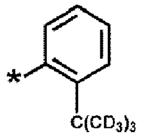
10-522
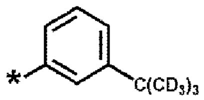
10-523
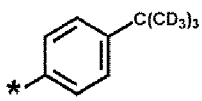
10-524
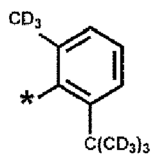
10-525
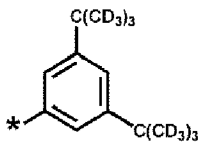
10-526
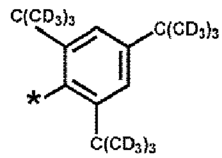
10-527

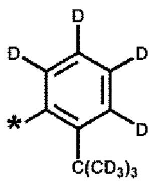
10-528
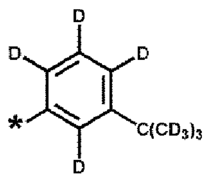
10-529
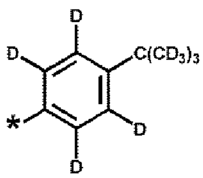
10-530
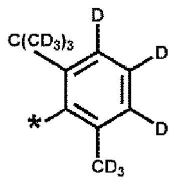
10-531
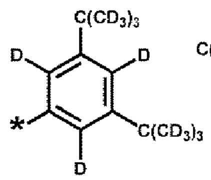
10-532
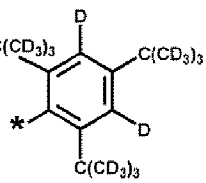
10-533

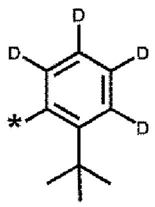
10-534
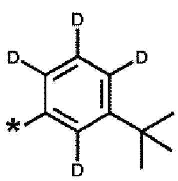
10-535
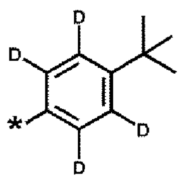
10-536
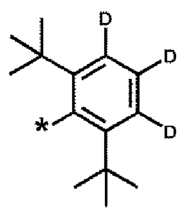
10-537
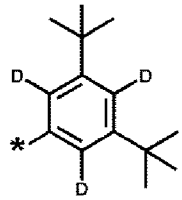
10-538
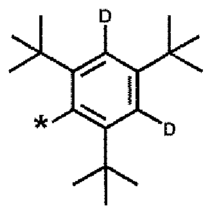
10-540

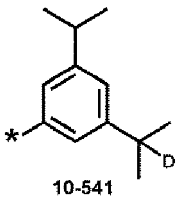 10-541 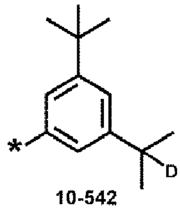 10-542 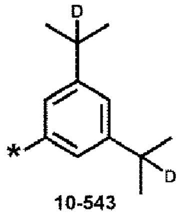 10-543 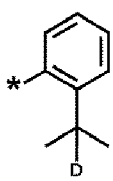 10-544 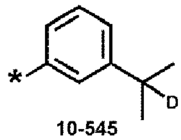 10-545 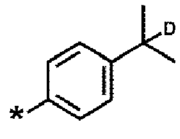 10-546

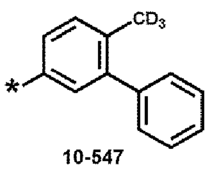 10-547 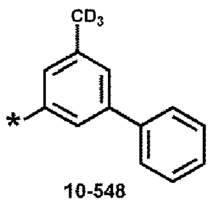 10-548 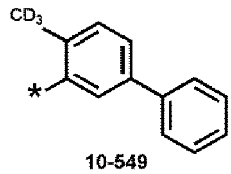 10-549 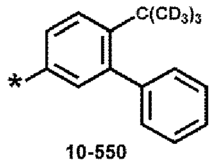 10-550 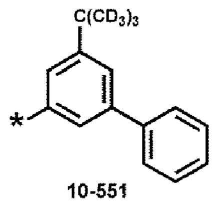 10-551

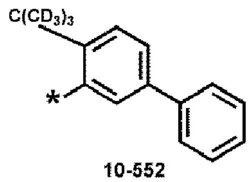 10-552

.

In an embodiment, in Formula 1, $A_{52}$ may be a group represented by one of Formulae 9-1 to 9-36 in which all hydrogens are substituted with deuterium, but embodiments of the present disclosure are not limited thereto.

In Formula 1, a1 to a4, a51, and a52 each indicate the number of $*\text{-}[(L_1)b_1\text{-}(R_1)_{c1}]$, $*\text{-}[(L_2)_{b2}\text{-}(R_2)_{c2}]$, $*\text{-}[(L_3)_{b3}\text{-}(R_3)_{c3}]$, $*\text{-}[(L_4)_{b4}\text{-}(R_4)_{c4}]$, $A_{51}$, and $A_{52}$, respectively, and may each independently be an integer from 0 to 10. When a1 is two or more, two or more groups $*\text{-}[(L_1)_{b1}\text{-}(R_1)c_1]$ may be identical to or different from each other, when a2 is two or more, two or more groups $*\text{-}[(L_2)_{b2}\text{-}(R_2)_{c2}]$ may be identical to or different from each other, when a3 is two or more, two or more groups $*\text{-}[(L_3)_{b3}\text{-}(R_3)_{c3}]$ may be identical to or different from each other, when a4 is two or more, two or more groups $*\text{-}[(L_4)_{b4}\text{-}(R_4)_{c4}]$ may be identical to or different from each other, when a51 is two or more, two or more groups $A_{51}$ may be identical to or different from each other, and when a52 is two or more, two or more groups $A_{52}$ may be identical to or different from each other, but embodiments of the present disclosure are not limited thereto.

For example, in Formula 1, a1 to a4, a51, and a52 may each independently be 0, 1, 2, 3, 4, 5, or 6, but embodiments of the present disclosure are not limited thereto.

For example, in Formula 1, a1 and a4 may each independently be 0, 1, 2, 3, or 4 and a2, a3, a51 and a52 may each independently be 0, 1, 2, or 3.

In Formula 1, the sum of a51 and a52 may be 1 or more. That is, in Formula 1, ring $CY_{51}$ may be essentially substituted with at least one selected from a group represented by $A_{51}$ and a group represented by $A_{52}$.

For example, the sum of a51 and a52 may be 1, 2, or 3. In an embodiment, the sum of a51 and a52 may be 1 or 2.

In Formula 1, a53 indicates the number of groups represented by

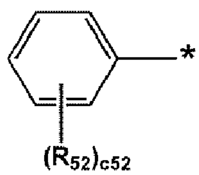,

and may be an integer from 1 to 10. Since a53 in Formula 1 is not 0, ring $CY_{51}$ in Formula 1 is essentially substituted with at least one group represented by

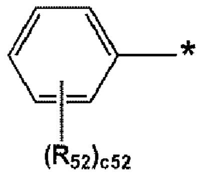

.

In an embodiment, in Formula 1,

$L_1$ to $L_3$ may be a single bond, and
$R_1$ to $R_3$ may each independently be:

hydrogen, deuterium, -F, a cyano group, $C_1$-$C_{20}$ alkyl group, or a $C_1$-$C_{20}$ alkoxy group;
a $C_1$-$C_{20}$ alkyl group or a $C_1$-$C_{20}$ alkoxy group, each substituted with deuterium, -F, $-CD_3$, $-CD_2H$, $-CDH_2$, $-CF_3$, $-CF_2H$, $-CFH_2$, a cyano group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof; or
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group (for example, $*-C(CD_3)_3$), a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

In an embodiment, in Formula 1, a3, and a4 may not be 0, and a group represented by $*-(L_3)_{b3}-(R_3)_{c3}$, and a group represented by $*-(L_4)_{b4}-(R_4)_{c4}$ may not be hydrogen.
In an embodiment, in Formula 1, a1, a3, and a4 may not be 0, and a group represented by $*-(L_1)_{b1}-(R_1)_{c1}$, a group represented by $*-(L_3)_{b3}-(R_3)_{c3}$, and a group represented by $*-(L_4)_{b4}-(R_4)_{c4}$ may not be hydrogen.
In an embodiment, in Formula 1, a1 may not be 0, a group represented by $*-(L_1)_{b1}-(R_1)_{c1}$ may not be hydrogen, and at least one group represented by $*-(L_1)_{b1}-(R_1)_{c1}$ in a number of a1 may include at least one deuterium.
In an embodiment, in Formula 1, a1 may be 2, two groups represented by $*-(L_1)_{b1}-(R_1)_{c1}$ may not be hydrogen, and two groups represented by $*-(L_1)_{b1}-(R_1)_{c1}$ may be identical to each other.
In an embodiment, in Formula 1, a1 may be 2, two groups represented by $*-(L_1)_{b1}-(R_1)_{c1}$ may not be hydrogen, and two groups represented by $*-(L_1)_{b1}-(R_1)_{c1}$ may be different from each other.
In an embodiment, in Formula 1, a4 may not be 0, a group represented by $*-(L_4)_{b4}-(R_4)_{c4}$ may not be hydrogen, and at least one group represented by $*-(L_4)_{b4}-(R_4)_{c4}$ in a number of a4 may include at least one deuterium.
In an embodiment, in Formula 1, a3 may not be 0, and at least one group represented by $*-(L_3)_{b3}-(R_3)_{c3}$ in a number of a3 may satisfy <Condition A> and <Condition B>:

<Condition A>

$L_3$ is a single bond.

<Condition B>

$R_3$ is
hydrogen, deuterium, -F, a cyano group, $C_1$-$C_{20}$ alkyl group, or a C1-C20 alkoxy group;
a $C_1$-$C_{20}$ alkyl group or a $C_1$-$C_{20}$ alkoxy group, each substituted with deuterium, -F, $-CD_3$, $-CD_2H$, $-CDH_2$, $-CF_3$, $-CF_2H$, $-CFH_2$, a cyano group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof; or
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

In an embodiment, in <Condition B>, $R_3$ may not hydrogen.

In an embodiment, in Formula 1, a3 may not be 0, and at least one group represented by $*-(L_3)_{b3}-(R_3)_{c3}$ in a number of a3 may satisfy <Condition A> and <Condition B(1)>:

<Condition A>

$L_3$ is a single bond.

<Condition B(1)>

$R_3$ is
a $C_4$-$C_{20}$ alkyl group unsubstituted or substituted with deuterium, -F,$-CD_3$, $-CD_2H$, $-CDH_2$, $-CF_3$, $-CF_2H$, $-CFH_2$, a cyano group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-C20 alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof; or
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cy-

clooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

In an embodiment, in Formula 1, a4 may not be 0, and at least one group represented by $*-(L_4)_{b4}-(R_4)_{c4}$ in a number of a4 may satisfy <Condition 1>, <Condition 2>, or combination thereof:

<Condition 1>

At least one $R_4$ in a number of c4 is a substituted or unsubstituted $C_6$-$C_{60}$ aryl group, a substituted or unsubstituted $C_1$-$C_{60}$ heteroaryl group, a substituted or unsubstituted monovalent non-aromatic condensed polycyclic group, or a substituted or unsubstituted monovalent non-aromatic condensed heteropolycyclic group.

<Condition 2>

$L_4$ is not a single bond.

In an embodiment, in Formula 1, a4 may not be 0, and at least one group represented by $*-(L_4)_{b4}-(R_4)_{c4}$ in a number of a4 may satisfy <Condition 1(1)>, <Condition 2(1)>, or combination thereof:

<Condition 1(1)>

At least one $R_4$ in a number of c4 is a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F, $-CD_3$,$-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

<Condition 2(1)>

$L_4$ is a benzene group unsubstituted or substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-C20 alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

In an embodiment, in Formula 1, a4 may not be 0, and at least one group represented by $*-(L_4)_{b4}-(R_4)_{c4}$ in a number of a4 may satisfy <Condition 3>, <Condition 4>, <Condition 5>, or combination thereof:

<Condition 3>

At least one $R_4$ in a number of c4 is a substituted $C_6$-$C_{60}$ aryl group.

<Condition 4>

$L_4$ is a $C_5$-$C_{30}$ carbocyclic group substituted with at least one $R_{10a}$,

<Condition 5>

$L_4$ is a $C_5$-$C_{30}$ carbocyclic group that is unsubstituted or substituted with at least one $R_{10a}$ and $R_4$ is not hydrogen.
In an embodiment, in Formula 1, a4 may not be 0, and at least one group represented by *-$(L_4)_{b4}$-$(R_4)_{c4}$ in a number of a4 may satisfy <Condition 3(1)>, <Condition 4(1)>, <Condition 5(1)>, or combination thereof:

<Condition 3(1)>

At least one $R_4$ in a number of c4 is a phenyl group, a ($C_1$-$C_{20}$ alkyl)phenyl group, a biphenyl group, or a terphenyl group, each substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-C20 alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

<Condition 4(1)>

$L_4$ is a benzene group substituted with deuterium, -F, $-CD_3$, $-CD_2H$,$-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

<Condition 5(1)>

$L_4$ is a benzene group unsubstituted or substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-C20 alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof, and

$R_4$ is

deuterium, -F, a cyano group, $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, -F, $-CD_3$, $-CD_2H$, $-CDH_2$, $-CF_3$, $-CF_2H$, $-CFH_2$, a cyano group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$

alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof; or

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F, $-CD_3$, $-CD_2H$, $-CDH_2$, $-CF_3$, $-CF_2H$, $-CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

In one or more embodiments, in Formula 1, a51 and a52 may each independently be 0, 1 or 2, the sum of a51 and a52 may be 1 or 2, and a53 may be 1 or 2.

**[0096]** * and *' each indicate a binding site to a neighboring atom.

For example, a group represented by

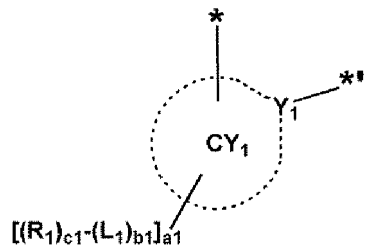

in Formula 1 may be a group represented by one of Formulae A1(1) to A1(30):

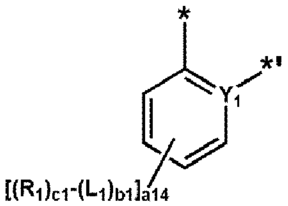

A1(1)

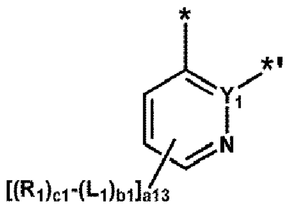

A1(2)

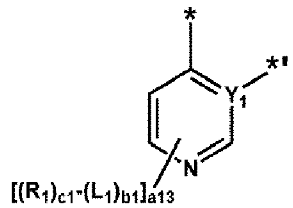

A1(3)

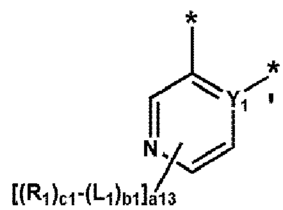

A1(4)

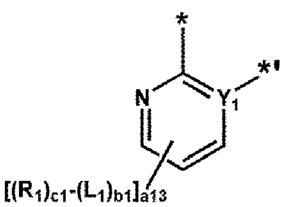

A1(5)

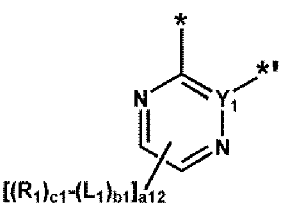

A1(6)

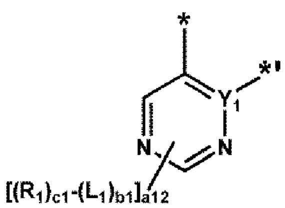

A1(7)

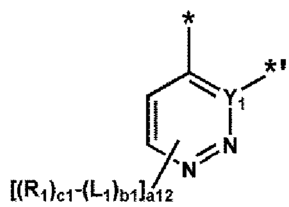

A1(8)

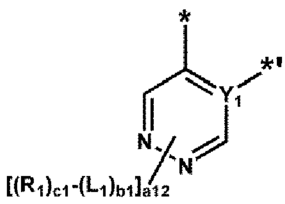
A1(9)

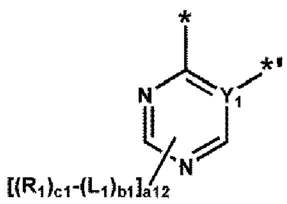
A1(10)

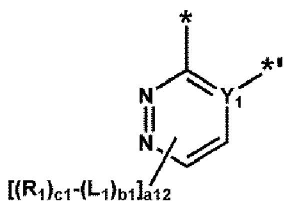
A1(11)

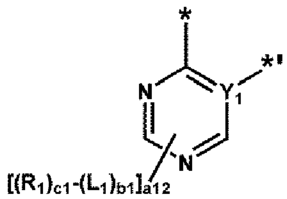
A1(12)

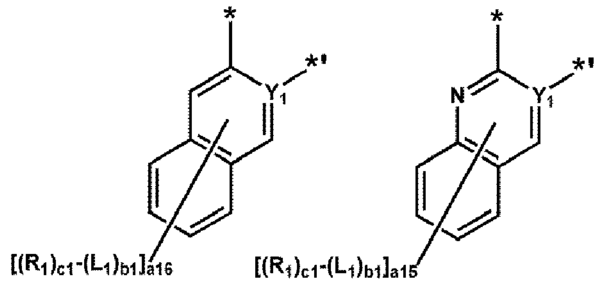
A1(13)

A1(14)

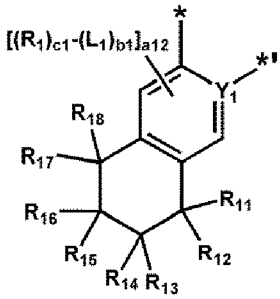
A1(15)

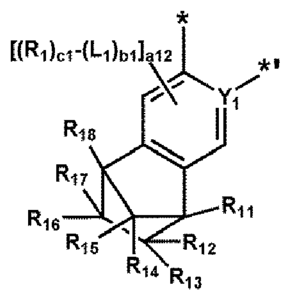
A1(16)

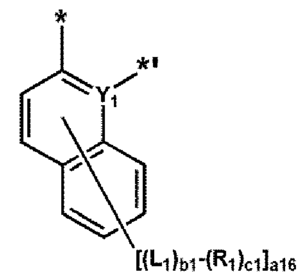
A1(17)

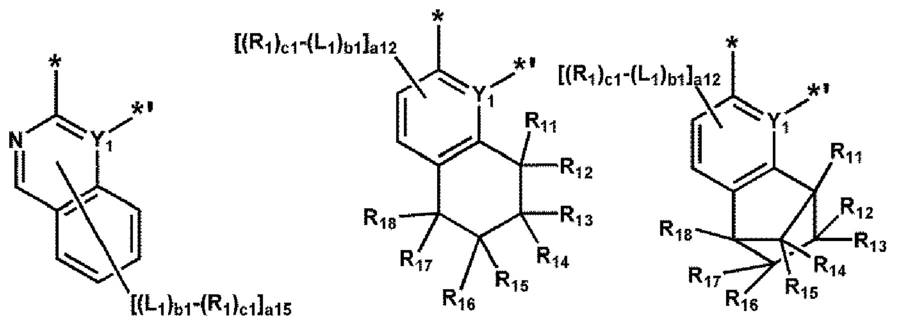
A1(18)

A1(19)

A1(20)

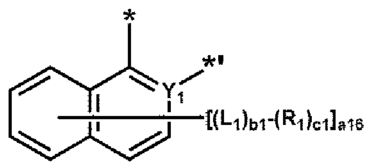
A1(21)

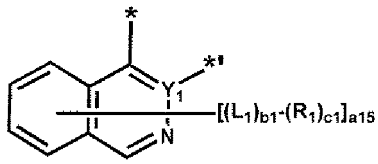
A1(22)

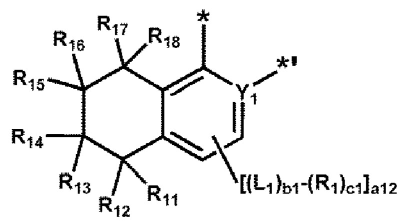
A1(23)

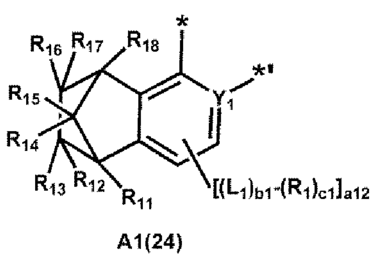
A1(24)

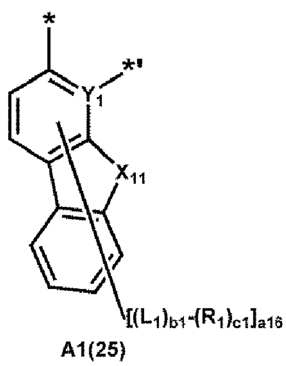
A1(25)

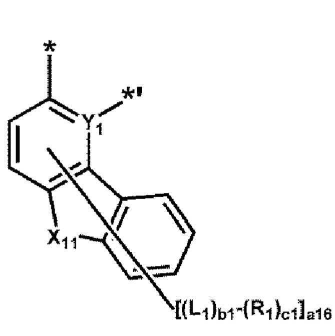
A1(26)

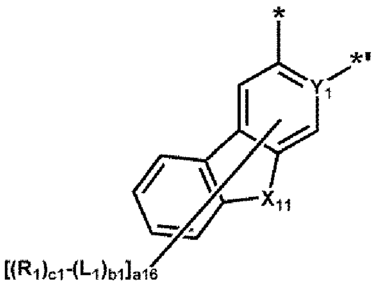

A1(27)

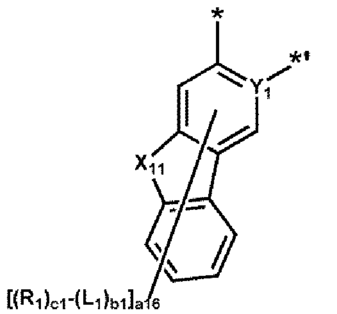

A1(28)

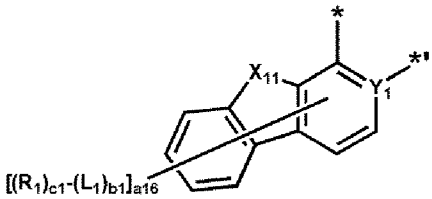

A1(29)

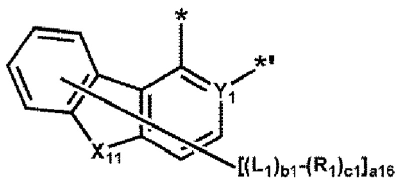

A1(30)

In Formulae A1(1) to A1(30),

$Y_1$, $L_1$, b1, $R_1$, and c1 may each independently be the same as described herein,
$X_{11}$ may be O, S, $N(R_{11})$, $C(R_{11})(R_{12})$, or $Si(R_{11})(R_{12})$,
$R_{11}$ to $R_{18}$ may each independently be the same as described in connection with $R_1$,
a12 may be an integer from 0 to 2,
a13 may be an integer from 0 to 3,
a14 may be an integer from 0 to 4,
a15 may be an integer from 0 to 5,
a16 may be an integer from 0 to 6,
*' indicates a binding site to $X_1$ in Formula 1, and
* indicates a binding site to $Y_3$ in Formula 1.

The group represented by

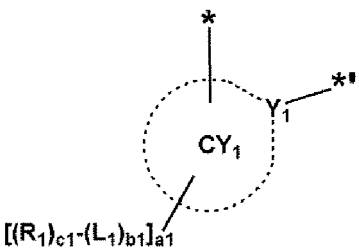

in Formula 1 may include at least one selected from deuterium and a deuterium-containing $C_1$-$C_{60}$ alkyl group unsubstituted or substituted with at least one $C_3$-$C_{10}$ cycloalkyl group. The number of deuterium atoms in the group represented by

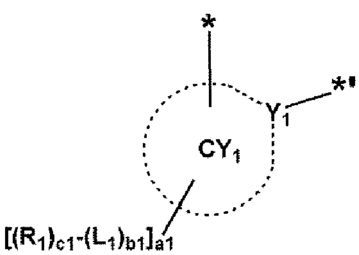

in Formula 1 may be from 1 to 20, for example, from 1 to 15, from 1 to 10, or from 1 to 5.

In an embodiment, a group represented by

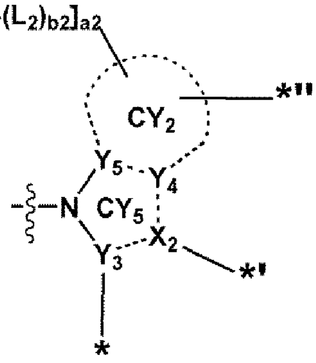

in Formula 1 may be a group represented by Formulae one of A2(1) to A2(4):

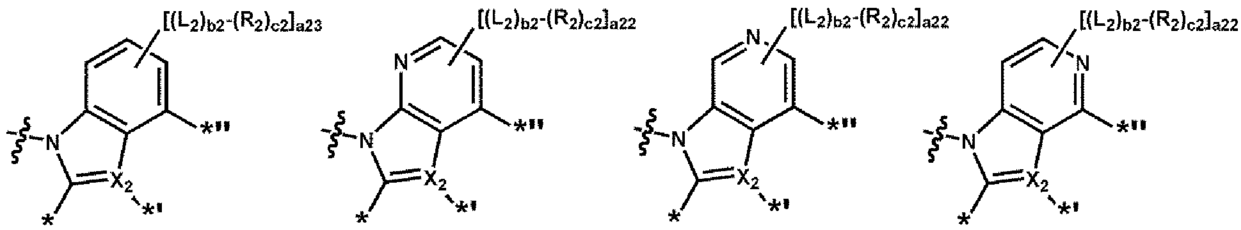

A2(1) A2(2) A2(3) A2(4)

.

In Formulae A2(1) to A2(4),

$X_2$, $L_2$, b2, $R_2$, and c2 may each independently be the same as described herein,
a22 may be an integer from 0 to 2,
a23 may be an integer from 0 to 3,

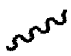 indicates a binding site to $L_{51}$ in Formula 1,
*" indicates a binding site to ring $CY_3$ in Formula 1,
*' indicates a binding site to M in Formula 1, and
* indicates a binding site to ring $CY_1$ in Formula 1.

The group represented by

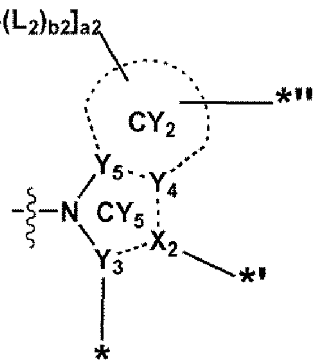

in Formula 1 may include at least one selected from deuterium and a deuterium-containing $C_1$-$C_{60}$ alkyl group unsubstituted or substituted with at least one $C_3$-$C_{10}$ cycloalkyl group. The number of deuterium atoms in the group represented

by

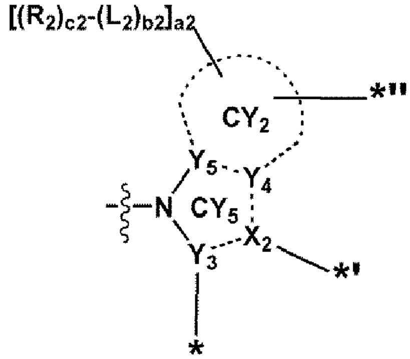

in Formula 1 may be from 1 to 20, for example, from 1 to 15, from 1 to 10, or from 1 to 5.

In one or more embodiments, a group represented by

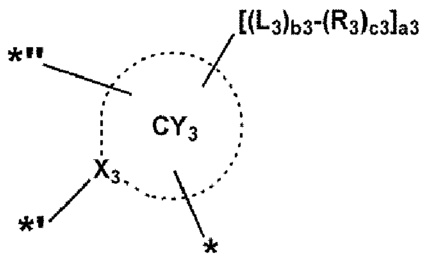

in Formula 1 may be a group represented by Formulae one of A3(1) to A3(17):

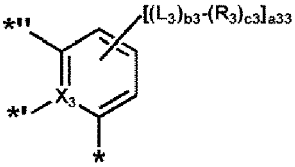

A3(1)

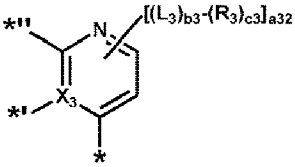

A3(2)

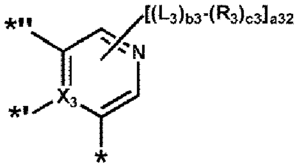

A3(3)

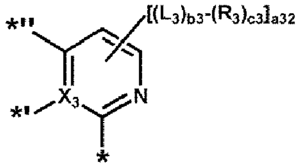

A3(4)

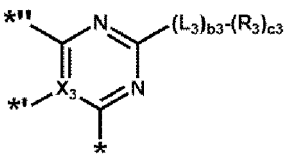

A3(5)

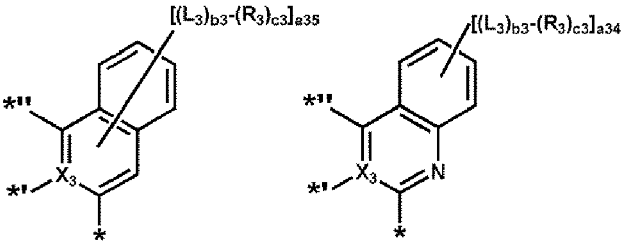

A3(6)

A3(7)

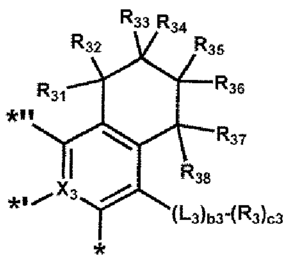

A3(8)

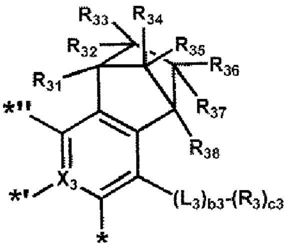
A3(9)

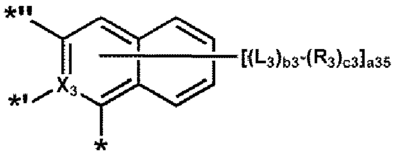
A3(10)

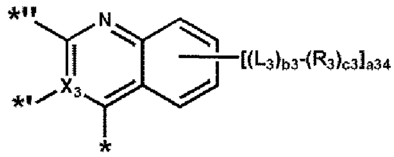
A3(11)

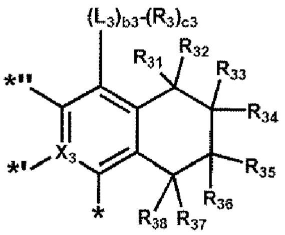
A3(12)

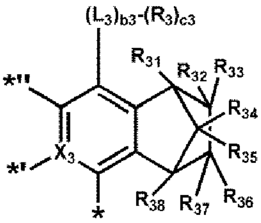
A3(13)

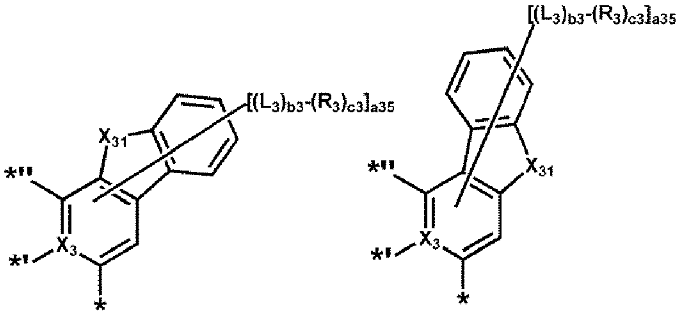
A3(14)

A3(15)

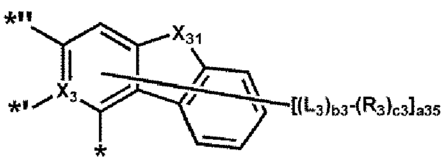
A3(16)

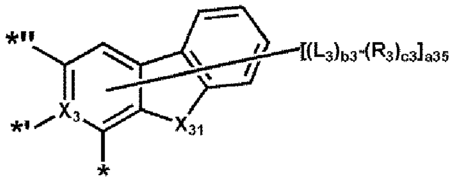
A3(17)

In Formulae A3(1) to A3(17),

$X_3$, $L_3$, b3, $R_3$, and c3 may each independently be the same as described herein,
$X_{31}$ may be O, S, $N(R_{31})$, $C(R_{31})(R_{32})$, or $Si(R_{31})(R_{32})$,
$R_{31}$ to $R_{38}$ may each independently be the same as described in connection with $R_3$,
a32 may be an integer from 0 to 2,
a33 may be an integer from 0 to 3,
a34 may be an integer from 0 to 4,
a35 may be an integer from 0 to 5,
*" indicates a binding site to ring $CY_2$ in Formula 1,
*' indicates a binding site to M in Formula 1, and
* indicates a binding site to $T_1$ in Formula 1.

The group represented by

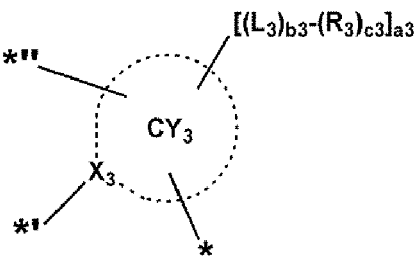

in Formula 1 may include at least one selected from deuterium and a deuterium-containing $C_1$-$C_{60}$ alkyl group unsubstituted or substituted with at least one $C_3$-$C_{10}$ cycloalkyl group. The number of deuterium atoms in the group represented by

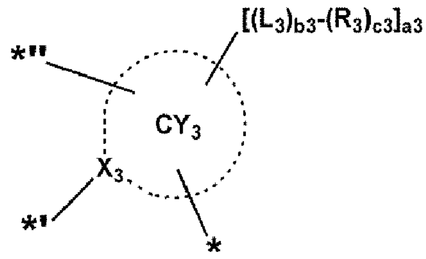

in Formula 1 may be from 1 to 20, for example, from 1 to 15, from 1 to 10, or from 1 to 5.

In one or more embodiments, a group represented by

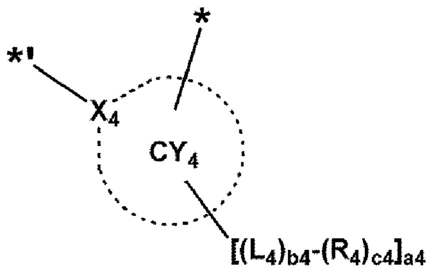

in Formula 1 may be a group represented by Formulae one of A4(1) to A4(45):

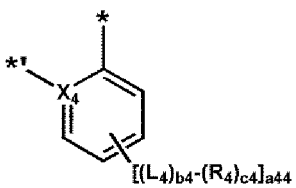
A4(1)

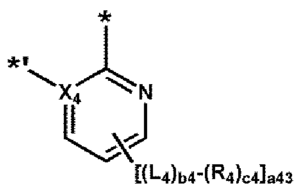
A4(2)

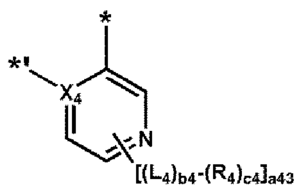
A4(3)

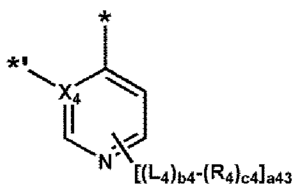
A4(4)

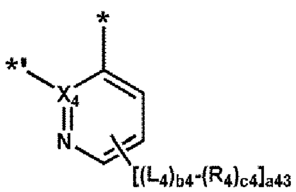
A4(5)

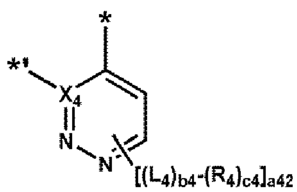
A4(6)

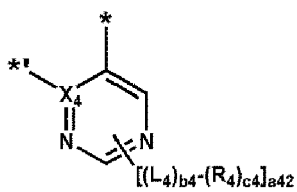
A4(7)

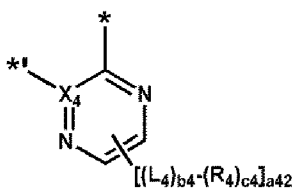
A4(8)

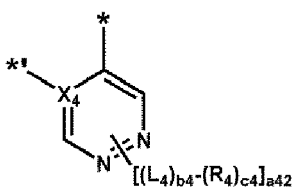
A4(9)

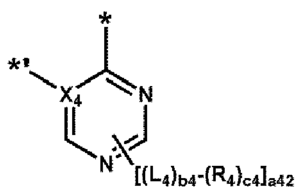
A4(10)

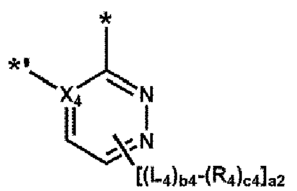
A4(11)

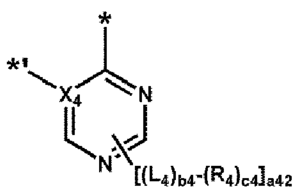
A4(12)

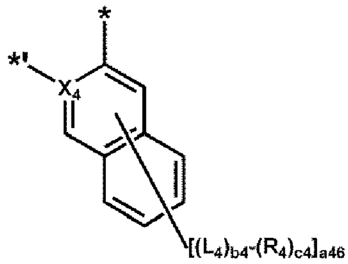

A4(13)

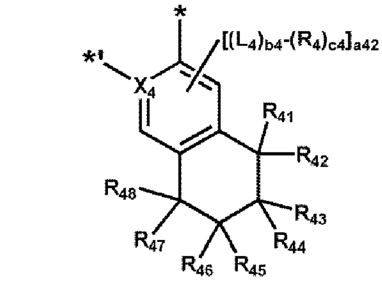

A4(14)

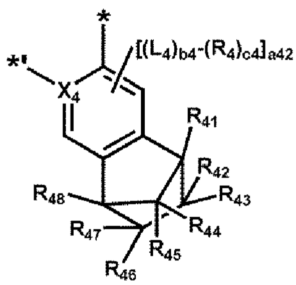

A4(15)

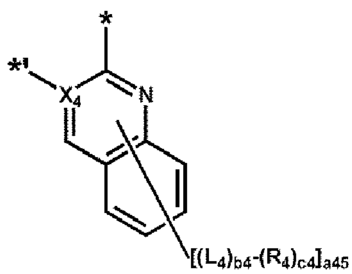

A4(16)

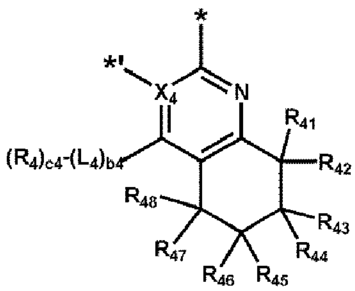

A4(17)

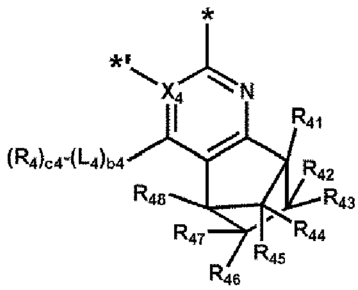

A4(18)

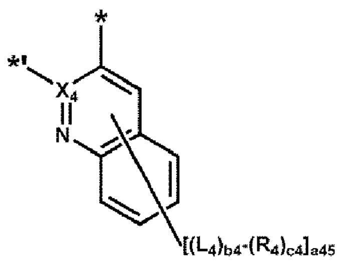

A4(19)

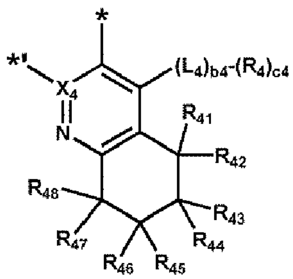

A4(20)

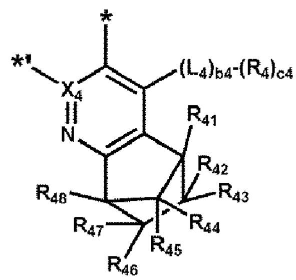

A4(21)

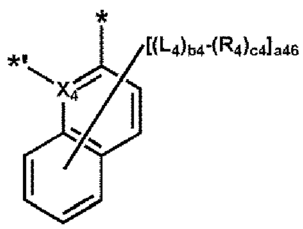

A4(22)

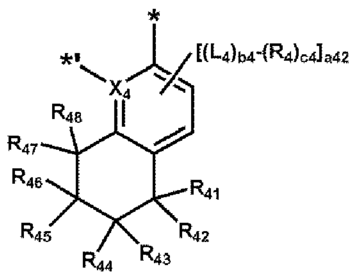

A4(23)

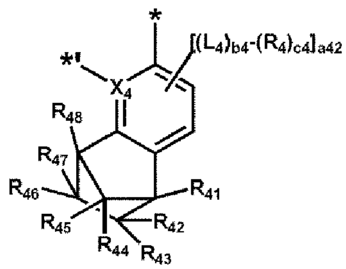

A4(24)

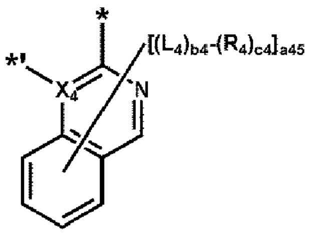

A4(25)

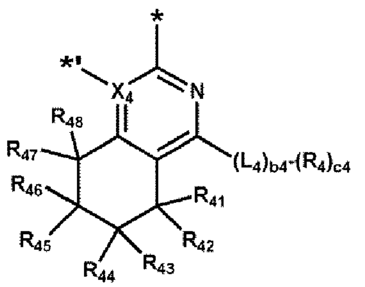

A4(26)

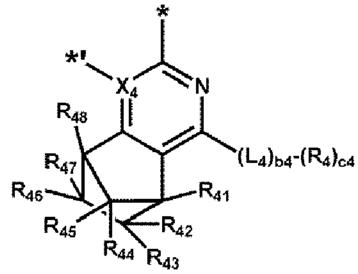

A4(27)

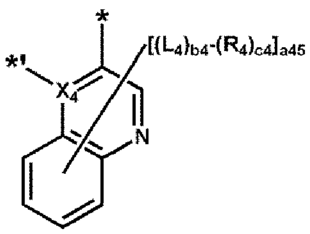

A4(28)

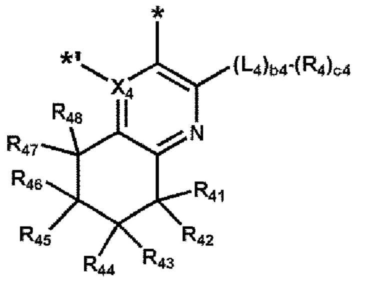

A4(29)

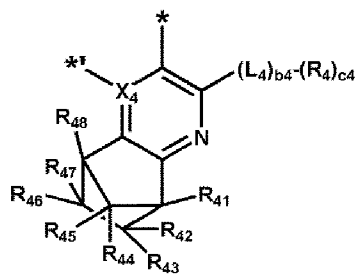

A4(30)

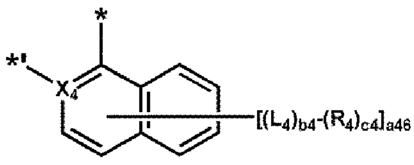

A4(31)

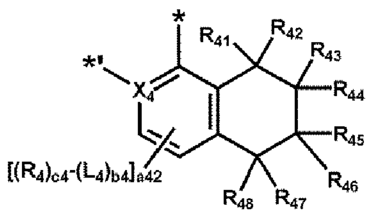

A4(32)

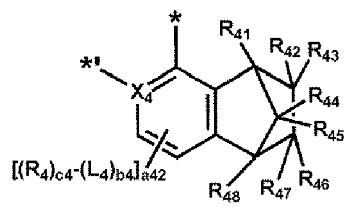

A4(33)

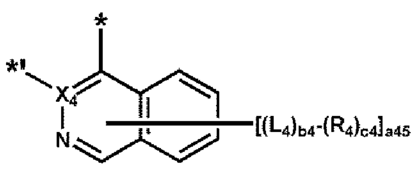

A4(34)

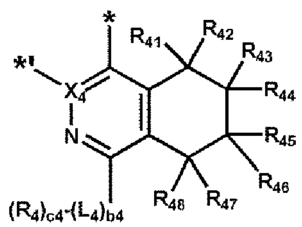

A4(35)

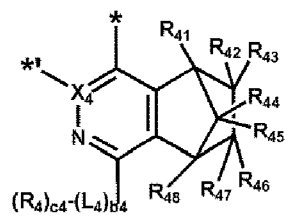

A4(36)

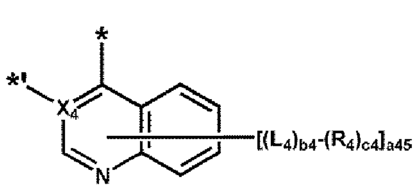

A4(37)

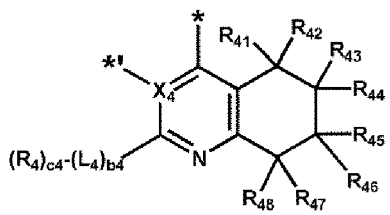

A4(38)

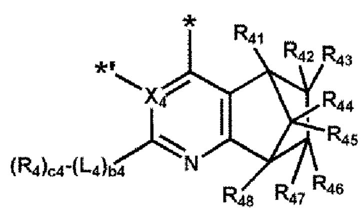

A4(39)

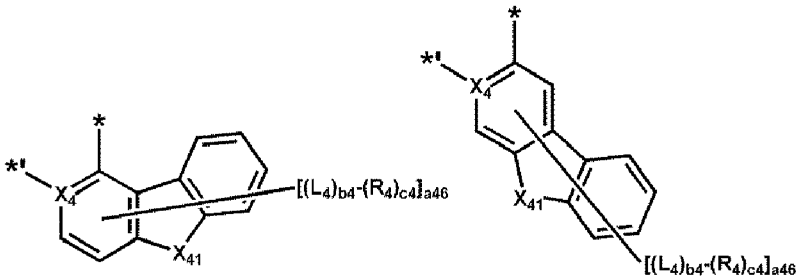

A4(40) A4(41)

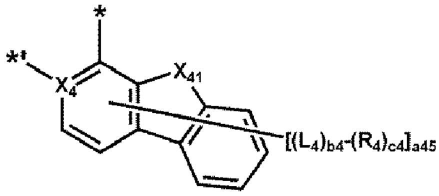

A4(42)

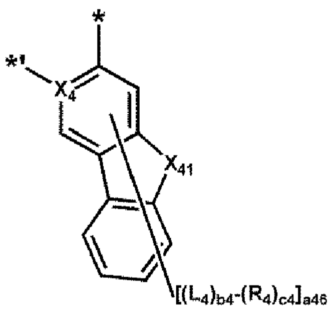

A4(43)

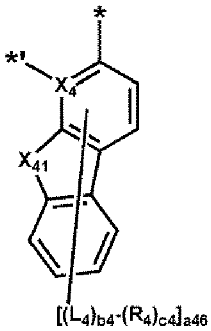

A4(44)

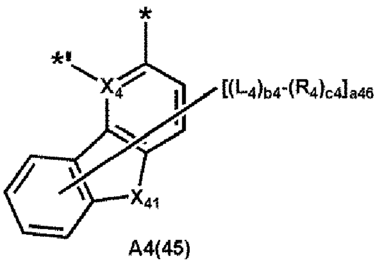

A4(45)

.

In Formulae A4(1) to A4(45),

$X_4$, $L_4$, b4, $R_4$, and c4 may each independently be the same as described herein,
$X_{41}$ may be O, S, $N(R_{41})$, $C(R_{41})(R_{42})$, or $Si(R_{41})(R_{42})$,
$R_{41}$ to $R_{48}$ may each independently be the same as described in connection with $R_4$,
a42 may be an integer from 0 to 2,
a43 may be an integer from 0 to 3,
a44 may be an integer from 0 to 4,
a45 may be an integer from 0 to 5,
a46 may be an integer from 0 to 6,
*' indicates a binding site to M in Formula 1, and
* indicates a binding site to $T_1$ in Formula 1.

The group represented by

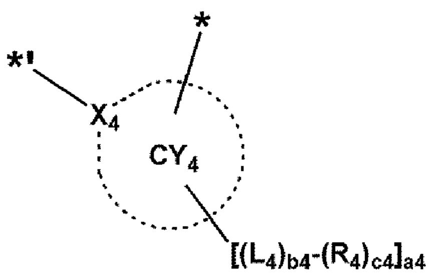

in Formula 1 may include at least one selected from deuterium and a deuterium-containing $C_1$-$C_{60}$ alkyl group unsubstituted or substituted with at least one $C_3$-$C_{10}$ cycloalkyl group. The number of deuterium atoms in the group represented by

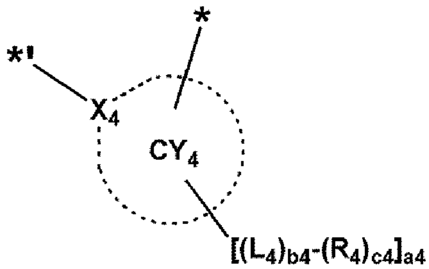

in Formula 1 may be from 1 to 20, for example, from 1 to 15, from 1 to 10, or from 1 to 5.

In one or more embodiments, in Formula 1, a group represented by

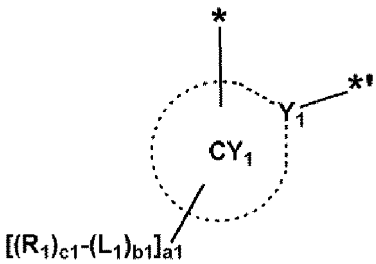

may be a group represented by Formulae one of CY1(1) to CY1(8), and/or

a group represented by

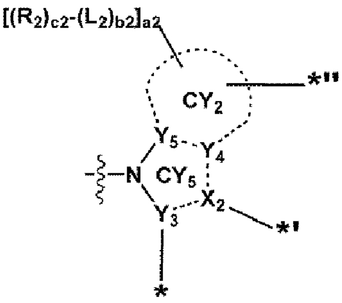

may be a group represented by Formulae one of CY2(1) to CY2(4), and/or

a group represented by

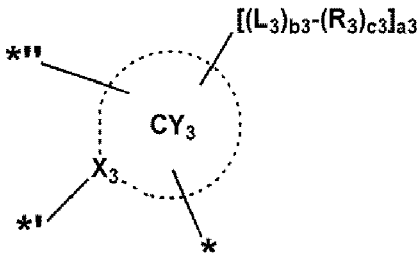

may be a group represented by Formulae one of CY3(1) to CY3(24), and/or

a group represented by

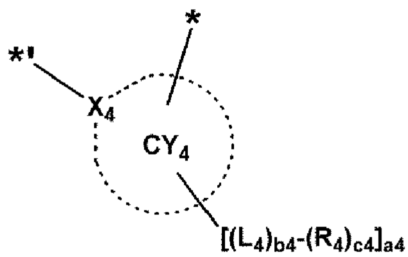

may be a group represented by Formulae one of CY4(1) to CY4(74), but embodiments of the present disclosure are not limited thereto:

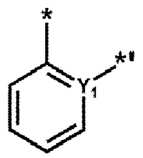

CY1(1)

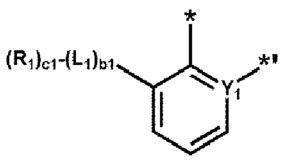

CY1(2)

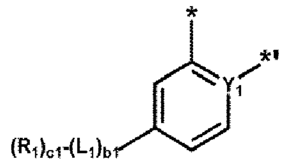

CY1(3)

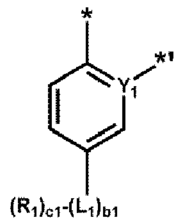

CY1(4)

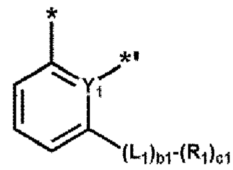

CY1(5)

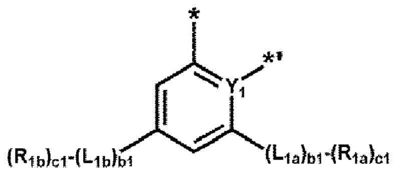

CY1(6)

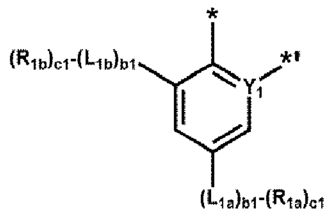

CY1(7)

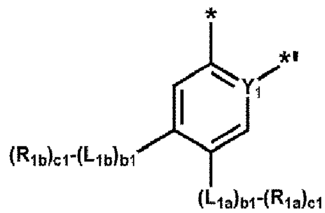

CY1(8)

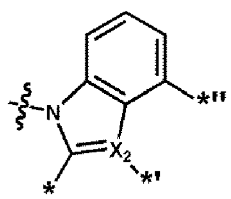

CY2(1)

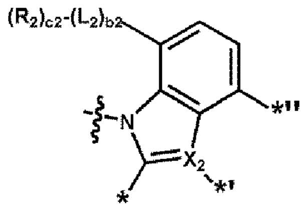

CY2(2)

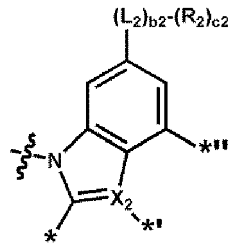

CY2(3)

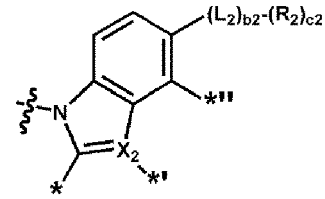

CY2(4)

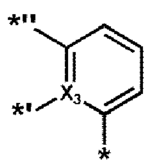

CY3(1)

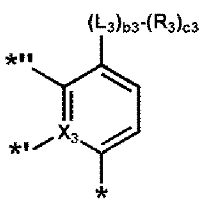

CY3(2)

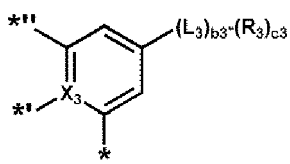

CY3(3)

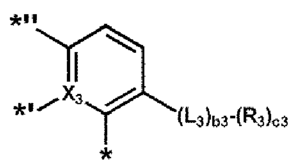

CY3(4)

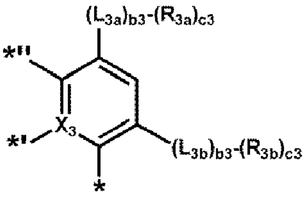

CY3(5)

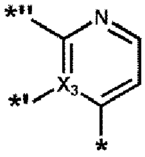

CY3(6)

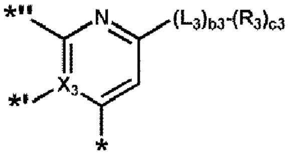

CY3(7)

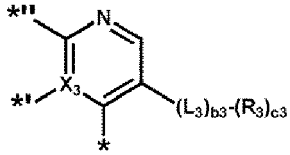

CY3(8)

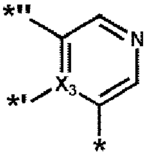

CY3(9)

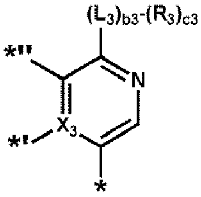

CY3(10)

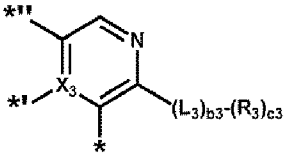

CY3(11)

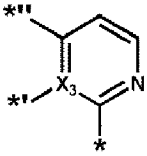

CY3(12)

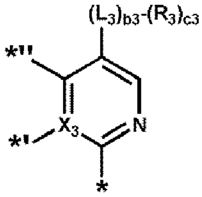

CY3(13)

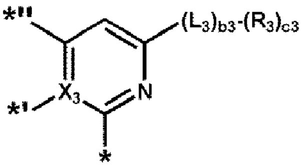

CY3(14)

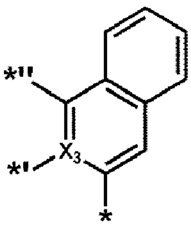

CY3(15)

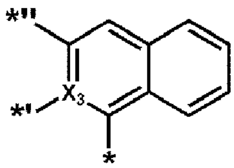

CY3(16)

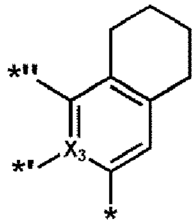

CY3(17)

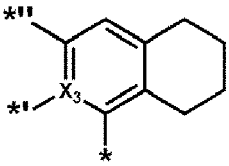

CY3(18)

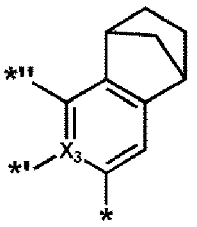

CY3(19)

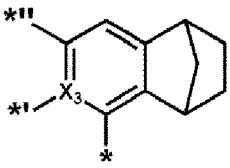

CY3(20)

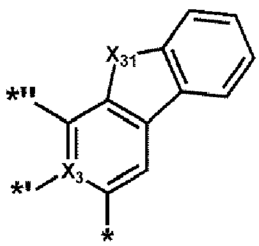

CY3(21)

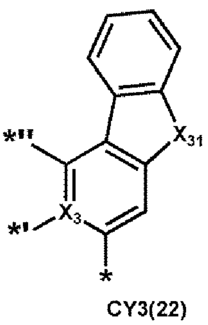

CY3(22)

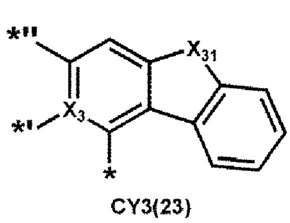

CY3(23)

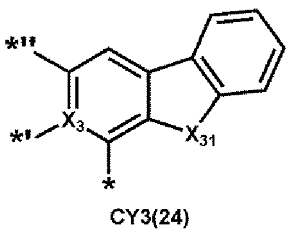

CY3(24)

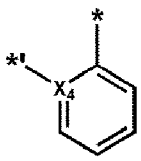
CY4(1)

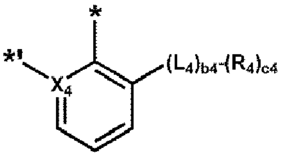
CY4(2)

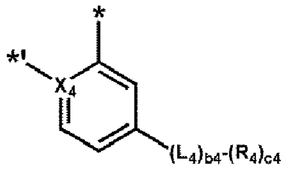
CY4(3)

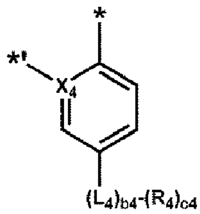
CY4(4)

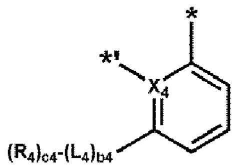
CY4(5)

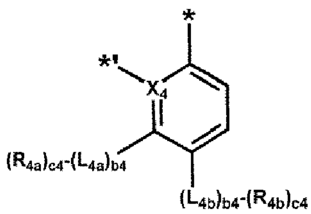
CY4(6)

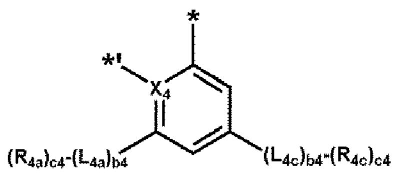
CY4(7)

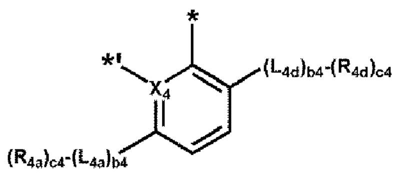
CY4(8)

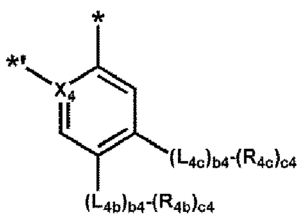
CY4(9)

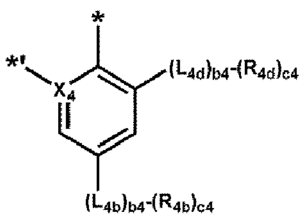
CY4(10)

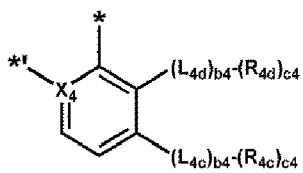
CY4(11)

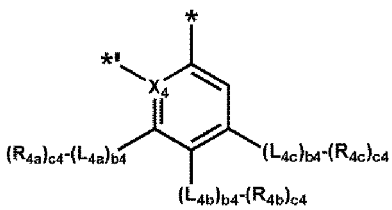
CY4(12)

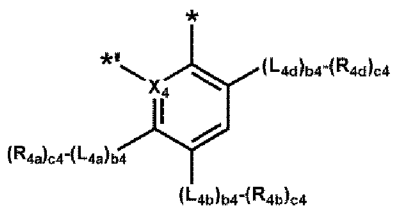
CY4(13)

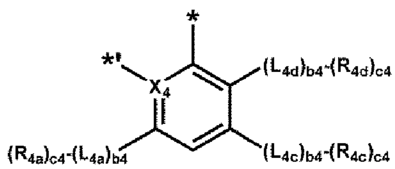
CY4(14)

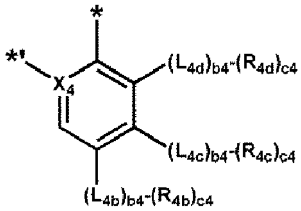
CY4(15)

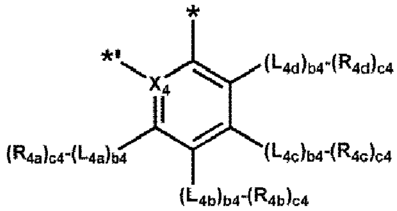
CY4(16)

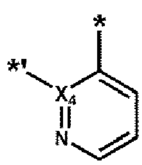
CY4(17)

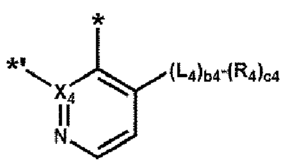
CY4(18)

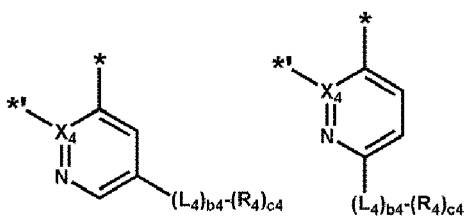
CY4(19) CY4(20)

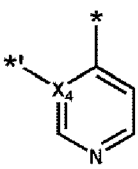
CY4(21)

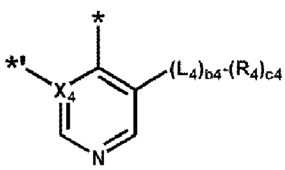
CY4(22)

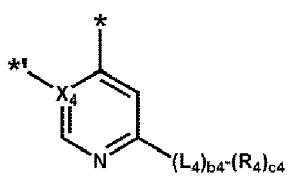
CY4(23)

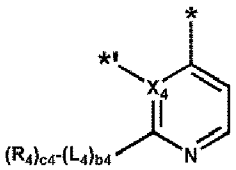
CY4(24)

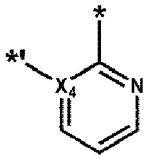
CY4(25)

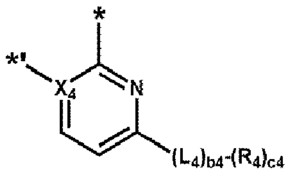
CY4(26)

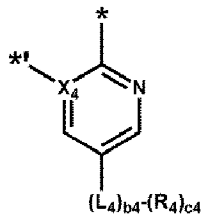
CY4(27)

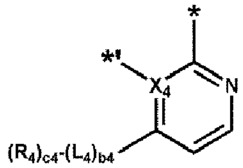
CY4(28)

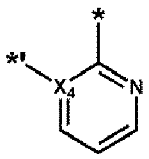
CY4(29)

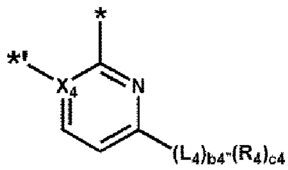
CY4(30)

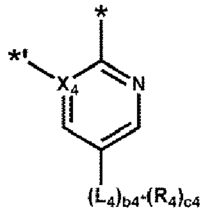
CY4(31)

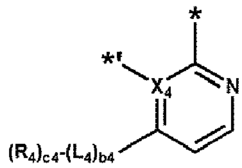
CY4(32)

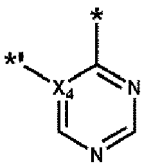
CY4(33)

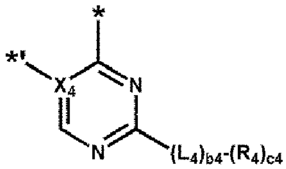
CY4(34)

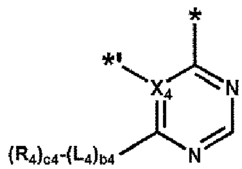
CY4(35)

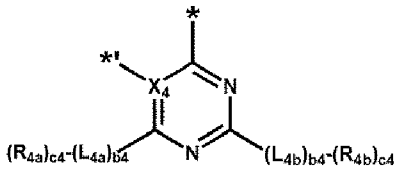
CY4(36)

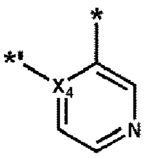
CY4(37)

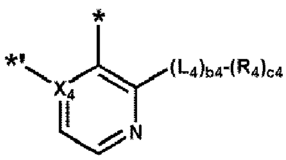
CY4(38)

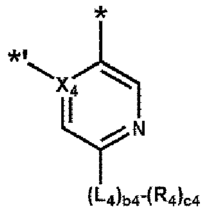
CY4(39)

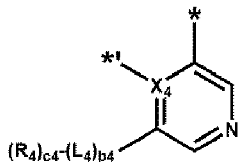
CY4(40)

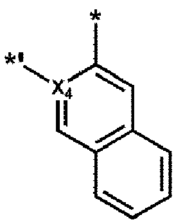
CY4(41)

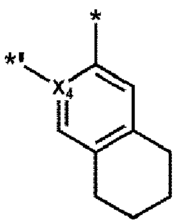
CY4(42)

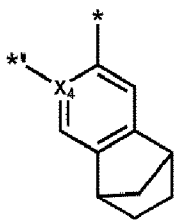
CY4(43)

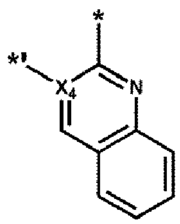
CY4(44)

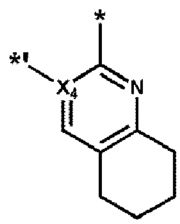
CY4(45)

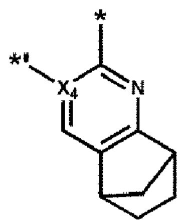
CY4(46)

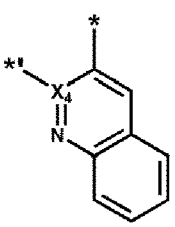
CY4(47)

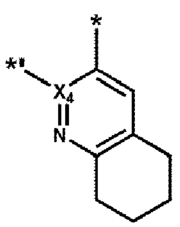
CY4(48)

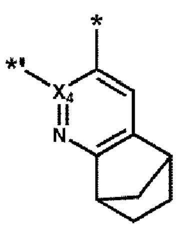
CY4(49)

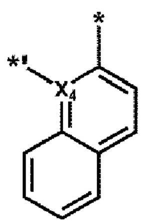
CY4(50)

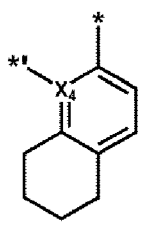
CY4(51)

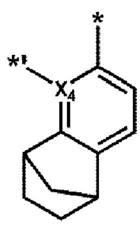
CY4(52)

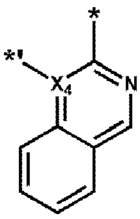
CY4(53)

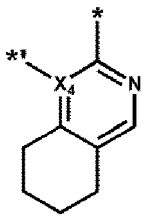
CY4(54)

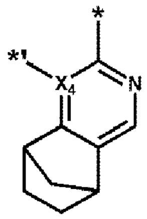
CY4(55)

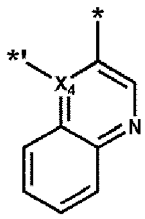
CY4(56)

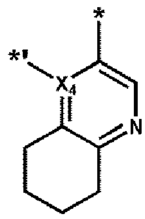
CY4(57)

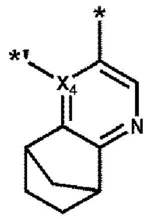
CY4(58)

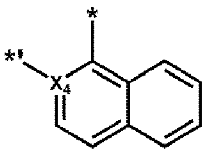
CY4(59)

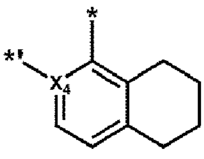
CY4(60)

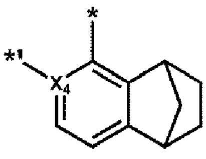
CY4(61)

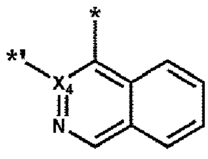
CY4(62)

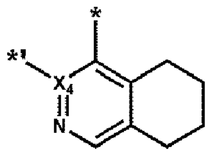
CY4(63)

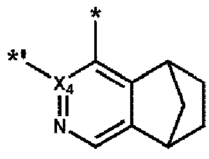
CY4(64)

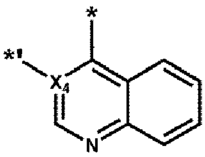
CY4(65)

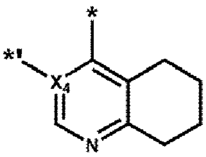
CY4(66)

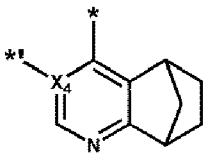
CY4(67)

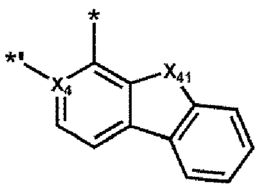
CY4(68)

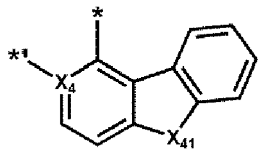
CY4(69)

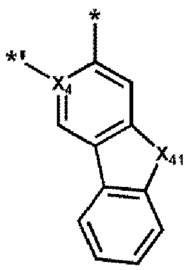
CY4(70)

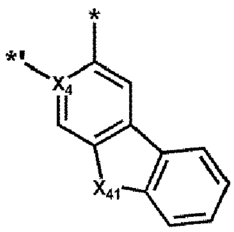
CY4(71)

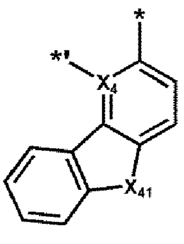
CY4(72)

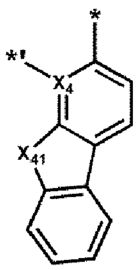
CY4(73)

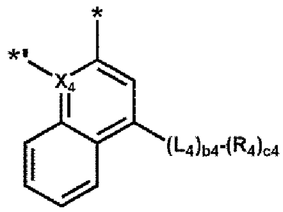
CY4(74)

In Formulae CY1(1) to CY1(8), CY2(1) to CY2(4), CY3(1) to CY3(24), and CY4(1) to CY4(74),

$X_2$ to $X_4$, $Y_1$, $L_1$ to $L_4$, b1 to b4, $R_1$ to $R_4$, and c1 to c4 may each independently be the same as described herein,
$X_{31}$ may be O, S, $N(R_{31})$, $C(R_{31})(R_{32})$, or $Si(R_{31})(R_{32})$,
$X_{41}$ may be O, S, $N(R_{41})$, $C(R_{41})(R_{42})$, or $Si(R_{41})(R_{42})$,
$L_{1a}$ and $L_{1b}$ may each independently be the same as described in connection with $L_1$,
$R_{1a}$ and $R_{1b}$ may each independently be the same as described in connection with $R_1$,
$L_{3a}$ and $L_{3b}$ may each independently be the same as described in connection with $L_3$,
$R_{3a}$, $R_{3b}$, $R_{31}$, and $R_{32}$ may each independently be the same as described in connection with $R_3$,
$L_{4a}$ to $L_{4d}$ may each independently be the same as described in connection with $L_4$,
$R_{4a}$ to $R_{4d}$, $R_{41}$, and $R_{42}$ may each independently be the same as described in connection with $R_4$,
$*-(L_1)_{b1}-(R_1)_{c1}$, $*-(L_{1a})_{b1}-(R_{1a})_{c1}$, $*-(L_{1b})_{b1}-(R_{1b})_{c1}$, $*-(L_2)_{b2}-(R_2)_{c2}$, $*-(L_3)_{b3}-(R_3)_{c3}$, $*-(L_{3a})_{b3}-(R_{3a})_{c3}$, $*-(L_{3b})_{b3}-(R_{3b})_{c3}$, $*-(L_4)_{b4}-(R_4)_{c4}$, $*-(L_{4a})_{b4}-(R_{4a})_{c4}$, $*-(L_{4b})_{b4}-(R_{4b})_{c4}$, $*-(L_{4c})_{b4}-(R_{4c})_{c4}$, and $*-(L_{4d})_{b4}-(R_{4d})_{c4}$ may not be hydrogen, and
* indicates a binding site to a neighboring atom,
in Formulae CY1(1) to CY1(8), *' indicates a binding site to $X_1$ in Formula 1,
in Formulae CY2(1) to CY2(4), CY3(1) to CY3(24), and CY4(1) to CY4(74), *' indicates a binding site to M in Formula 1,
in Formulae CY1(1) to CY1(8), * indicates a binding site to $Y_3$ in Formula 1,
in Formulae CY2(1) to CY2(4), * indicates a binding site to ring $CY_1$ in Formula 1, and *" indicates a binding site to ring $CY_3$ in Formula 1,
in Formulae CY3(1) to CY3(24), *" indicates a binding site to ring $CY_2$ in Formula 1, and * indicates a binding site

to $T_1$ in Formula 1, and
in Formulae CY4(1) to CY4(74), * indicates a binding site to $T_1$ in Formula 1.

In an embodiment, a group represented by

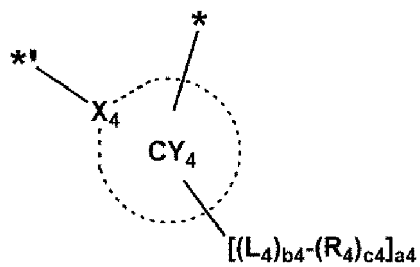

may be a group represented by Formula CY4-1 or CY4-2:

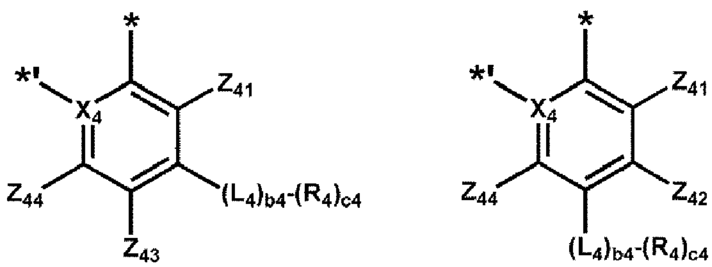

CY4-1 CY4-2 ,

in Formulae CY4-1 and CY4-2,
$X_4$, $L_4$, b4, $R_4$, and c4 may each independently be the same as described herein,
$Z_{41}$ to $Z_{44}$ may each independently be the same as described in connection with $R_4$ herein,
*' indicates a binding site to M in Formula 1, and
* indicates a binding site to $T_1$ in Formula 1.

For example, Formulae CY4-1 and CY4-2 may satisfy:

1) <Condition 1>, <Condition 2>, or any combination thereof (or, <Condition 1(1)>, <Condition 2(1)>, or any combination thereof), and/or
2) <Condition 3>, <Condition 4>, <Condition 5>, or any combination thereof (or, <Condition 3(1)>, <Condition 4(1)>, <Condition 5(1)>, or any combination thereof).

In an embiment, in Formula 1, a group represented by

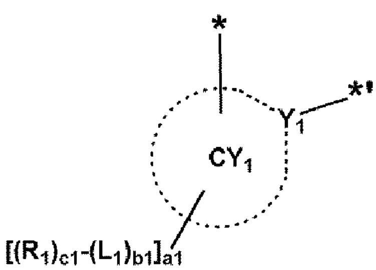

may be a group represented by Formula CY1(1) or CY1(6), and/or

a group represented by

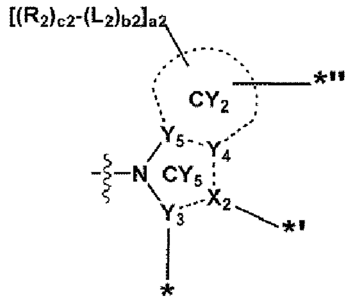

may be a group represented by Formula CY2(1), and/or

a group represented by

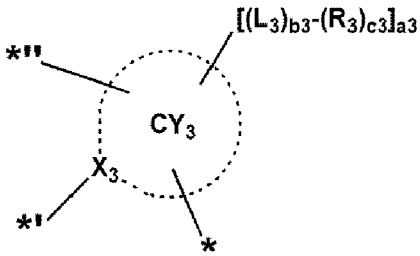

may be a group represented by Formula CY3(3), and/or

a group represented by

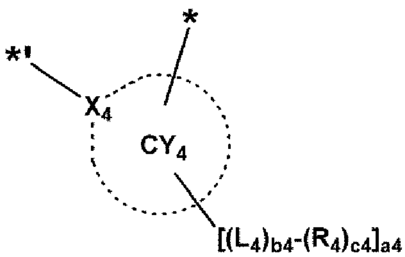

may be a group represented by Formula CY4(3), CY4(4) or CY4(16).

In an embodiment, a group represented by

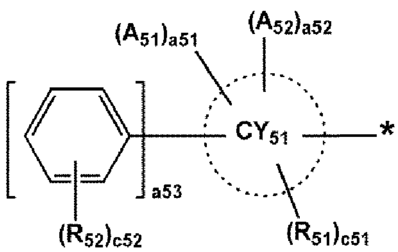

in Formula 1 may be a group represented by one of Formulae 51-1 to 51-32:

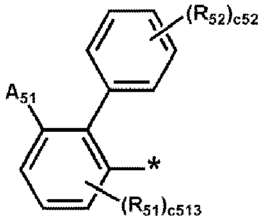
51-1

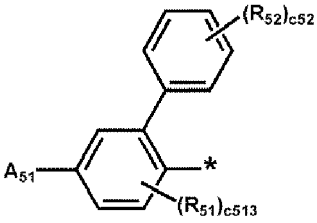
51-2

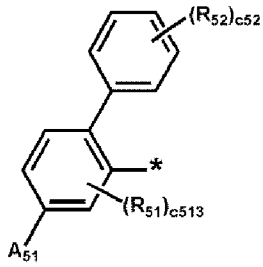
51-3

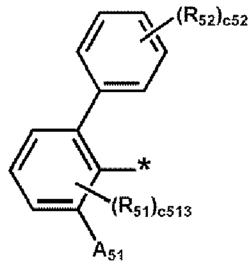
51-4

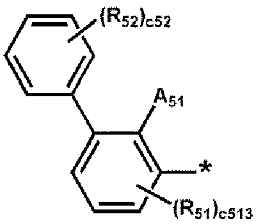
51-5

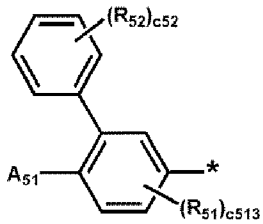
51-6

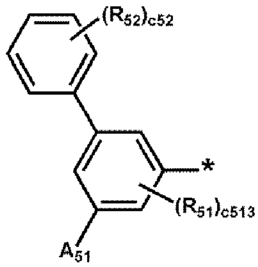
51-7

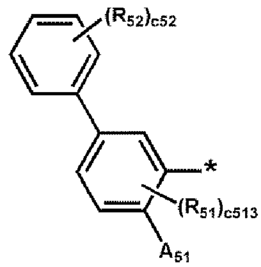
51-8

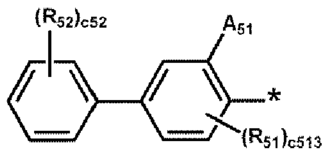
51-9

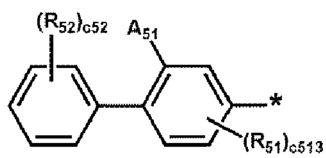
51-10

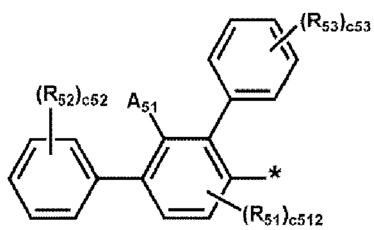
51-11

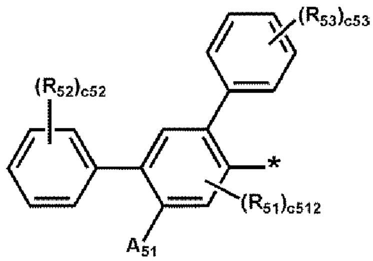
51-12

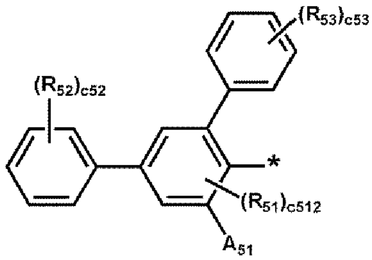
51-13

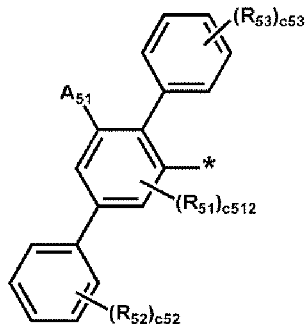
51-14

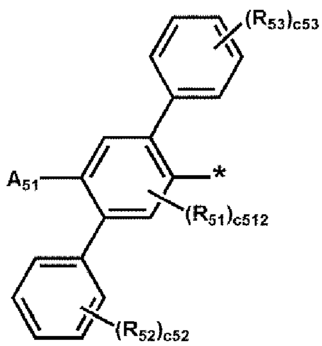
51-15

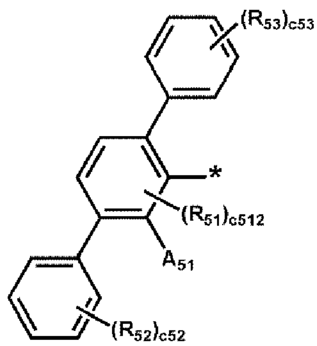
51-16

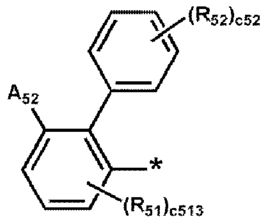
51-17

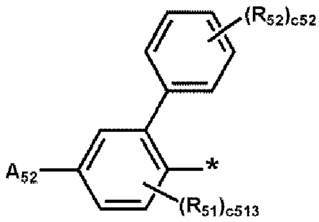
51-18

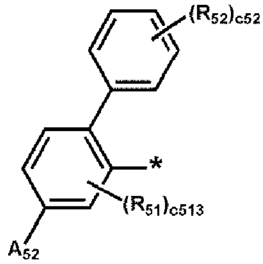
51-19

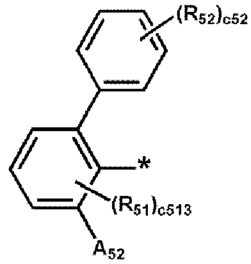
51-20

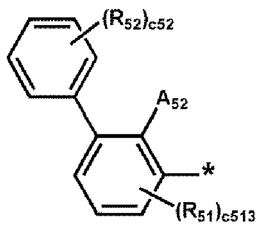
51-21

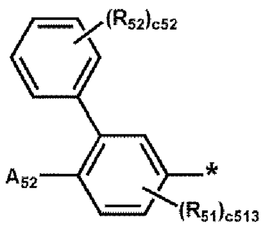
51-22

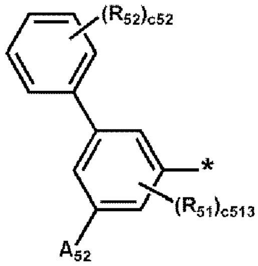
51-23

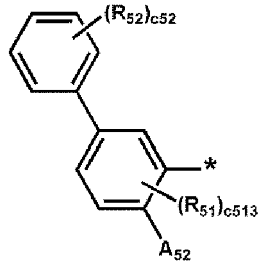
51-24

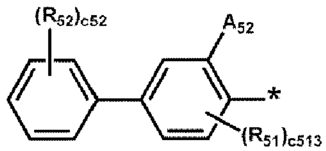
51-25

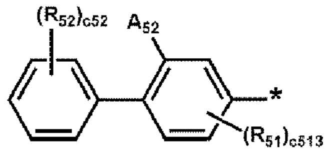
51-26

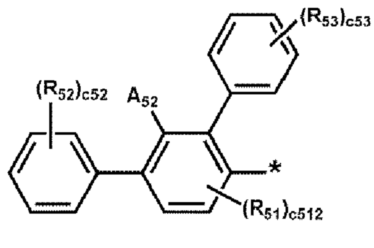
51-27

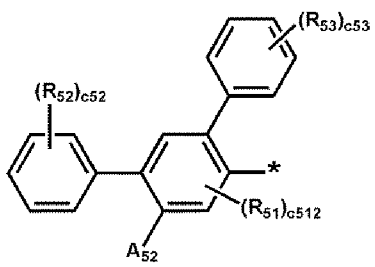
51-28

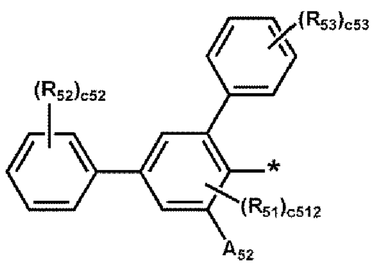
51-29

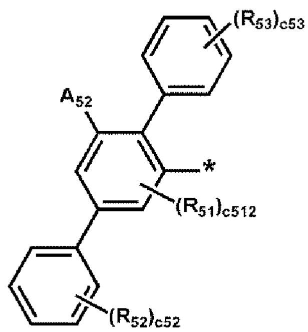
51-30

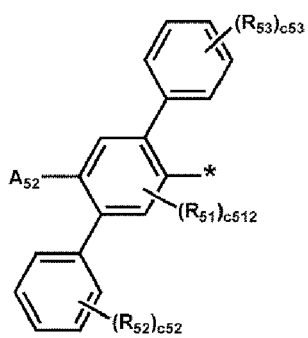
51-31

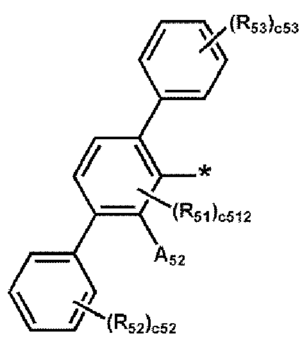
51-32

.

In Formulae 51-1 to 51-32, $R_{51}$, $R_{52}$, c52, $A_{51}$, and $A_{52}$ may each independently be the same as described herein, $R_{53}$ and c53 may each independently be the same as described in connection with $R_{52}$ and c52, c512 is an integer of 0 to 2, c513 is an integer of 0 to 3, and * indicates a binding site to $L_{51}$.

In embodiment, the organometallic compound may be represented by Formula 1-1 or 1-2:

<Formula 1-1>

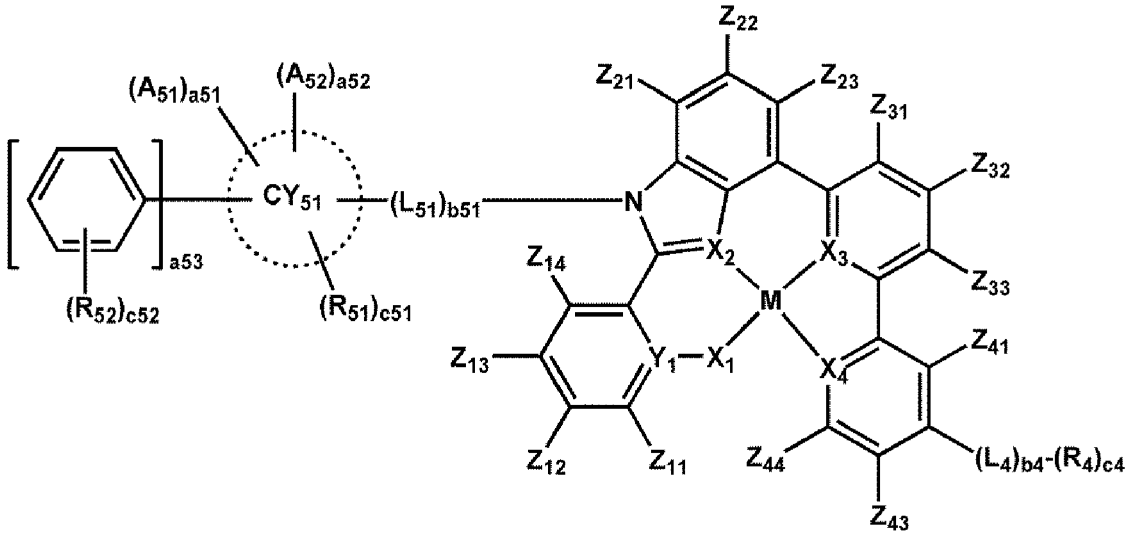

<Formula 1-2>

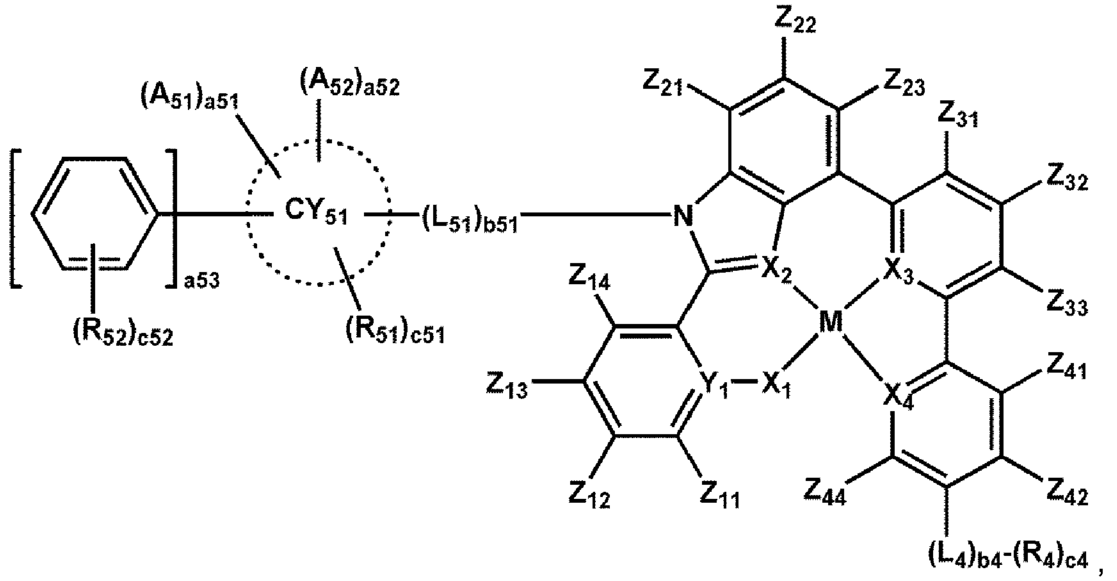

in Formulae 1-1 and 1-2,

M, $X_1$ to $X_4$, $Y_1$, $Y_3$ to $Y_5$, $L_4$, $L_{51}$, b4, b51, $R_{51}$, $R_{52}$, c51, c52, $A_{51}$, $A_{52}$, a51, a52 and a53 may each independently be the same as described herein,
$Z_{11}$ to $Z_{14}$ may each independently be the same as described in connection with $R_1$,
$Z_{21}$ to $Z_{23}$ may each independently be the same as described in connection with $R_2$,
$Z_{31}$ to $Z_{33}$ may each independently be the same as described in connection with $R_3$,
$Z_{41}$ to $Z_{44}$ may each independently be the same as described in connection with $R_4$,
two or more groups selected from $Z_{11}$ to $Z_{14}$ may be optionally linked to form a $C_5$-$C_{30}$carbocyclic group that is unsubstituted or substituted with at least one $R_{10a}$ or a $C_1$-$C_{30}$ heterocyclic group that is unsubstituted or substituted

with at least one $R_{10a}$,
two or more groups selected from $Z_{21}$ to $Z_{23}$ may be optionally linked to form a $C_5$-$C_{30}$carbocyclic group that is unsubstituted or substituted with at least one $R_{10a}$ or a $C_1$-$C_{30}$ heterocyclic group that is unsubstituted or substituted with at least one $R_{10a}$,
two or more groups selected from $Z_{31}$ to $Z_{33}$ may be optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,
two or more groups selected from $Z_{41}$ to $Z_{44}$ may be optionally linked to form a $C_5$-$C_{30}$carbocyclic group that is unsubstituted or substituted with at least one $R_{10a}$ or a $C_1$-$C_{30}$ heterocyclic group that is unsubstituted or substituted with at least one $R_{10a}$, and
$R_{10a}$ is the same as described in connection with $R_1$.

Descriptions for Formula 1 described herein can be applied for Formulae 1-1 and 1-2.
For example,

1) Formulae 1-1 and 1-2 may satisfy <Condition A>, <Condition B>, or any combination thereof (or, <Condition A>, <Condition B(1)>, or any combination thereof), and/or
2) Formulae 1-1 and 1-2 may satisfy <Condition 1>, <Condition 2>, or any combination thereof (or, <Condition 1(1)>, <Condition 2(1)>, or any combination thereof), and/or
3) Formulae 1-1 and 1-2 may satisfy <Condition 3>, <Condition 4>, <Condition 5>, or any combination thereof (or, <Condition 3(1)>, <Condition 4(1)>, <Condition 5(1)>, or any combination thereof), and/or
4) a group represented by

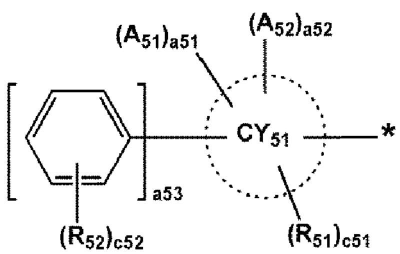

in Formulae 1-1 and 1-2 may be a group represented by one of Formulae 51-1 to 51-32.

In embodiment, $Z_{12}$, $Z_{14}$, $Z_{21}$ to $Z_{23}$, $Z_{31}$, $Z_{33}$ and $Z_{41}$ to $Z_{44}$ in Formulae 1-1 and 1-2 may each independently be hydrogen, deuterium, $-CH_3$, or $-CD_3$.
In embodiment, $Z_{12}$, $Z_{14}$, $Z_{21}$ to $Z_{23}$, $Z_{31}$, $Z_{33}$ and $Z_{41}$ to $Z_{44}$ in Formulae 1-1 and 1-2 may each independently be hydrogen, or deuterium.
In embodiment, $Z_{11}$ and $Z_{13}$ in Formulae 1-1 and 1-2 may not be hydrogen and at least one of $Z_{11}$ and $Z_{13}$ may include at least one deuterium.
In embodiment, $Z_{11}$ and $Z_{13}$ in Formulae 1-1 and 1-2 may not be hydrogen and $Z_{11}$ and $Z_{13}$ may be identical to each other.
In embodiment, $Z_{11}$ and $Z_{13}$ in Formulae 1-1 and 1-2 may not be hydrogen and $Z_{11}$ and $Z_{13}$ may be different from each other.
In Formula 1, i) two or more groups selected from a plurality of groups $R_1$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$, ii) two or more groups selected from a plurality of groups $R_2$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$, iii) two or more groups selected from a plurality of groups $R_3$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$, iv) two or more groups selected from a plurality of groups $R_4$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$, and v) two or more groups selected from $R_1$ to $R_6$ may optionally be linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$. $R_{10a}$ may be the same as described in connection with $R_1$.
For example, a $C_5$-$C_{30}$carbocyclic group (that is unsubstituted or substituted with at least one $R_{10a}$) and a $C_1$-$C_{30}$ heterocyclic group (that is unsubstituted or substituted with at least one $R_{10a}$) may each independently be a

cyclopentane group, a silole group, an azasilole group, a diazasilole group, a triazasilole group, an adamantane group, a norbornane group, a norbornene group, a bicyclo[1.1.1]pentane group, a bicyclo[2.1.1]hexane group, a bicyclo[2.2.1]heptane group, a bicyclo[2.2.2]octane group, a cyclohexane group, a cyclohexene group, a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene-5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one $R_{1a}$, but embodiments of the present disclosure are not limited thereto.

Non-limiting examples of the $C_1$-$C_{60}$ alkyl group, $C_1$-$C_{20}$ alkyl group and/or $C_1$-$C_{10}$ alkyl group include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group, each unsubstituted or substituted with a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or any combination thereof, and the like, but embodiments of the present disclosure are not limited thereto.

Non-limiting examples of the $C_1$-$C_{60}$ alkoxy group, $C_1$-$C_{20}$ alkoxy group and/or $C_1$-$C_{10}$ alkoxy group include a methoxy group, an ethoxy group, a propoxy group or a buoxy group, and the like, but embodiments of the present disclosure are not limited thereto.

Non-limiting examples of the $C_3$-$C_{10}$ cycloalkyl group include a cyclopropyl group, a cyclobutyl group, a cyclopentyl group, a cyclohexyl group, a cycloheptyl group, a cyclooctyl group, an adamantanyl group, a norbornanyl group, a bicyclo[1.1.1]pentyl group, a bicyclo[2.1.1]hexyl group, a bicyclo[2.2.1]heptyl group, a bicyclo[2.2.2]octyl group, and the like, but embodiments of the present disclosure are not limited thereto.

An angle formed by a plane including the transition dipole moment of the organometallic compound represented by Formula 1 and a plane including four atoms of a tetradentate ligand linked to a metal M of Formula 1 may be about 10° or less. In addition, a horizontal orientation ratio of the transition dipole moment of the organometallic compound represented by Formula 1 may be in a range of about 80 % to about 100 %.

For example, the angle formed by the plane including the transition dipole moment of the organometallic compound and the plane including the four atoms of the tetradentate ligand linked to the metal (or platinum) of Formula 1 may be 0° to 10°, 0° to 9°, 0° to 8°, 0° to 7°, 0° to 6°, 0° to 5°, 0° to 4°, 0° to 3°, 0° to 2°, or 0° to 1°, but embodiments of the present disclosure are not limited thereto. When the angle formed by the plane including the transition dipole moment of the organometallic compound represented by the Formula 1 and the plane including the four atoms of the tetradentate ligand linked to the metal of Formula 1 is within these ranges, the organometallic compound may have excellent planarity, and a thin film formed by using the organometallic compound may have excellent electric characteristics.

In an embodiment, the horizontal orientation ratio of the transition dipole moment of the organometallic compound may be in a range of, for example, 80 % to 100 %, 81 % to 100 %, 82 % to 100 %, 83 % to 100 %, 84 % to 100 %, 85 % to 100 %, 86 % to 100 %, 87 % to 100 %, 88 % to 100 %, 89 % to 100 %, 90 % to 100 %, 91 % to 100 %, 92 % to 100 %, 93 % to 100 %, 94 % to 100 %, 95 % to 100 %, 96 % to 100 %, 97 % to 100 %, 98 % to 100 %, 99 % to

100 %, or 100 %, but embodiments of the present disclosure are not limited thereto.

The horizontal orientation ratio of the transition dipole moment means a ratio of the organometallic compound having a transition dipole moment horizontal to the film including the organometallic compound to the total organometallic compound in the film including the organometallic compound.

The horizontal orientation ratio of the transition dipole moment may be evaluated by using an angle-dependent PL measurement apparatus. The angle-dependent PL measurement apparatus may be understood by referring to Korean Patent Application No. 10-2013-0150834.

Since the organometallic compound has a high horizontal orientation ratio of a transition dipole moment, the organometallic compound has a large horizontal orientation transition dipole moment (that is, a large horizontal optical orientation). Therefore, a large amount of electric field traveling in a direction perpendicular to the film including the organometallic compound may be emitted. Light emitted due to such a mechanism may have high external extraction efficiency (that is, efficiency of extracting light emitted in the organometallic compound from a device (for example, an organic light-emitting device) including a film (for example, an emission layer described below) including the organometallic compound) to the outside, and thus, an electronic device, for example, an organic light-emitting device, which includes the organometallic compound, may have high luminescent efficiency.

The terms "an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, and an azadibenzothiophene 5,5-dioxide group" as used herein each refer to a heterocyclic group having the same backbone as that of each of "an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, and a dibenzothiophene 5,5-dioxide group", respectively, wherein at least one of ring-forming carbons of the rings above is substituted with nitrogen.

In addition, $R_{10a}$ is defined the same as $R_1$.

For example, the organometallic compound represented by Formula 1 may include at least one deuterium.

In an embodiment, the organometallic compound may be one of the following Compounds 1 to 3599:

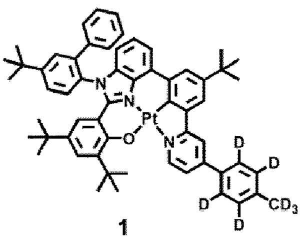
1

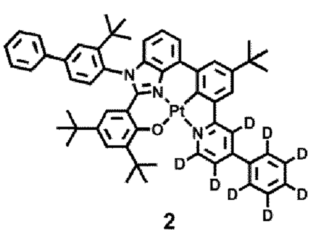
2

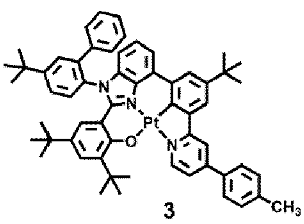
3

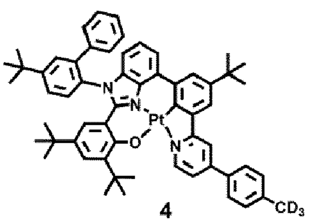
4

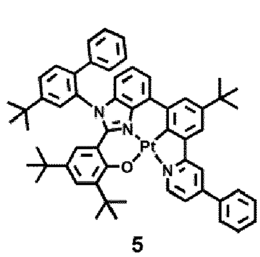
5

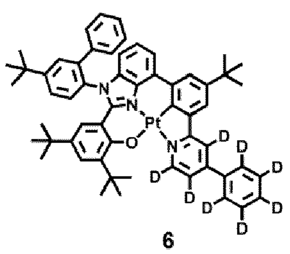
6

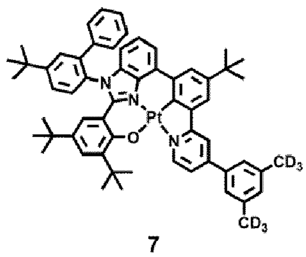
7

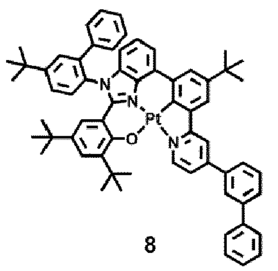
8

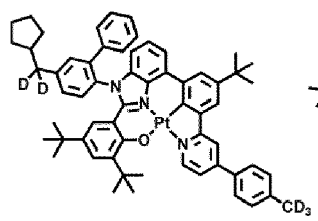
9

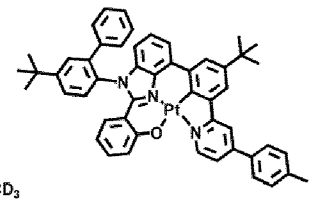
10

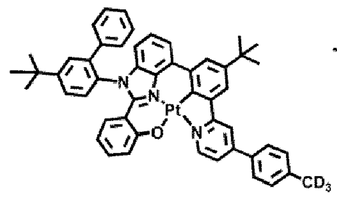
11

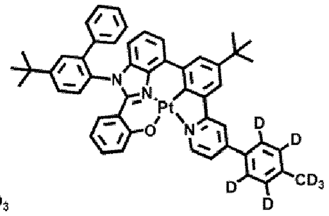
12

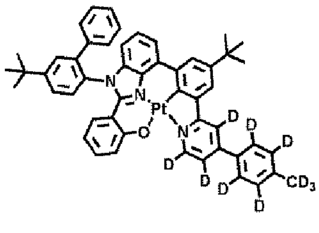
13

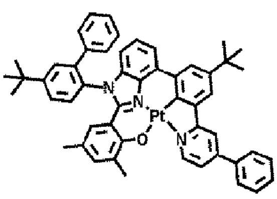
14

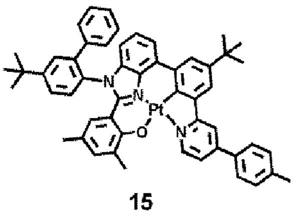
15

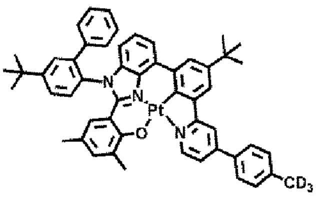
16

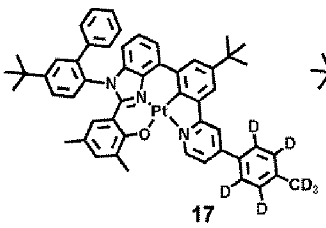
17

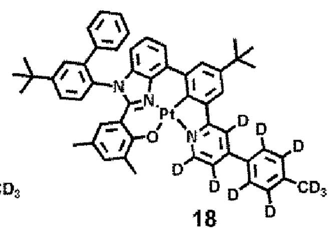
18

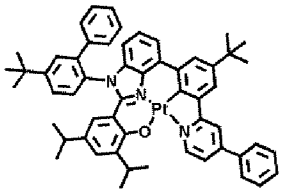
19

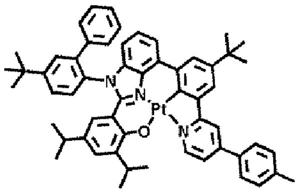
20

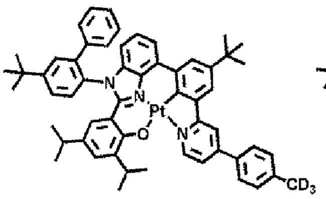
21

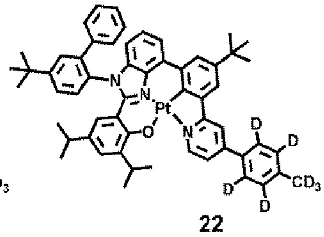
22

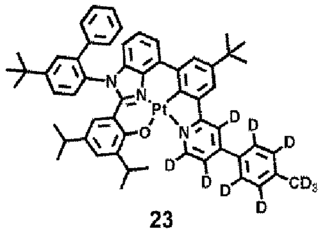
23

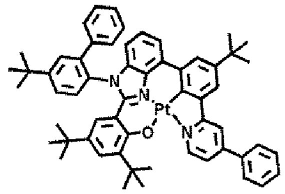
24

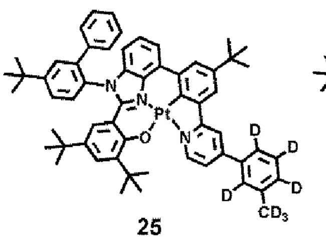
25

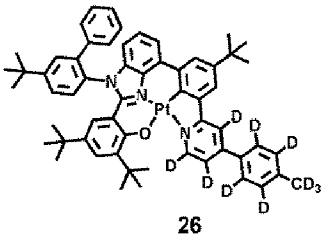
26

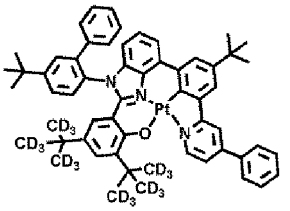
27

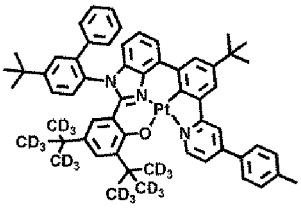
28

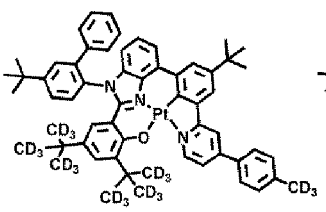
29

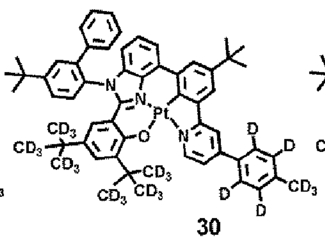
30

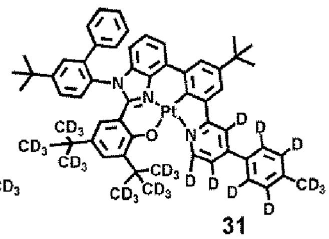
31

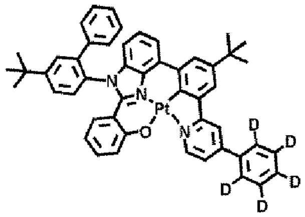
32

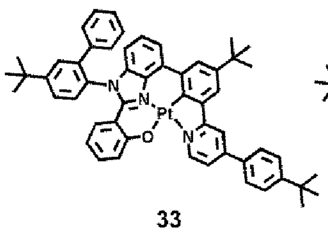
33

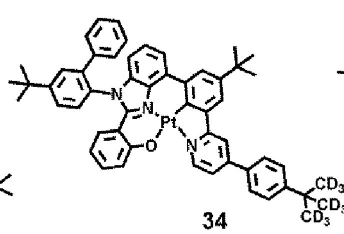
34

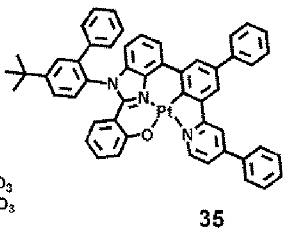
35

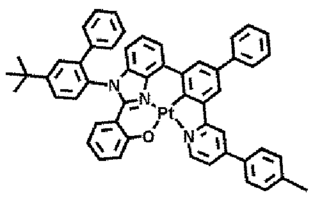
36

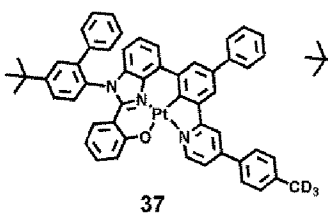
37

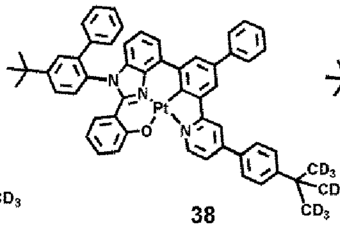
38

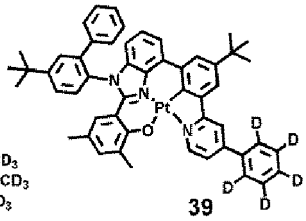
39

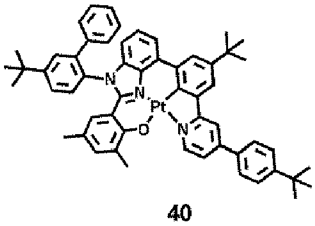
40

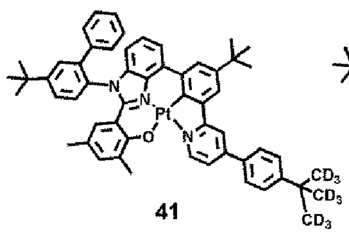
41

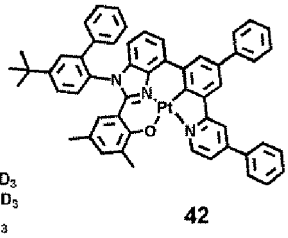
42

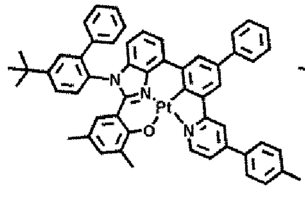
43

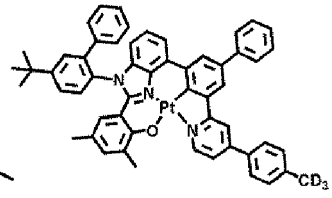
44

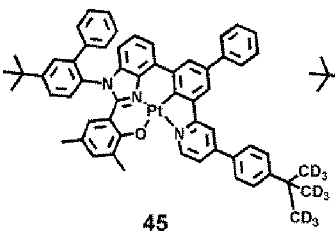
45

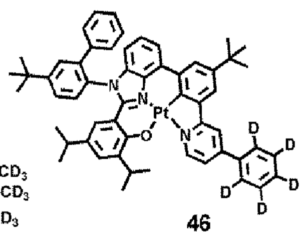
46

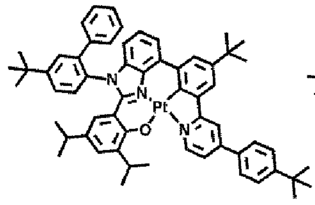
47

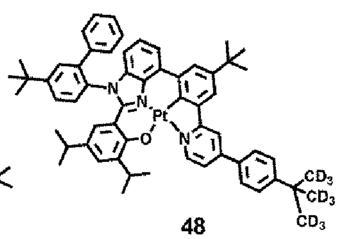
48

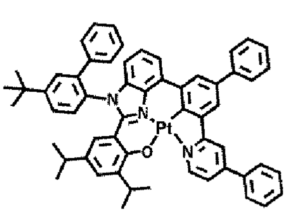
49

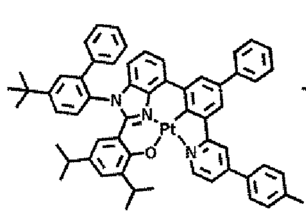
50

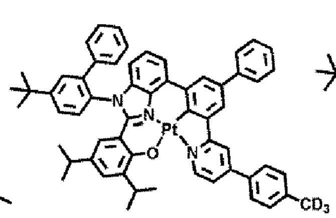
51

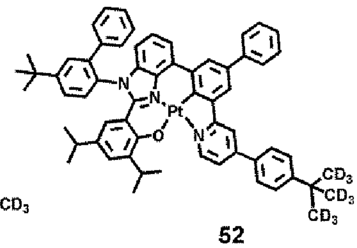
52

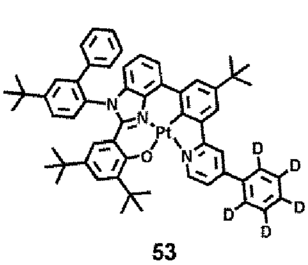
53

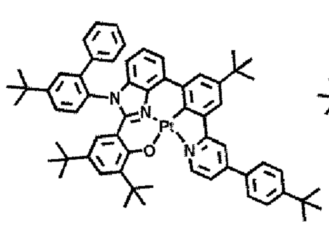
54

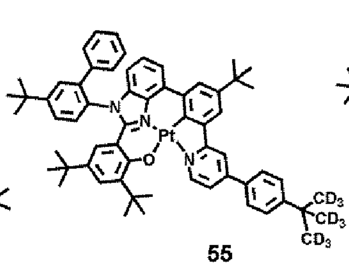
55

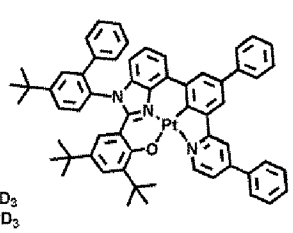
56

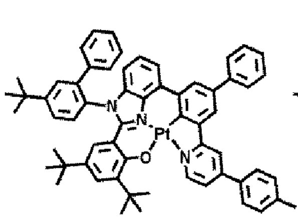
57
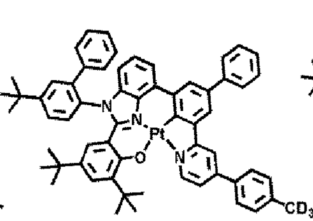
58
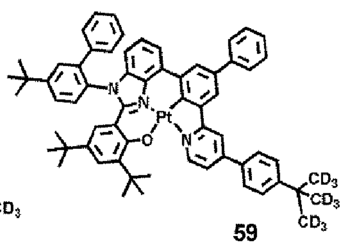
59
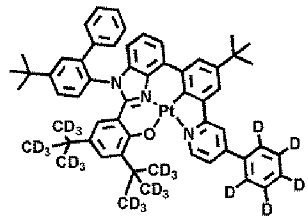
60

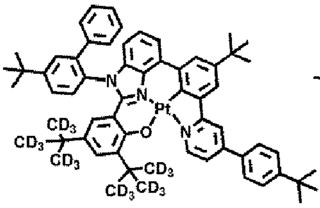
61
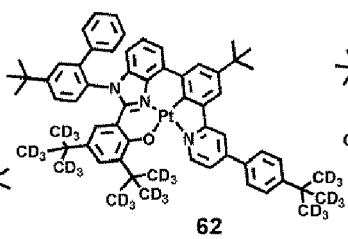
62
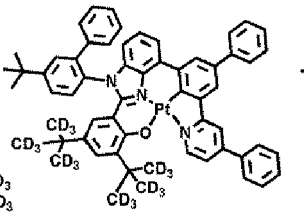
63
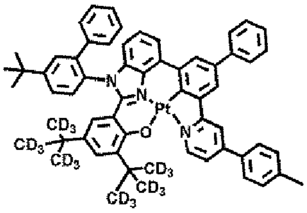
64

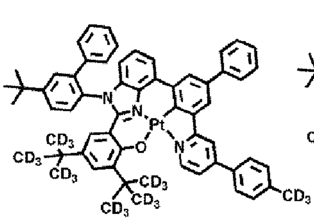
65
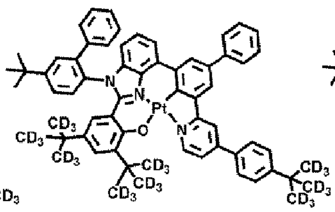
66
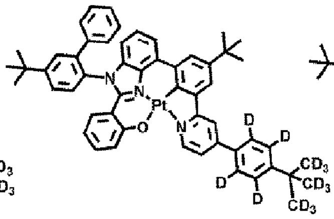
67
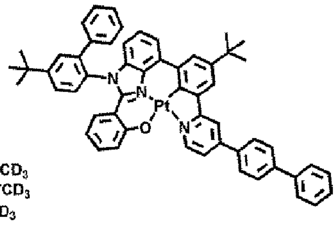
68

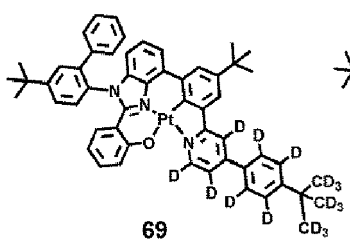
69
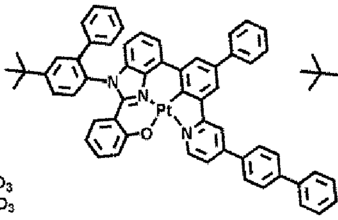
70
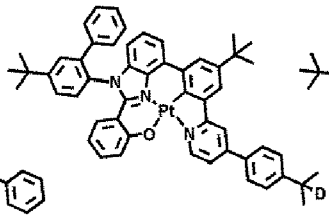
71
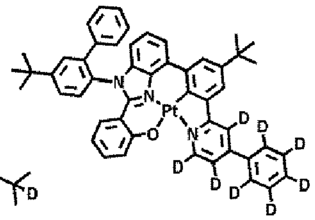
72

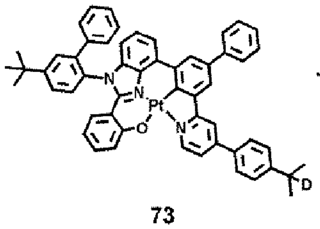
73
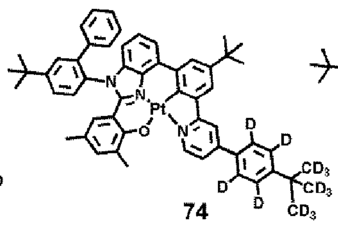
74
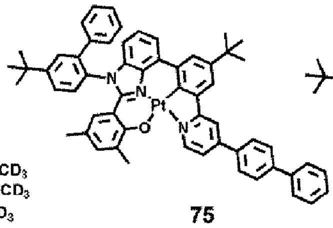
75
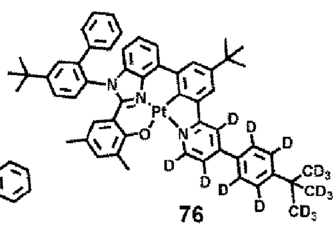
76

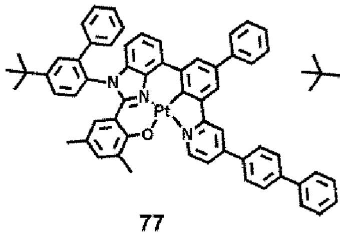
77
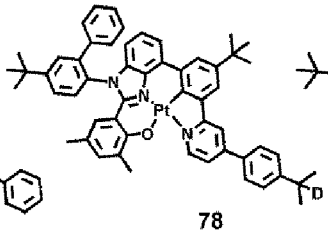
78
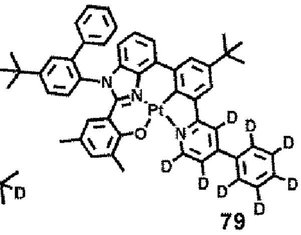
79
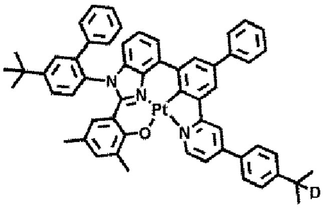
80

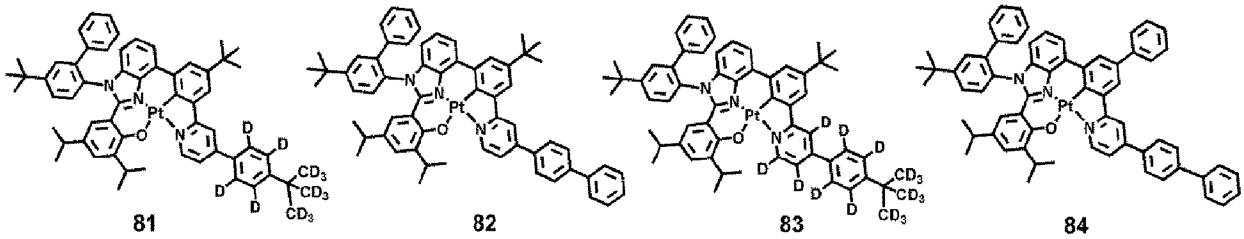

81 82 83 84

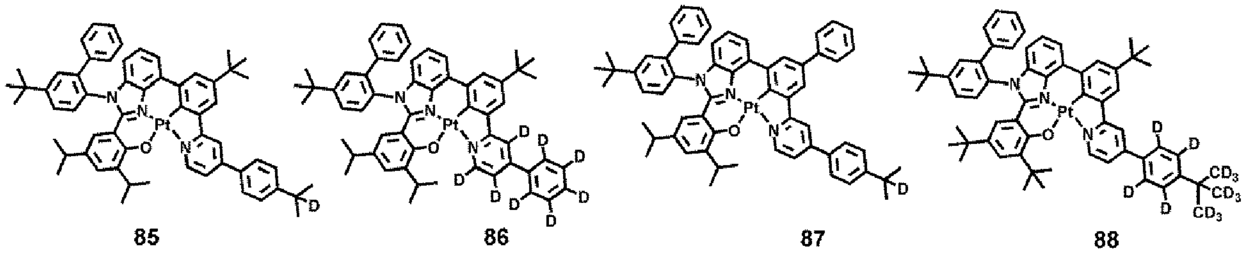

85 86 87 88

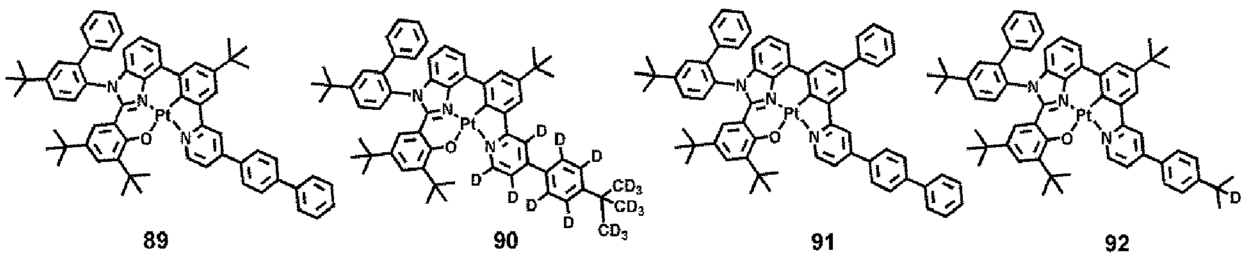

89 90 91 92

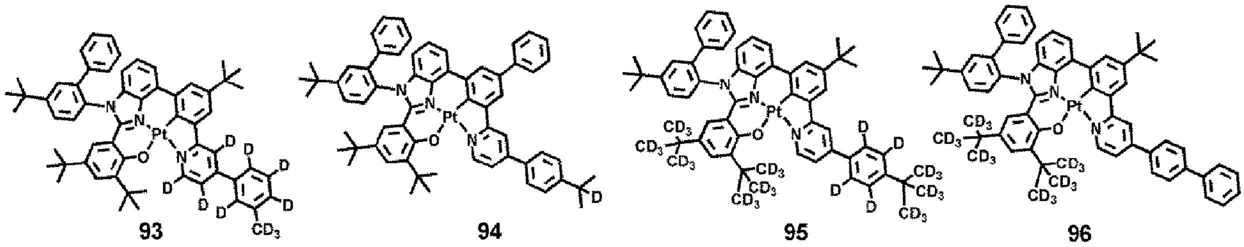

93 94 95 96

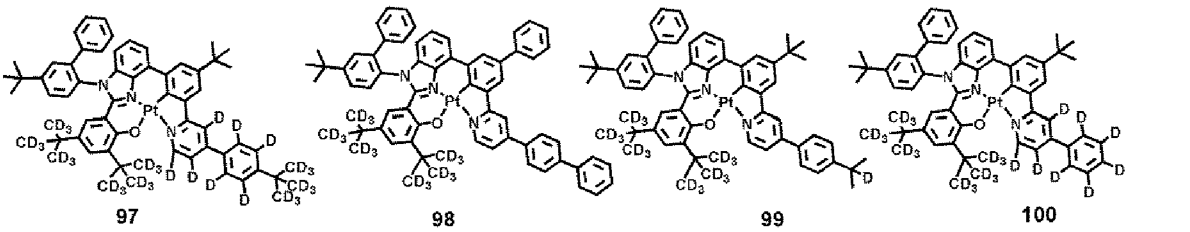

97 98 99 100

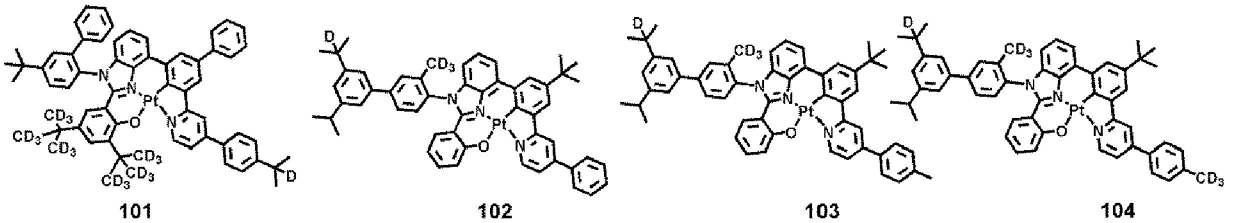

101 102 103 104

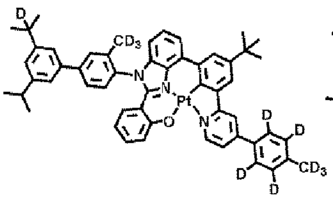
105

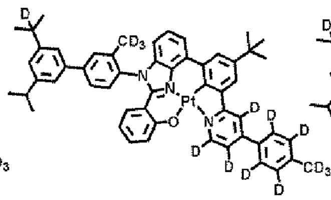
106

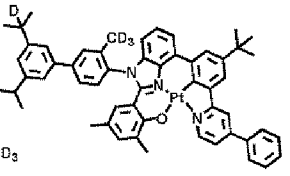
107

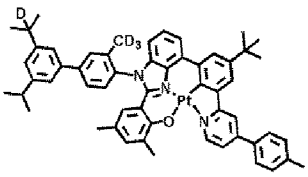
108

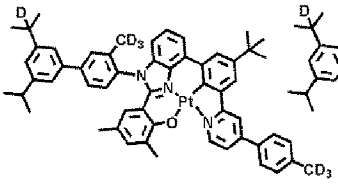
109

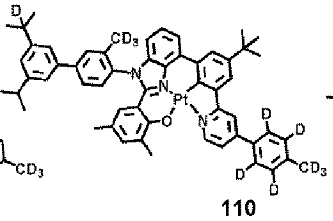
110

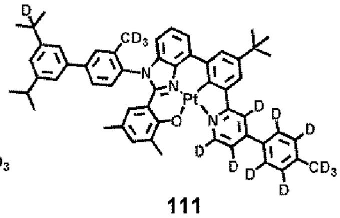
111

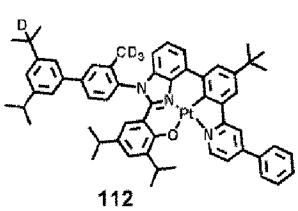
112

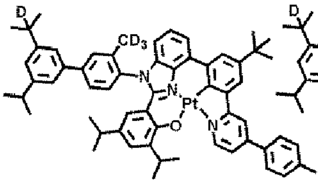
113

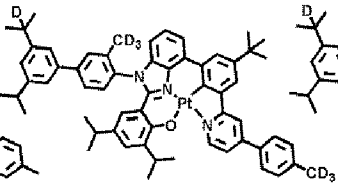
114

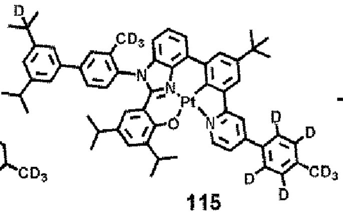
115

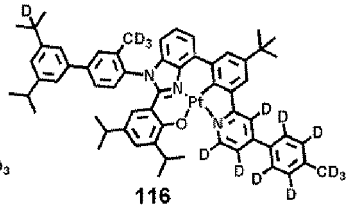
116

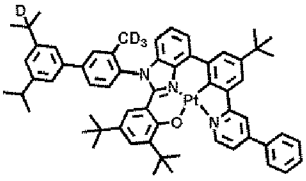
117

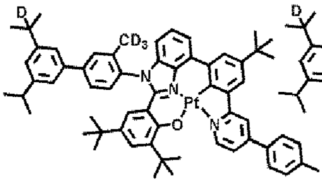
118

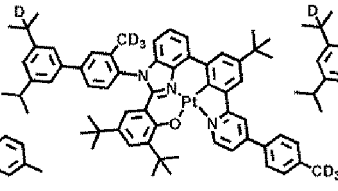
119

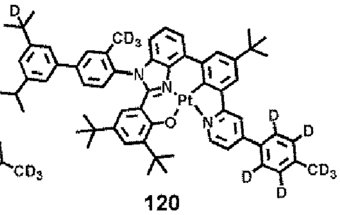
120

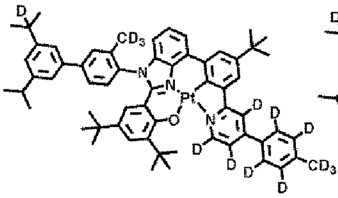
121

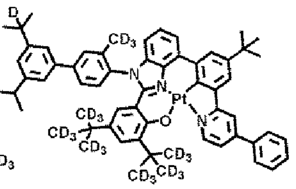
122

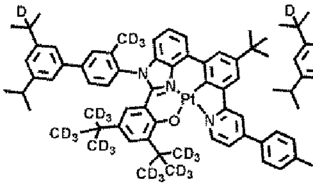
123

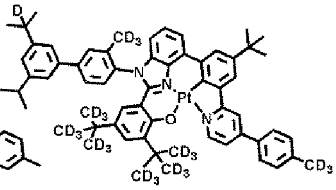
124

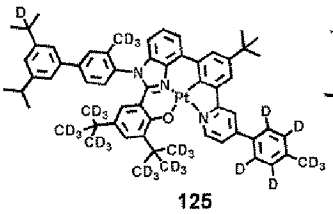
125

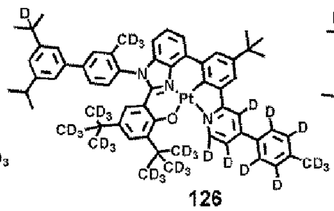
126

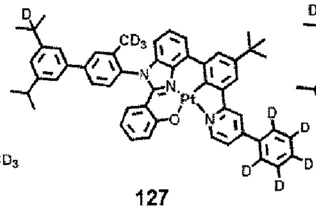
127

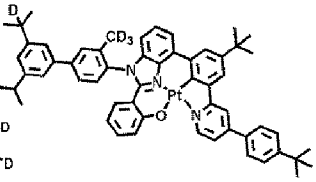
128

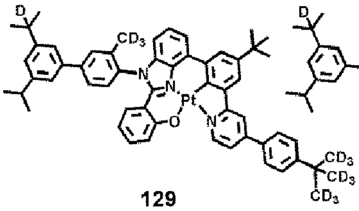
129

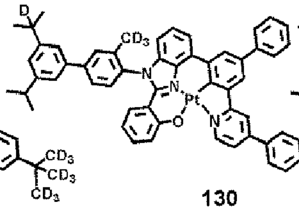
130

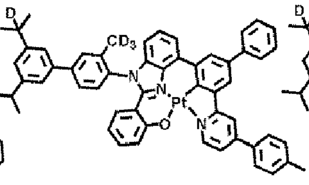
131

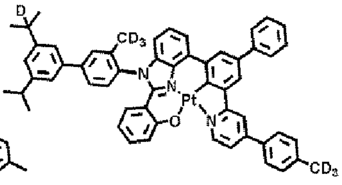
132

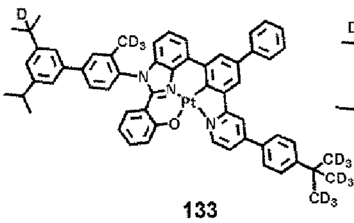
133

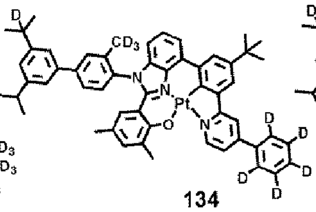
134

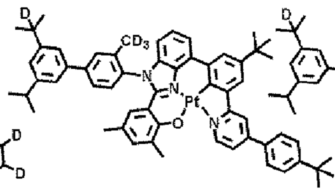
135

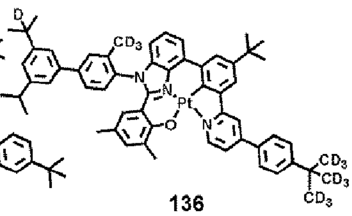
136

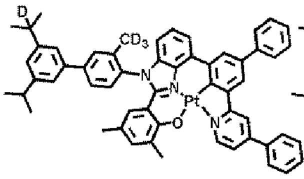
137

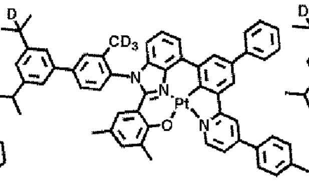
138

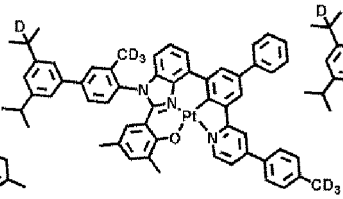
139

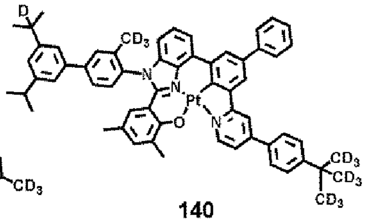
140

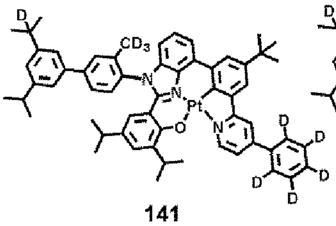
141

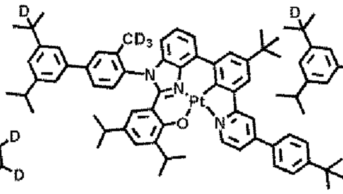
142

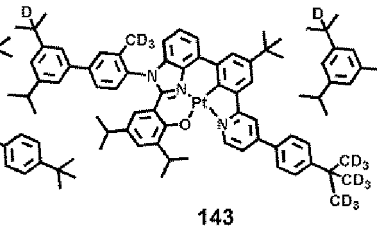
143

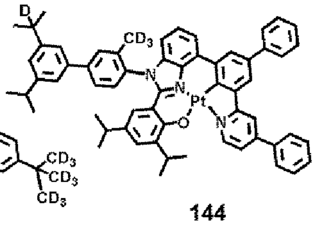
144

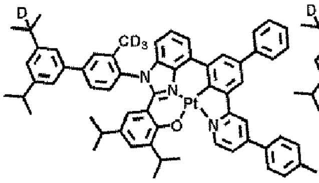
145

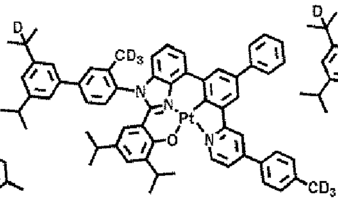
146

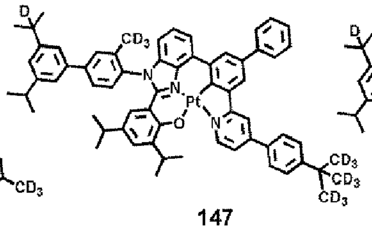
147

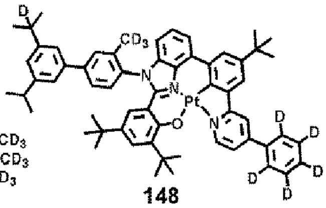
148

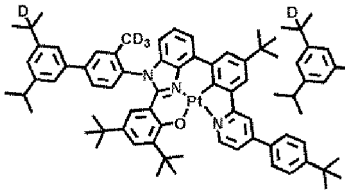
149

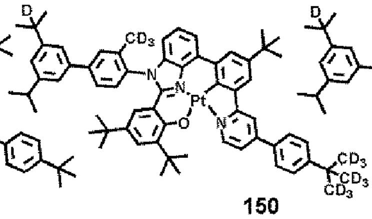
150

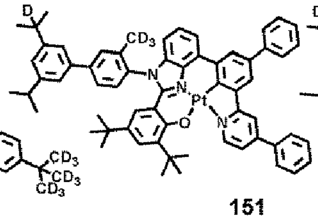
151

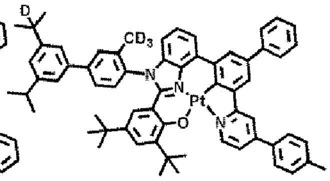
152

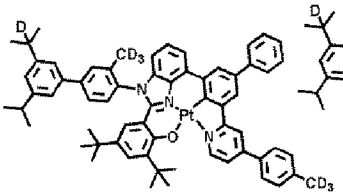
153

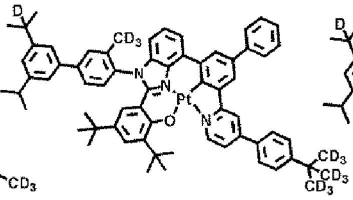
154

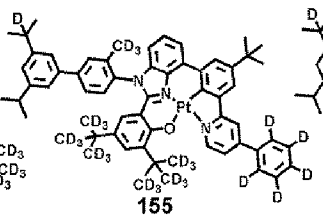
155

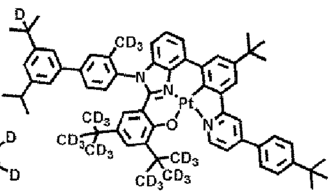
156

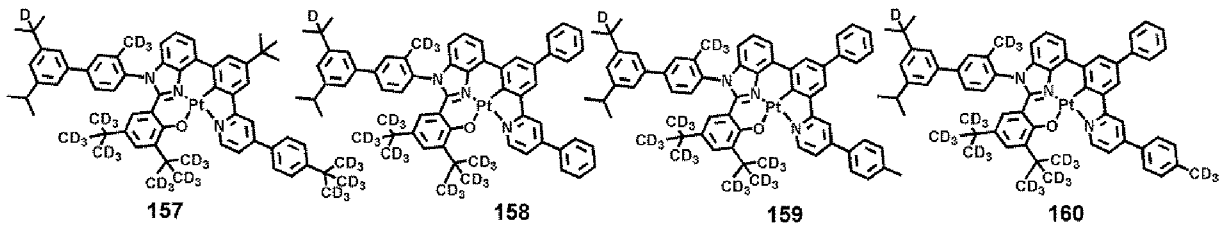

157 158 159 160

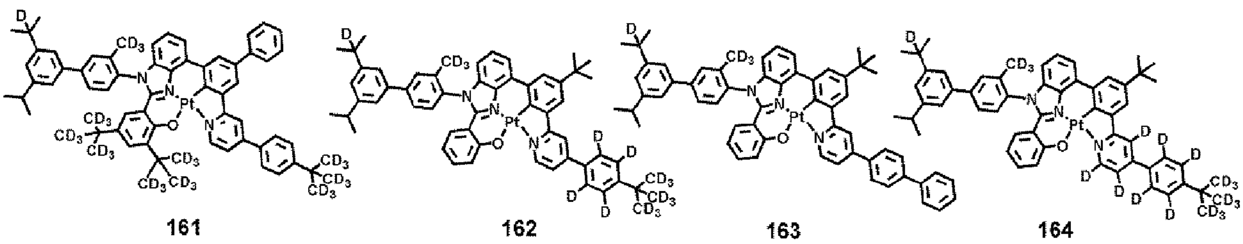

161 162 163 164

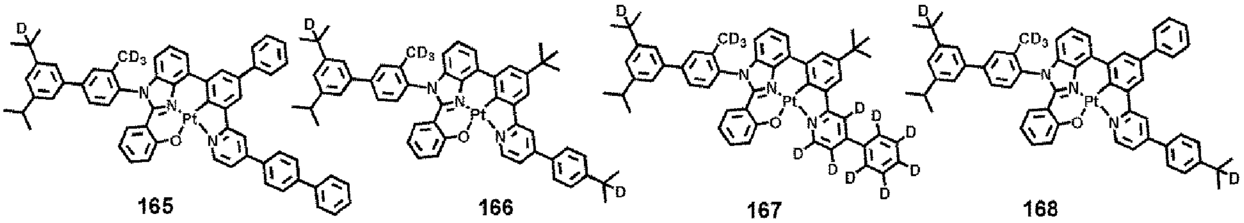

165 166 167 168

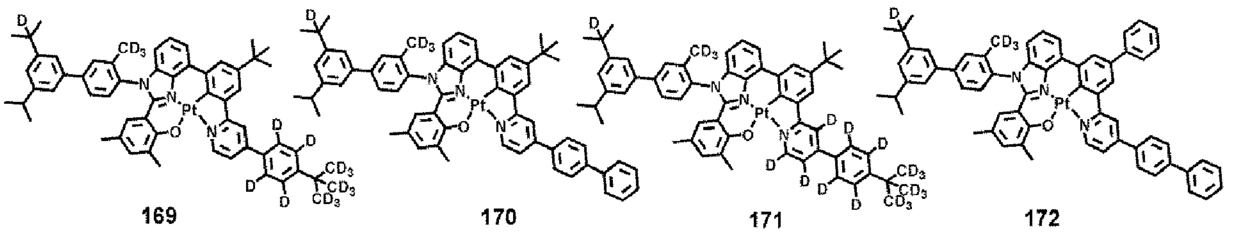

169 170 171 172

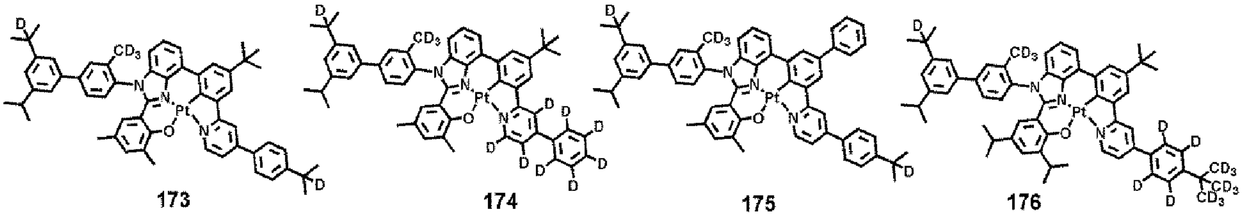

173 174 175 176

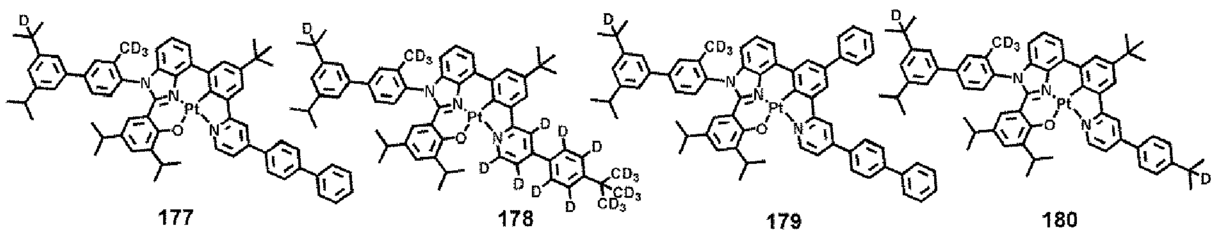

177 178 179 180

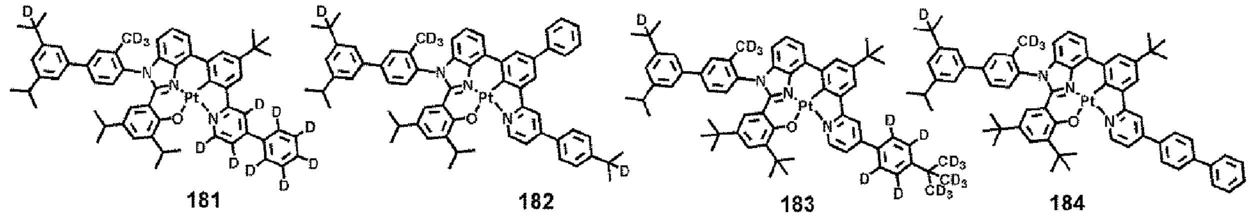

181 182 183 184

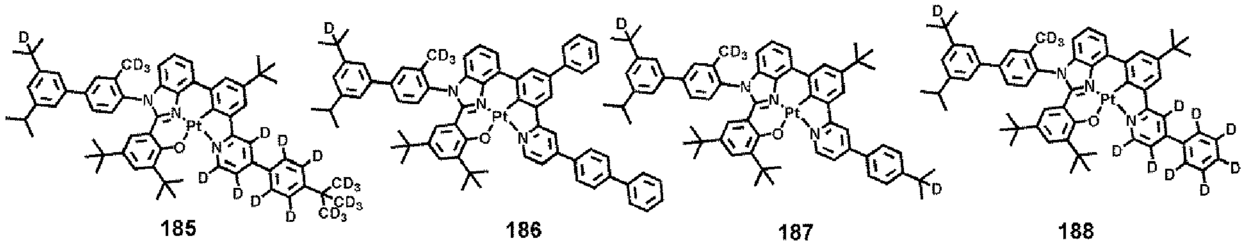

185 186 187 188

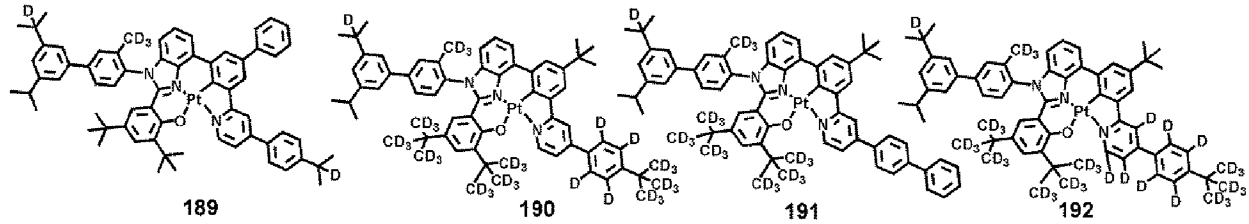

189 190 191 192

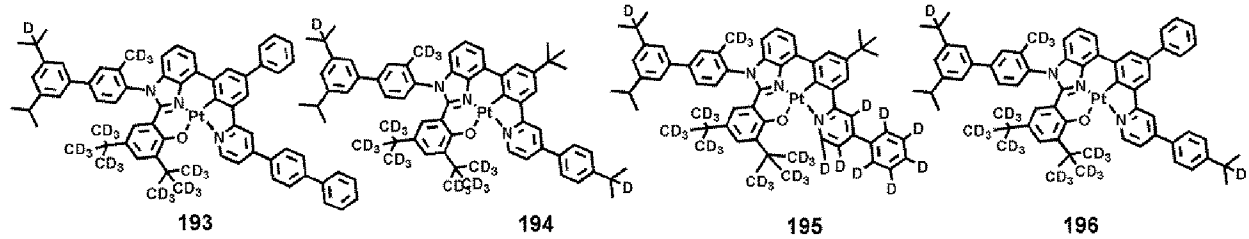

193 194 195 196

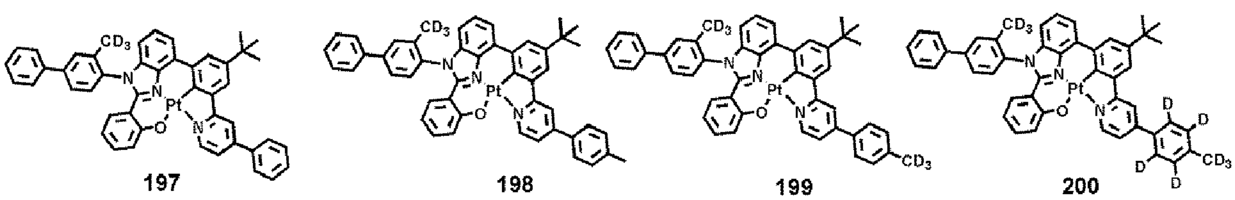

197 198 199 200

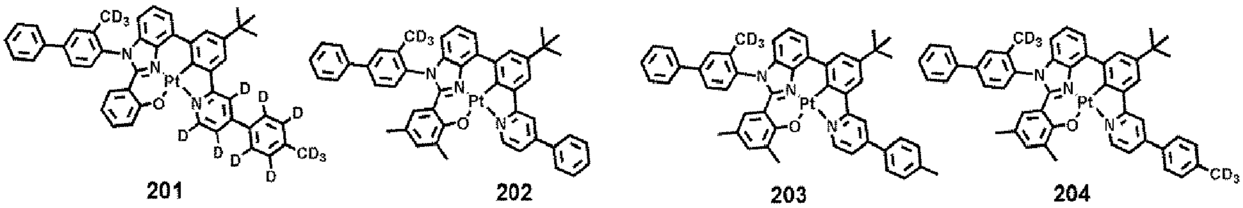

201 202 203 204

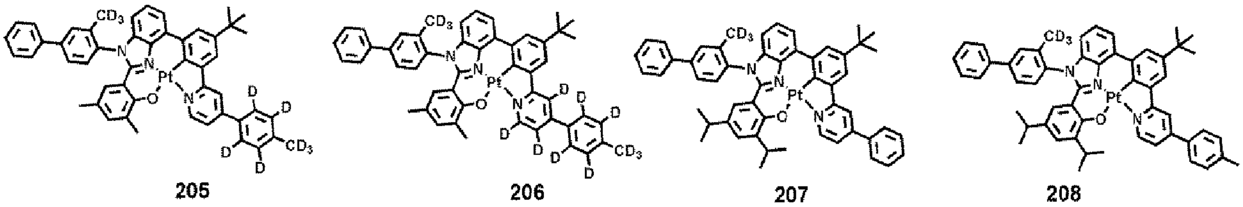

205 206 207 208

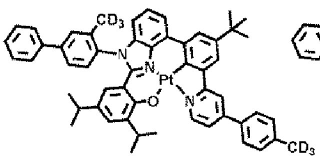
209

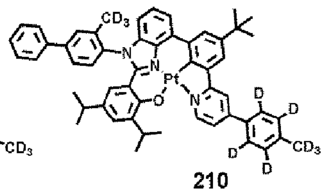
210

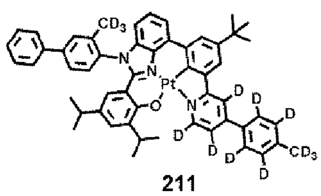
211

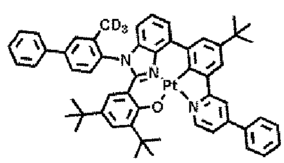
212

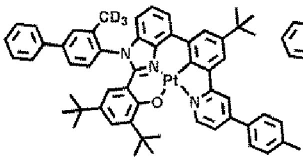
213

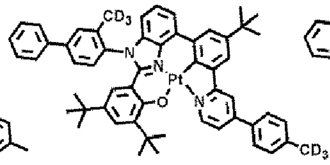
214

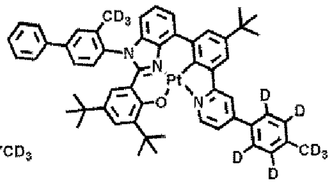
215

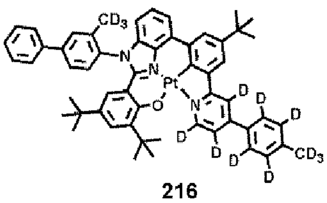
216

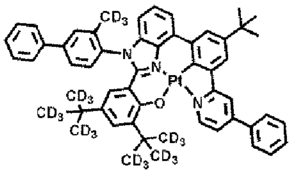
217

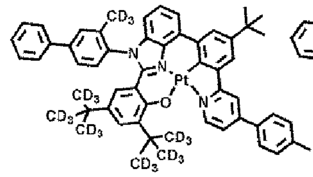
218

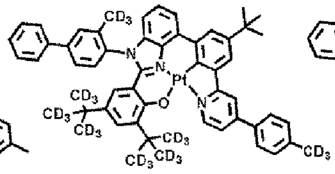
219

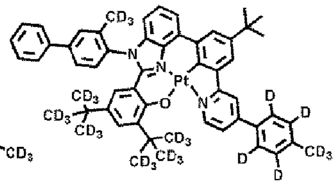
220

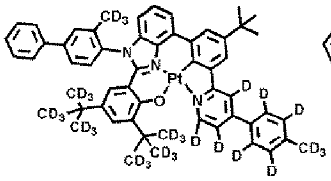
221

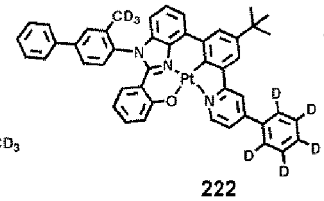
222

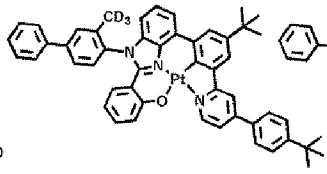
223

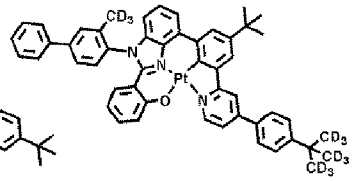
224

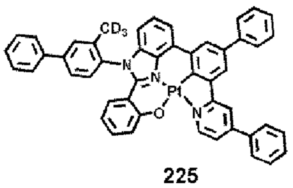
225

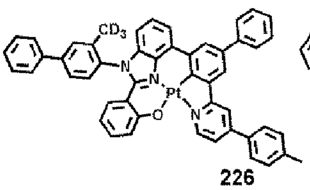
226

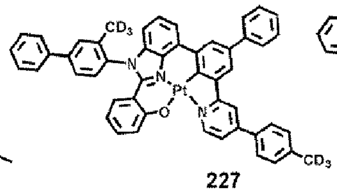
227

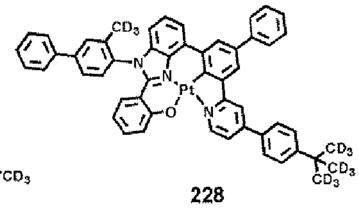
228

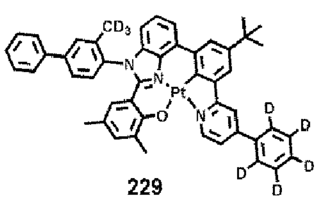
229

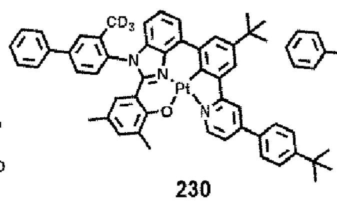
230

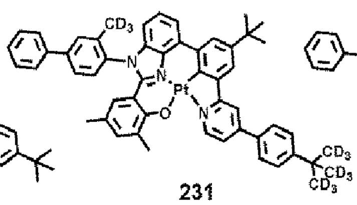
231

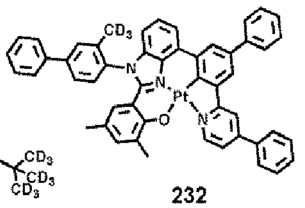
232

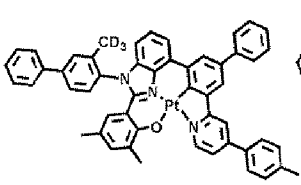
233

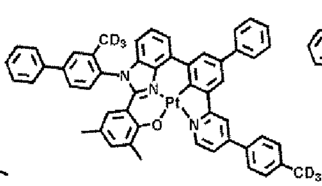
234

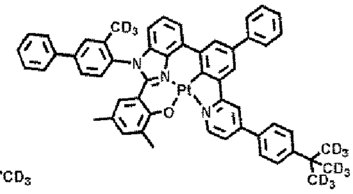
235

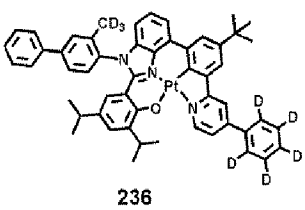
236

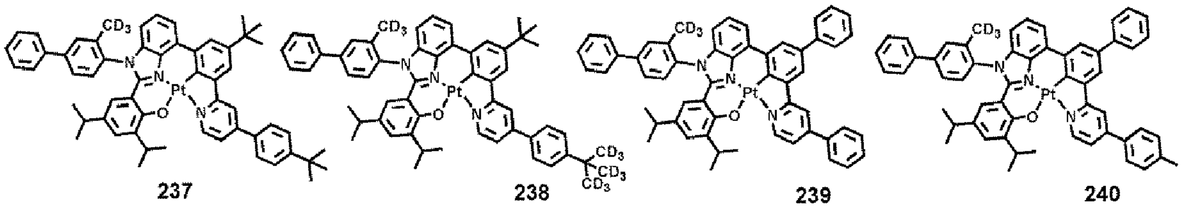

237 238 239 240

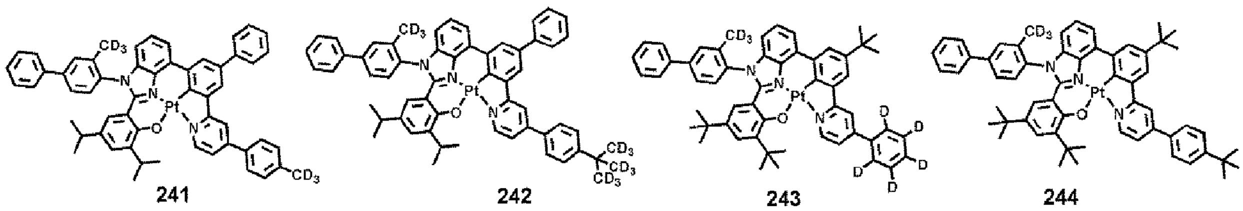

241 242 243 244

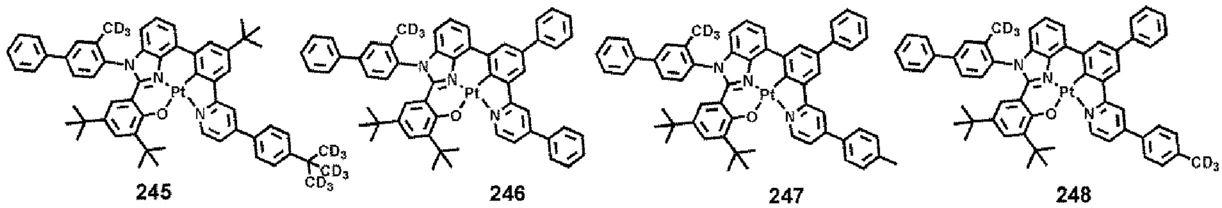

245 246 247 248

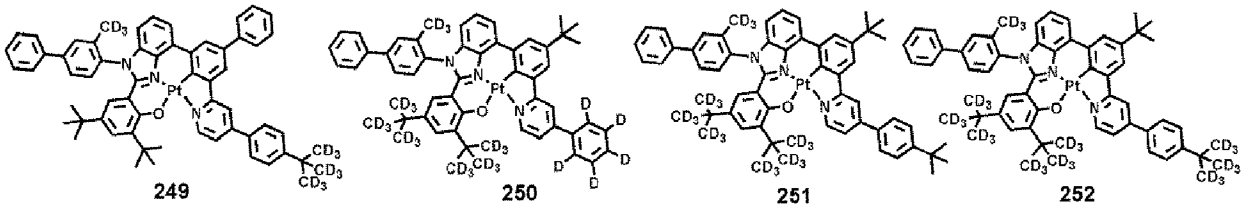

249 250 251 252

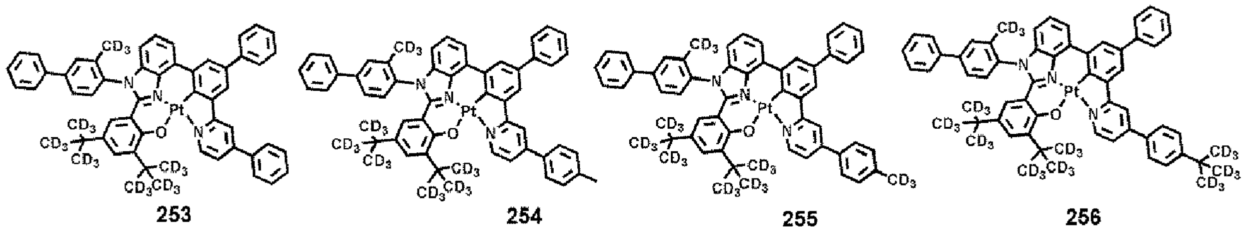

253 254 255 256

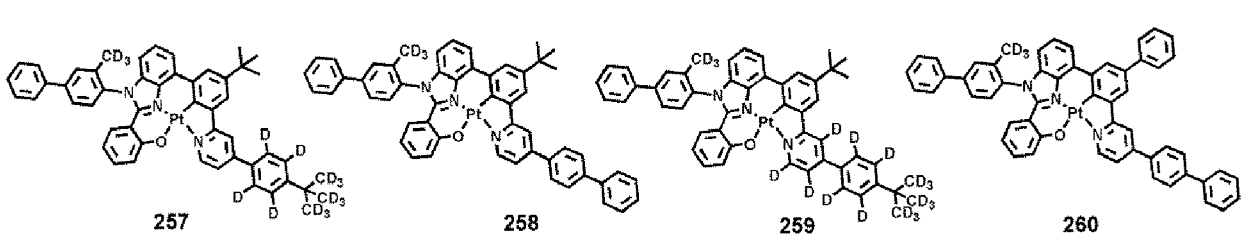

257 258 259 260

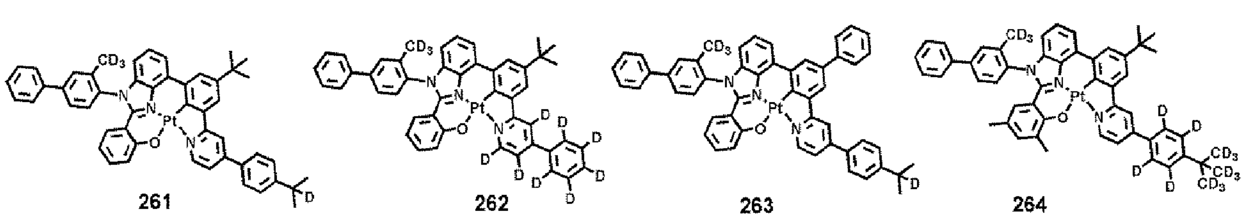

261 262 263 264

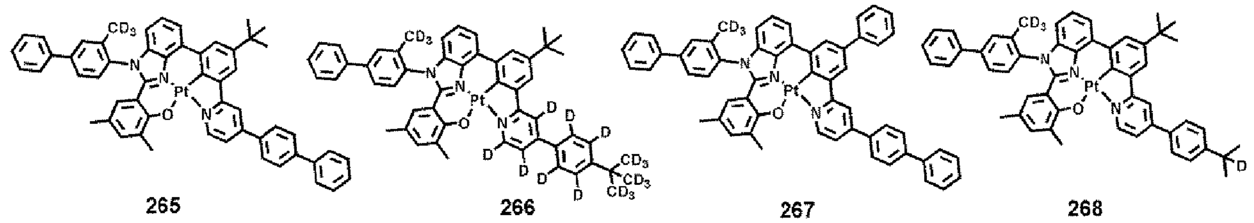

265 266 267 268

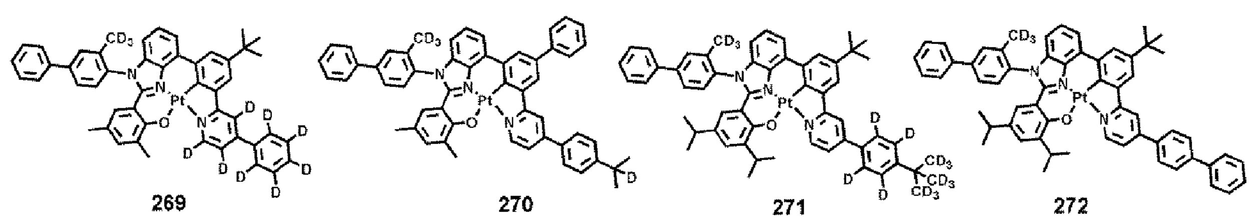

269 270 271 272

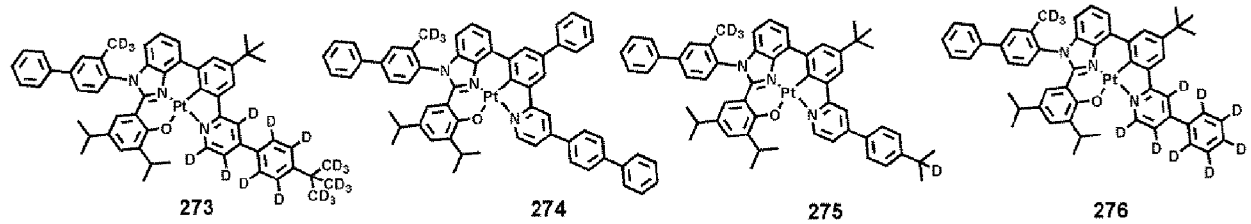

273 274 275 276

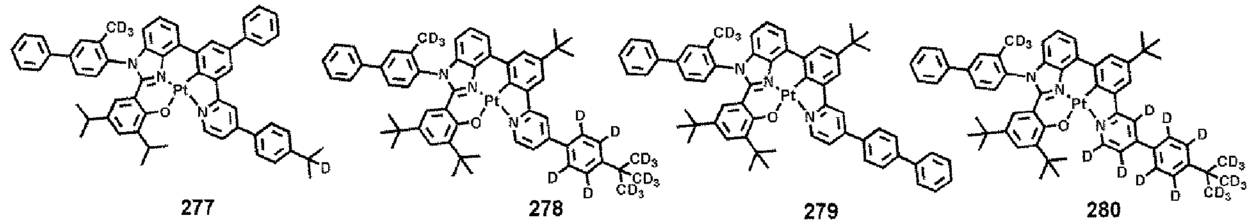

277 278 279 280

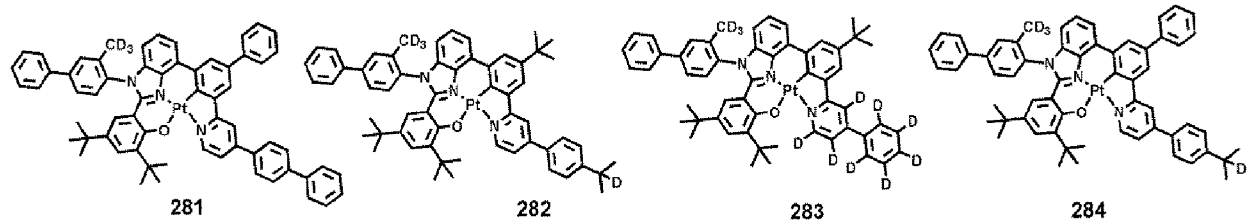

281 282 283 284

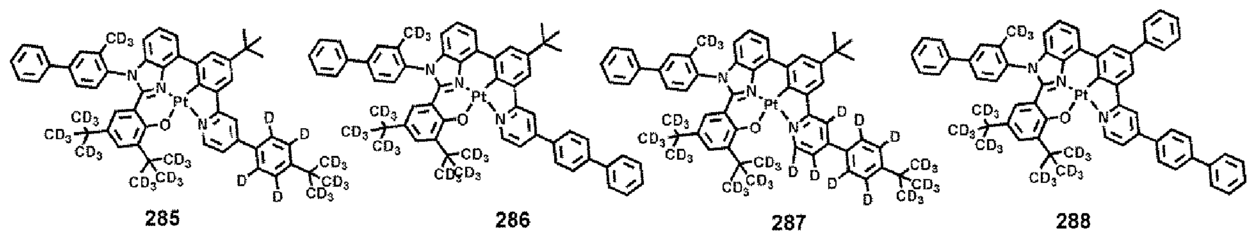

285 286 287 288

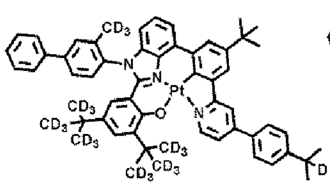
289

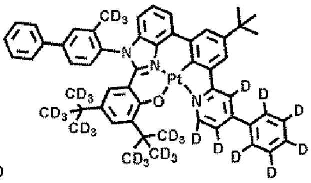
290

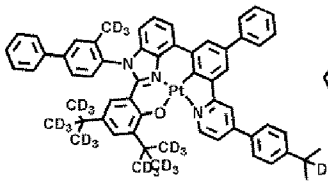
291

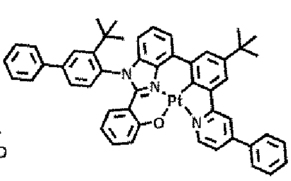
292

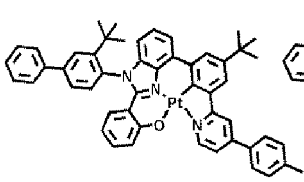
293

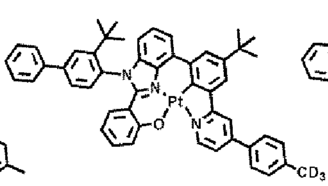
294

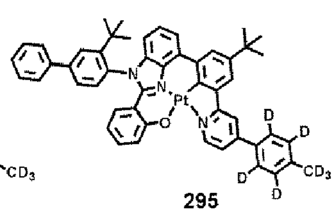
295

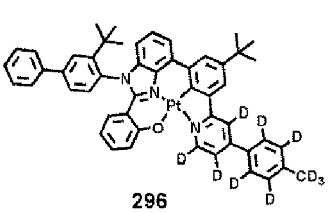
296

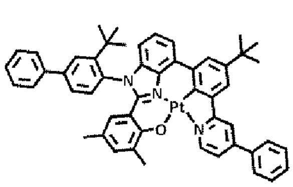
297

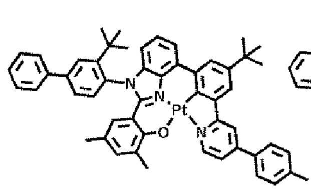
298

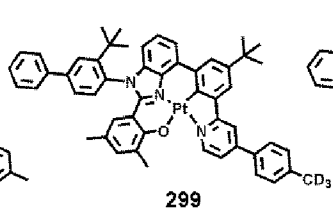
299

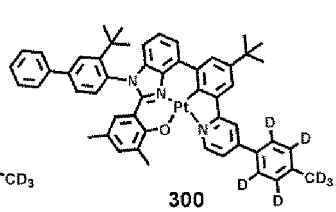
300

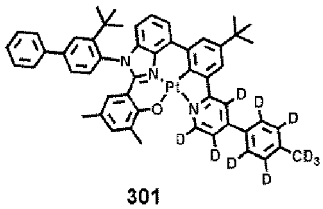
301

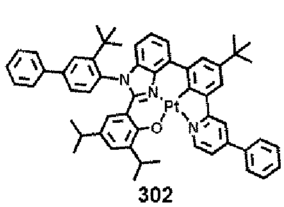
302

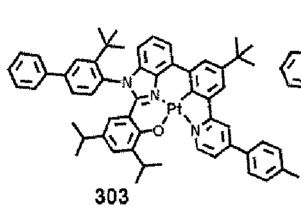
303

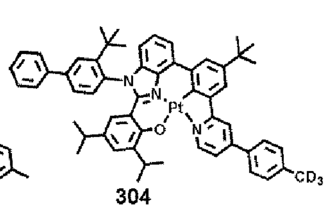
304

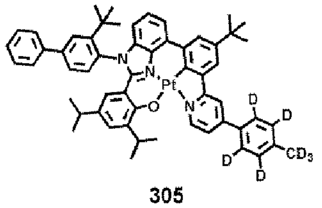
305

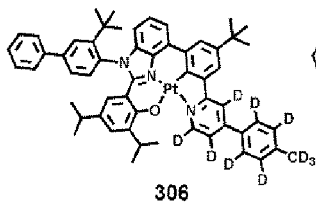
306

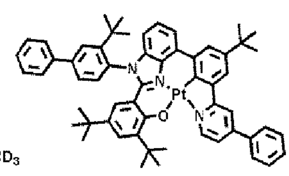
307

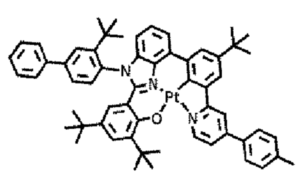
308

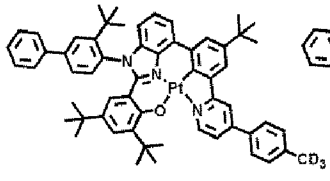
309

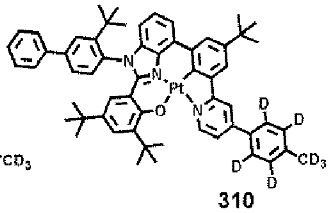
310

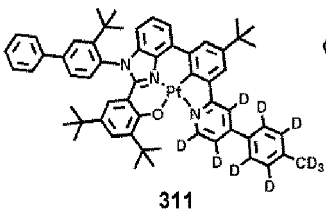
311

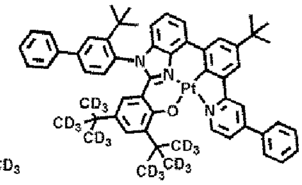
312

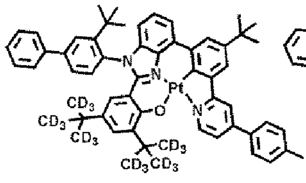
313

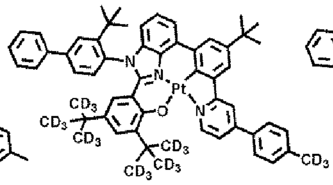
314

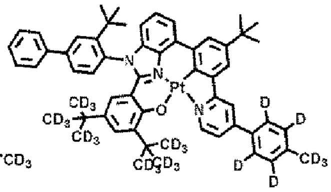
315

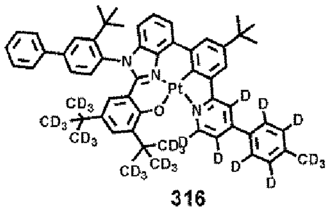
316

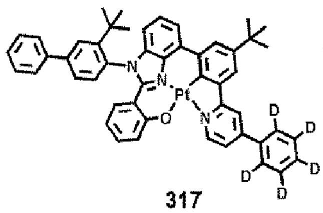

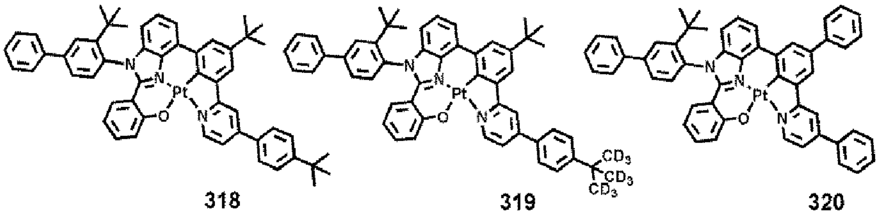

317 318 319 320

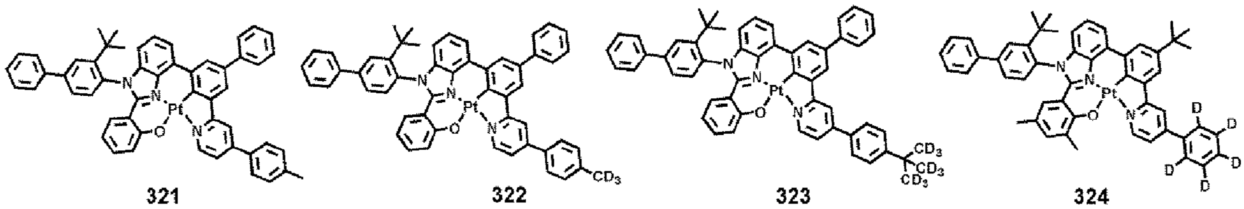

321 322 323 324

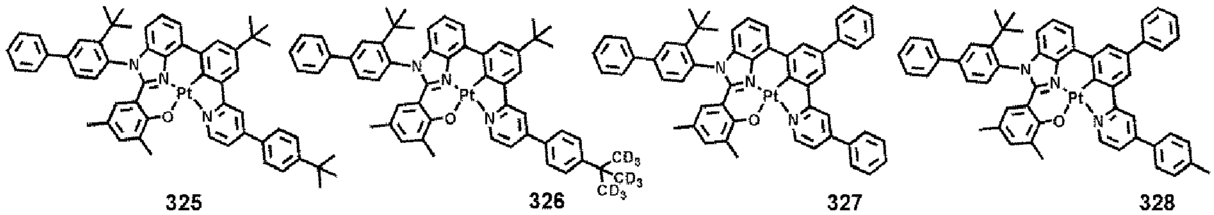

325 326 327 328

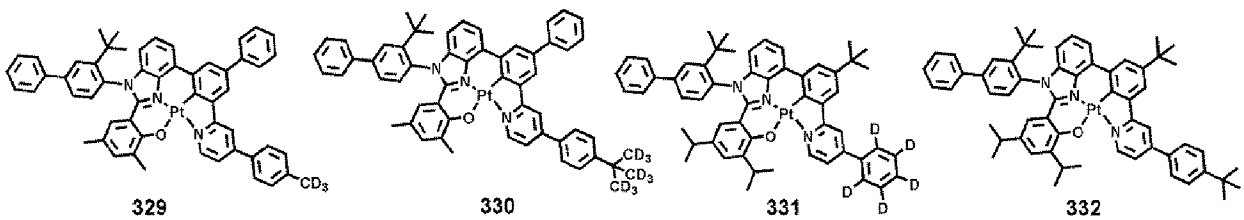

329 330 331 332

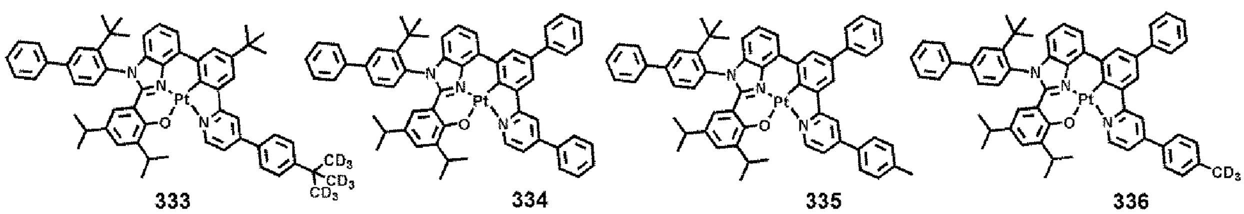

333 334 335 336

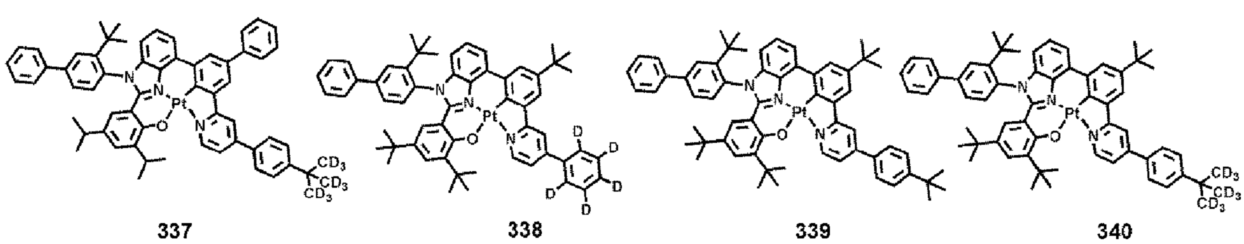

337 338 339 340

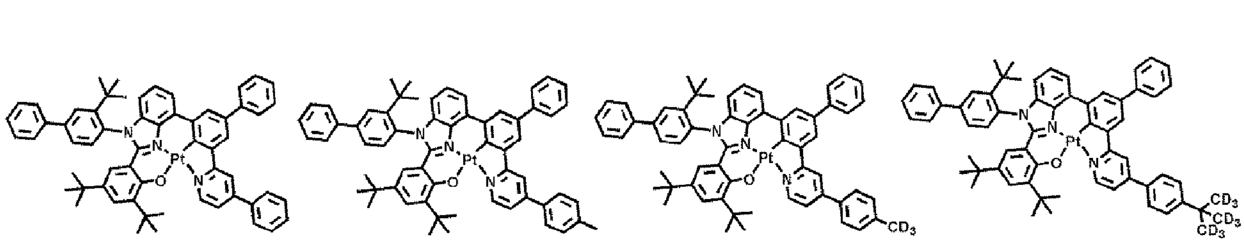

341 342 343 344

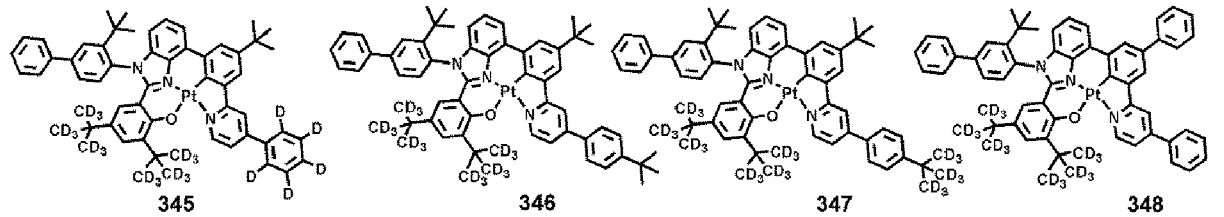

345 346 347 348

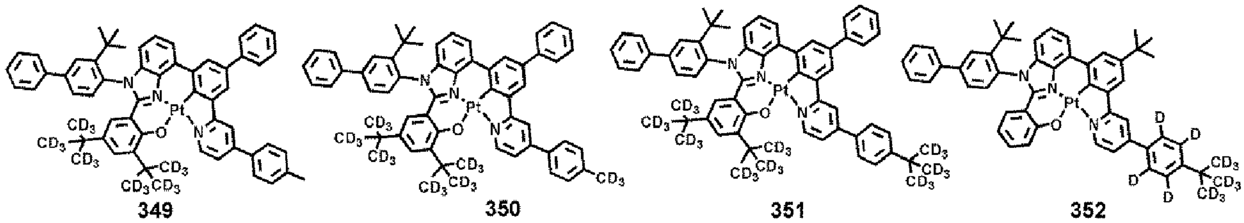

349 350 351 352

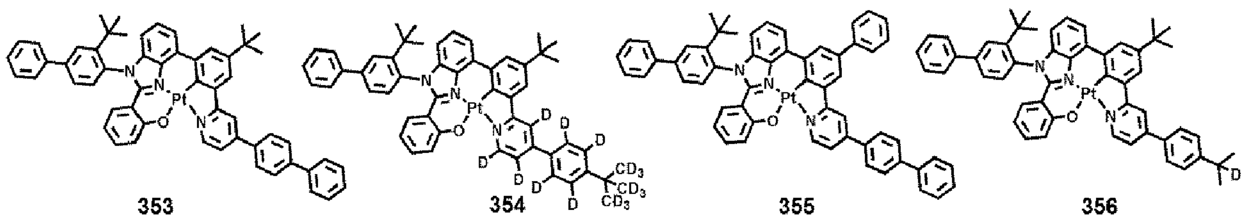

353 354 355 356

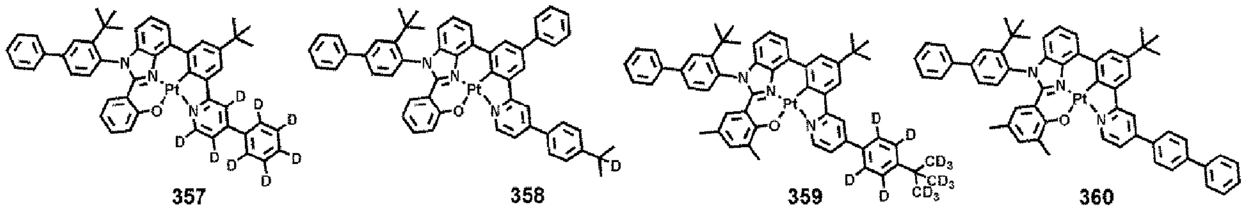

357 358 359 360

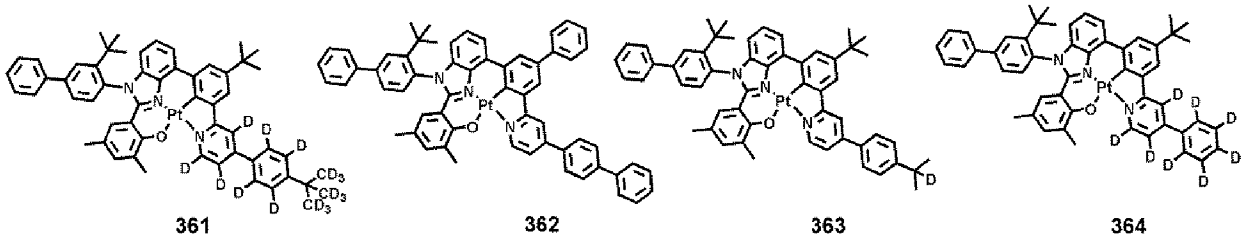

361 362 363 364

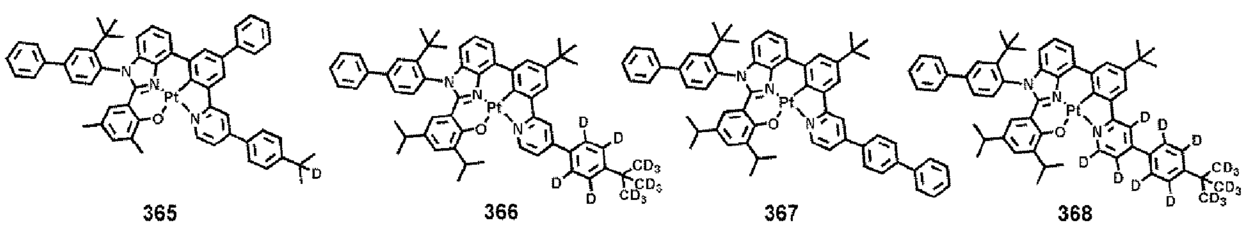

365 366 367 368

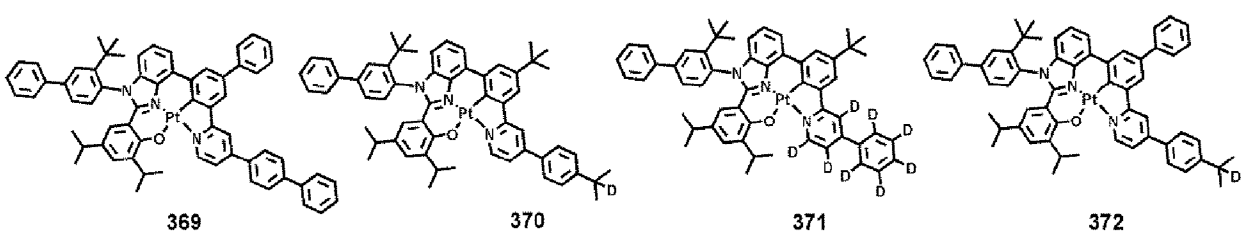

369 370 371 372

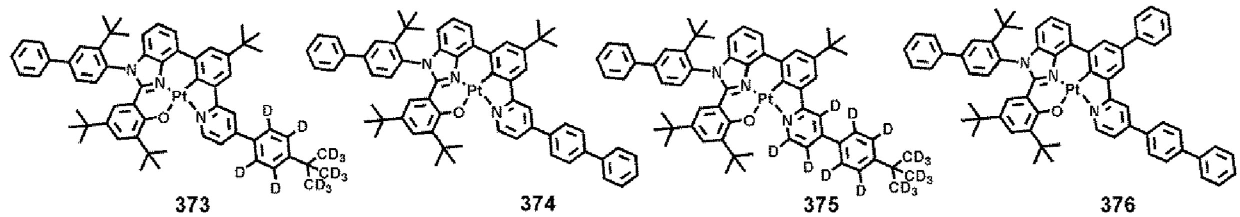

373 374 375 376

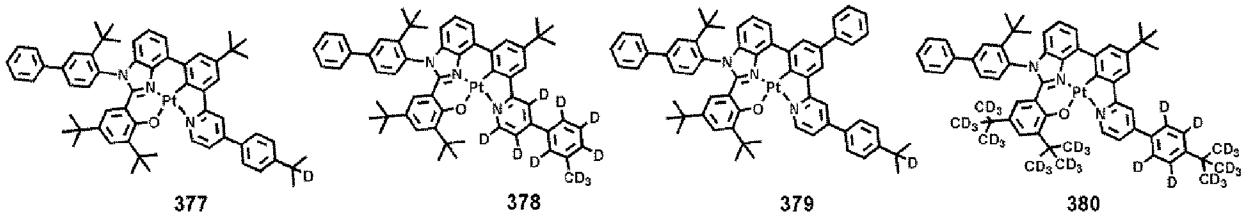

377 378 379 380

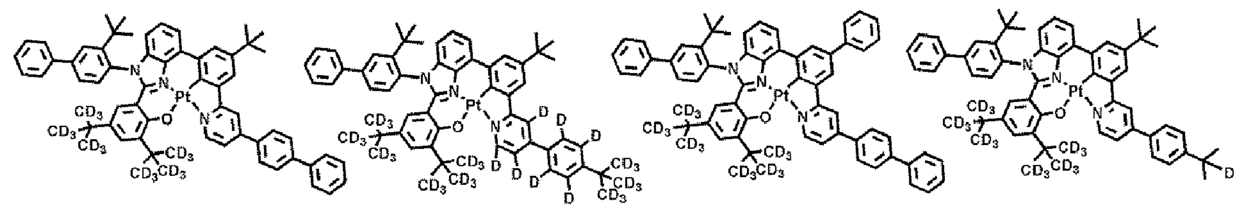

381 382 383 384

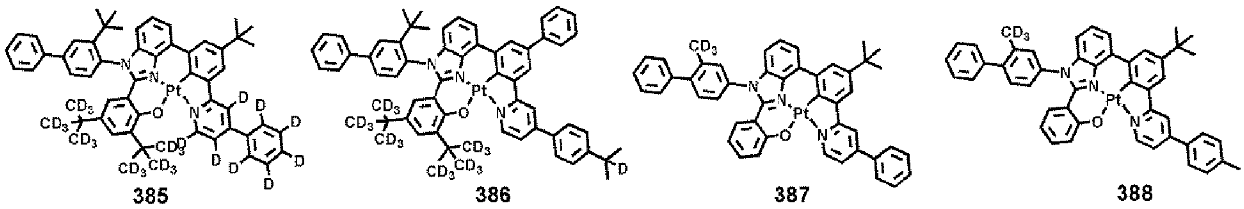

385 386 387 388

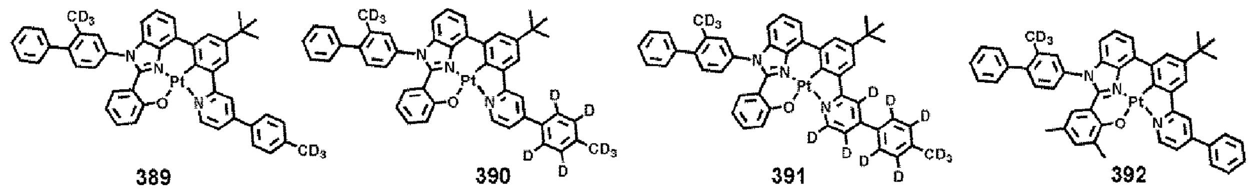

389 390 391 392

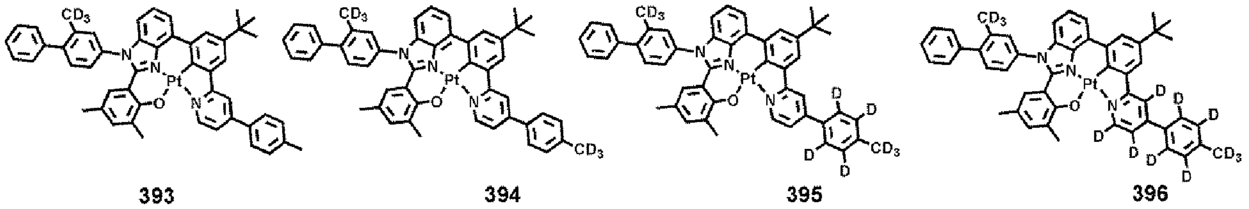

393 394 395 396

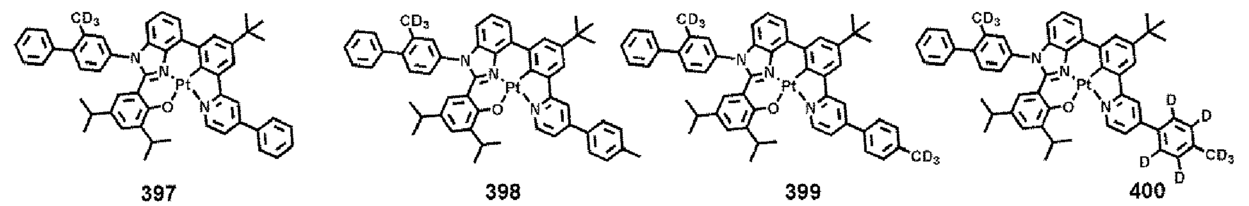

397 398 399 400

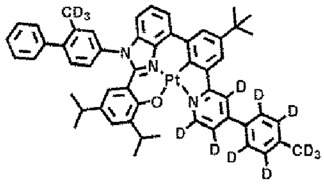
401
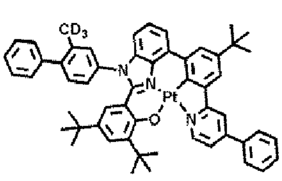
402
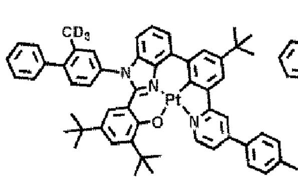
403
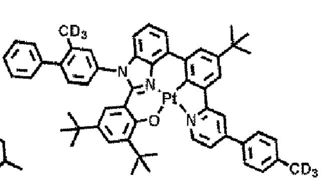
404

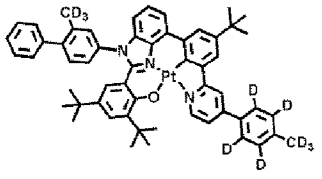
405
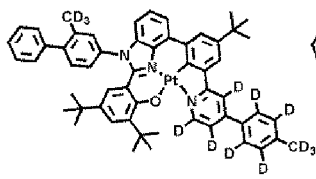
406
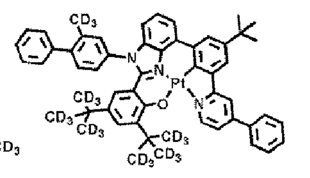
407
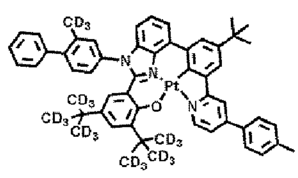
408

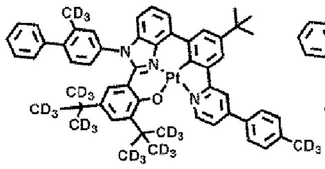
409
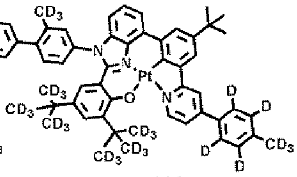
410
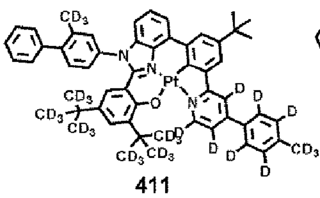
411
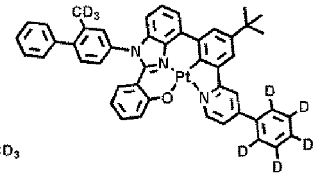
412

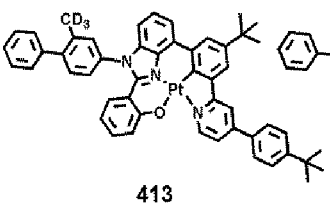
413
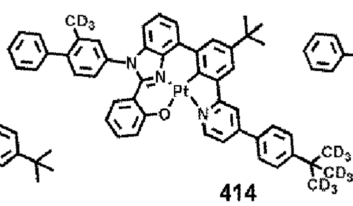
414
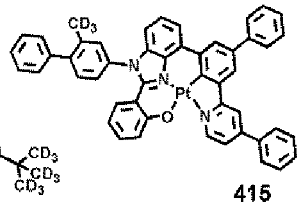
415
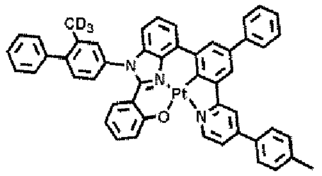
416

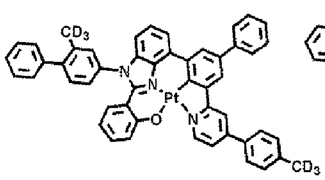
417
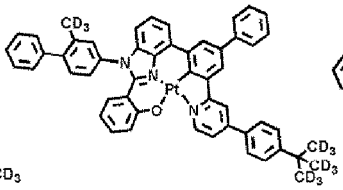
418
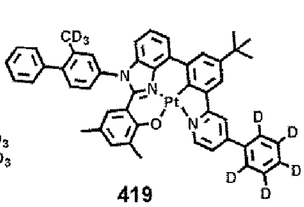
419
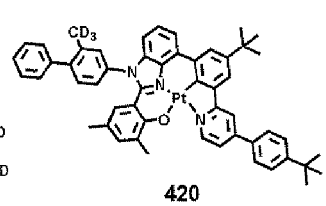
420

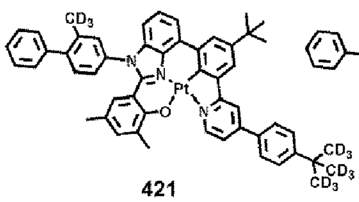
421
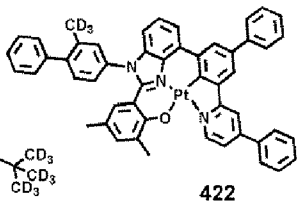
422
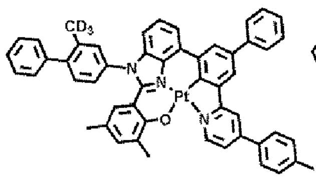
423
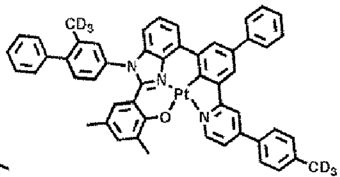
424

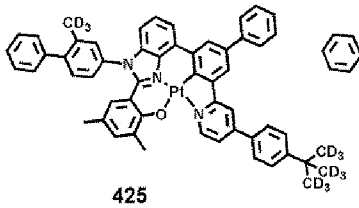
425
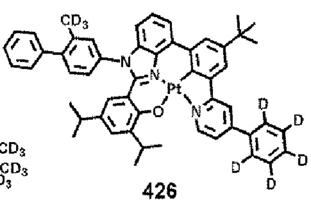
426
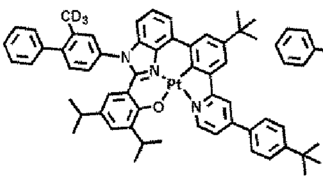
427
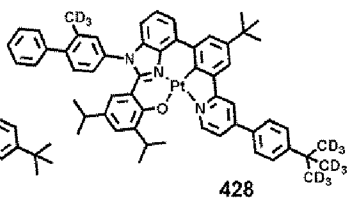
428

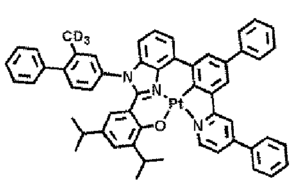
429

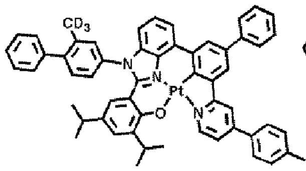
430

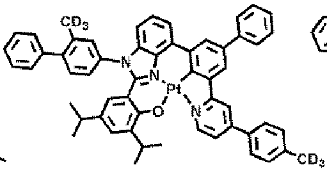
431

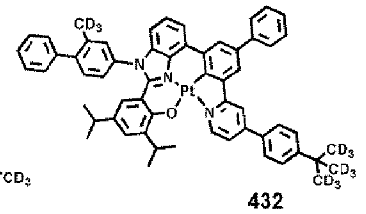
432

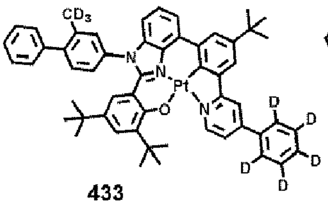
433

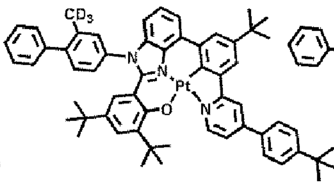
434

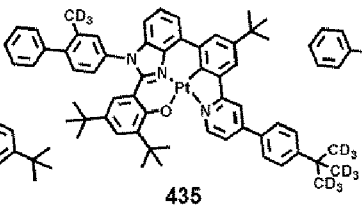
435

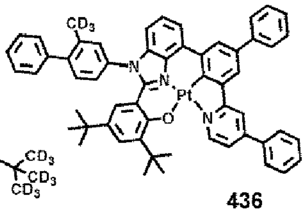
436

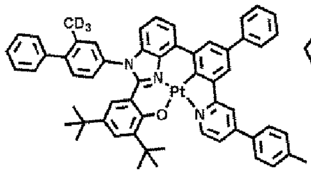
437

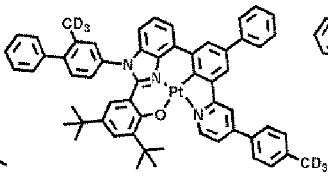
438

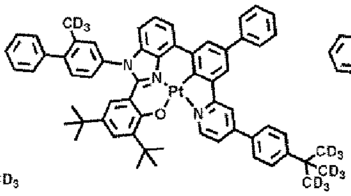
439

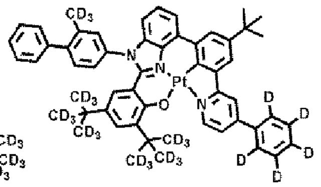
440

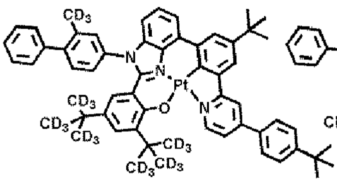
441

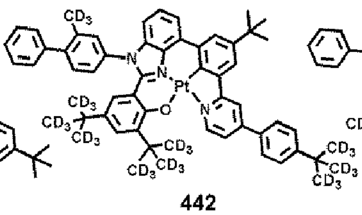
442

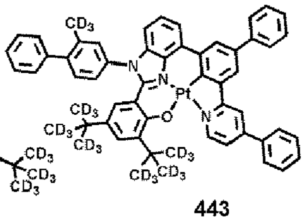
443

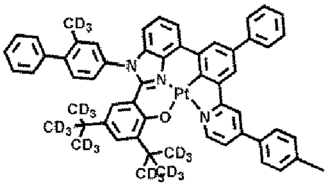
444

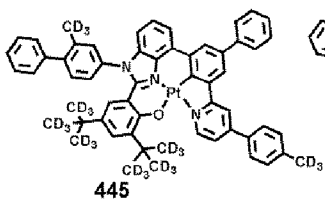
445

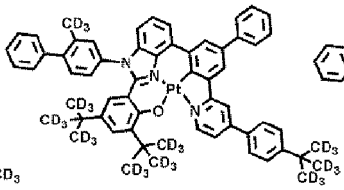
446

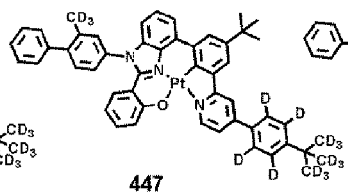
447

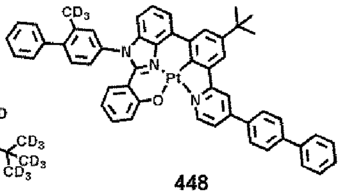
448

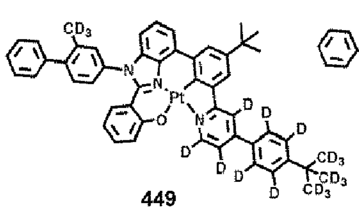
449

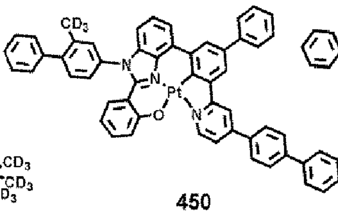
450

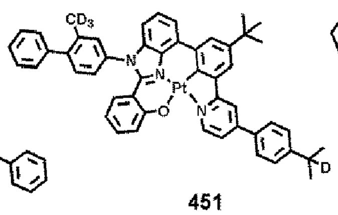
451

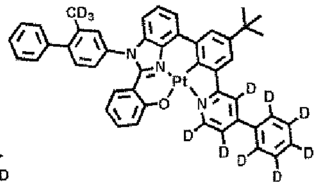
452

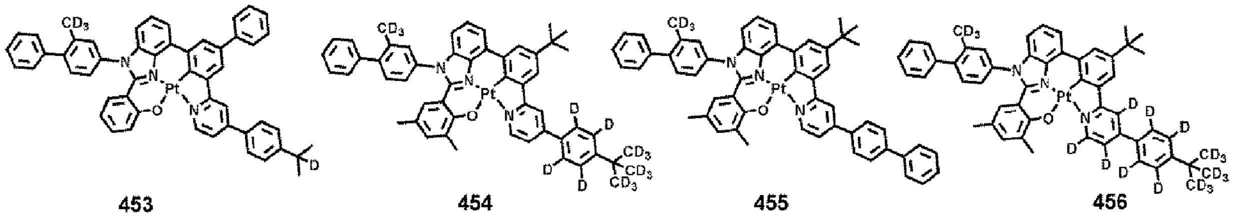

453 454 455 456

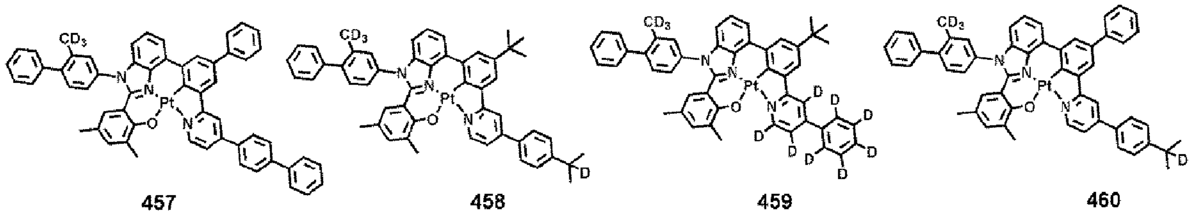

457 458 459 460

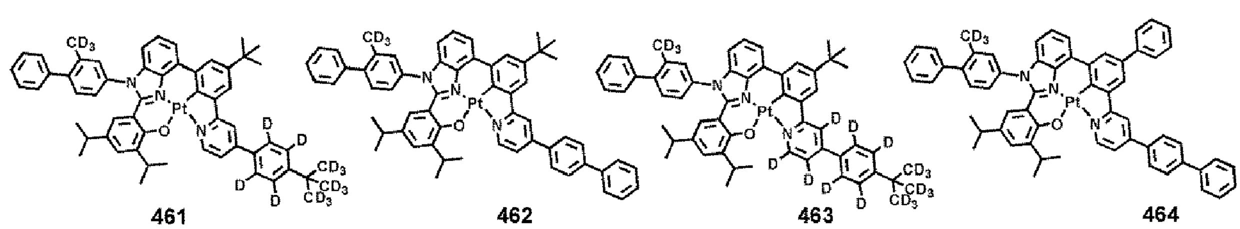

461 462 463 464

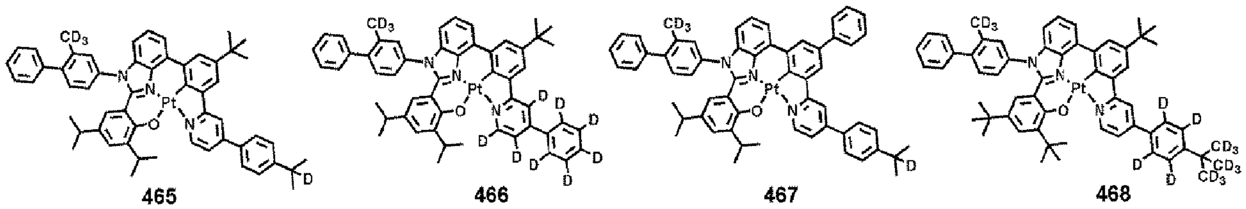

465 466 467 468

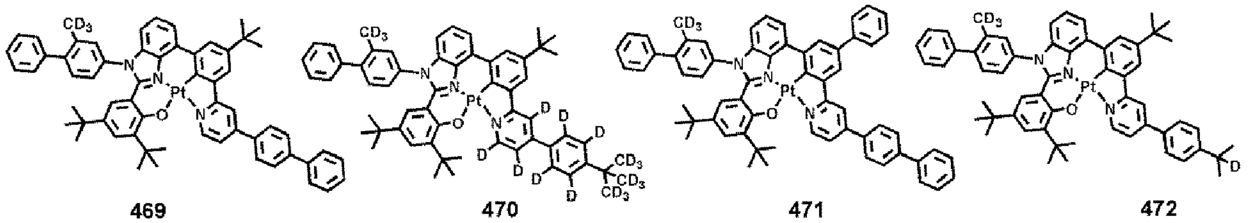

469 470 471 472

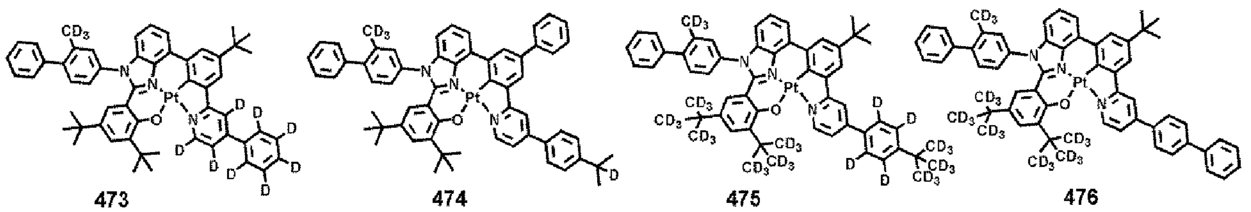

473 474 475 476

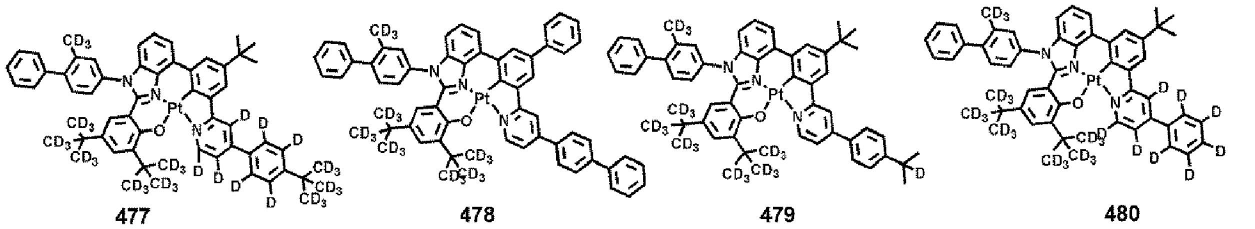

477 478 479 480

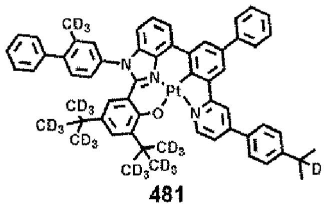
481

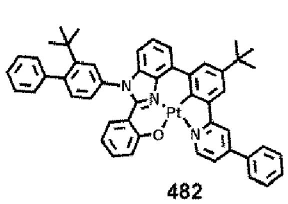
482

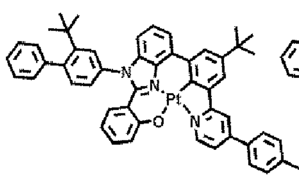
483

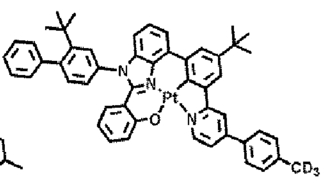
484

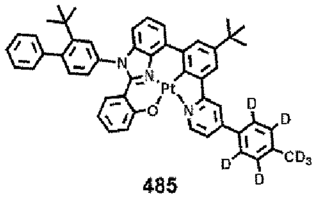
485

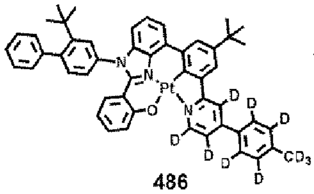
486

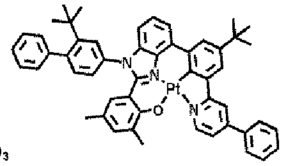
487

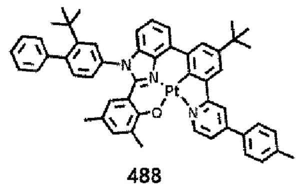
488

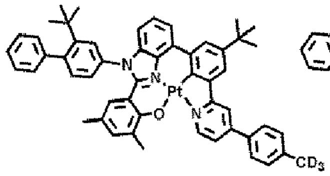
489

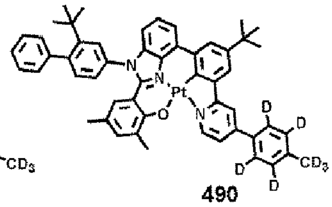
490

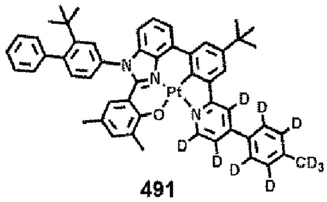
491

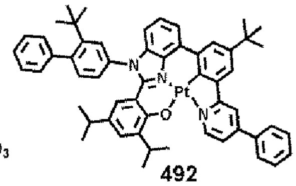
492

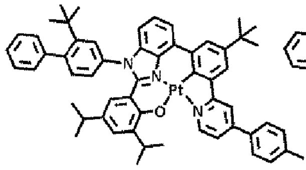
493

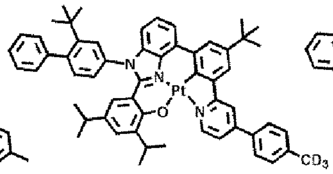
494

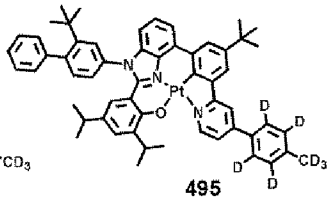
495

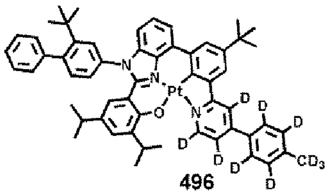
496

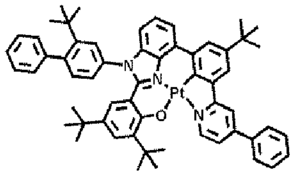
497

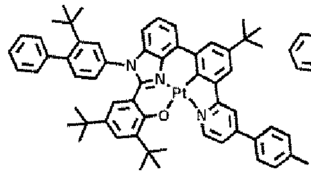
498

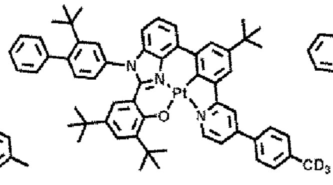
499

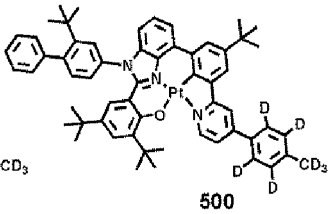
500

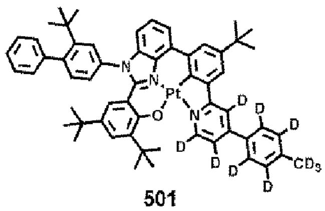
501

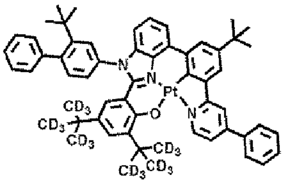
502

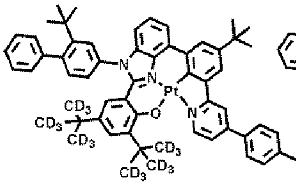
503

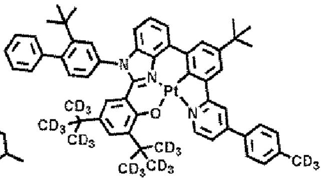
504

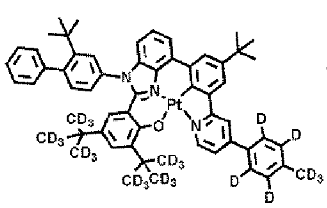
505

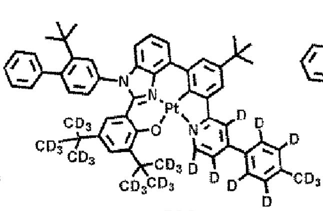
506

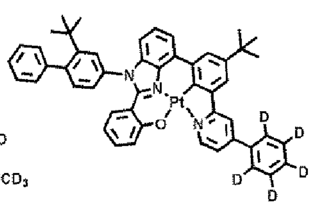
507

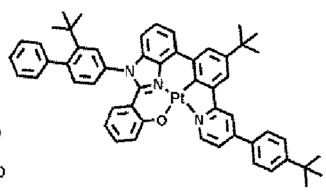
508

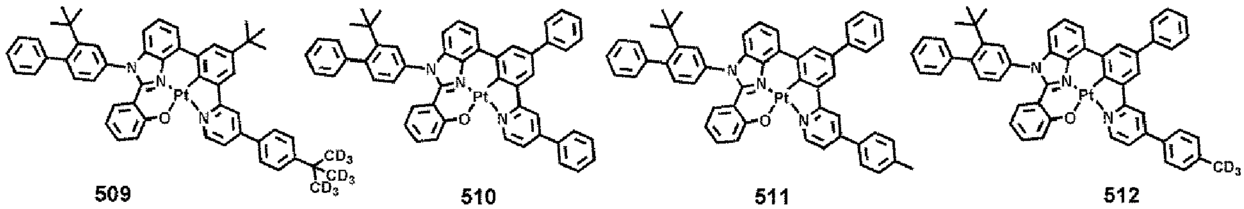

509 510 511 512

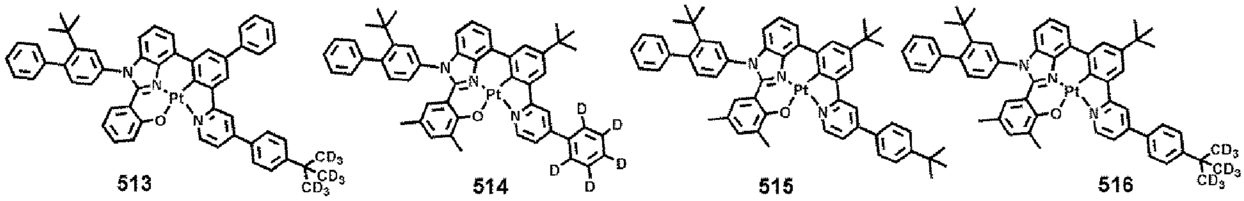

513 514 515 516

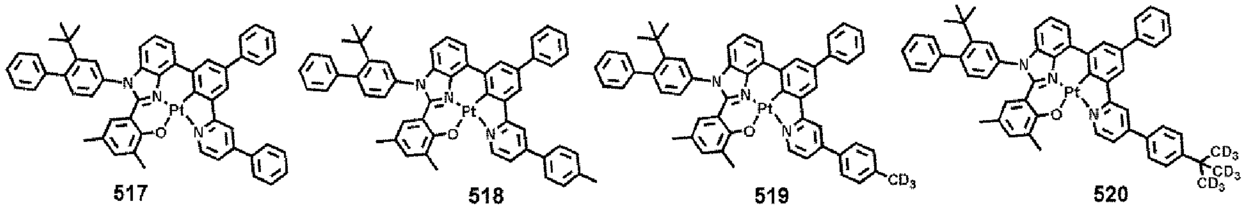

517 518 519 520

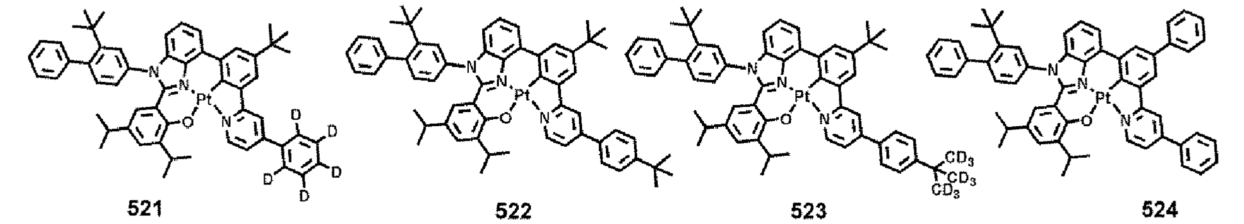

521 522 523 524

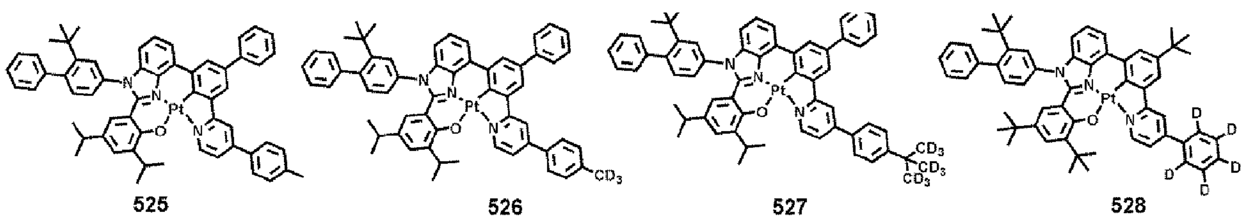

525 526 527 528

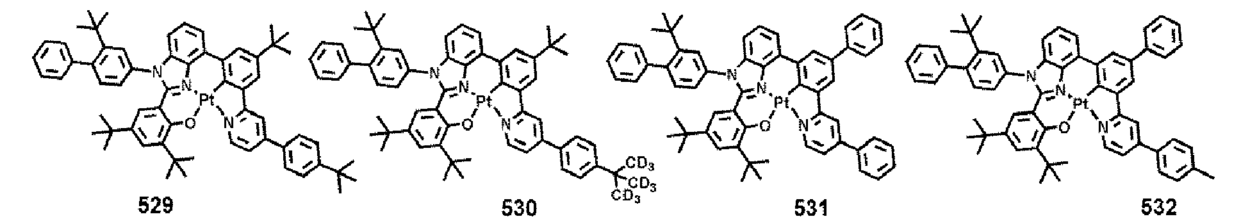

529 530 531 532

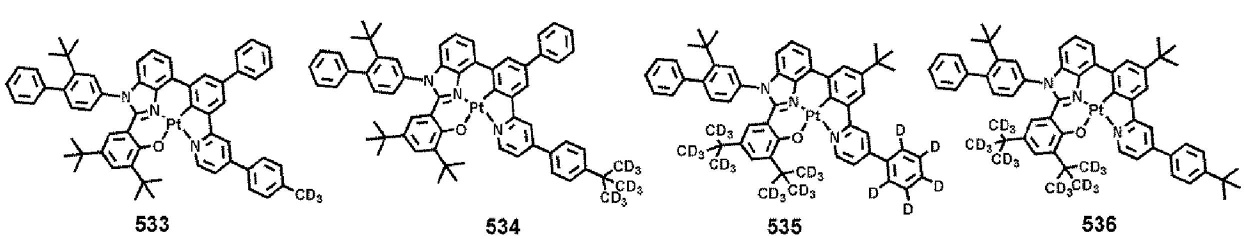

533 534 535 536

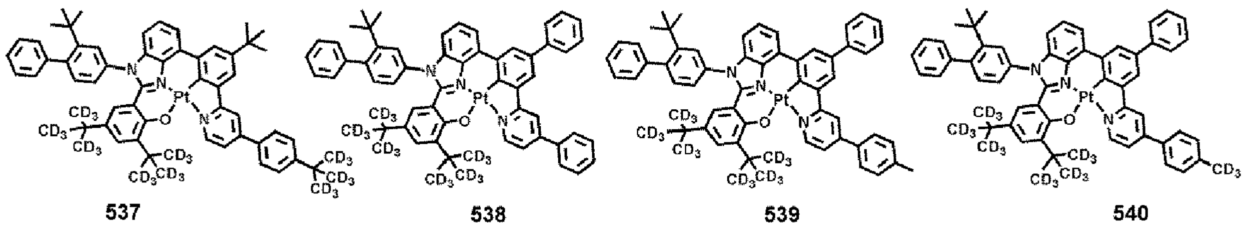

537 538 539 540

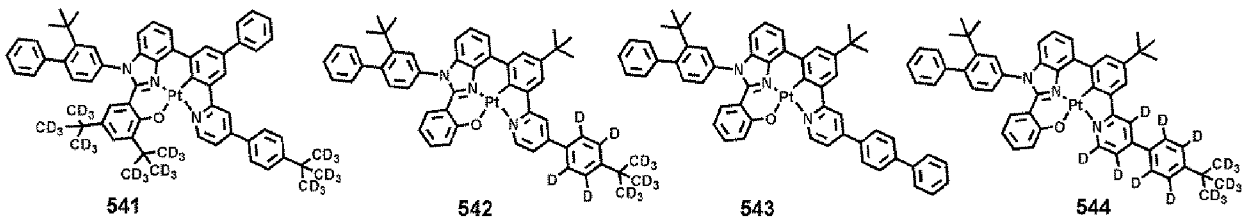

541 542 543 544

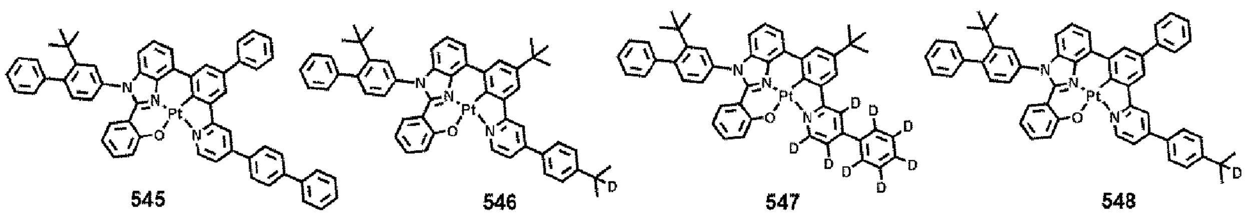

545 546 547 548

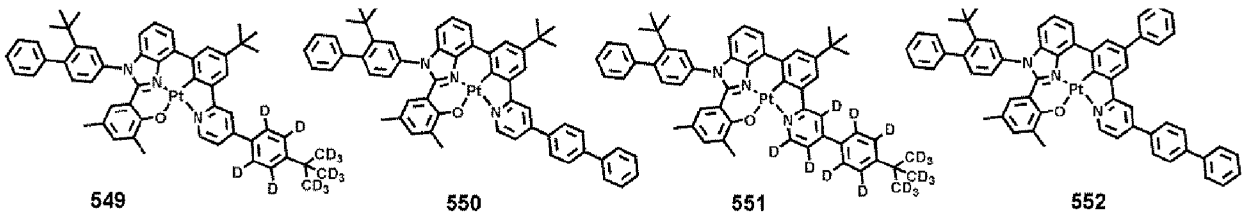

549 550 551 552

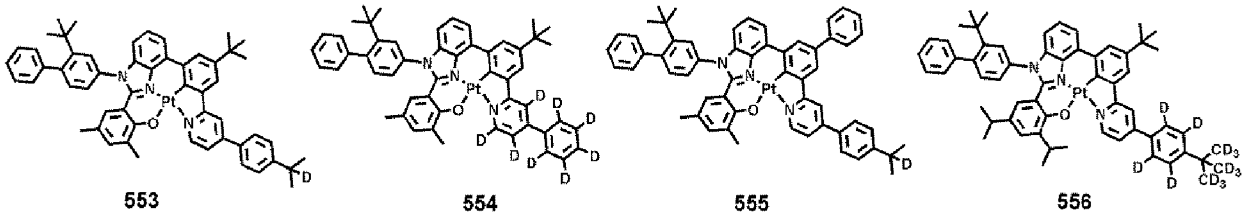

553 554 555 556

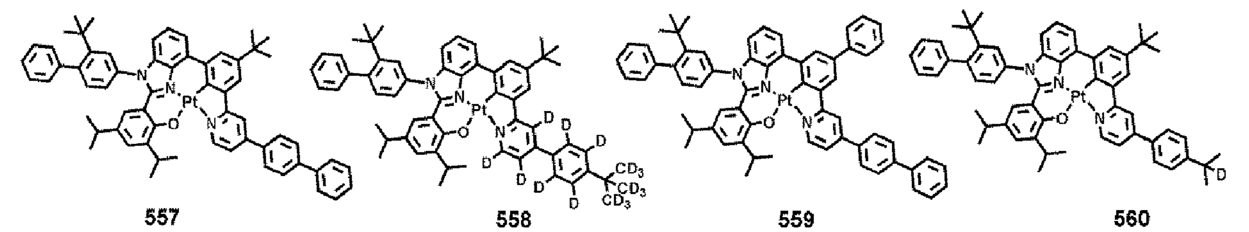

557 558 559 560

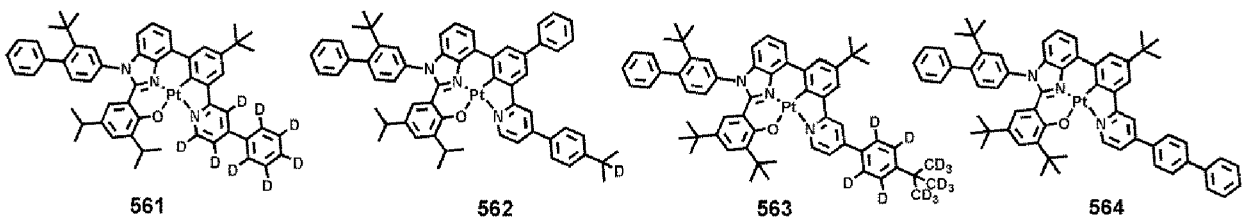

561 562 563 564

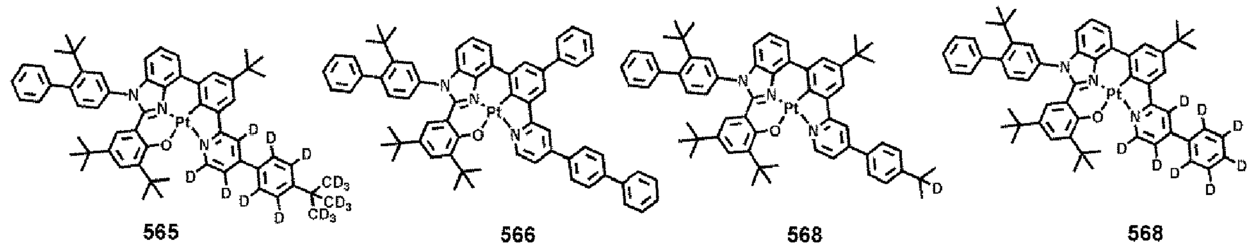

565 566 568 568

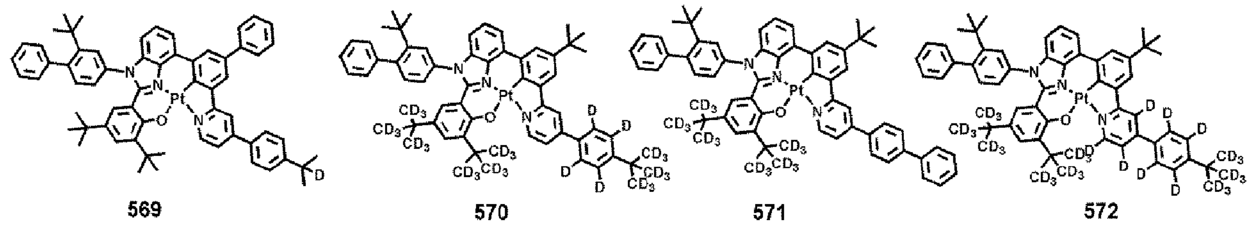

569 570 571 572

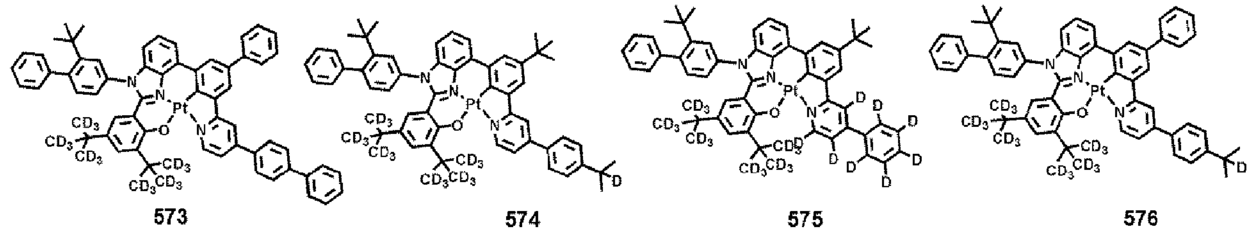

573 574 575 576

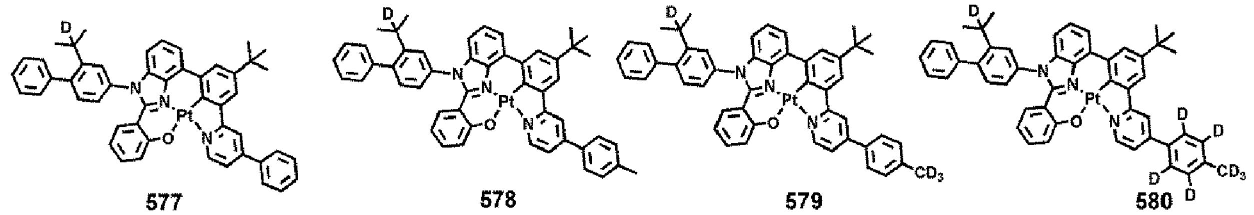

577 578 579 580

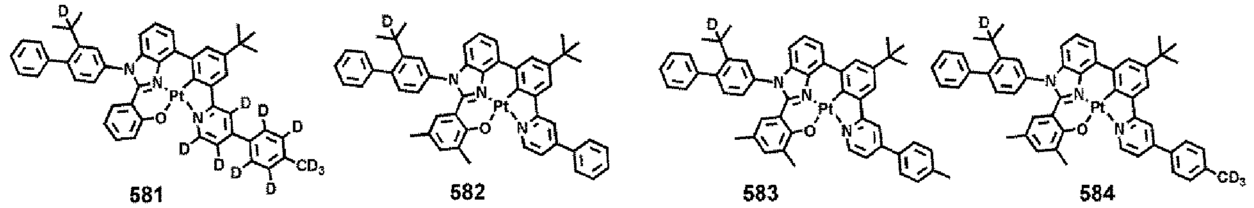

581 582 583 584

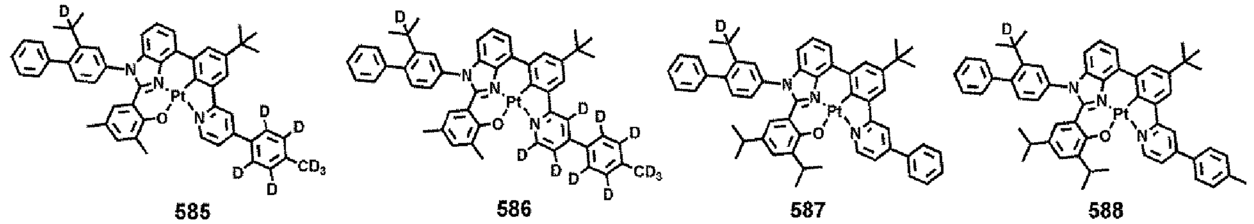

585 586 587 588

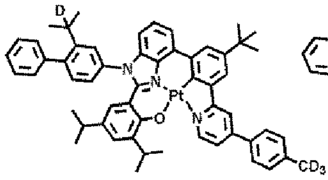
589
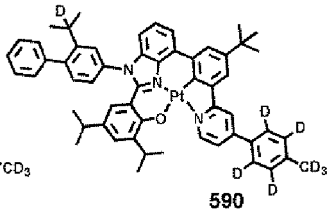
590
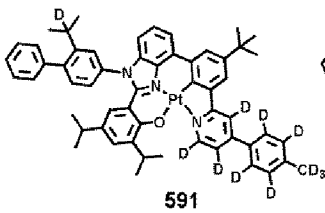
591
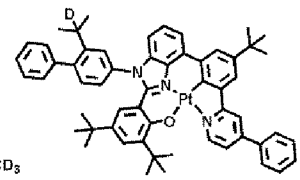
592

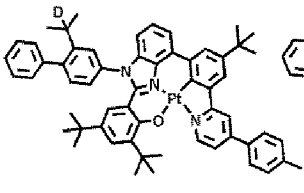
593
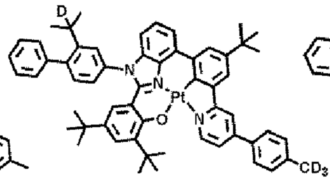
594
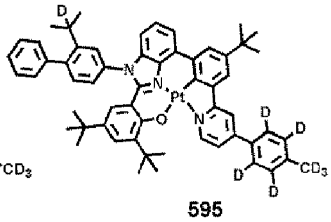
595
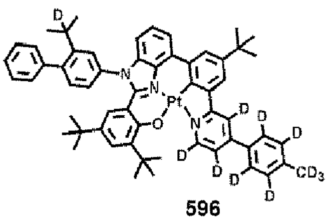
596

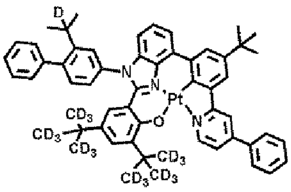
597
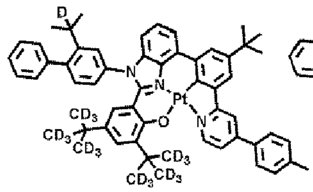
598
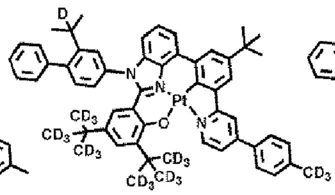
599
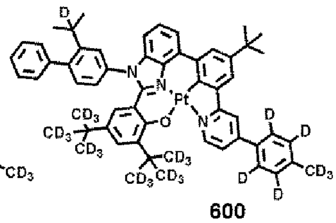
600

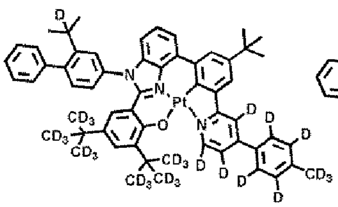
601
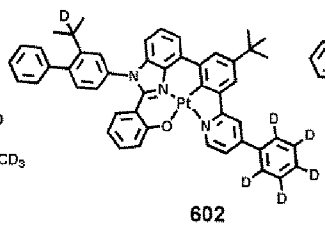
602
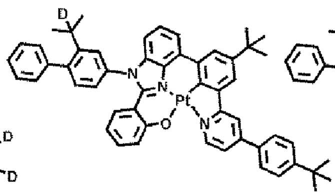
603
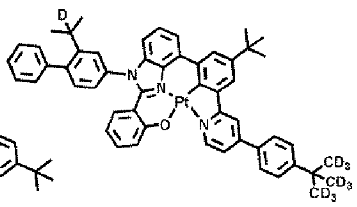
604

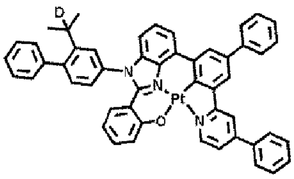
605
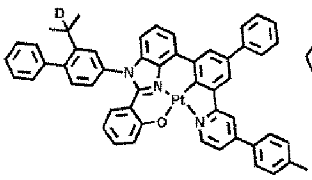
606
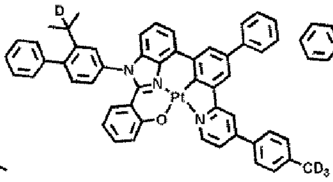
607
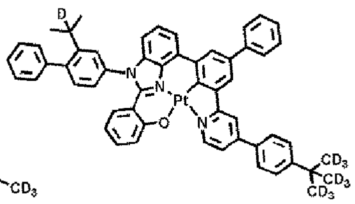
608

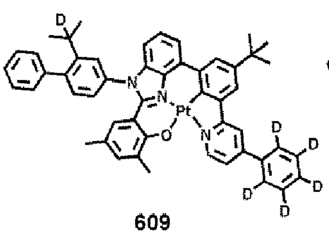
609
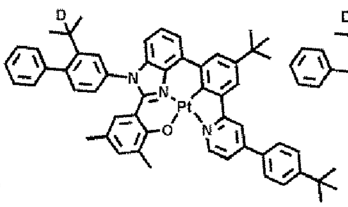
610
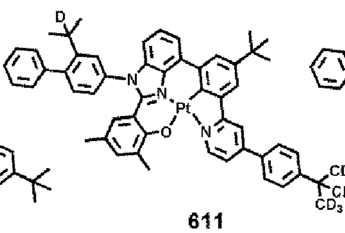
611
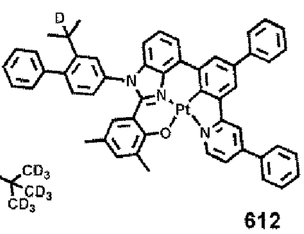
612

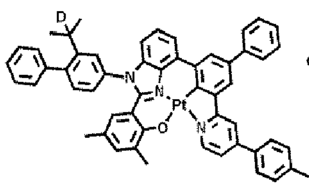
613

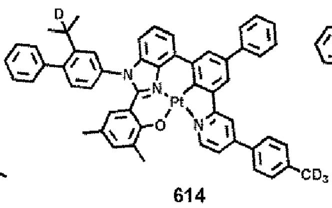
614

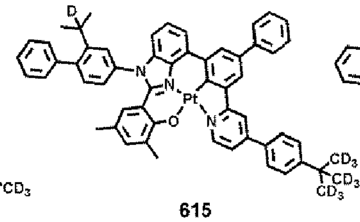
615

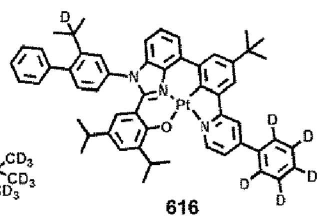
616

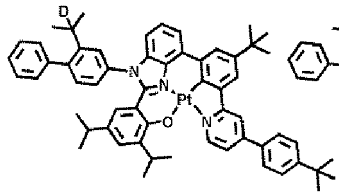
617

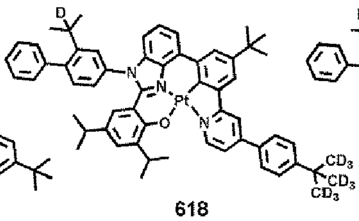
618

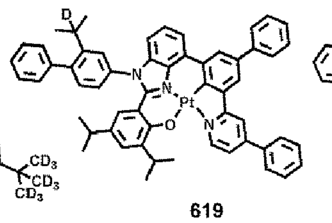
619

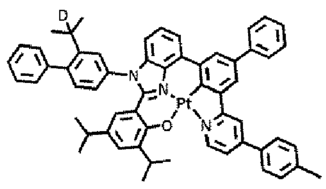
620

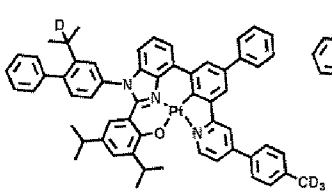
621

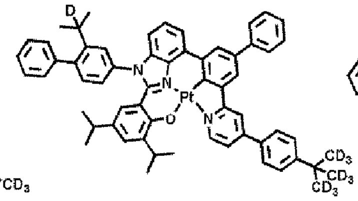
622

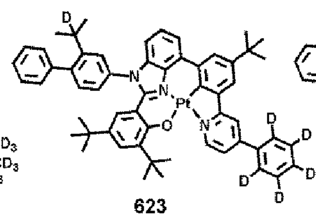
623

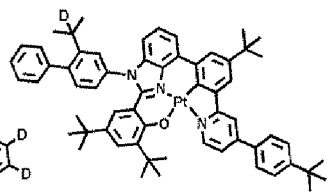
624

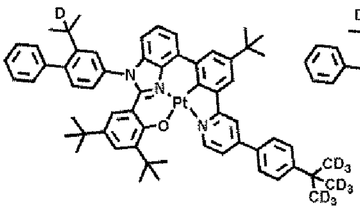
625

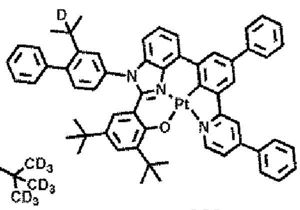
626

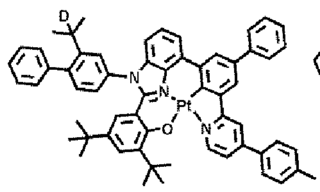
627

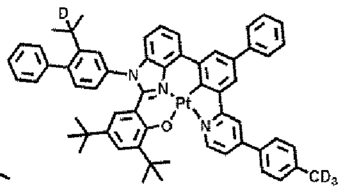
628

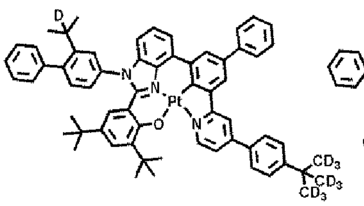
629

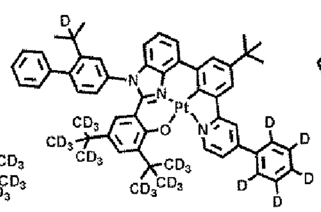
630

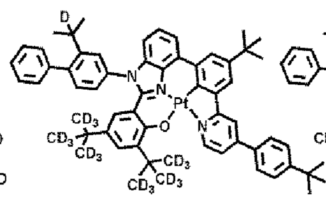
631

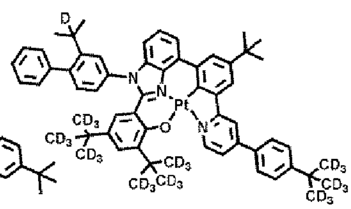
632

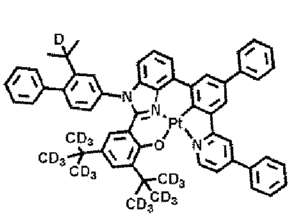
633

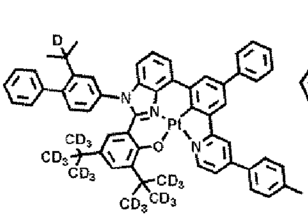
634

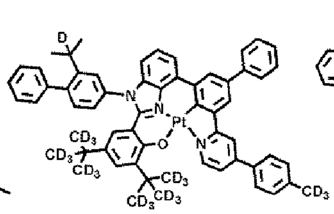
635

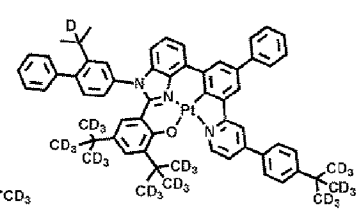
636

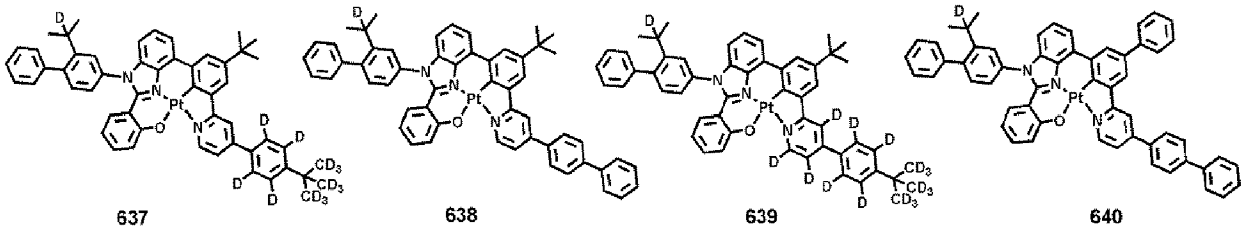

637 638 639 640

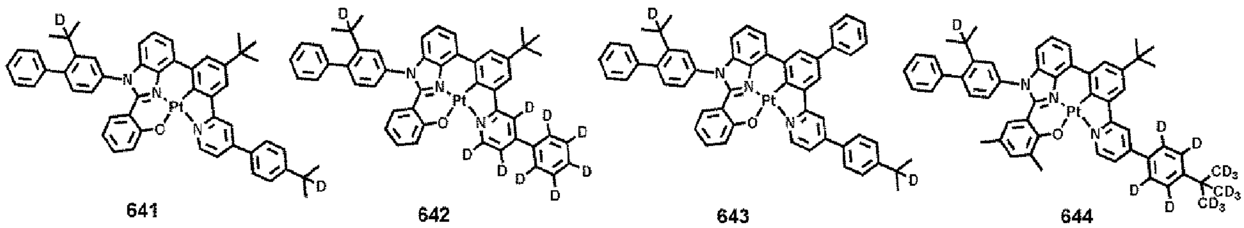

641 642 643 644

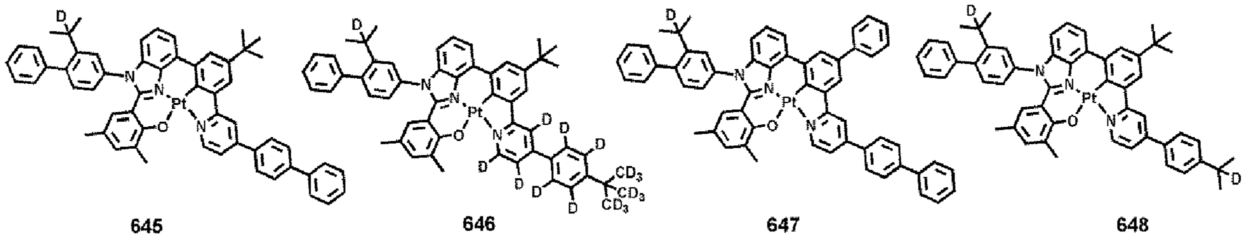

645 646 647 648

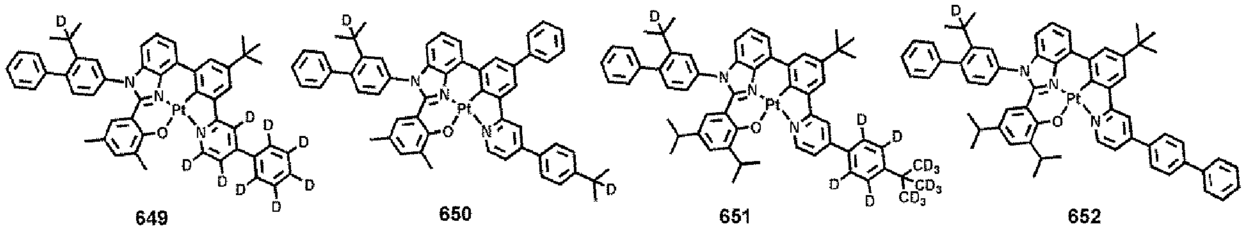

649 650 651 652

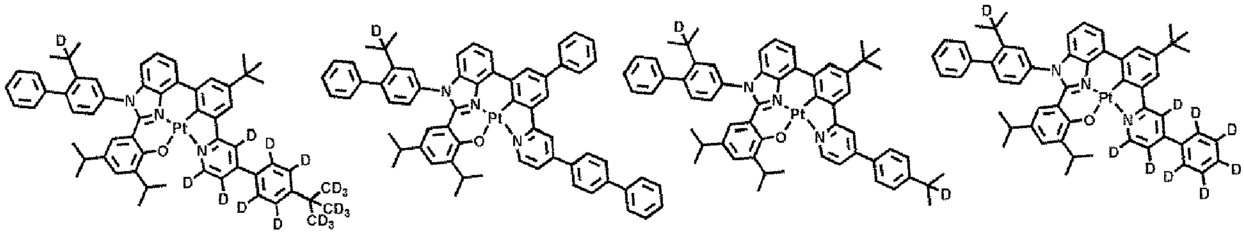

653 654 655 656

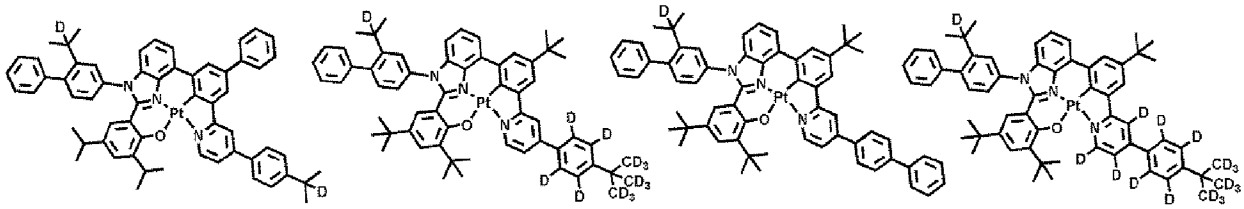

657 658 659 660

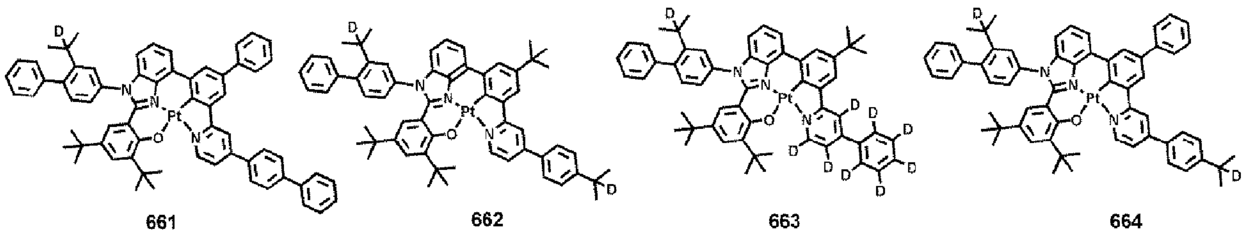

661 662 663 664

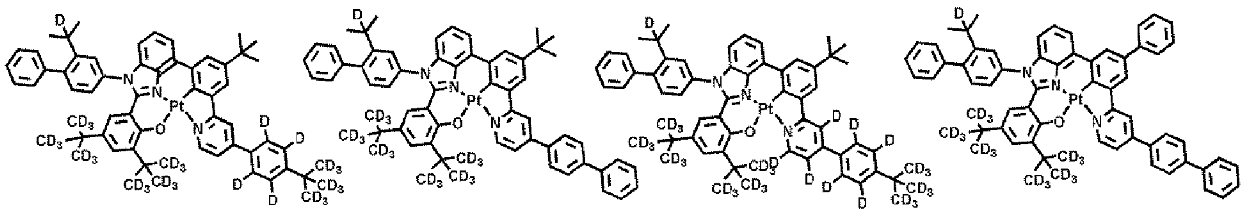

665 666 667 668

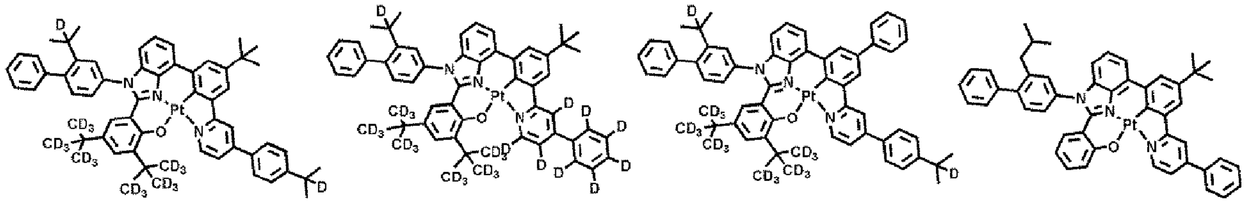

669 670 671 672

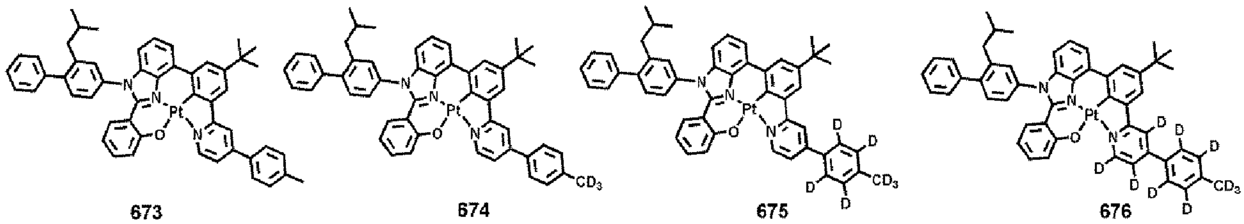

673 674 675 676

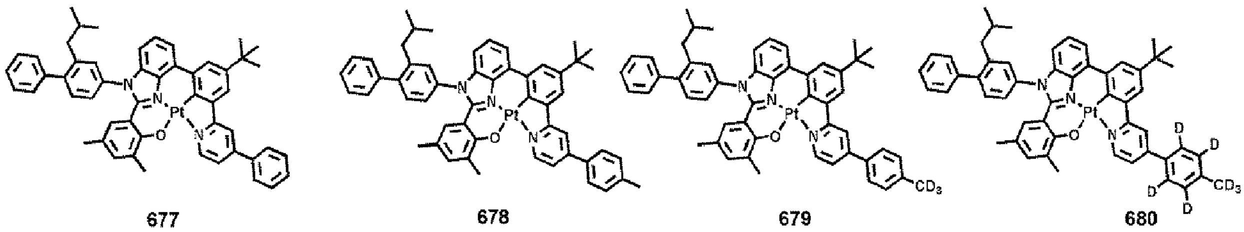

677 678 679 680

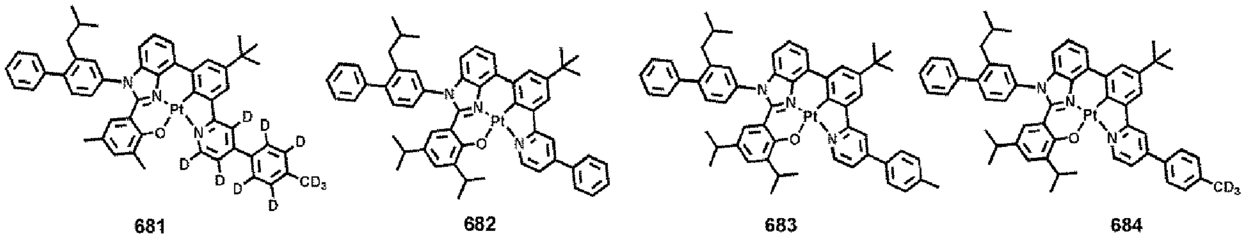

681 682 683 684

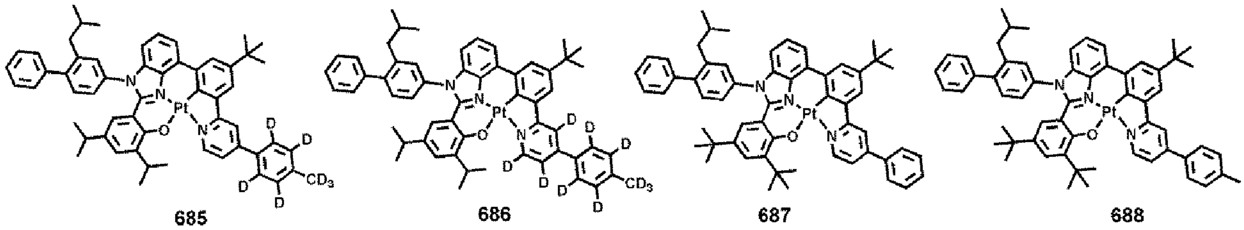

685 686 687 688

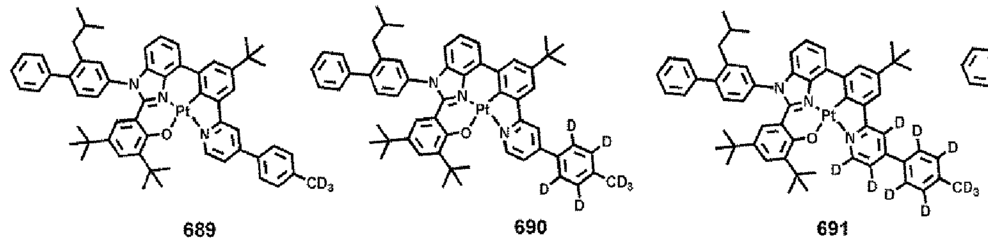

689 690 691 692

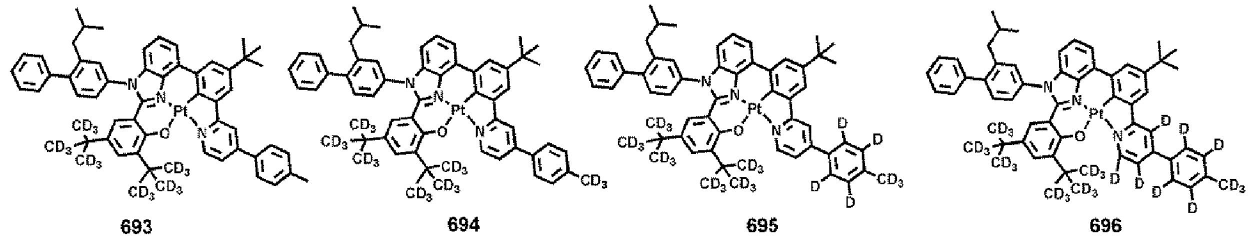

693 694 695 696

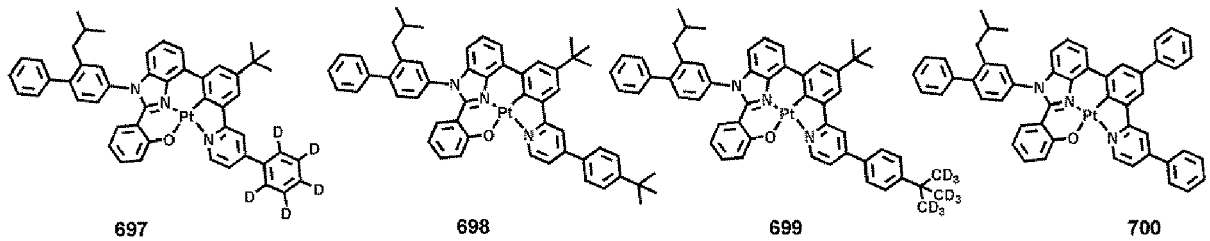

697 698 699 700

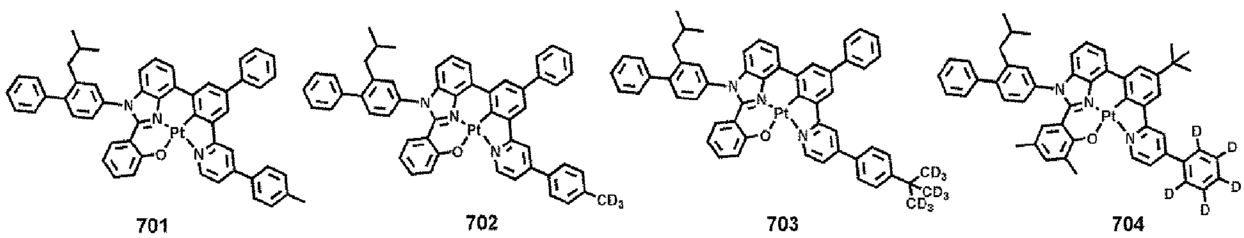

701 702 703 704

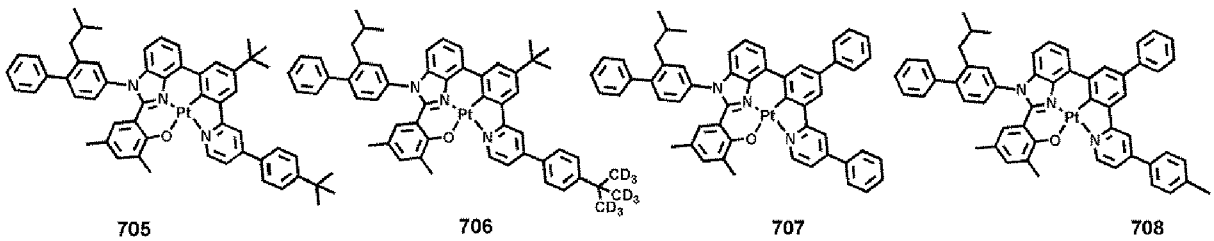

705 706 707 708

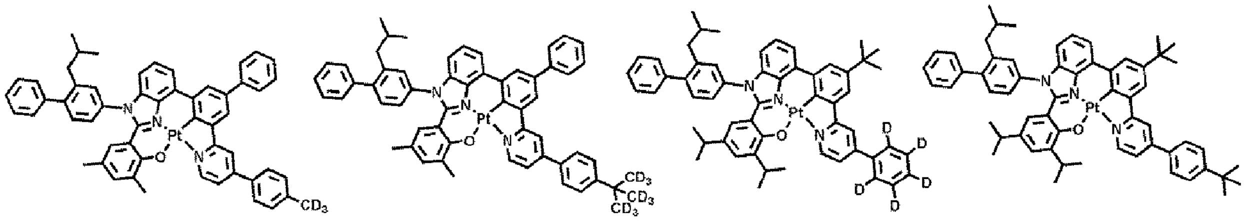

709 710 711 712

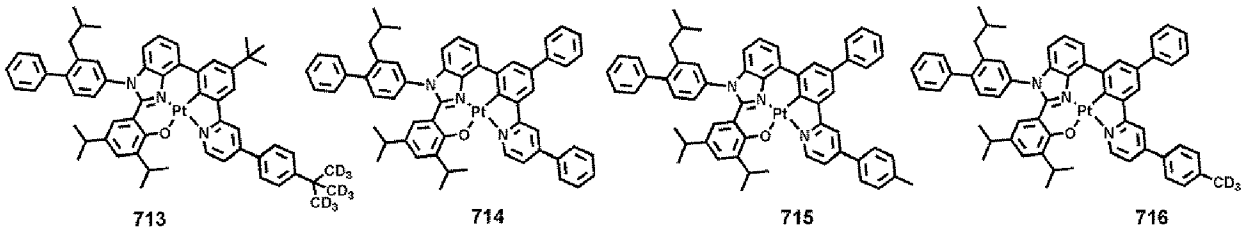

713 714 715 716

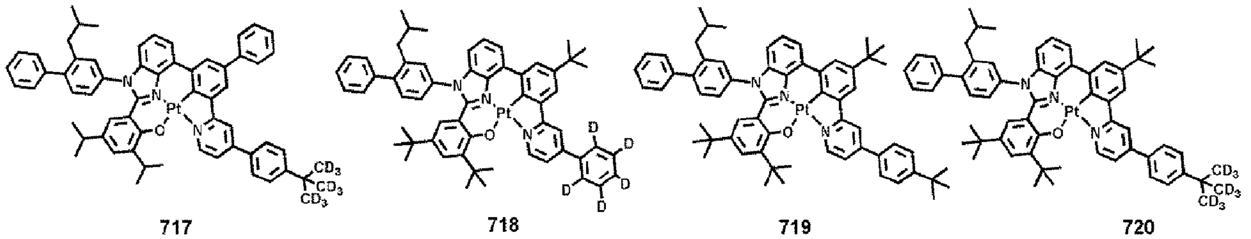

717 718 719 720

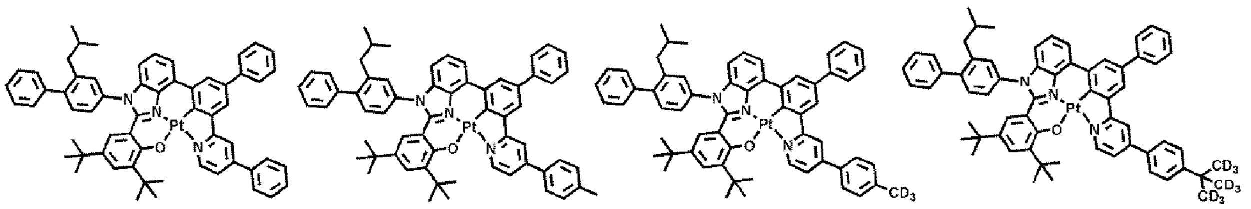

721 722 723 724

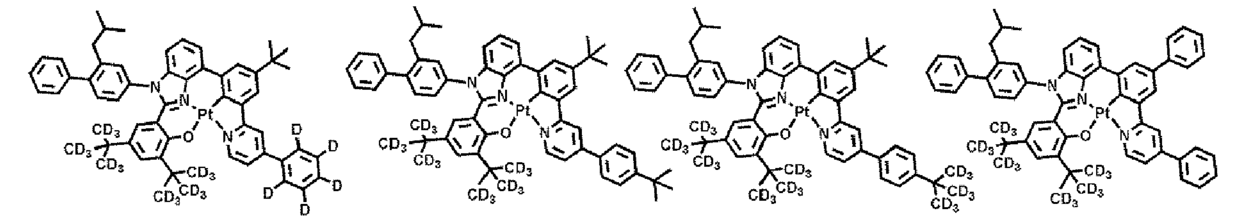

725 726 727 728

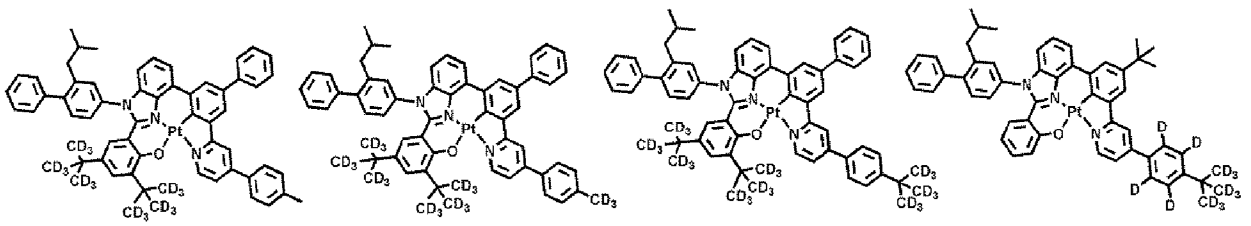

729 730 731 732

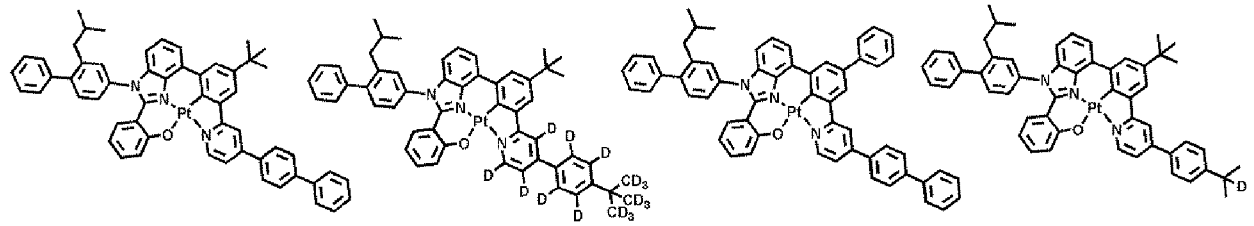

733 734 735 736

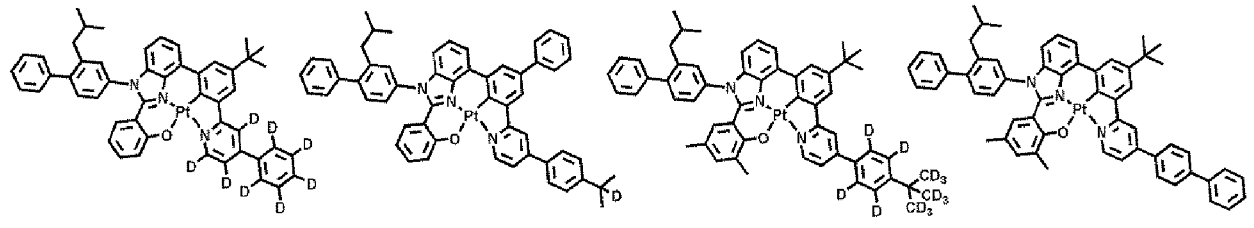

737 738 738 740

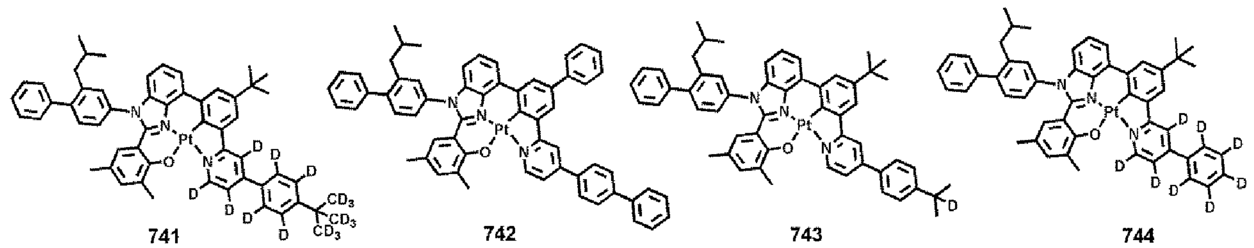

741 742 743 744

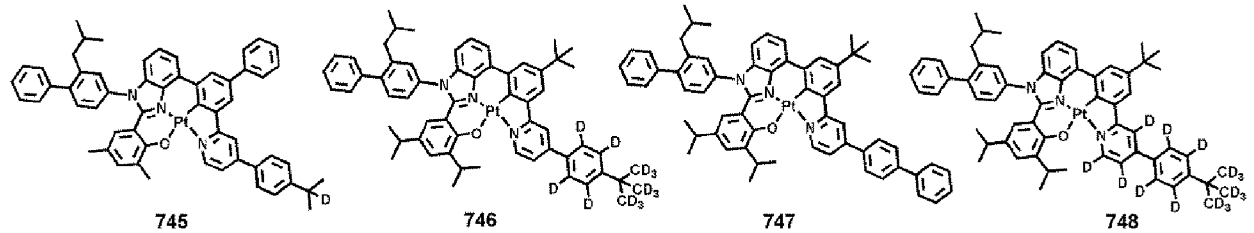

745 746 747 748

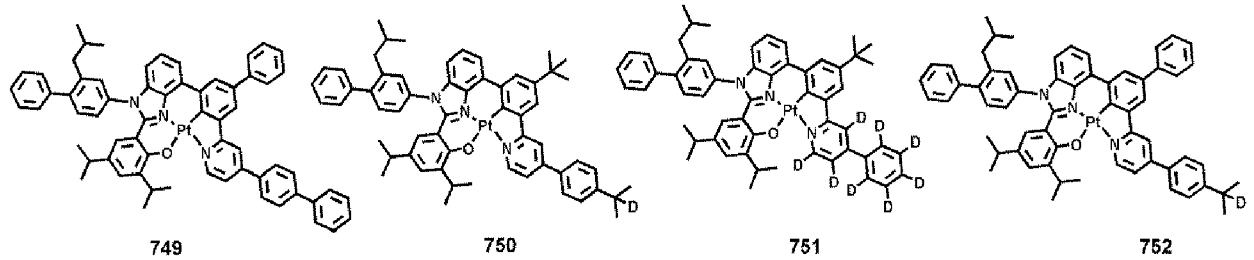

749 750 751 752

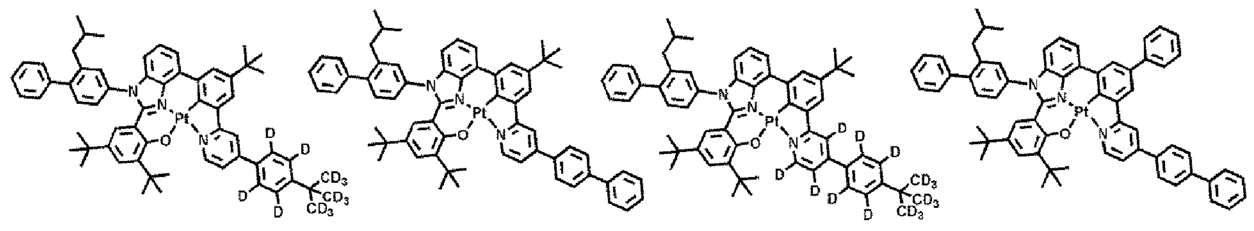

753 754 755 756

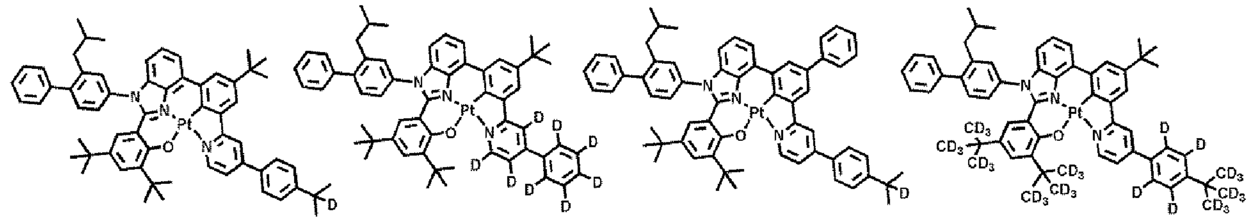

757 758 759 760

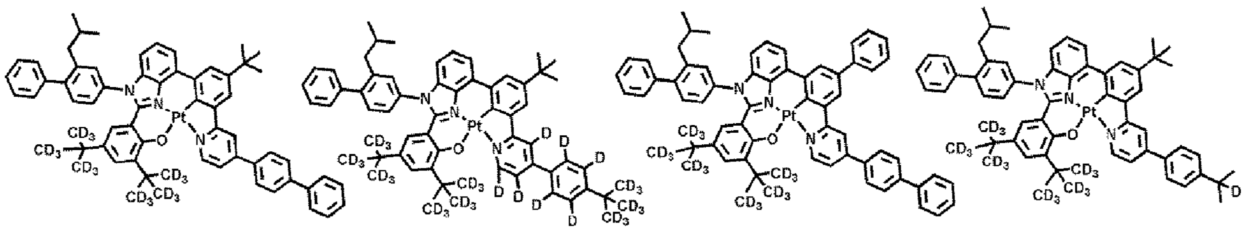

761 762 763 764

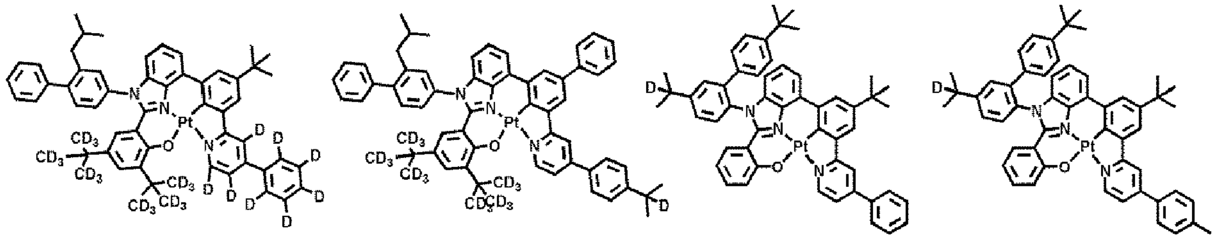

765 766 767 768

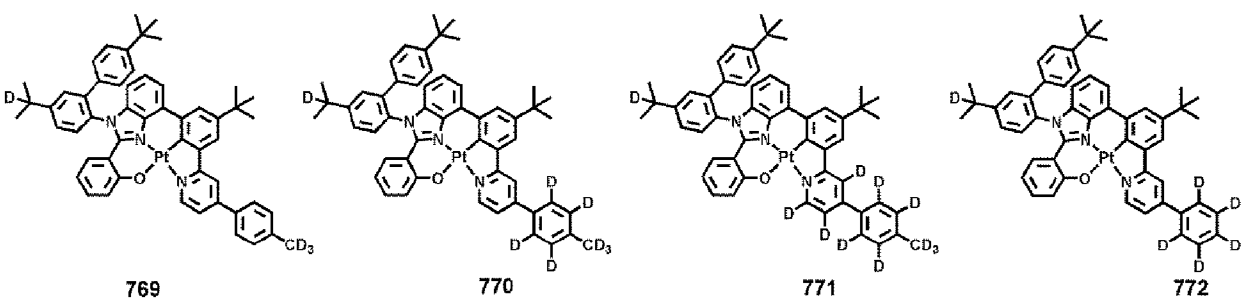

769 770 771 772

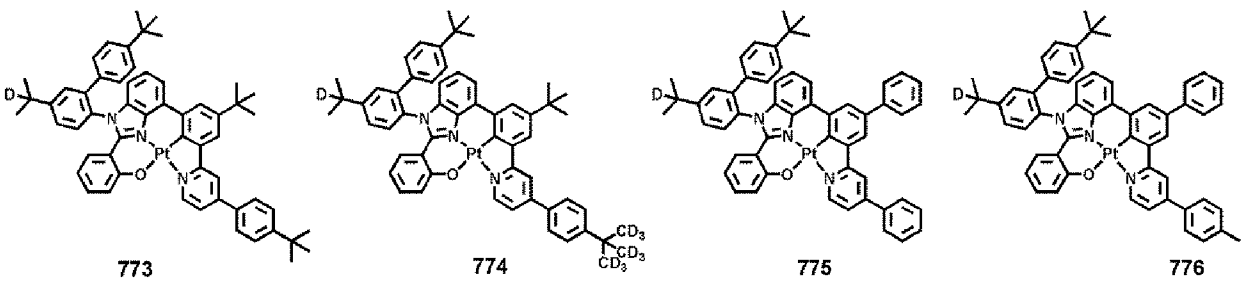

773 774 775 776

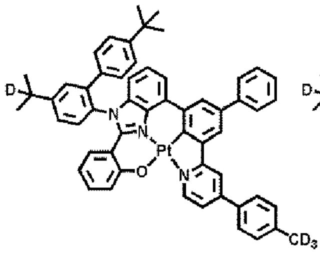
777

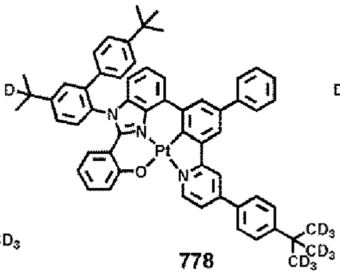
778

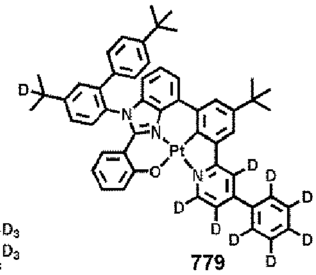
779

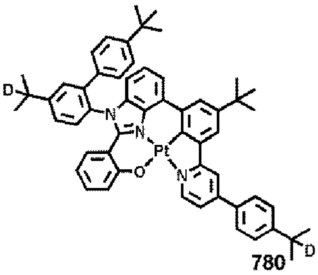
780

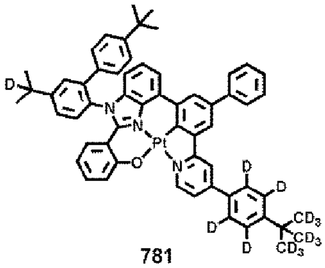
781

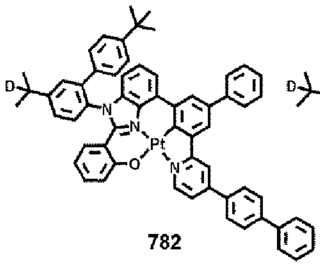
782

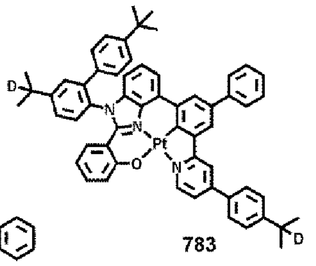
783

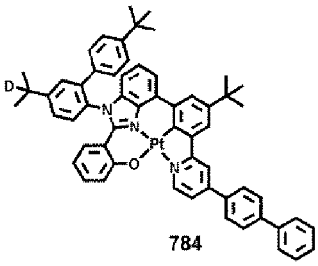
784

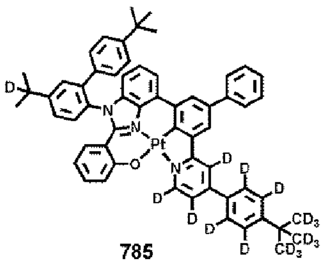
785

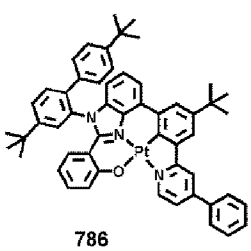
786

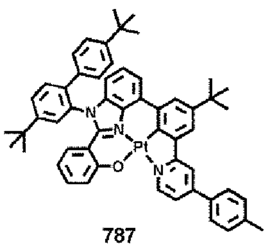
787

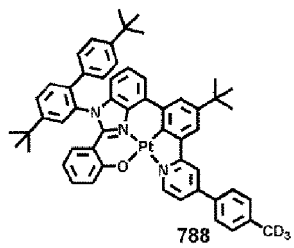
788

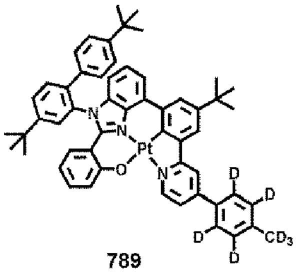
789

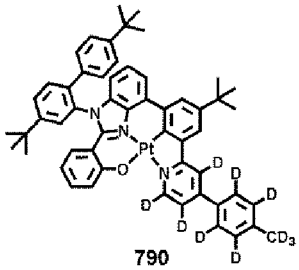
790

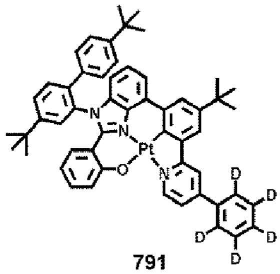
791

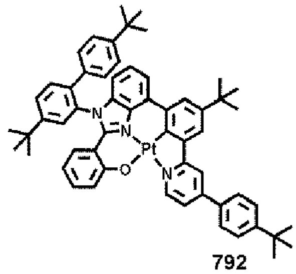
792

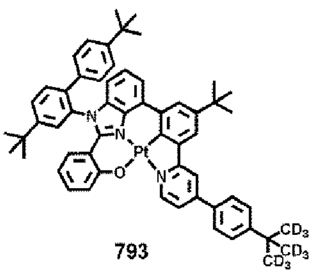
793

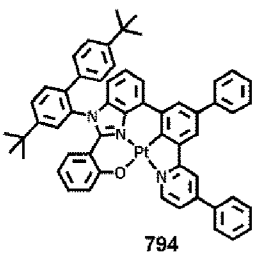
794

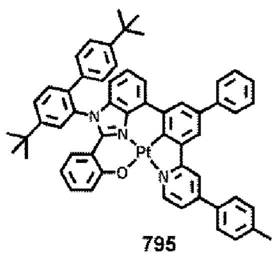
795

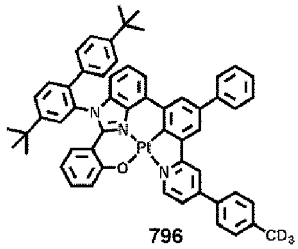
796

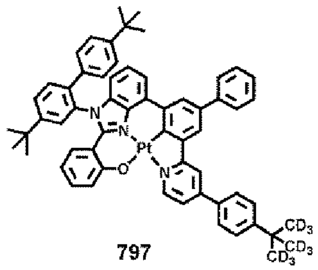
797

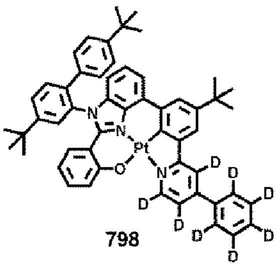
798

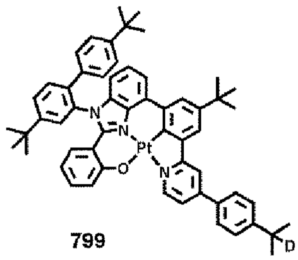
799

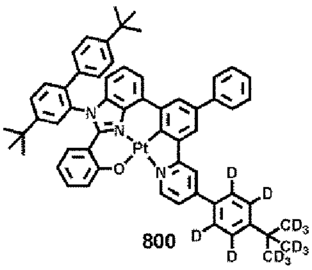
800

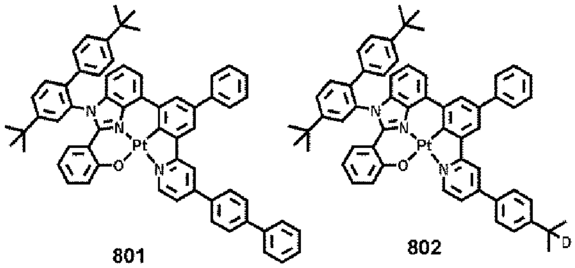
801

802

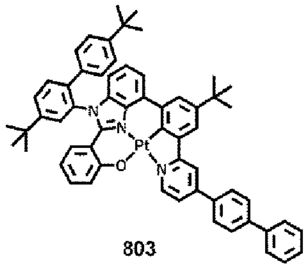
803

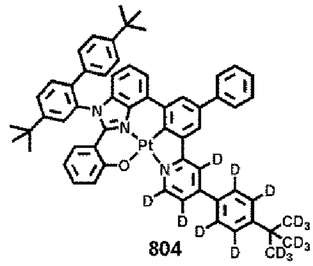
804

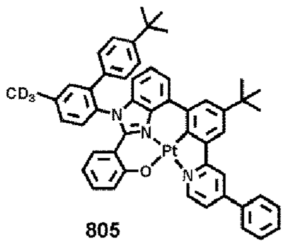
805

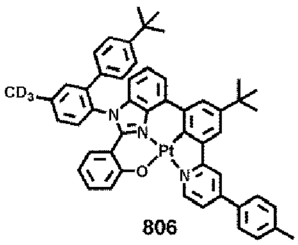
806

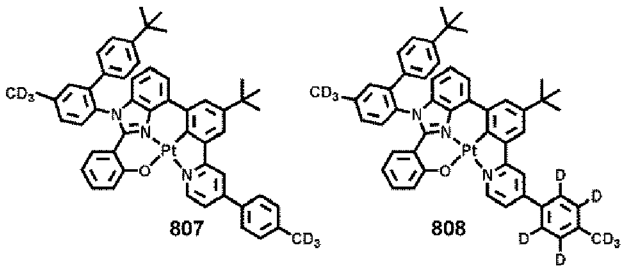
807

808

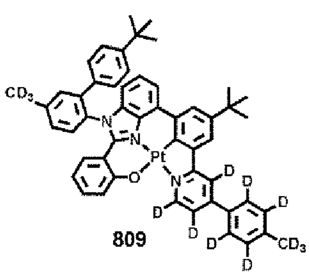
809

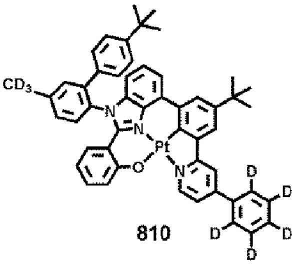
810

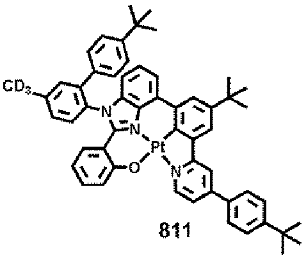
811

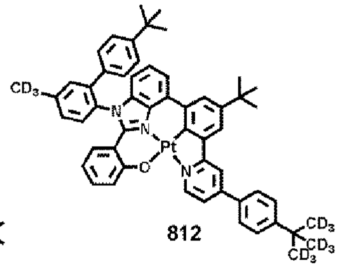
812

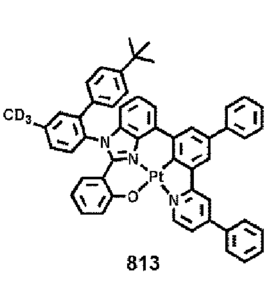
813

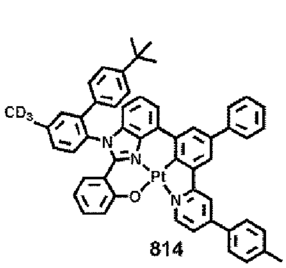
814

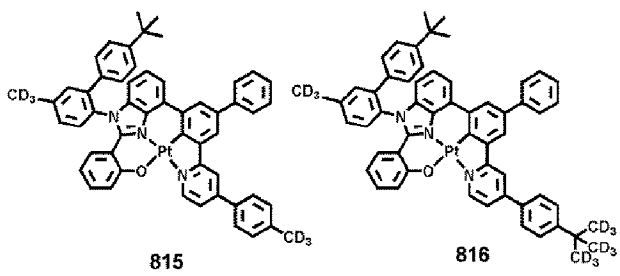
815

816

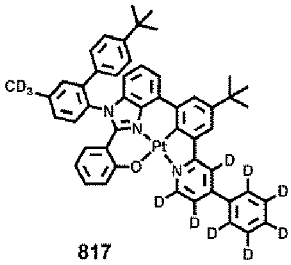
817

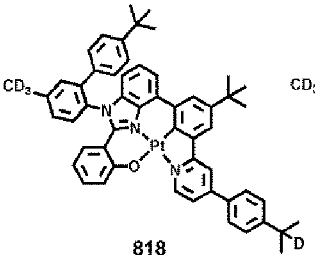
818

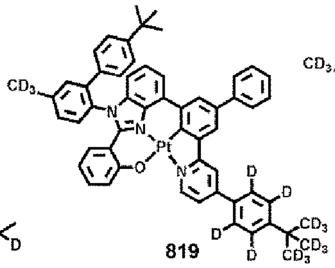
819

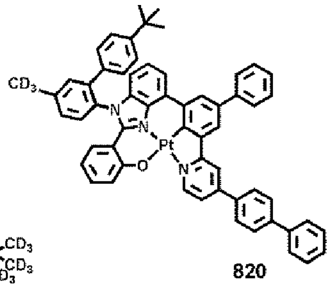
820

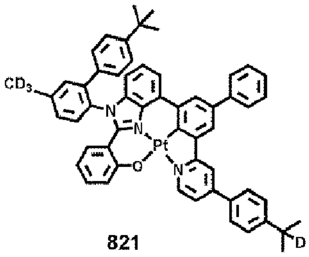
821

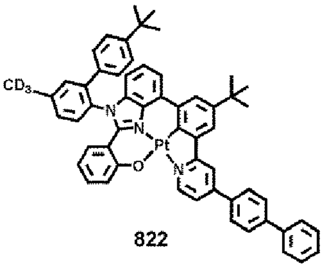
822

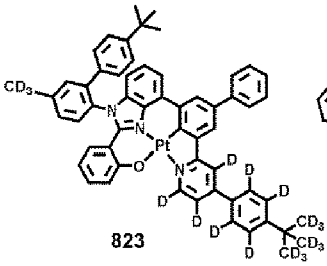
823

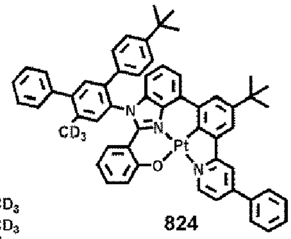
824

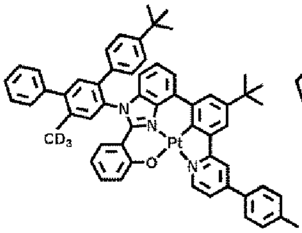
825

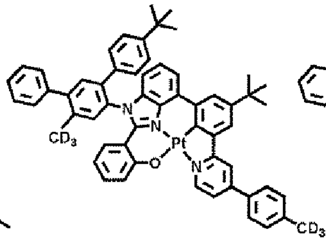
826

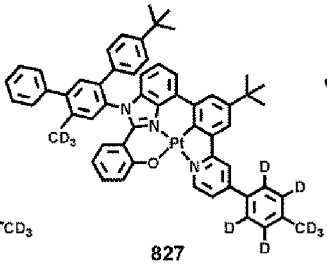
827

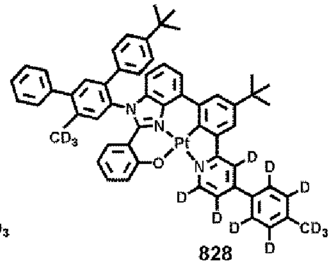
828

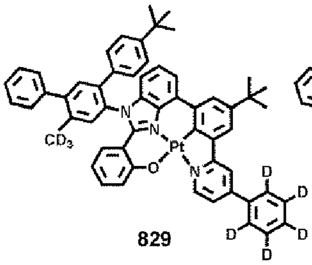
829

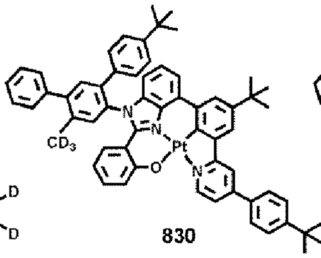
830

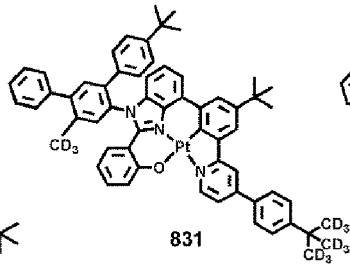
831

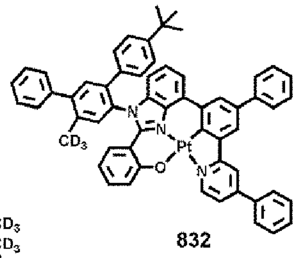
832

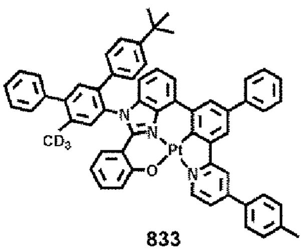
833

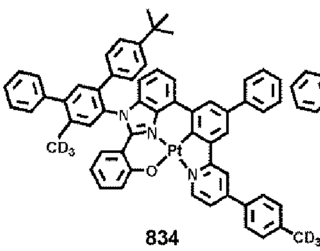
834

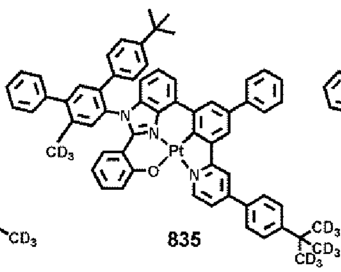
835

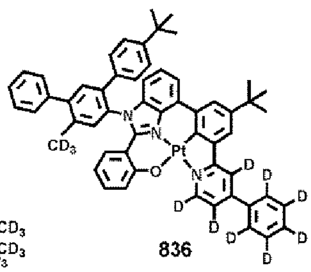
836

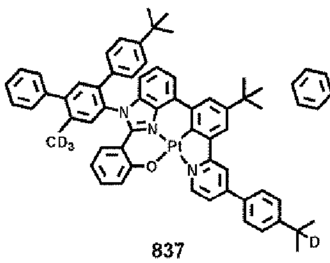
837

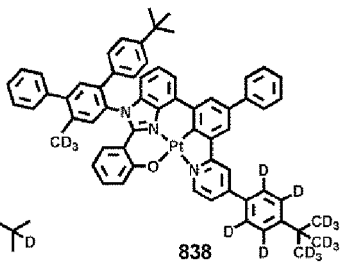
838

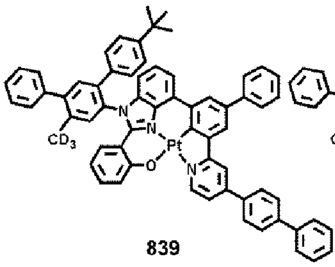
839

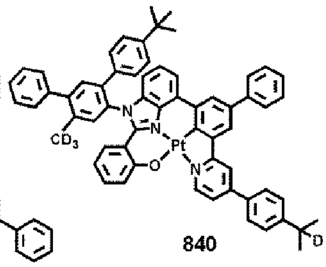
840

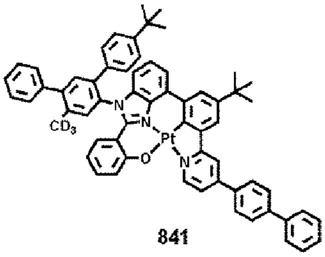
841

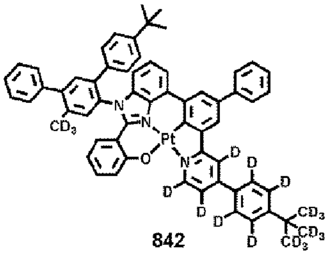
842

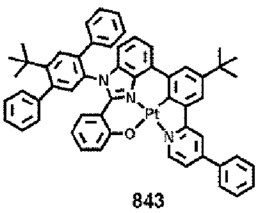
843

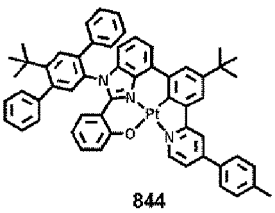
844

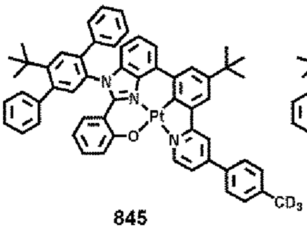
845

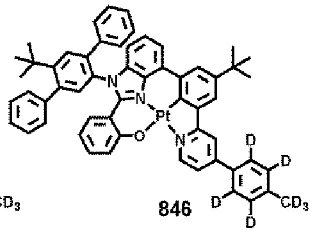
846

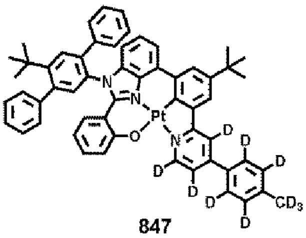
847

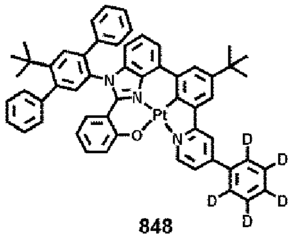
848

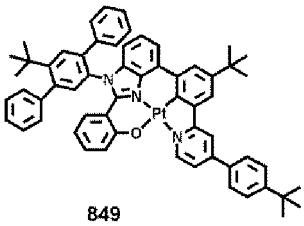
849

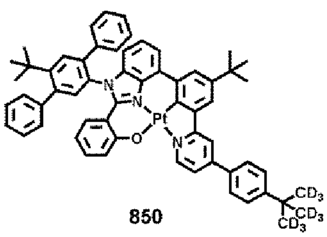
850

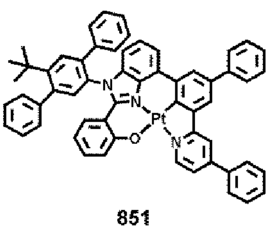
851

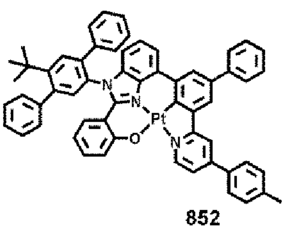
852

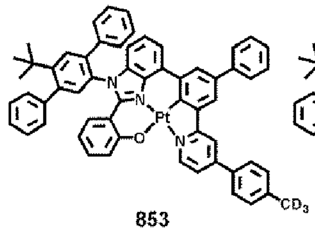
853

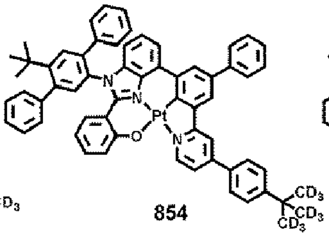
854

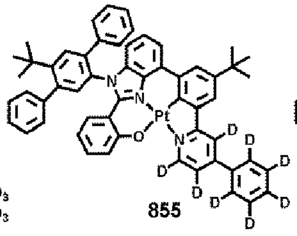
855

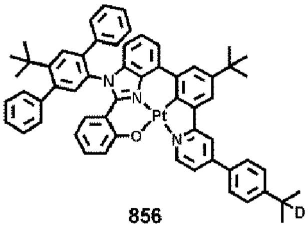
856

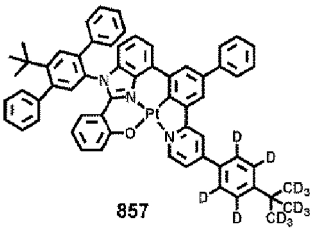
857

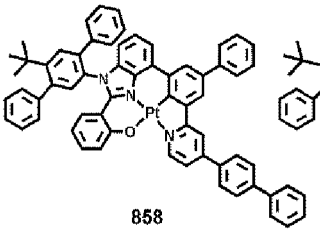
858

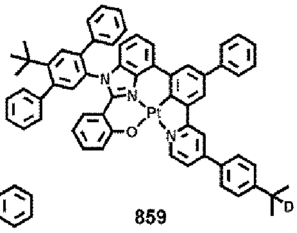
859

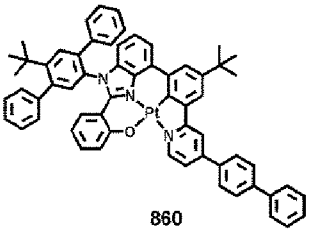
860

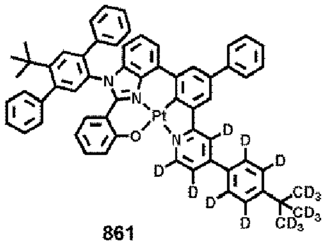
861

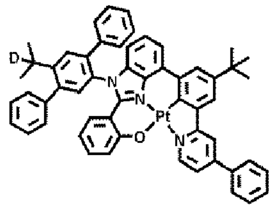
862

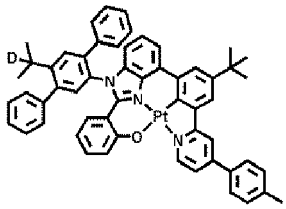
863

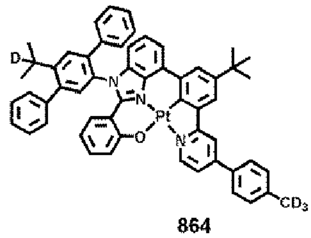
864

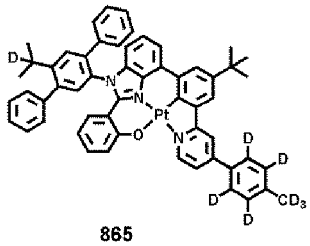
865

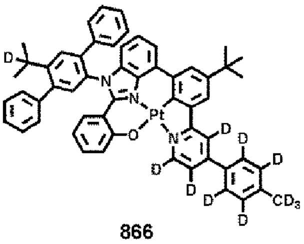
866

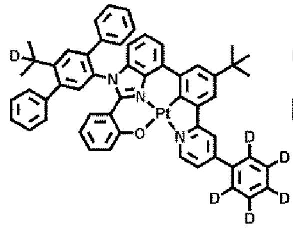
867

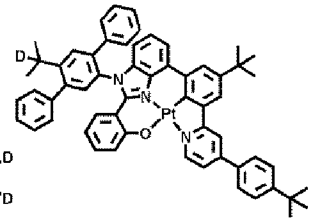
868

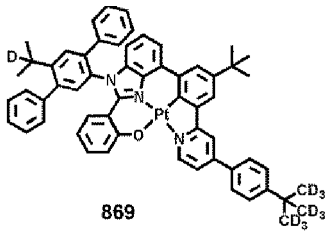
869

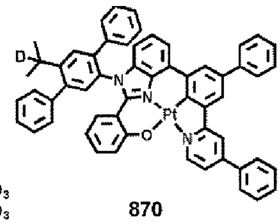
870

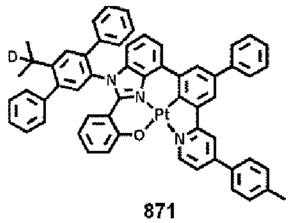
871

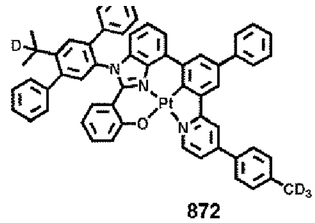
872

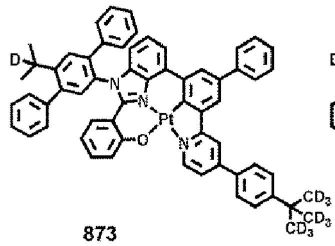
873

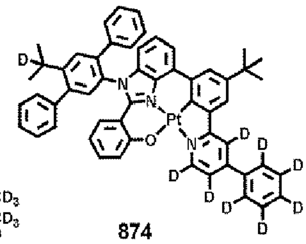
874

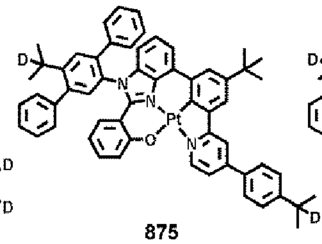
875

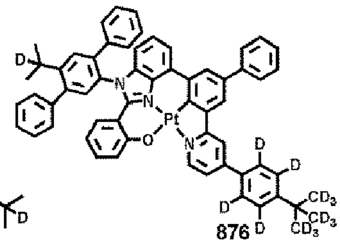
876

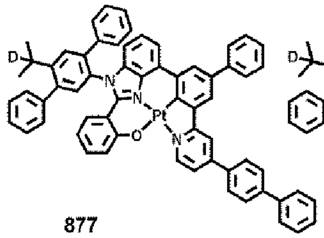
877

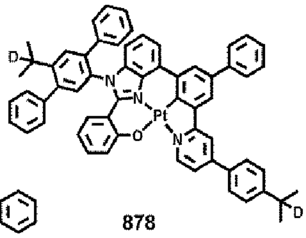
878

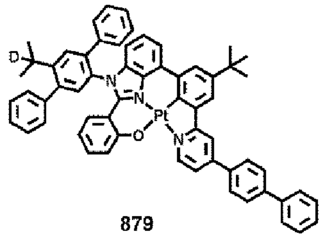
879

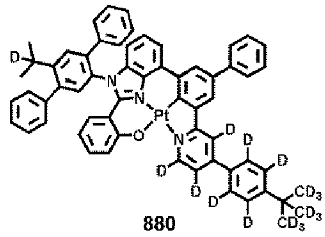
880

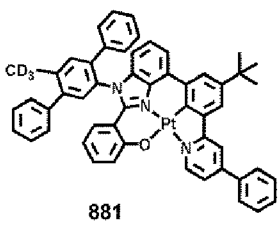
881

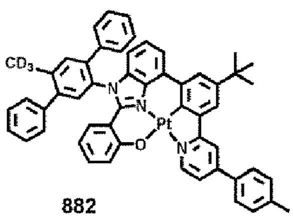
882

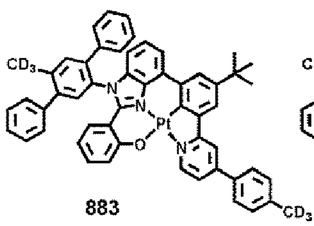
883

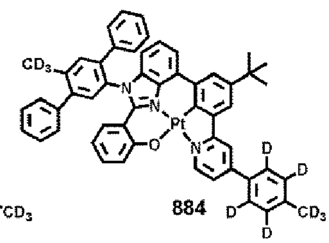
884

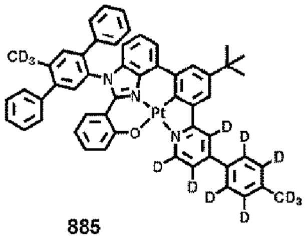
885

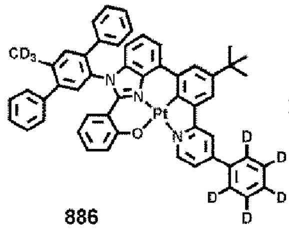
886

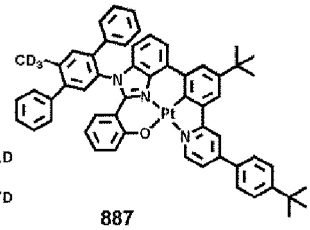
887

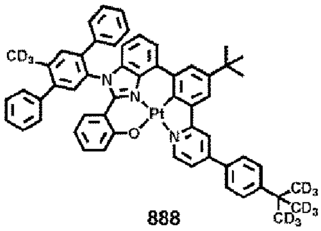
888

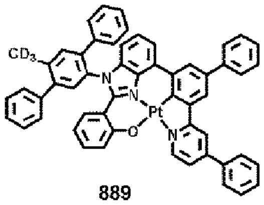
889

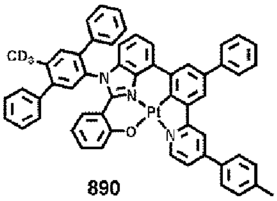
890

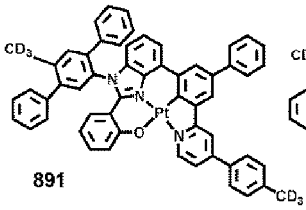
891

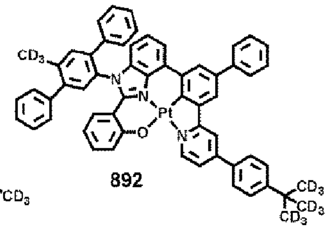
892

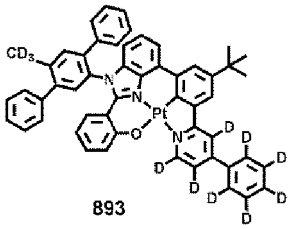
893

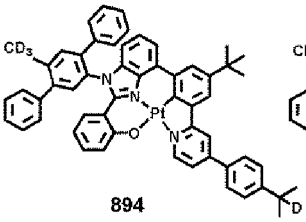
894

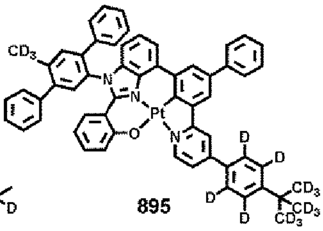
895

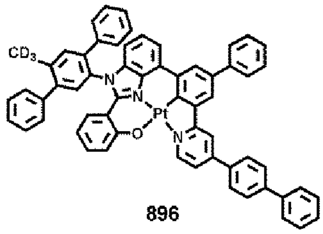
896

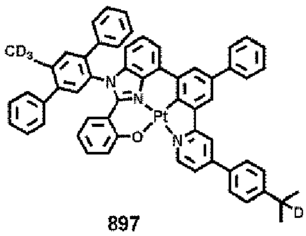
897

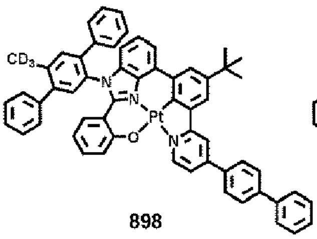
898

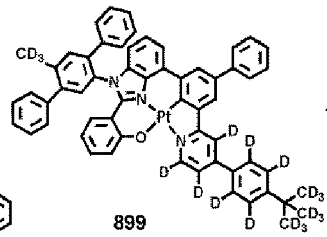
899

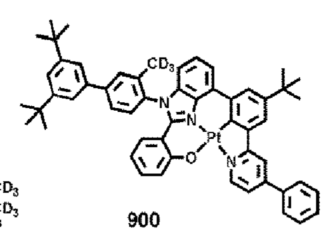
900

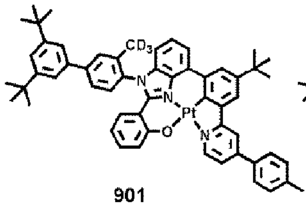
901

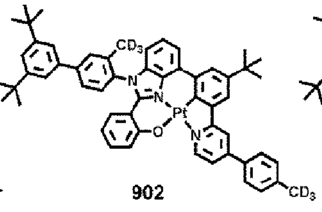
902

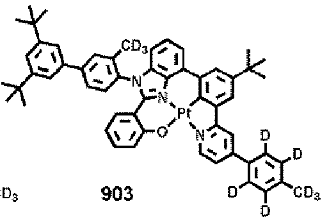
903

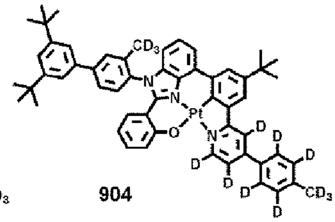
904

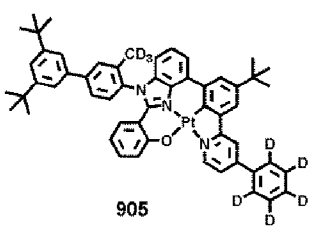
905

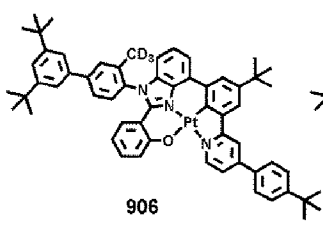
906

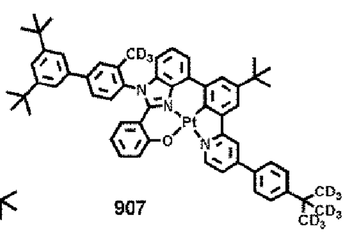
907

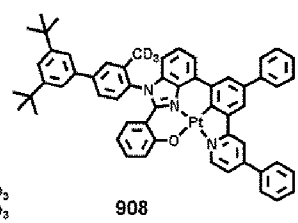
908

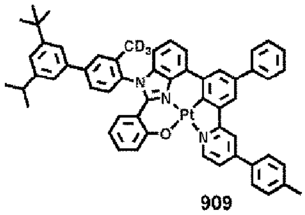

909

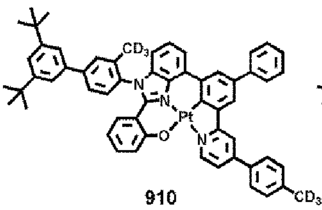

910

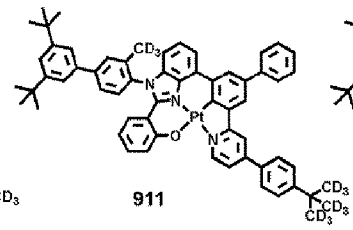

911

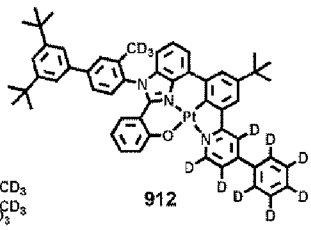

912

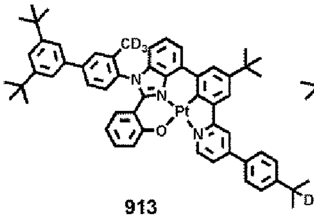

913

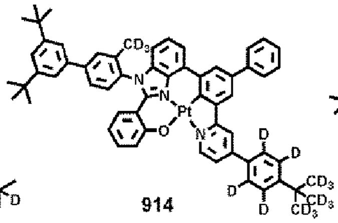

914

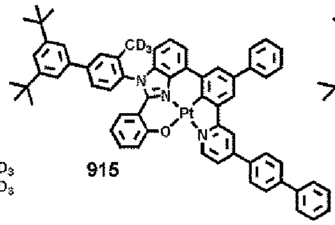

915

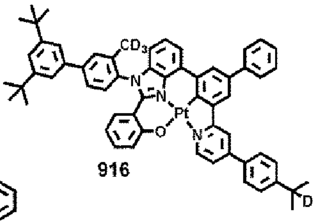

916

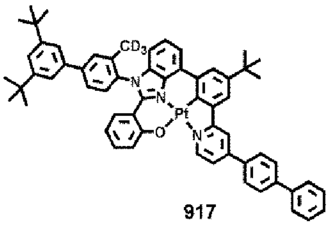

917

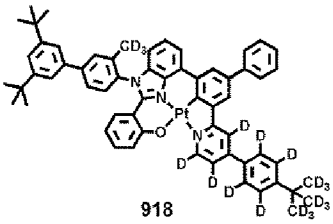

918

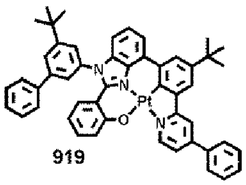

919

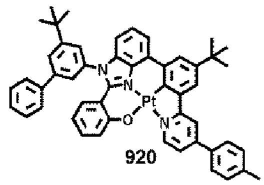

920

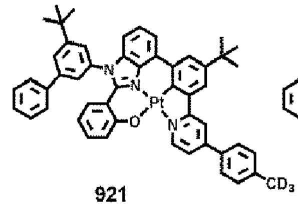

921

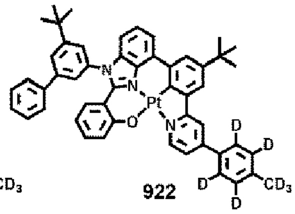

922

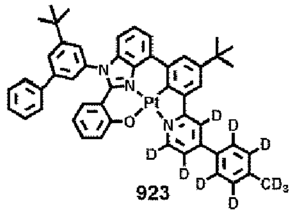

923

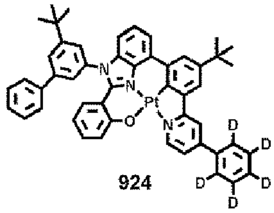

924

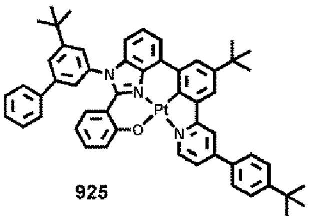

925

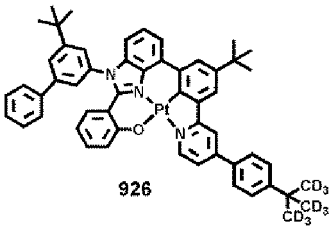

926

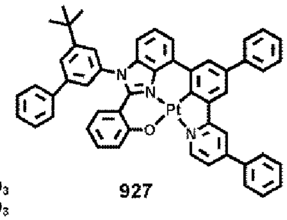

927

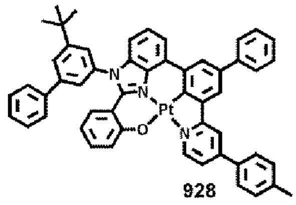

928

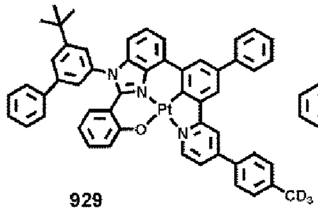

929

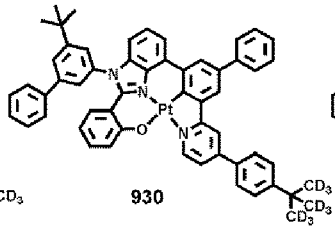

930

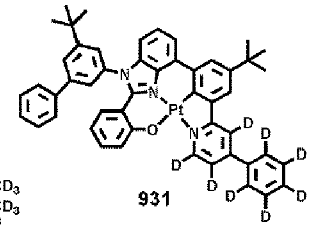

931

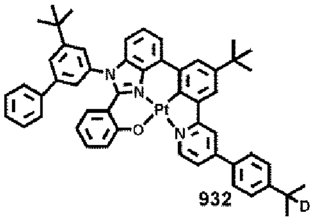

932

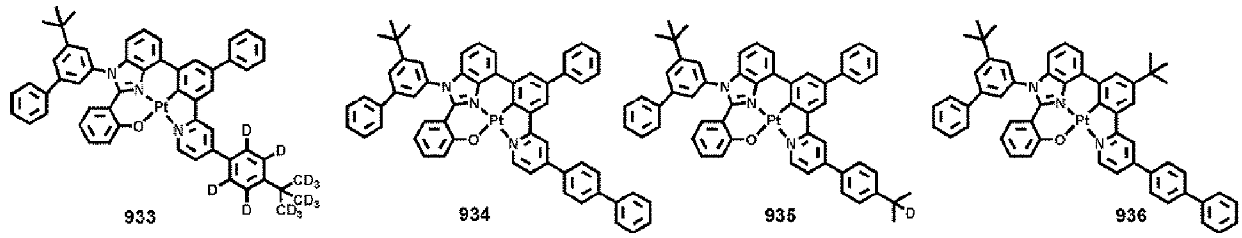

933 934 935 936

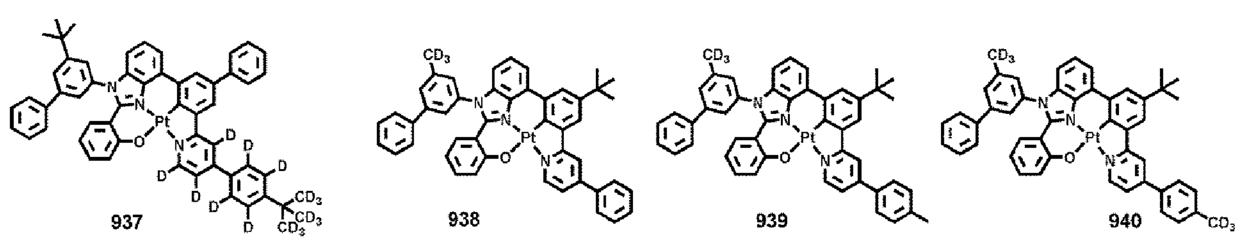

937 938 939 940

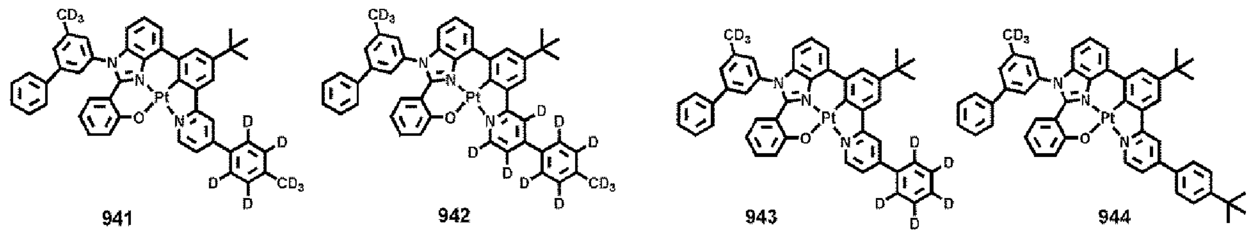

941 942 943 944

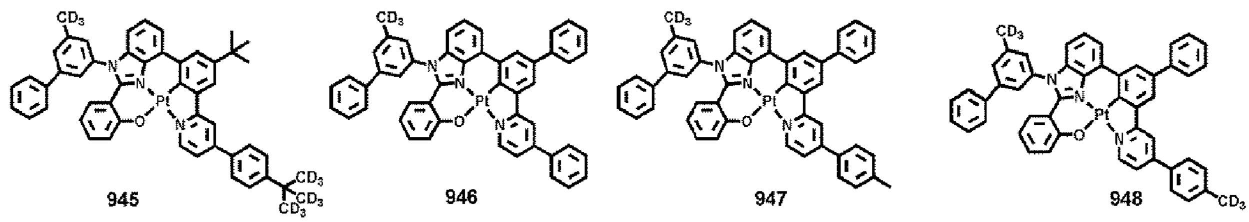

945 946 947 948

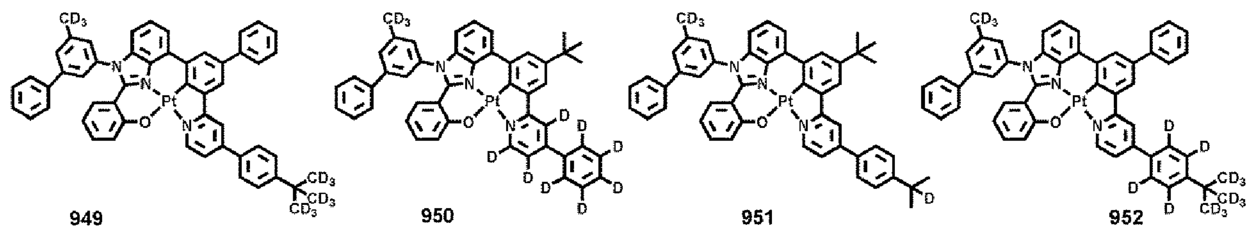

949 950 951 952

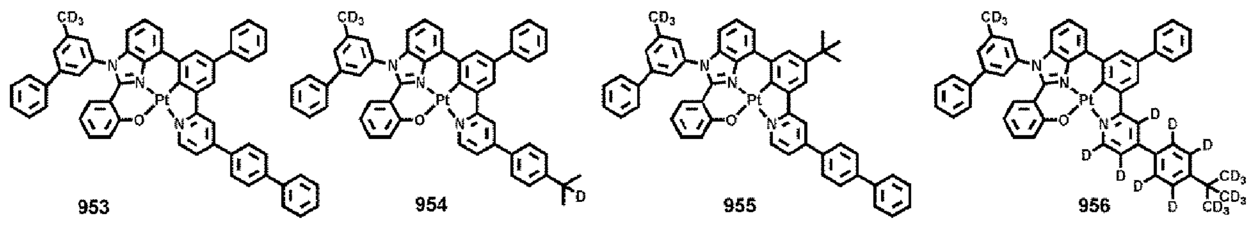

953 954 955 956

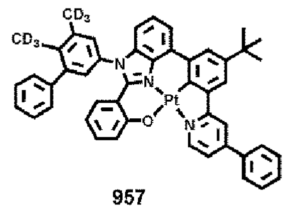
957

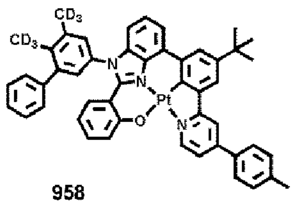
958

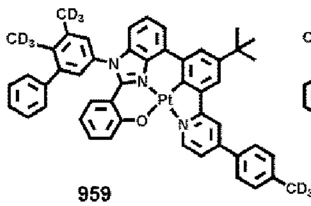
959

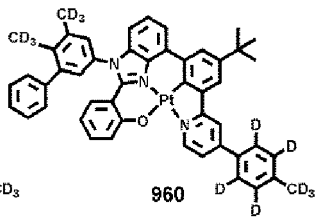
960

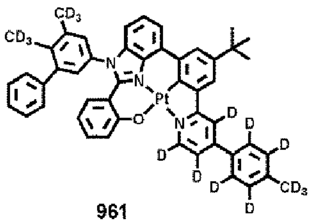
961

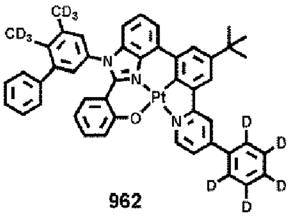
962

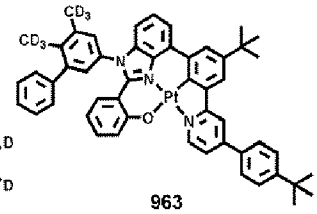
963

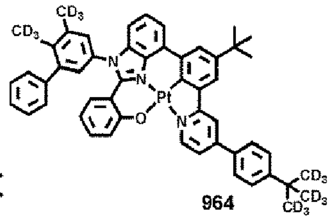
964

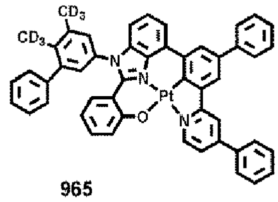
965

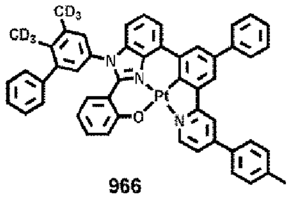
966

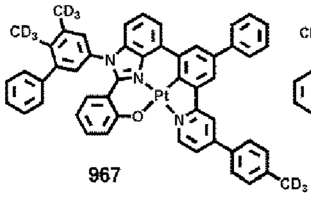
967

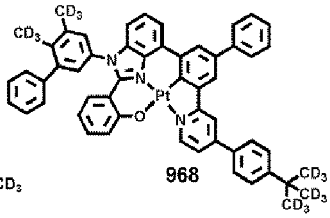
968

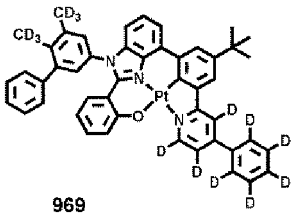
969

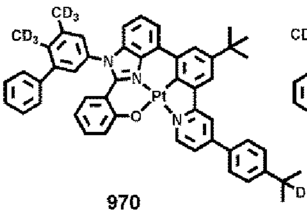
970

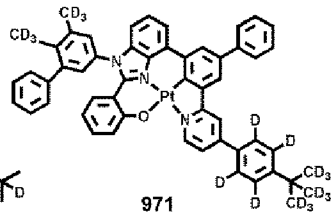
971

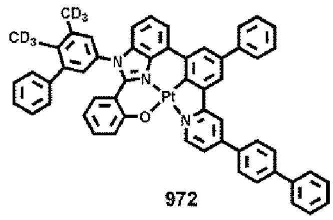
972

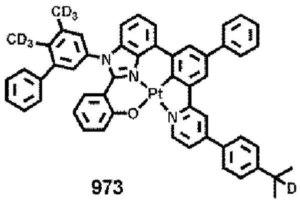
973

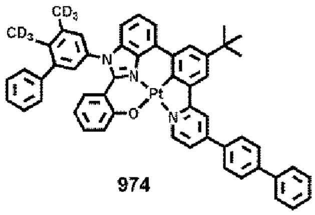
974

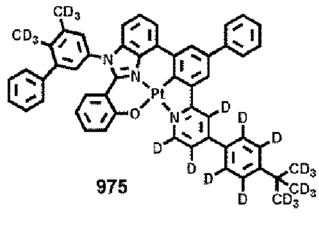
975

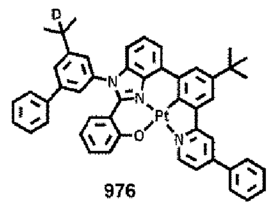
976

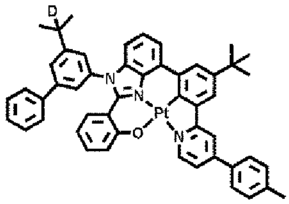
977

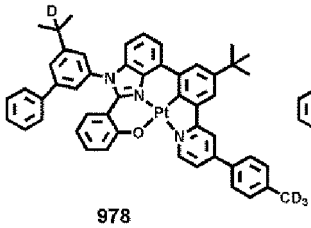
978

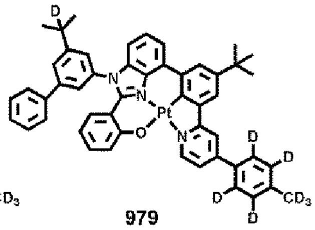
979

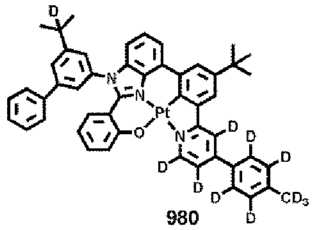
980

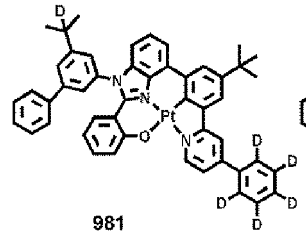
981
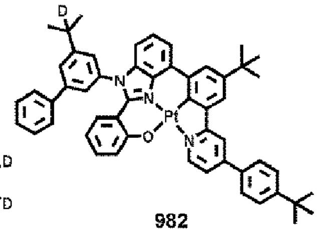
982
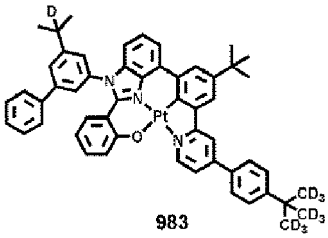
983
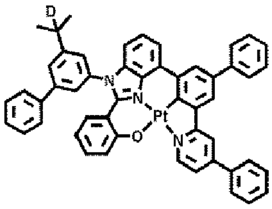
984
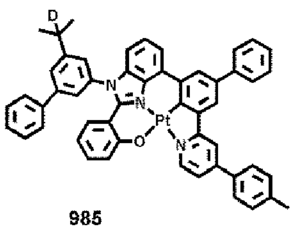
985
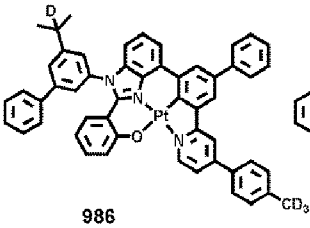
986
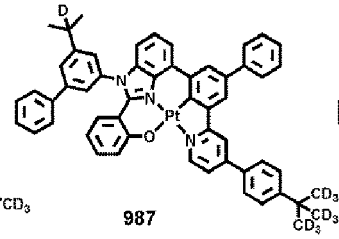
987
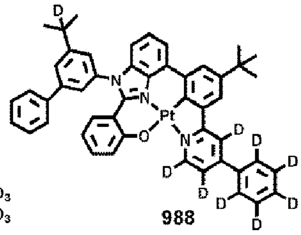
988
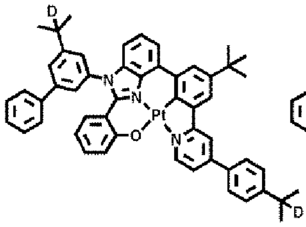
989
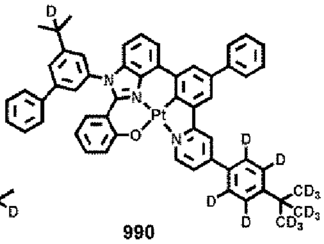
990
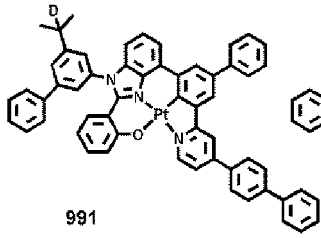
991
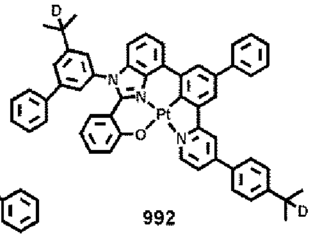
992
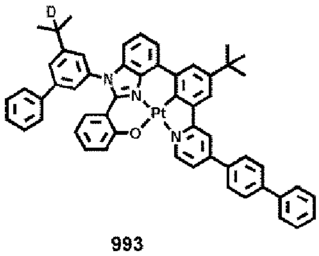
993
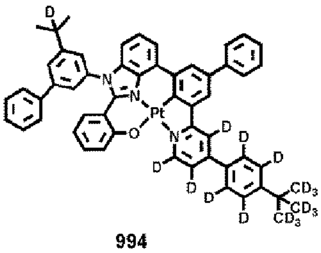
994
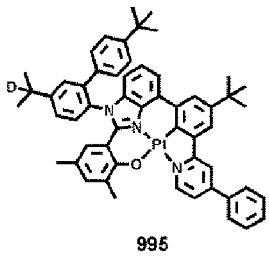
995
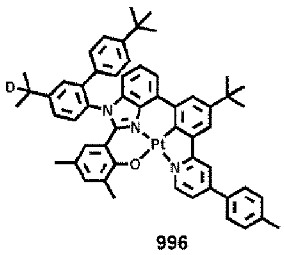
996
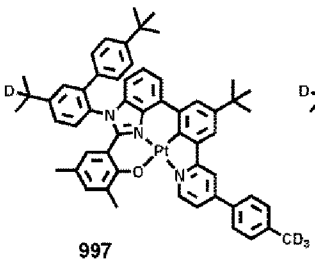
997
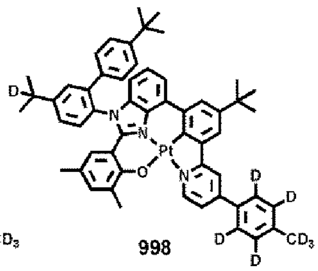
998
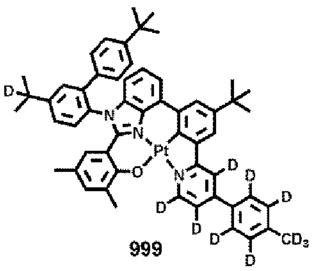
999
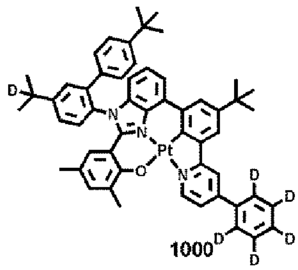
1000
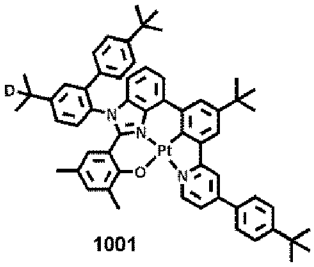
1001
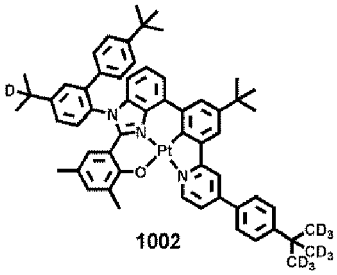
1002
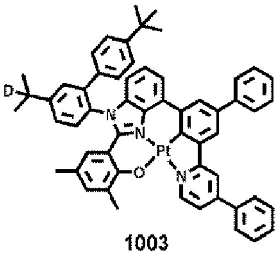
1003
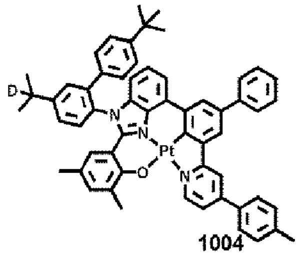
1004

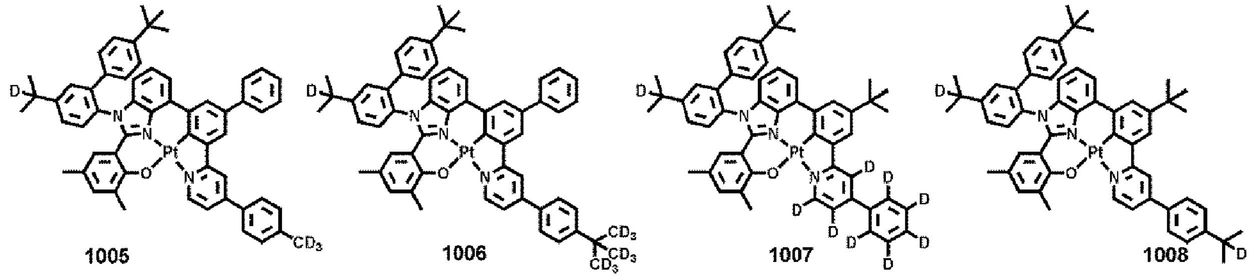

1005 1006 1007 1008

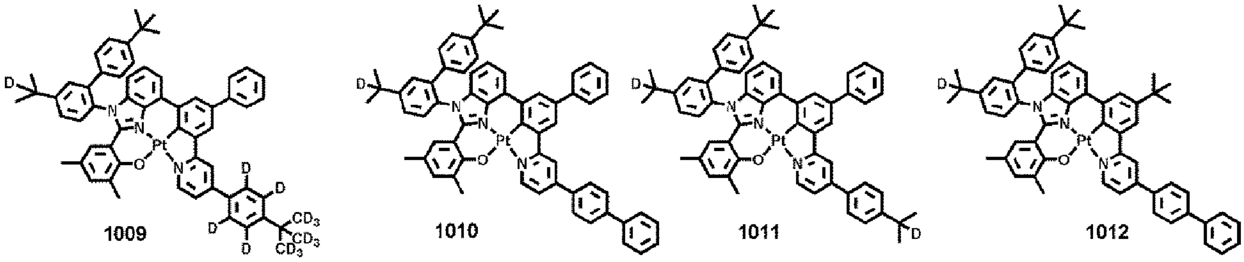

1009 1010 1011 1012

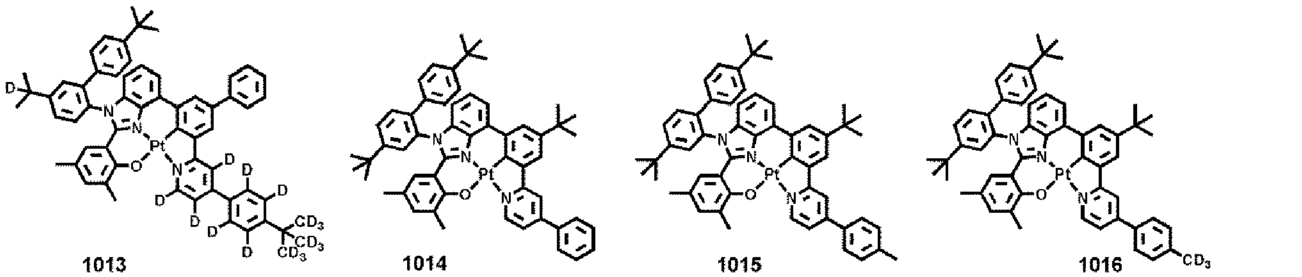

1013 1014 1015 1016

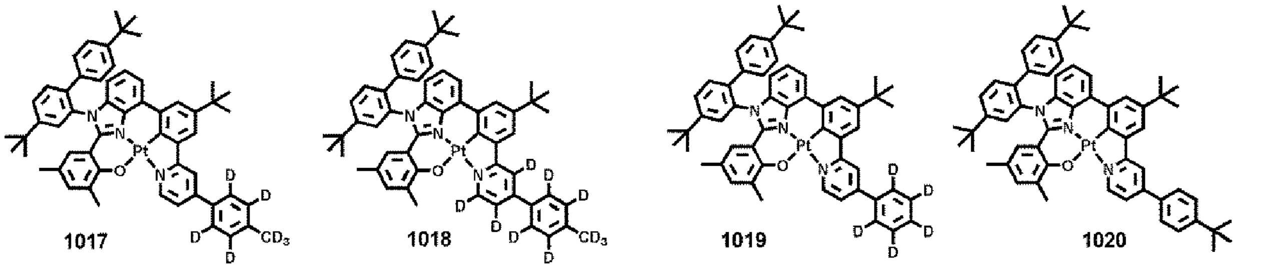

1017 1018 1019 1020

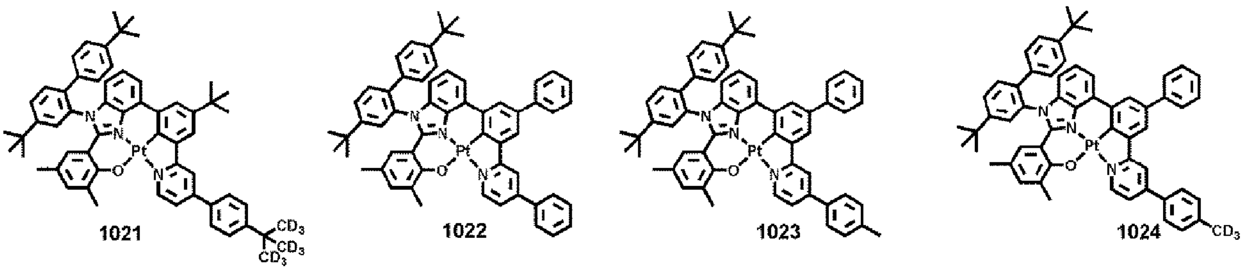

1021 1022 1023 1024

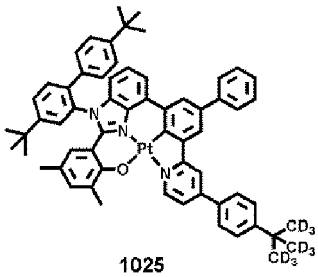
1025

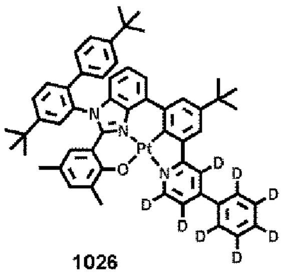
1026

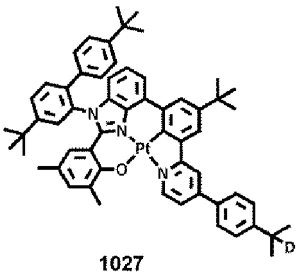
1027

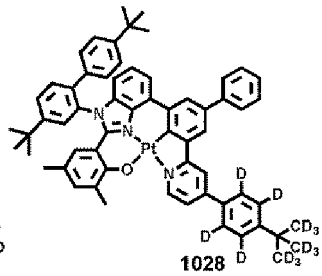
1028

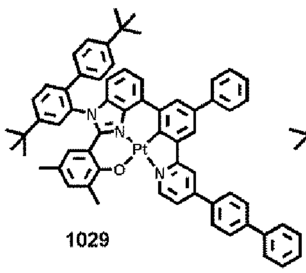
1029

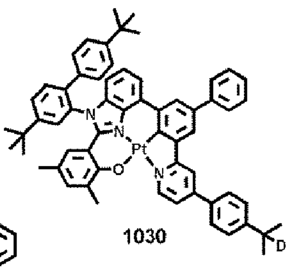
1030

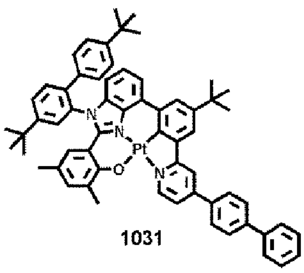
1031

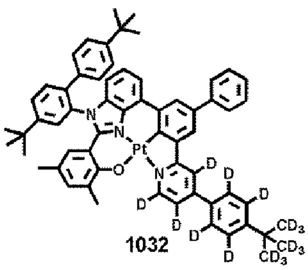
1032

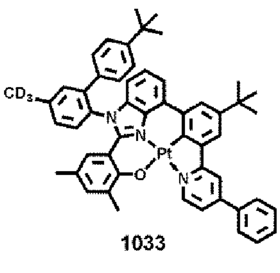
1033

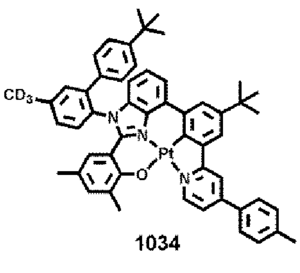
1034

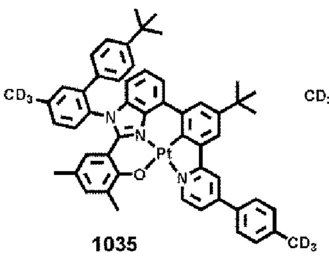
1035

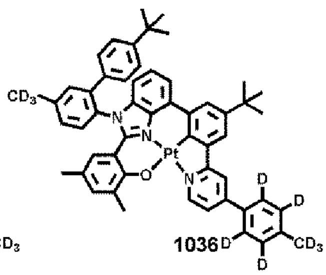
1036

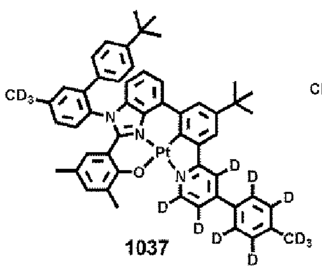
1037

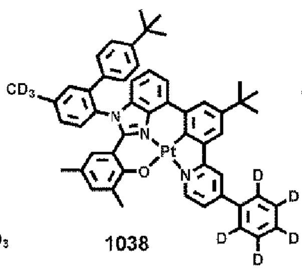
1038

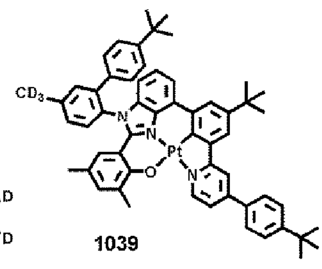
1039

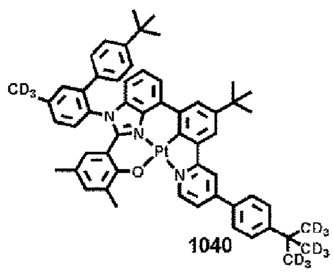
1040

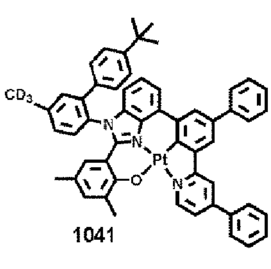
1041

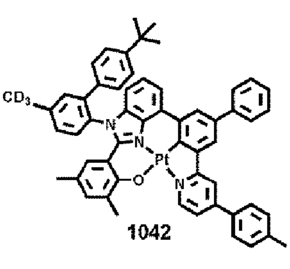
1042

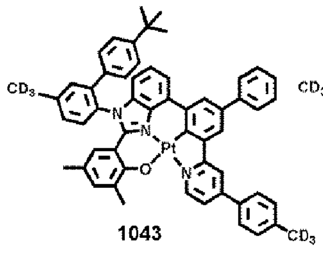
1043

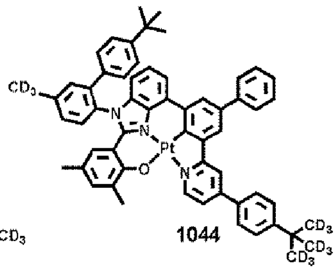
1044

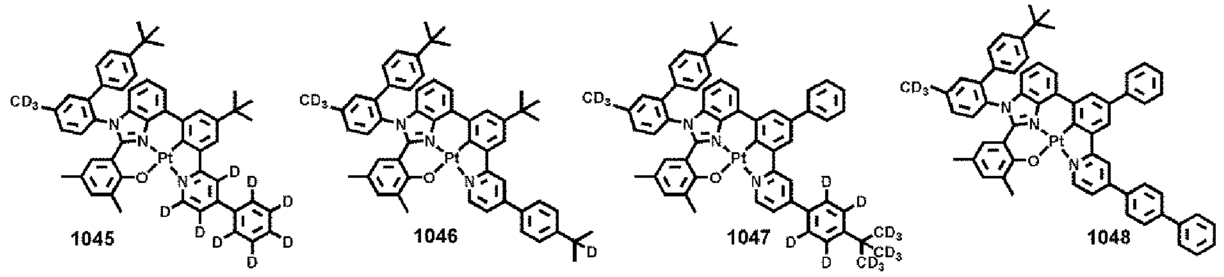

1045 1046 1047 1048

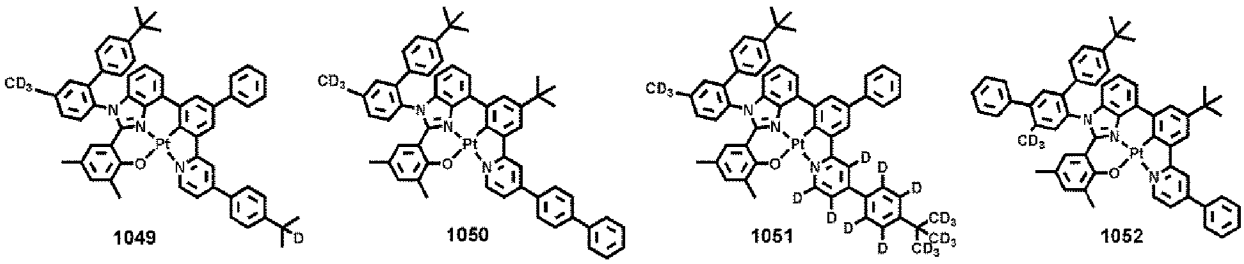

1049 1050 1051 1052

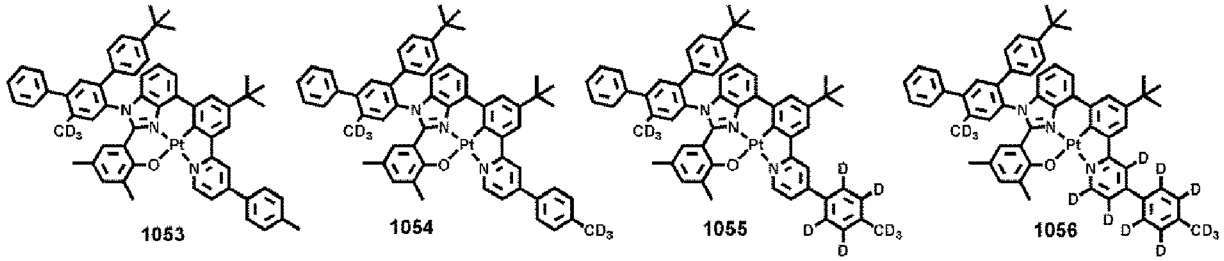

1053 1054 1055 1056

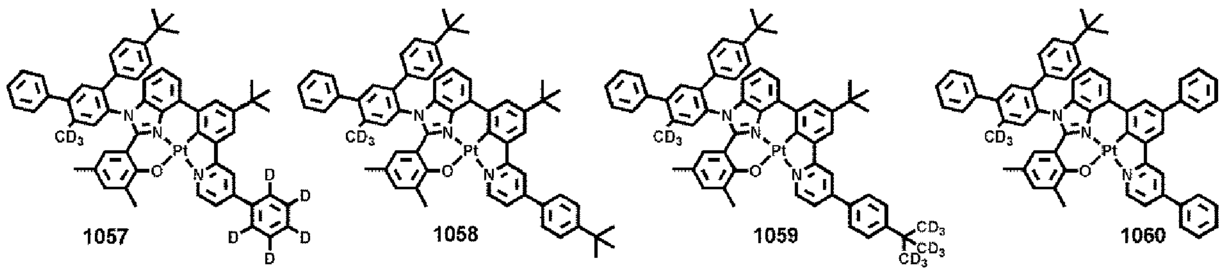

1057 1058 1059 1060

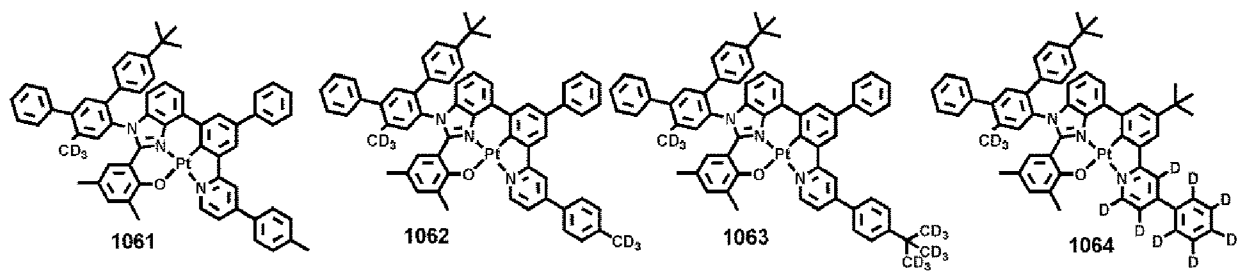

1061 1062 1063 1064

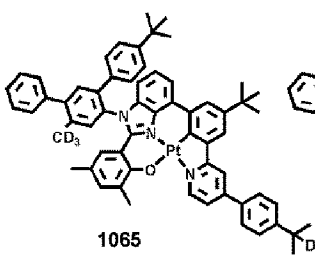
1065

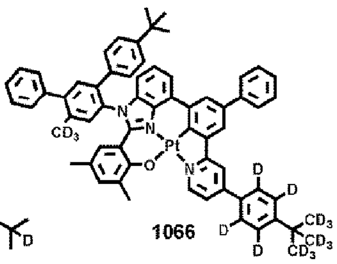
1066

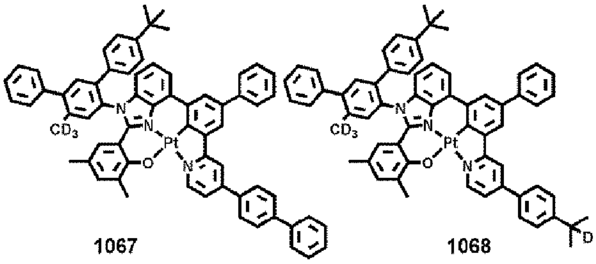
1067

1068

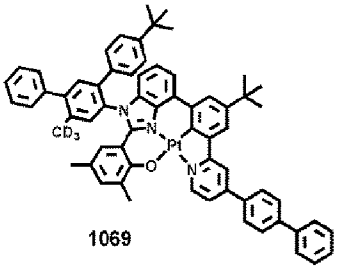
1069

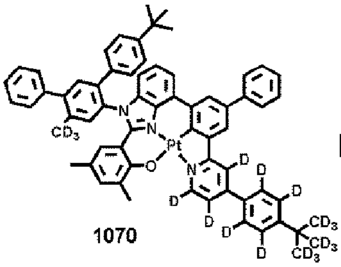
1070

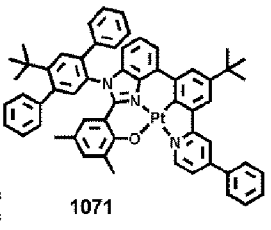
1071

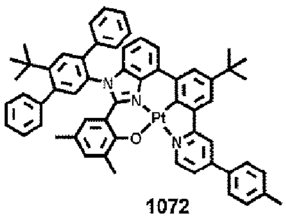
1072

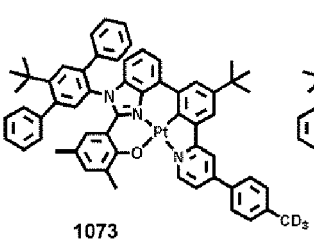
1073

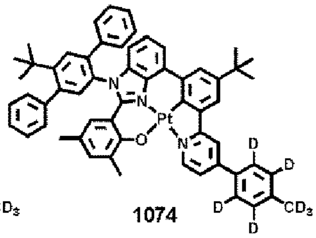
1074

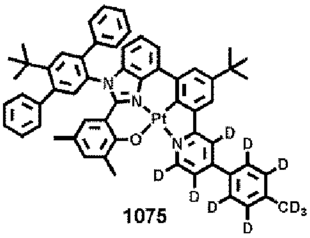
1075

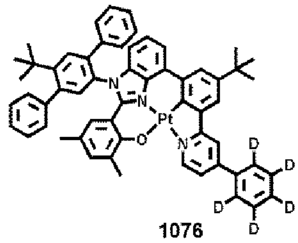
1076

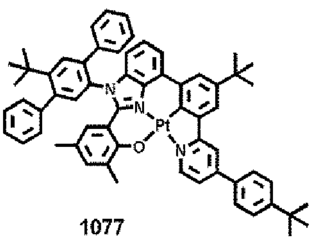
1077

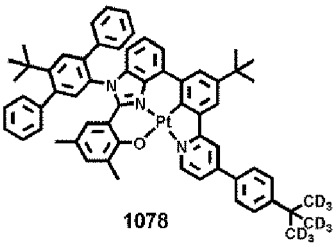
1078

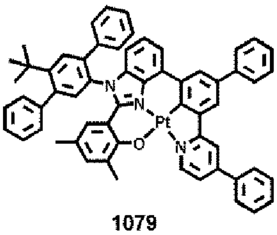
1079

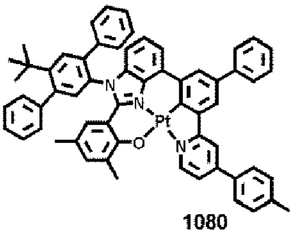
1080

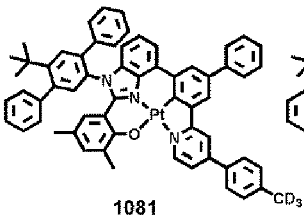
1081

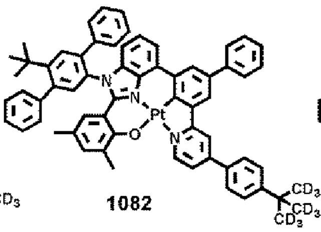
1082

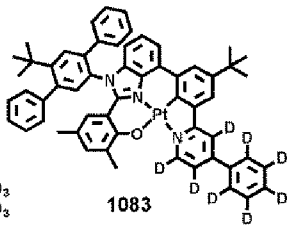
1083

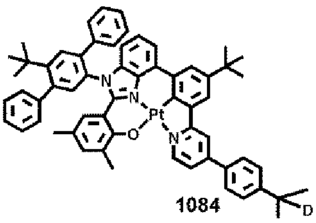
1084

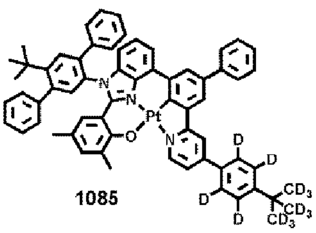
1085

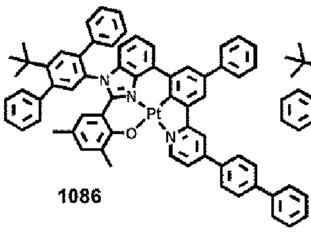
1086

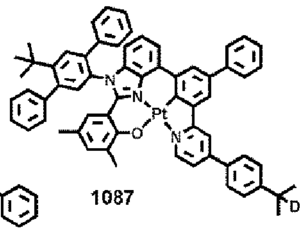
1087

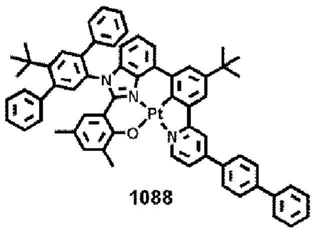
1088

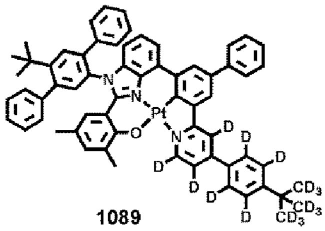
1089

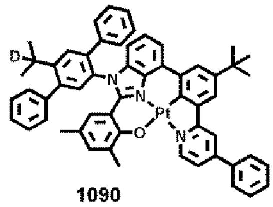
1090

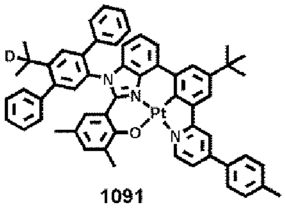
1091

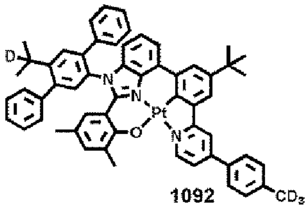
1092

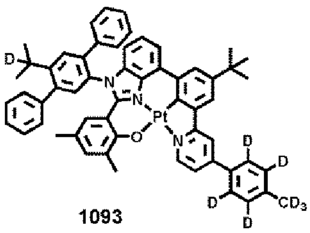
1093

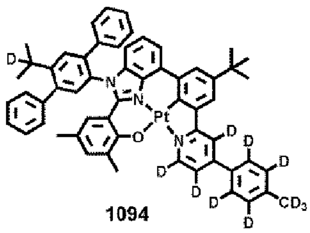
1094

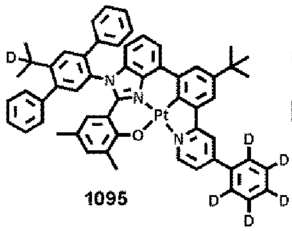
1095

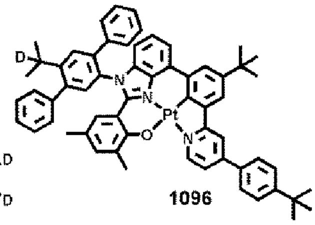
1096

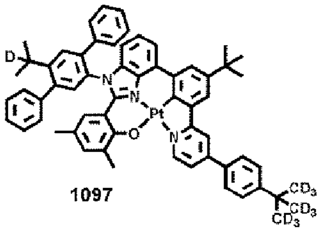
1097

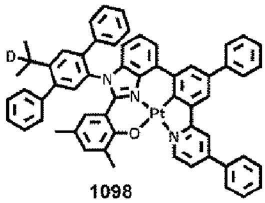
1098

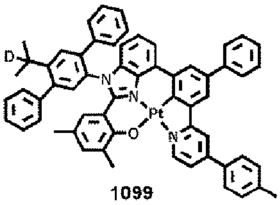
1099

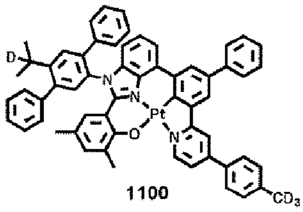
1100

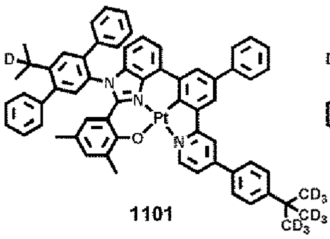
1101

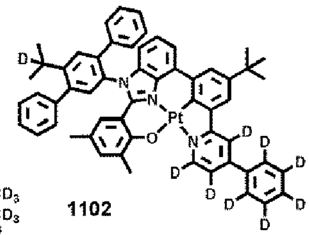
1102

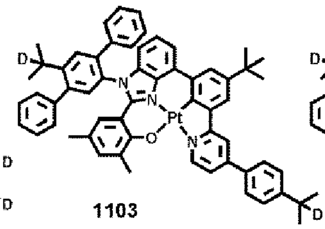
1103

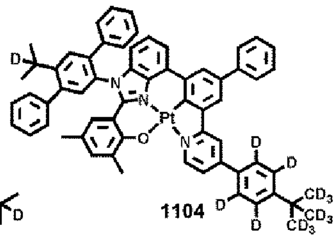
1104

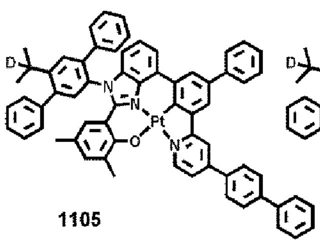
1105

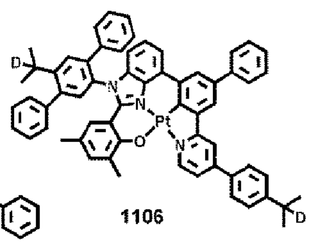
1106

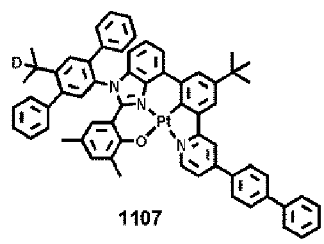
1107

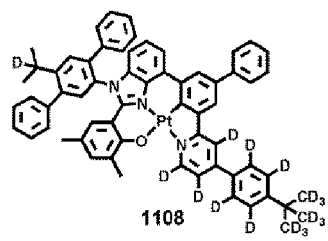
1108

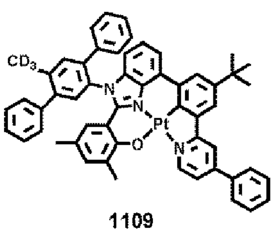
1109

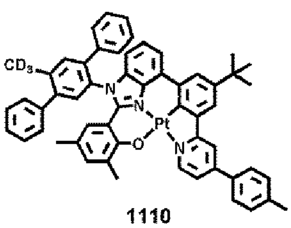
1110

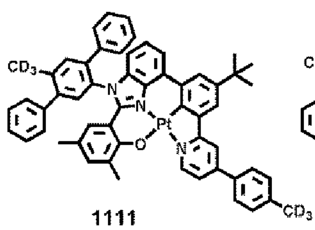
1111

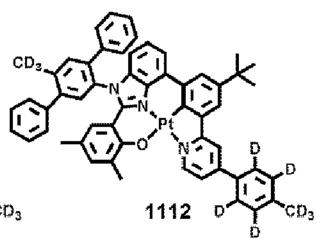
1112

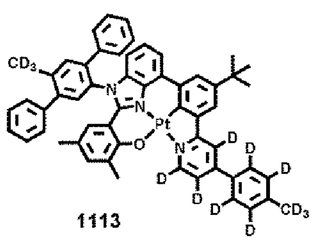
1113

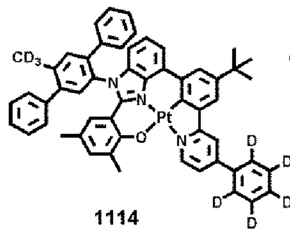
1114

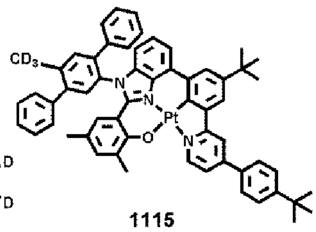
1115

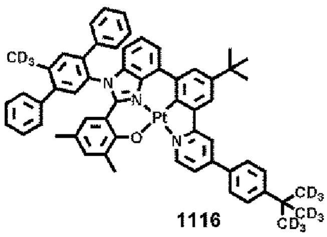
1116

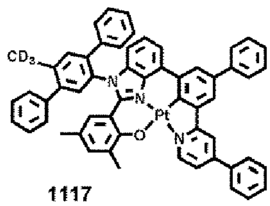
1117

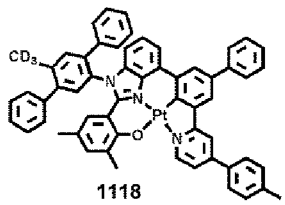
1118

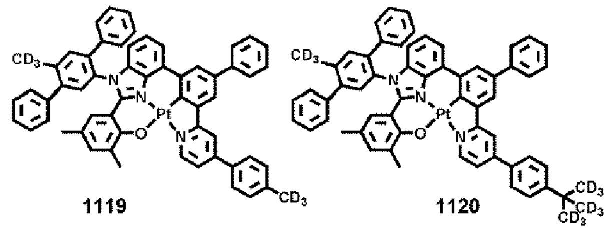
1119

1120

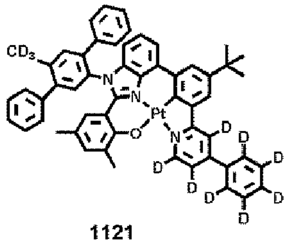
1121

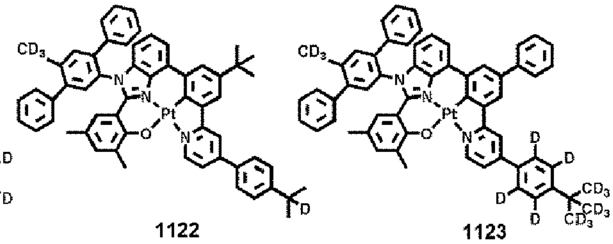
1122

1123

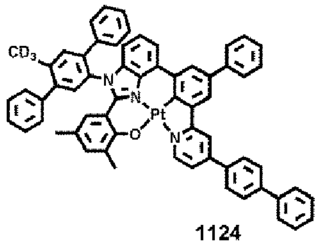
1124

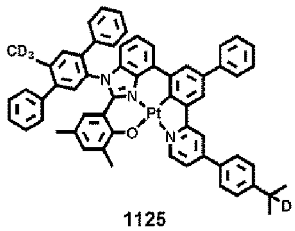
1125

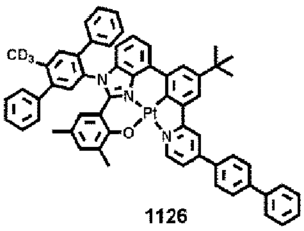
1126

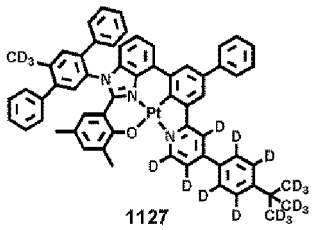
1127

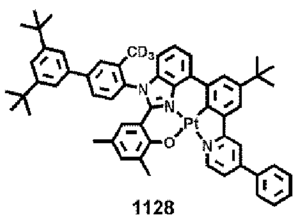
1128

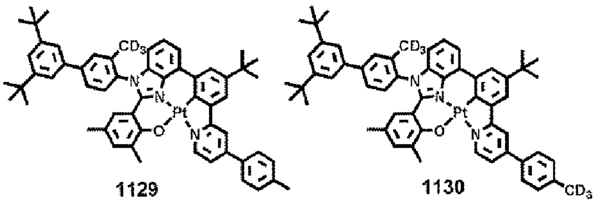
1129

1130

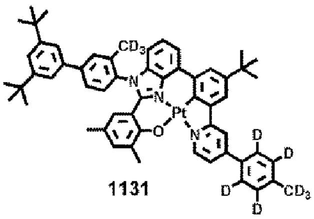
1131

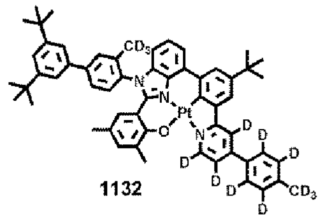
1132

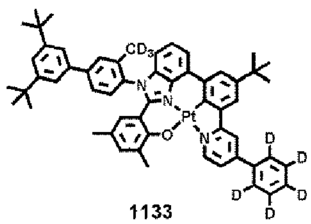
1133

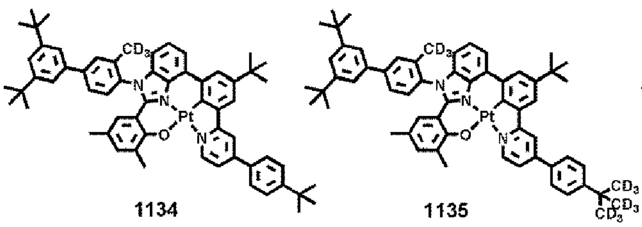
1134

1135

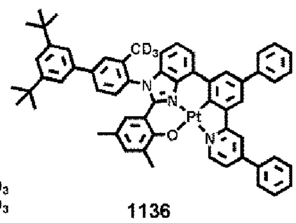
1136

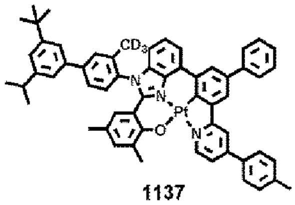
1137

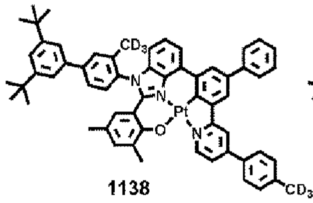
1138

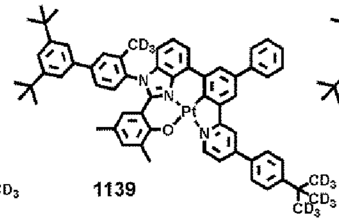
1139

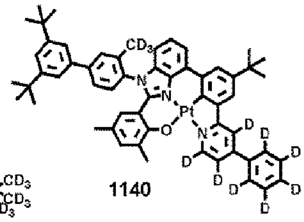
1140

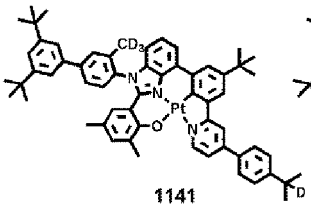
1141

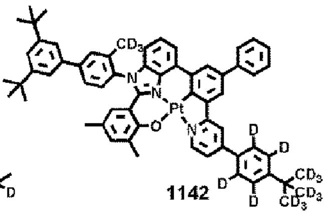
1142

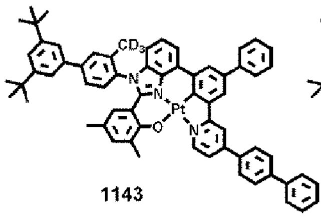
1143

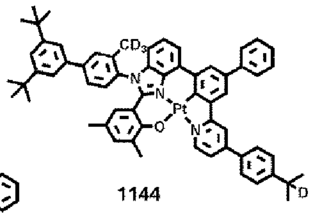
1144

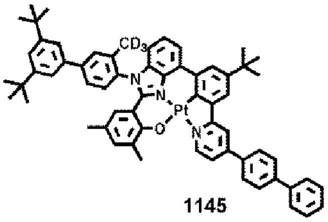
1145

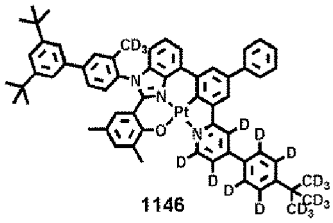
1146

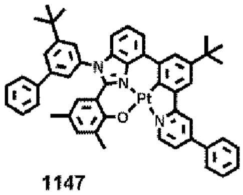
1147

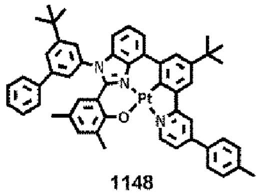
1148

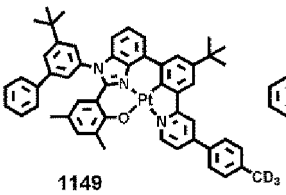
1149

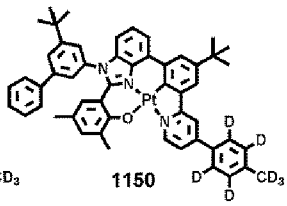
1150

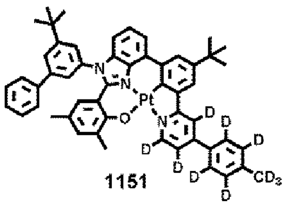
1151

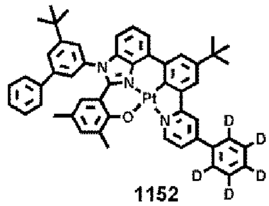
1152

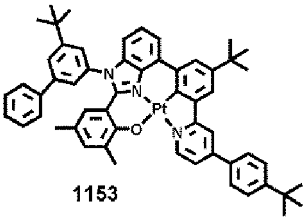
1153

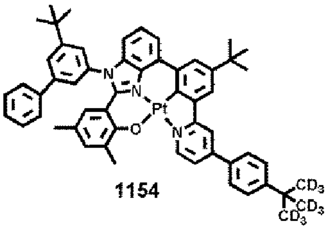
1154

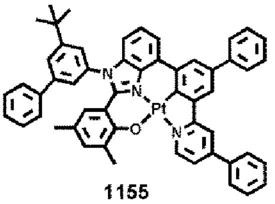
1155

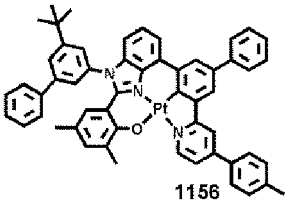
1156

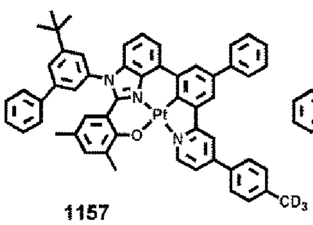
1157

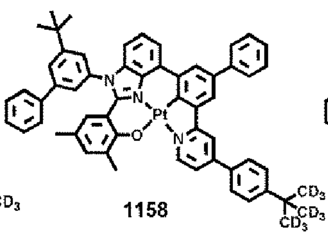
1158

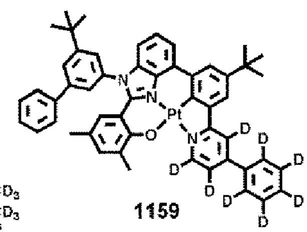
1159

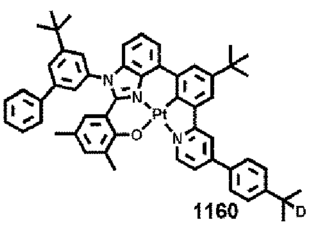
1160

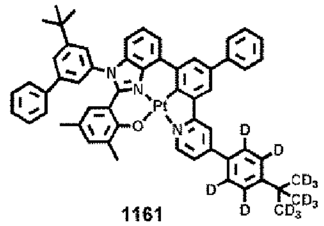
1161

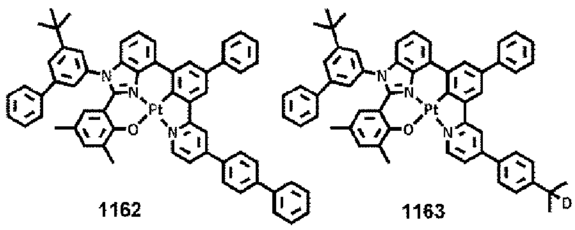
1162

1163

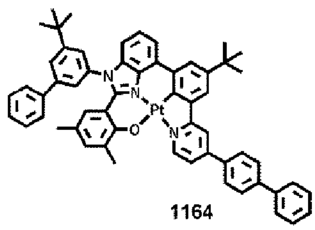
1164

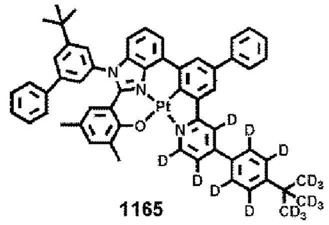
1165

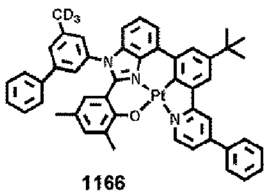
1166

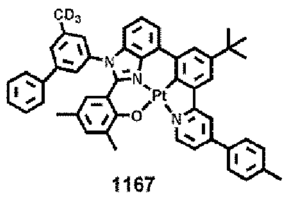
1167

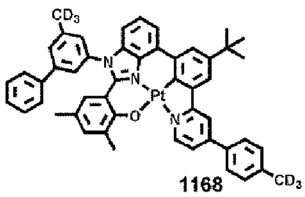
1168

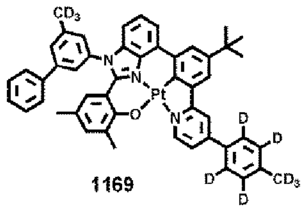
1169

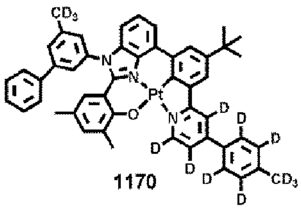
1170

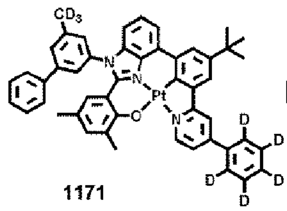
1171

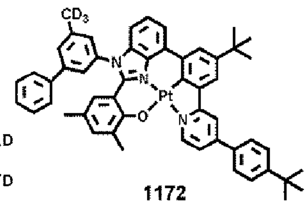
1172

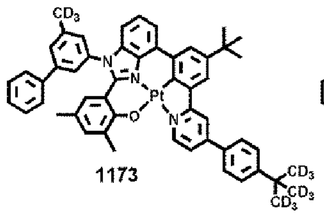
1173

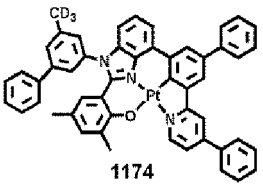
1174

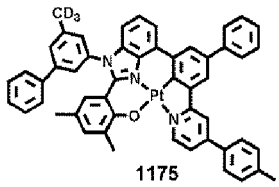
1175

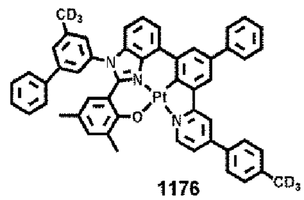
1176

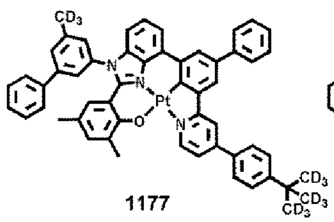
1177

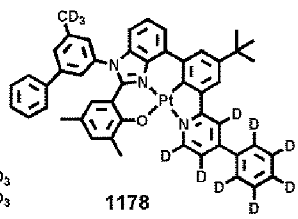
1178

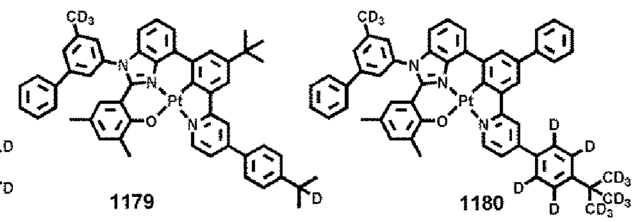
1179

1180

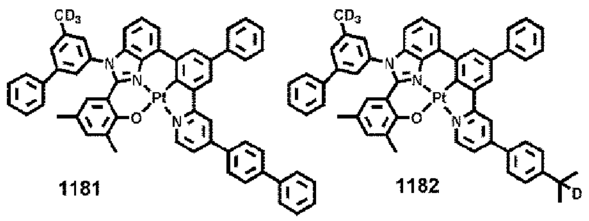
1181

1182

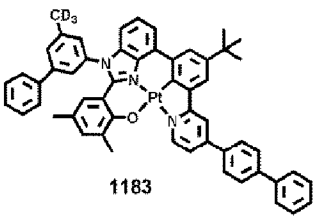
1183

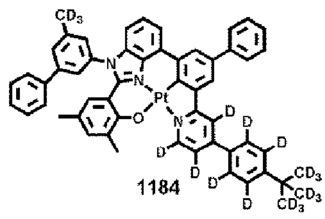
1184

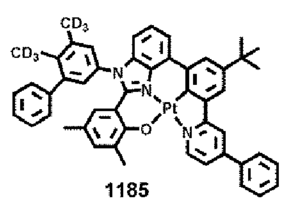
1185

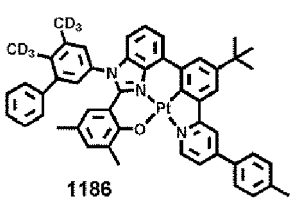
1186

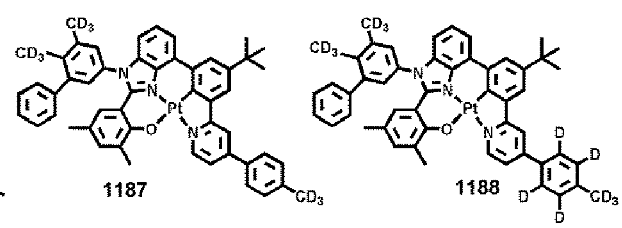
1187

1188

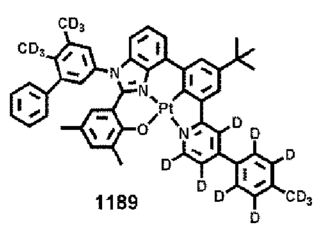
1189

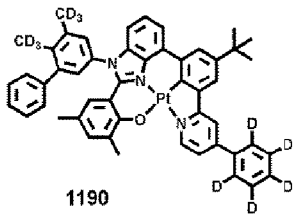
1190

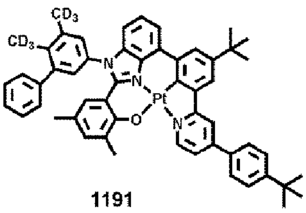
1191

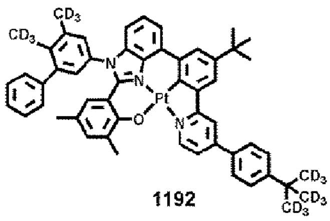
1192

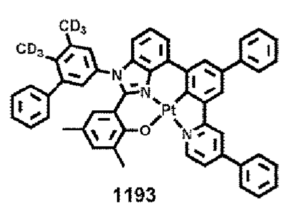
1193

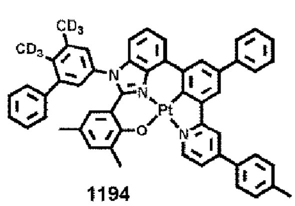
1194

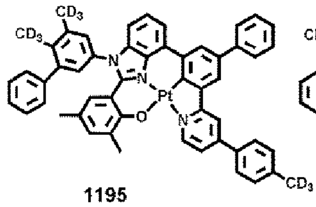
1195

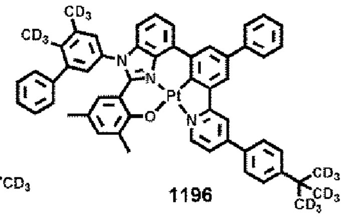
1196

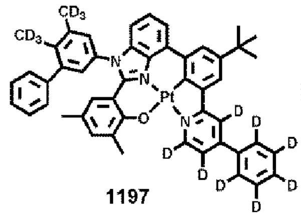
1197

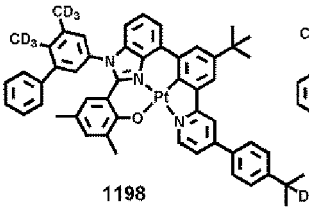
1198

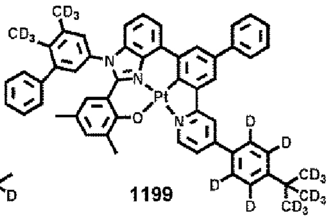
1199

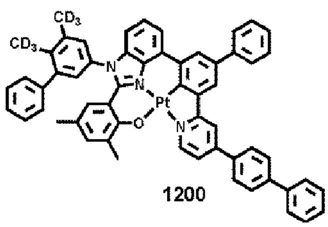
1200

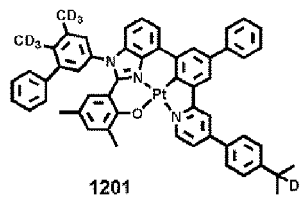
1201

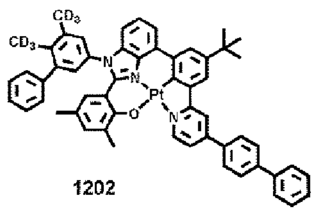
1202

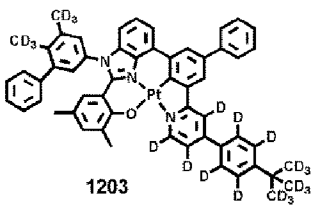
1203

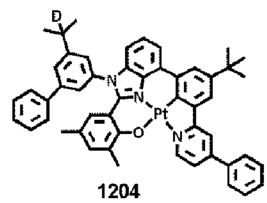
1204

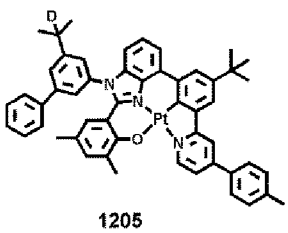
1205

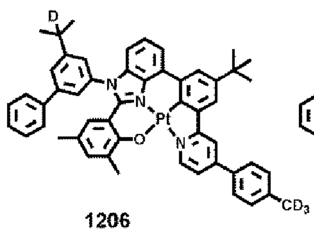
1206

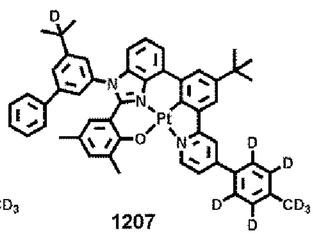
1207

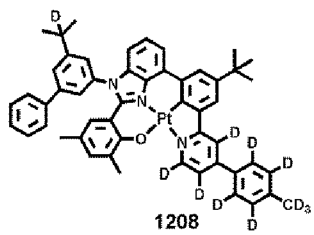
1208

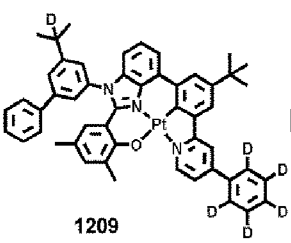
1209

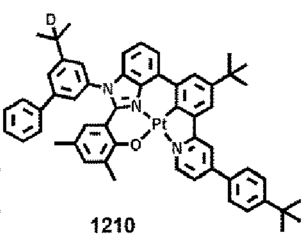
1210

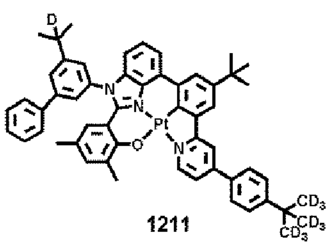
1211

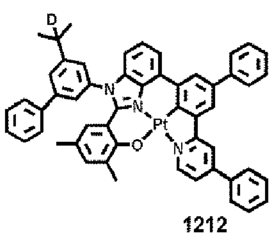
1212

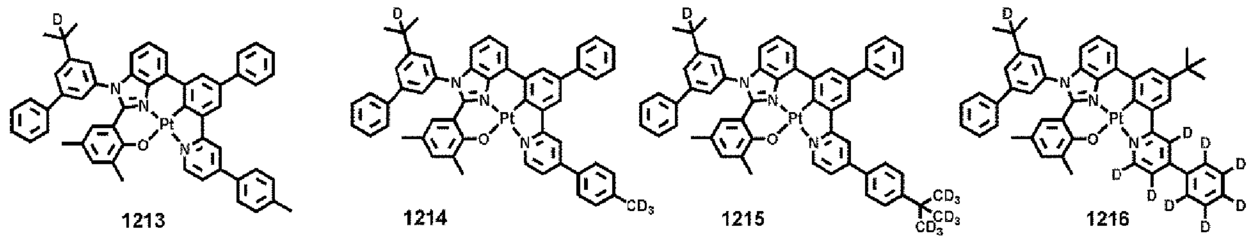

1213 1214 1215 1216

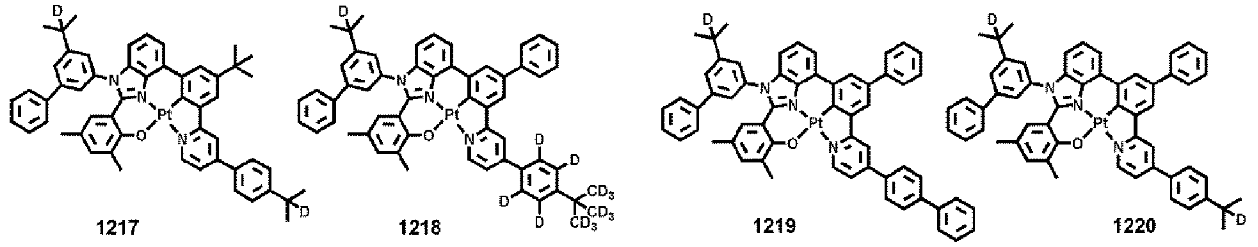

1217 1218 1219 1220

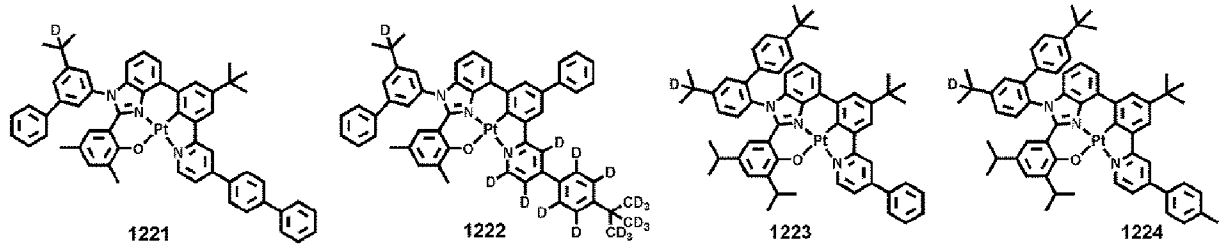

1221 1222 1223 1224

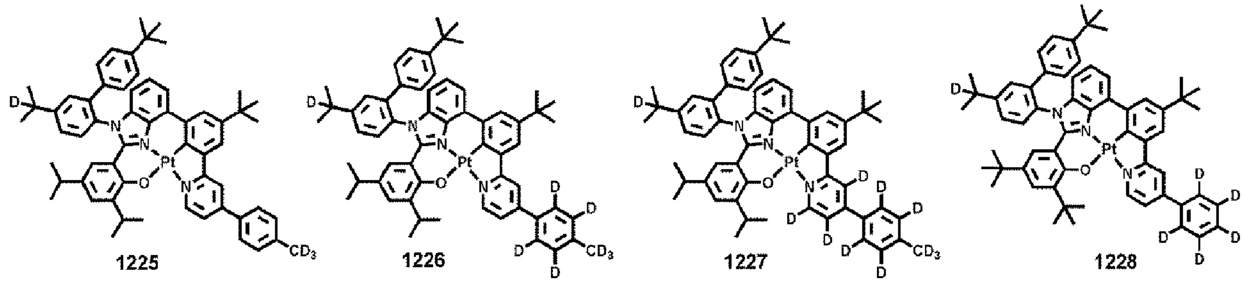

1225 1226 1227 1228

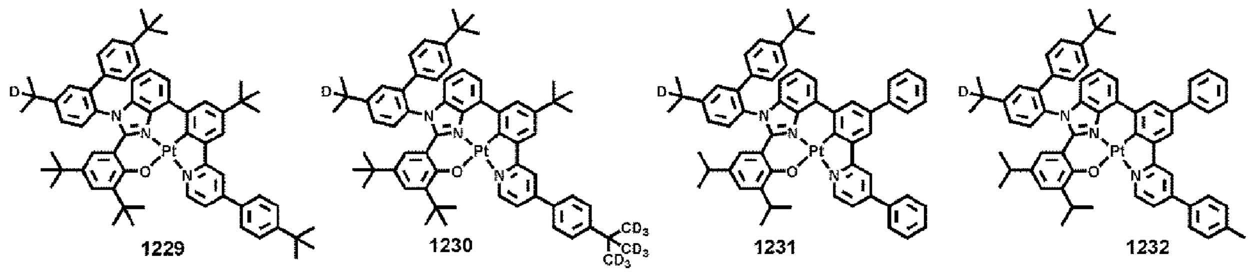

1229 1230 1231 1232

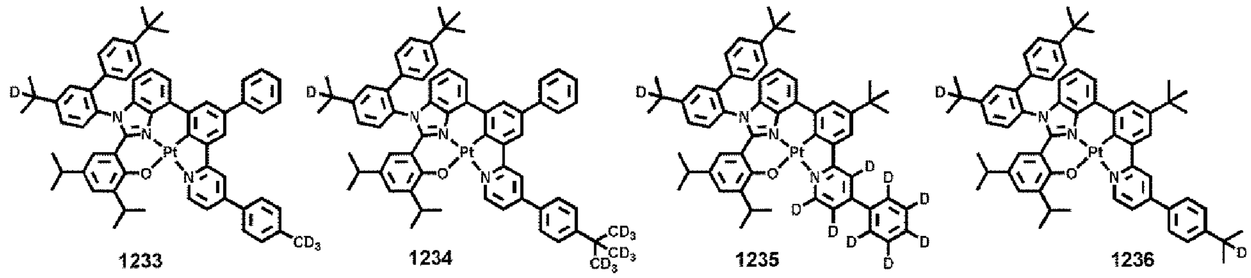

1233 1234 1235 1236

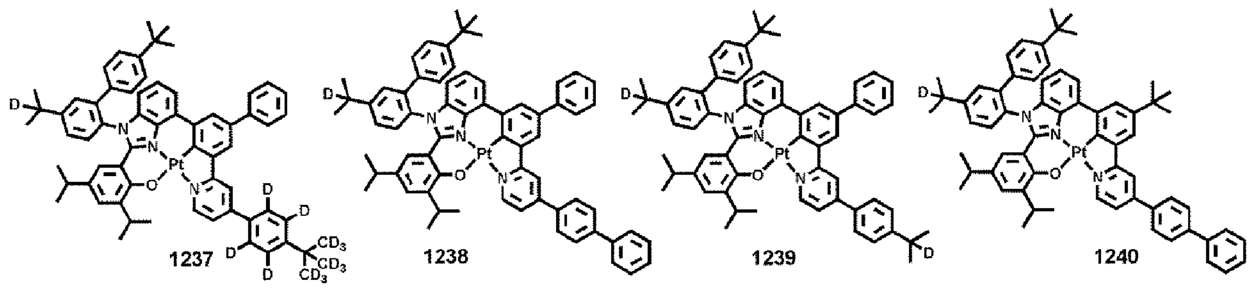

1237 1238 1239 1240

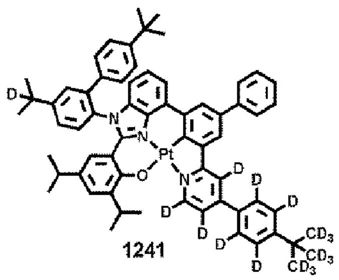

1241

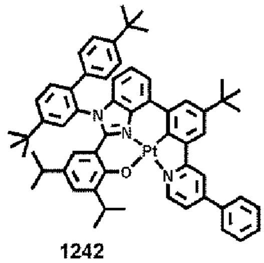

1242

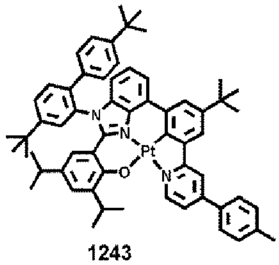

1243

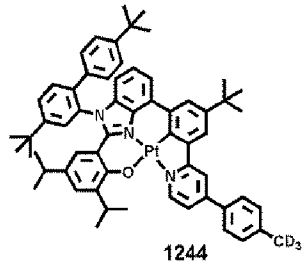

1244

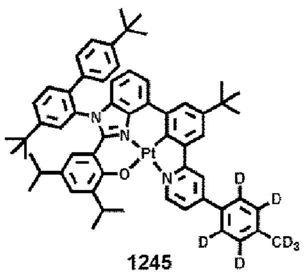

1245

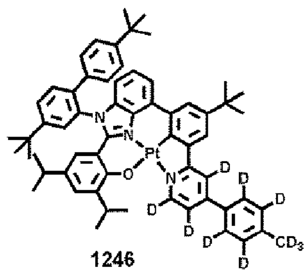

1246

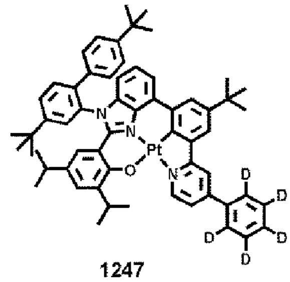

1247

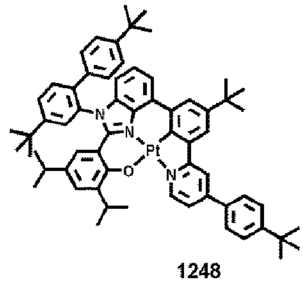

1248

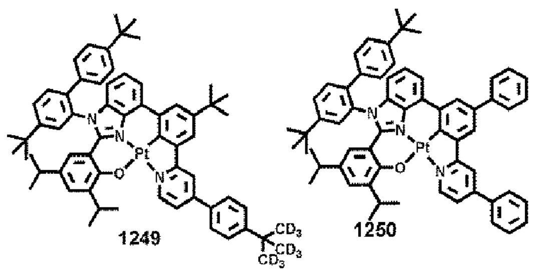

1249 1250

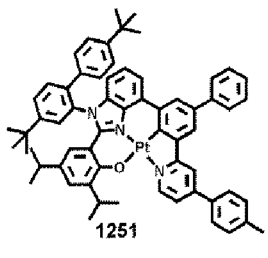

1251

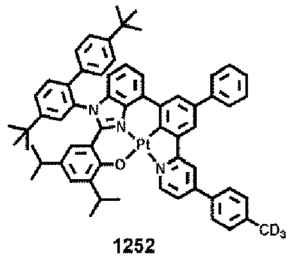

1252

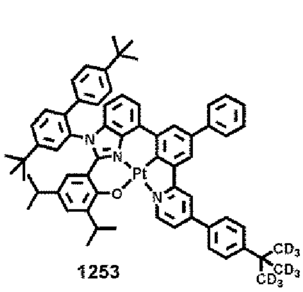

1253

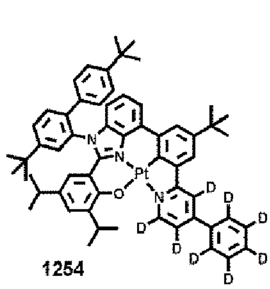

1254

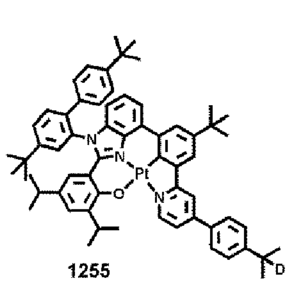

1255

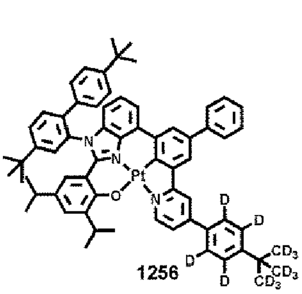

1256

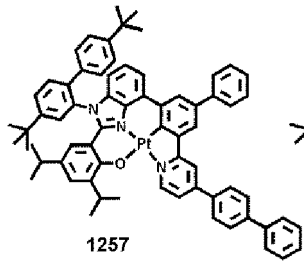
1257

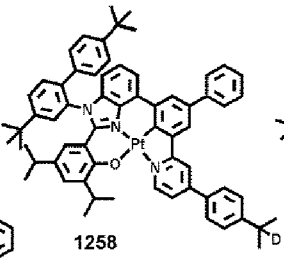
1258

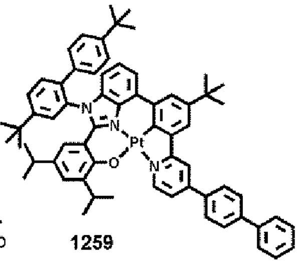
1259

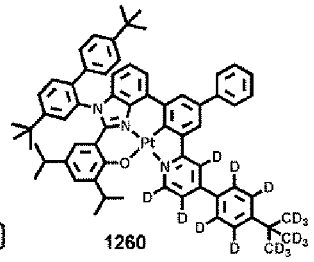
1260

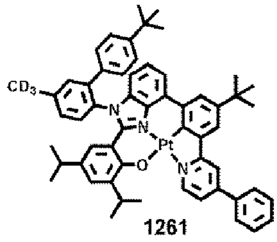
1261

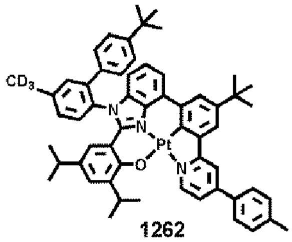
1262

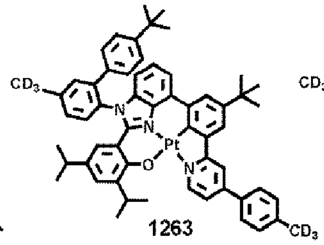
1263

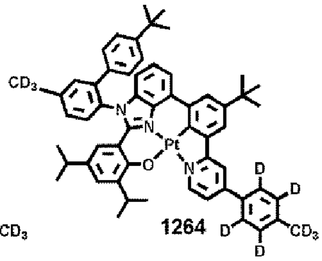
1264

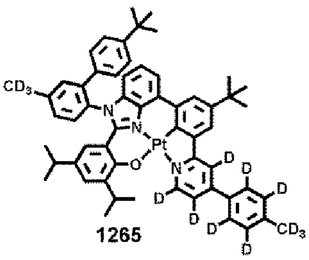
1265

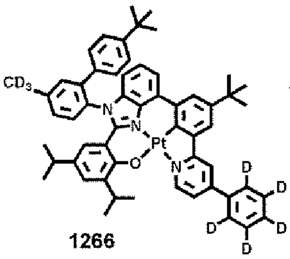
1266

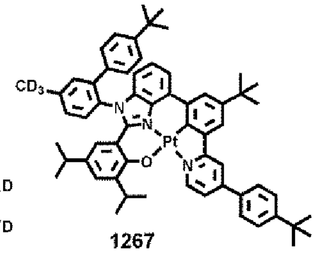
1267

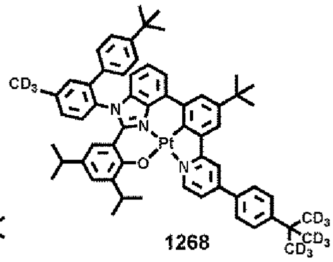
1268

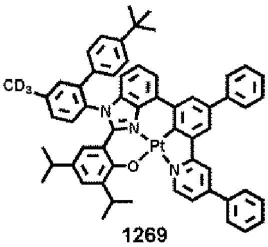
1269

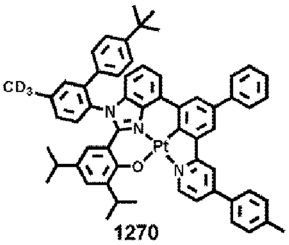
1270

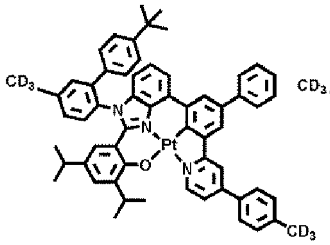
1271

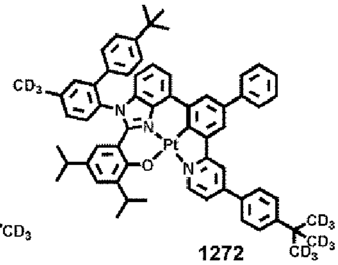
1272

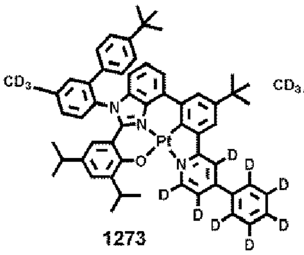
1273

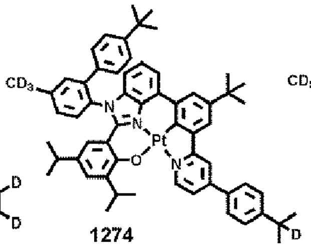
1274

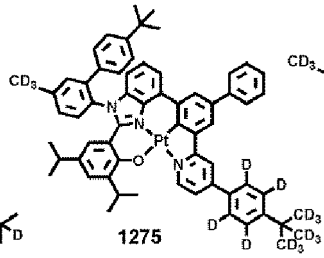
1275

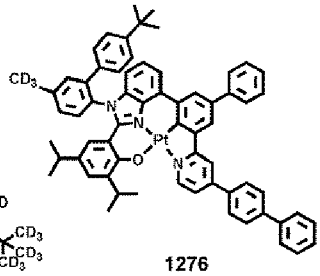
1276

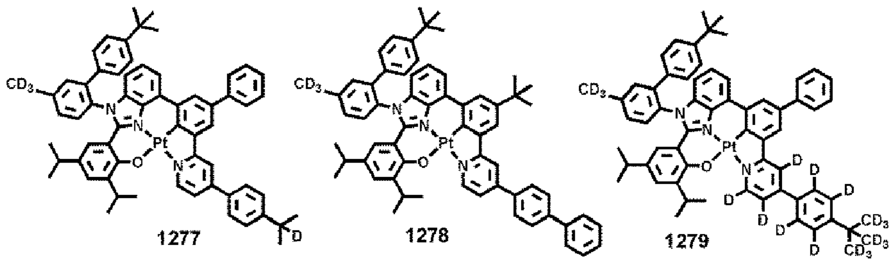

1277 1278 1279

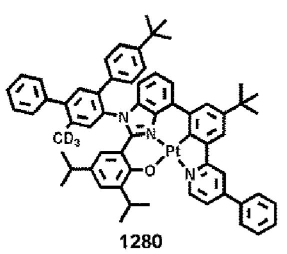

1280

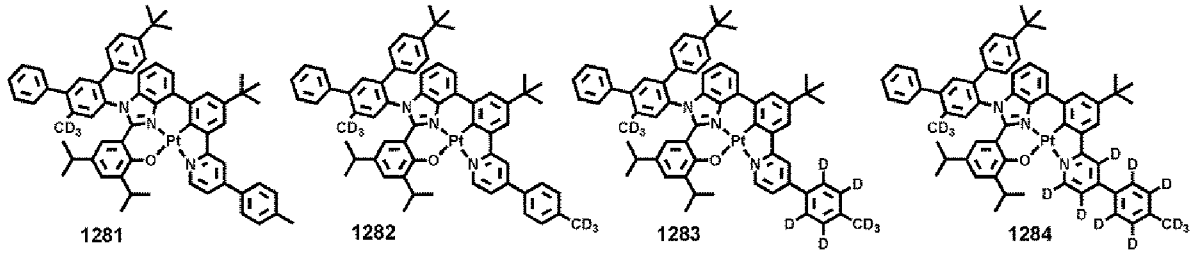

1281 1282 1283 1284

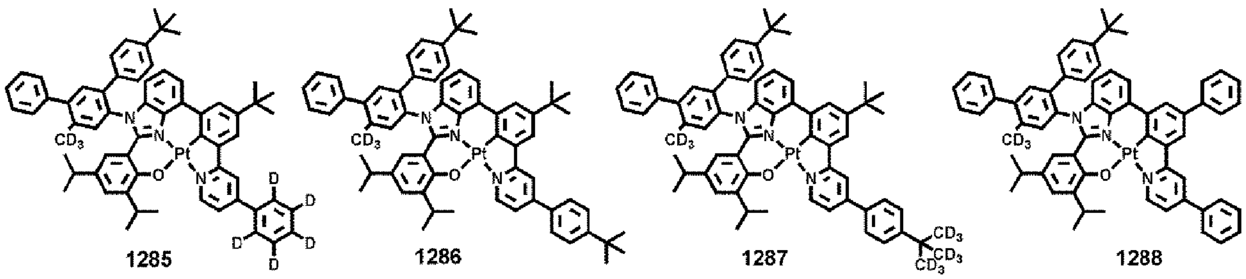

1285 1286 1287 1288

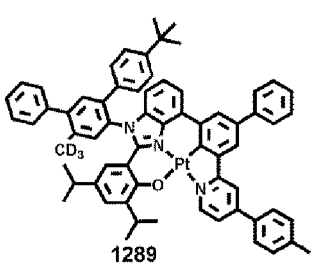

1289

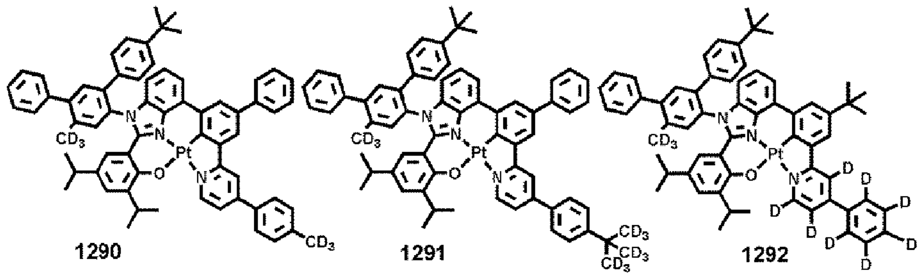

1290 1291 1292

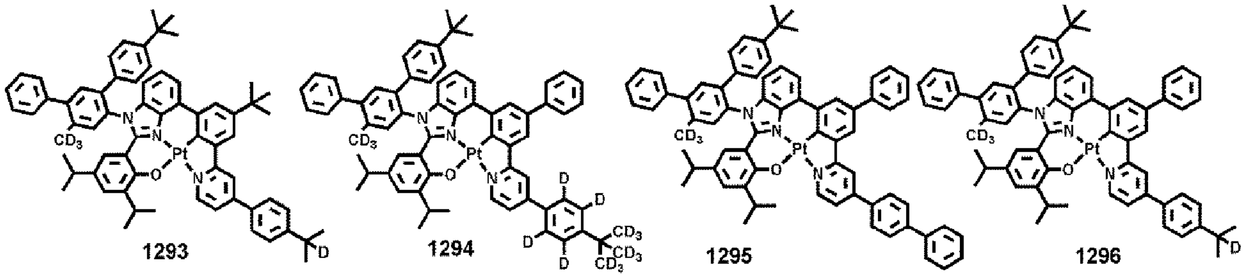

1293 1294 1295 1296

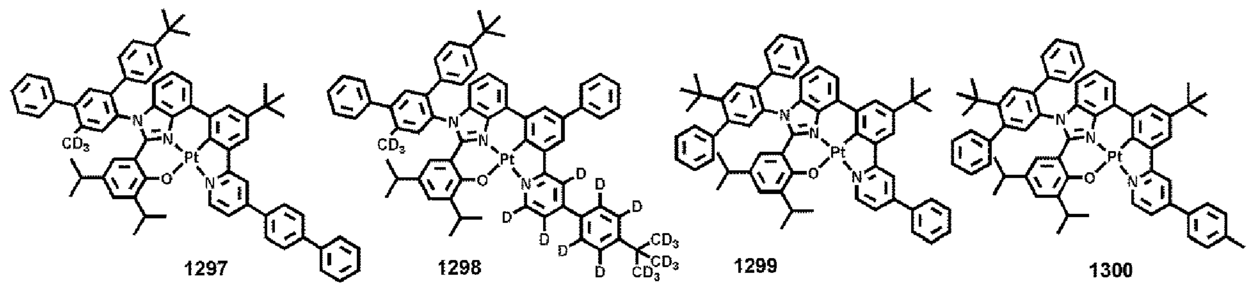

1297 1298 1299 1300

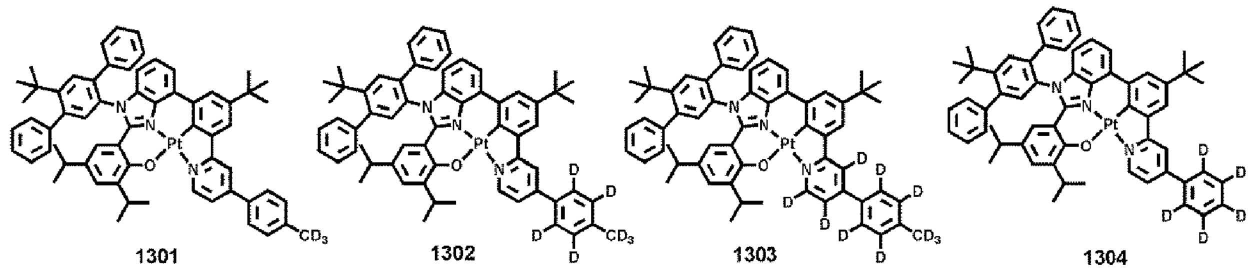

1301 1302 1303 1304

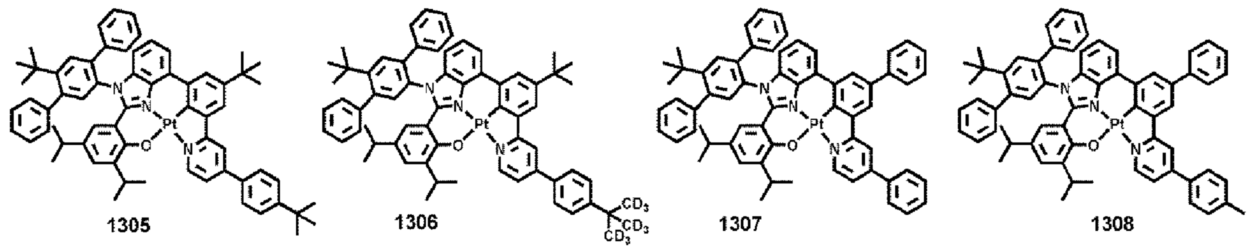

1305 1306 1307 1308

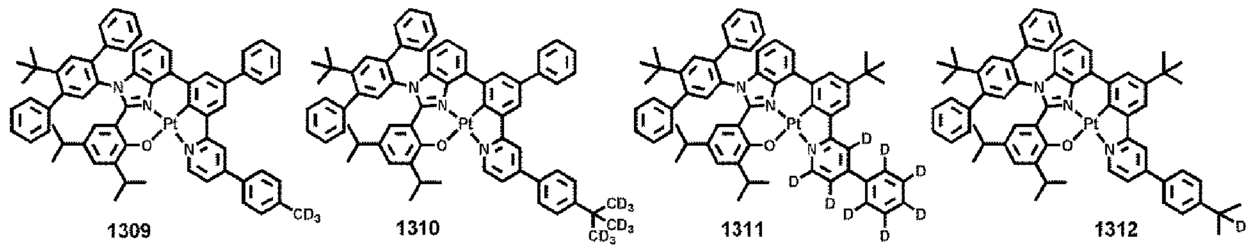

1309 1310 1311 1312

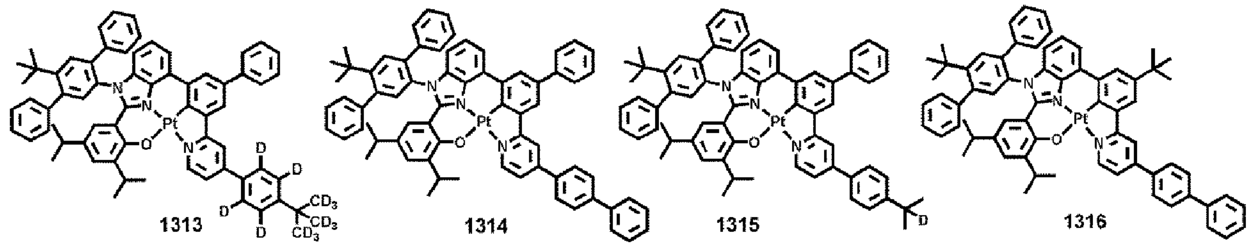

1313 1314 1315 1316

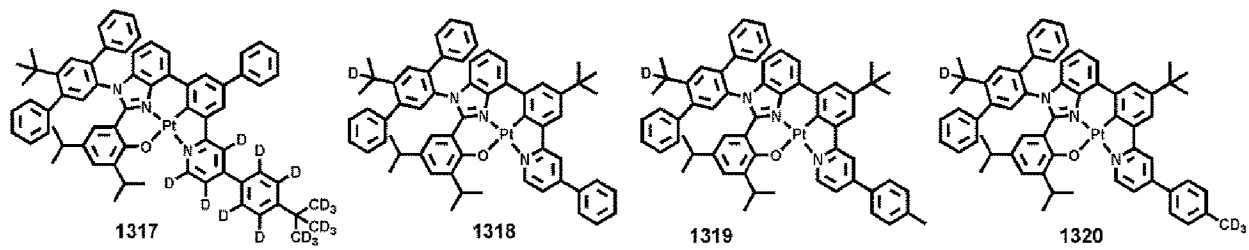

1317 1318 1319 1320

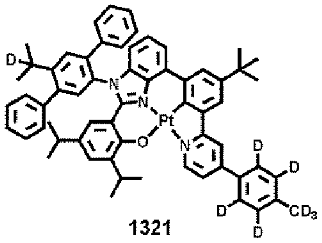
1321

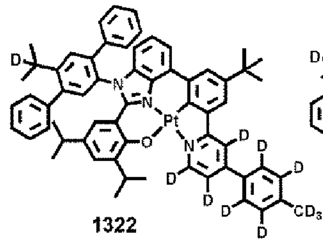
1322

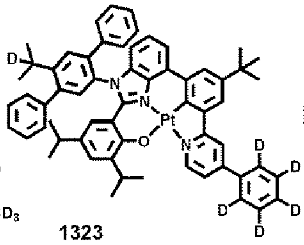
1323

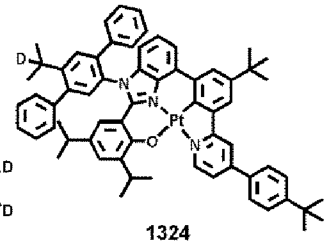
1324

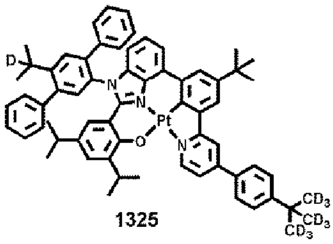
1325

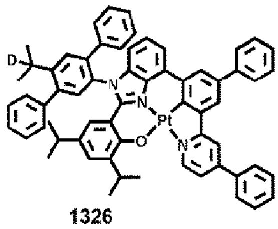
1326

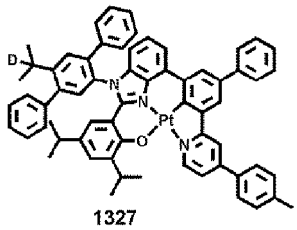
1327

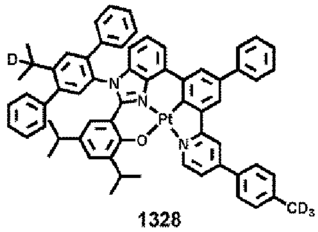
1328

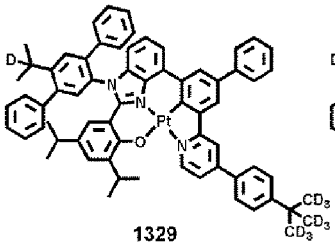
1329

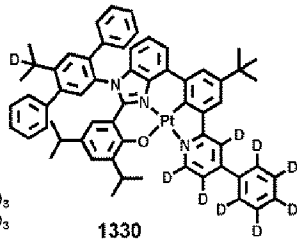
1330

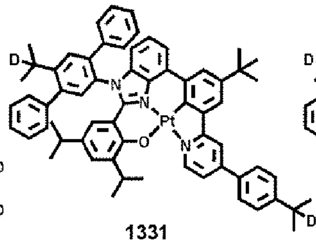
1331

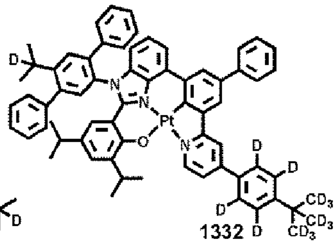
1332

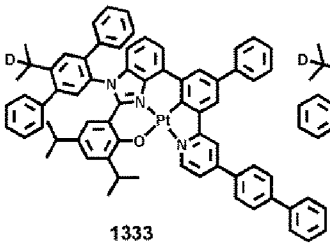
1333

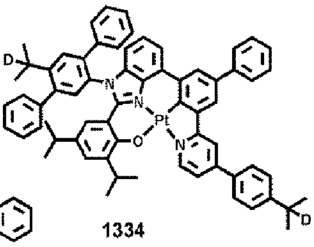
1334

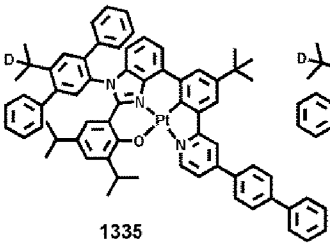
1335

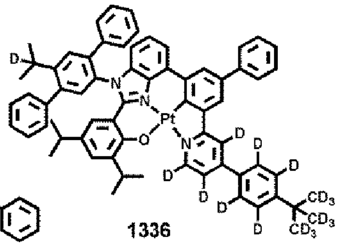
1336

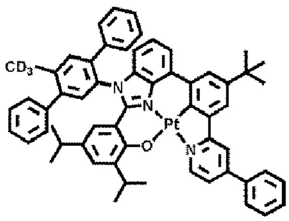
1337

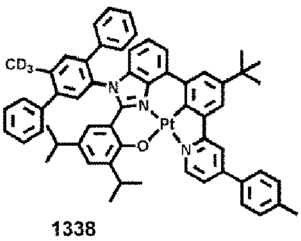
1338

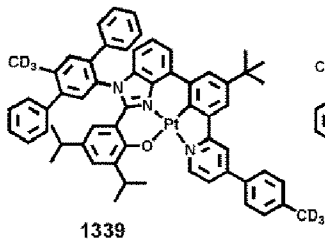
1339

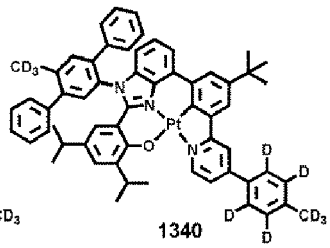
1340

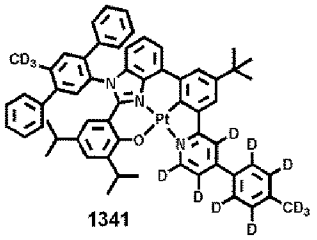
1341

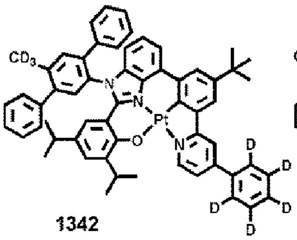
1342

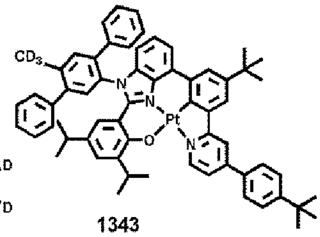
1343

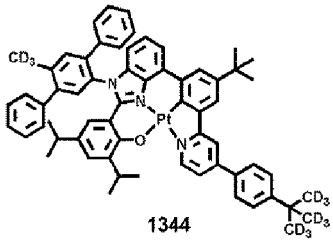
1344

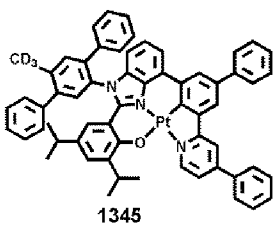
1345

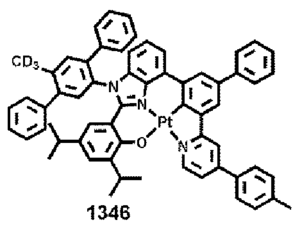
1346

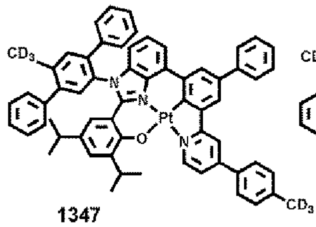
1347

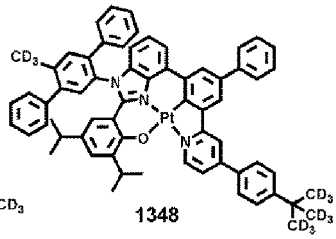
1348

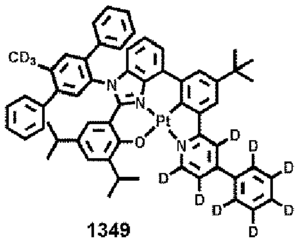
1349

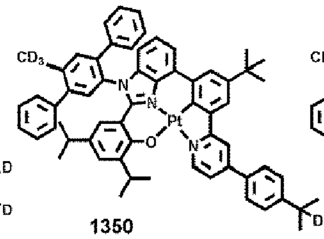
1350

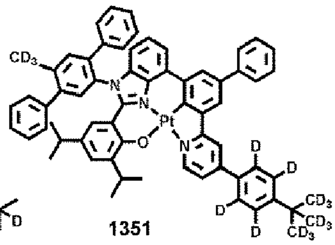
1351

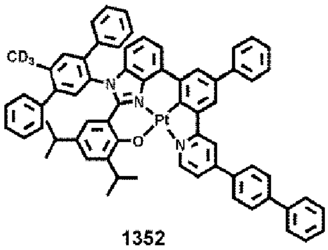
1352

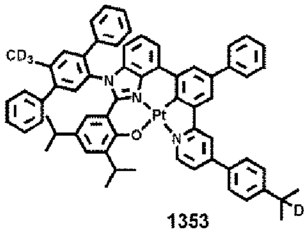
1353

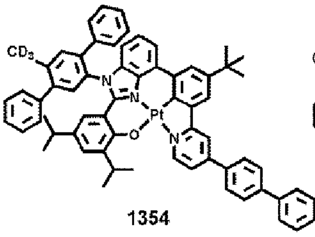
1354

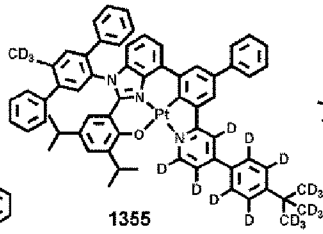
1355

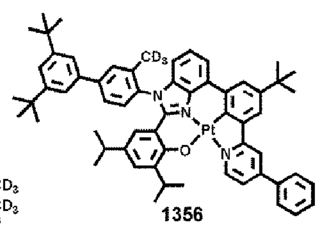
1356

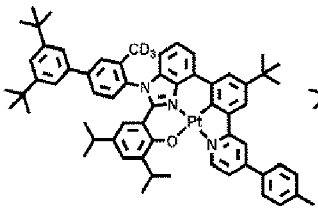
1357

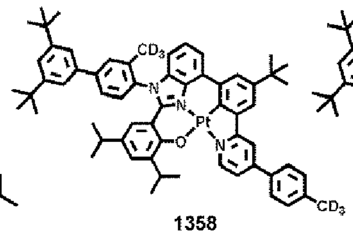
1358

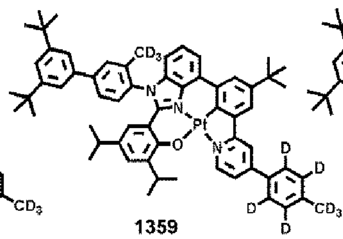
1359

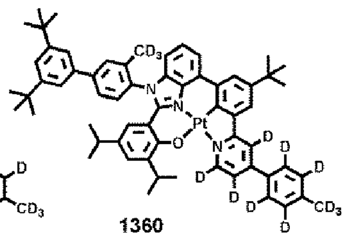
1360

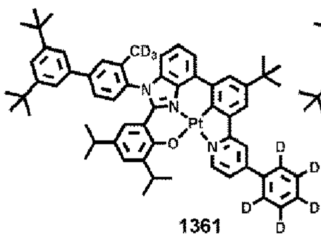
1361

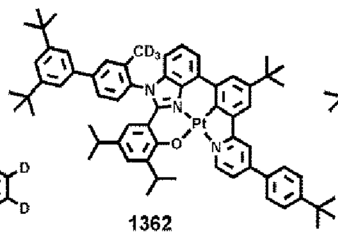
1362

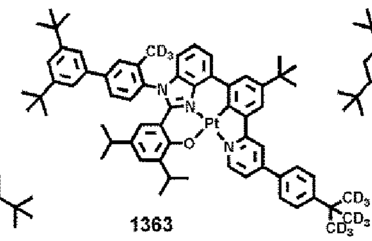
1363

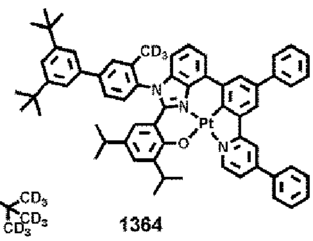
1364

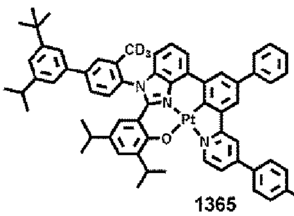
1365

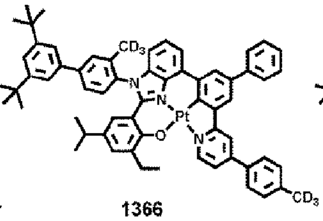
1366

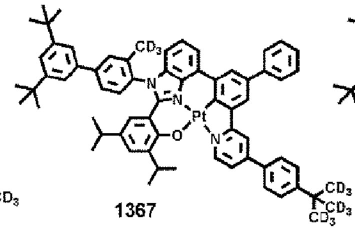
1367

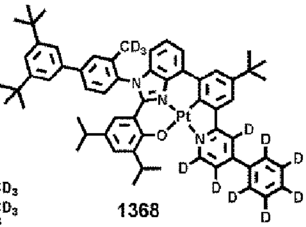
1368

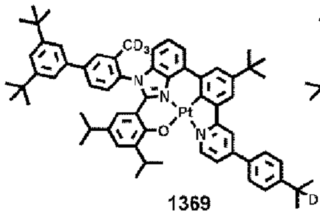
1369

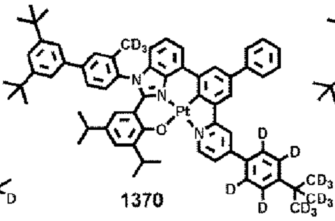
1370

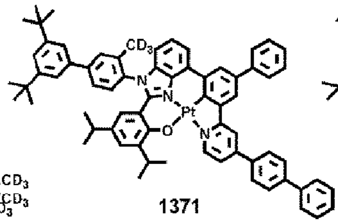
1371

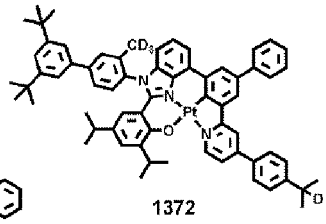
1372

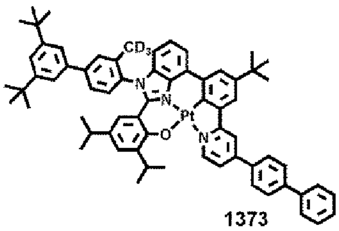
1373

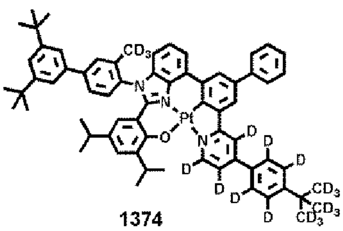
1374

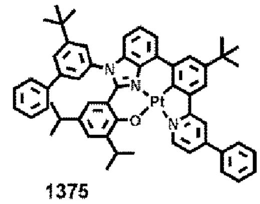
1375

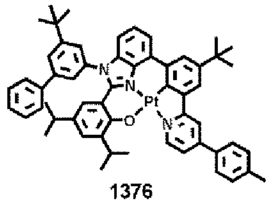
1376

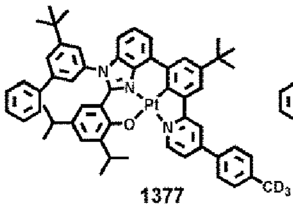
1377

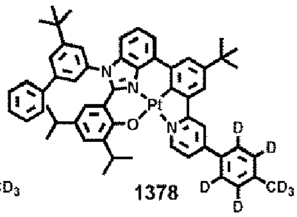
1378

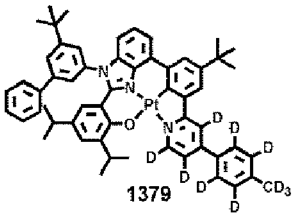
1379

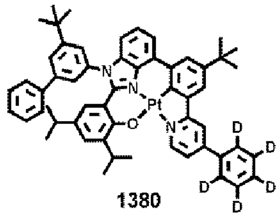
1380

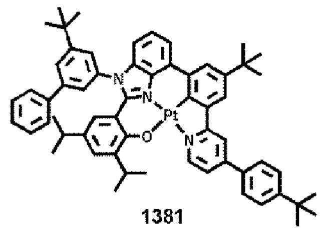
1381

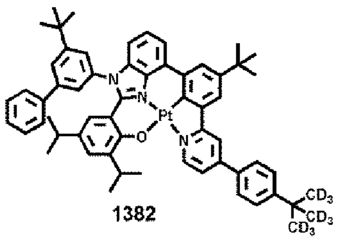
1382

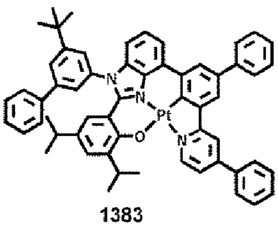
1383

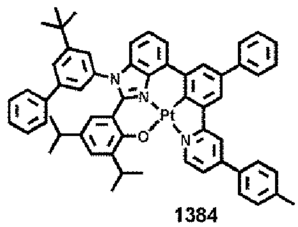
1384

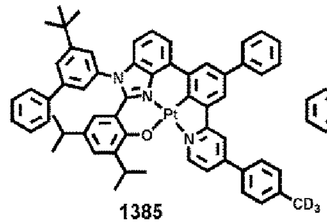
1385

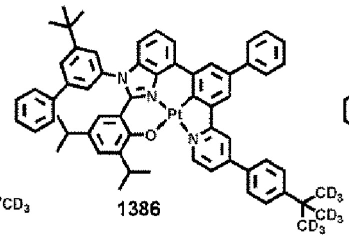
1386

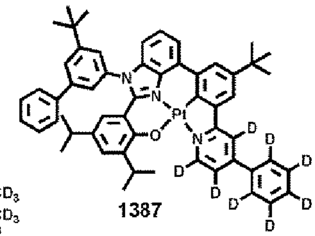
1387

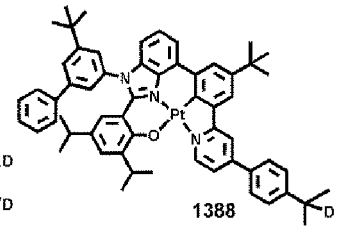
1388

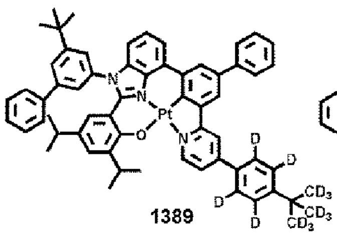
1389

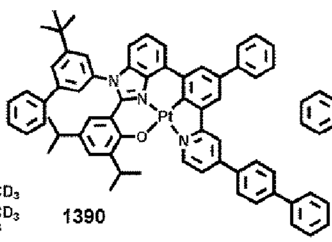
1390

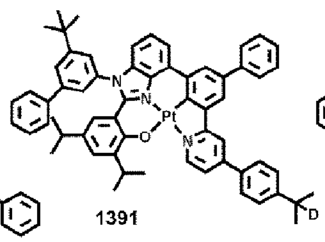
1391

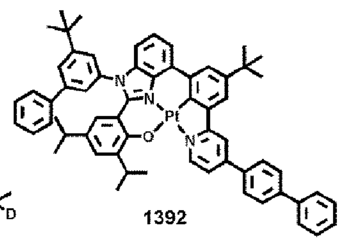
1392

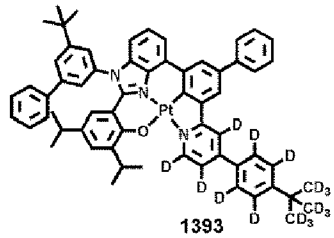
1393

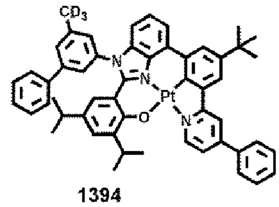
1394

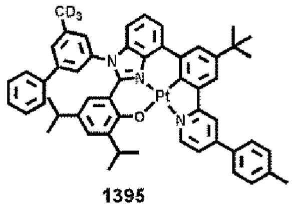
1395

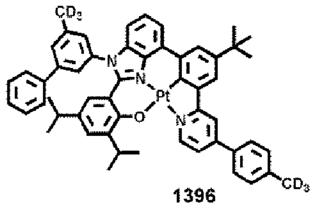
1396

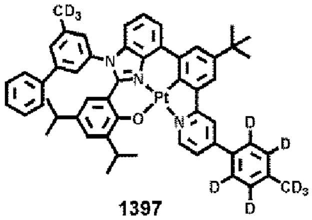
1397

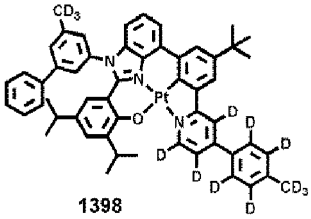
1398

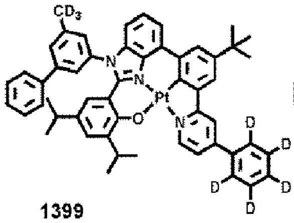
1399

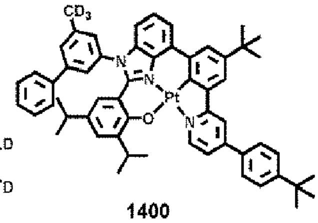
1400

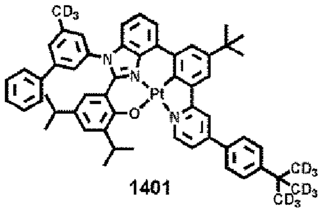
1401

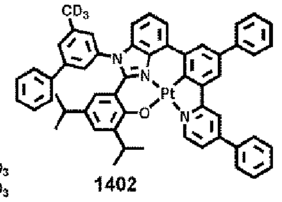
1402

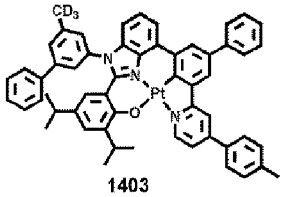
1403

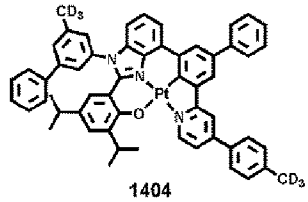
1404

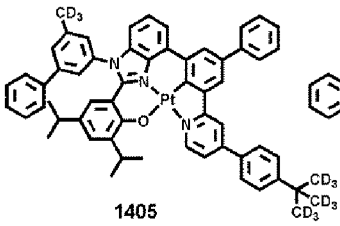
1405

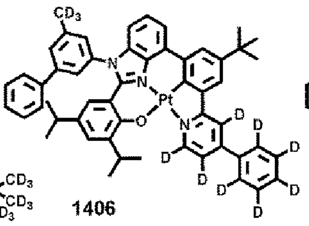
1406

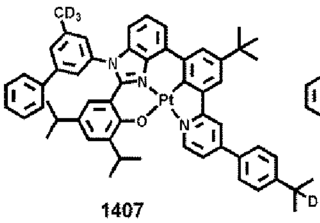
1407

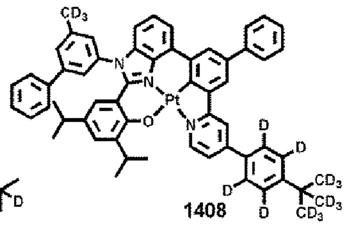
1408

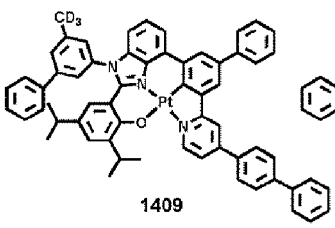
1409

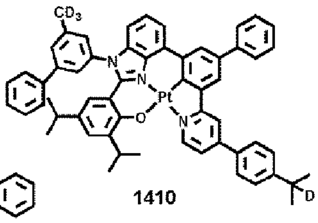
1410

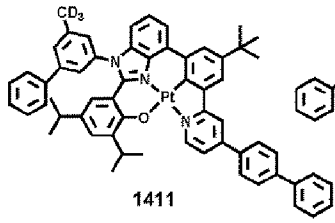
1411

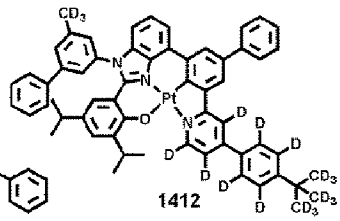
1412

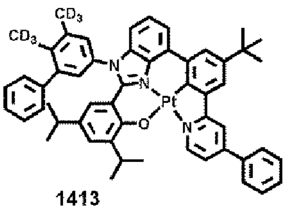
1413

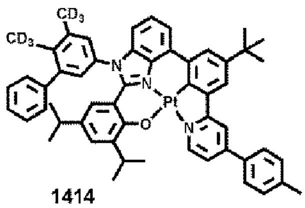
1414

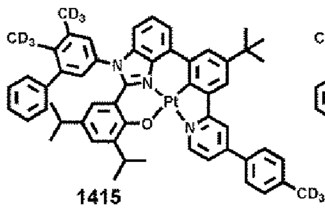
1415

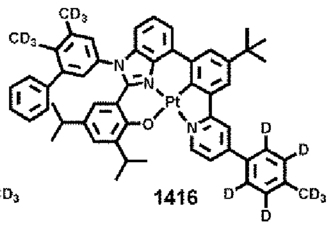
1416

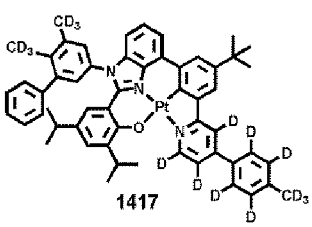
1417

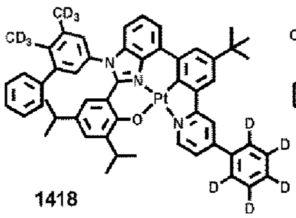
1418

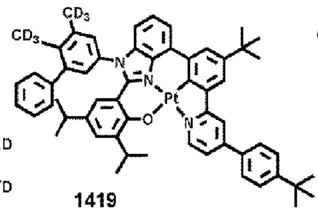
1419

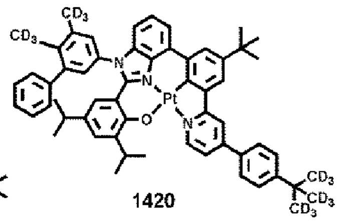
1420

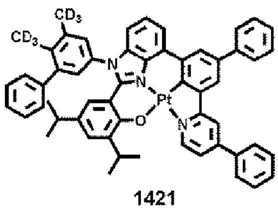
1421

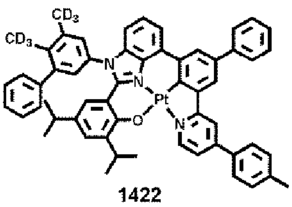
1422

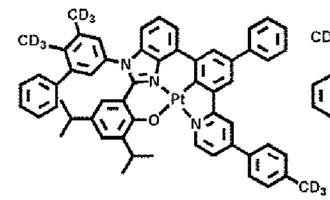
1423

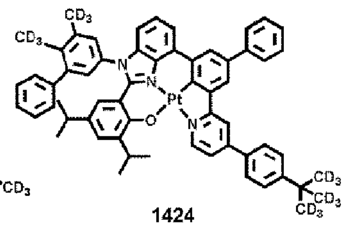
1424

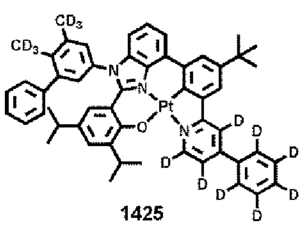
1425

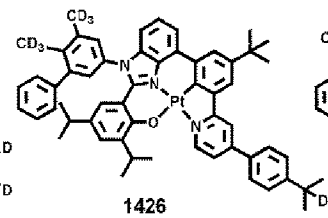
1426

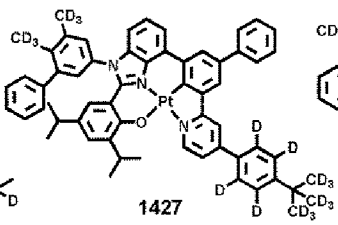
1427

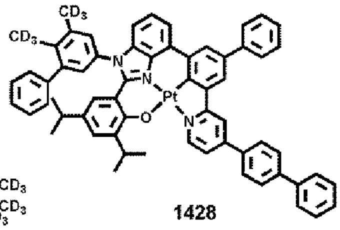
1428

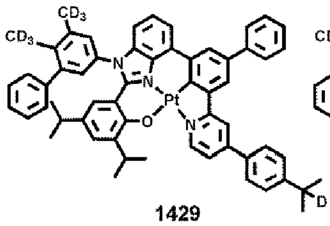
1429

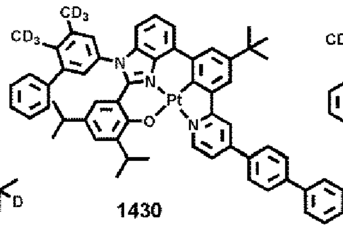
1430

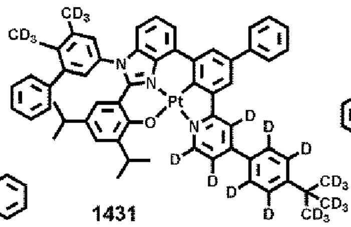
1431

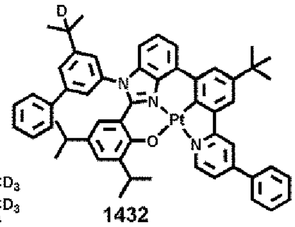
1432

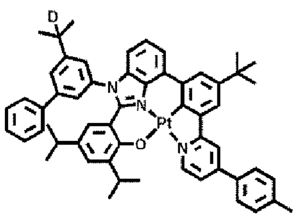
1433

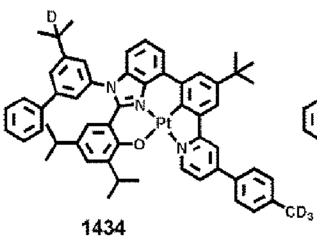
1434

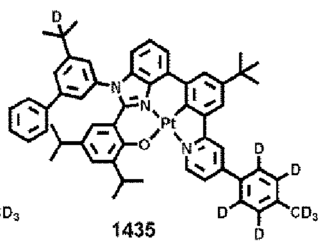
1435

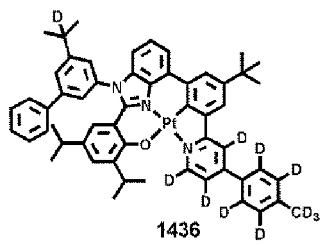
1436

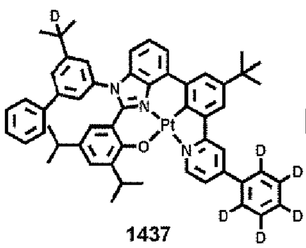
1437

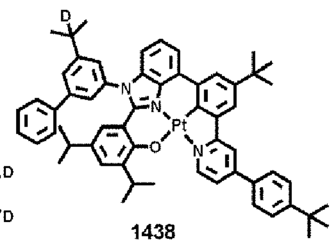
1438

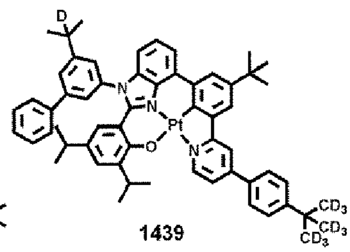
1439

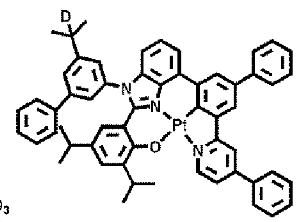
1440

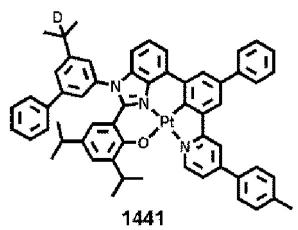
1441

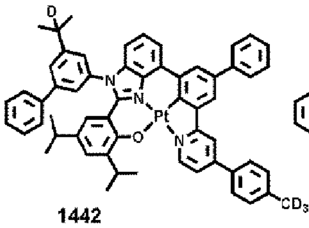
1442

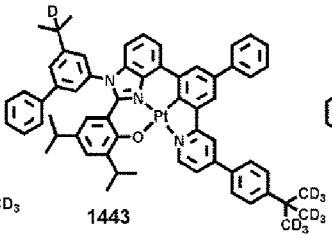
1443

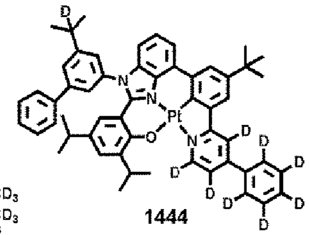
1444

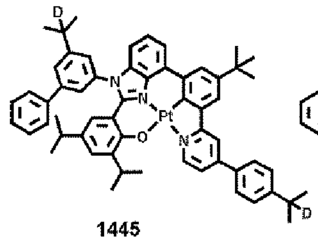
1445

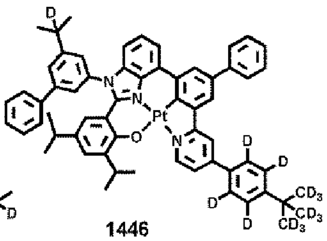
1446

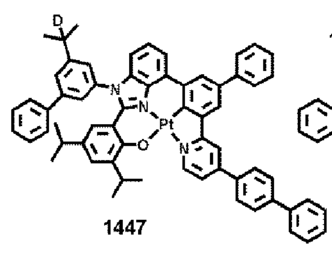
1447

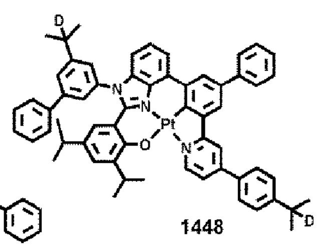
1448

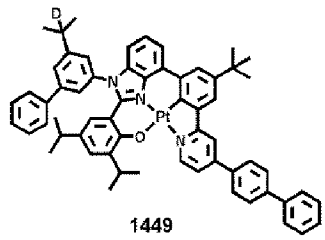
1449

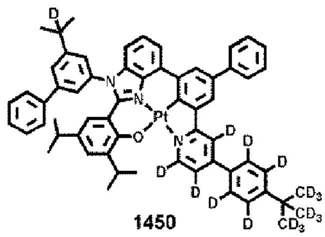
1450

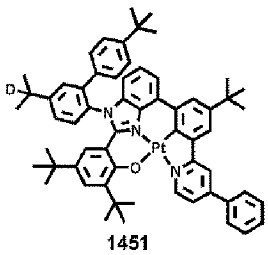
1451

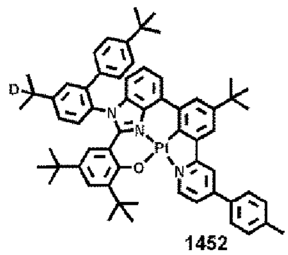
1452

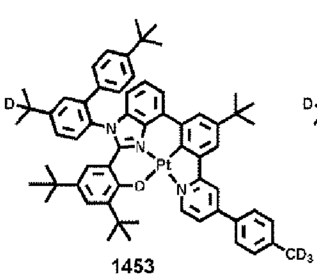
1453

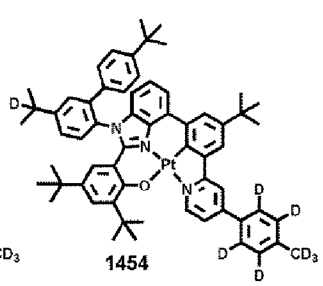
1454

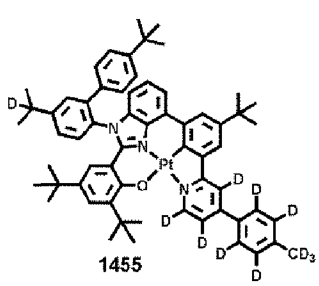
1455

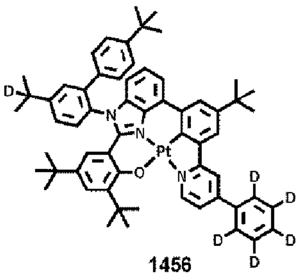
1456

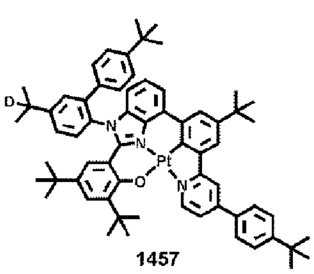
1457

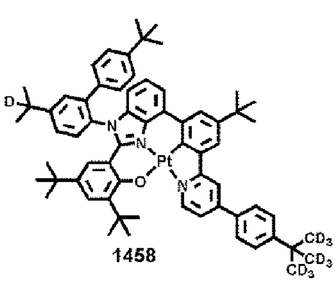
1458

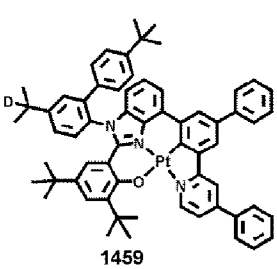
1459

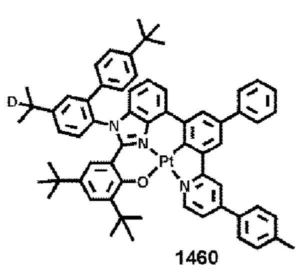
1460

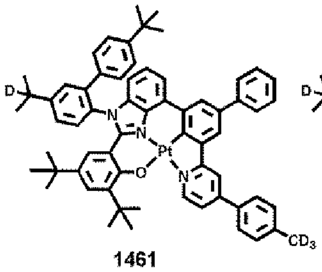
1461

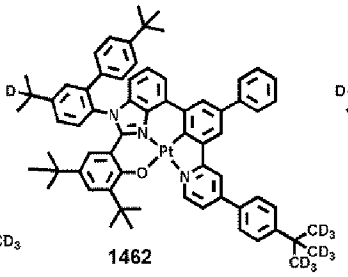
1462

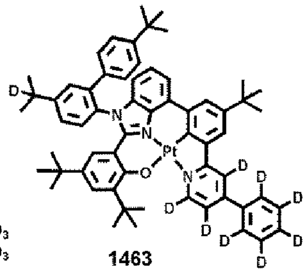
1463

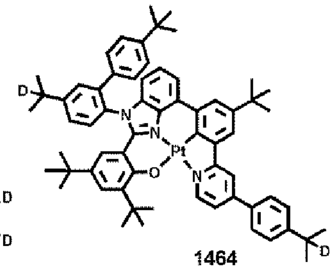
1464

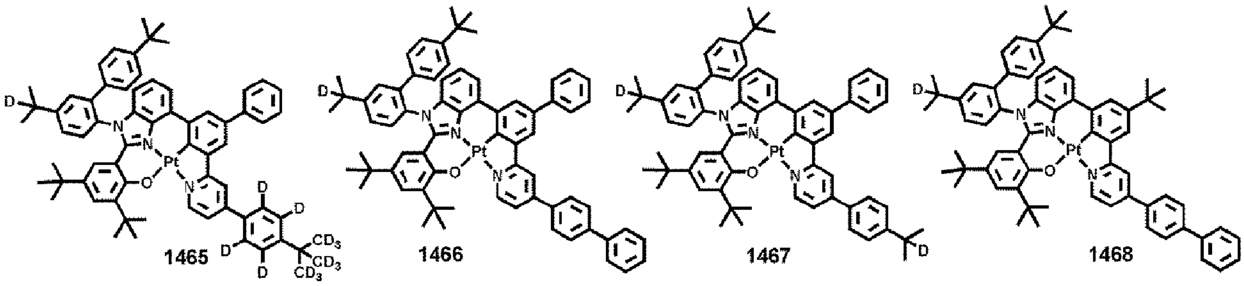

1465 1466 1467 1468

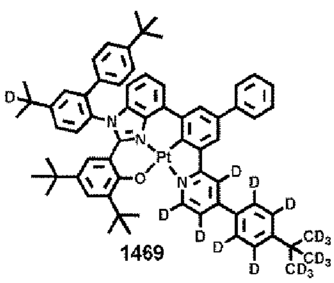

1469

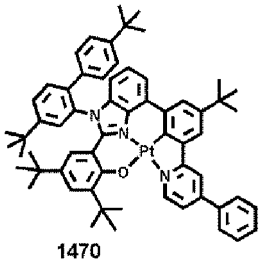

1470

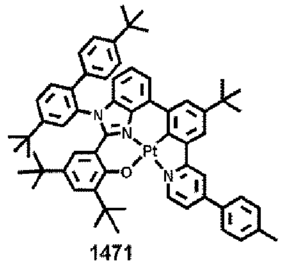

1471

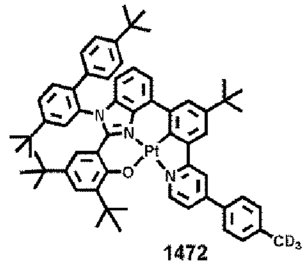

1472

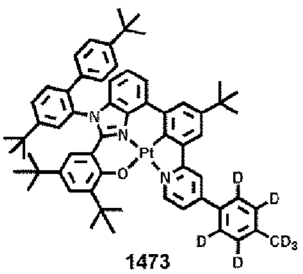

1473

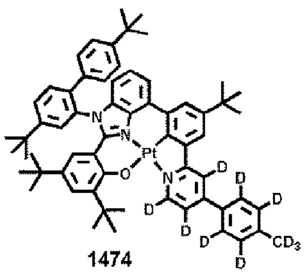

1474

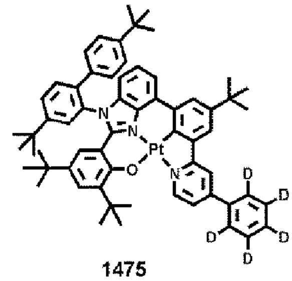

1475

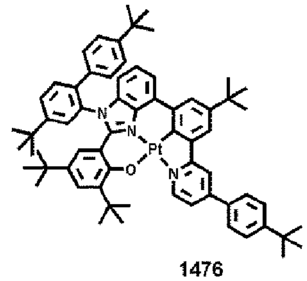

1476

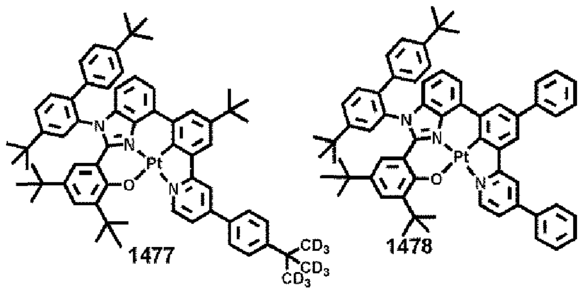

1477 1478

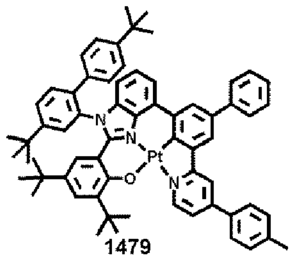

1479

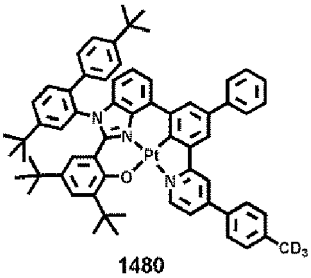

1480

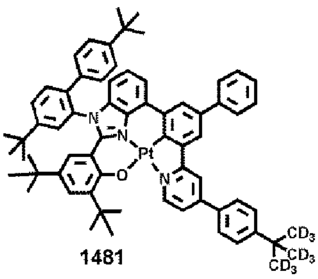

1481

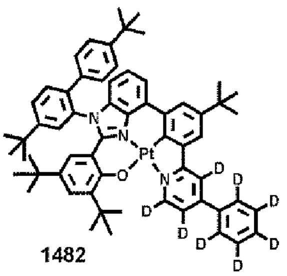

1482

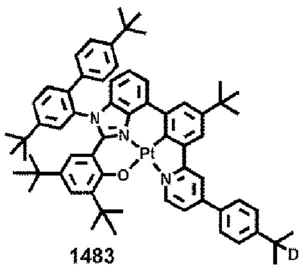

1483

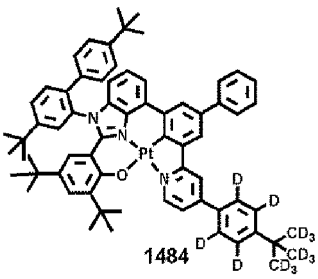

1484

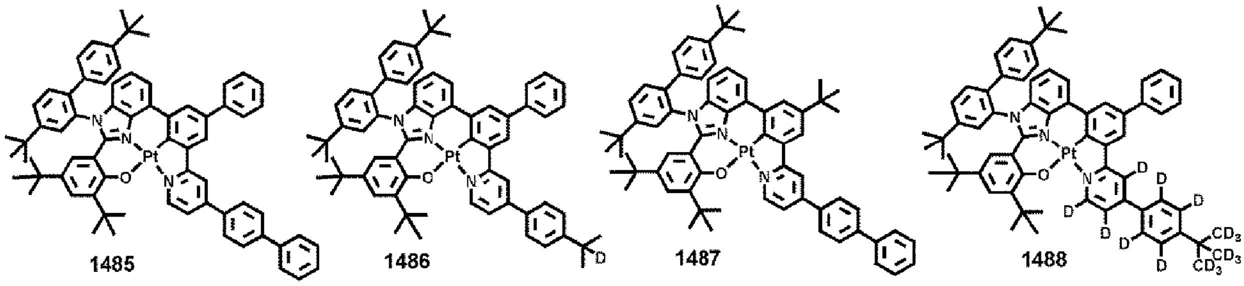

1485 1486 1487 1488

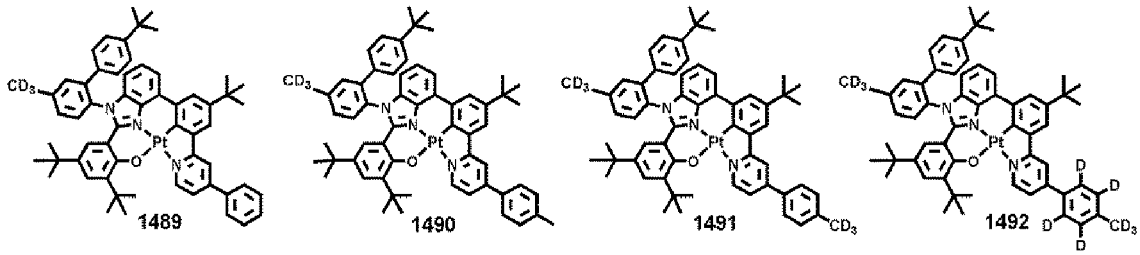

1489 1490 1491 1492

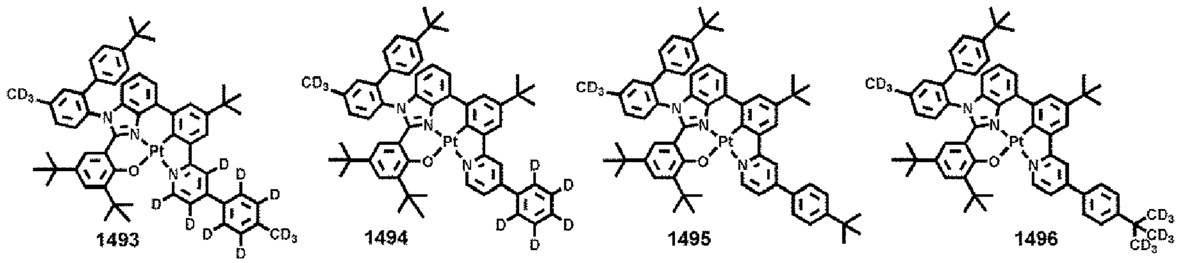

1493 1494 1495 1496

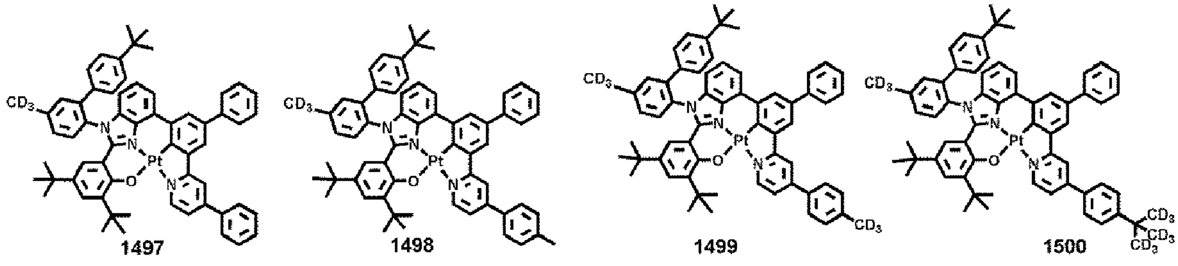

1497 1498 1499 1500

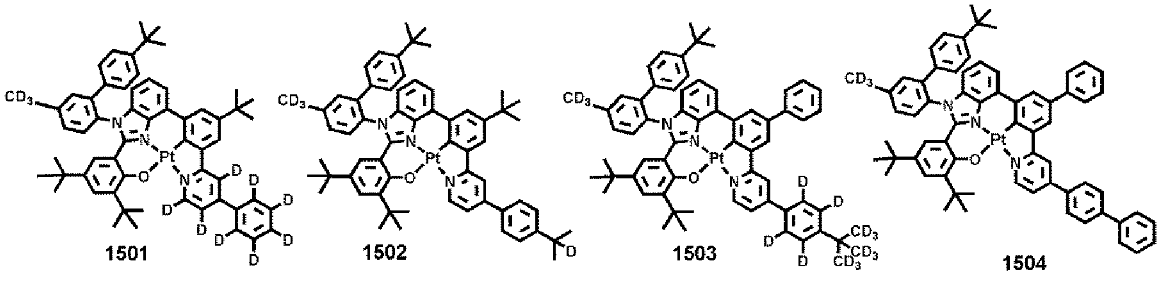

1501 1502 1503 1504

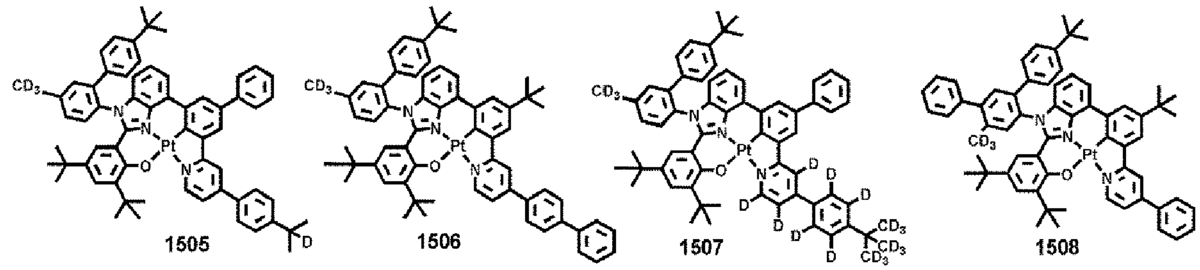

1505 1506 1507 1508

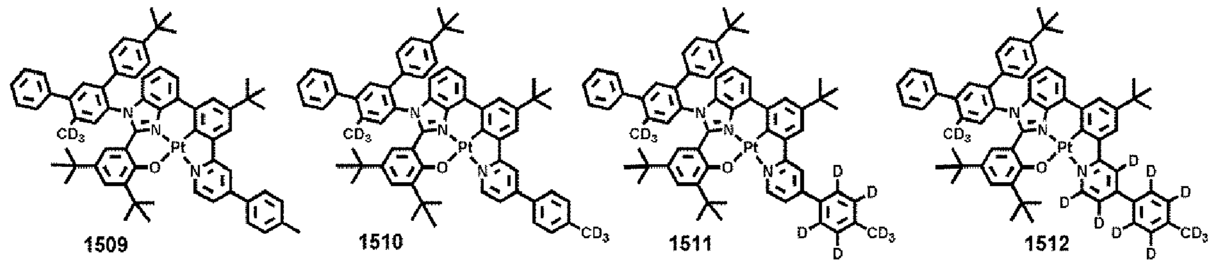

1509 1510 1511 1512

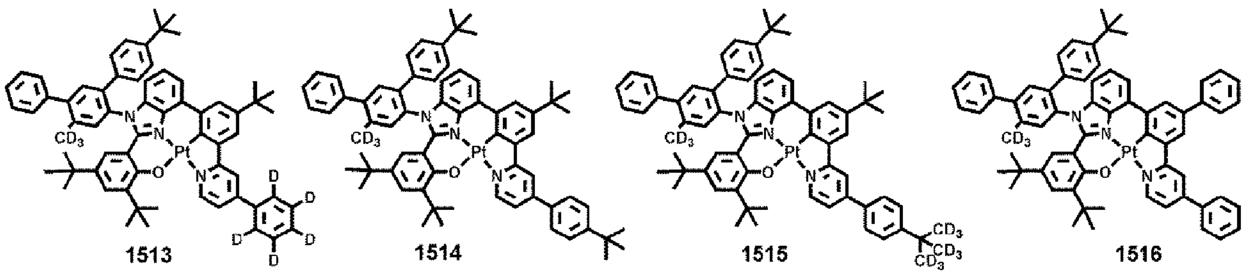

1513 1514 1515 1516

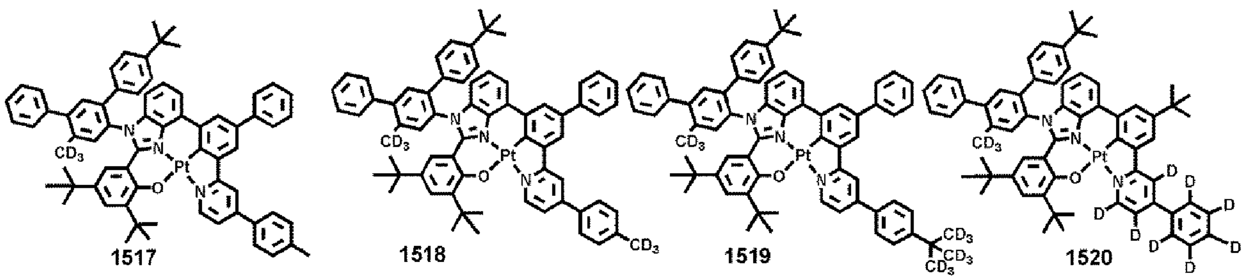

1517 1518 1519 1520

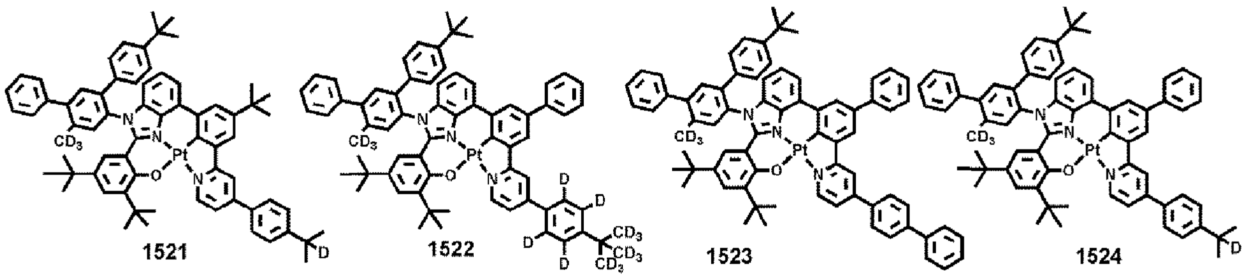

1521 1522 1523 1524

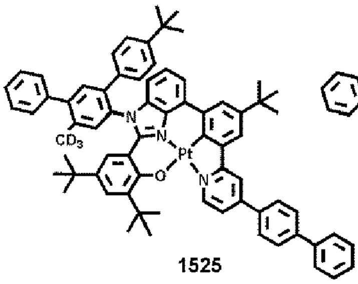
1525

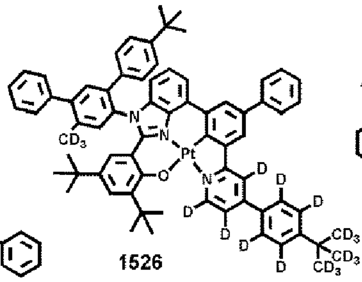
1526

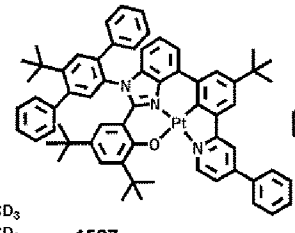
1527

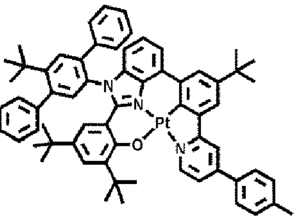
1528

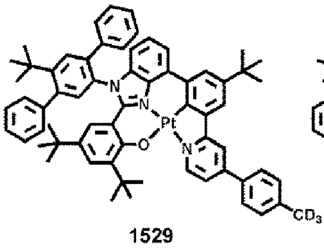
1529

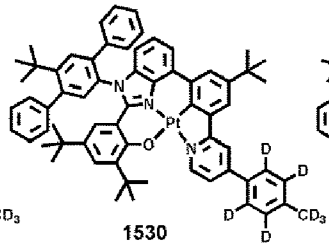
1530

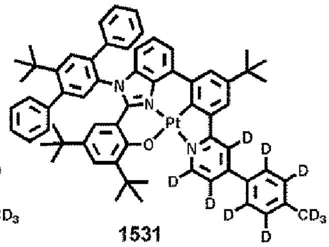
1531

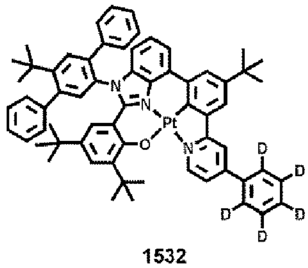
1532

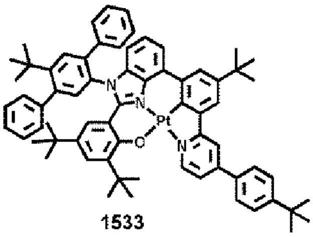
1533

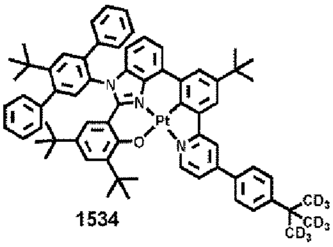
1534

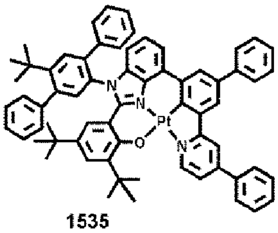
1535

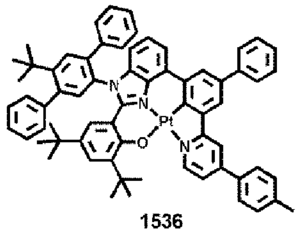
1536

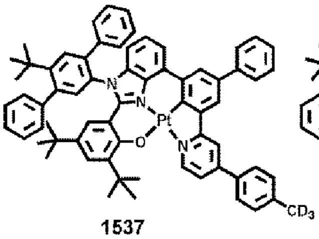
1537

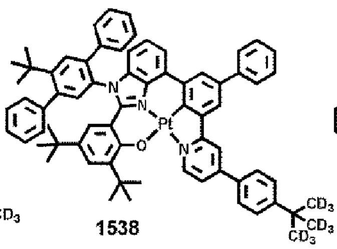
1538

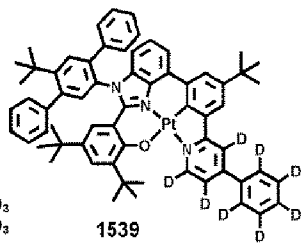
1539

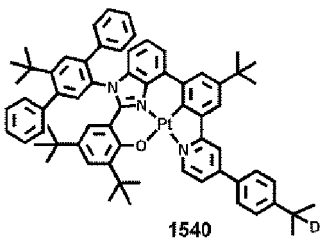
1540

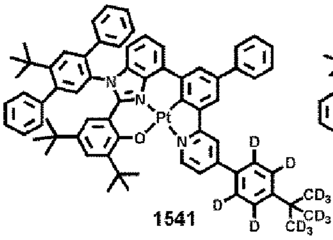
1541

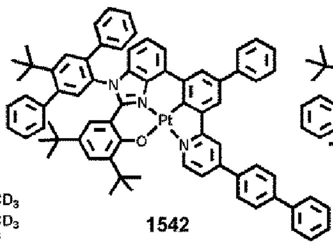
1542

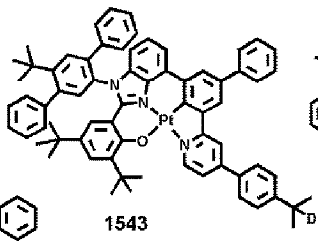
1543

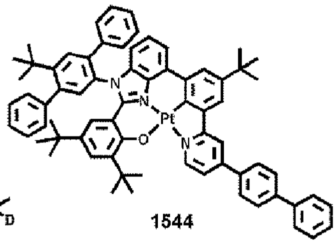
1544

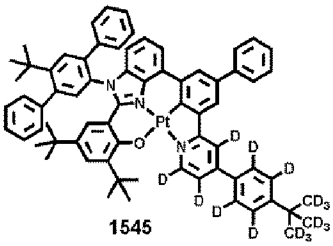
1545

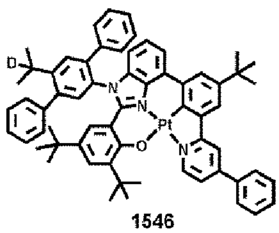
1546

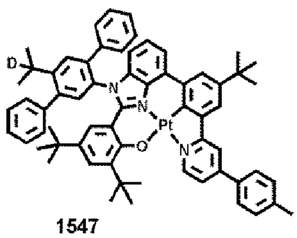
1547

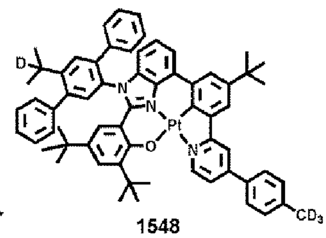
1548

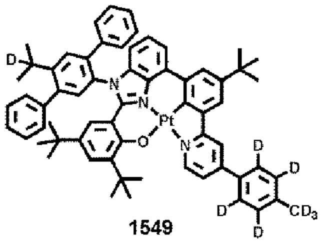
1549

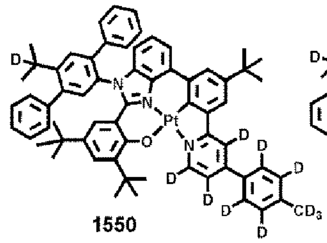
1550

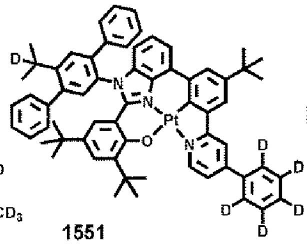
1551

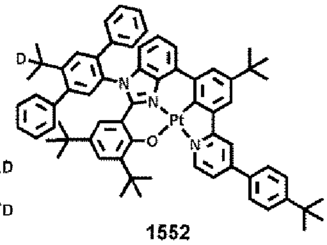
1552

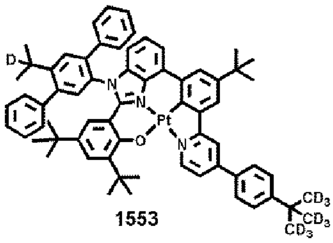
1553

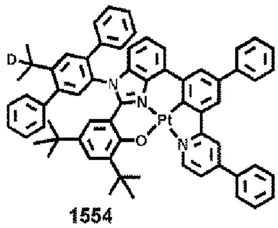
1554

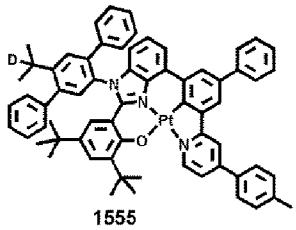
1555

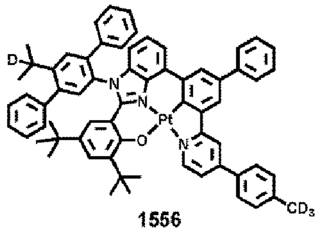
1556

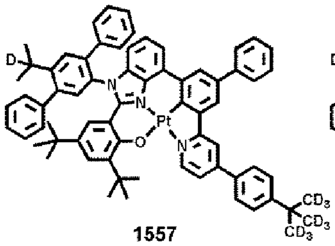
1557

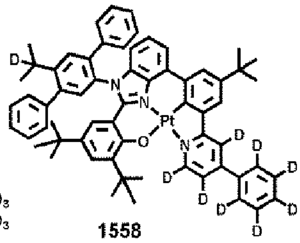
1558

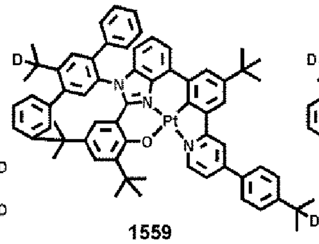
1559

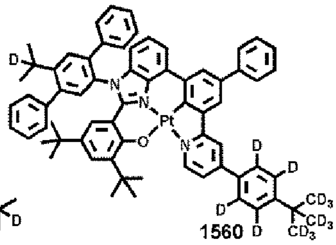
1560

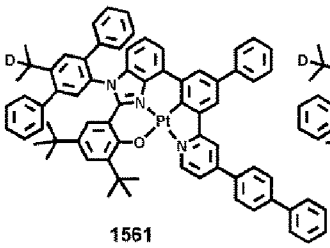
1561

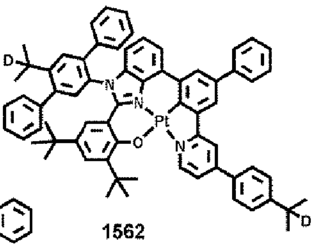
1562

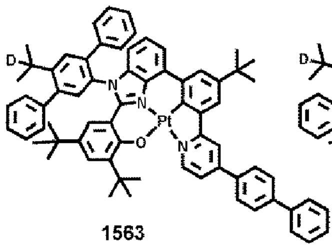
1563

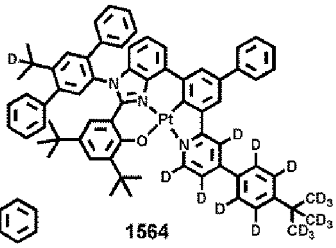
1564

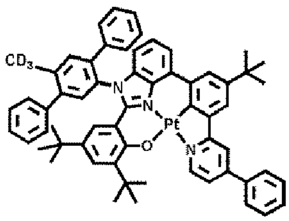
1565

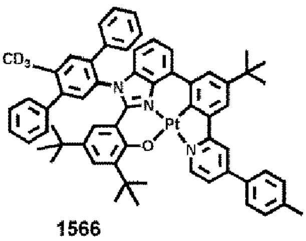
1566

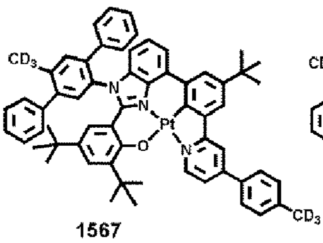
1567

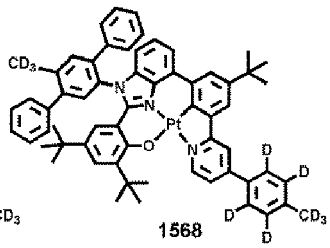
1568

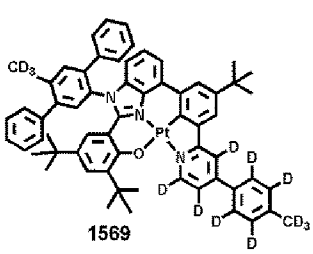
1569

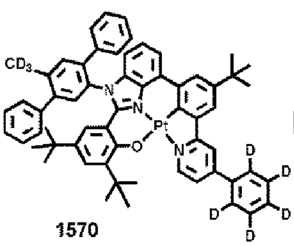
1570

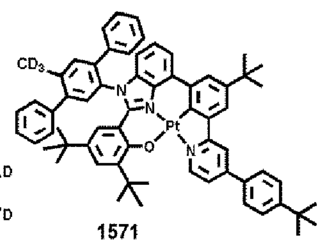
1571

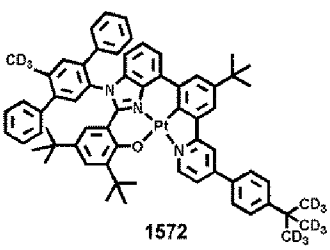
1572

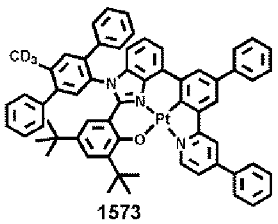

1573

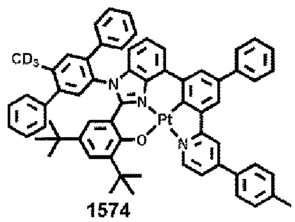

1574

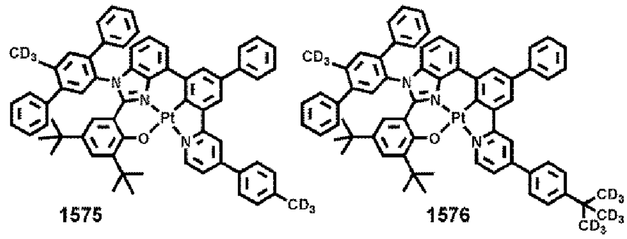

1575

1576

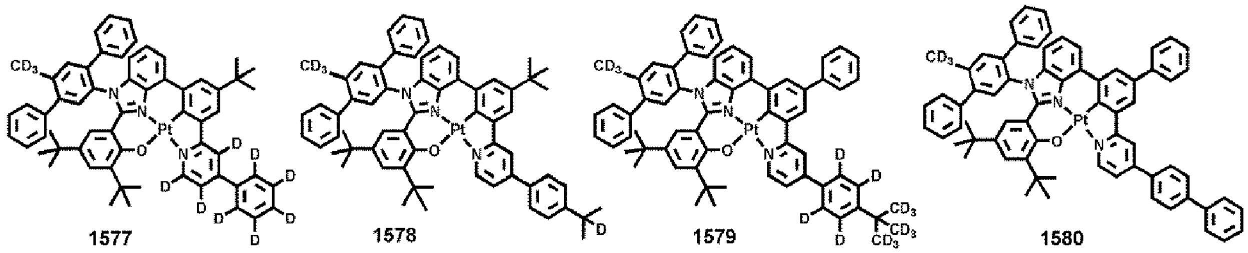

1577

1578

1579

1580

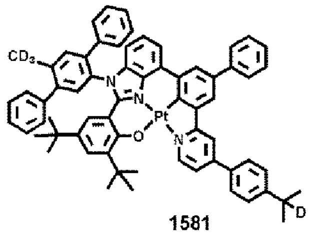

1581

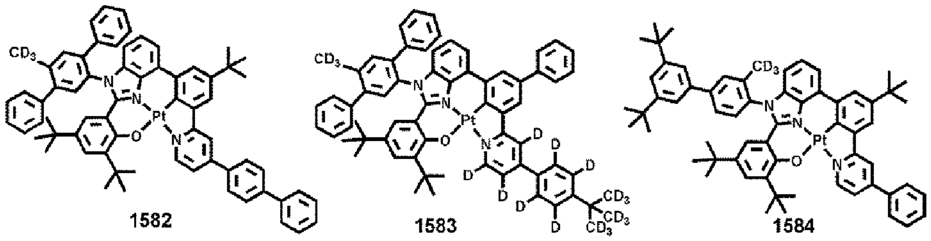

1582

1583

1584

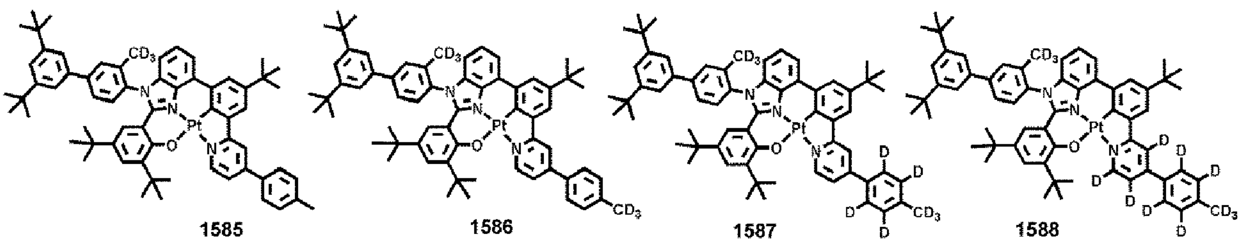

1585

1586

1587

1588

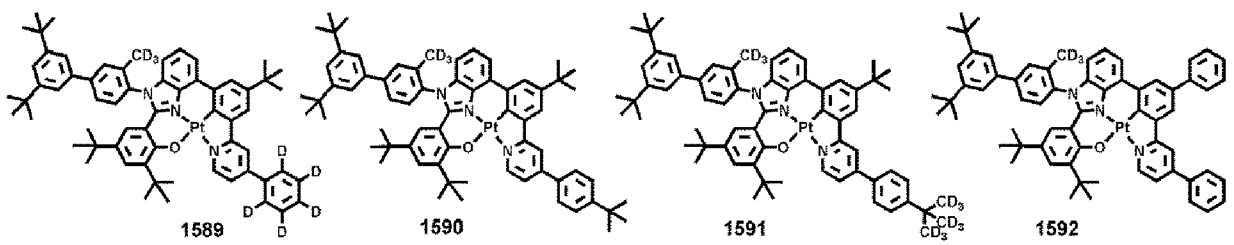

1589

1590

1591

1592

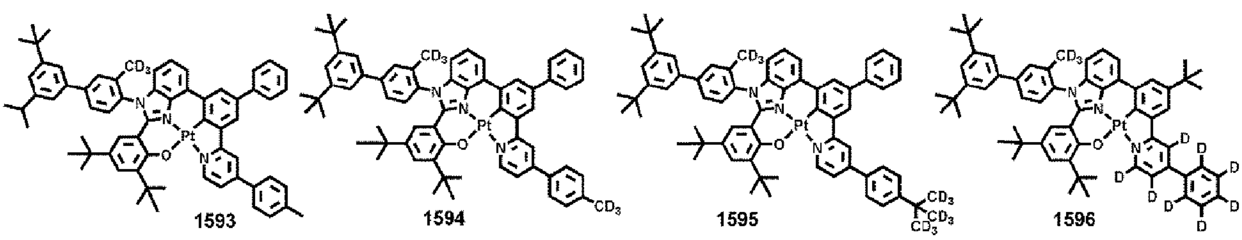

1593

1594

1595

1596

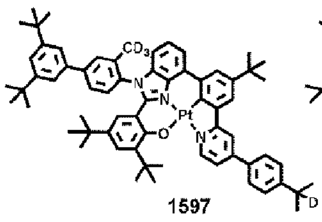
1597
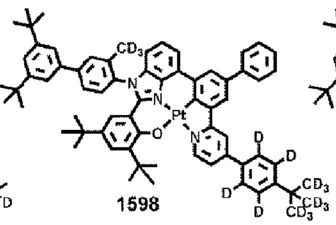
1598
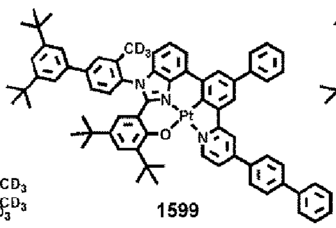
1599
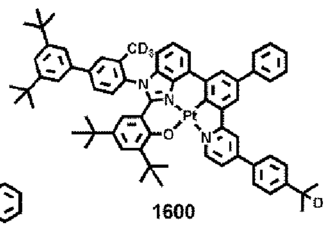
1600

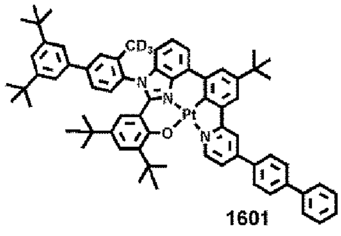
1601
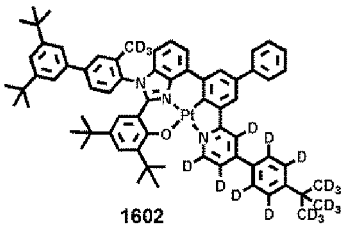
1602
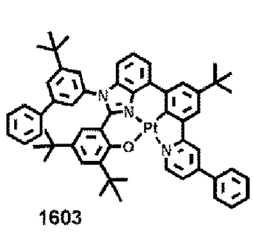
1603
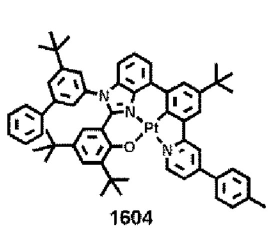
1604

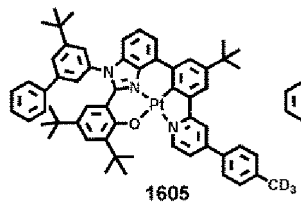
1605
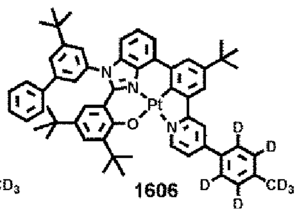
1606
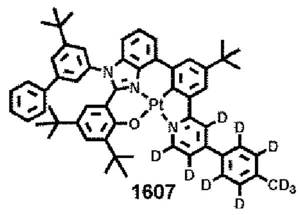
1607
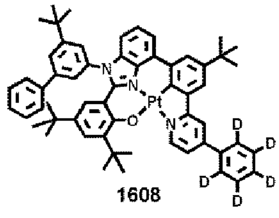
1608

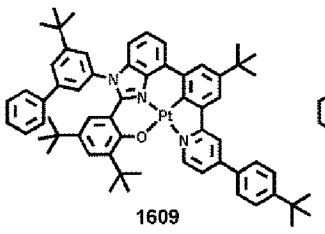
1609
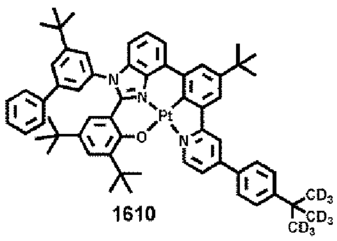
1610
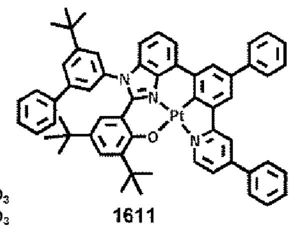
1611
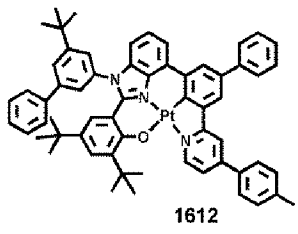
1612

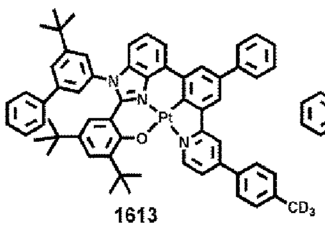
1613
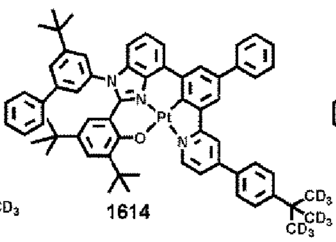
1614
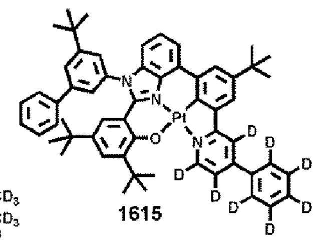
1615
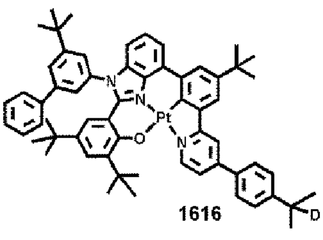
1616

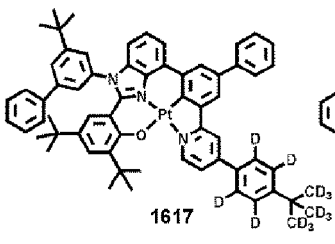
1617
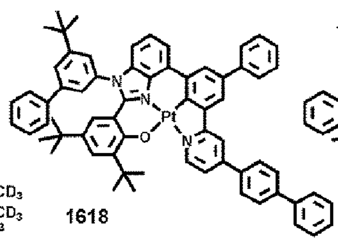
1618
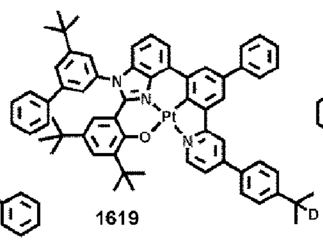
1619
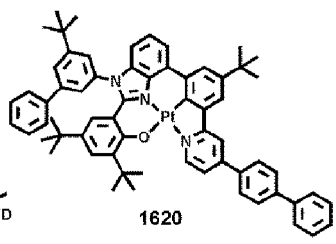
1620

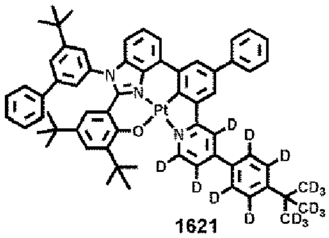
1621

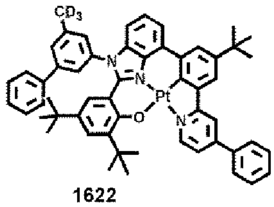
1622

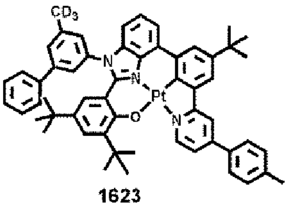
1623

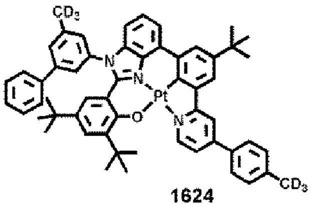
1624

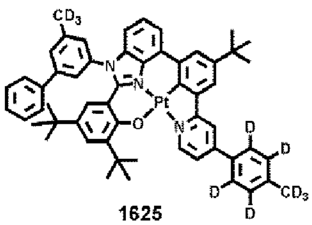
1625

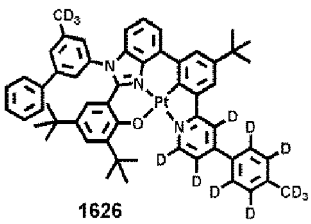
1626

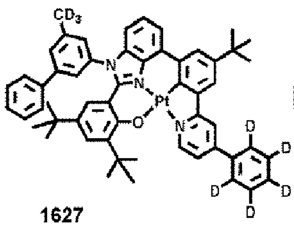
1627

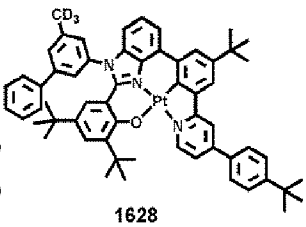
1628

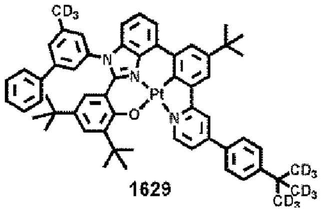
1629

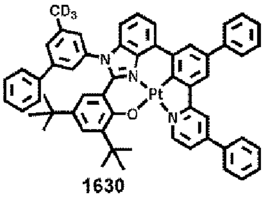
1630

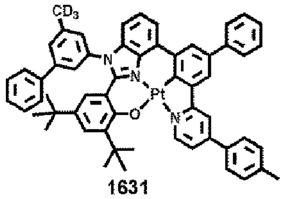
1631

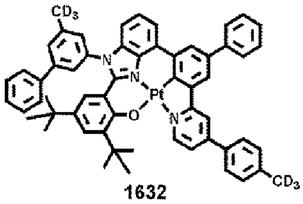
1632

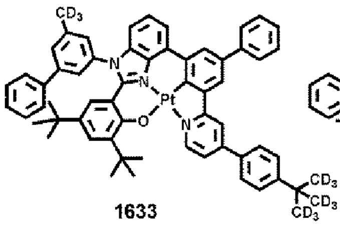
1633

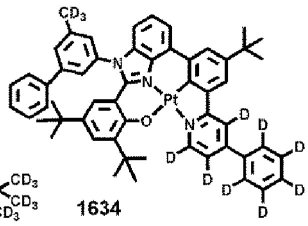
1634

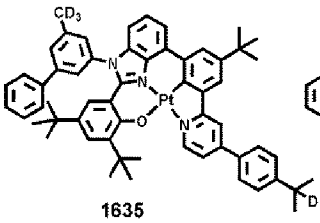
1635

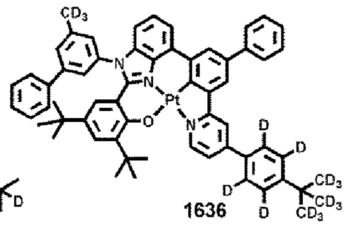
1636

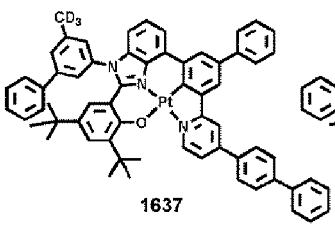
1637

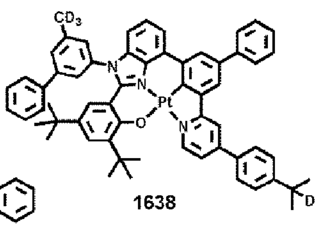
1638

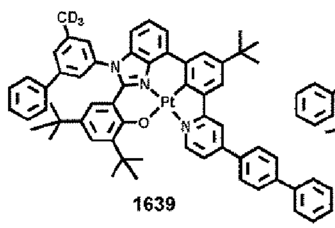
1639

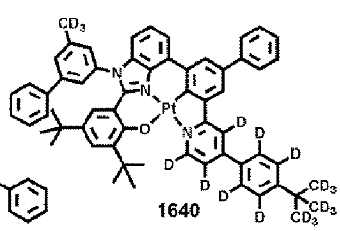
1640

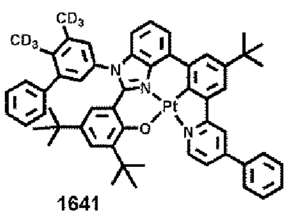
1641

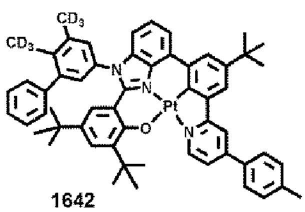
1642

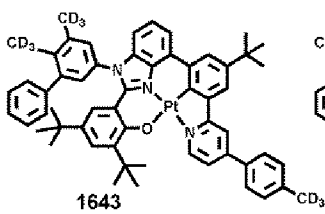
1643

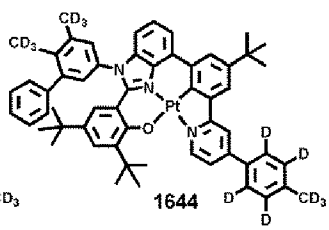
1644

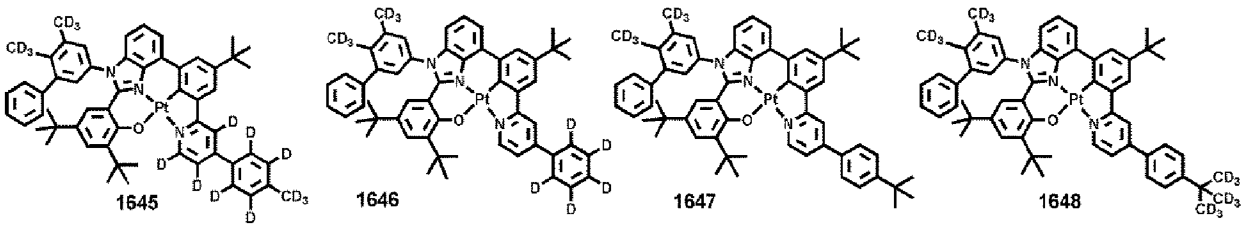

1645 1646 1647 1648

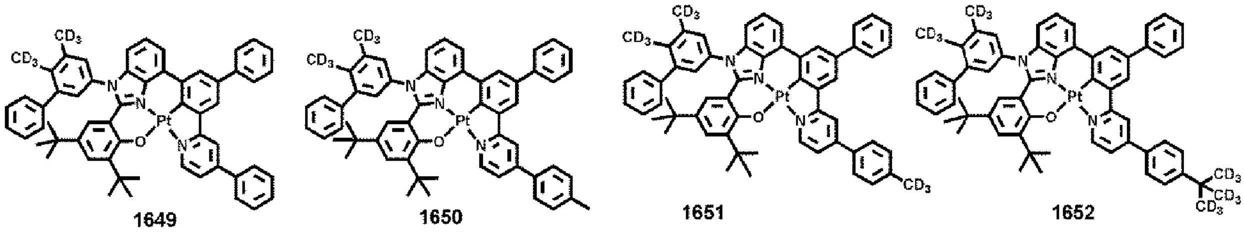

1649 1650 1651 1652

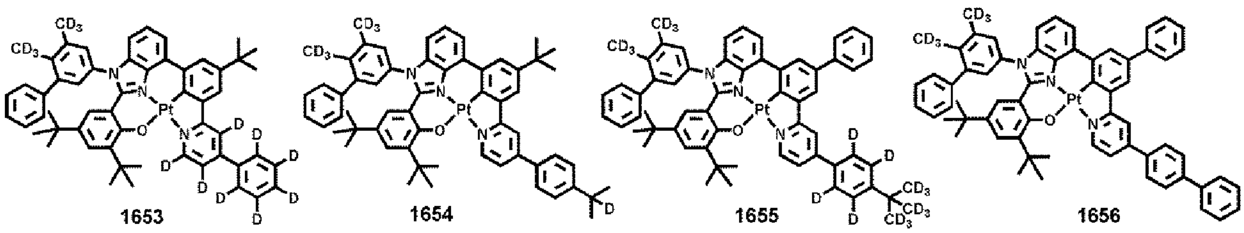

1653 1654 1655 1656

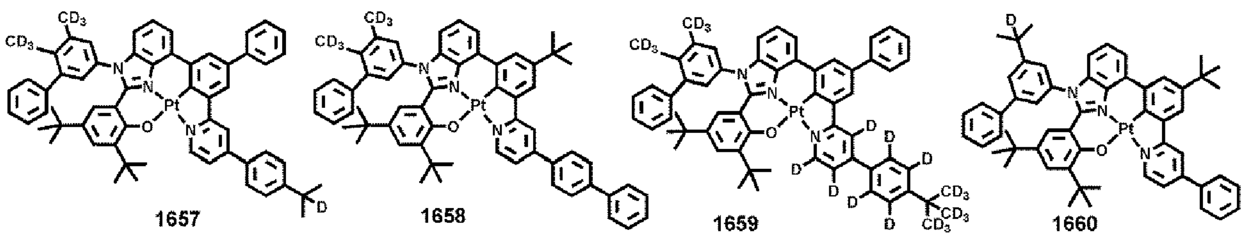

1657 1658 1659 1660

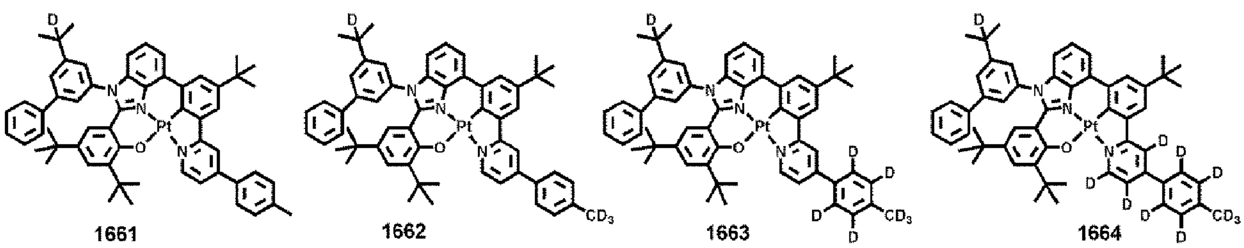

1661 1662 1663 1664

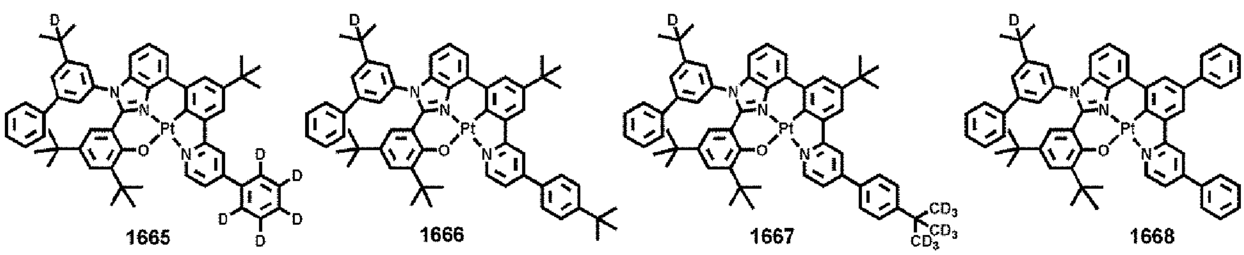

1665 1666 1667 1668

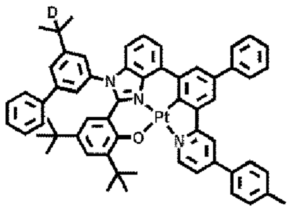
1669

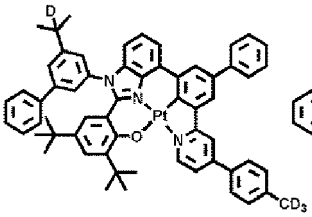
1670

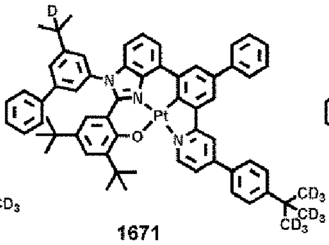
1671

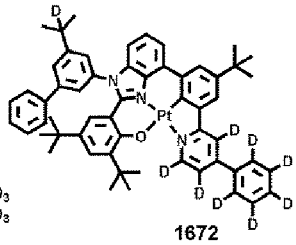
1672

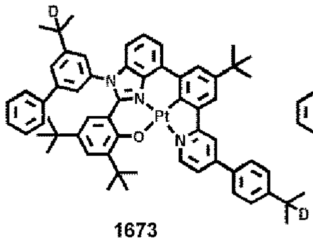
1673

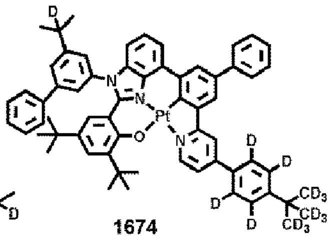
1674

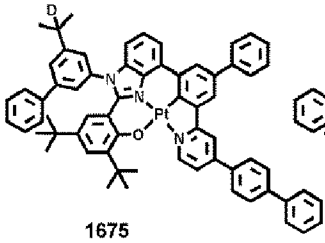
1675

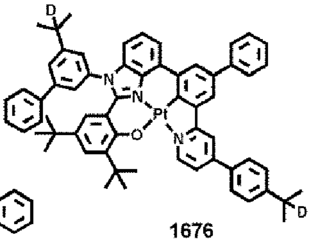
1676

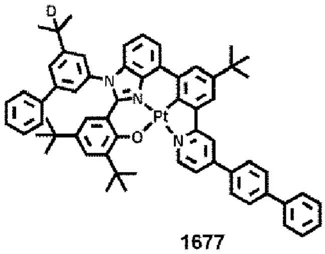
1677

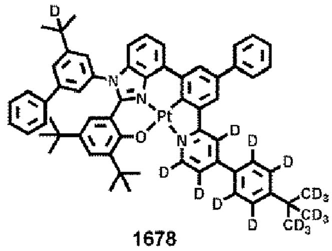
1678

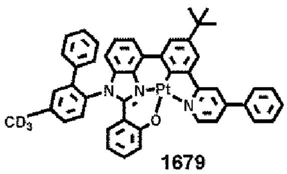
1679

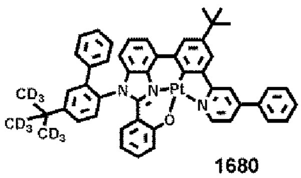
1680

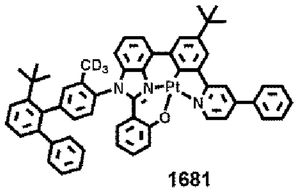
1681

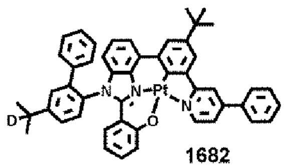
1682

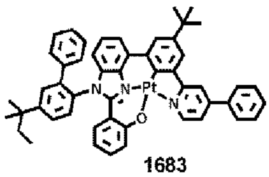
1683

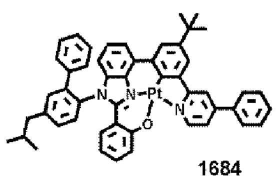
1684

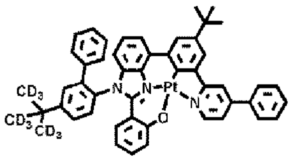
1685

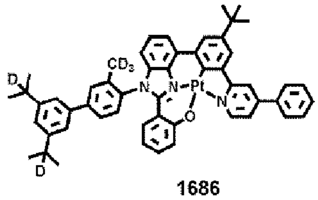
1686

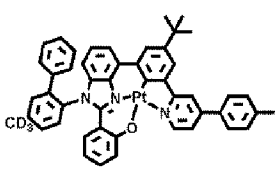
1687

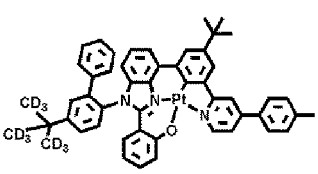
1688

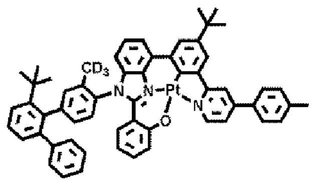
1689

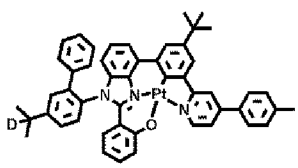
1690

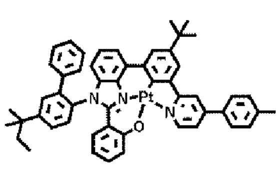
1691

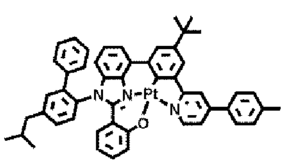
1692

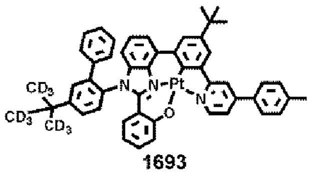
1693

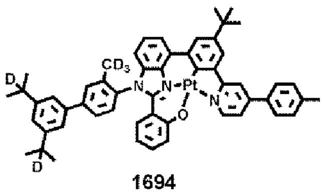
1694

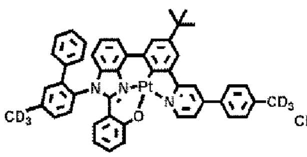
1695

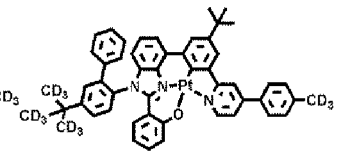
1696

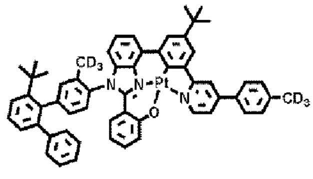
1697

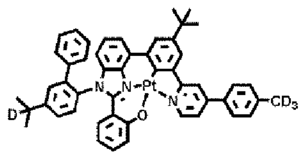
1698

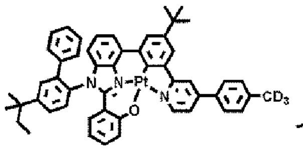
1699

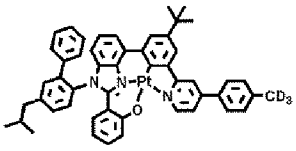
1700

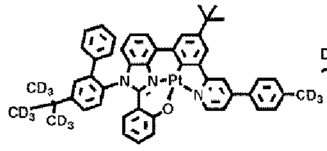
1701

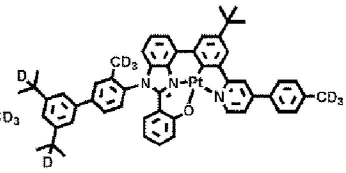
1702

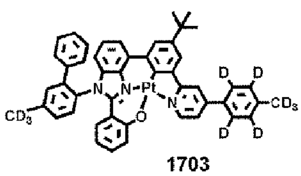
1703

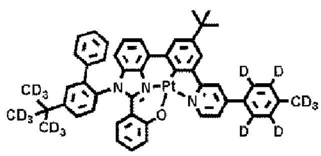
1704

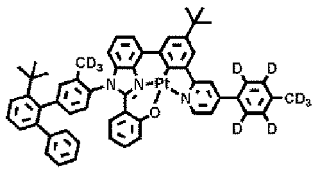
1705

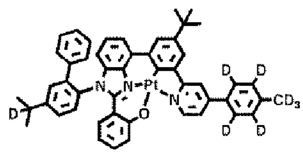
1706

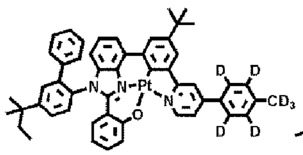
1707

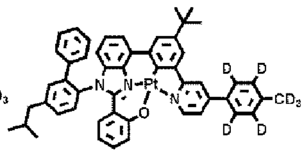
1708

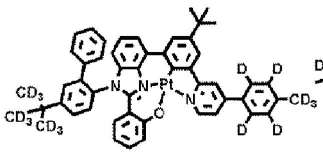
1709

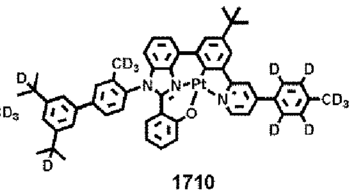
1710

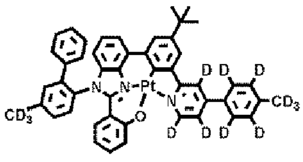
1711

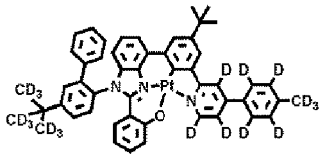
1712

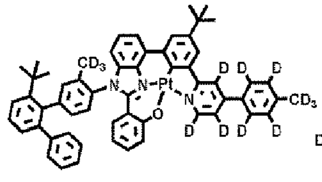
1713

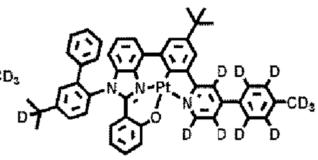
1714

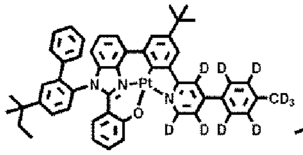
1715

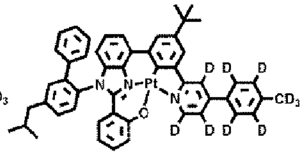
1716

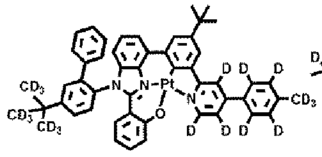
1717

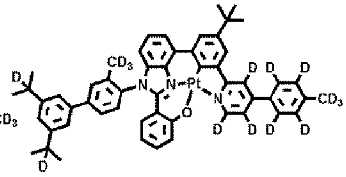
1718

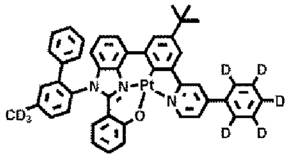

1719

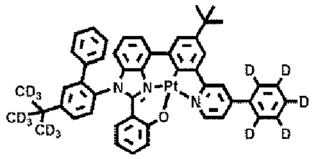

1720

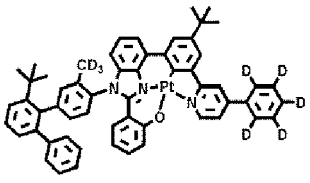

1721

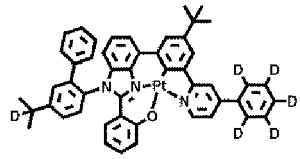

1722

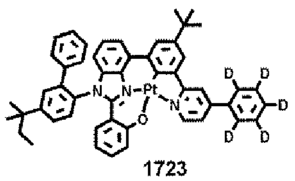

1723

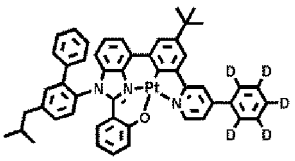

1724

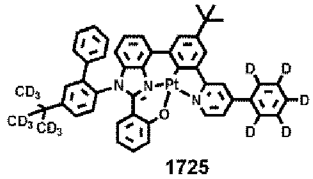

1725

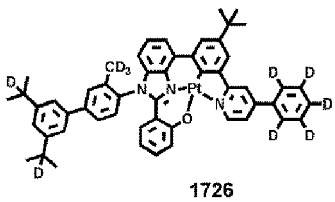

1726

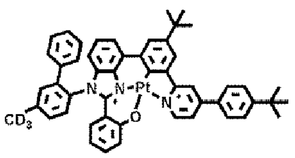

1727

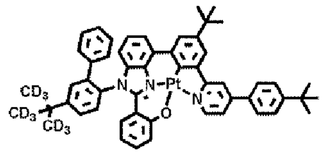

1728

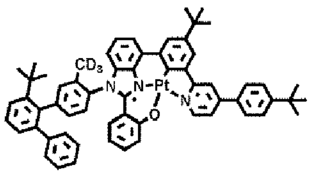

1729

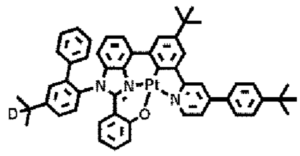

1730

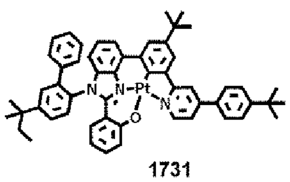

1731

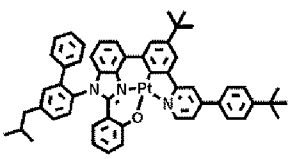

1732

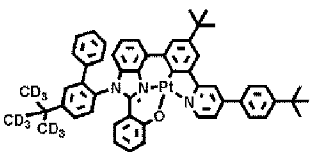

1733

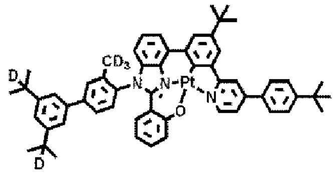

1734

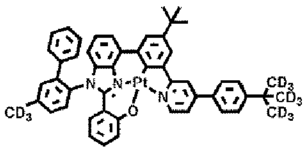

1735

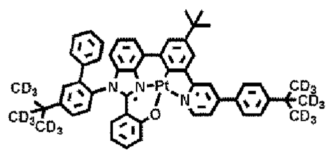

1736

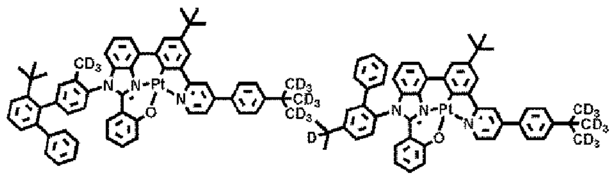

1737

1738

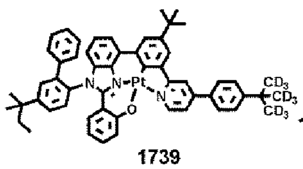

1739

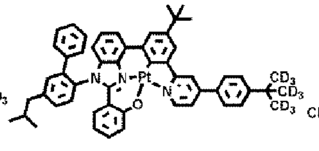

1740

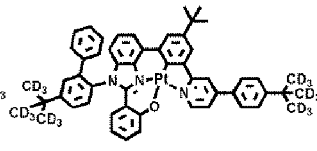

1741

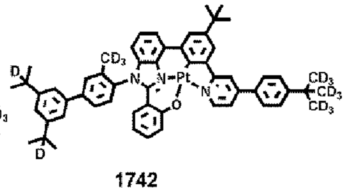

1742

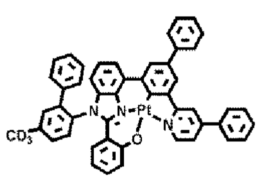

1743

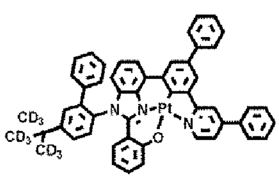

1744

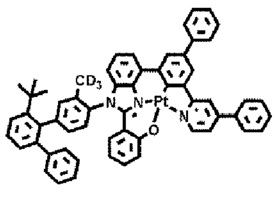

1745

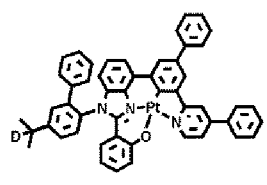

1746

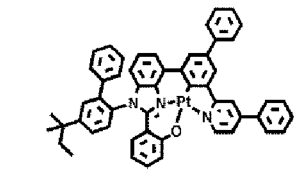
1747

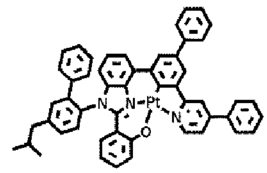
1748

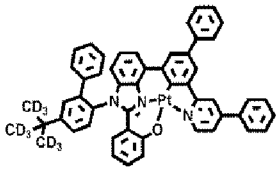
1749

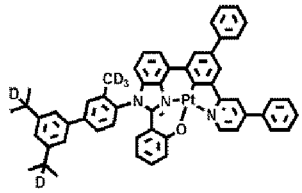
1750

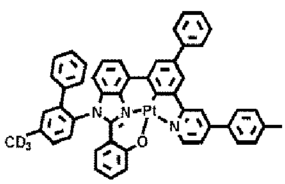
1751

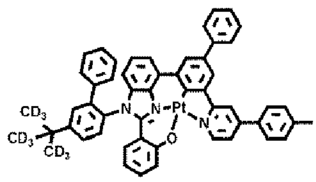
1752

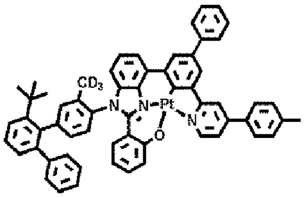
1753

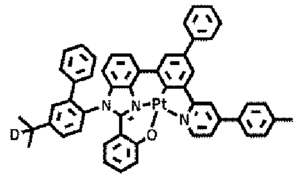
1754

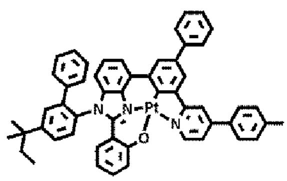
1755

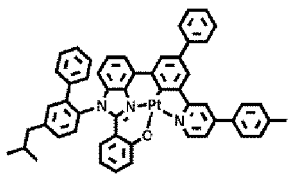
1756

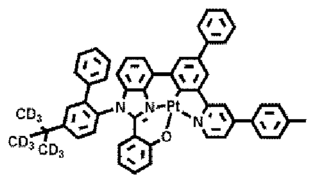
1757

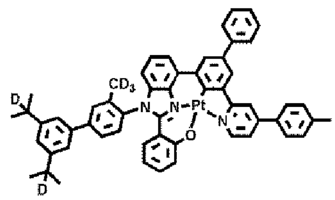
1758

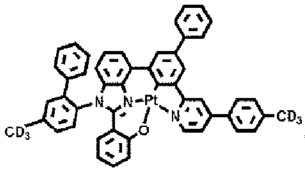
1759

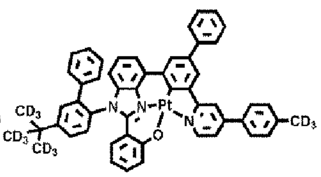
1760

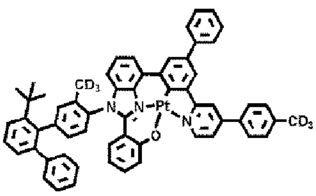
1761

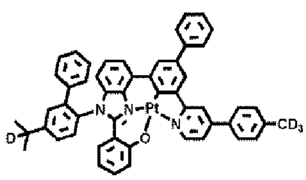
1762

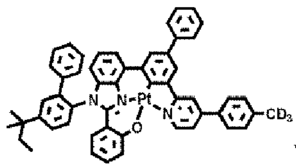
1763

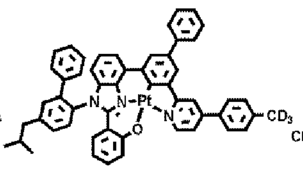
1764

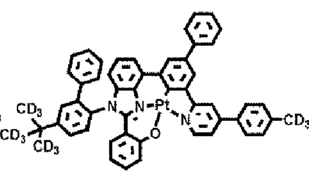
1765

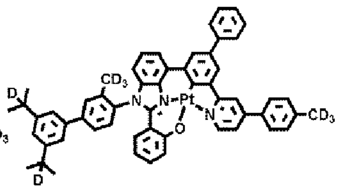
1766

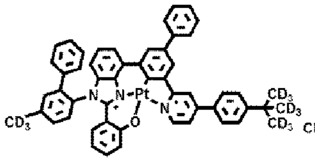
1767

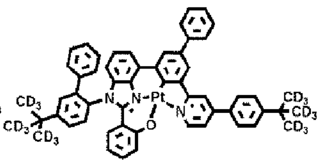
1768

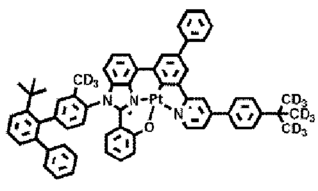
1769

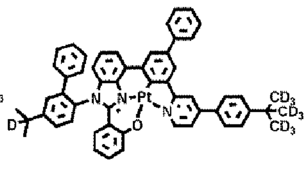
1770

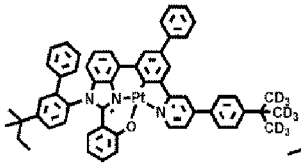
1771
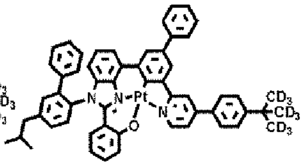
1772
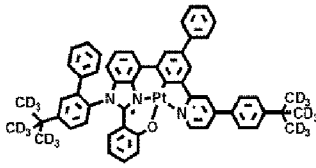
1773
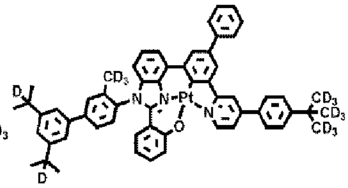
1774

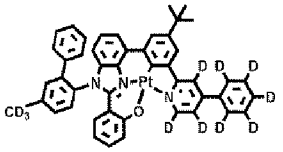
1775
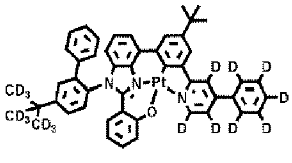
1776
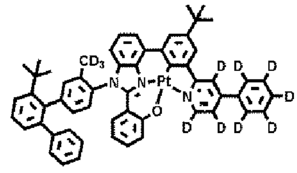
1777
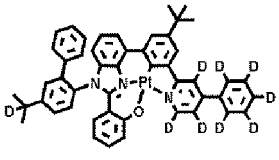
1778

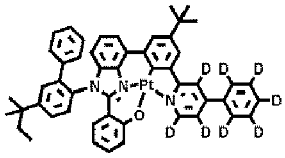
1779
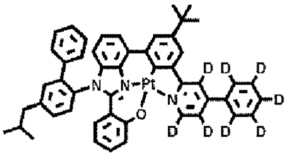
1780
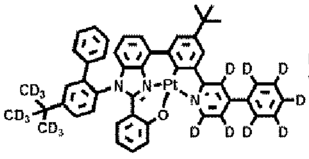
1781
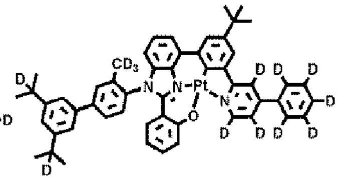
1782

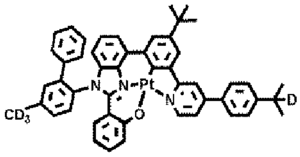
1783
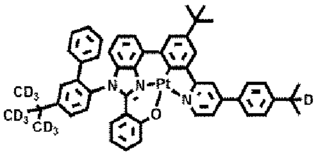
1784
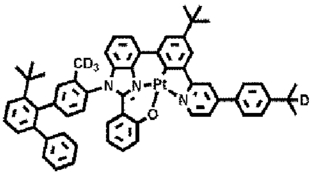
1785
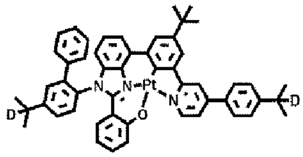
1786

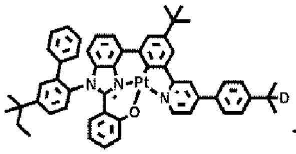
1787
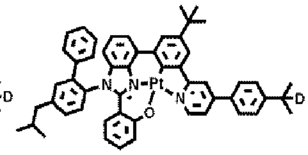
1788
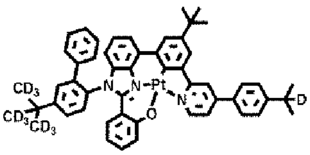
1789
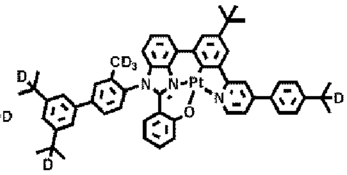
1790

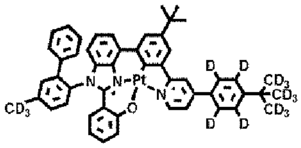
1791
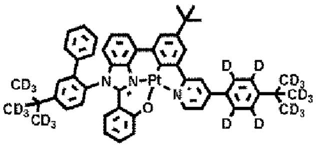
1792
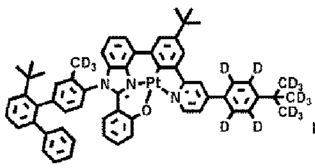
1793
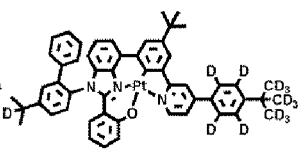
1794

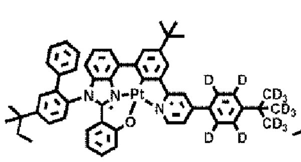
1795
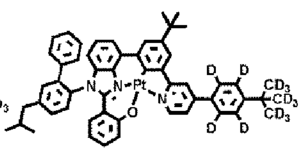
1796
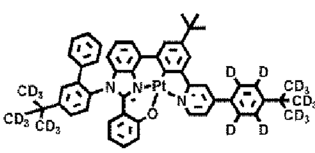
1797
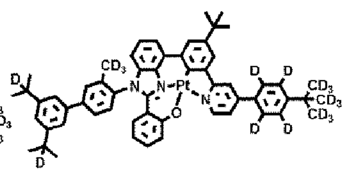
1798

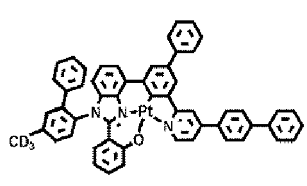
1799

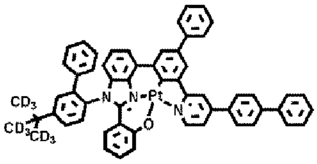
1800

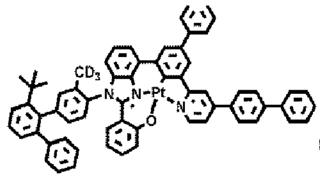
1801

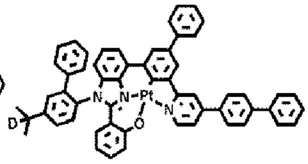
1802

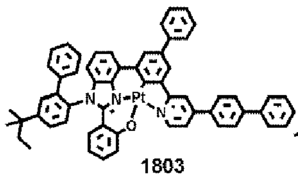
1803

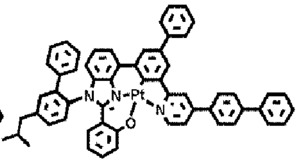
1804

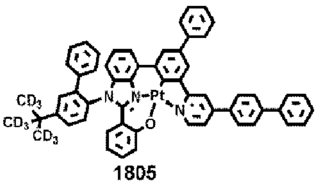
1805

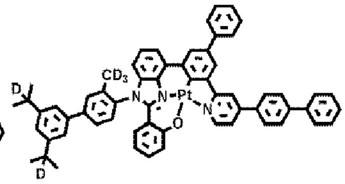
1806

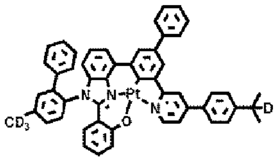
1807

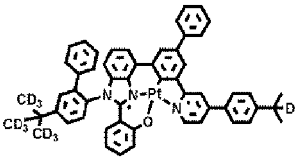
1808

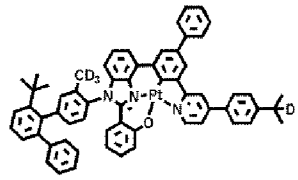
1809

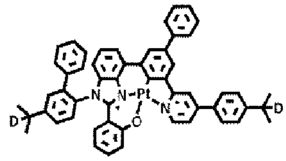
1810

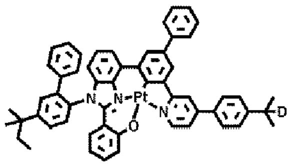
1811

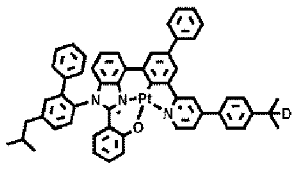
1812

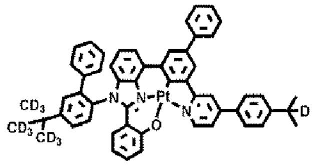
1813

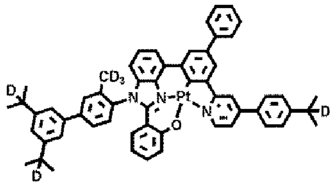
1814

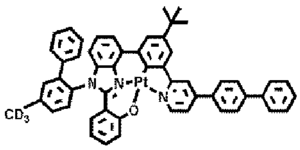
1815

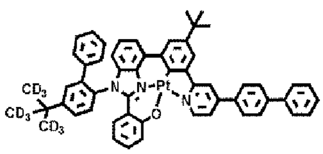
1816

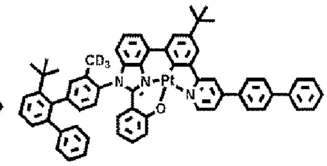
1817

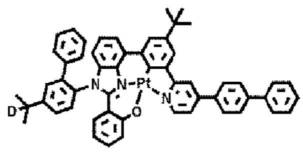
1818

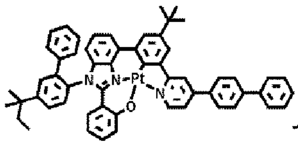
1819

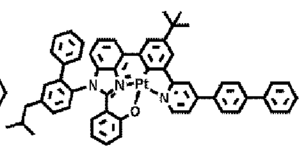
1820

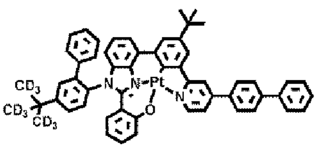
1821

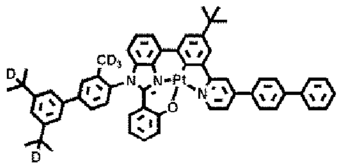
1822

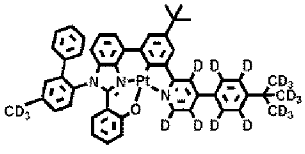
1823

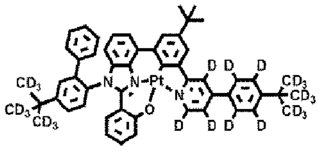
1824

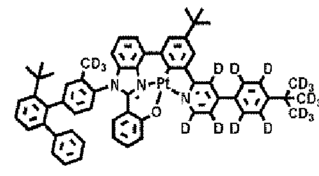
1825

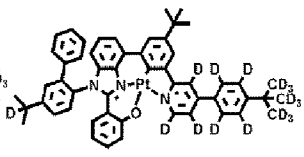
1826

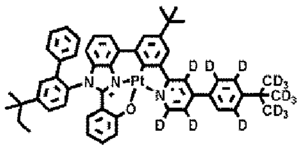
1827

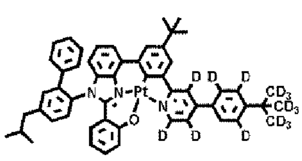
1828

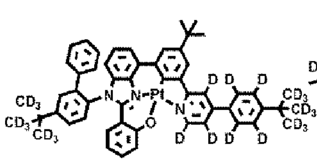
1829

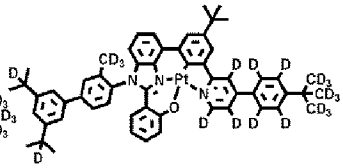
1830

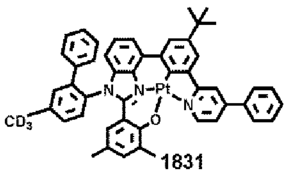
1831

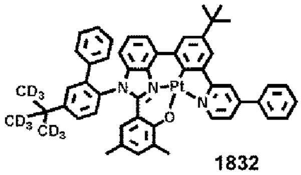
1832

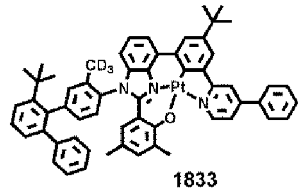
1833

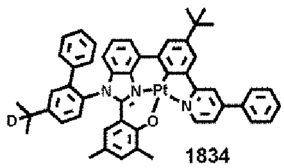
1834

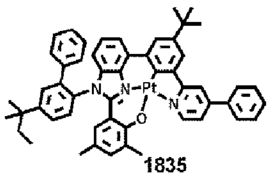
1835

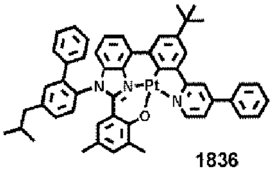
1836

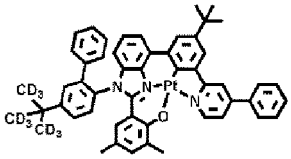
1837

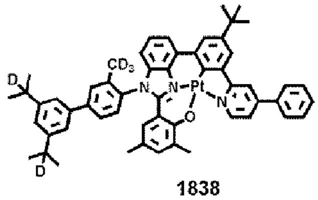
1838

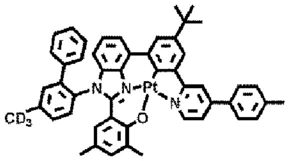
1839

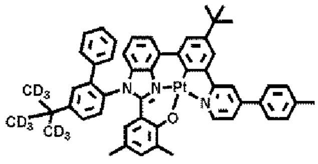
1840

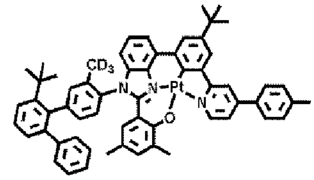
1841

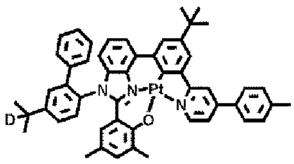
1842

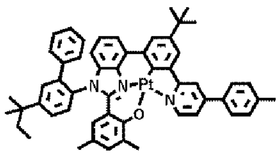
1843

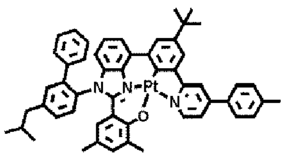
1844

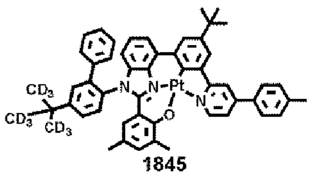
1845

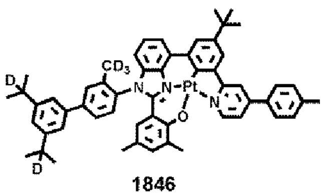
1846

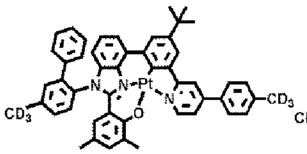
1847

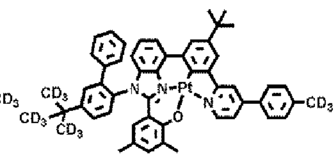
1848

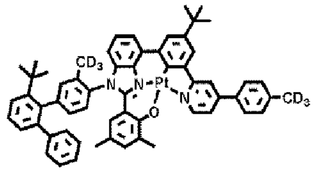
1849

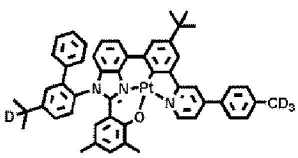
1850

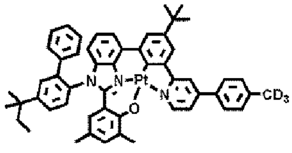
1851

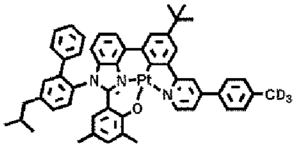
1852

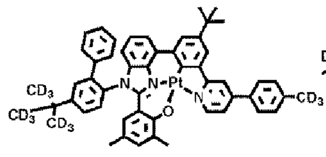
1853

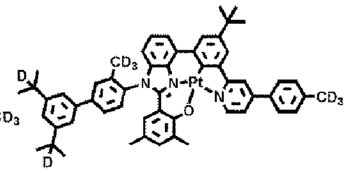
1854

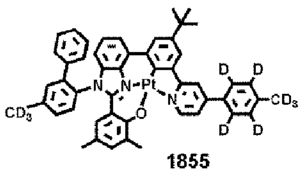

1855

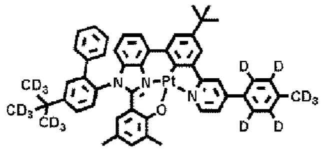

1856

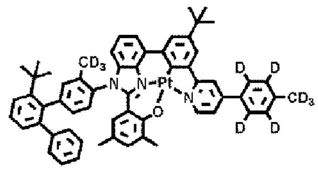

1857

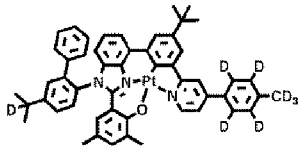

1858

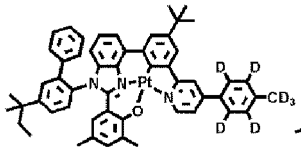

1859

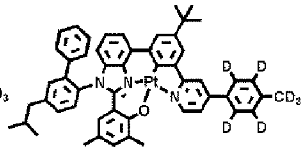

1860

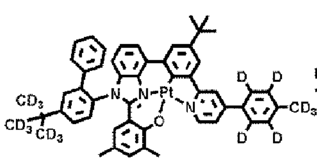

1861

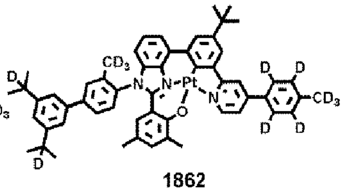

1862

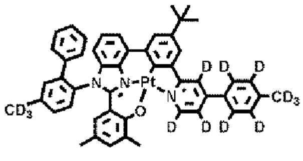

1863

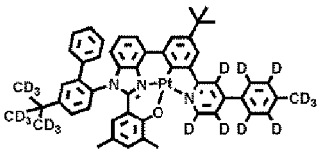

1864

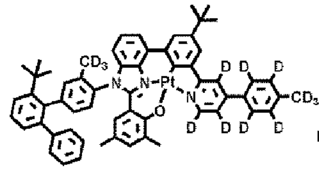

1865

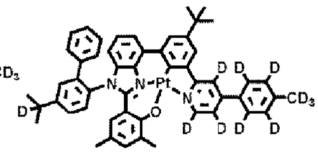

1866

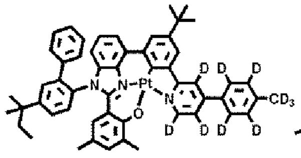

1867

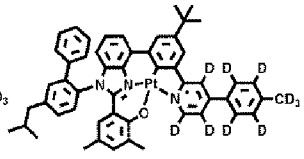

1868

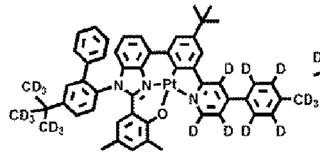

1869

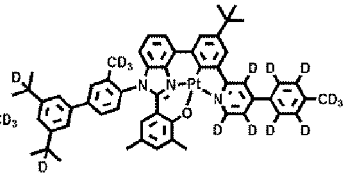

1870

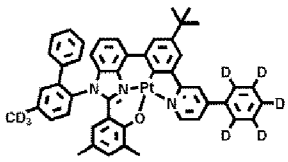

1871

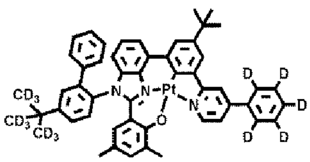

1872

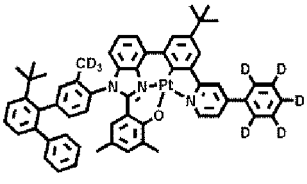

1873

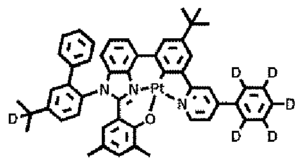

1874

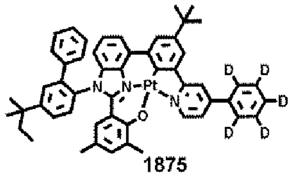

1875

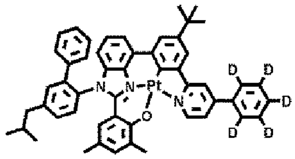

1876

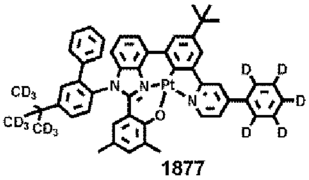

1877

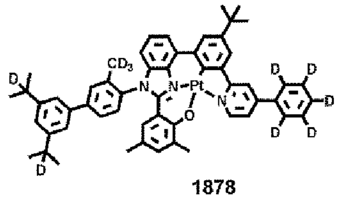

1878

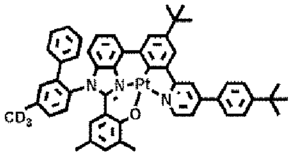

1879

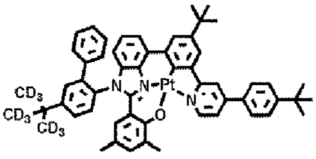

1880

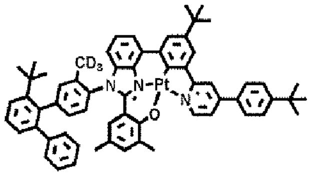

1881

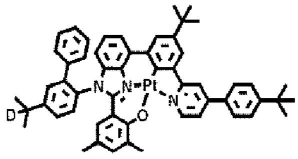

1882

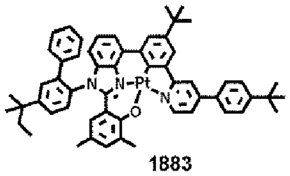
1883

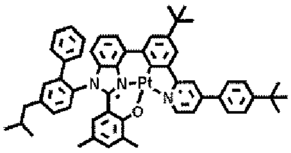
1884

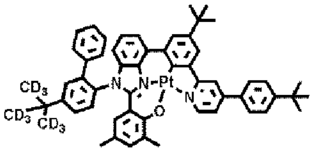
1885

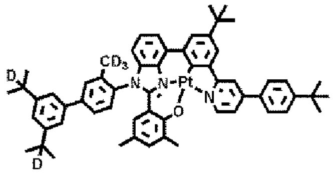
1886

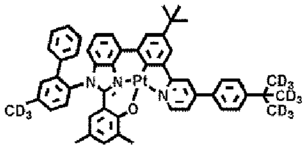
1887

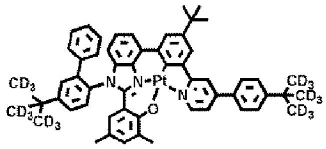
1888

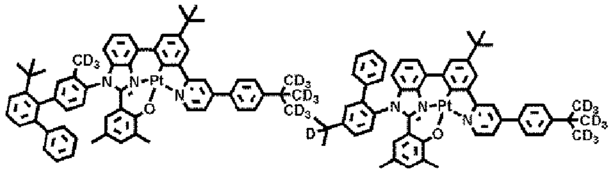
1889

1890

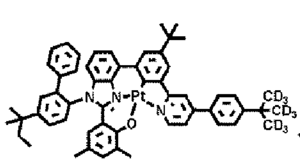
1891

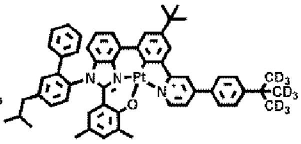
1892

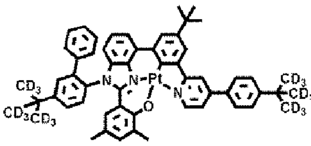
1893

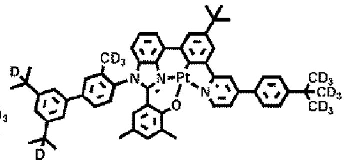
1894

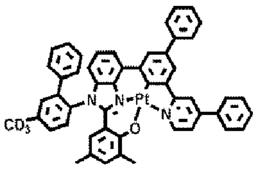
1895

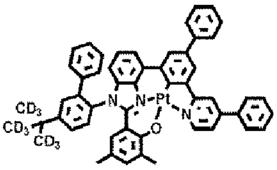
1896

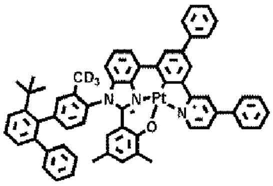
1897

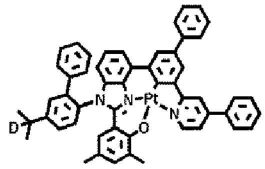
1898

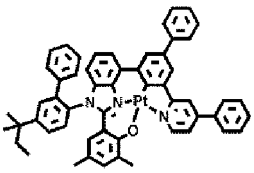
1899

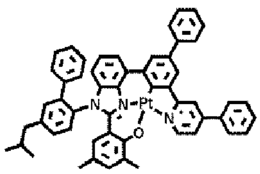
1900

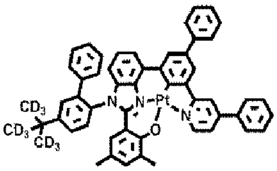
1901

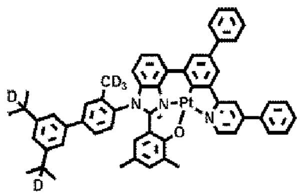
1902

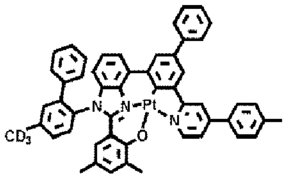
1903

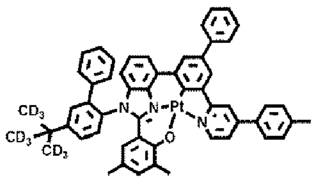
1904

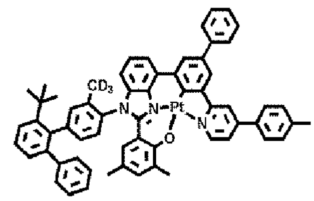
1905

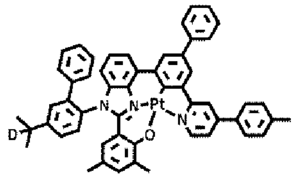
1906

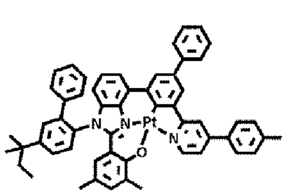
1907
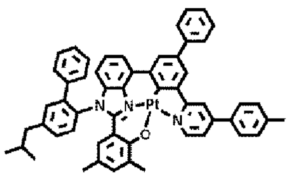
1908
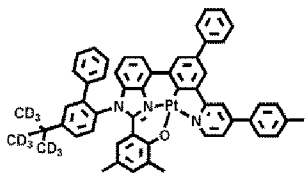
1909
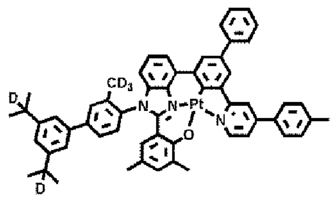
1910

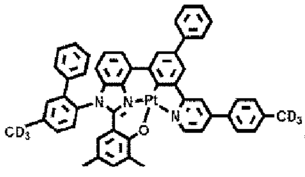
1911
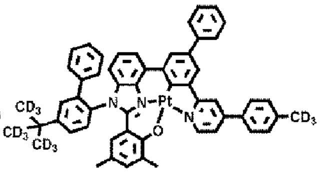
1912
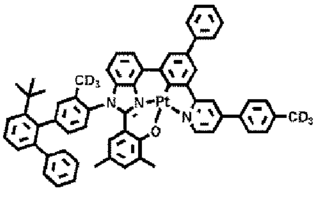
1913
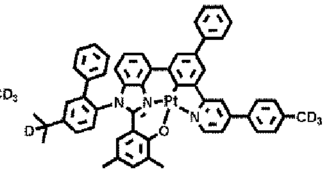
1914

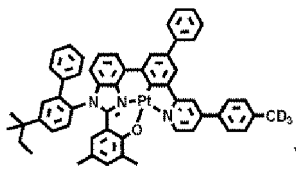
1915
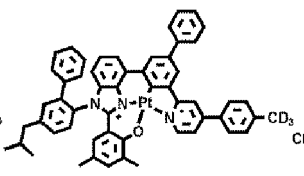
1916
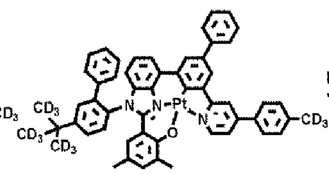
1917
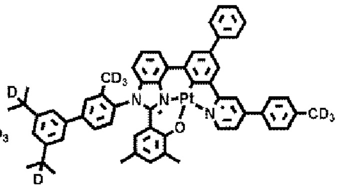
1918

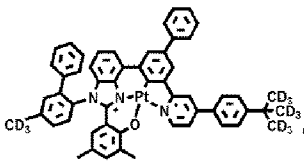
1919
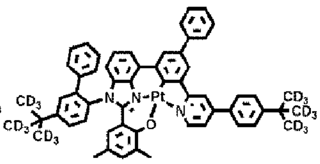
1920
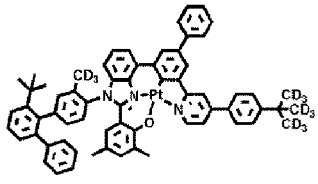
1921
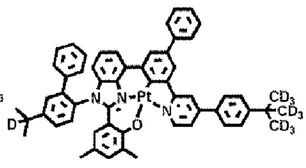
1922

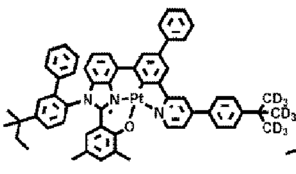
1923
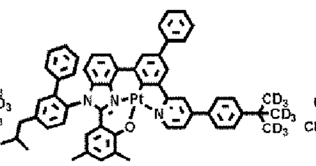
1924
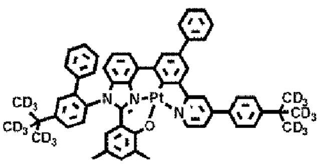
1925
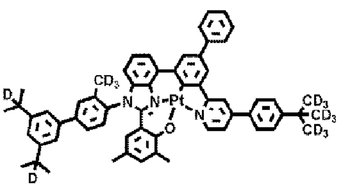
1926

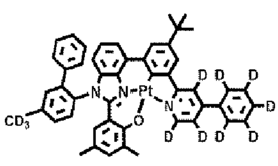
1927
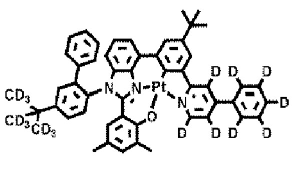
1928
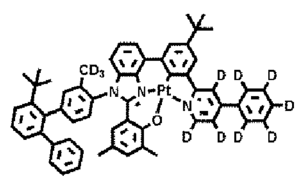
1929
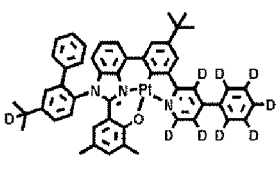
1930

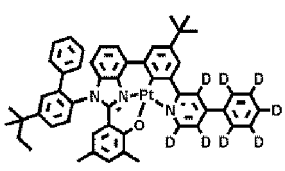
1931
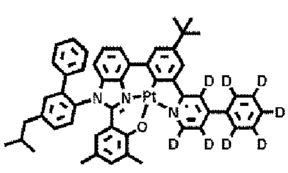
1932
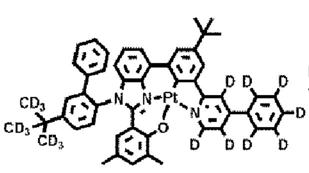
1933
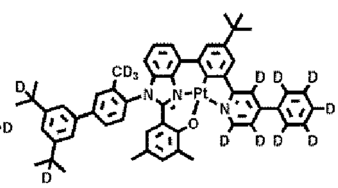
1934

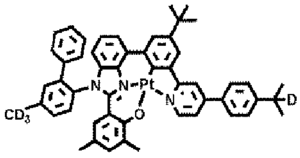
1935

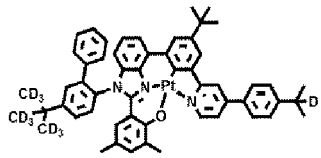
1936

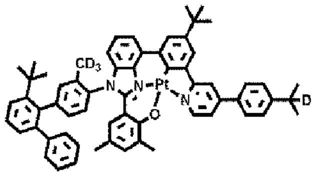
1937

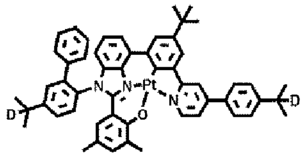
1938

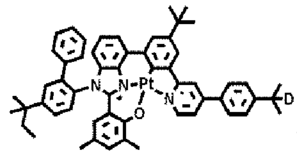
1939

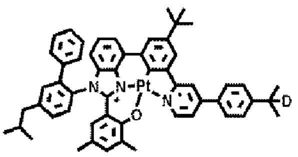
1940

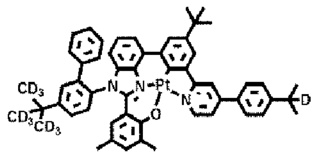
1941

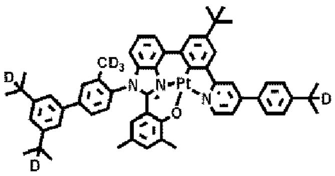
1942

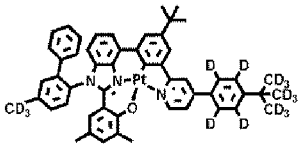
1943

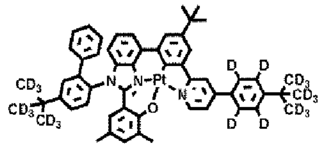
1944

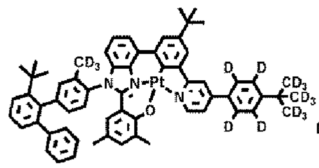
1945

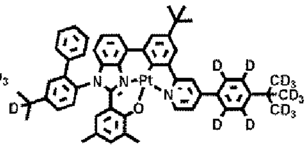
1946

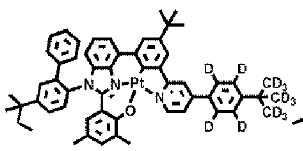
1947

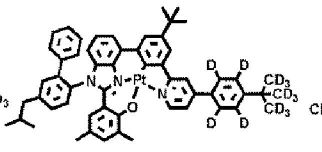
1948

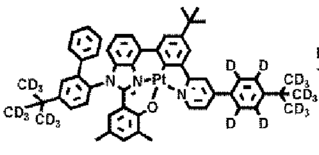
1949

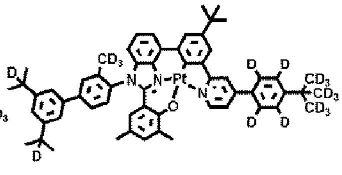
1950

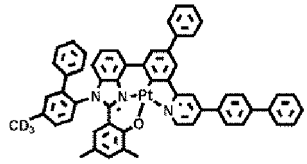
1951

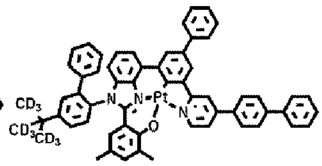
1952

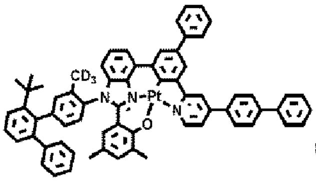
1953

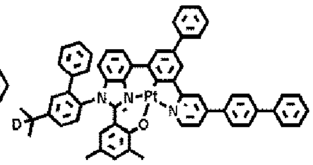
1954

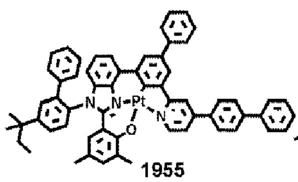
1955

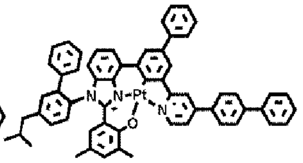
1956

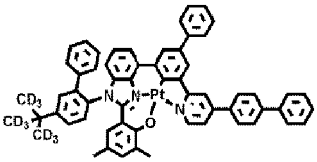
1957

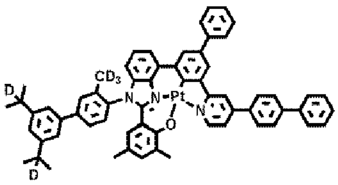
1958

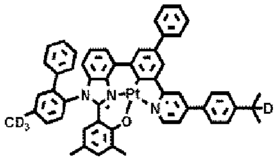
1959

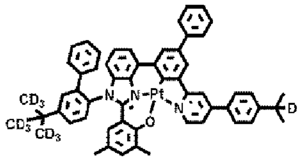
1960

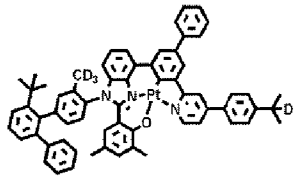
1961

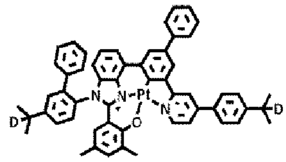
1962

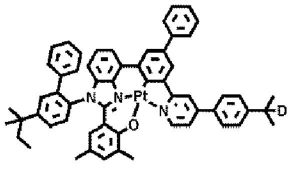
1963

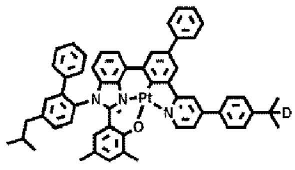
1964

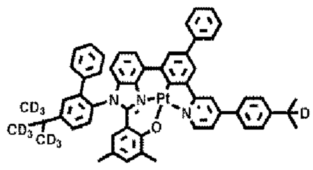
1965

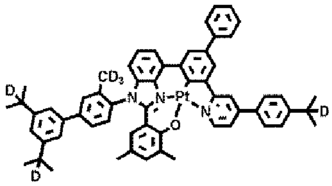
1966

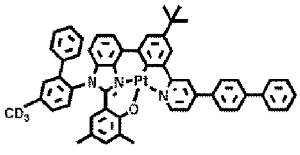
1967

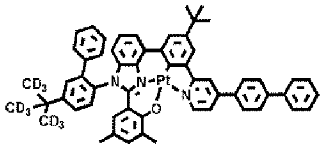
1968

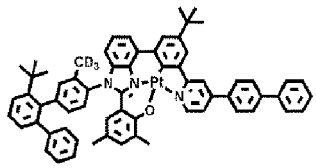
1969

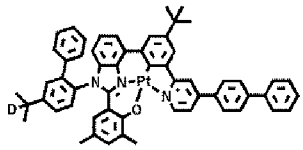
1970

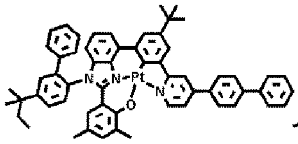
1971

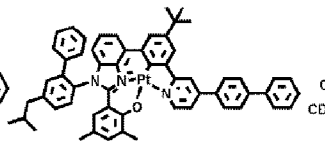
1972

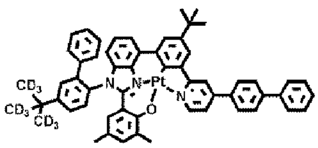
1973

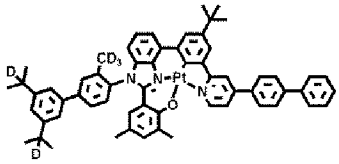
1974

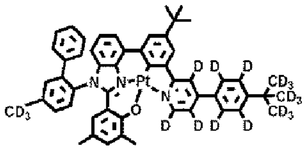
1975

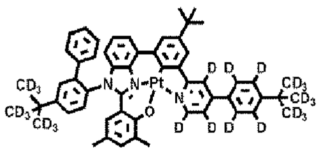
1976

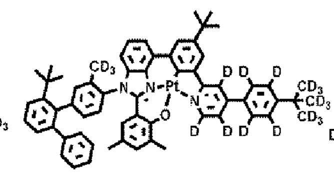
1977

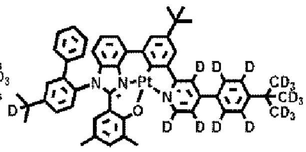
1978

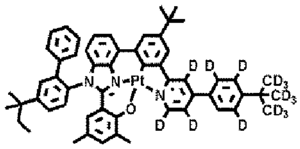
1979

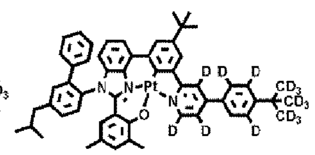
1980

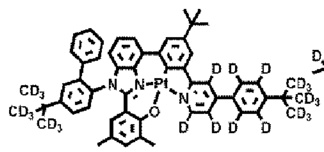
1981

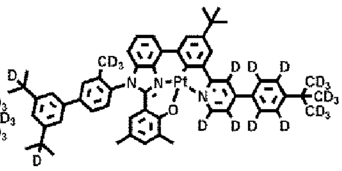
1982

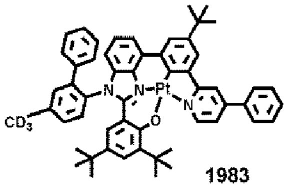
1983

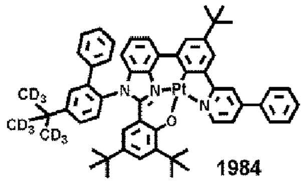
1984

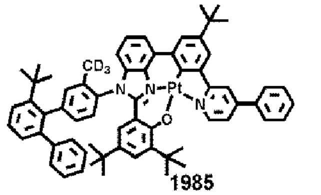
1985

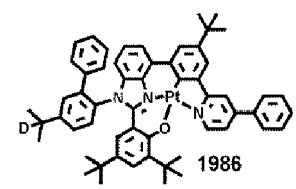
1986

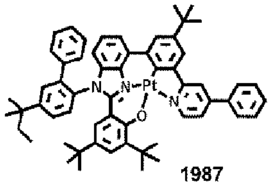
1987

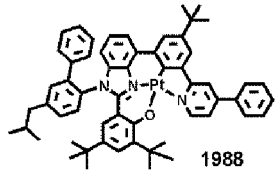
1988

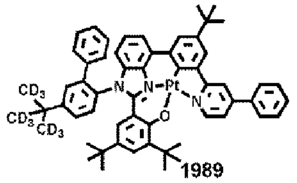
1989

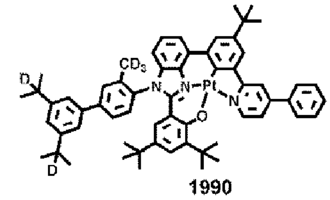
1990

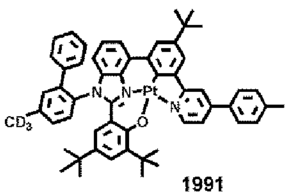
1991

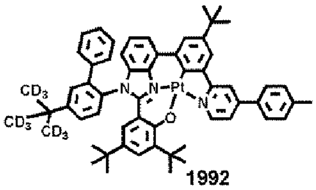
1992

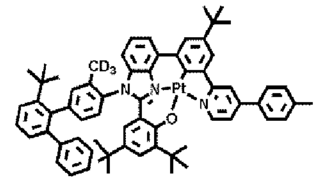
1993

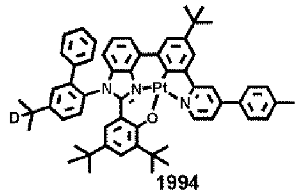
1994

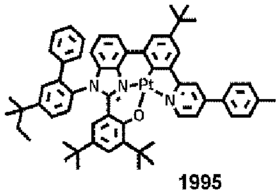
1995

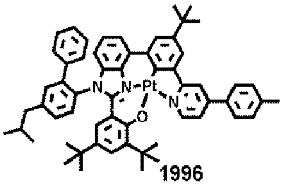
1996

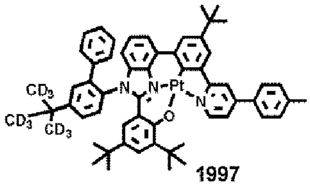
1997

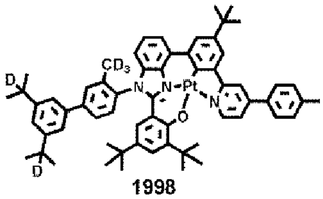
1998

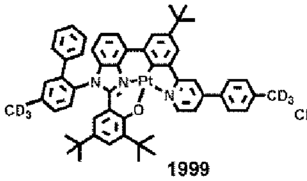
1999

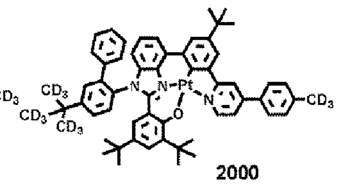
2000

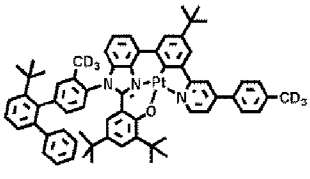
2001

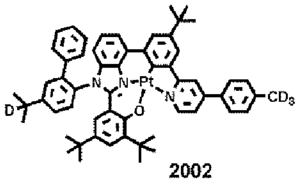
2002

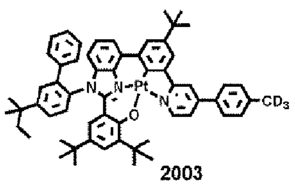
2003

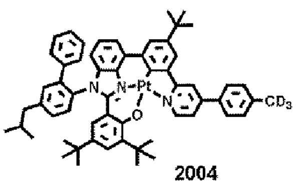
2004

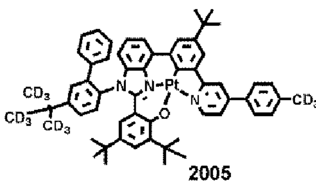
2005

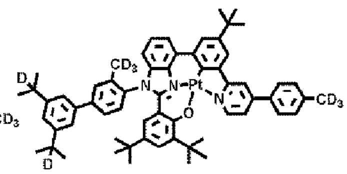
2006

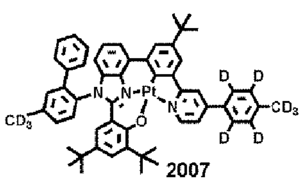
2007

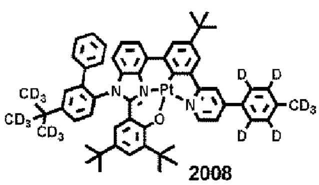
2008

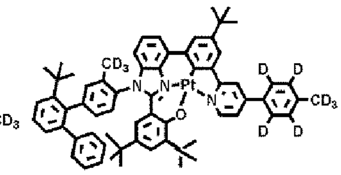
2009

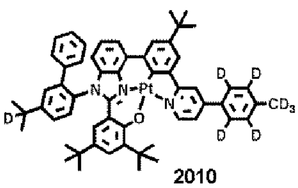
2010

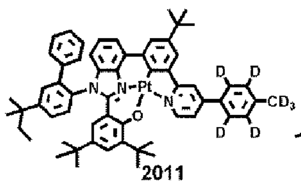
2011

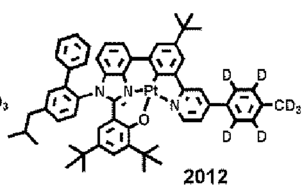
2012

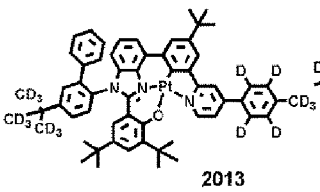
2013

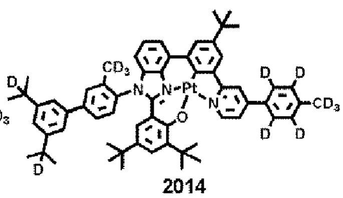
2014

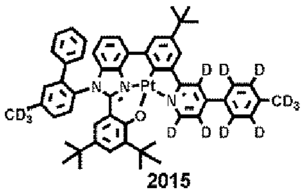
2015

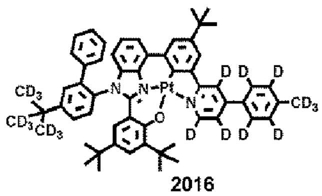
2016

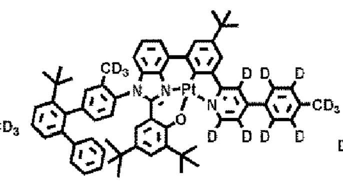
2017

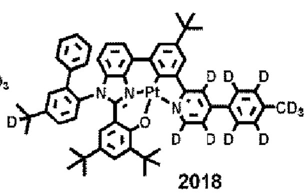
2018

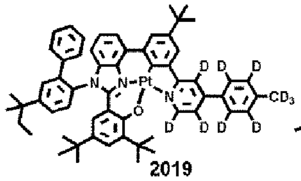
2019

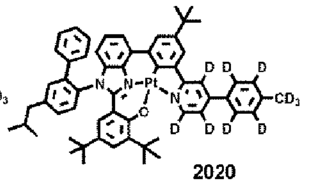
2020

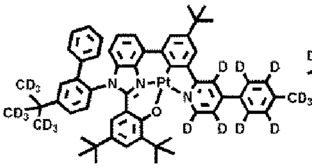
2021

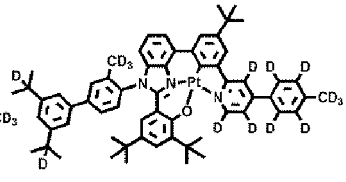
2022

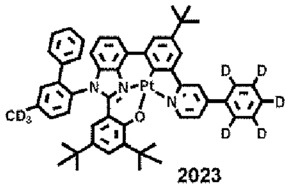
2023

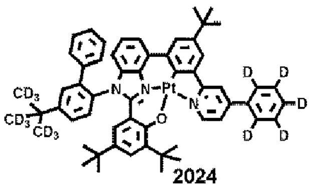
2024

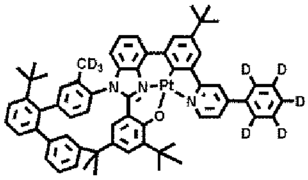
2025

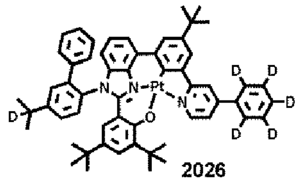
2026

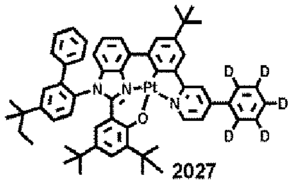
2027

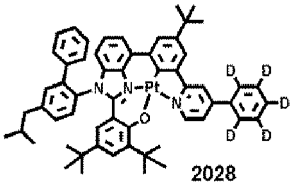
2028

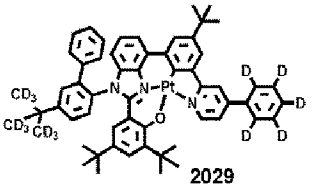
2029

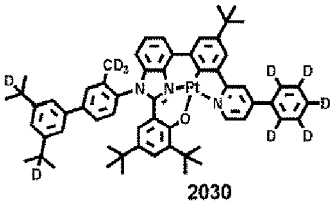
2030

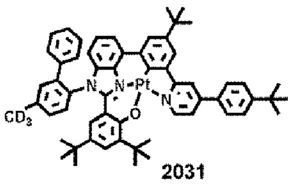
2031

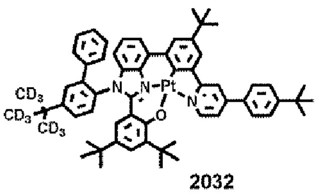
2032

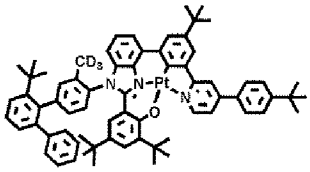
2033

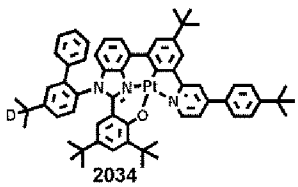
2034

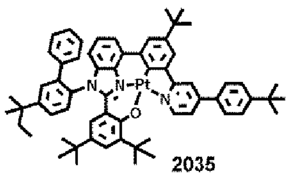
2035

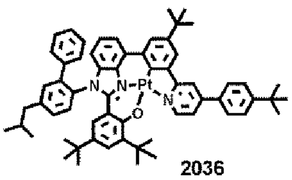
2036

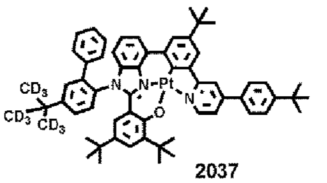
2037

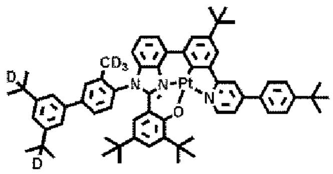
2038

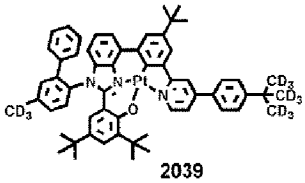
2039

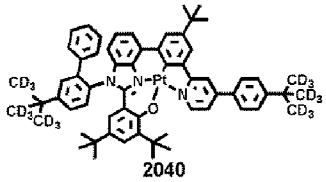
2040

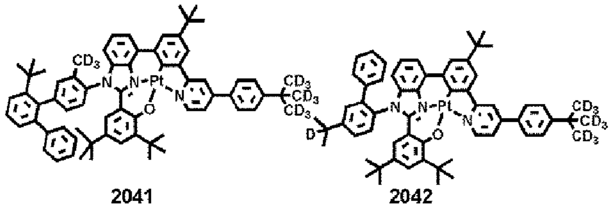
2041

2042

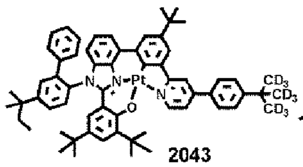
2043

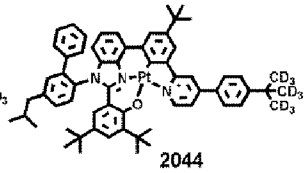
2044

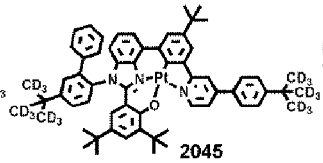
2045

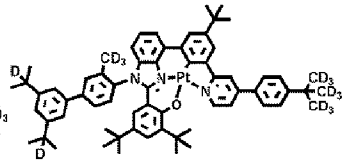
2046

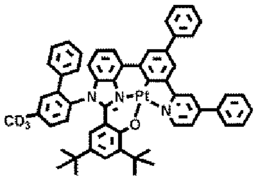
2047

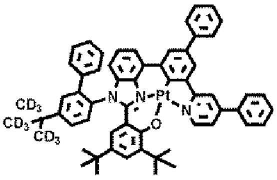
2048

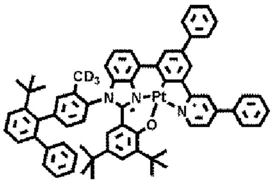
2049

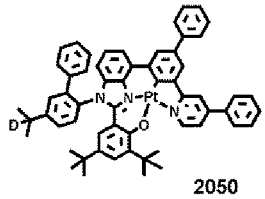
2050

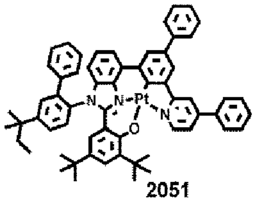
2051

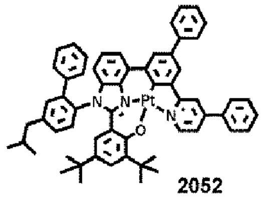
2052

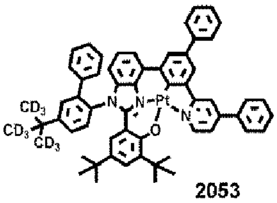
2053

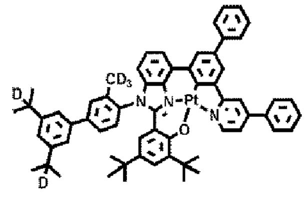
2054

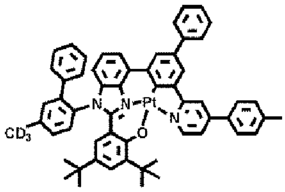
2055

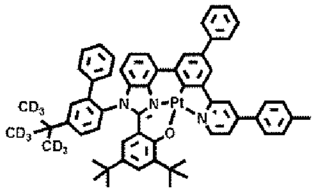
2056

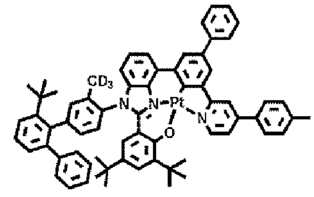
2057

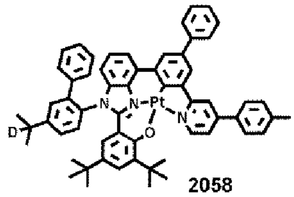
2058

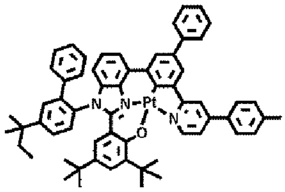
2059

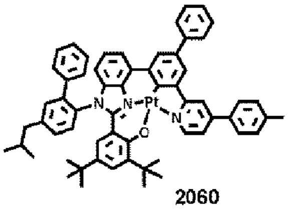
2060

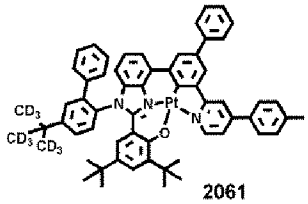
2061

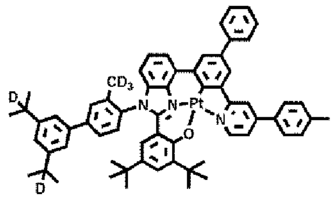
2062

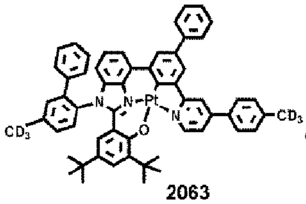
2063

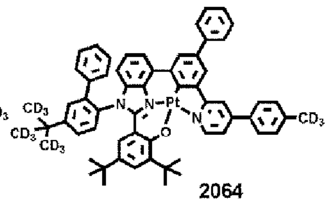
2064

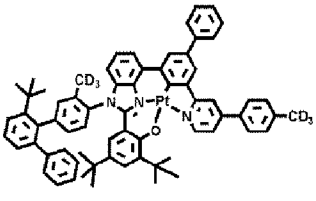
2065

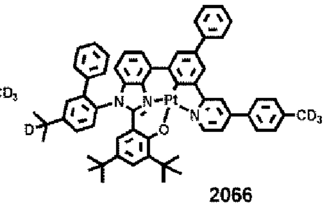
2066

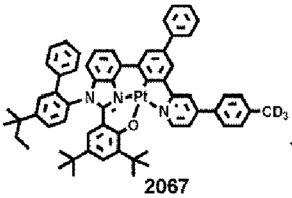
2067

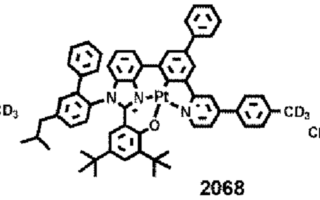
2068

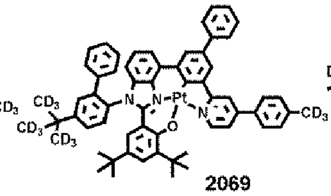
2069

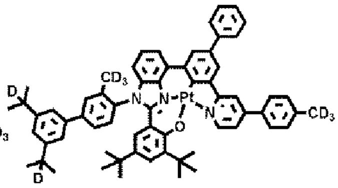
2070

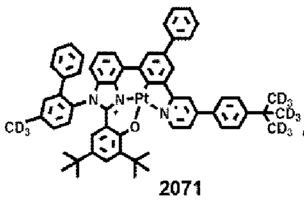
2071

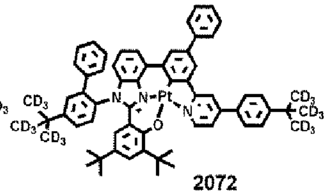
2072

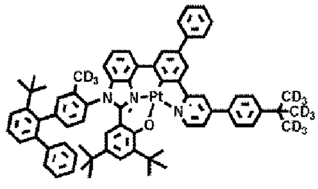
2073

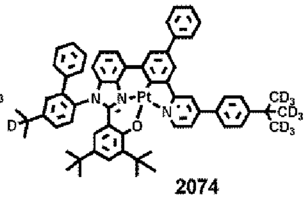
2074

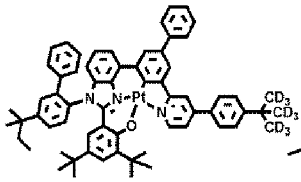
2075

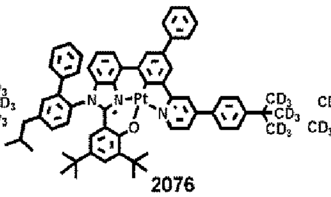
2076

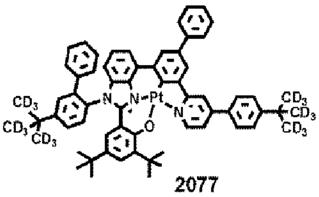
2077

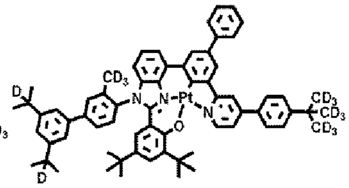
2078

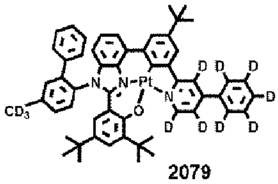
2079

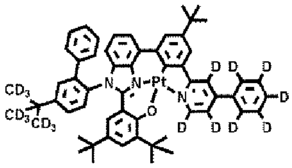
2080

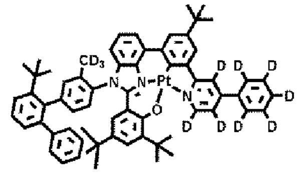
2081

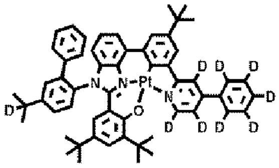
2082

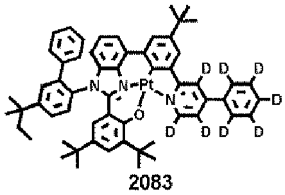
2083

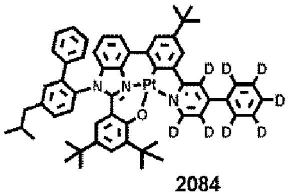
2084

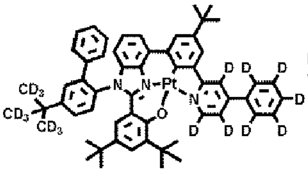
2085

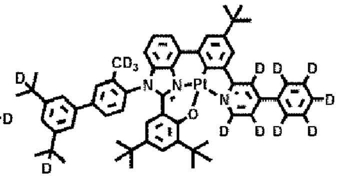
2086

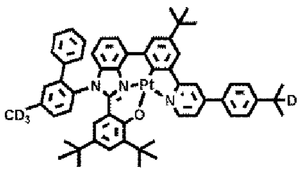
2087

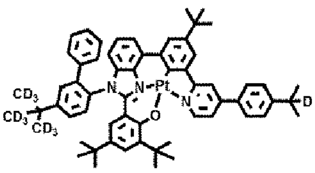
2088

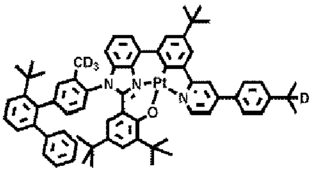
2089

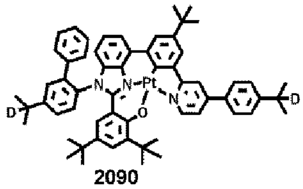
2090

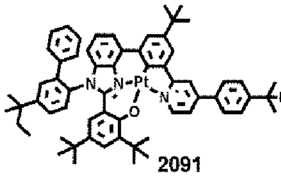
2091

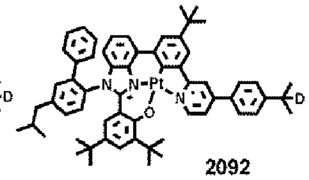
2092

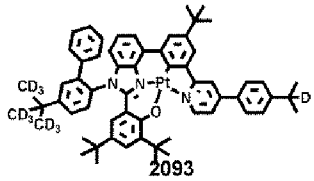
2093

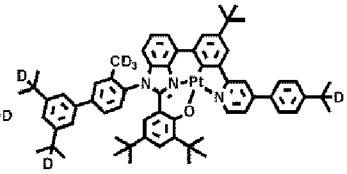
2094

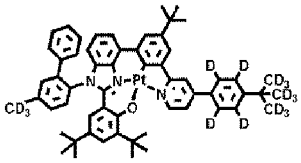
2095

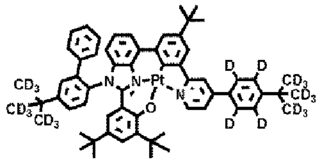
2096

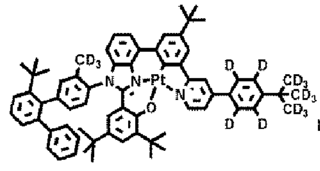
2097

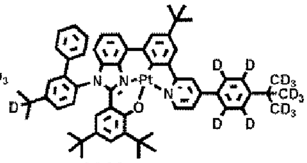
2098

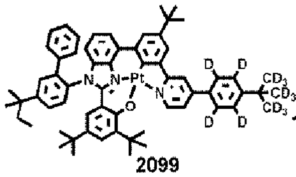
2099

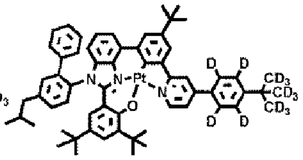
2100

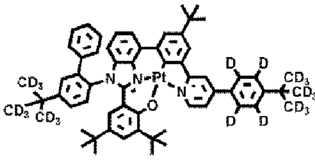
2101

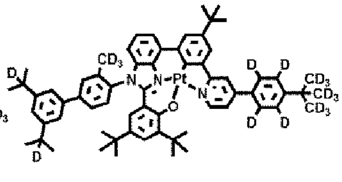
2102

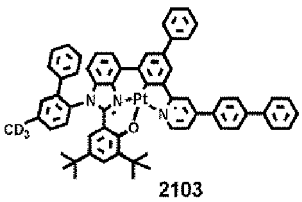
2103

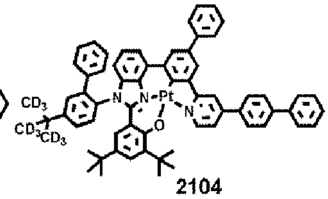
2104

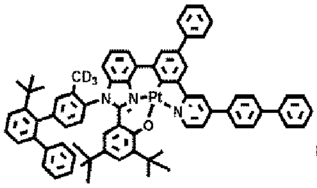
2105

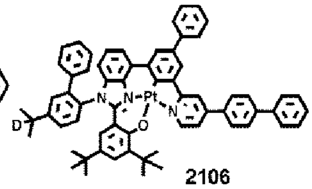
2106

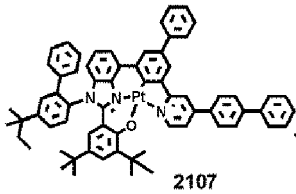
2107

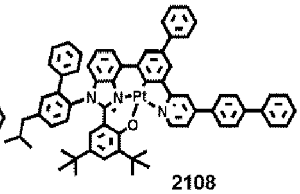
2108

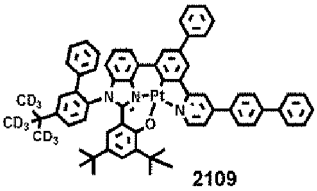
2109

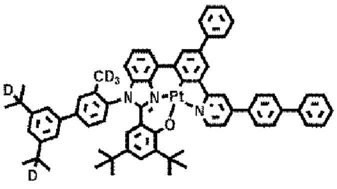
2110

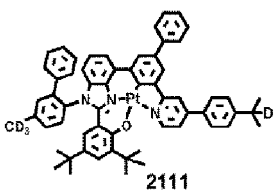
2111

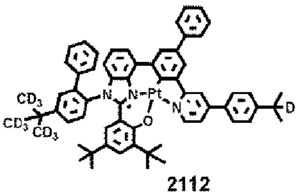
2112

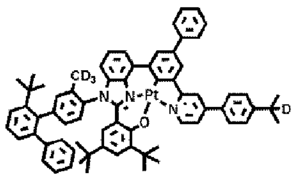
2113

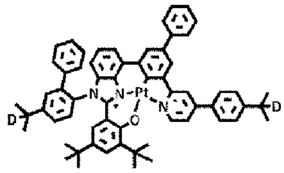
2114

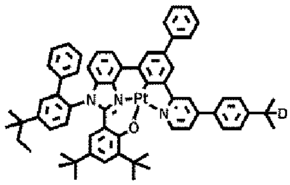
2115

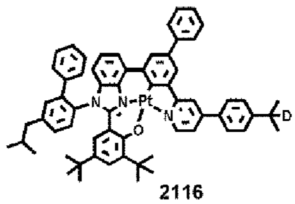
2116

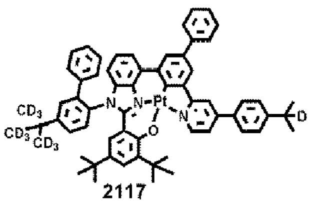
2117

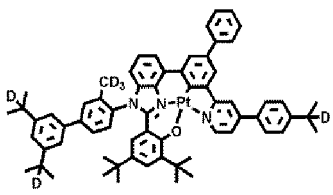
2118

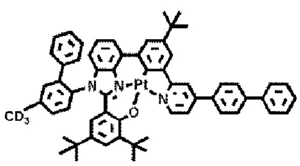
2119

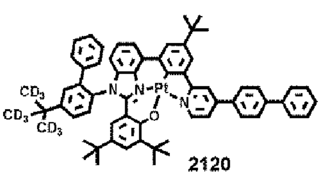
2120

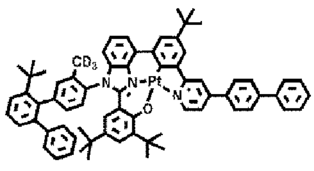
2121

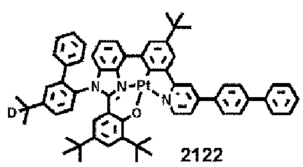
2122

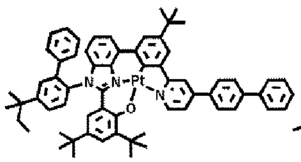
2123

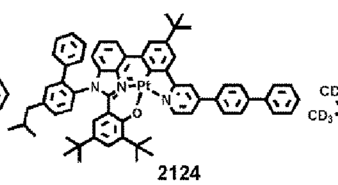
2124

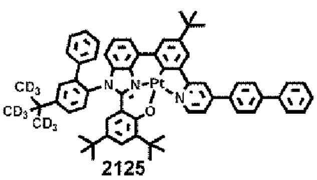
2125

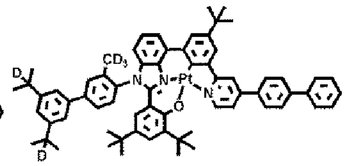
2126

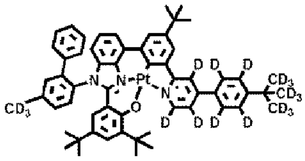

2127

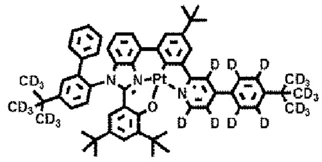

2128

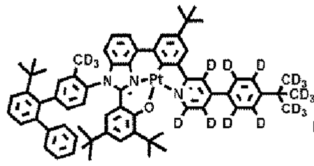

2129

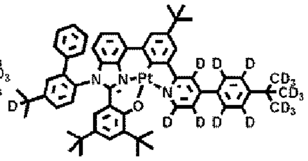

2130

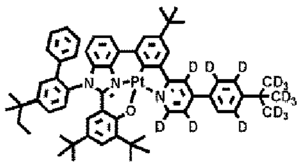

2131

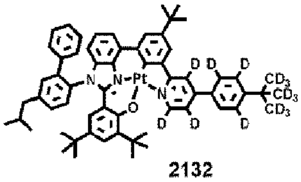

2132

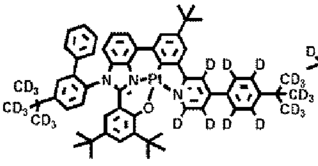

2133

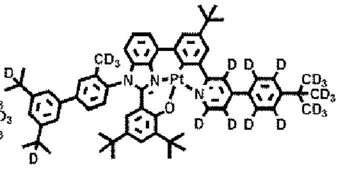

2134

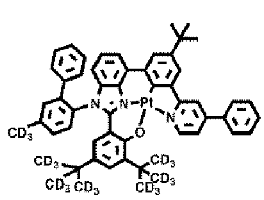

2135

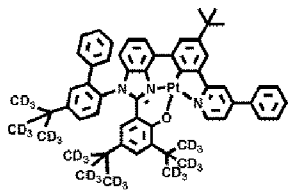

2136

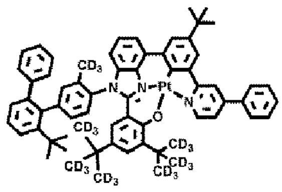

2137

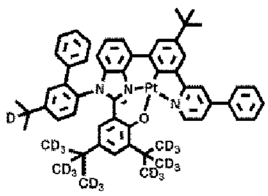

2138

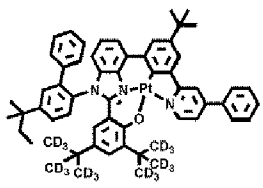

2139

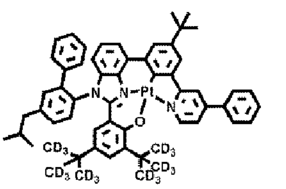

2140

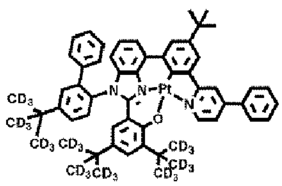

2141

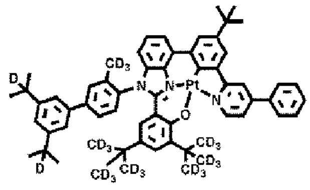

2142

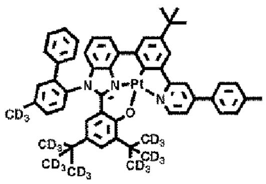

2143

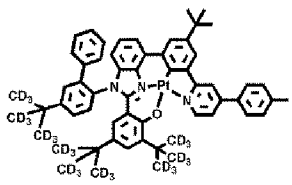

2144

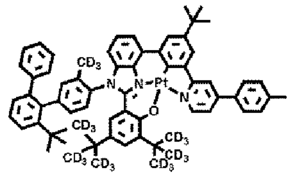

2145

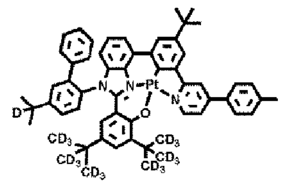

2146

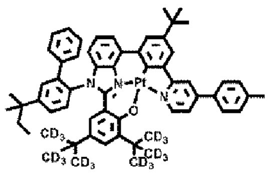

2147

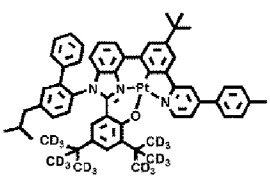

2148

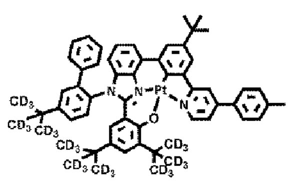

2149

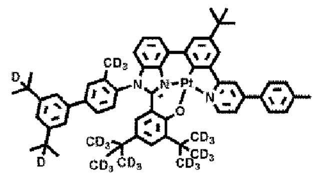

2150

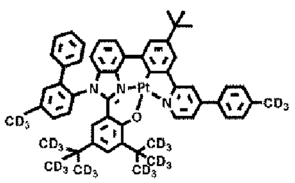

2151

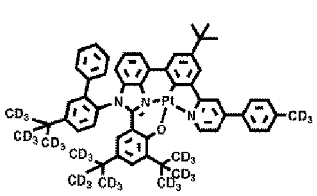

2152

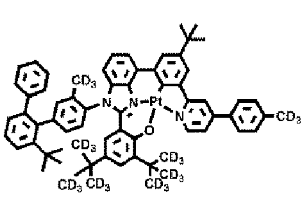

2153

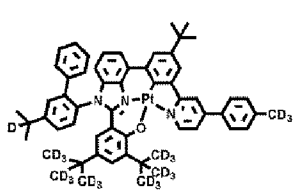

2154

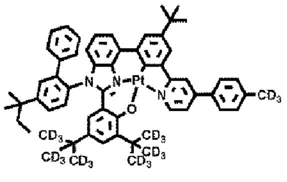
2155

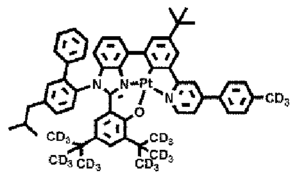
2156

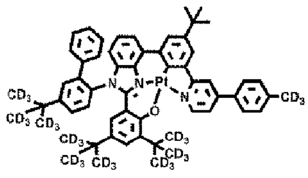
2157

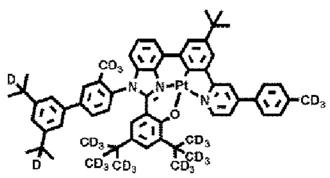
2158

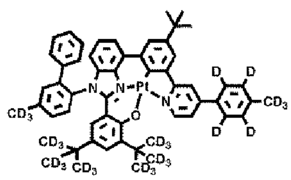
2159

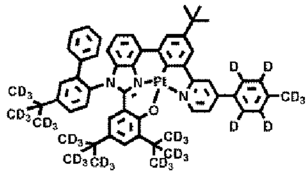
2160

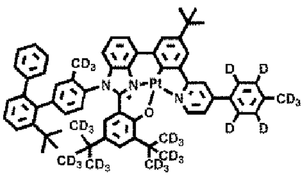
2161

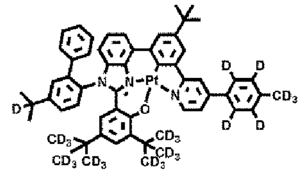
2162

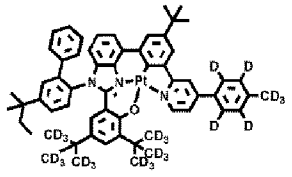
2163

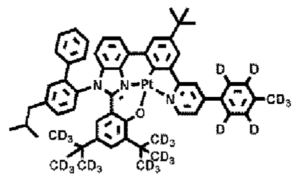
2164

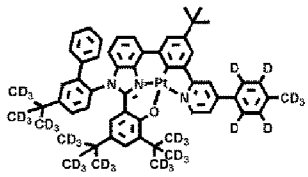
2165

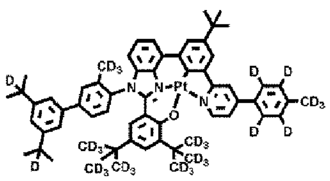
2166

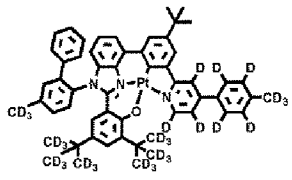
2167

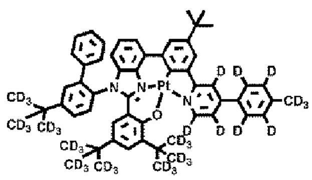
2168

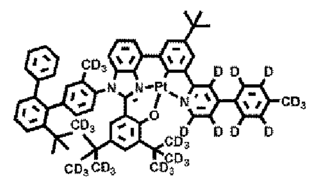
2169

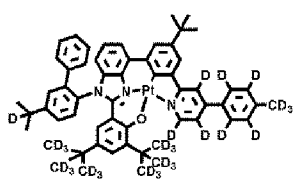
2170

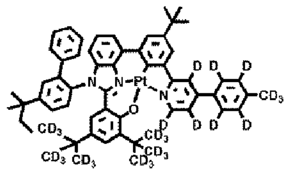
2171

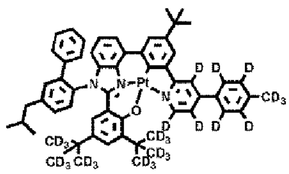
2172

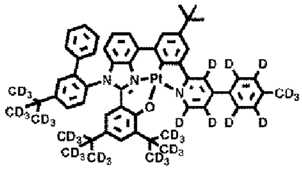
2173

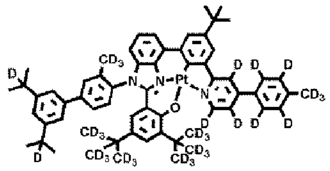
2174

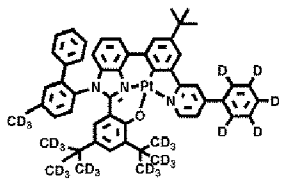
2175

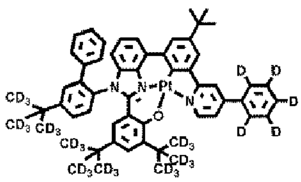
2176

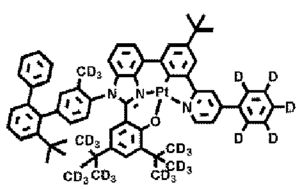
2177

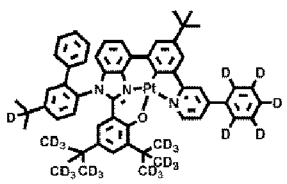
2178

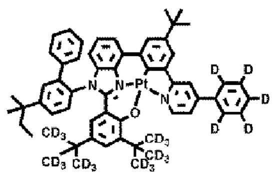
2179

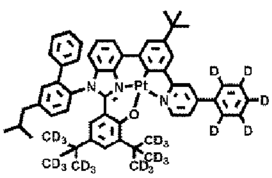
2180

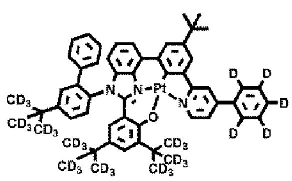
2181

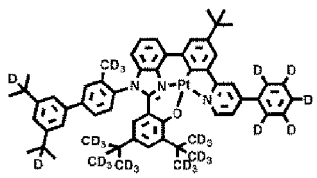
2182

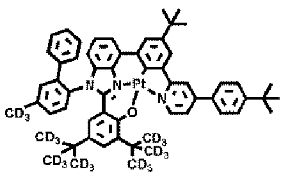
2183

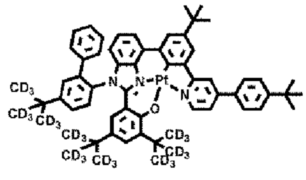
2184

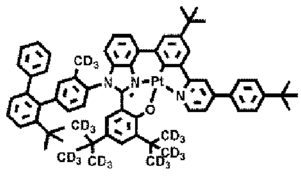
2185

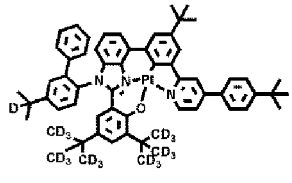
2186

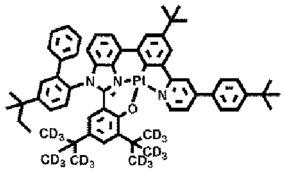
2187

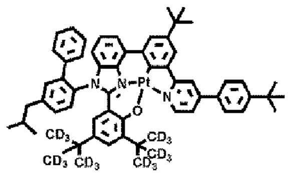
2188

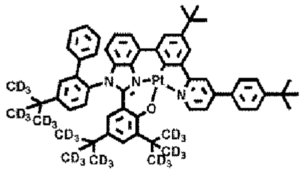
2189

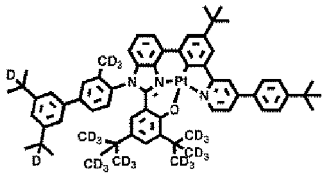
2190

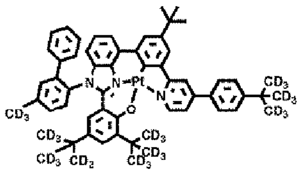
2191

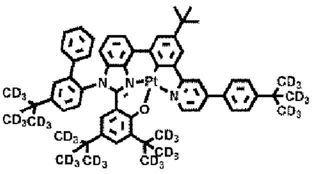
2192

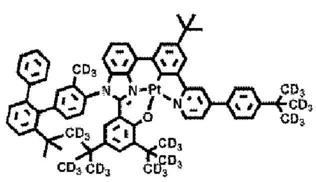
2193

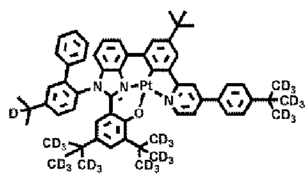
2194

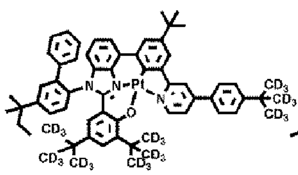
2195

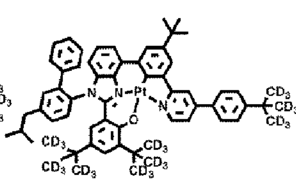
2196

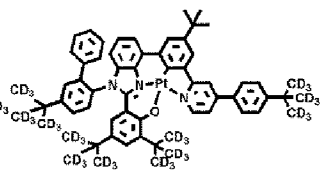
2197

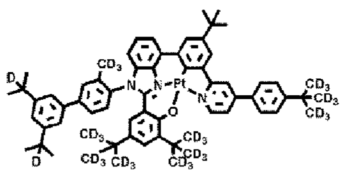
2198

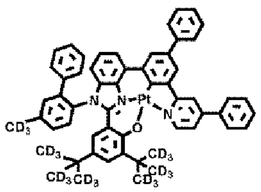
2199

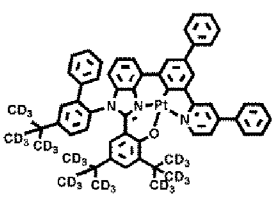
2200

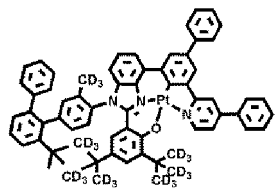
2201

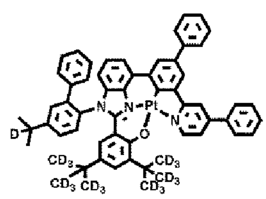
2202

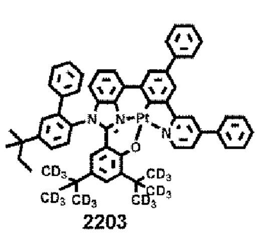
2203

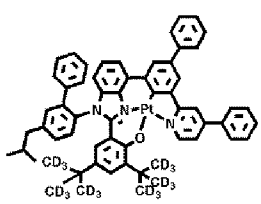
2204

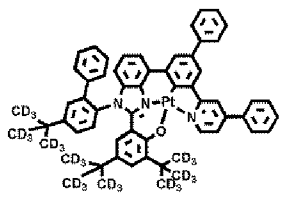
2205

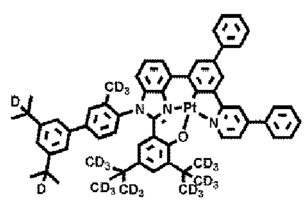
2206

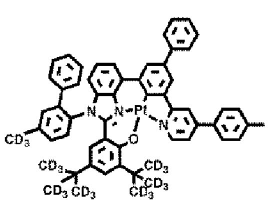
2207

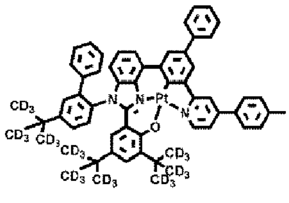
2208

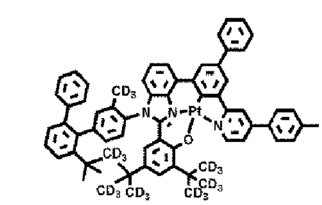
2209

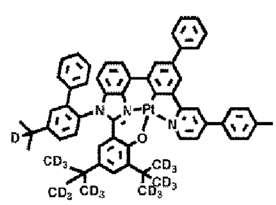
2210

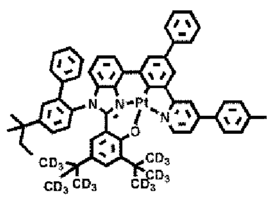
2211
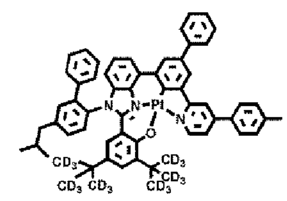
2212
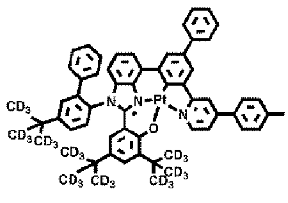
2213
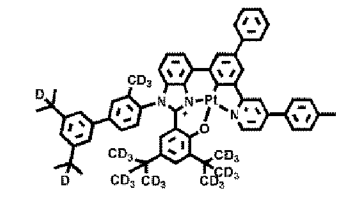
2214

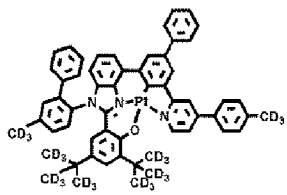
2215
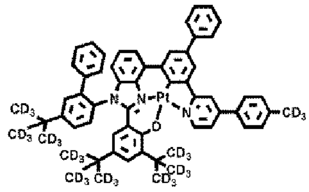
2216
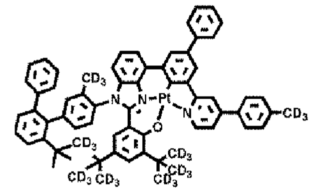
2217
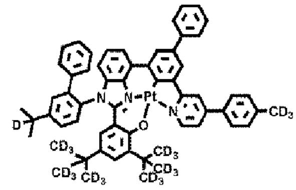
2218

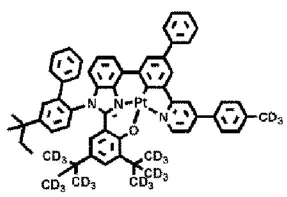
2219
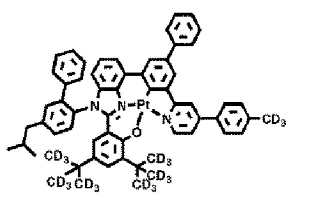
2220
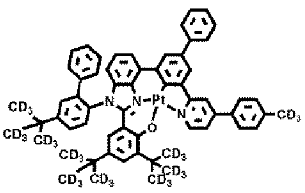
2221
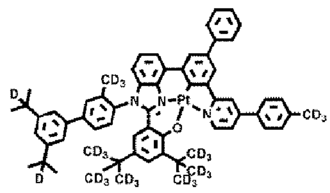
2222

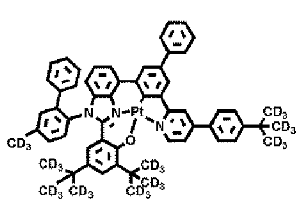
2223
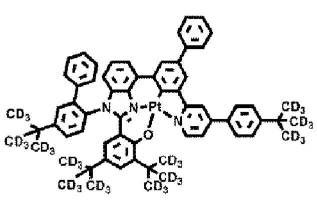
2224
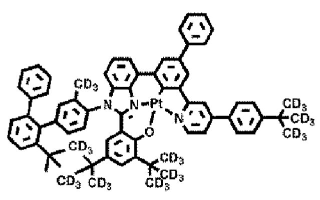
2225
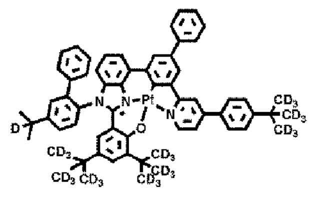
2226

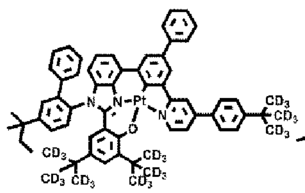
2227
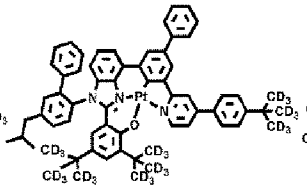
2228
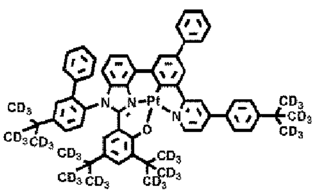
2229
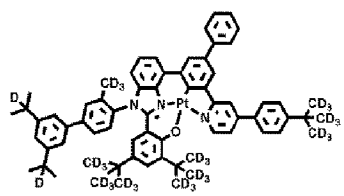
2230

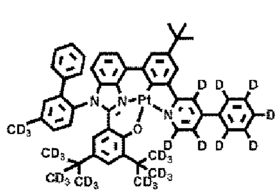
2231
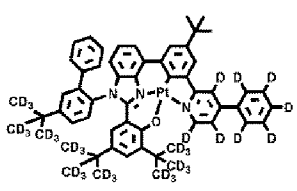
2232
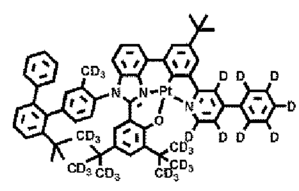
2233
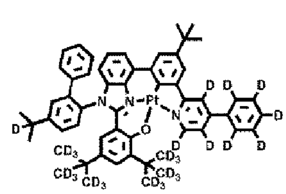
2234

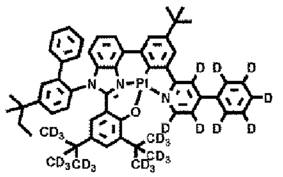

2235

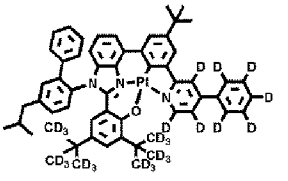

2236

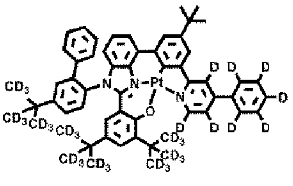

2237

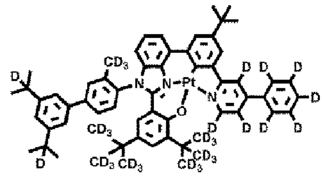

2238

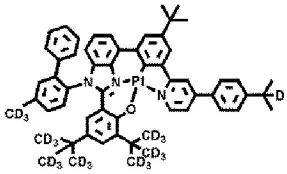

2239

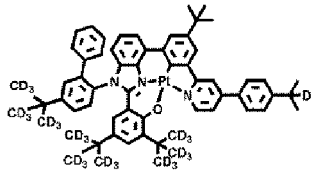

2240

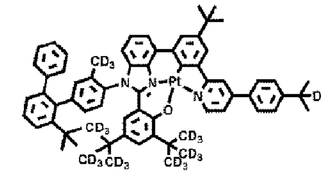

2241

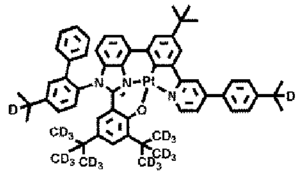

2242

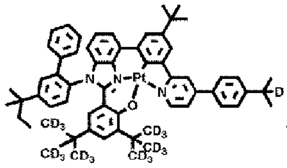

2243

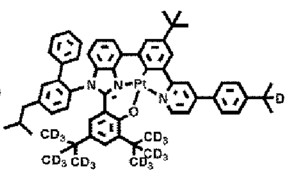

2244

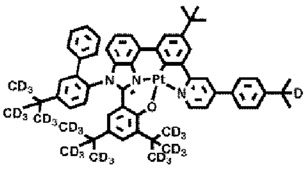

2245

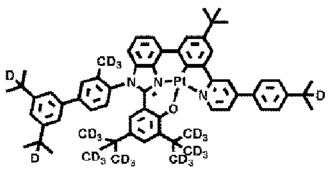

2246

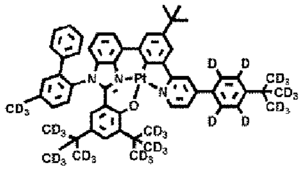

2247

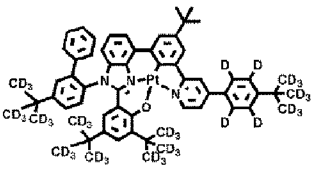

2248

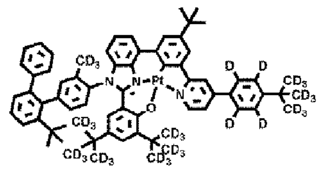

2249

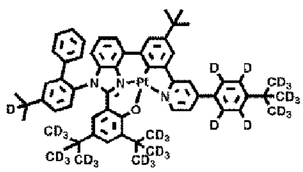

2250

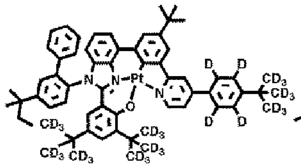

2251

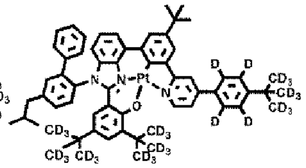

2252

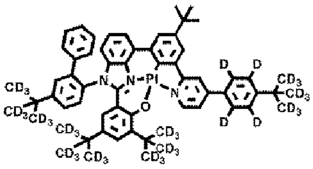

2253

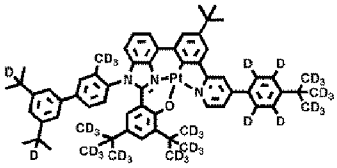

2254

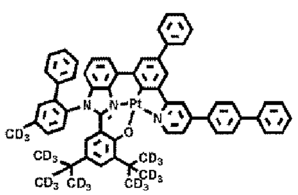

2255

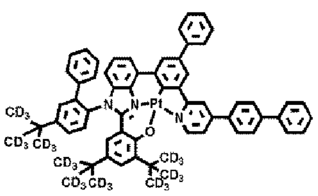

2256

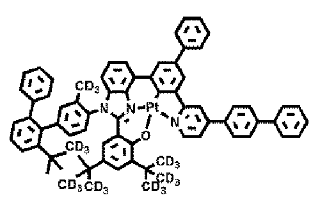

2257

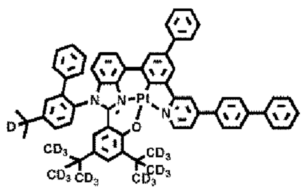

2258

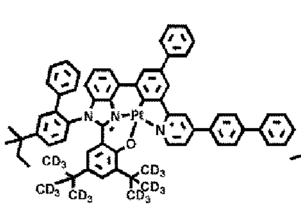

2259

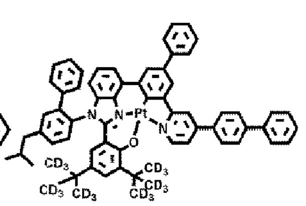

2260

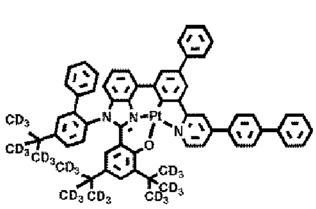

2261

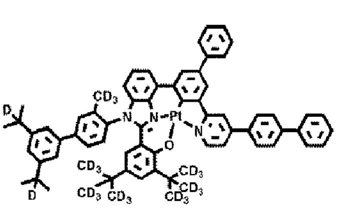

2262

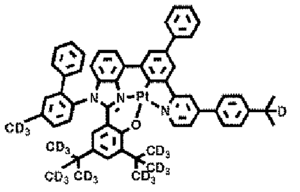
2263

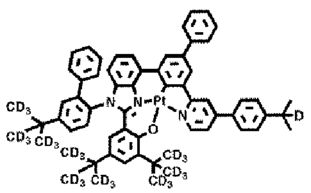
2264

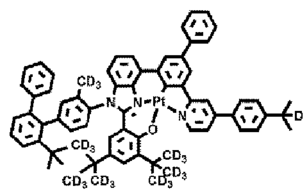
2265

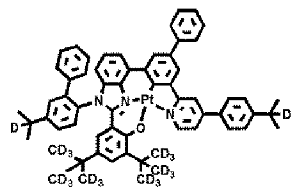
2266

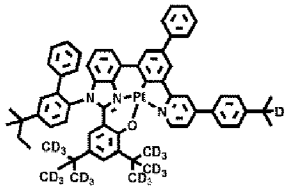
2267

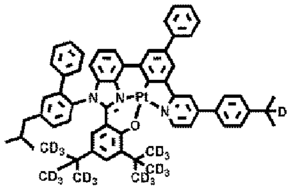
2268

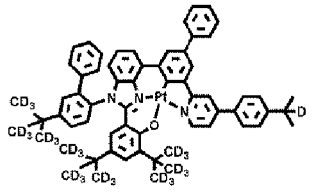
2269

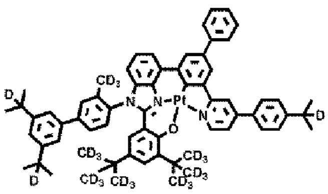
2270

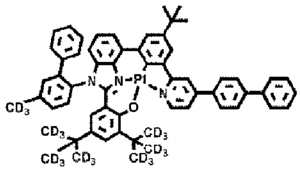
2271

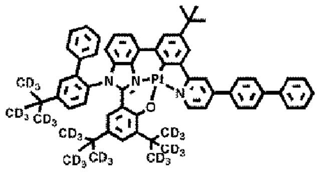
2272

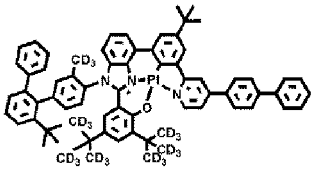
2273

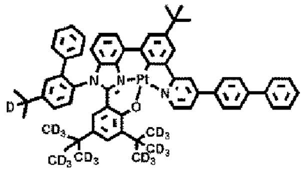
2274

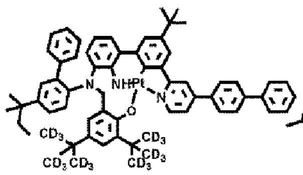
2275

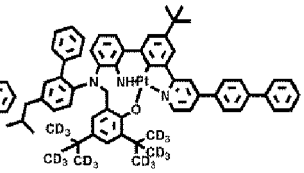
2276

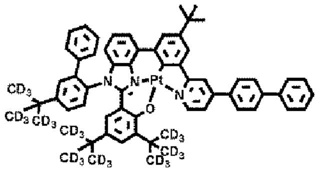
2277

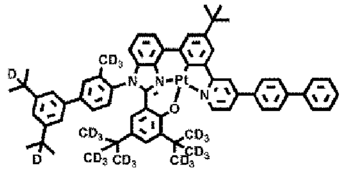
2278

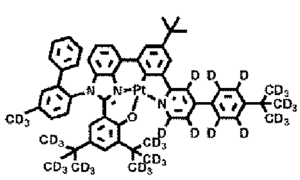
2279

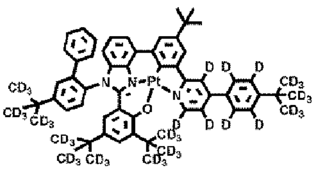
2280

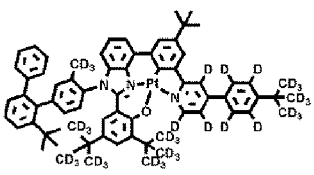
2281

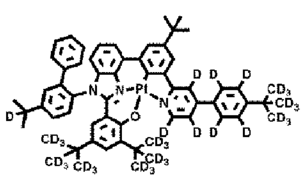
2282

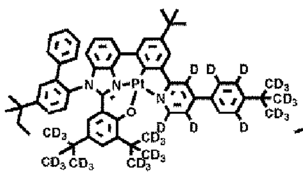
2283

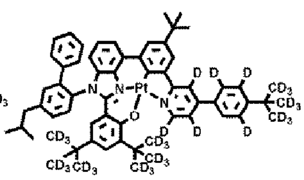
2284

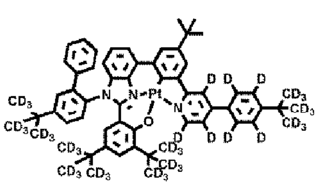
2285

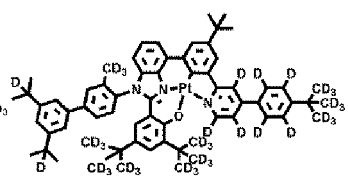
2286

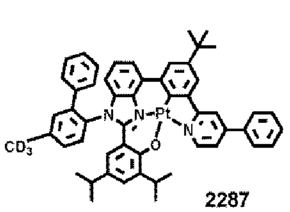
2287

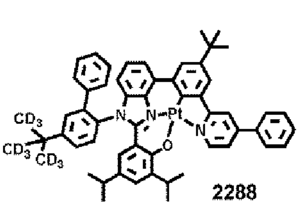
2288

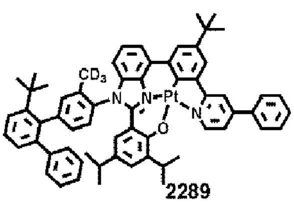
2289

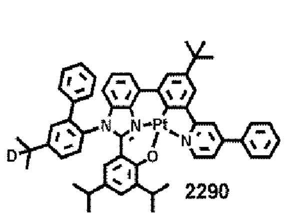
2290

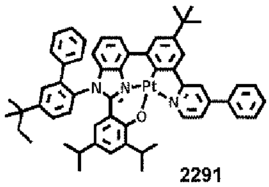
2291

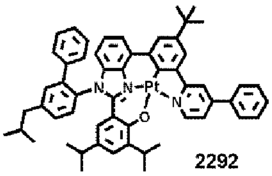
2292

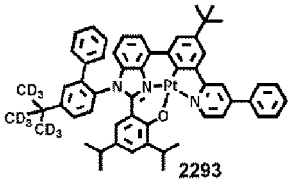
2293

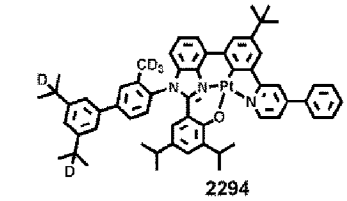
2294

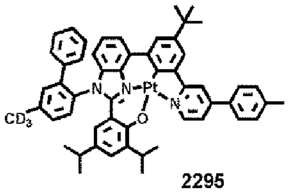
2295

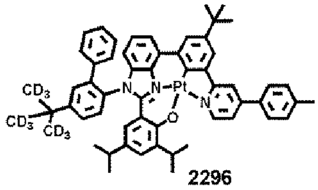
2296

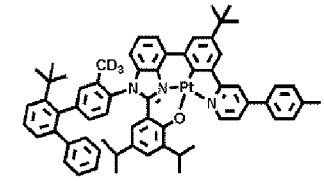
2297

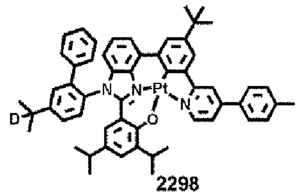
2298

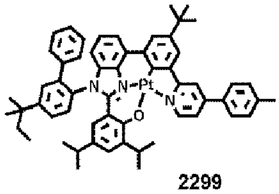
2299

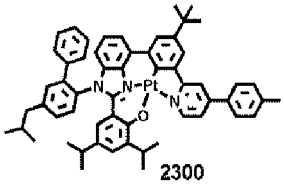
2300

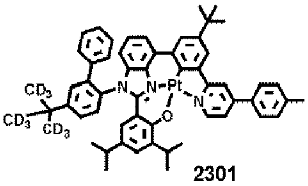
2301

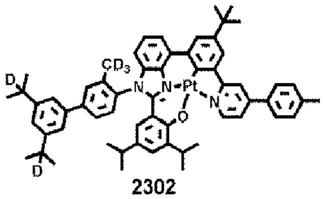
2302

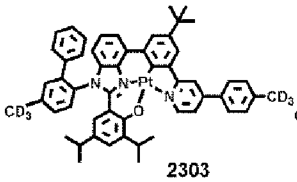
2303

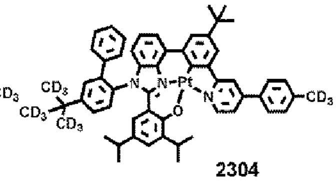
2304

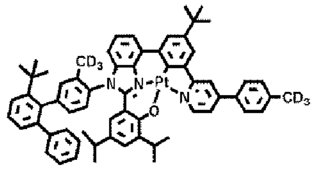
2305

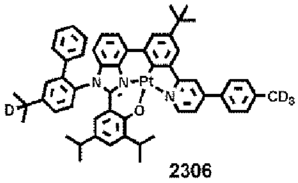
2306

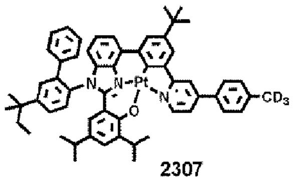
2307

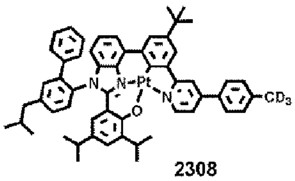
2308

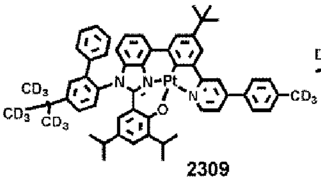
2309

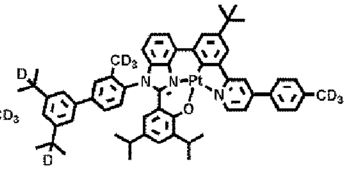
2310

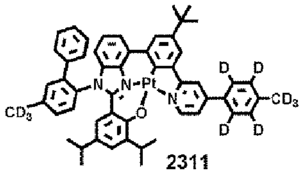
2311

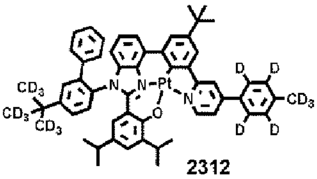
2312

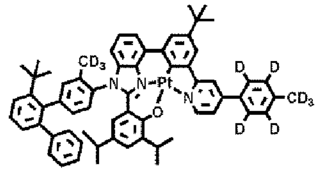
2313

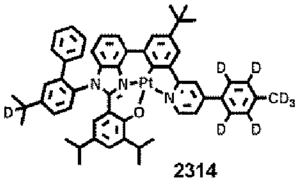
2314

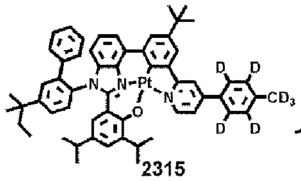
2315

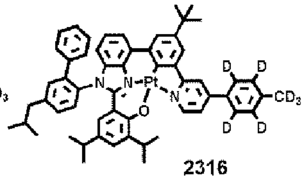
2316

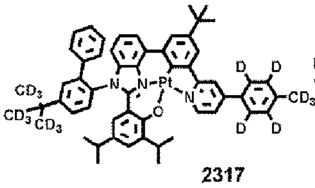
2317

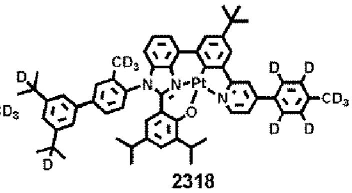
2318

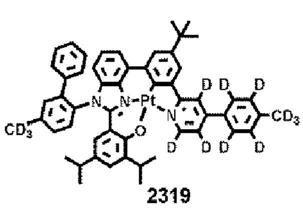
2319

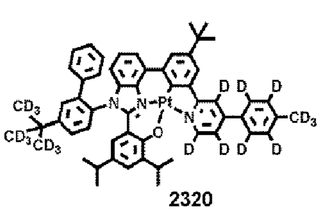
2320

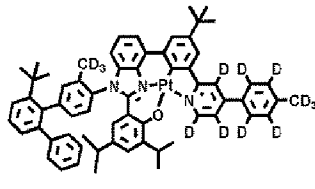
2321

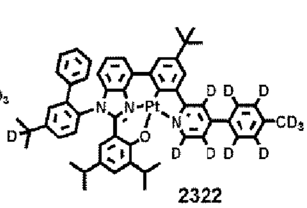
2322

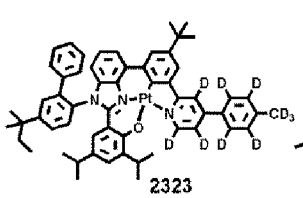
2323

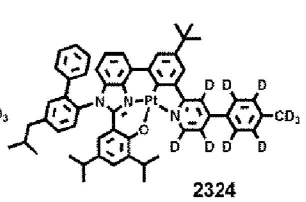
2324

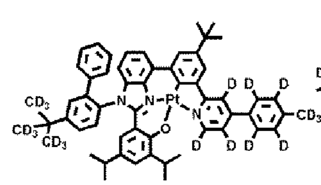
2325

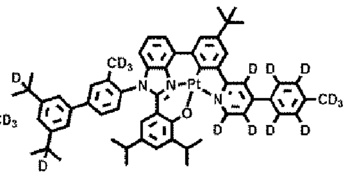
2326

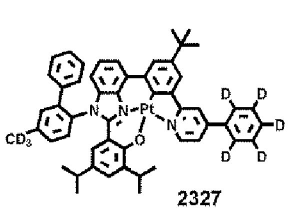
2327

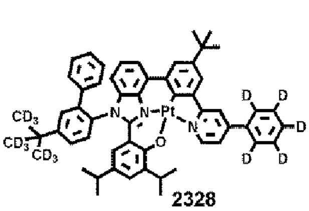
2328

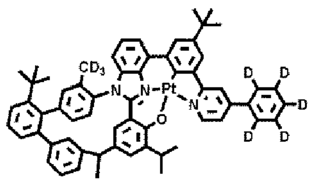
2329

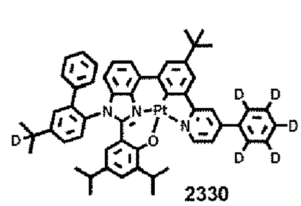
2330

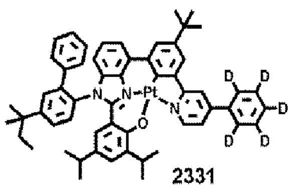
2331

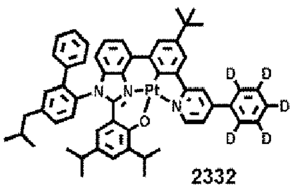
2332

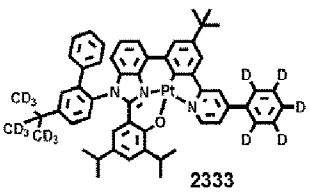
2333

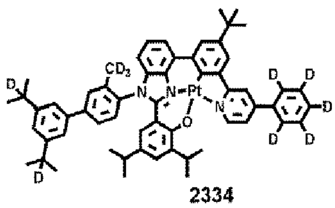
2334

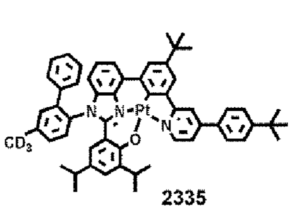
2335

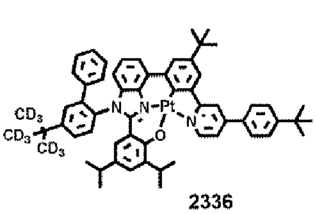
2336

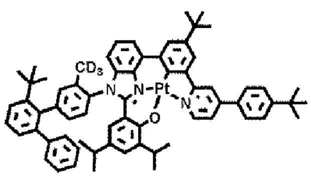
2337

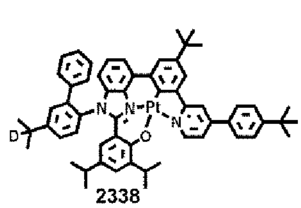
2338

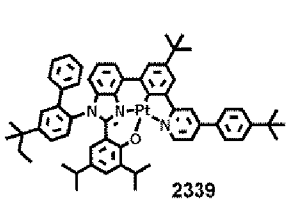
2339

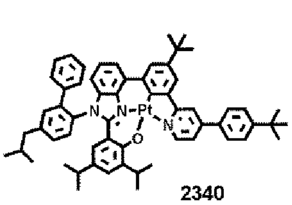
2340

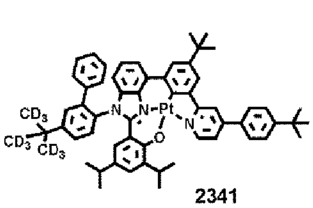
2341

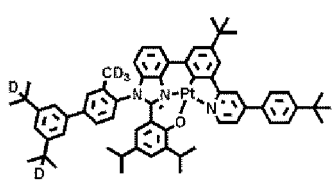
2342

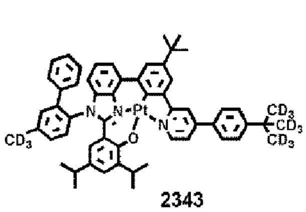
2343

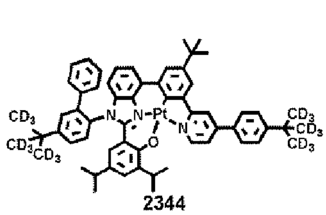
2344

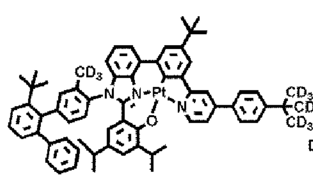
2345

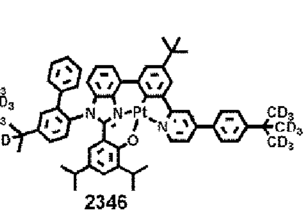
2346

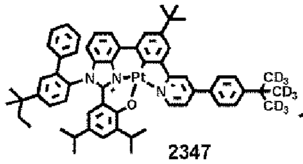
2347

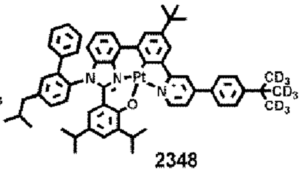
2348

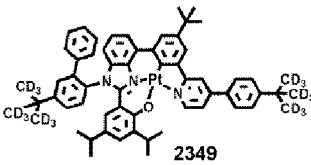
2349

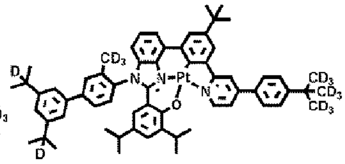
2350

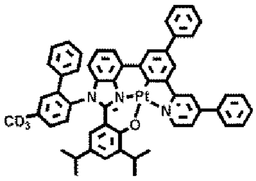
2351

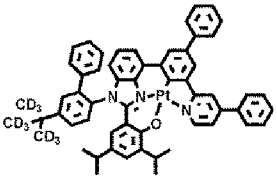
2352

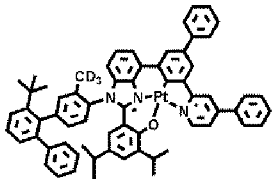
2353

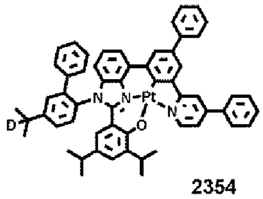
2354

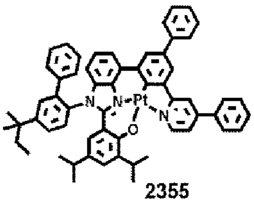
2355

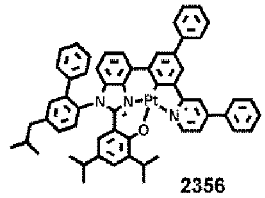
2356

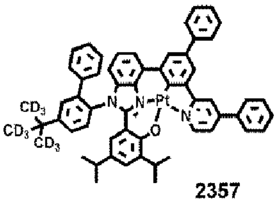
2357

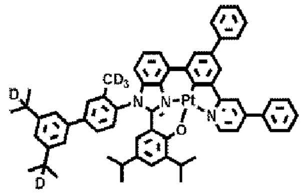
2358

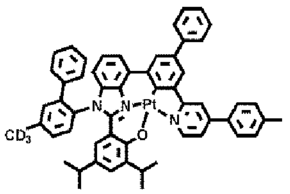
2359

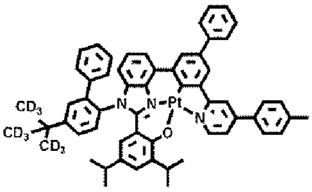
2360

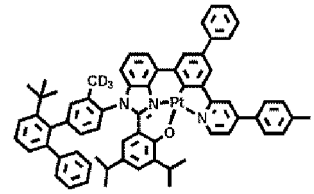
2361

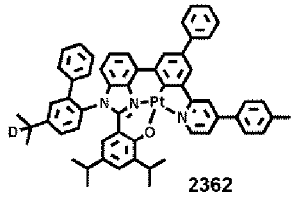
2362

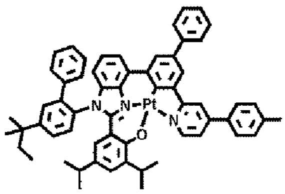
2363

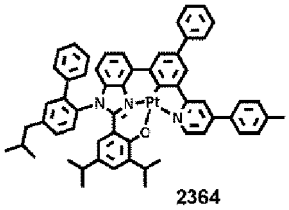
2364

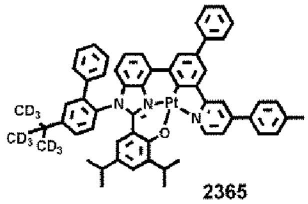
2365

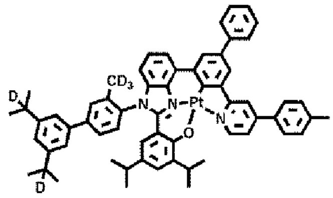
2366

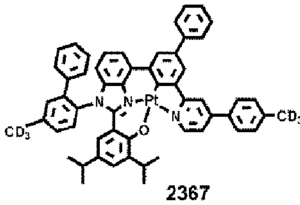
2367

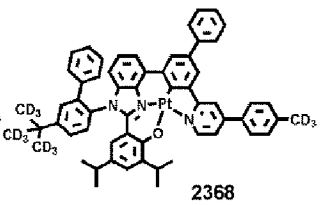
2368

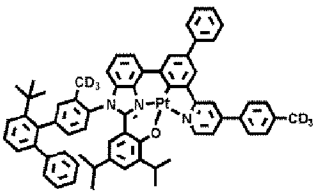
2369

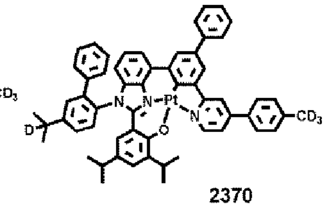
2370

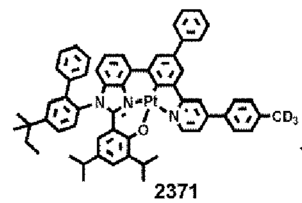
2371
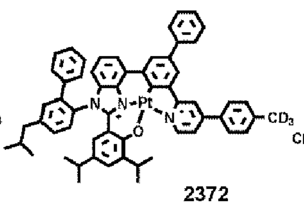
2372
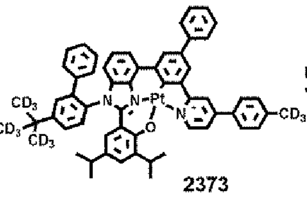
2373
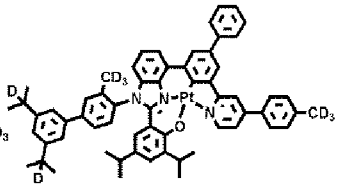
2374

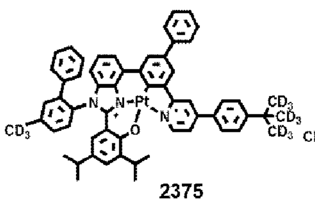
2375
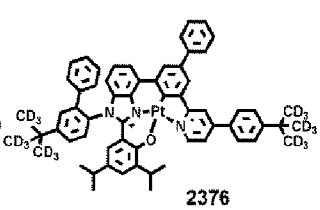
2376
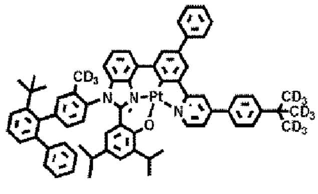
2377
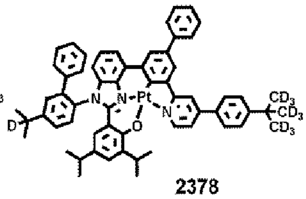
2378

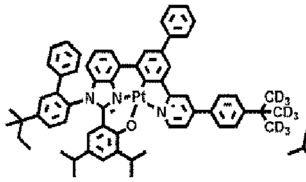
2379
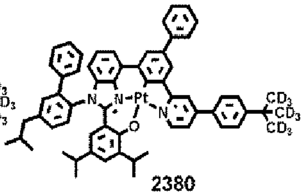
2380
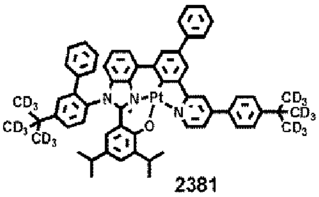
2381
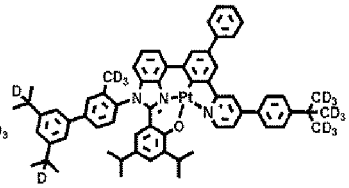
2382

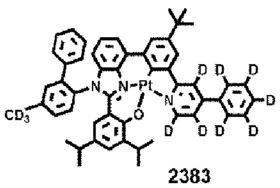
2383
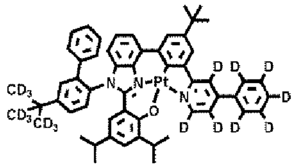
2384
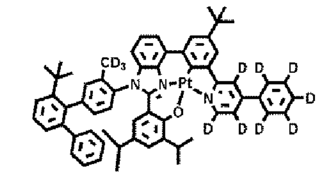
2385
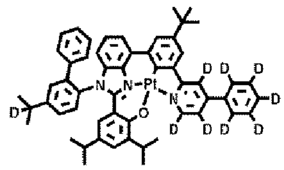
2386

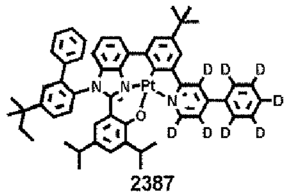
2387
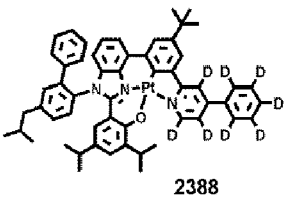
2388
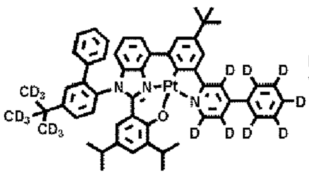
2389
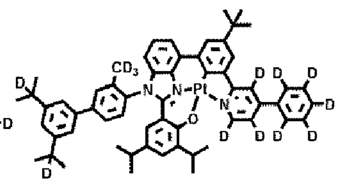
2390

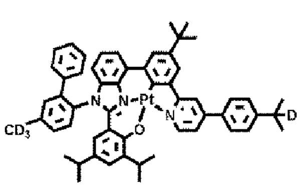
2391
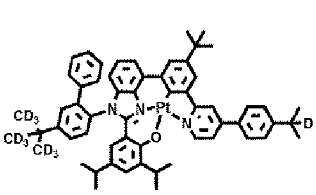
2392
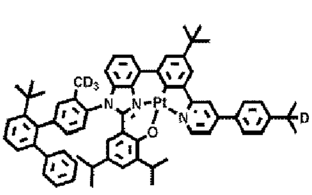
2393
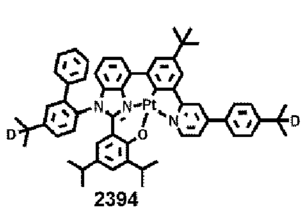
2394

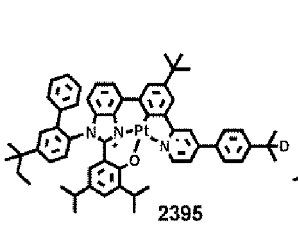
2395
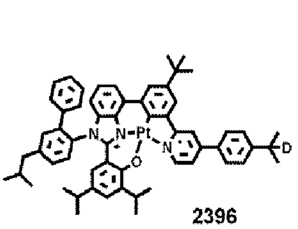
2396
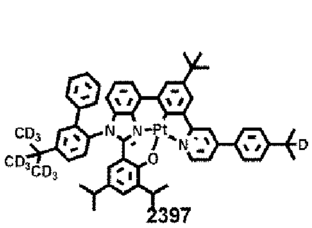
2397
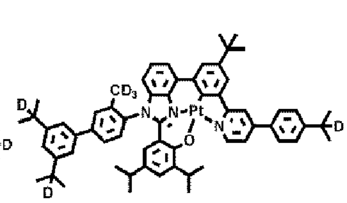
2398

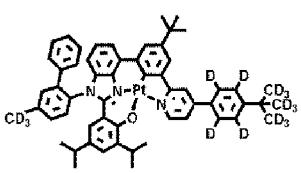
2399

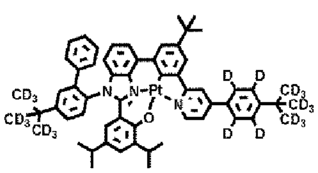
2400

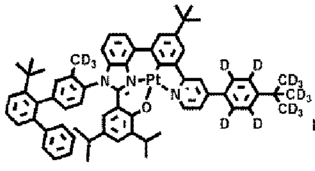
2401

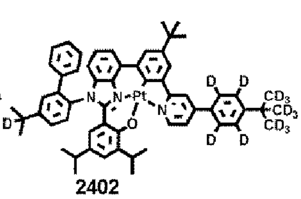
2402

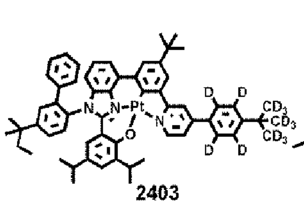
2403

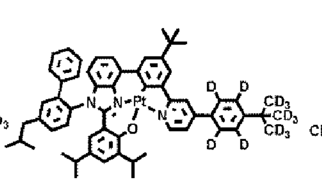
2404

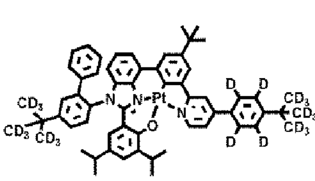
2405

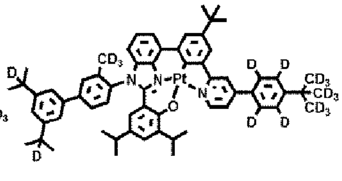
2406

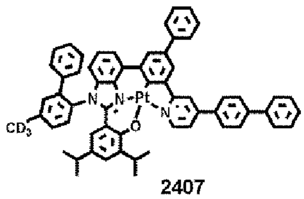
2407

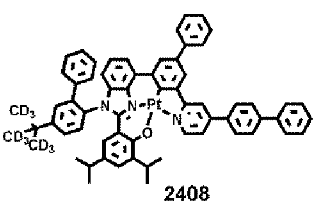
2408

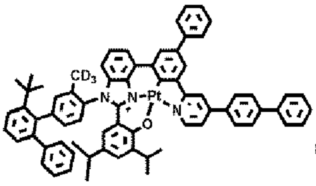
2409

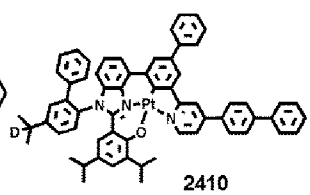
2410

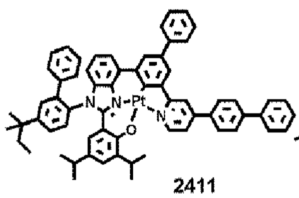
2411

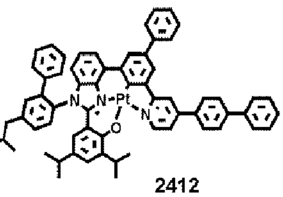
2412

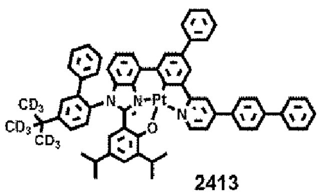
2413

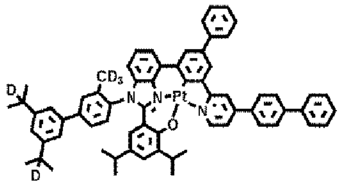
2414

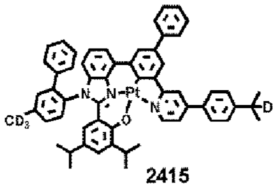
2415

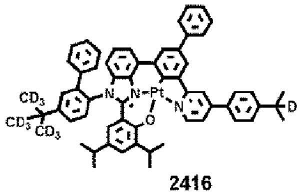
2416

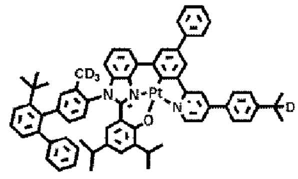
2417

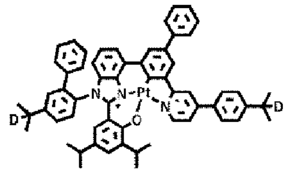
2418

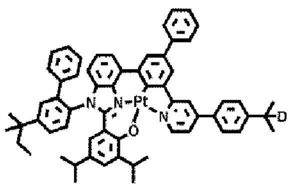
2419

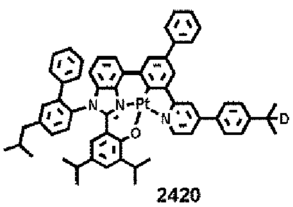
2420

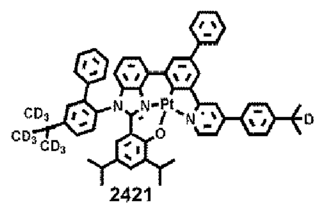
2421

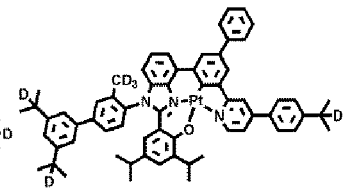
2422

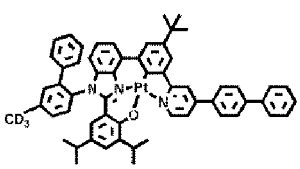
2423

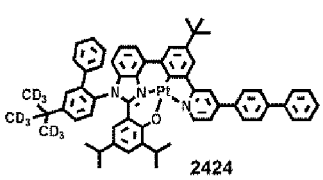
2424

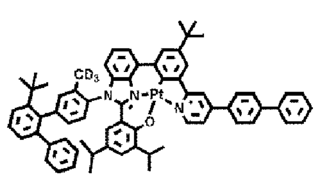
2425

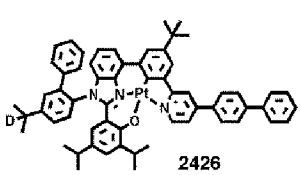
2426

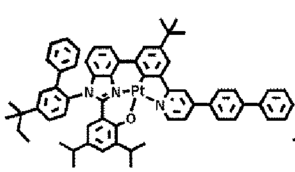
2427

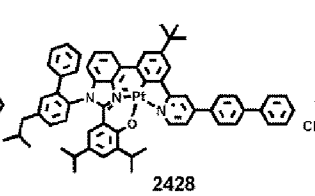
2428

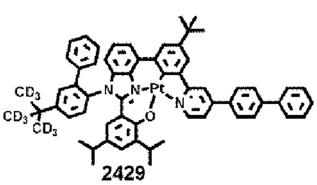
2429

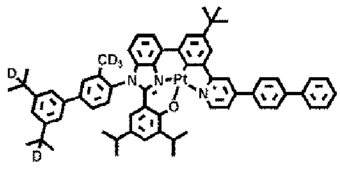
2430

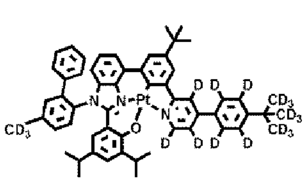
2431

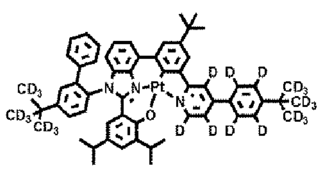
2432

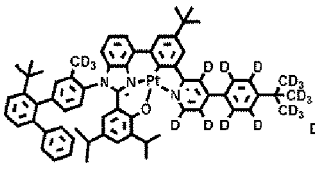
2433

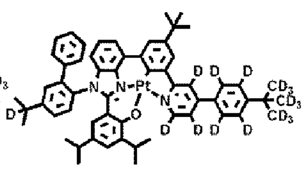
2434

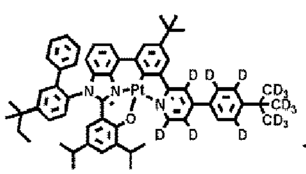
2435

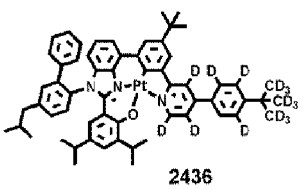
2436

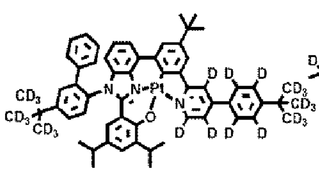
2437

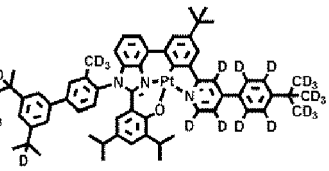
2438

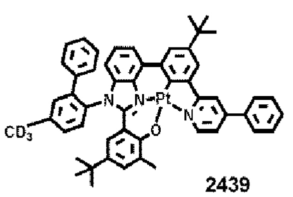
2439

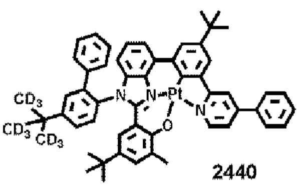
2440

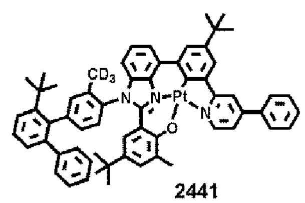
2441

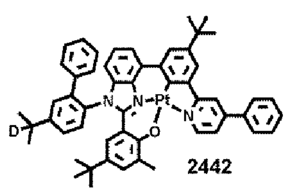
2442

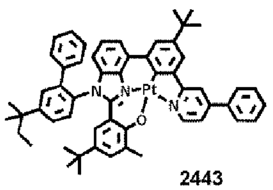
2443

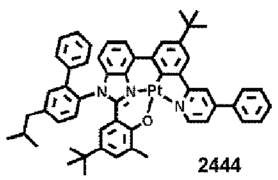
2444

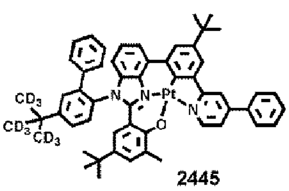
2445

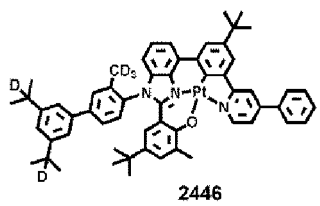
2446

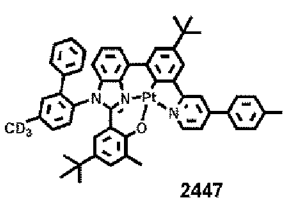
2447

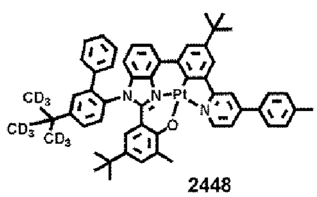
2448

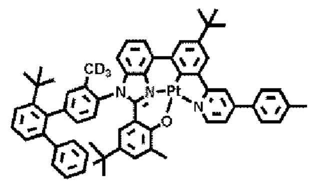
2449

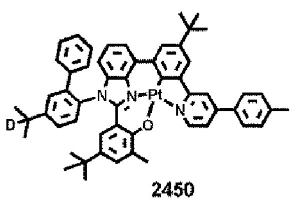
2450

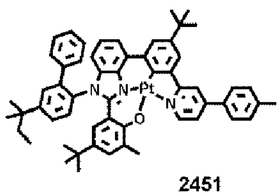
2451

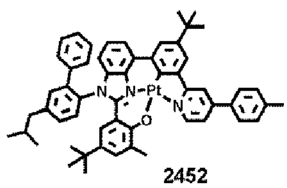
2452

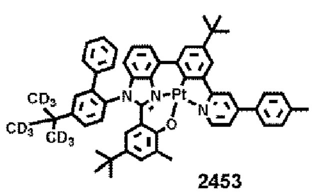
2453

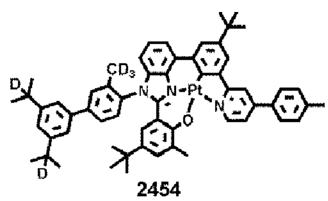
2454

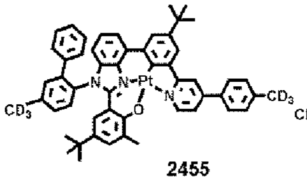
2455

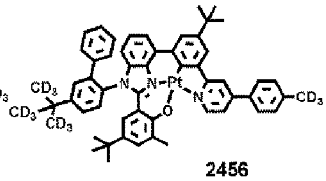
2456

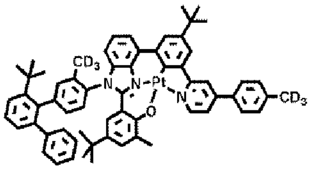
2457

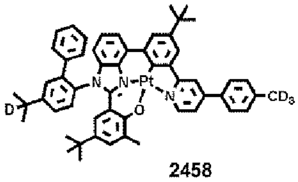
2458

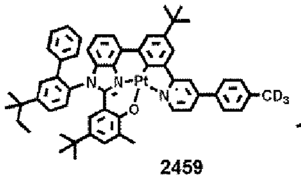
2459

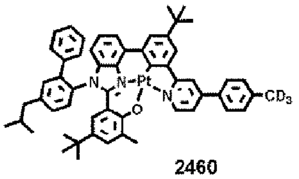
2460

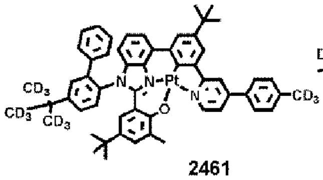
2461

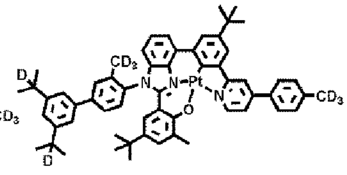
2462

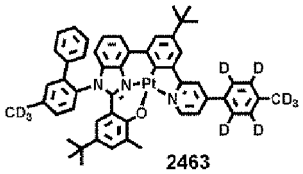
2463

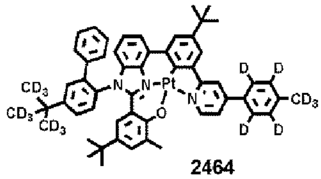
2464

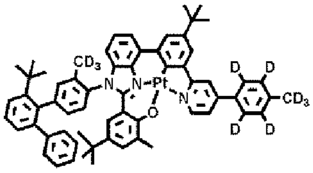
2465

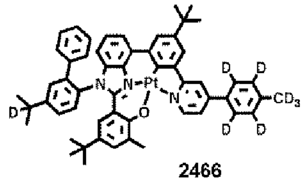
2466

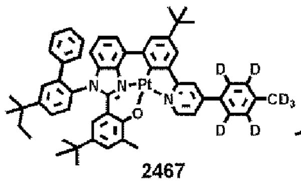
2467

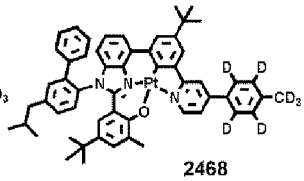
2468

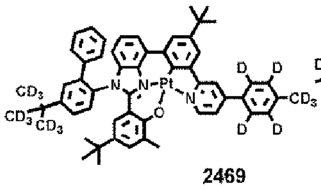
2469

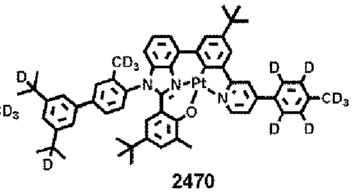
2470

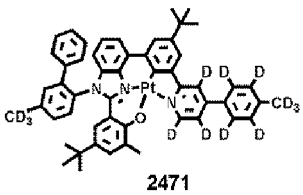
2471

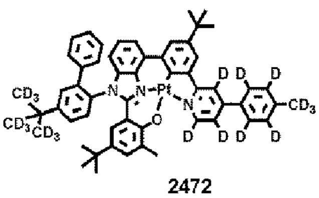
2472

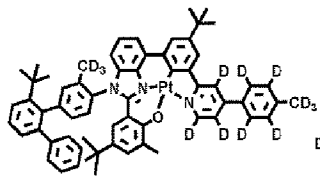
2473

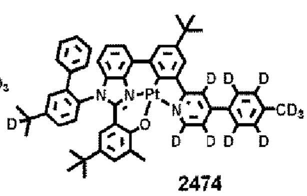
2474

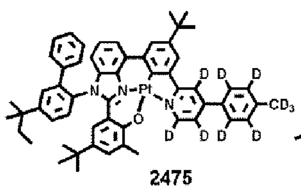
2475

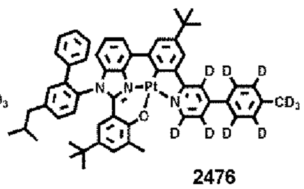
2476

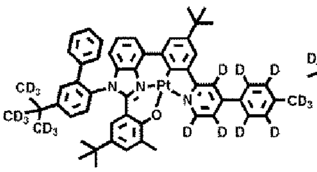
2477

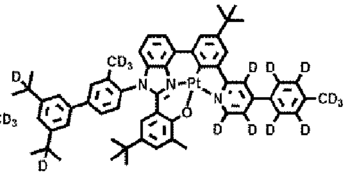
2478

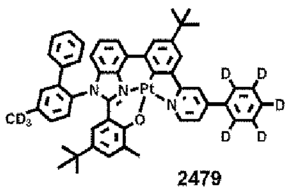
2479

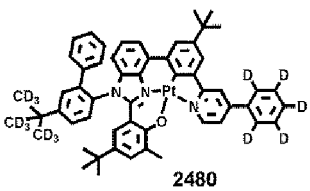
2480

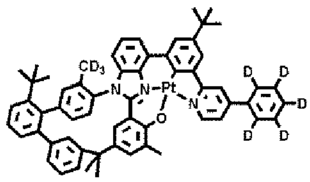
2481

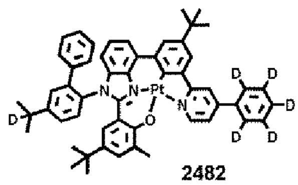
2482

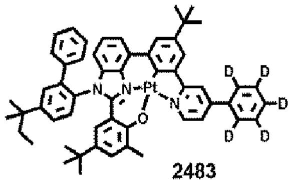
2483

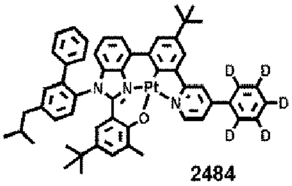
2484

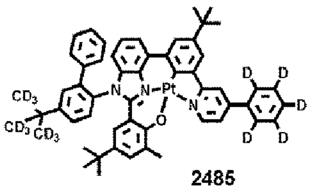
2485

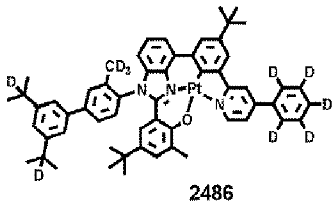
2486

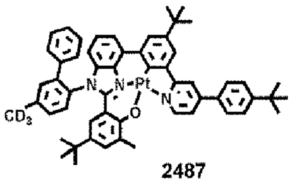
2487

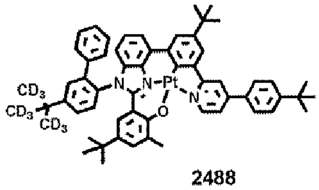
2488

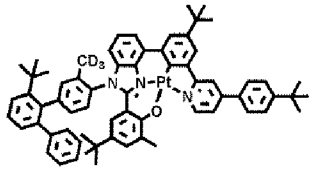
2489

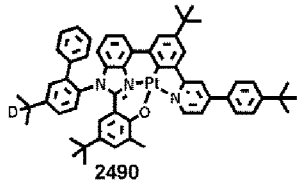
2490

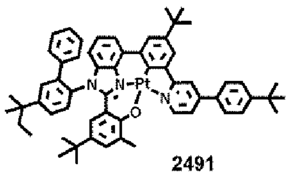
2491

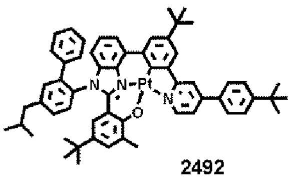
2492

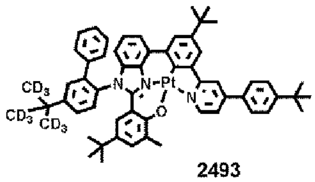
2493

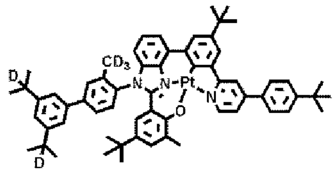
2494

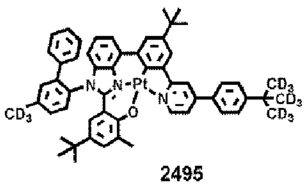
2495

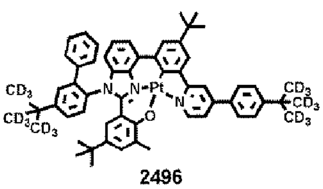
2496

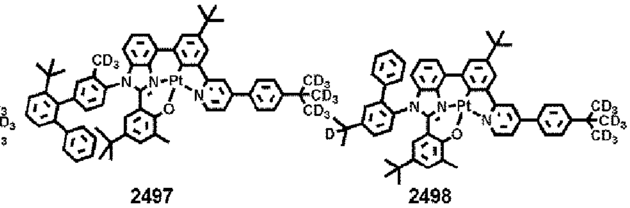
2497

2498

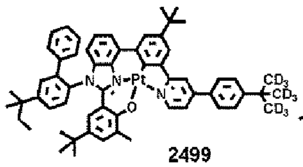
2499

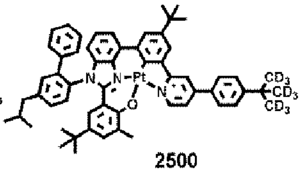
2500

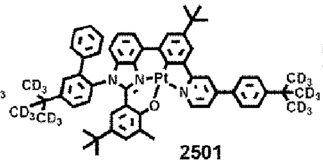
2501

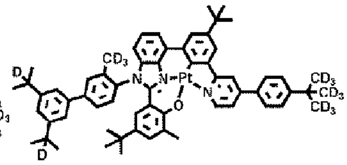
2502

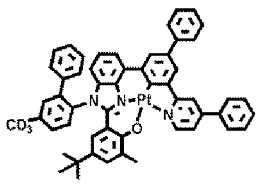
2503

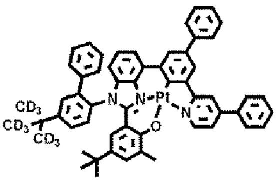
2504

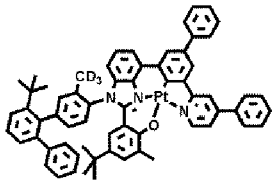
2505

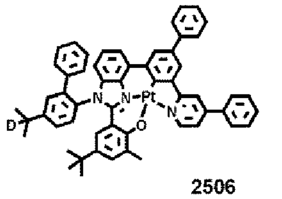
2506

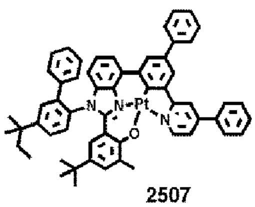
2507

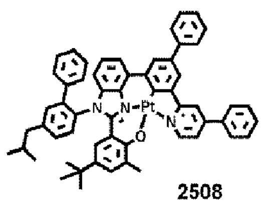
2508

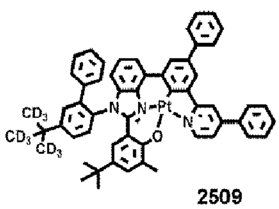
2509

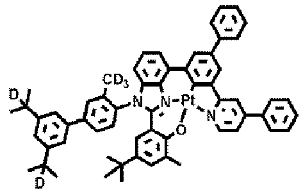
2510

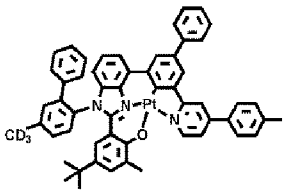
2511

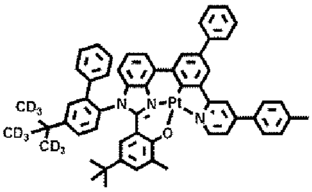
2512

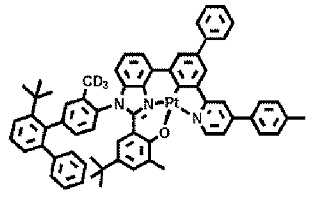
2513

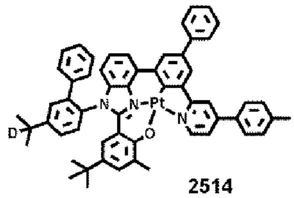
2514

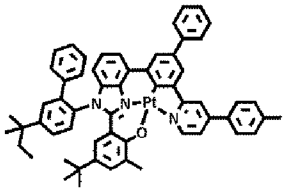
2515

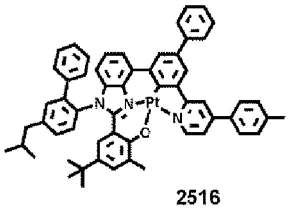
2516

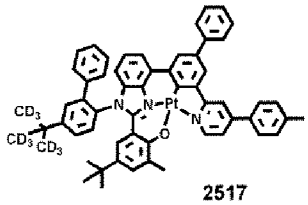
2517

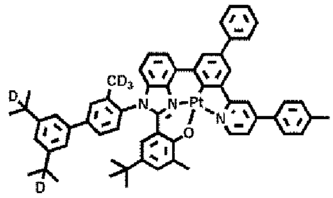
2518

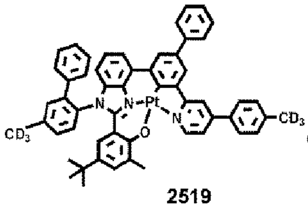
2519

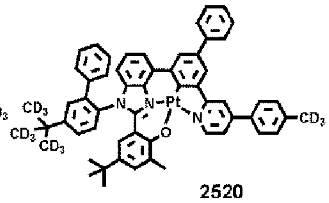
2520

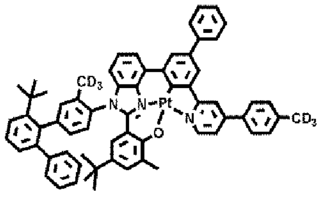
2521

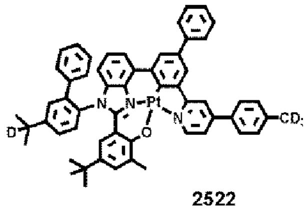
2522

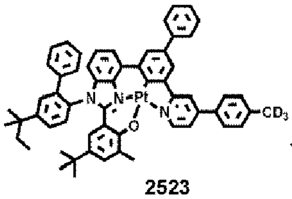
2523

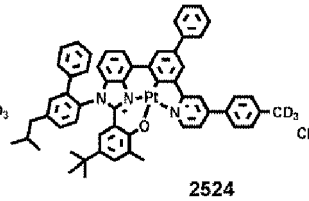
2524

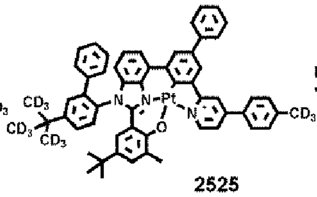
2525

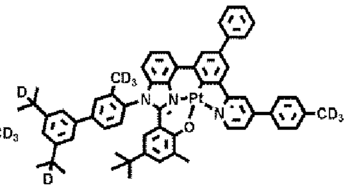
2526

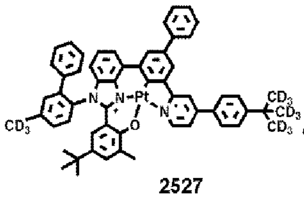
2527

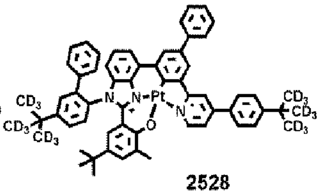
2528

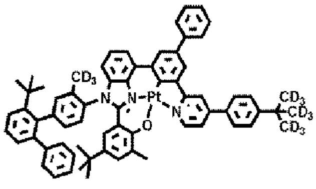
2529

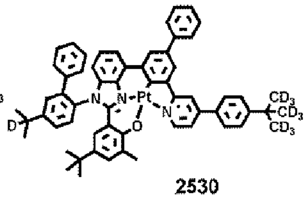
2530

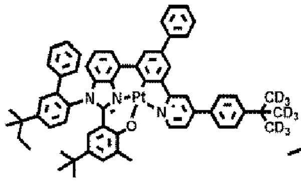
2531

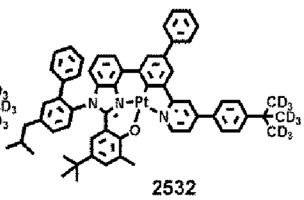
2532

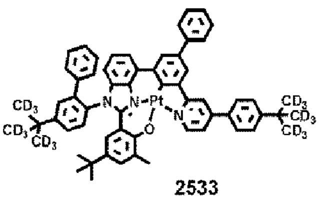
2533

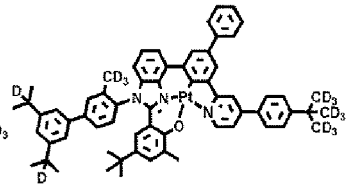
2534

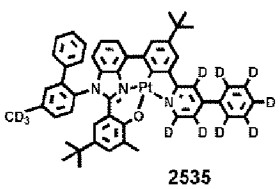
2535
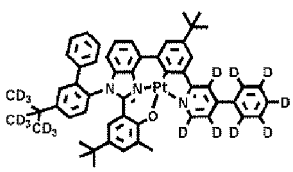
2536
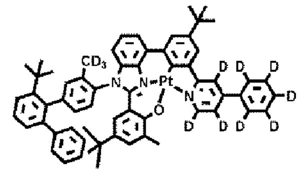
2537
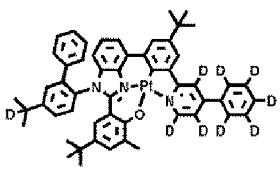
2538

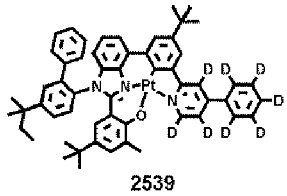
2539
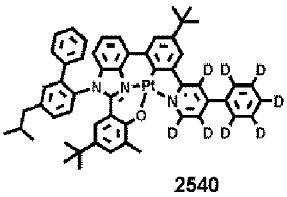
2540
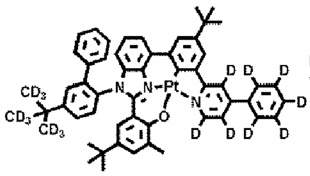
2541
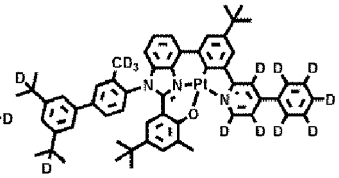
2542

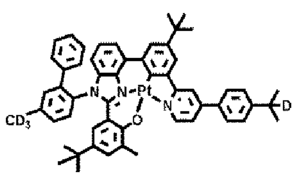
2543
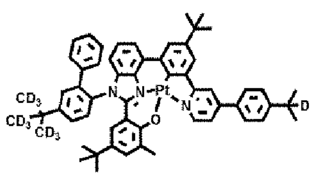
2544
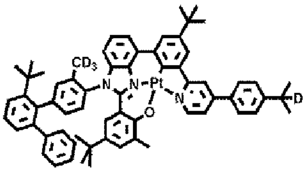
2545
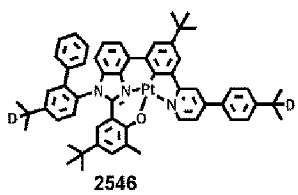
2546

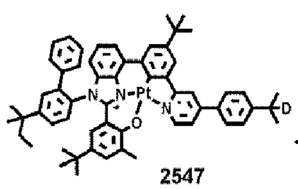
2547
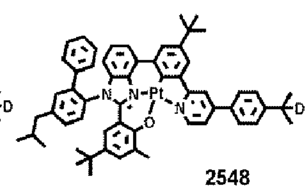
2548
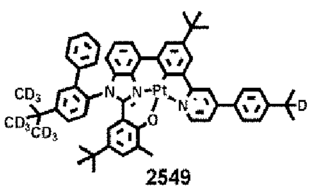
2549
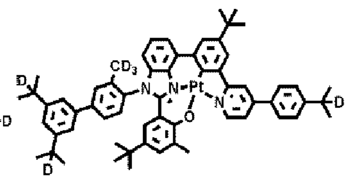
2550

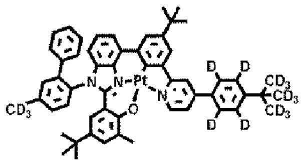
2551
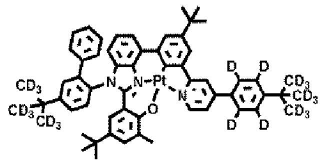
2552
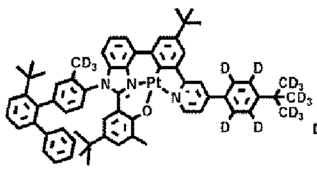
2553
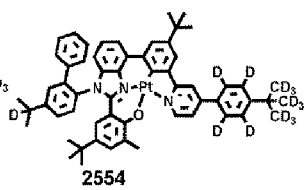
2554

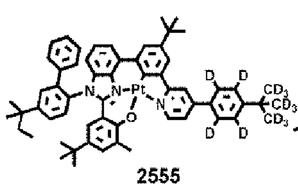
2555
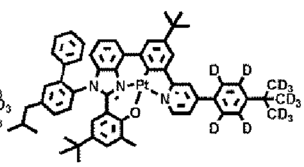
2556
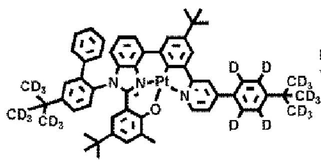
2557
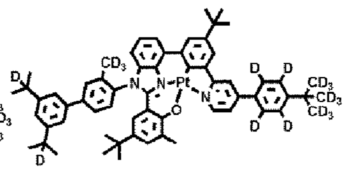
2558

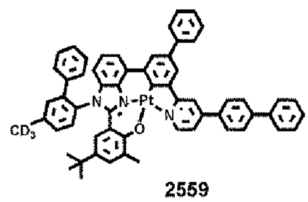
2559
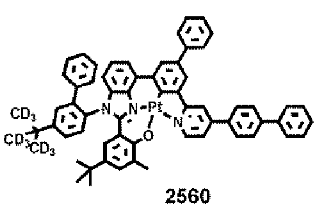
2560
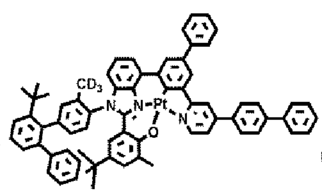
2561
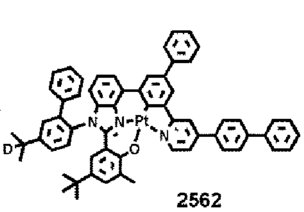
2562

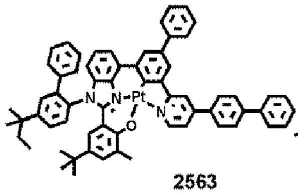
2563
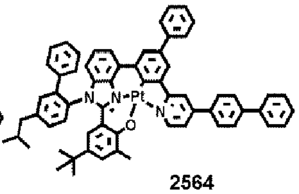
2564
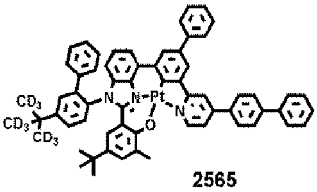
2565
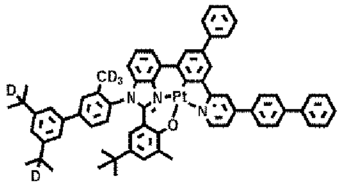
2566
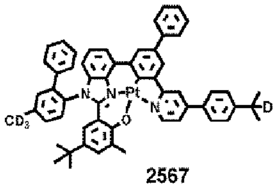
2567
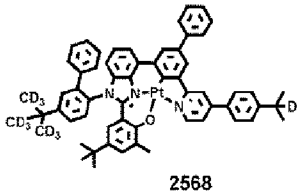
2568
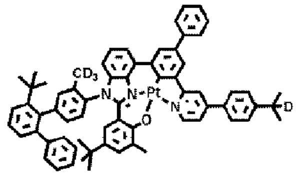
2569
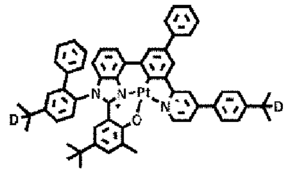
2570
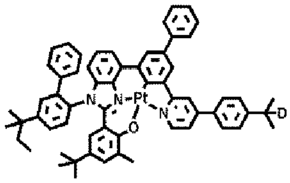
2571
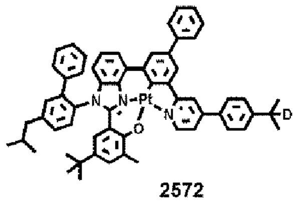
2572
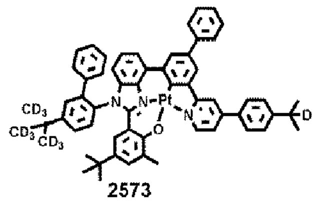
2573
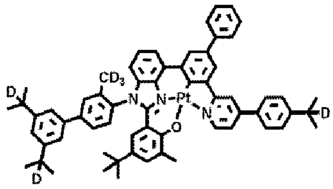
2574
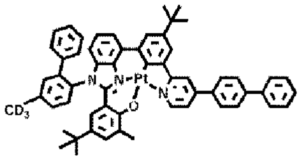
2575
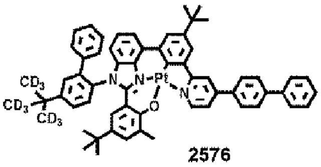
2576
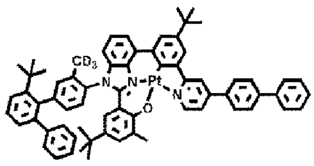
2577
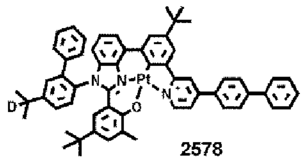
2578
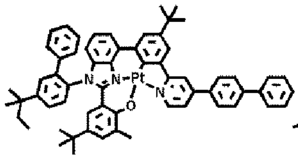
2579
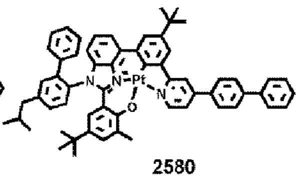
2580
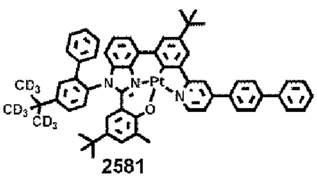
2581
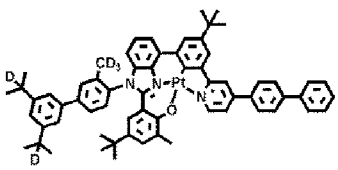
2582
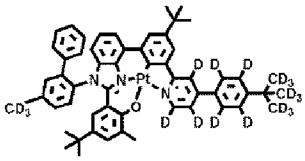
2583
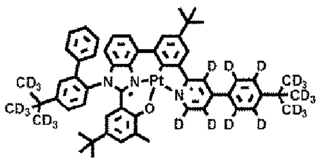
2584
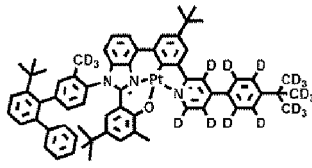
2585
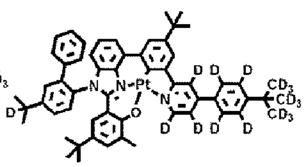
2586
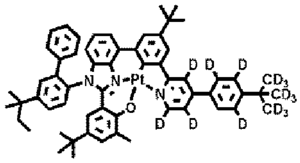
2587
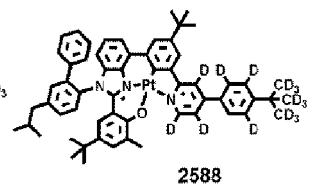
2588
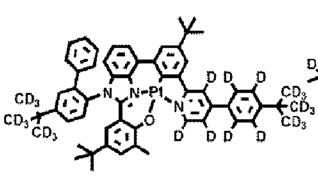
2589
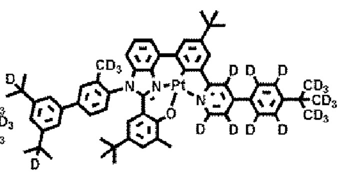
2590

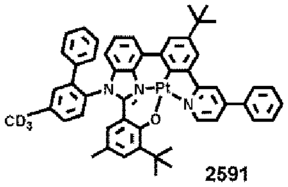
2591

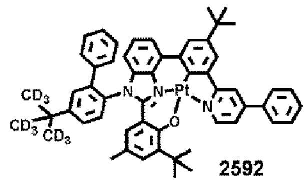
2592

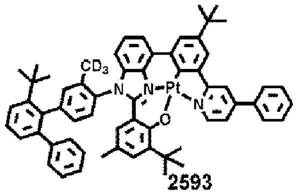
2593

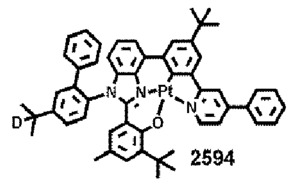
2594

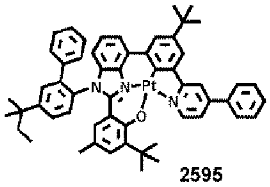
2595

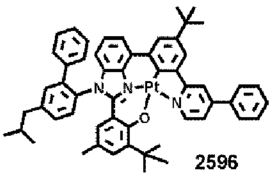
2596

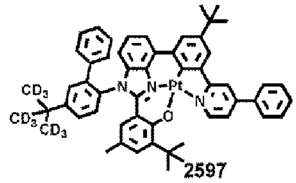
2597

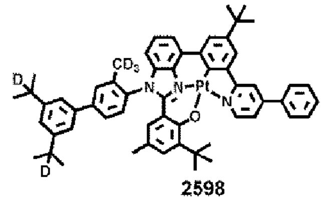
2598

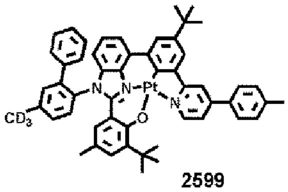
2599

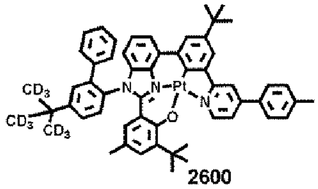
2600

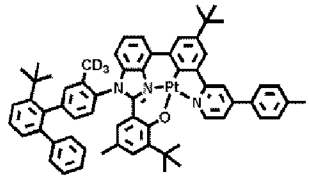
2601

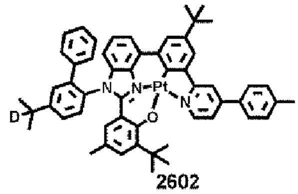
2602

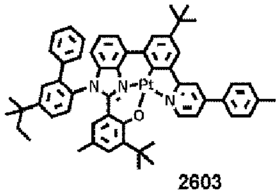
2603

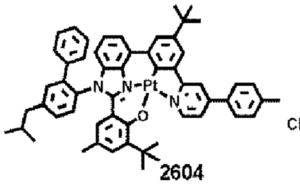
2604

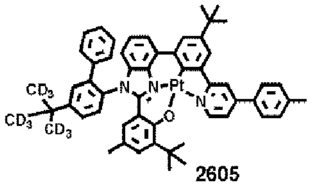
2605

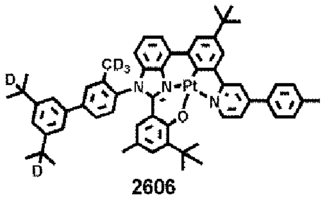
2606

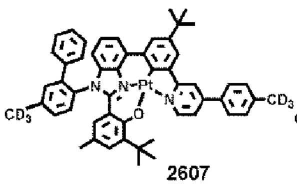
2607

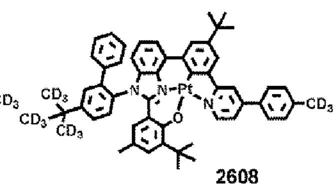
2608

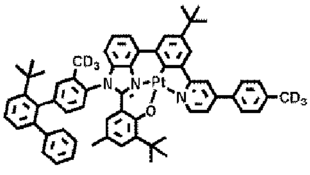
2609

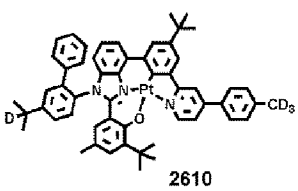
2610

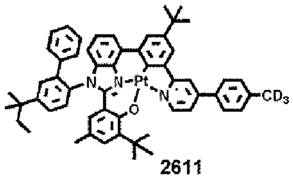
2611

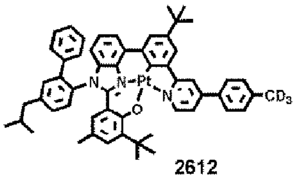
2612

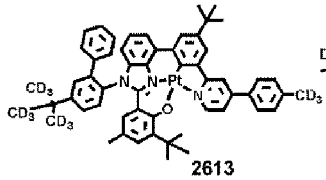
2613

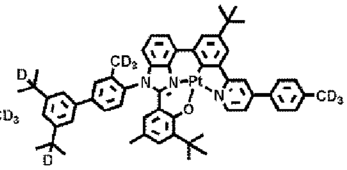
2614

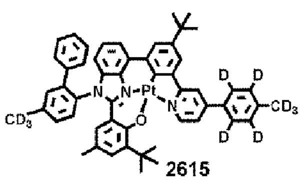
2615

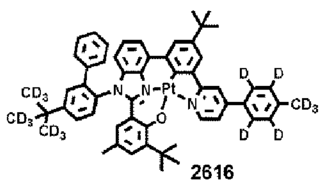
2616

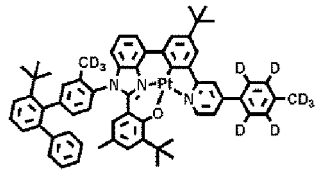
2617

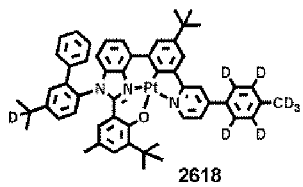
2618

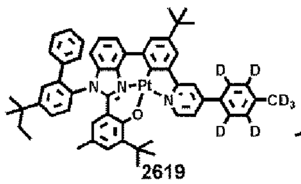
2619

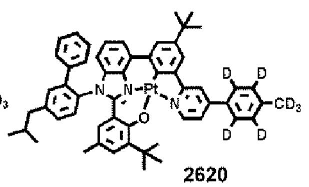
2620

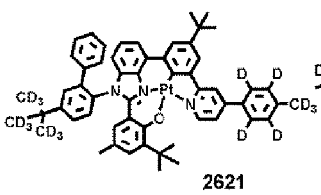
2621

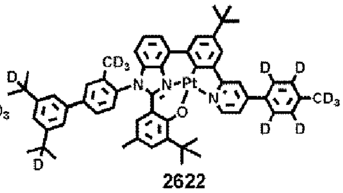
2622

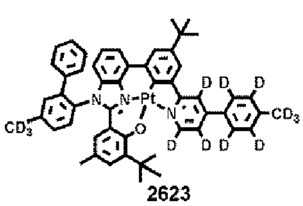
2623

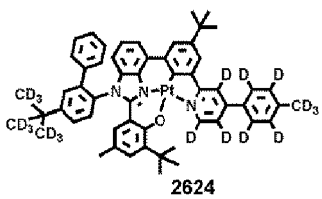
2624

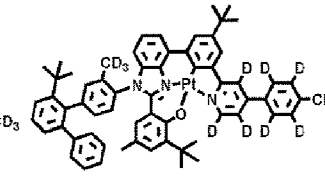
2625

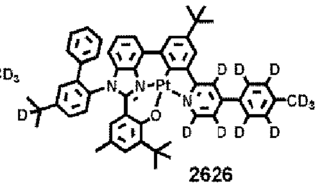
2626

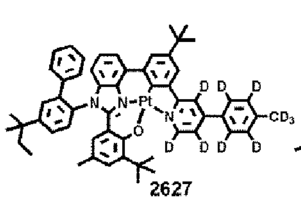
2627

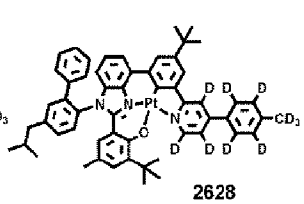
2628

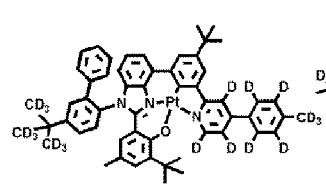
2629

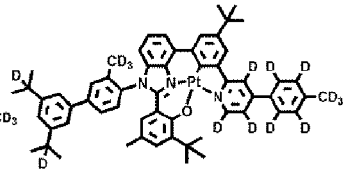
2630

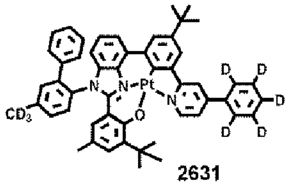
2631

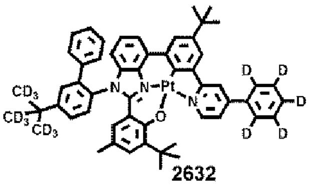
2632

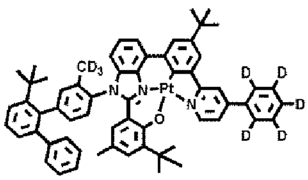
2633

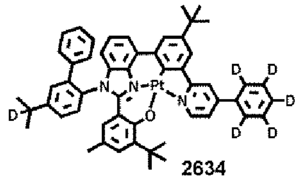
2634

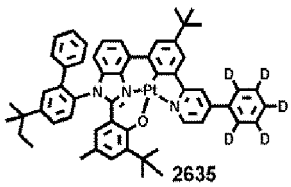
2635

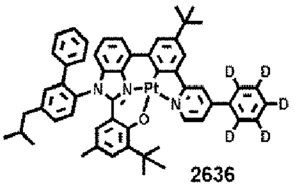
2636

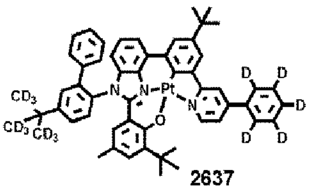
2637

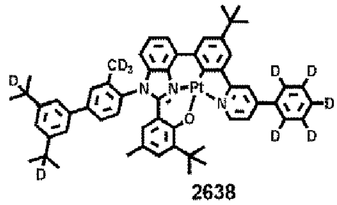
2638

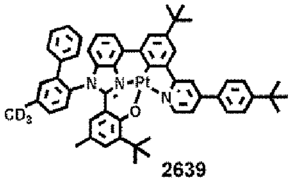
2639

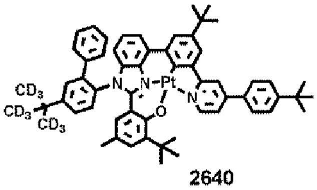
2640

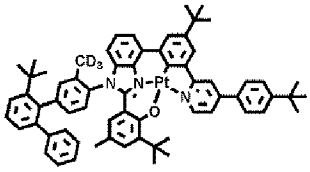
2641

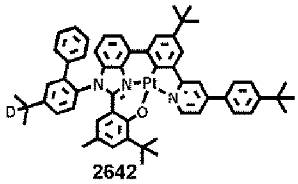
2642

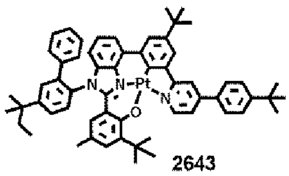
2643

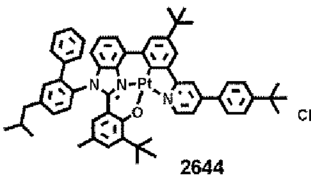
2644

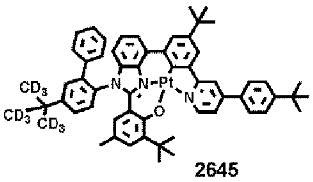
2645

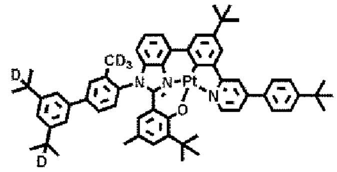
2646

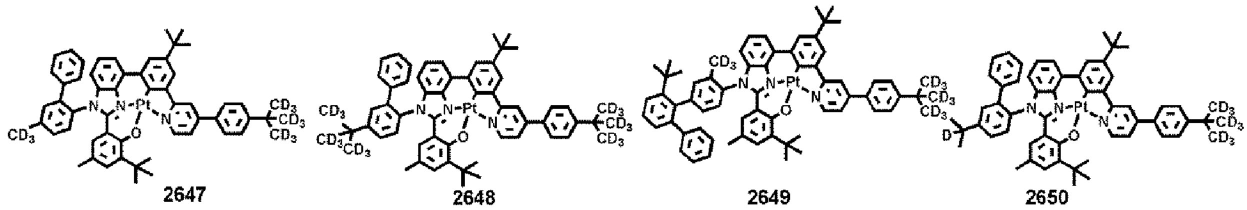

2647 2648 2649 2650

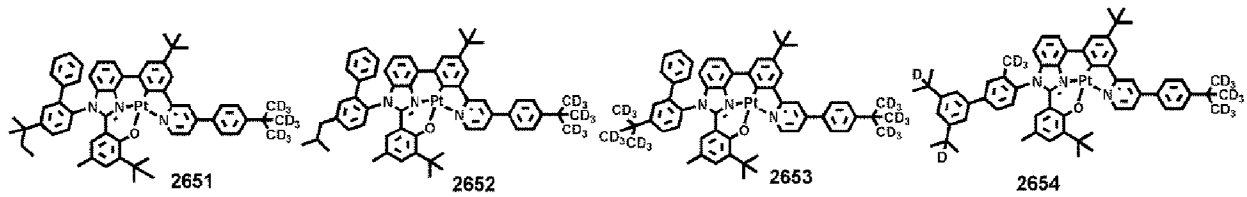

2651 2652 2653 2654

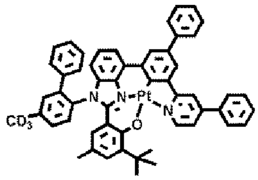

2655

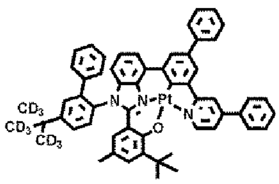

2656

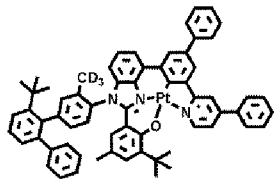

2657

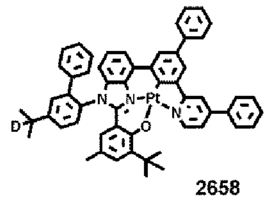

2658

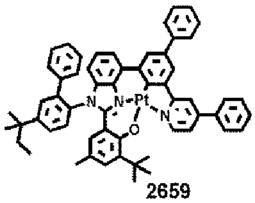

2659

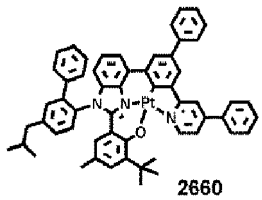

2660

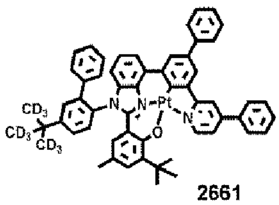

2661

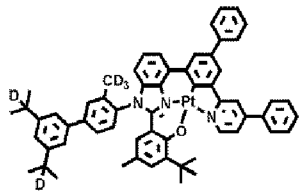

2662

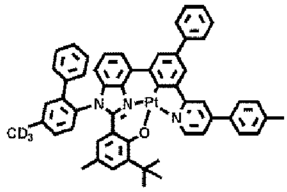

2663

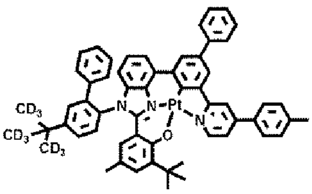

2664

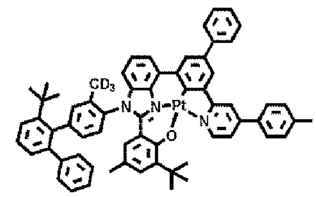

2665

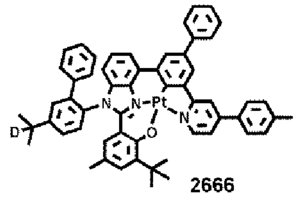

2666

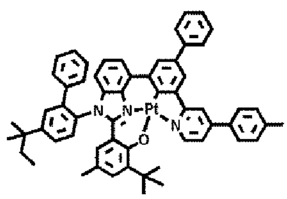

2667

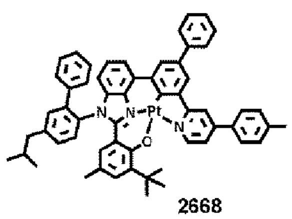

2668

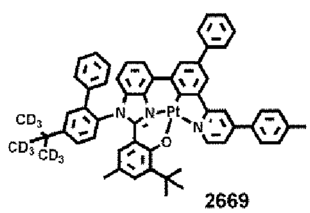

2669

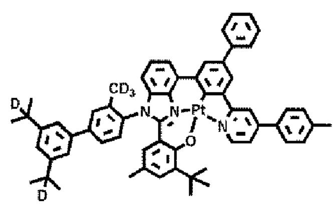

2670

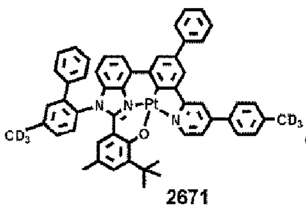
2671

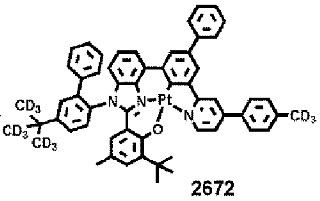
2672

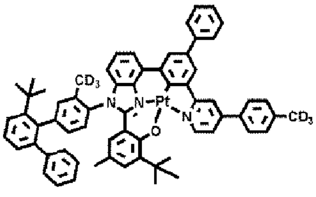
2673

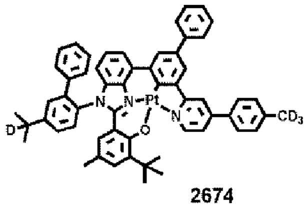
2674

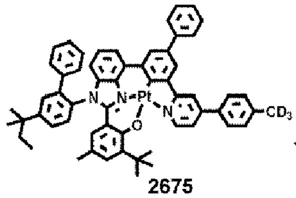
2675

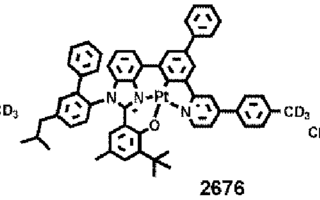
2676

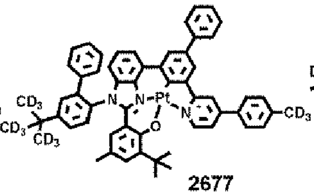
2677

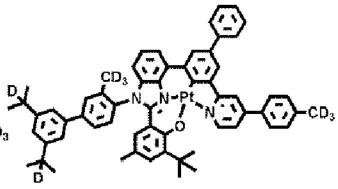
2678

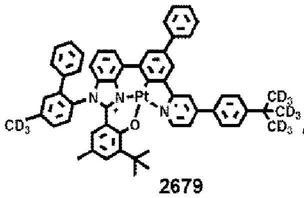
2679

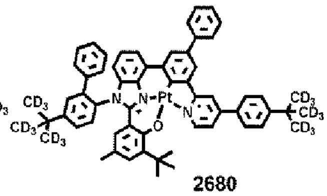
2680

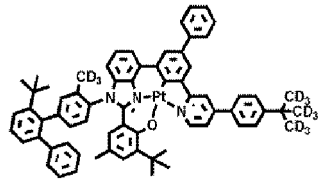
2681

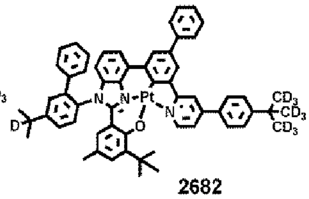
2682

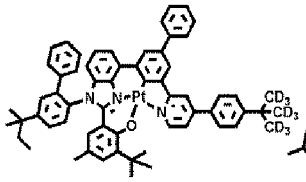
2683

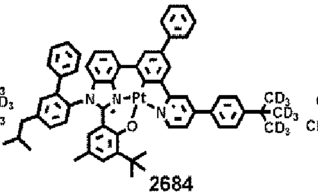
2684

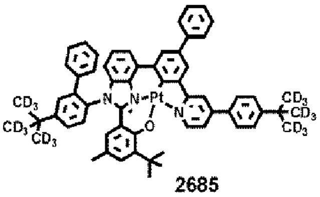
2685

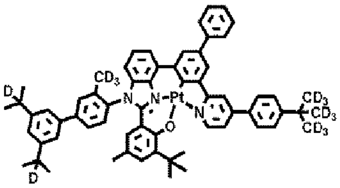
2686

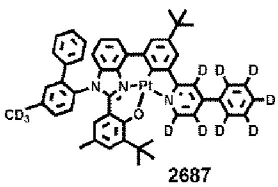
2687

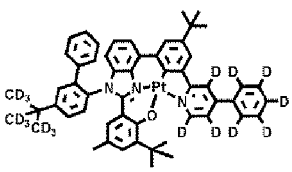
2688

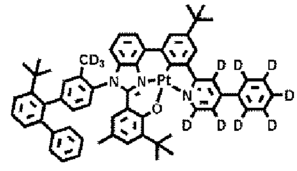
2689

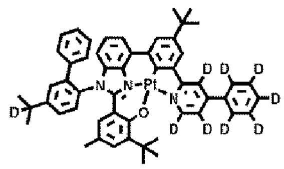
2690

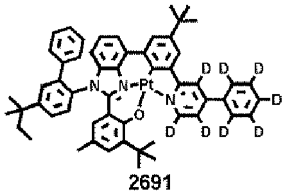
2691

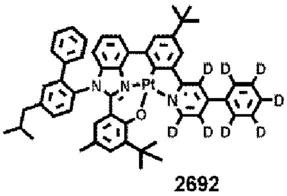
2692

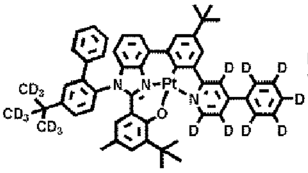
2693

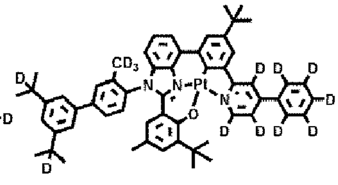
2694

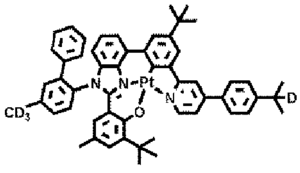
2695

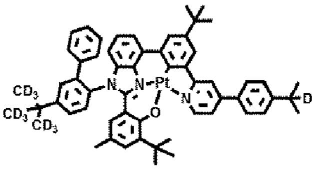
2696

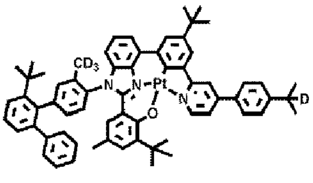
2697

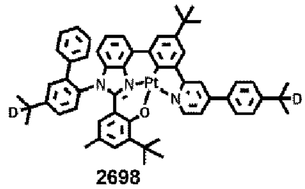
2698

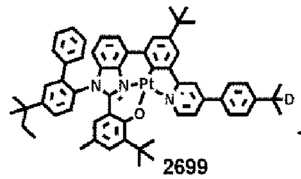
2699

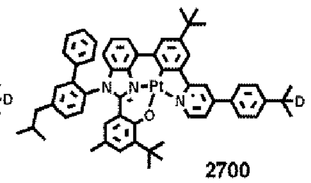
2700

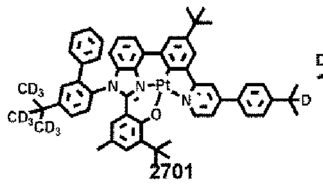
2701

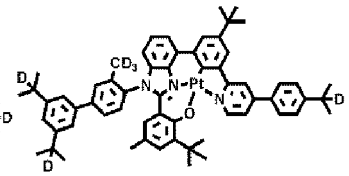
2702

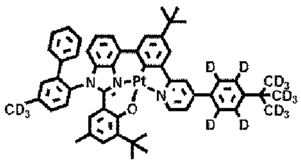
2703

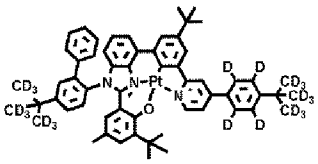
2704

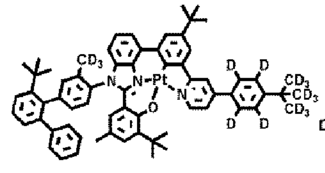
2705

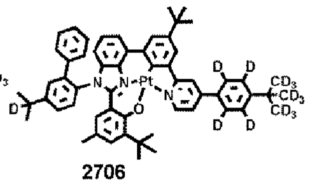
2706

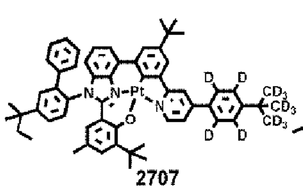
2707

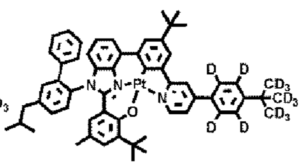
2708

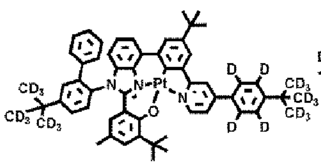
2709

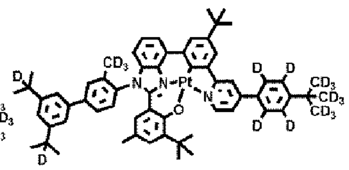
2710

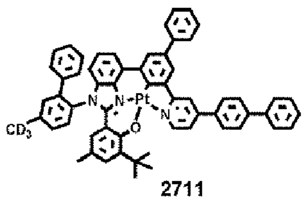
2711

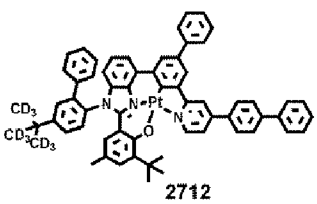
2712

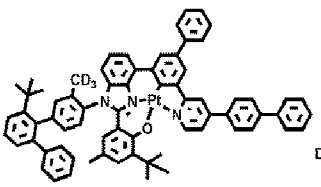
2713

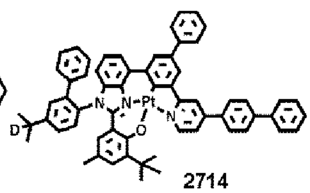
2714

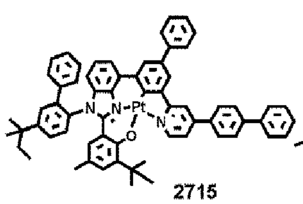
2715

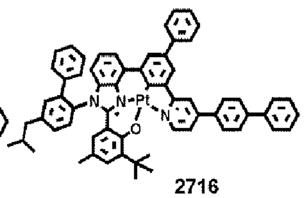
2716

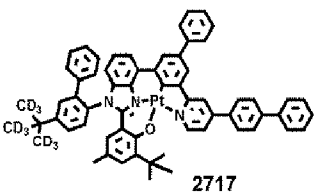
2717

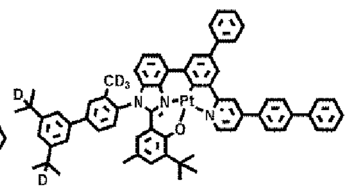
2718

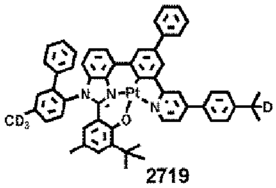
2719

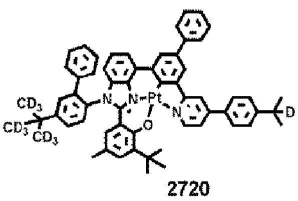
2720

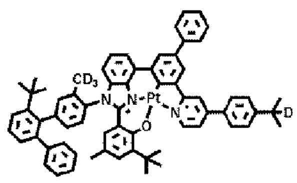
2721

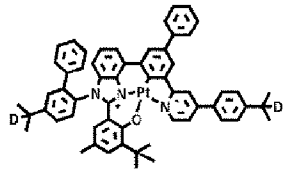
2722

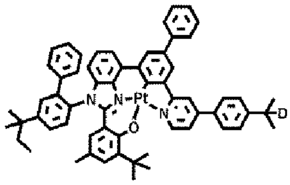
2723

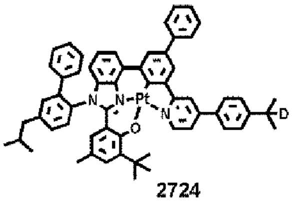
2724

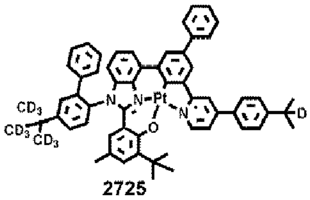
2725

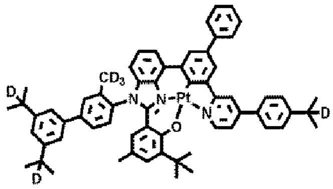
2726

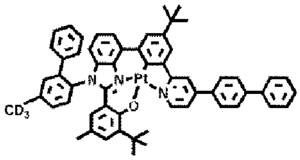
2727

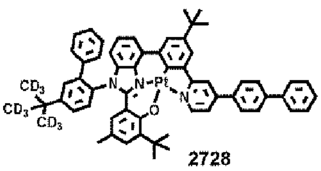
2728

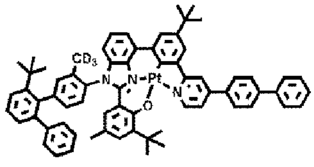
2729

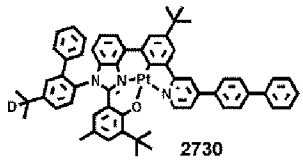
2730

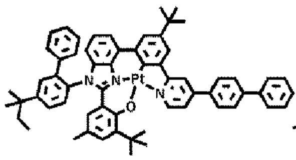
2731

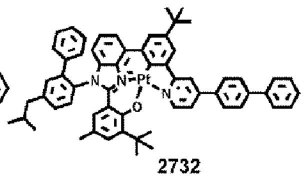
2732

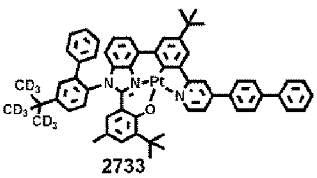
2733

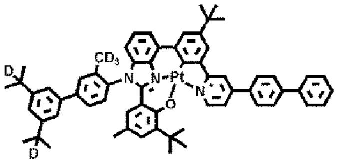
2734

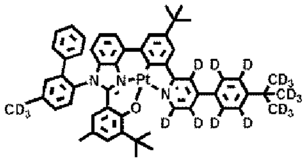
2735

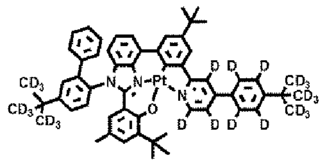
2736

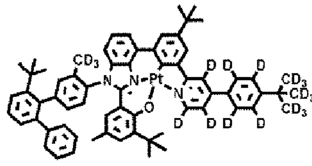
2737

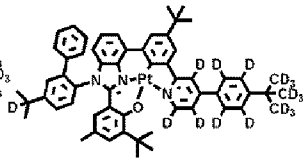
2738

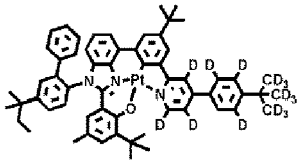
2739

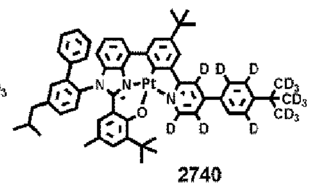
2740

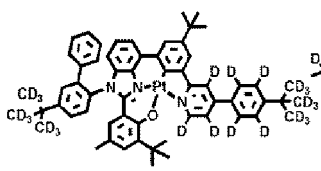
2741

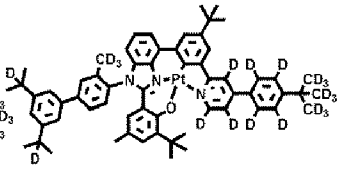
2742

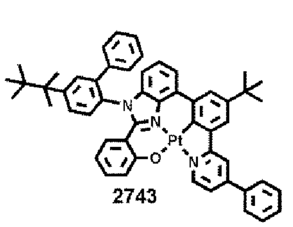
2743

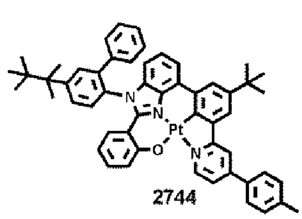
2744

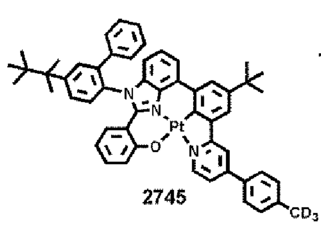
2745

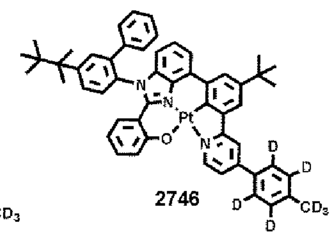
2746

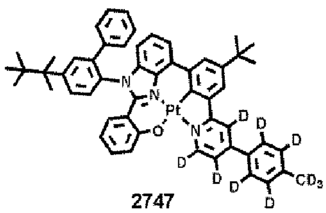
2747

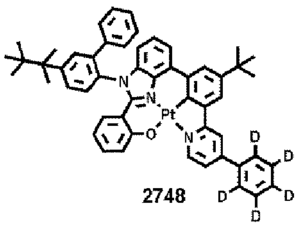
2748

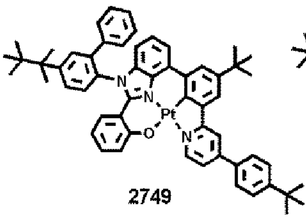
2749

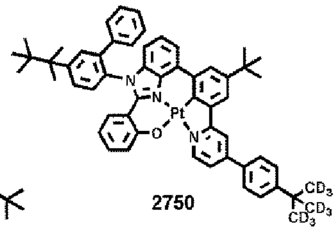
2750

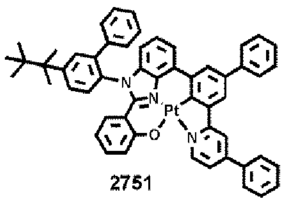
2751

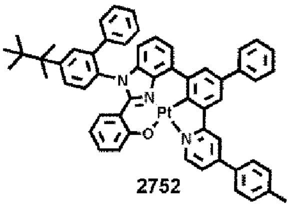
2752

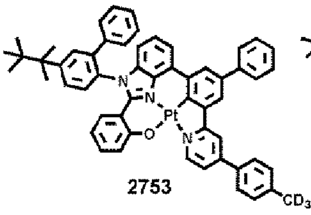
2753

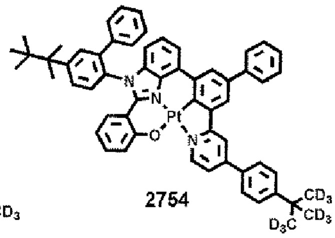
2754

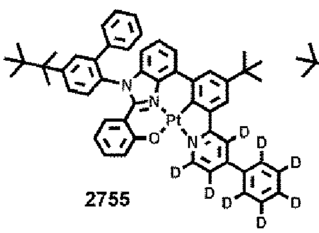
2755

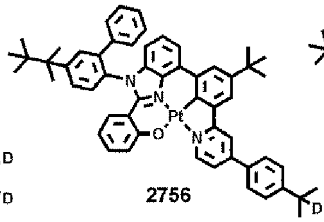
2756

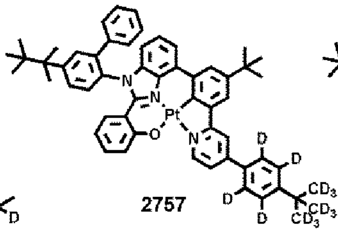
2757

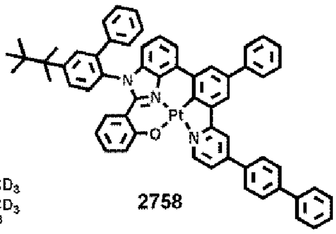
2758

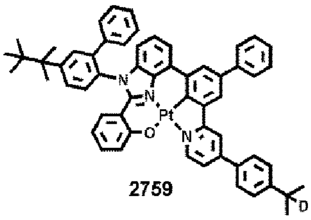
2759

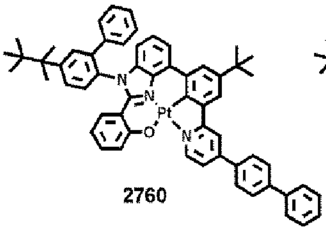
2760

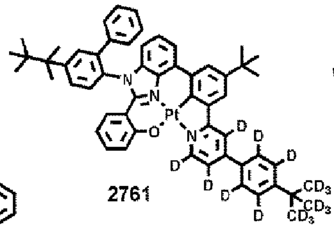
2761

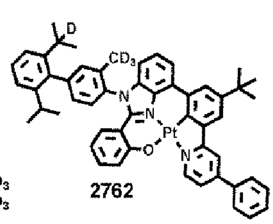
2762

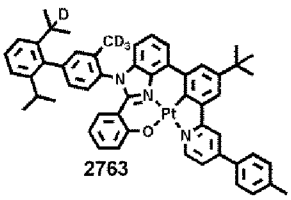
2763

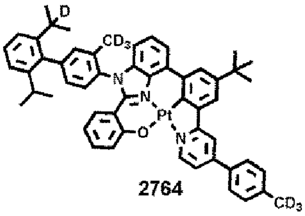
2764

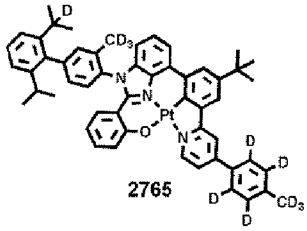
2765

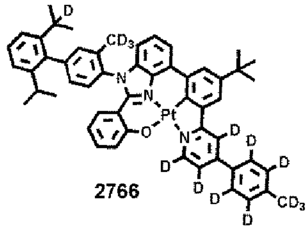
2766

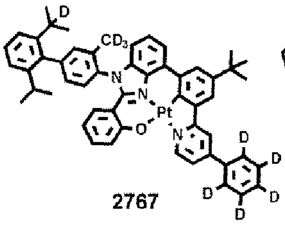
2767

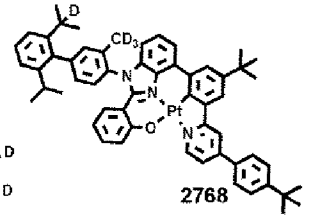
2768

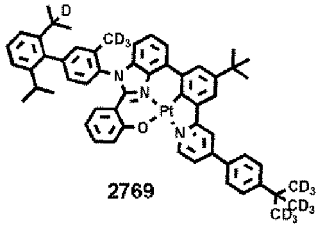
2769

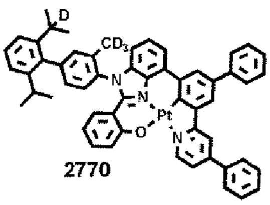
2770

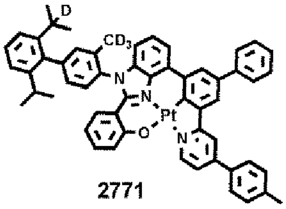
2771

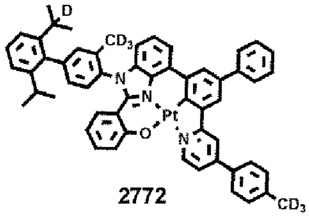
2772

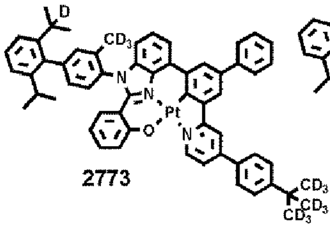
2773

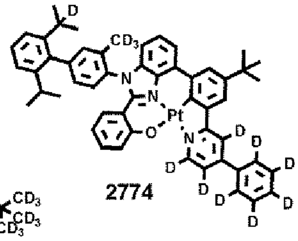
2774

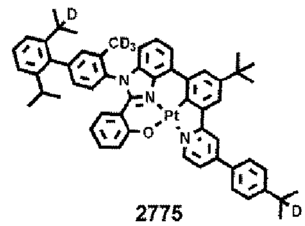
2775
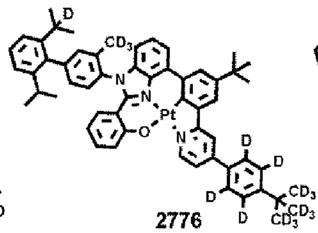
2776
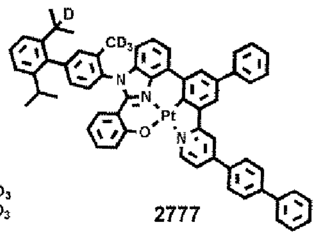
2777
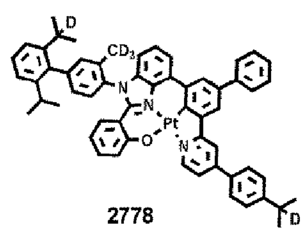
2778

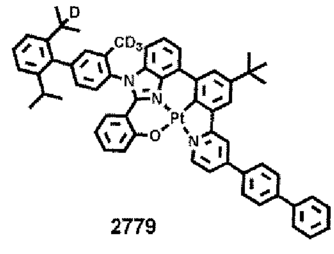
2779
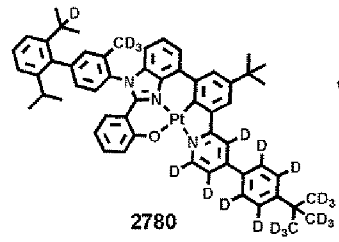
2780
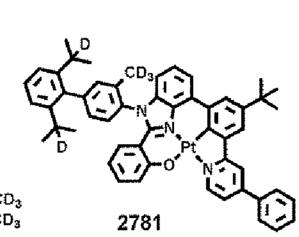
2781
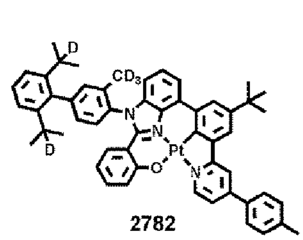
2782

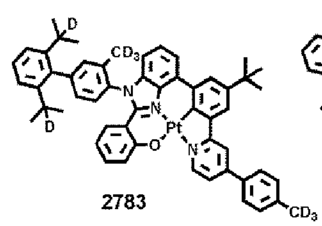
2783
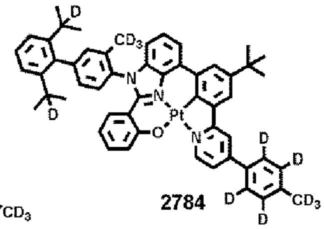
2784
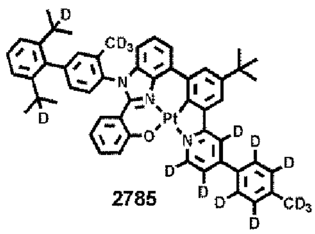
2785
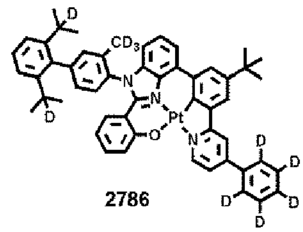
2786

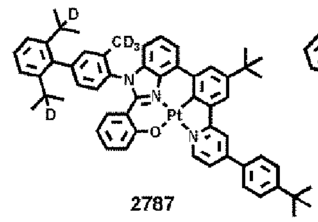
2787
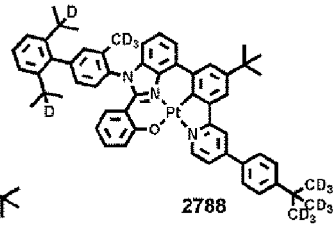
2788
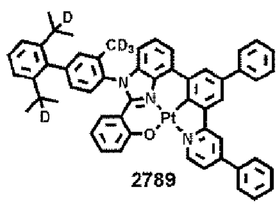
2789
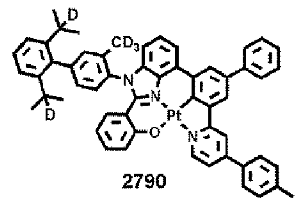
2790

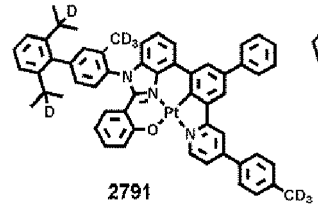
2791
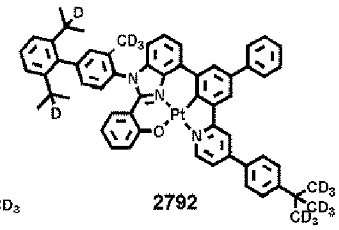
2792
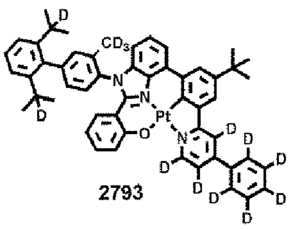
2793
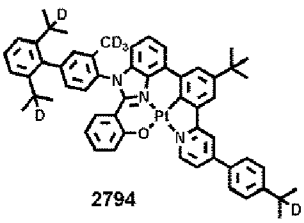
2794

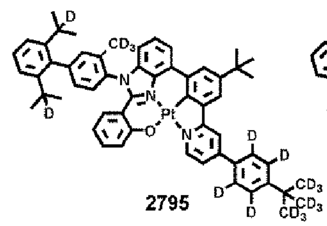
2795
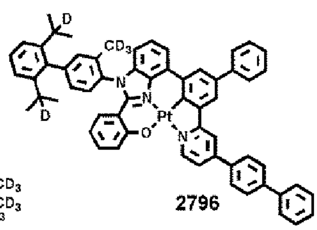
2796
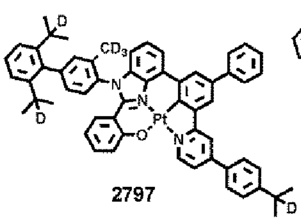
2797
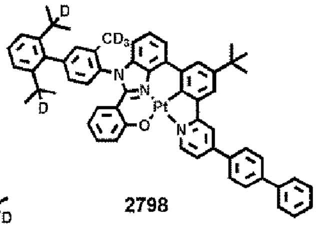
2798

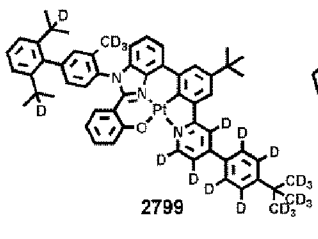
2799

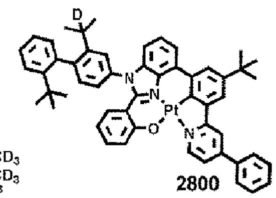
2800

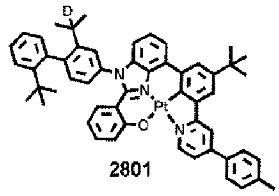
2801

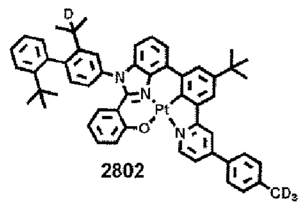
2802

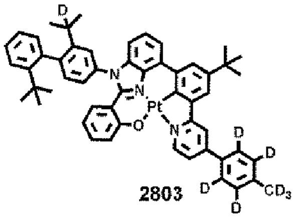
2803

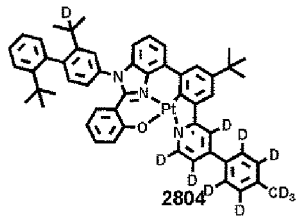
2804

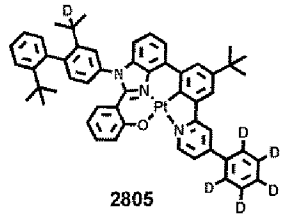
2805

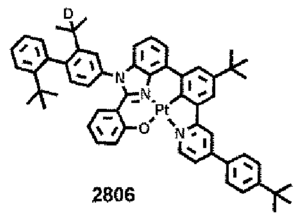
2806

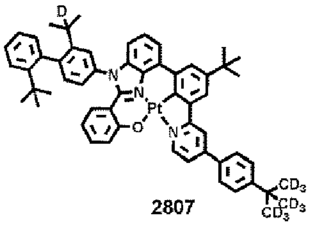
2807

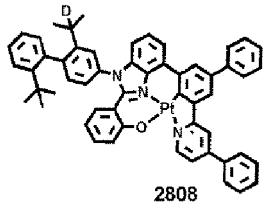
2808

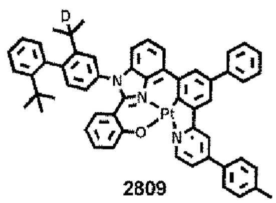
2809

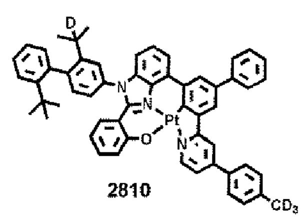
2810

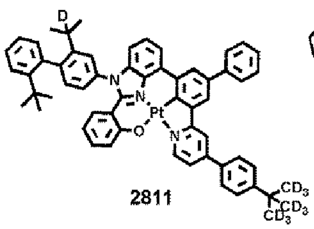
2811

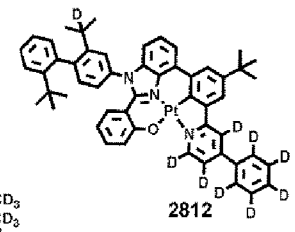
2812

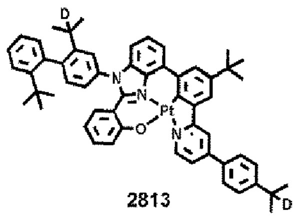
2813

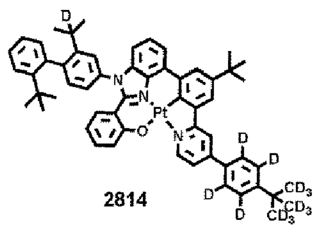
2814

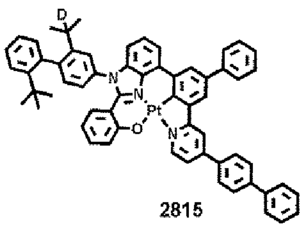
2815

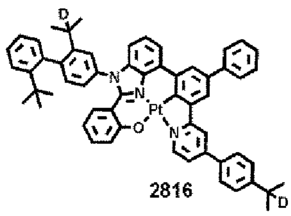
2816

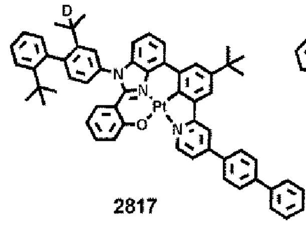
2817

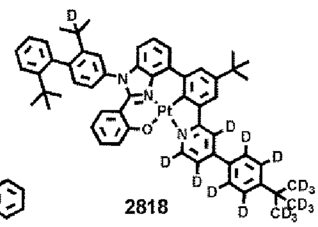
2818

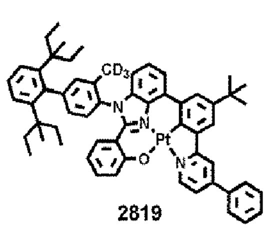
2819

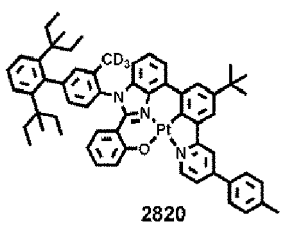
2820

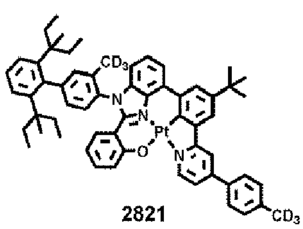
2821

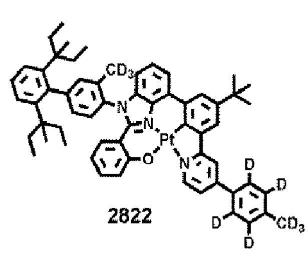
2822

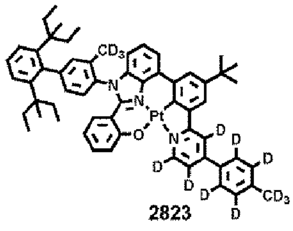
2823

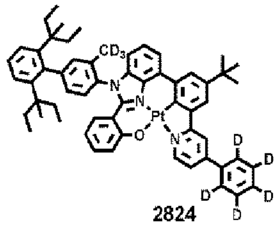
2824

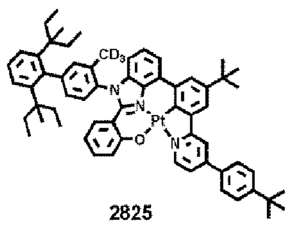
2825

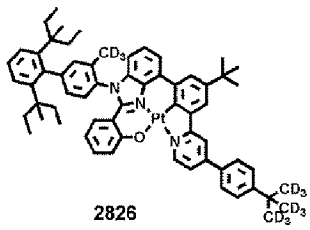
2826

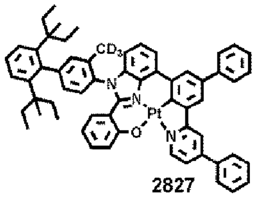
2827

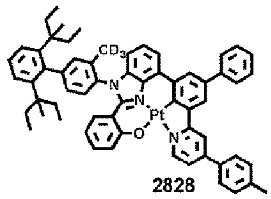
2828

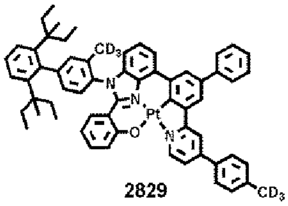
2829

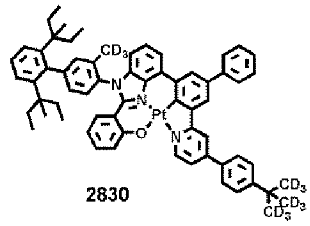
2830

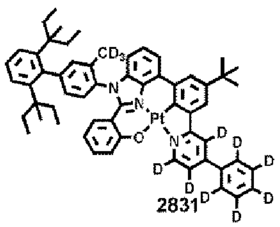
2831

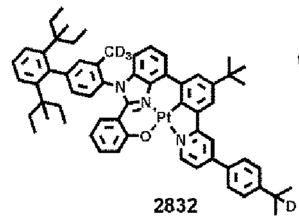
2832

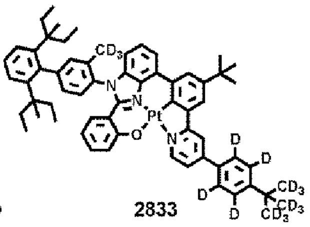
2833

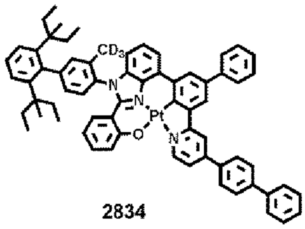
2834

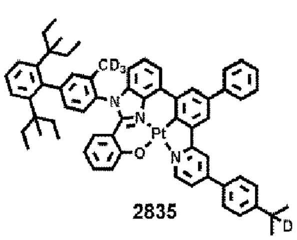
2835

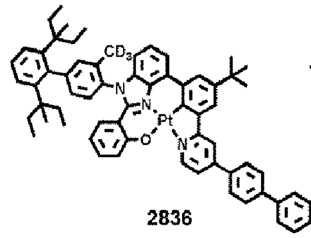
2836

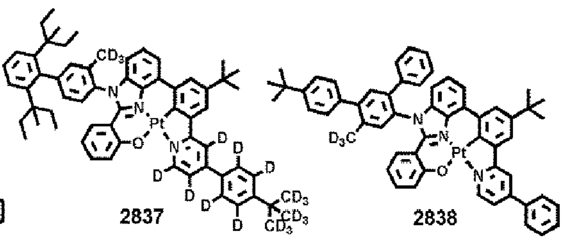
2837

2838

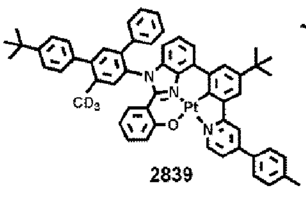
2839

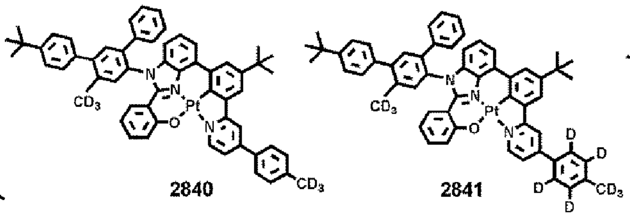
2840

2841

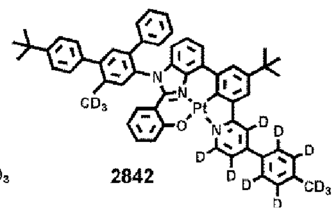
2842

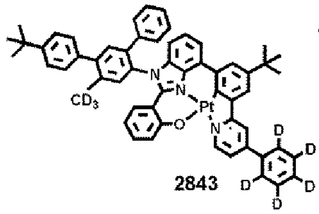
2843

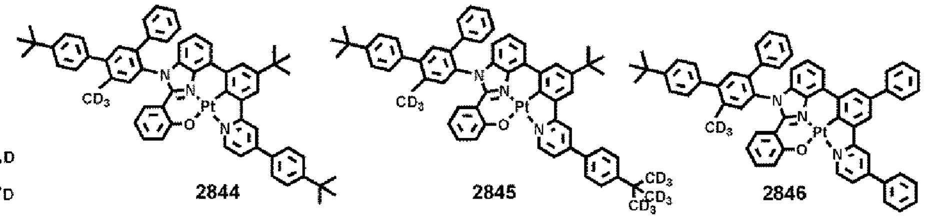
2844

2845

2846

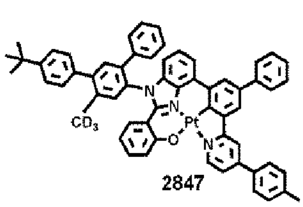
2847

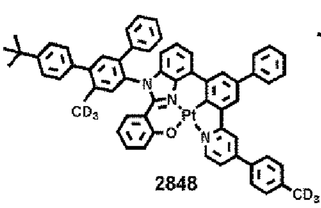
2848

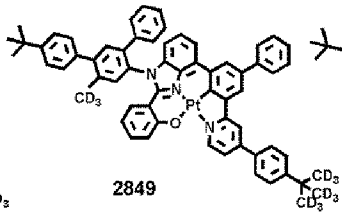
2849

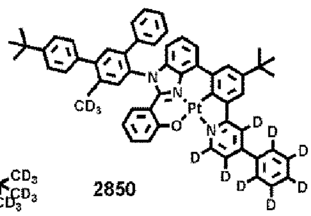
2850

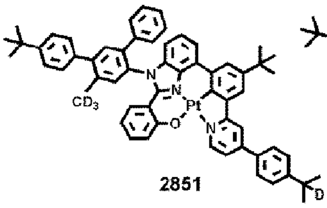
2851

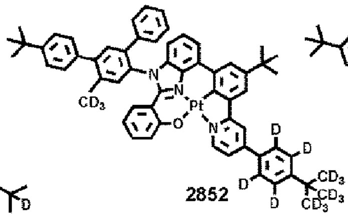
2852

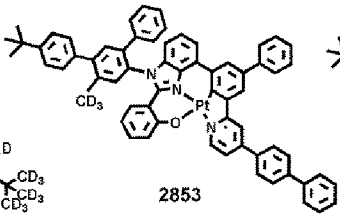
2853

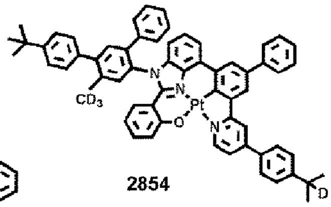
2854

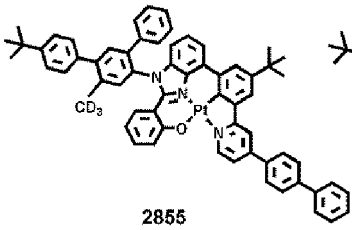
2855

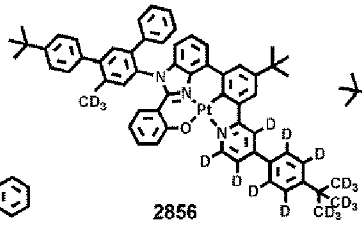
2856

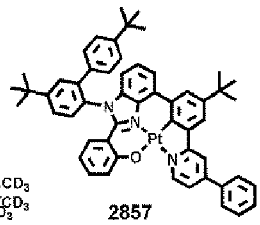
2857

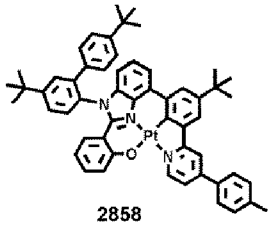
2858

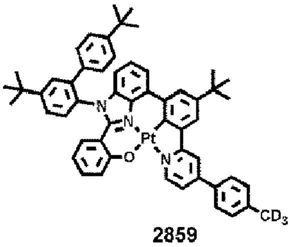
2859

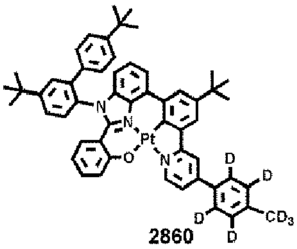
2860

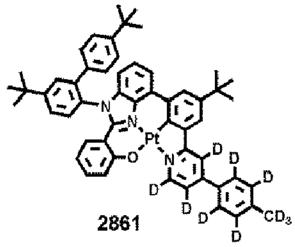
2861

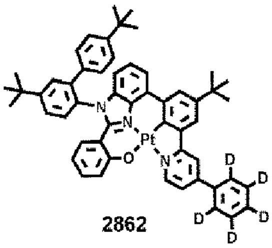
2862

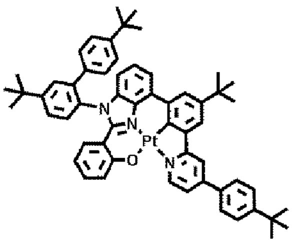
2863

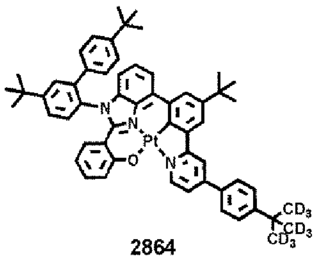
2864

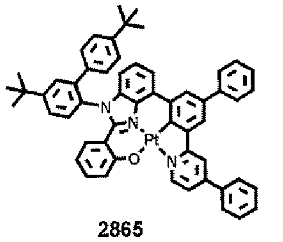
2865

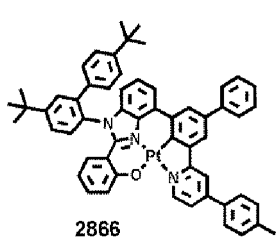
2866

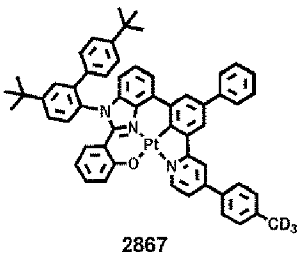
2867

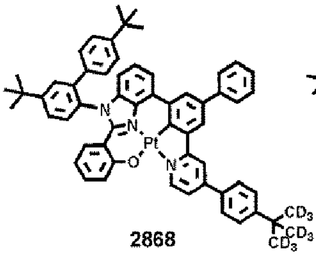
2868

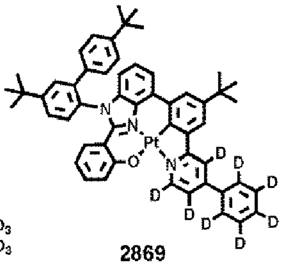
2869

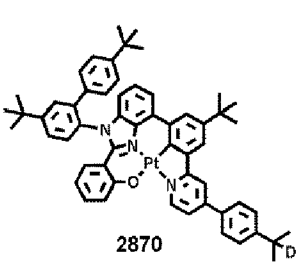
2870

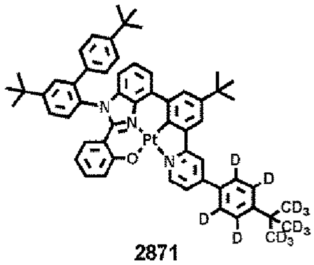
2871

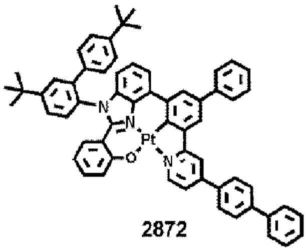
2872

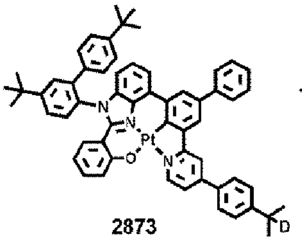
2873

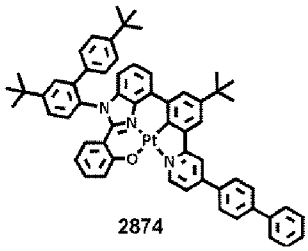
2874

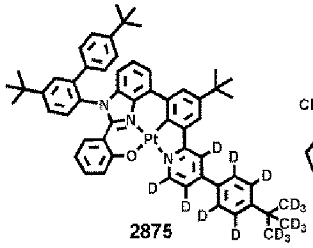
2875

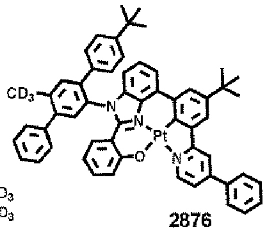
2876

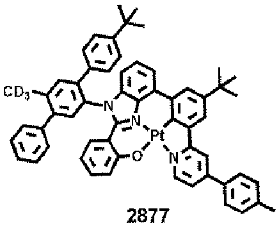
2877

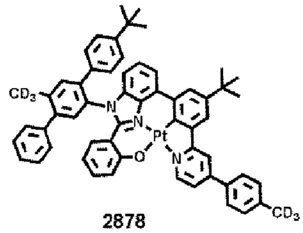
2878

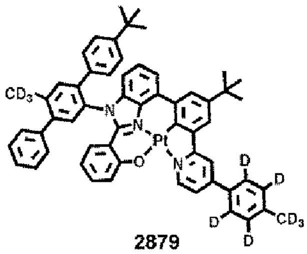
2879

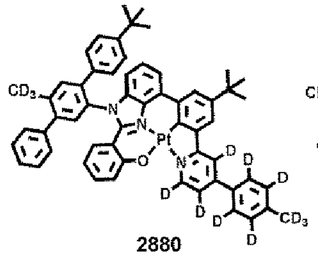
2880

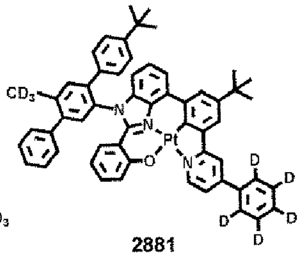
2881

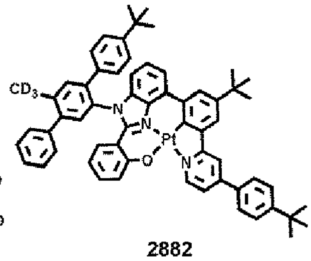
2882

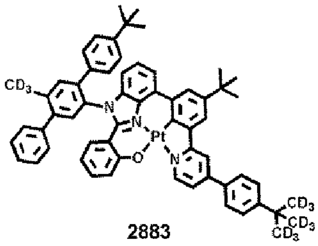
2883

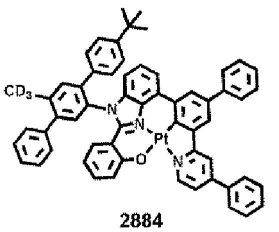
2884

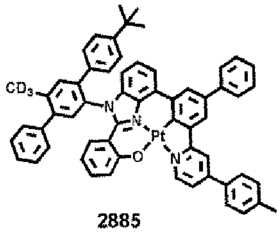
2885

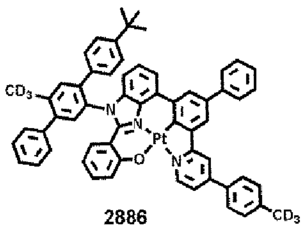
2886

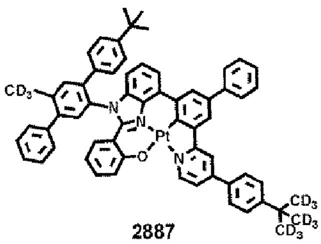
2887

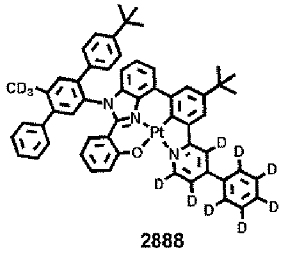
2888

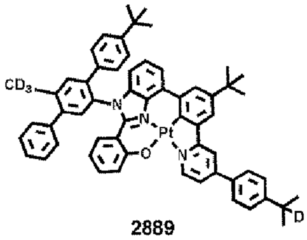
2889

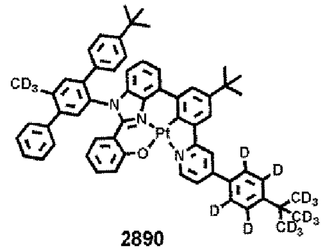
2890

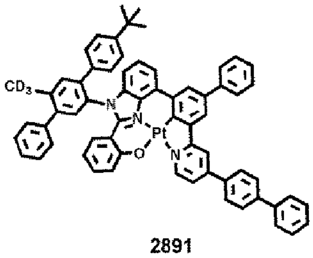
2891

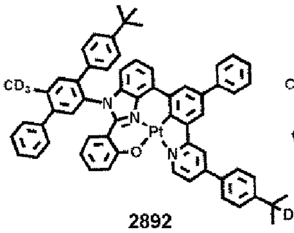
2892

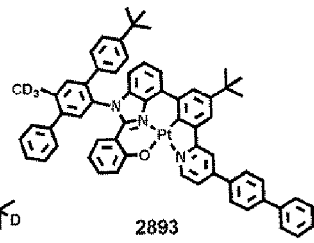
2893

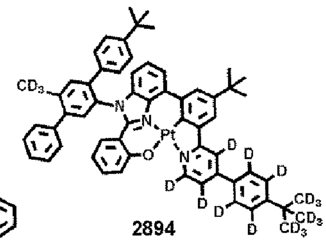
2894

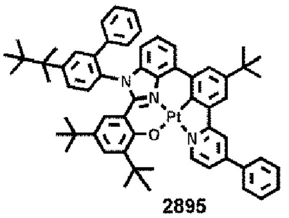
2895

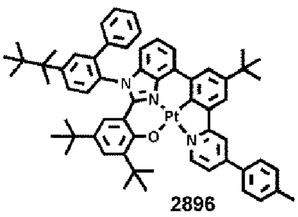
2896

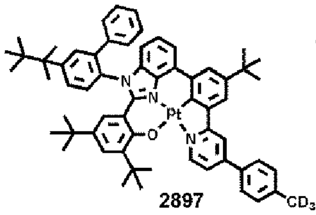
2897

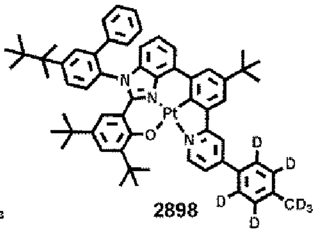
2898

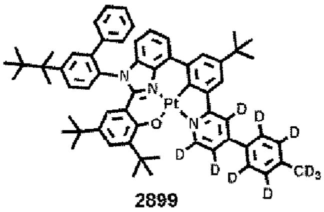
2899

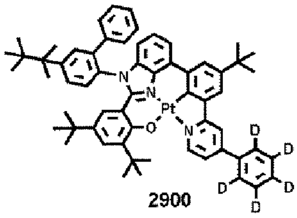
2900

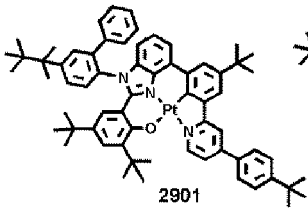
2901

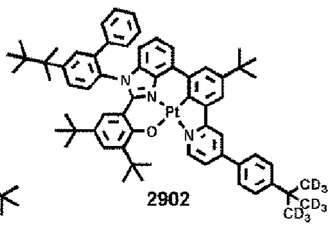
2902

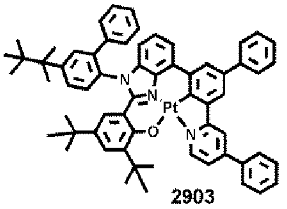
2903

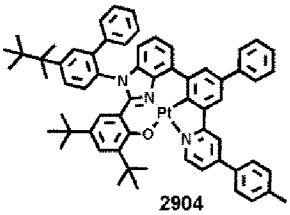
2904

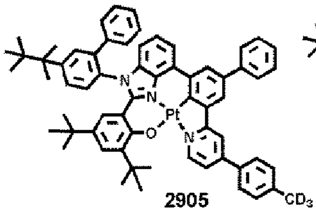
2905

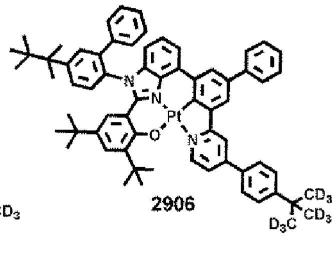
2906

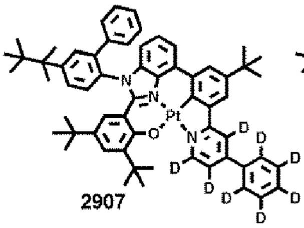
2907

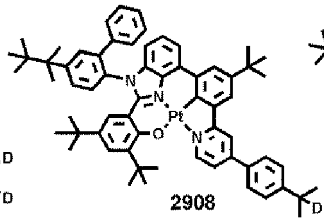
2908

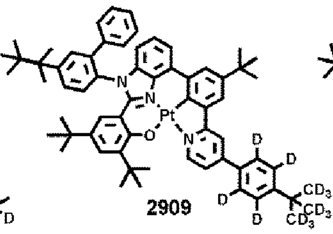
2909

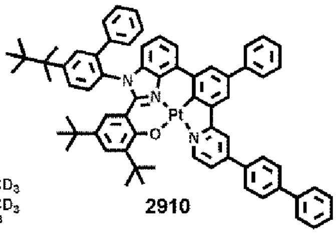
2910

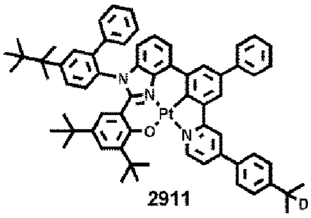
2911

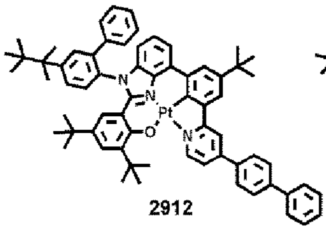
2912

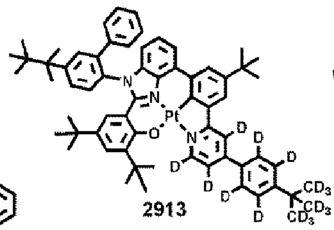
2913

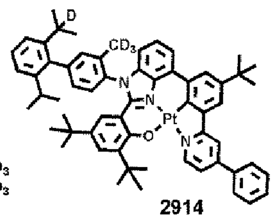
2914

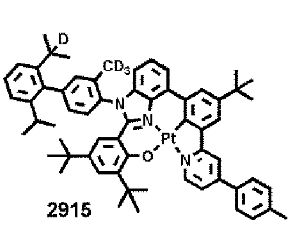
2915

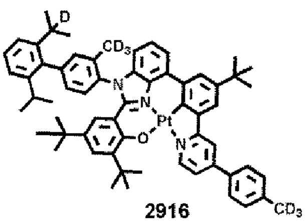
2916

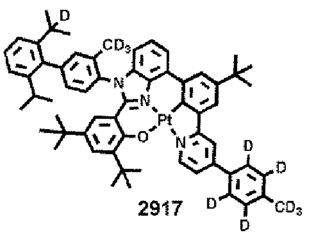
2917

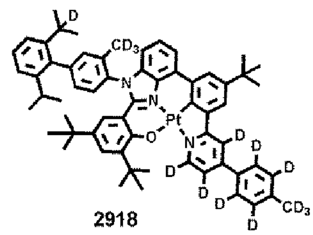
2918

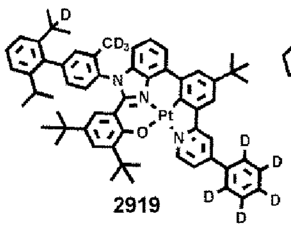
2919

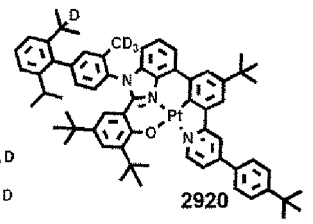
2920

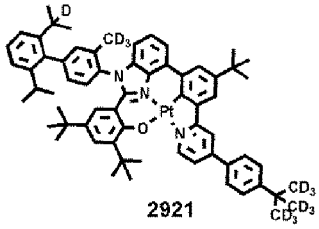
2921

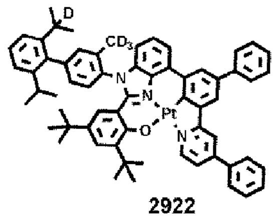
2922

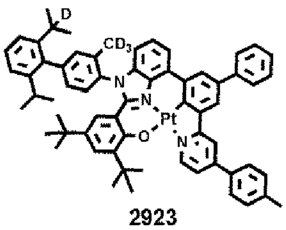
2923

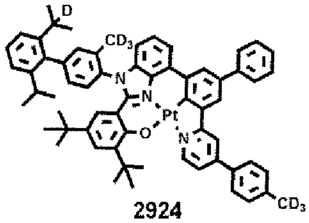
2924

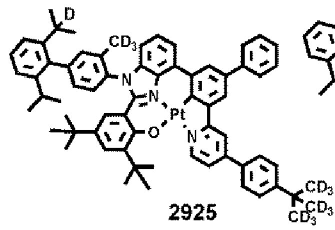
2925

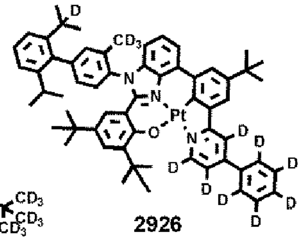
2926

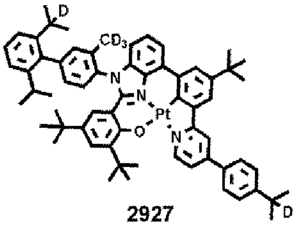
2927

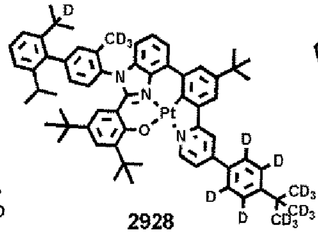
2928

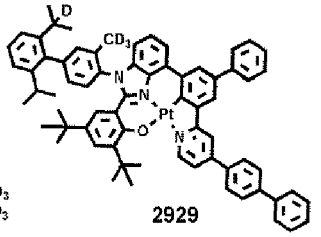
2929

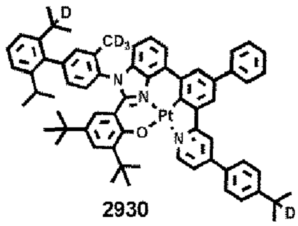
2930

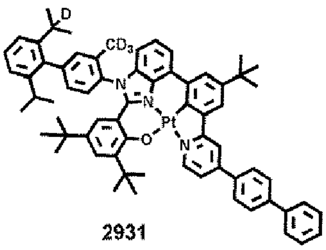
2931

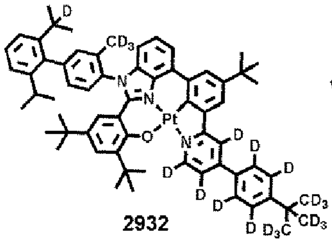
2932

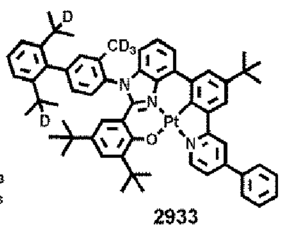
2933

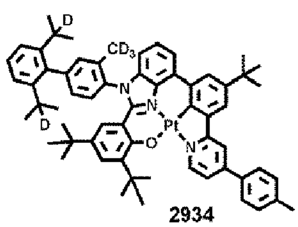
2934

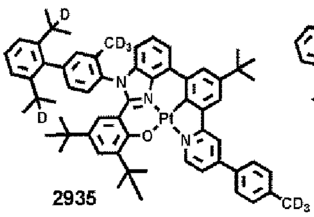
2935

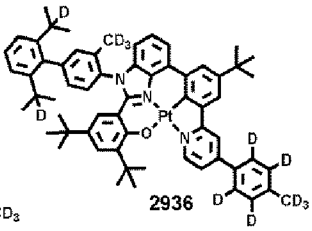
2936

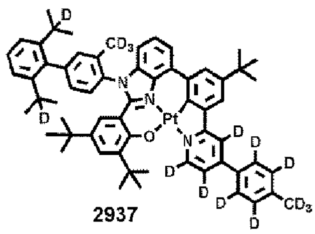
2937

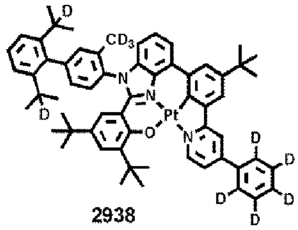
2938

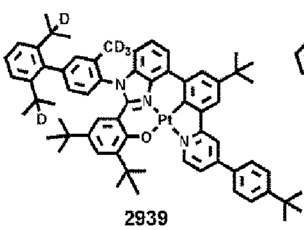
2939

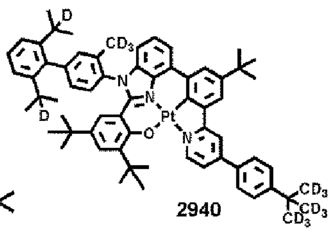
2940

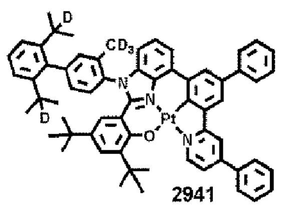
2941

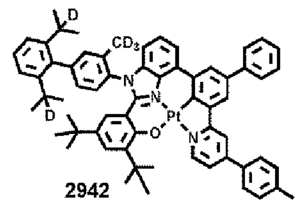
2942

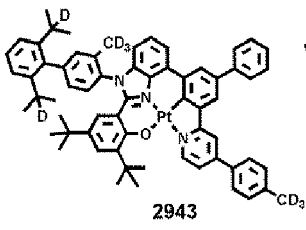
2943
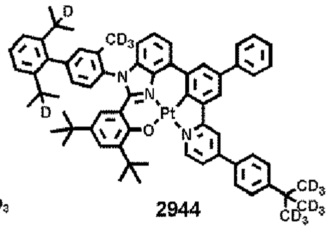
2944
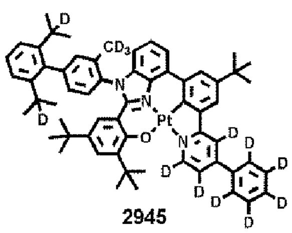
2945
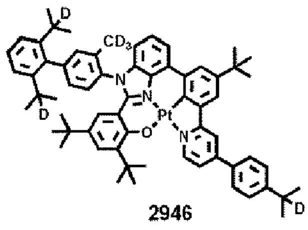
2946

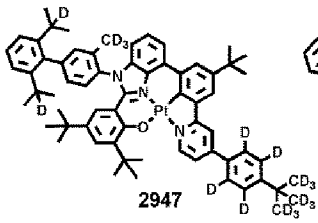
2947
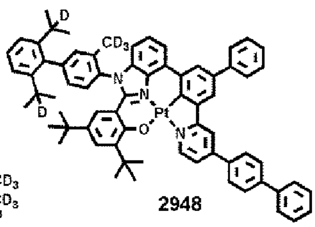
2948
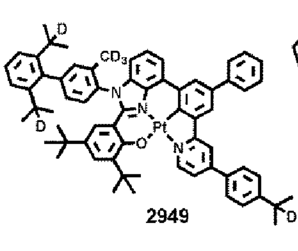
2949
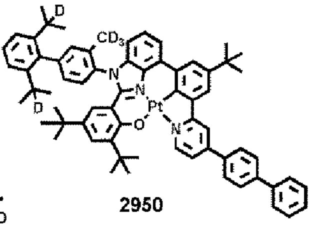
2950

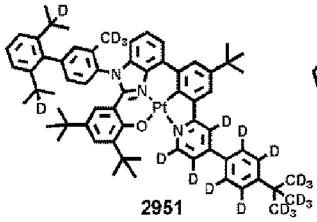
2951
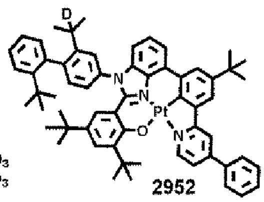
2952
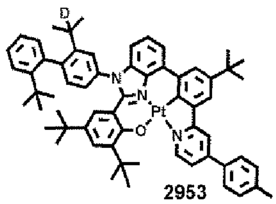
2953
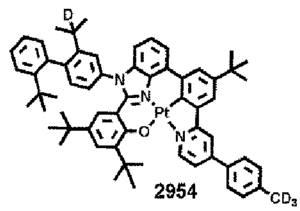
2954

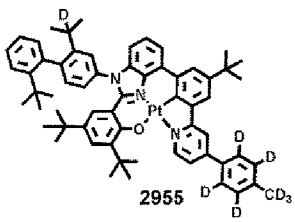
2955
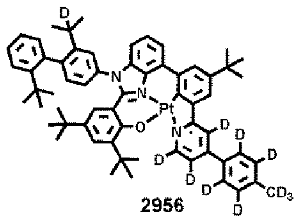
2956
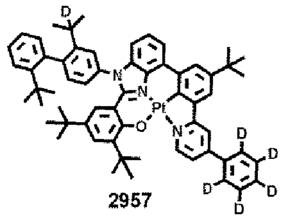
2957
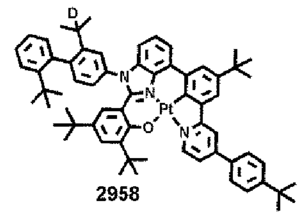
2958

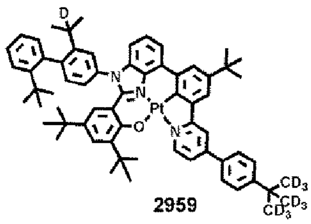
2959
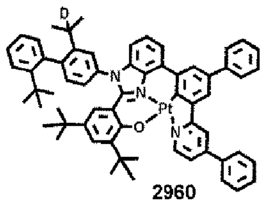
2960
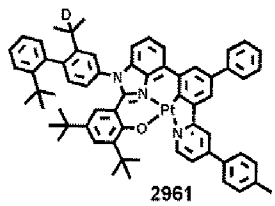
2961
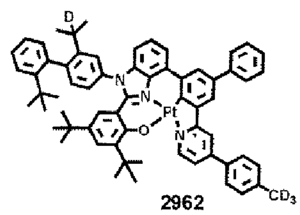
2962

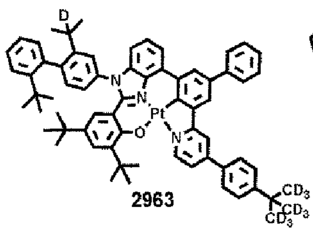
2963
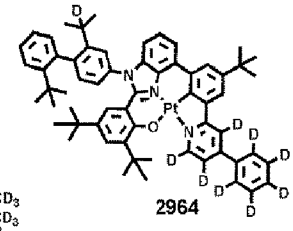
2964
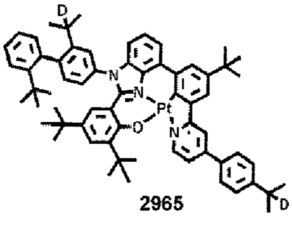
2965
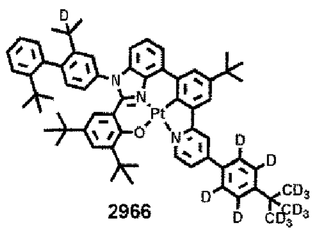
2966

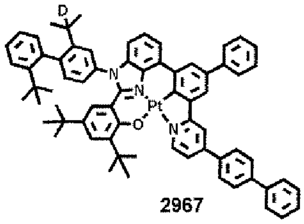
2967

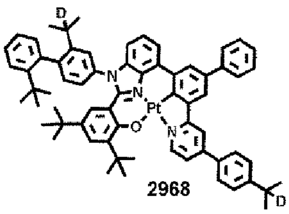
2968

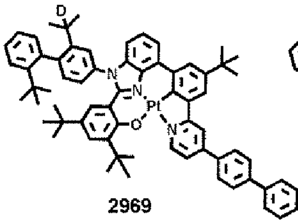
2969

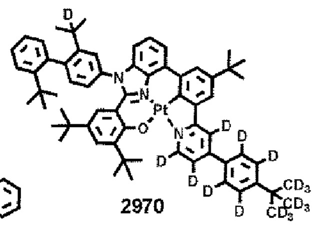
2970

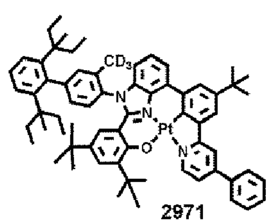
2971

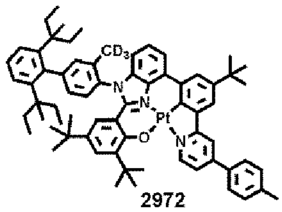
2972

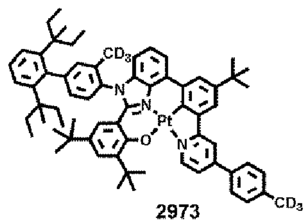
2973

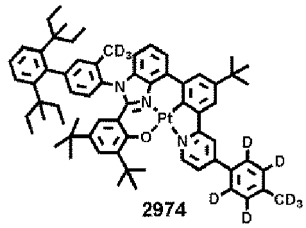
2974

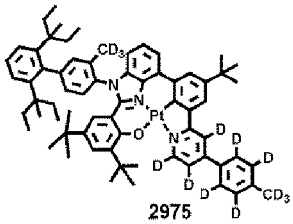
2975

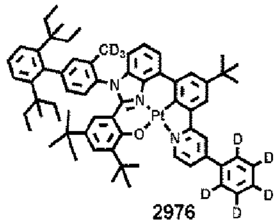
2976

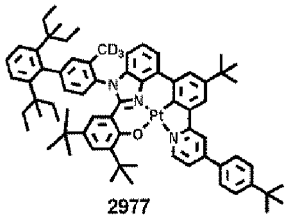
2977

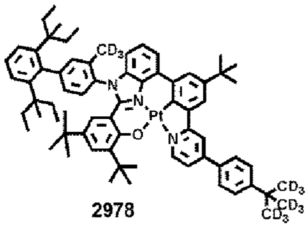
2978

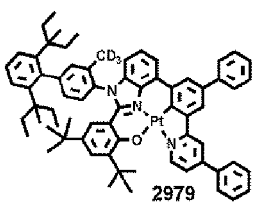
2979

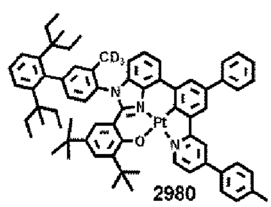
2980

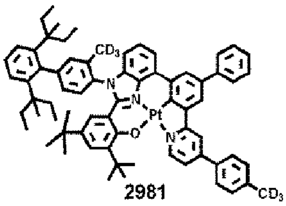
2981

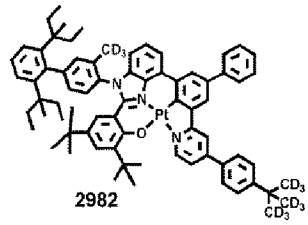
2982

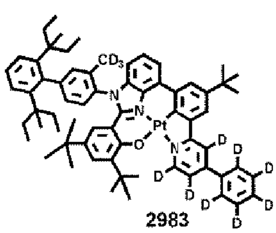
2983

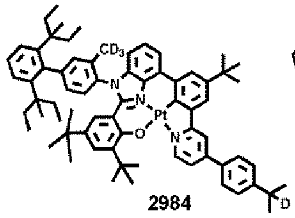
2984

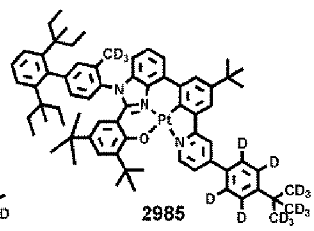
2985

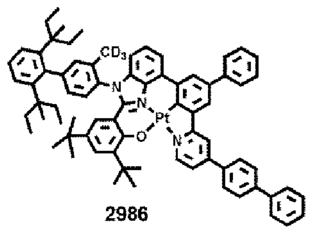
2986

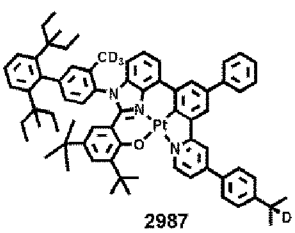
2987

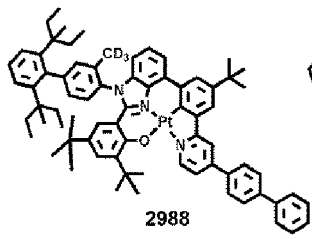
2988

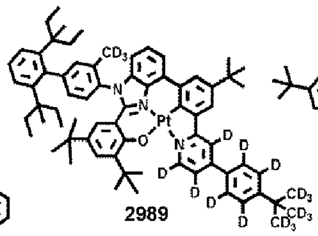
2989

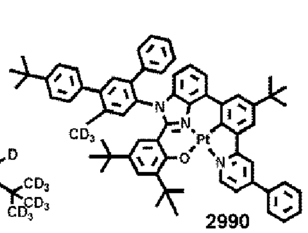
2990

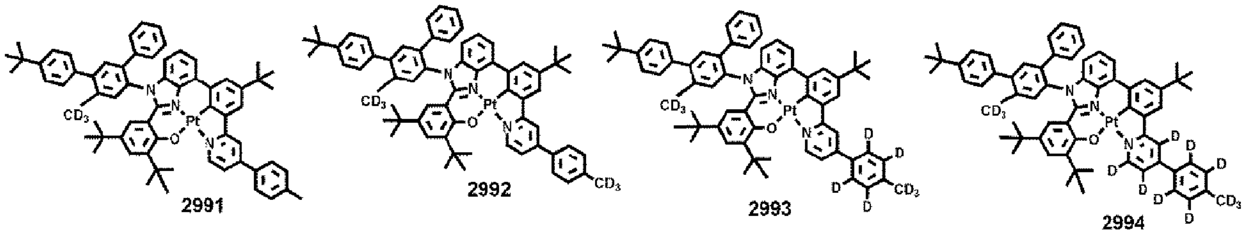

2991 2992 2993 2994

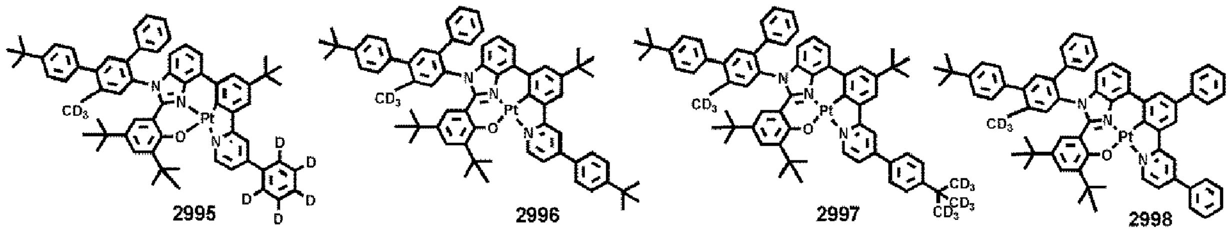

2995 2996 2997 2998

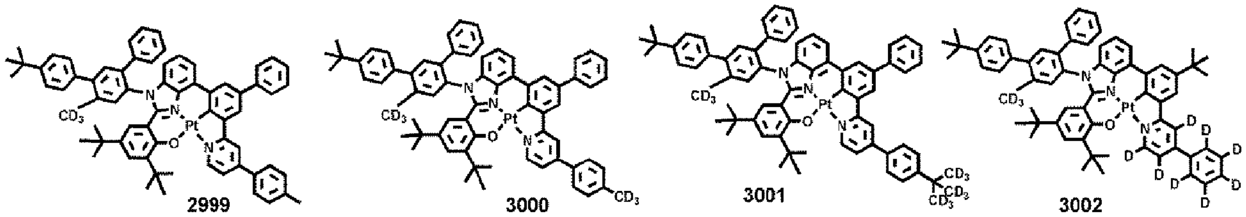

2999 3000 3001 3002

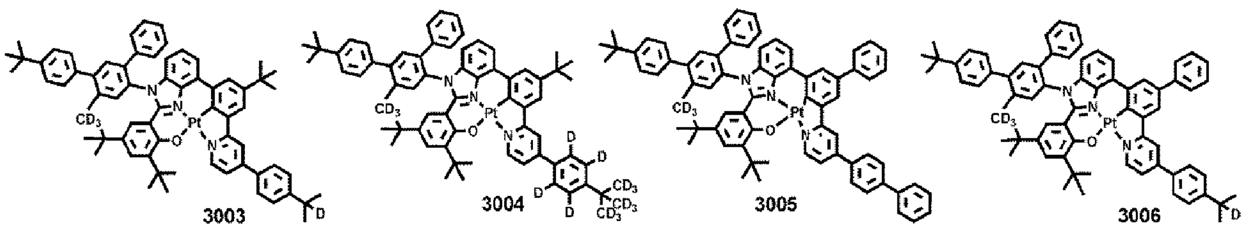

3003 3004 3005 3006

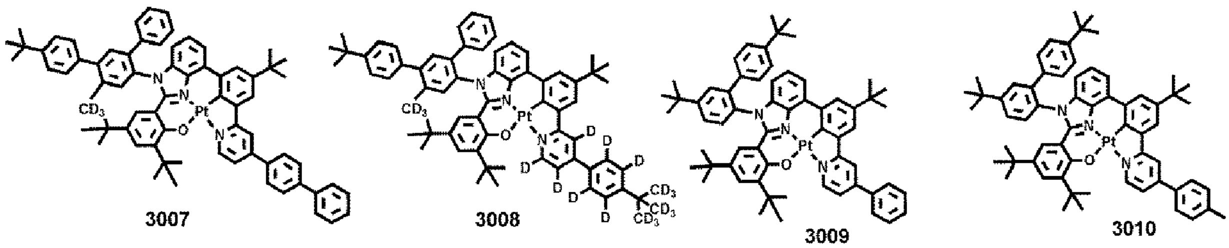

3007 3008 3009 3010

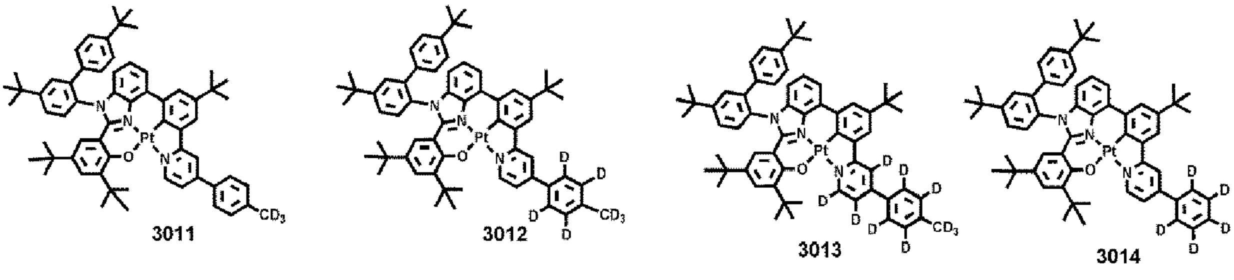

3011 3012 3013 3014

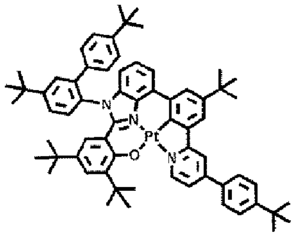
3015

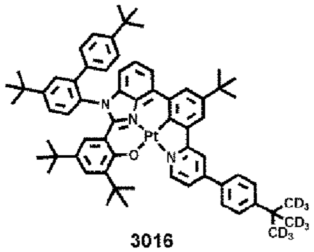
3016

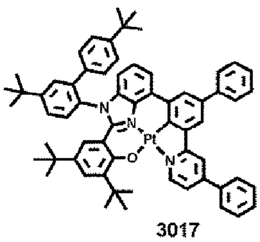
3017

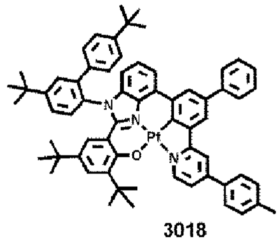
3018

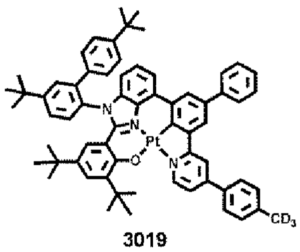
3019

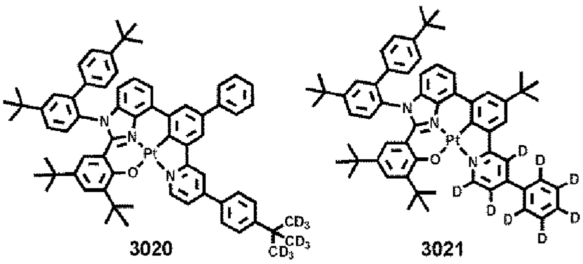
3020

3021

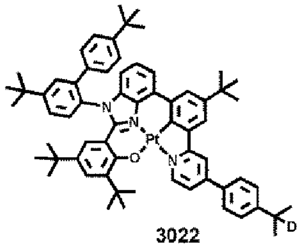
3022

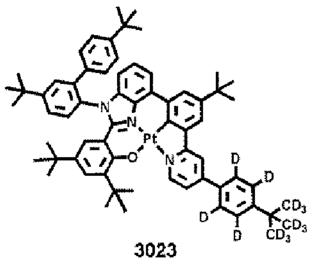
3023

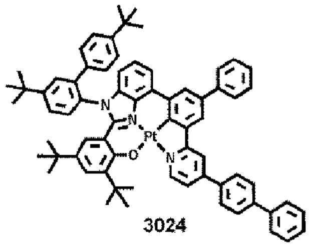
3024

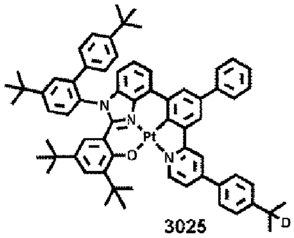
3025

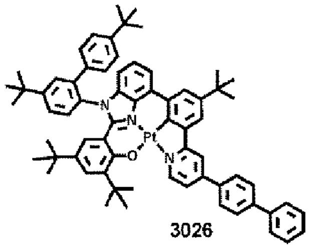
3026

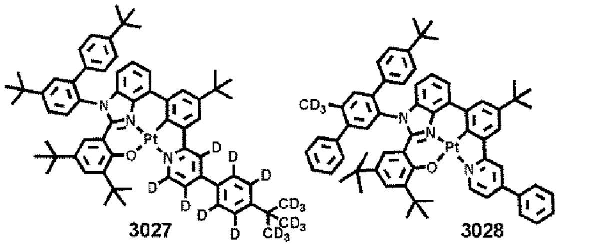
3027

3028

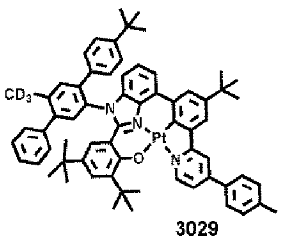
3029

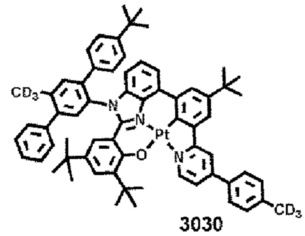
3030

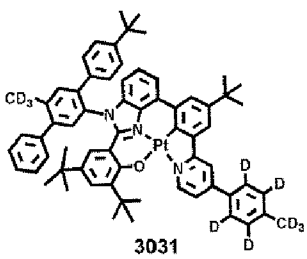
3031

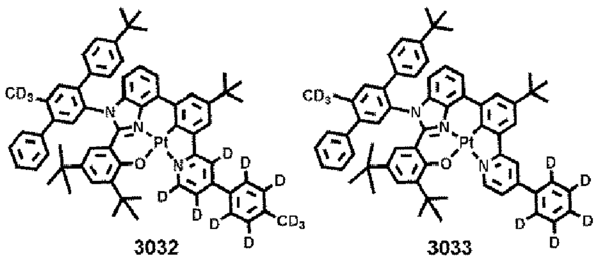
3032

3033

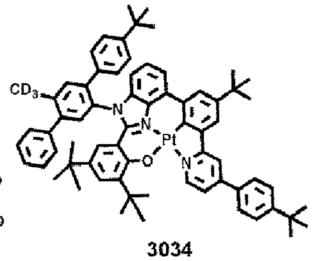
3034

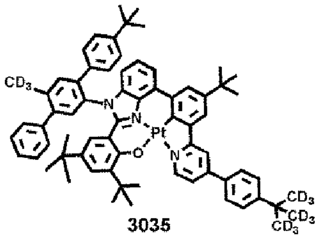
3035

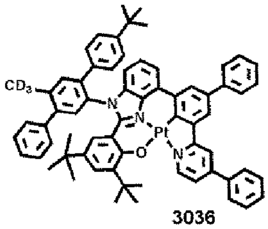
3036

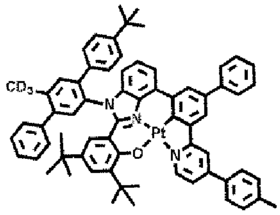
3037

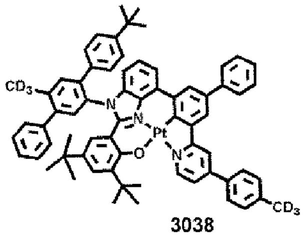
3038

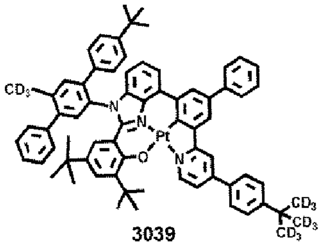
3039

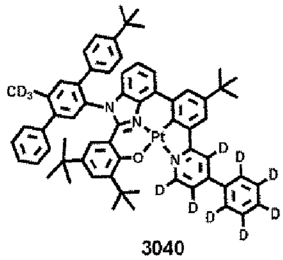
3040

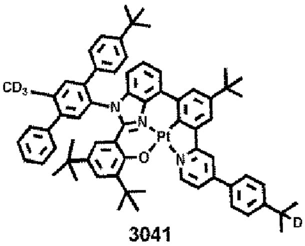
3041

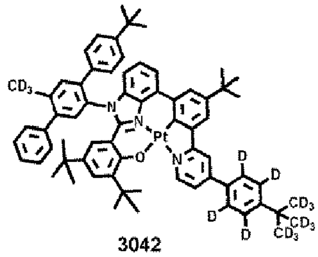
3042

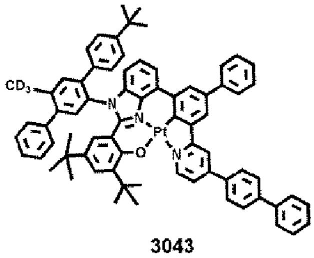
3043

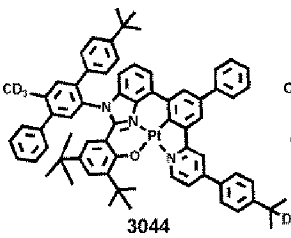
3044

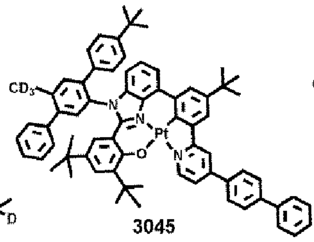
3045

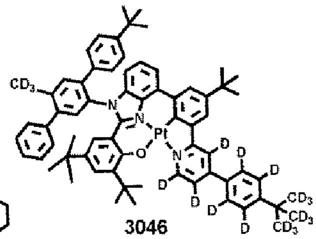
3046

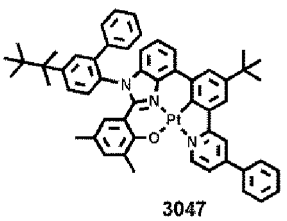
3047

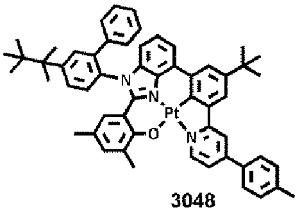
3048

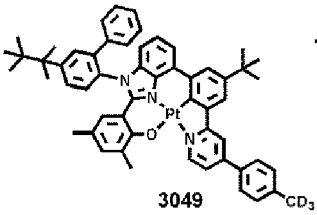
3049

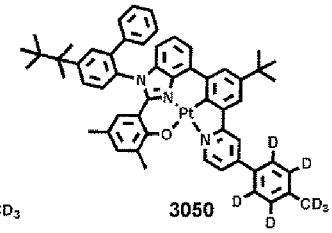
3050

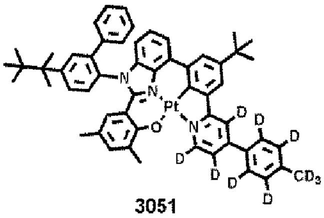
3051

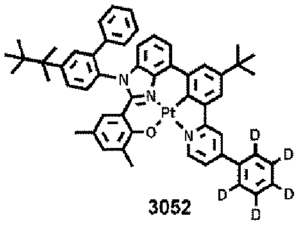
3052

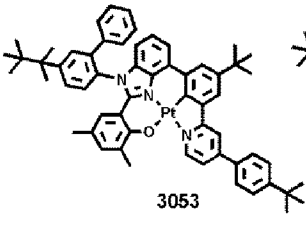
3053

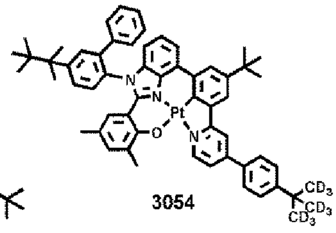
3054

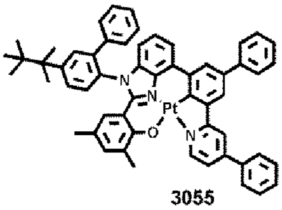
3055

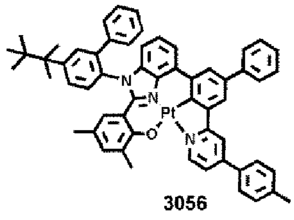
3056

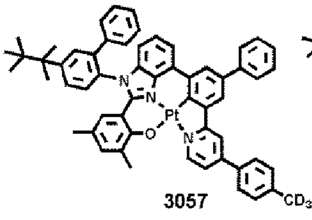
3057

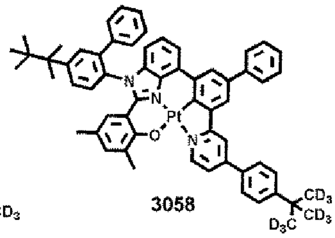
3058

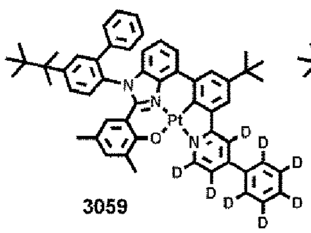
3059

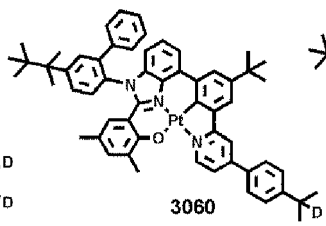
3060

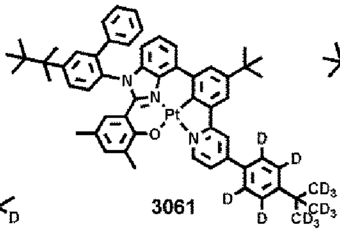
3061

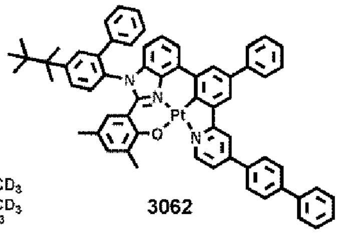
3062

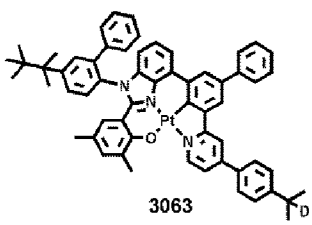
3063

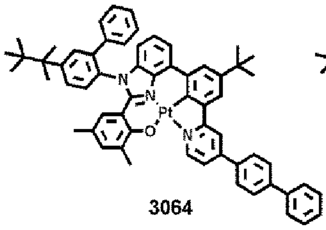
3064

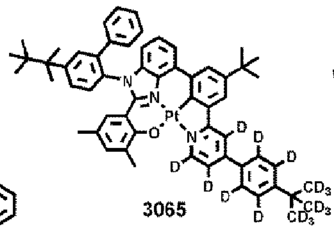
3065

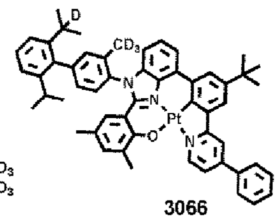
3066

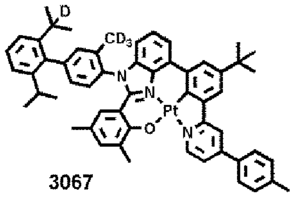
3067

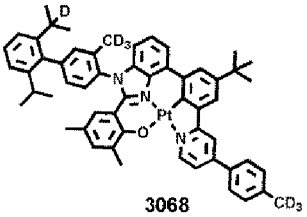
3068

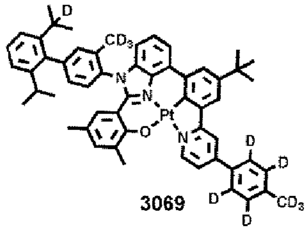
3069

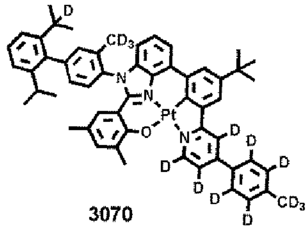
3070

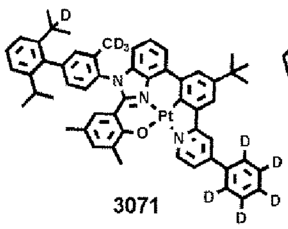
3071

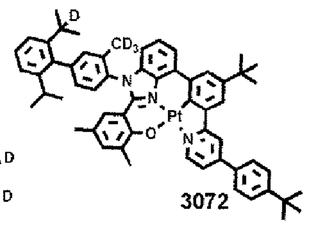
3072

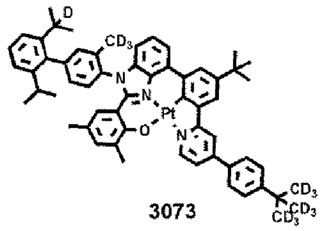
3073

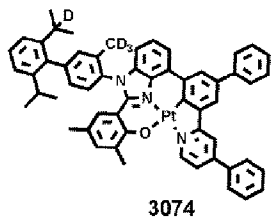
3074

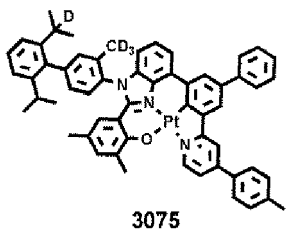
3075

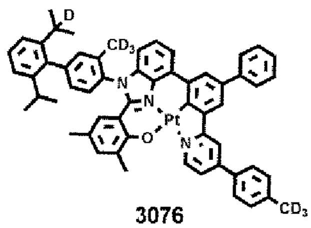
3076

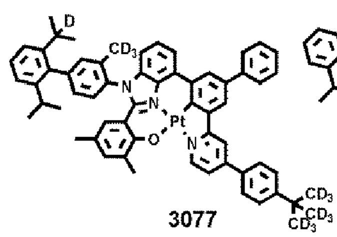
3077

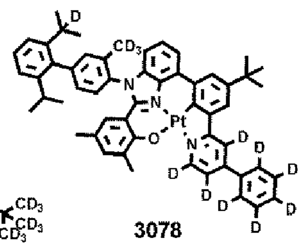
3078

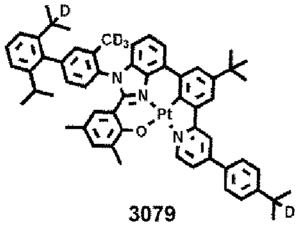
3079

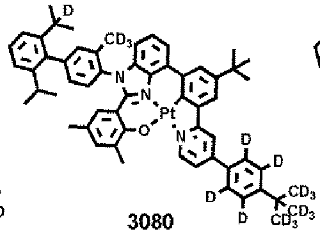
3080

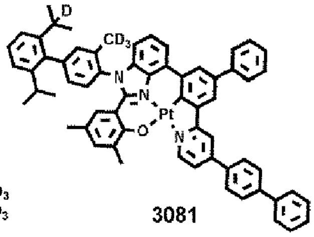
3081

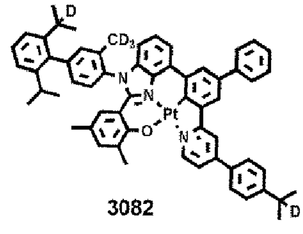
3082

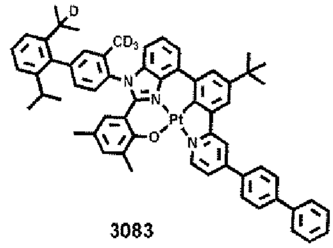
3083

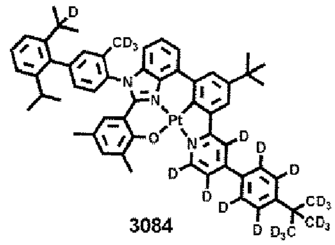
3084

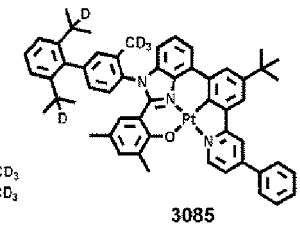
3085

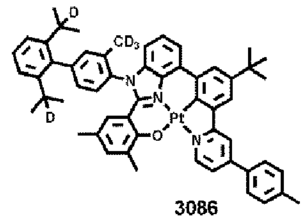
3086

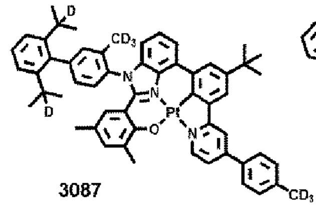
3087

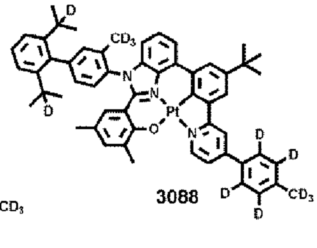
3088

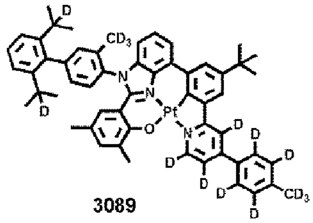
3089

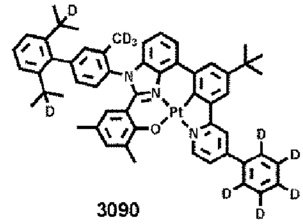
3090

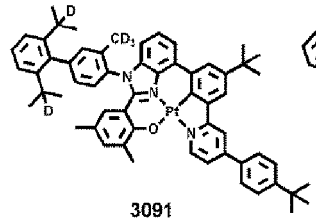
3091

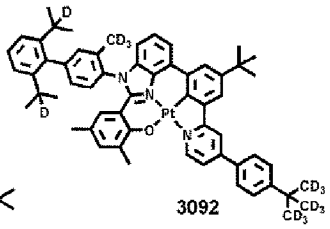
3092

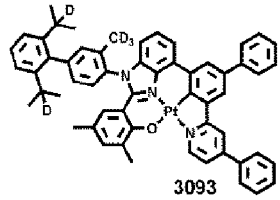
3093

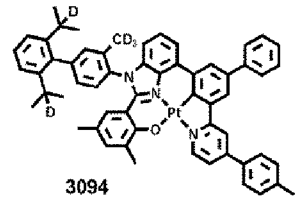
3094

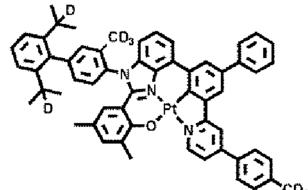
3095

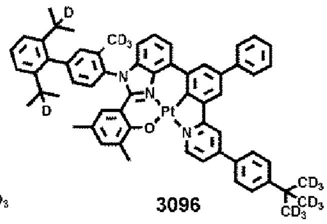
3096

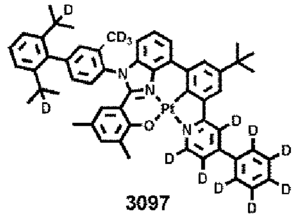
3097

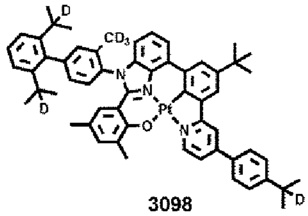
3098

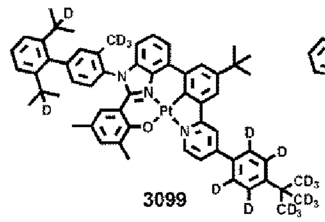
3099

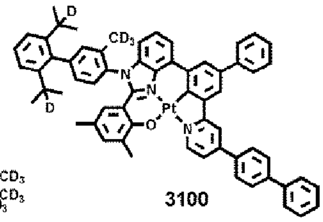
3100

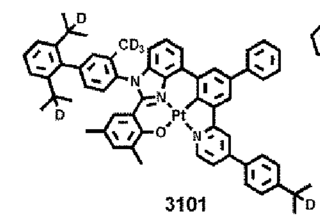
3101

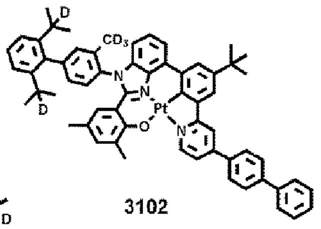
3102

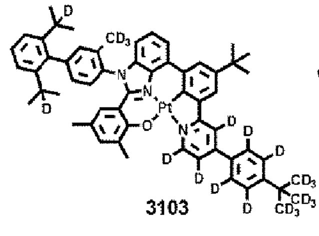
3103

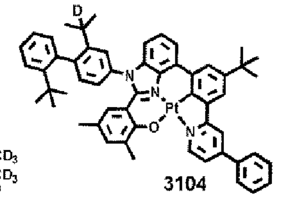
3104

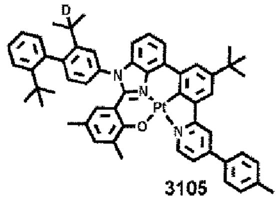
3105

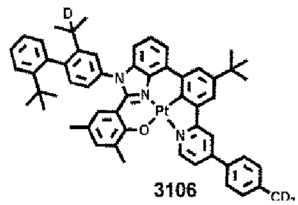
3106

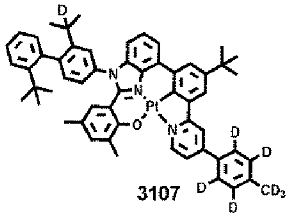
3107

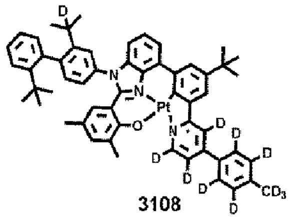
3108

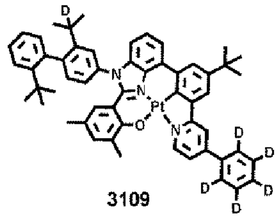
3109

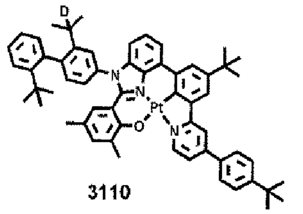
3110

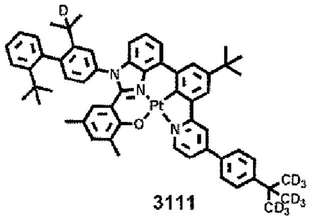
3111

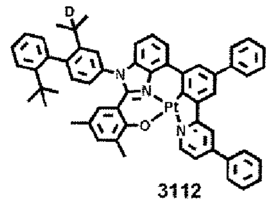
3112

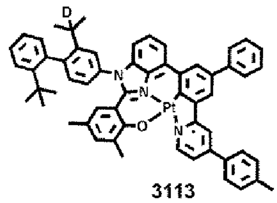
3113

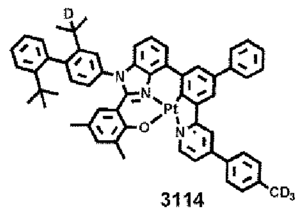
3114

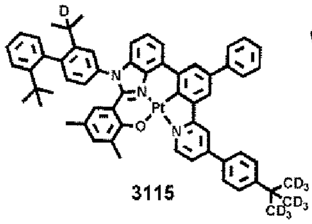
3115

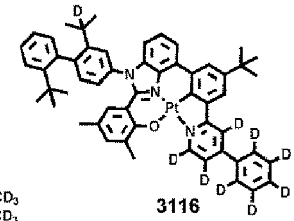
3116

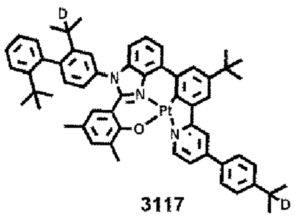
3117

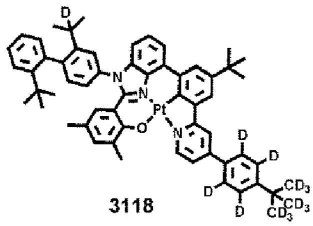
3118

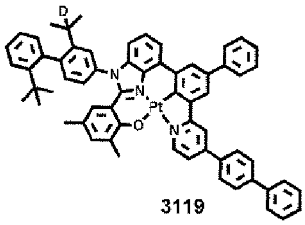
3119

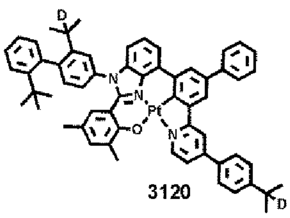
3120

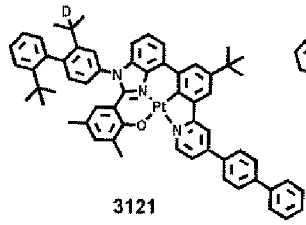
3121

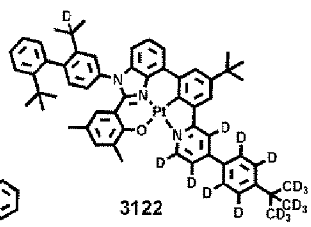
3122

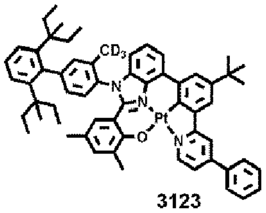
3123

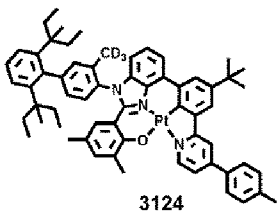
3124

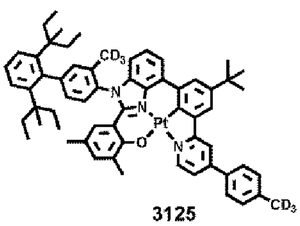
3125

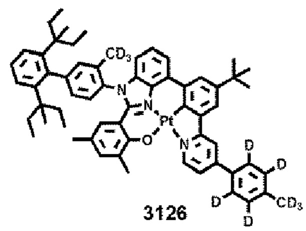
3126

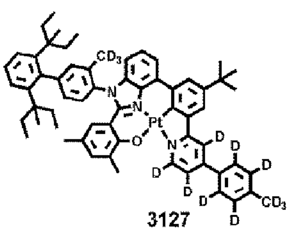
3127

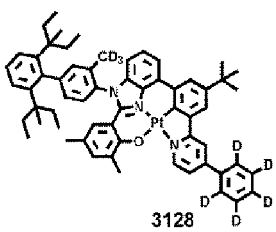
3128

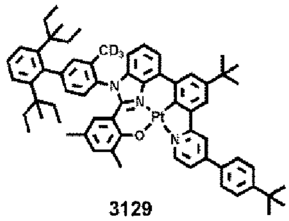
3129

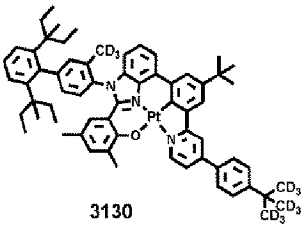
3130

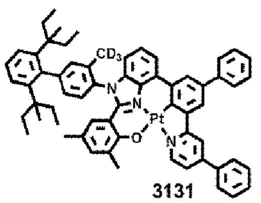
3131

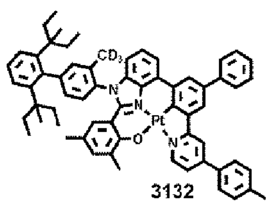
3132

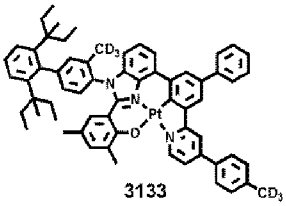
3133

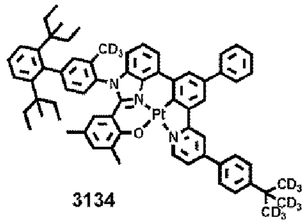
3134

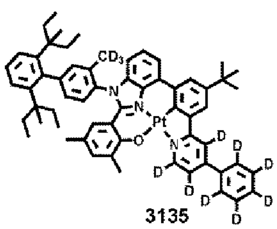
3135

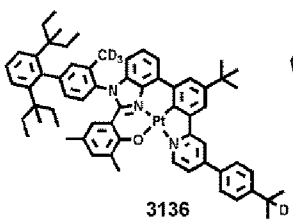
3136

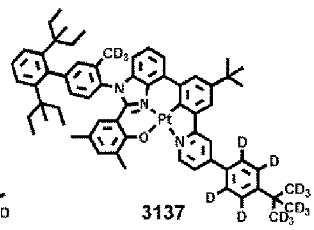
3137

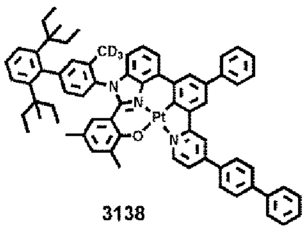
3138

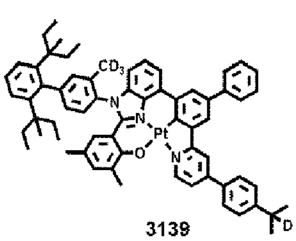
3139

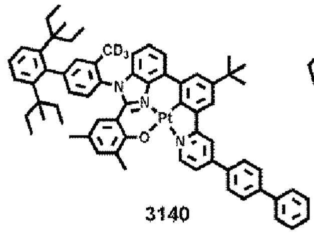
3140

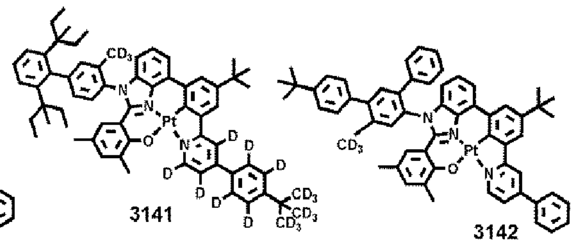
3141

3142

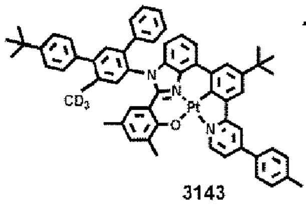
3143

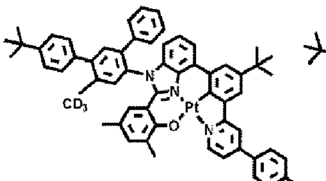
3144

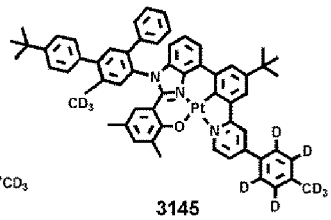
3145

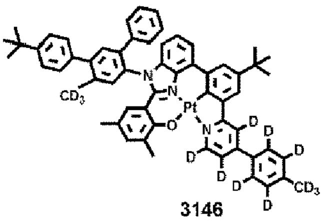
3146

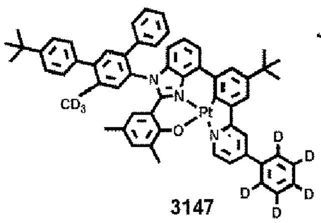
3147

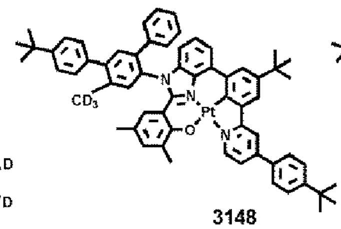
3148

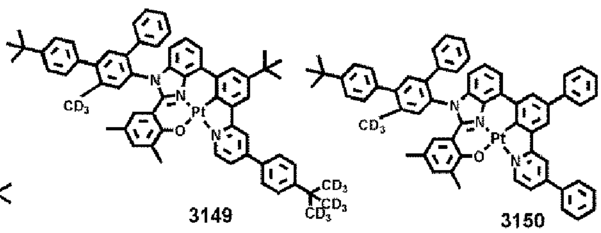
3149

3150

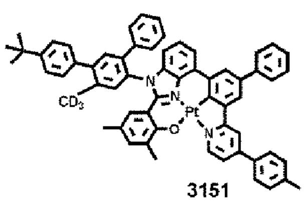
3151

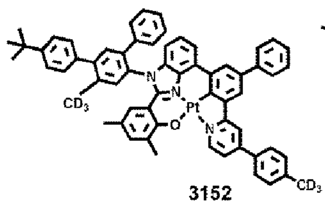
3152

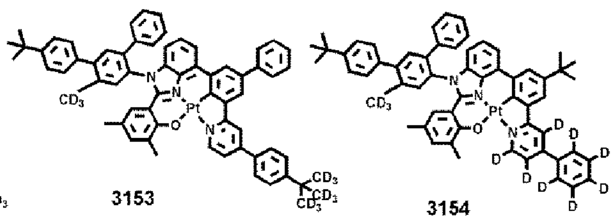
3153

3154

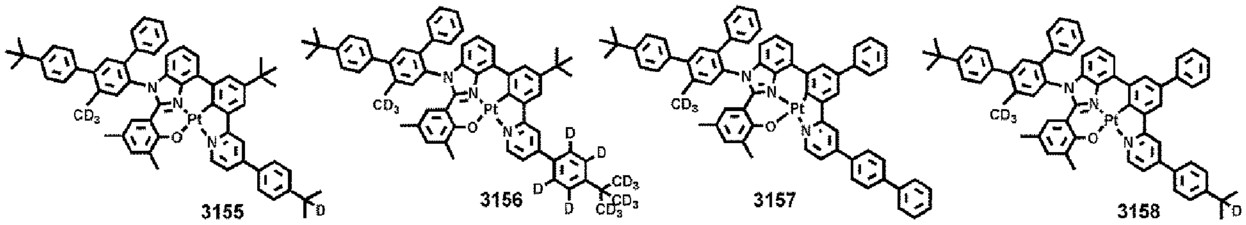

3155 3156 3157 3158

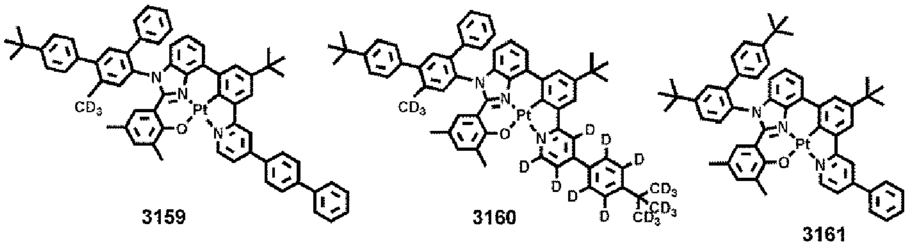

3159 3160 3161

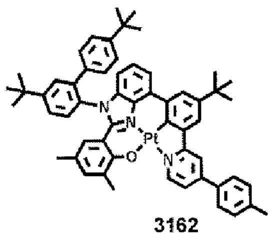

3162

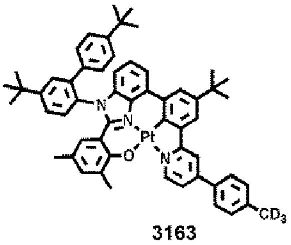

3163

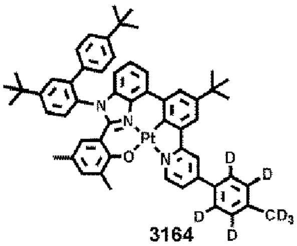

3164

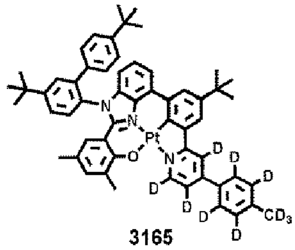

3165

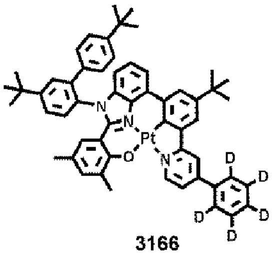

3166

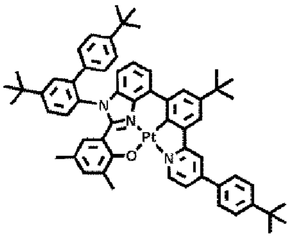

3167

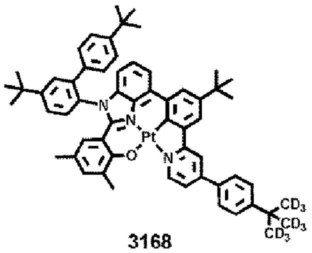

3168

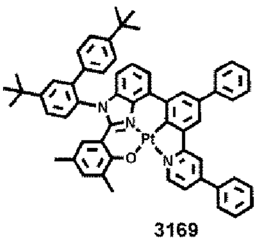

3169

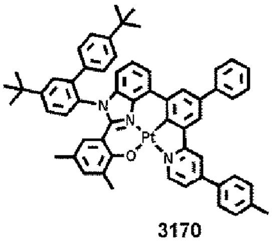

3170

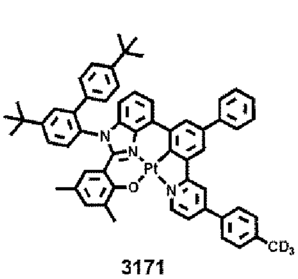

3171

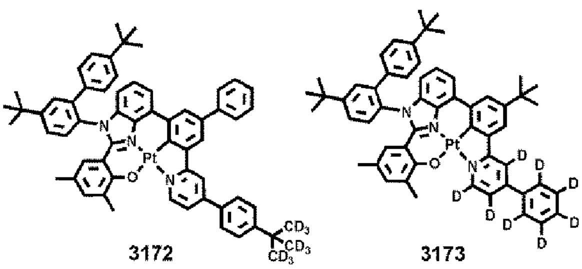

3172 3173

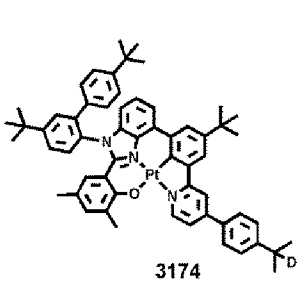

3174

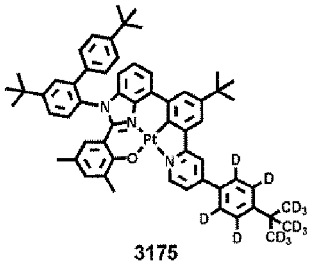

3175

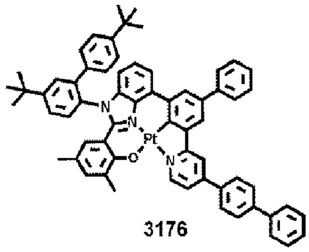

3176

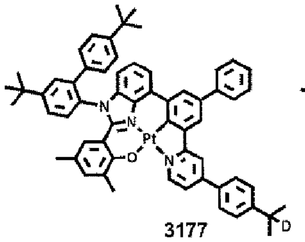

3177

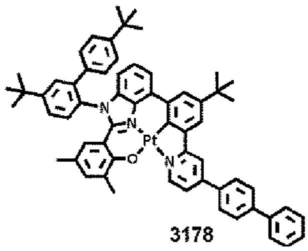

3178

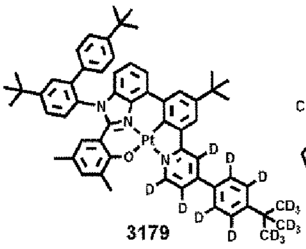

3179

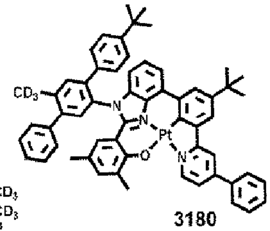

3180

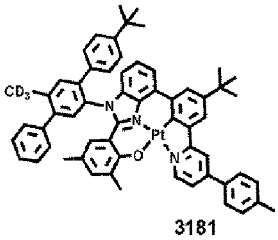

3181

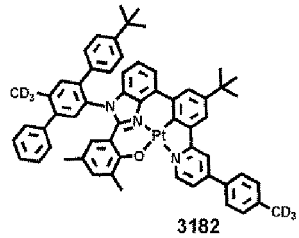

3182

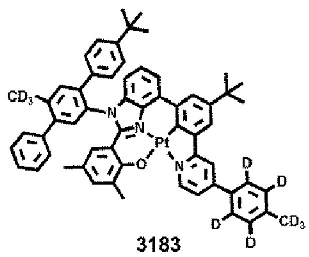

3183

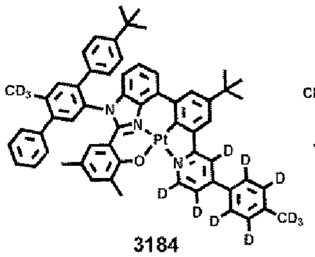

3184

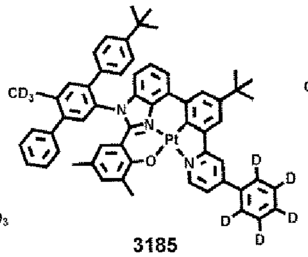

3185

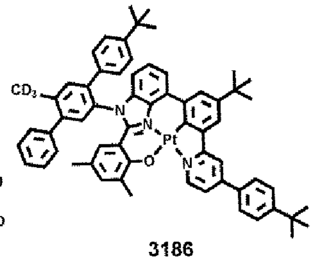

3186

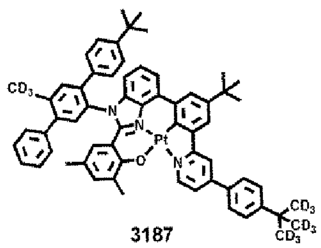

3187

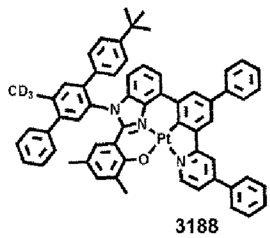

3188

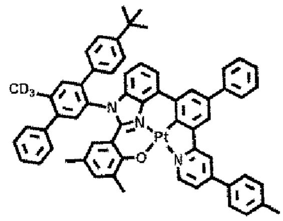

3189

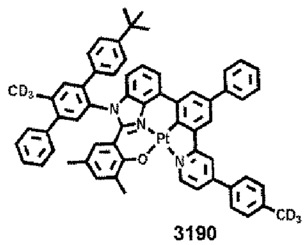

3190

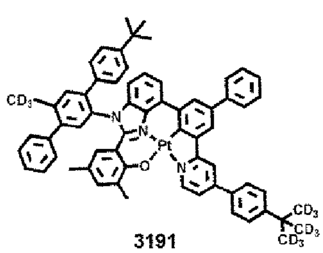

3191

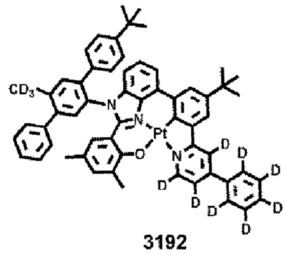

3192

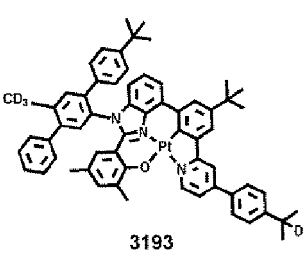

3193

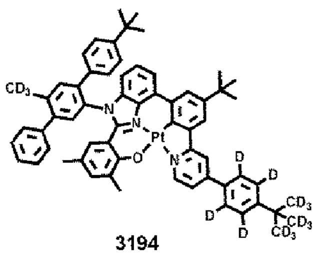

3194

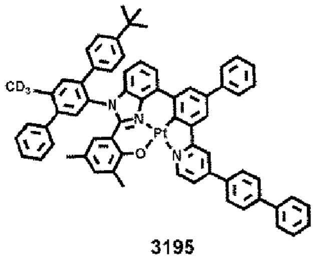

3195

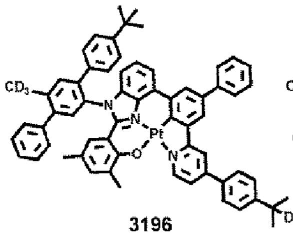

3196

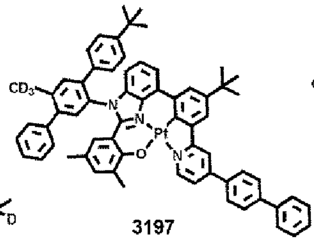

3197

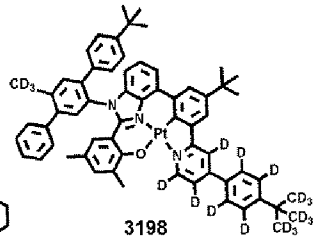

3198

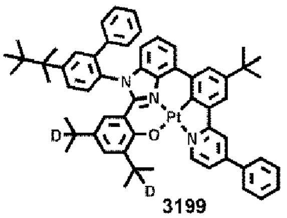

3199

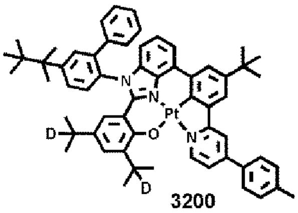

3200

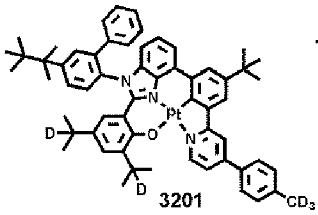

3201

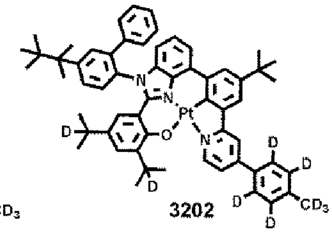

3202

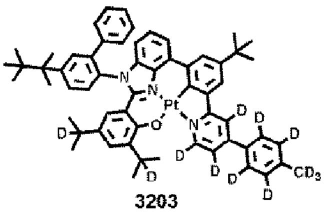

3203

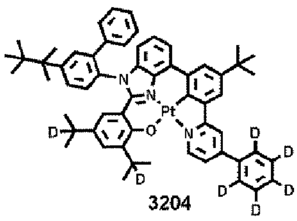

3204

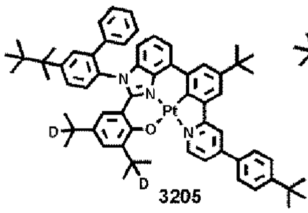

3205

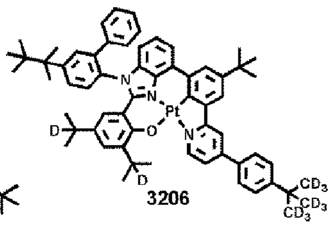

3206

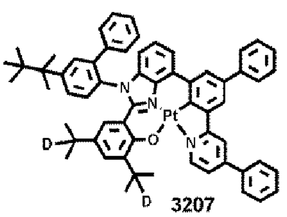

3207

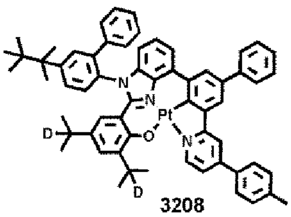

3208

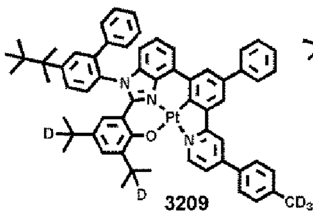

3209

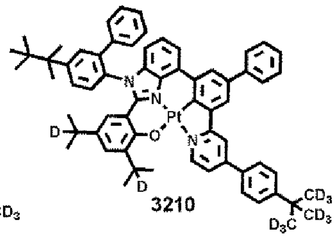

3210

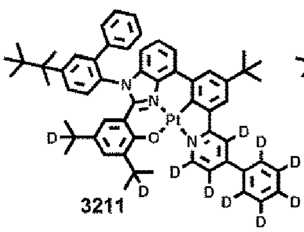

3211

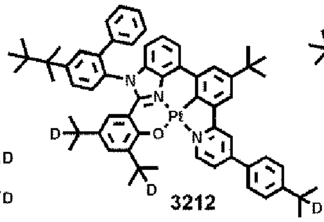

3212

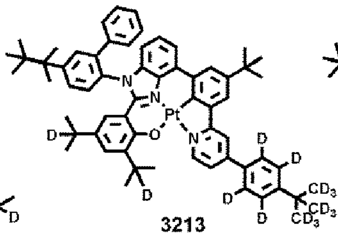

3213

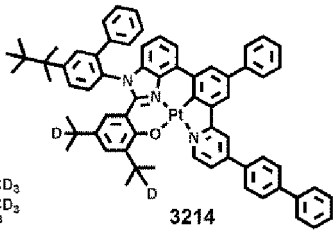

3214

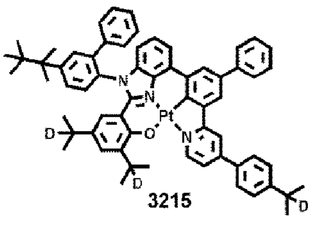

3215

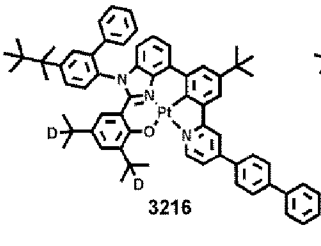

3216

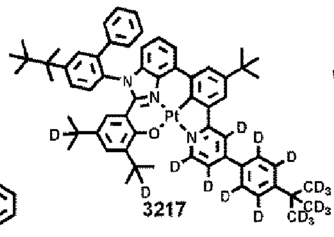

3217

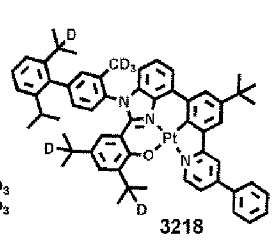

3218

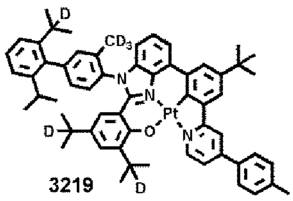
3219

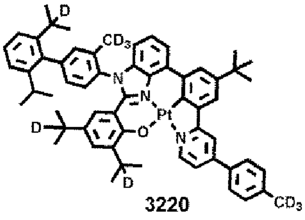
3220

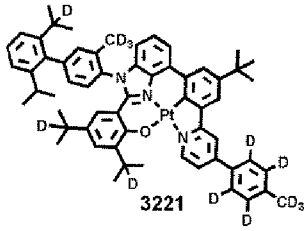
3221

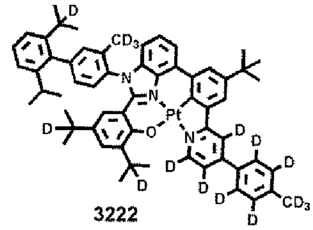
3222

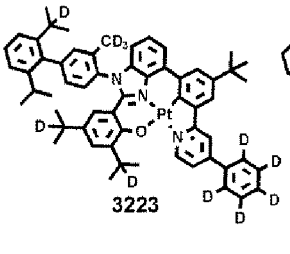
3223

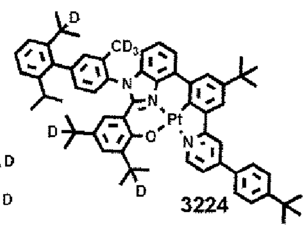
3224

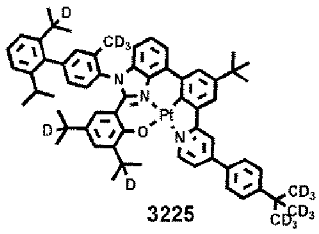
3225

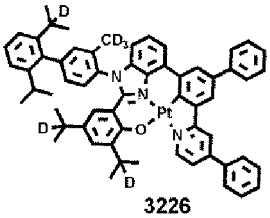
3226

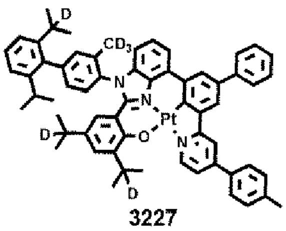
3227

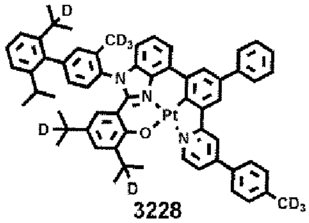
3228

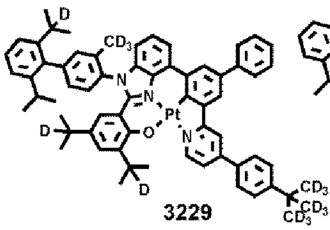
3229

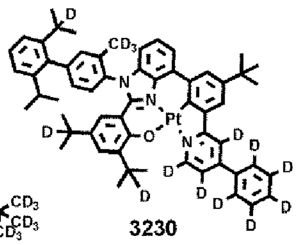
3230

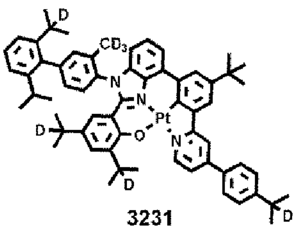
3231

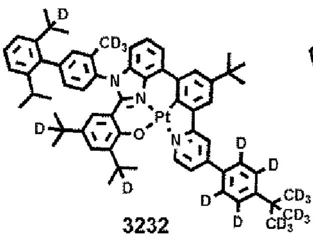
3232

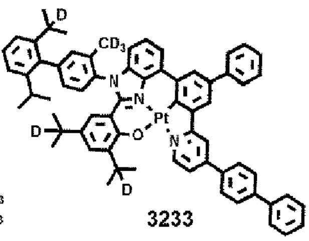
3233

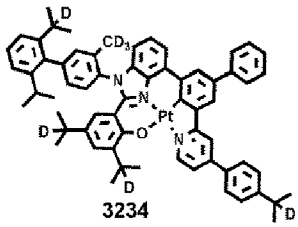
3234

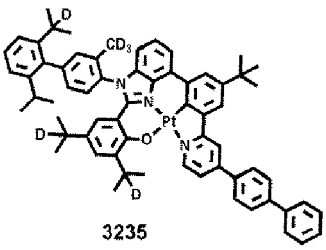
3235

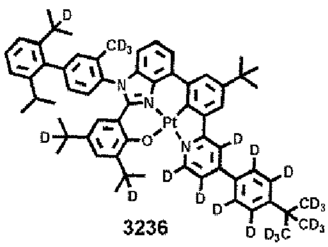
3236

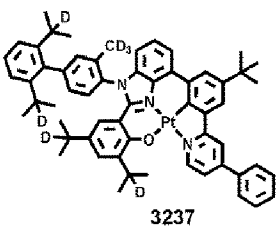
3237

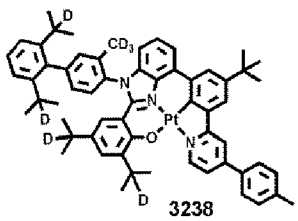
3238

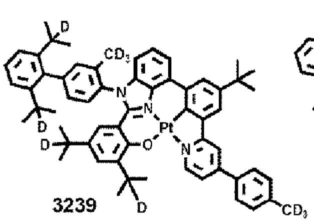
3239

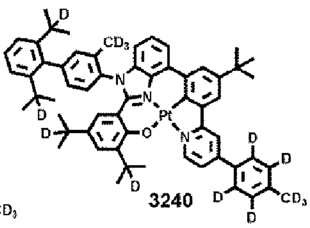
3240

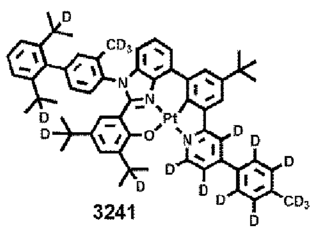
3241

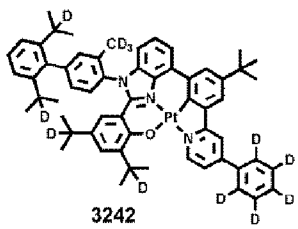
3242

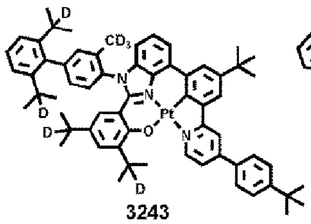
3243

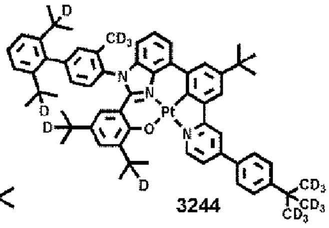
3244

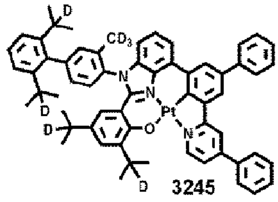
3245

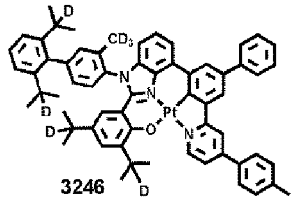
3246

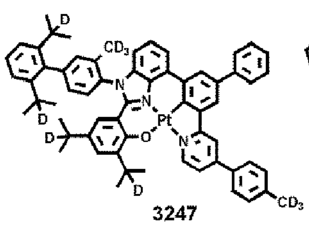
3247

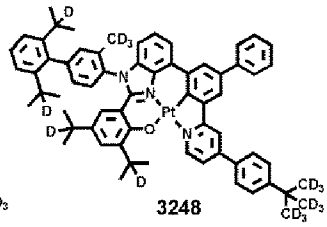
3248

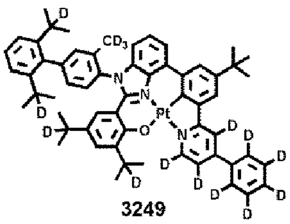
3249

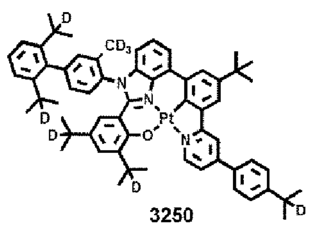
3250

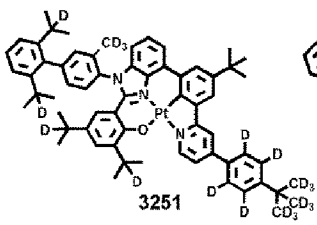
3251

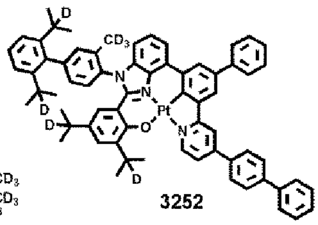
3252

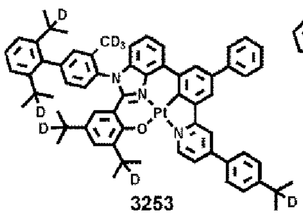
3253

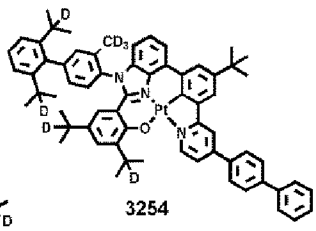
3254

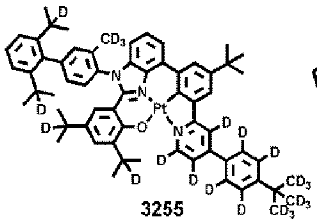
3255

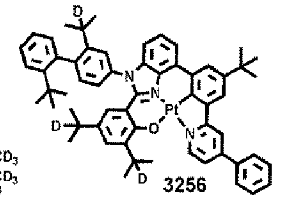
3256

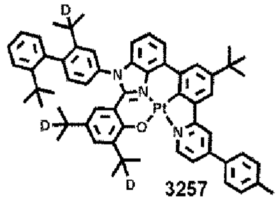
3257

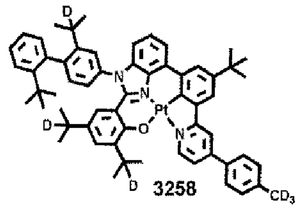
3258

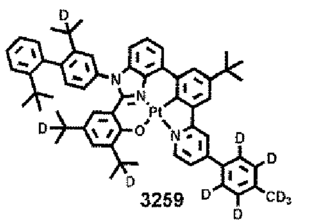
3259

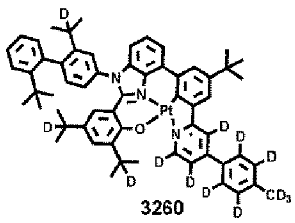
3260

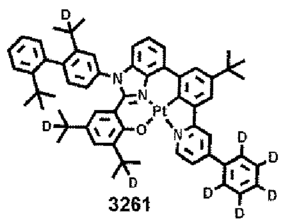
3261

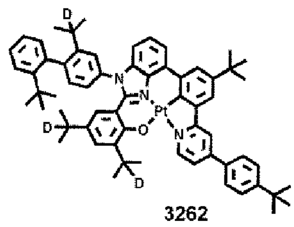
3262

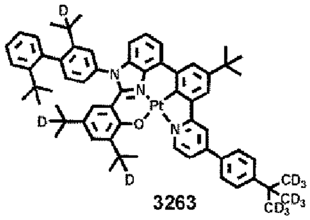
3263

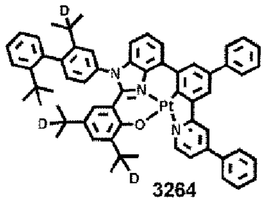
3264

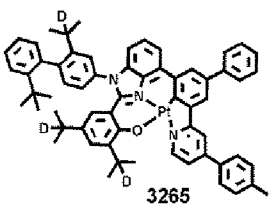
3265

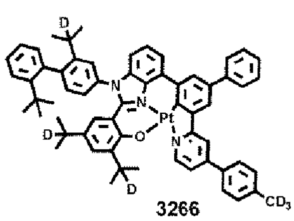
3266

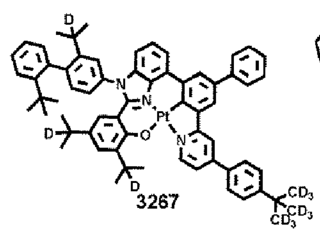
3267

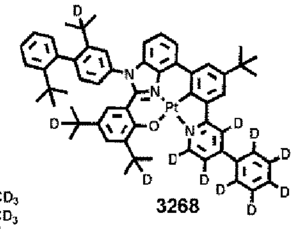
3268

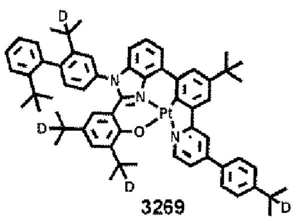
3269

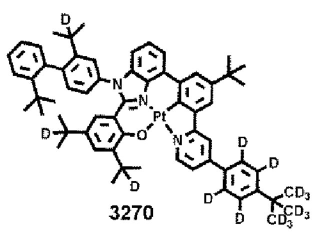
3270

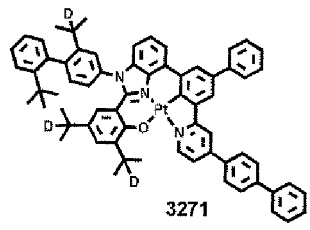
3271

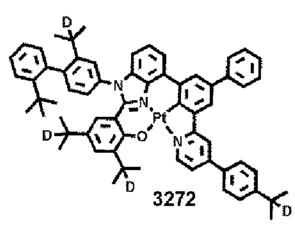
3272

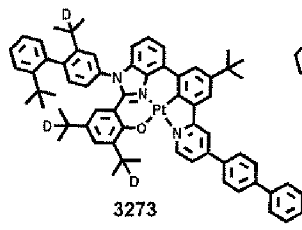
3273

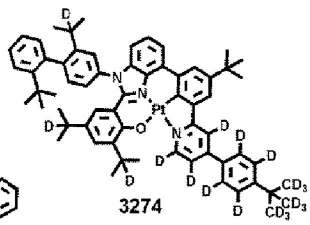
3274

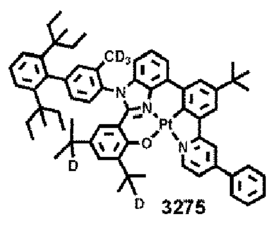
3275

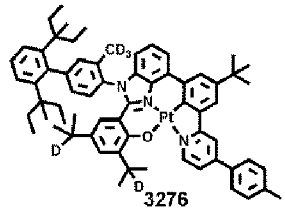
3276

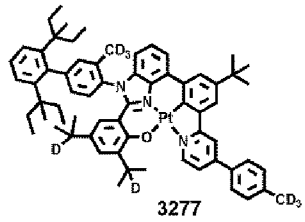
3277

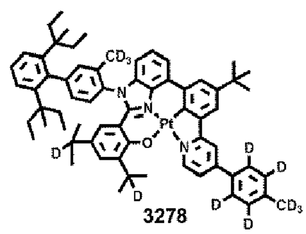
3278

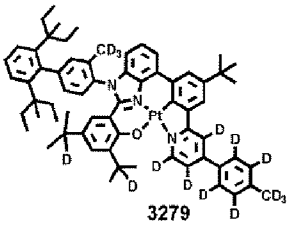
3279

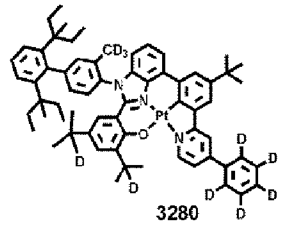
3280

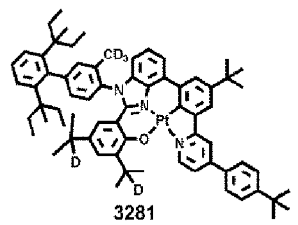
3281

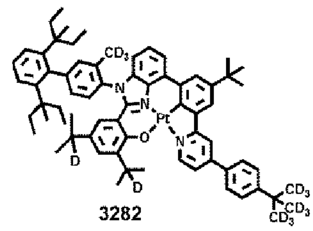
3282

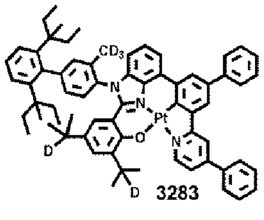
3283

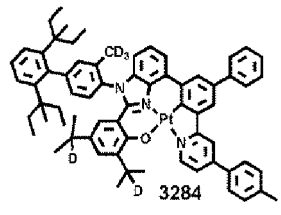
3284

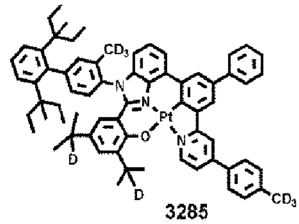
3285

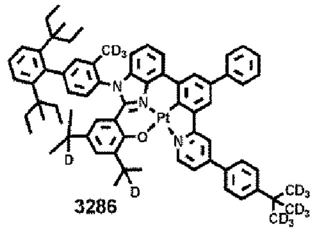
3286

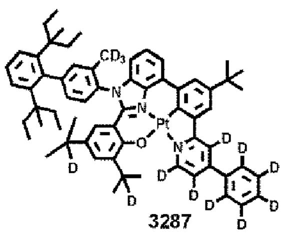
3287

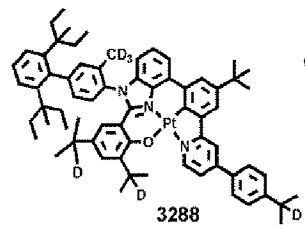
3288

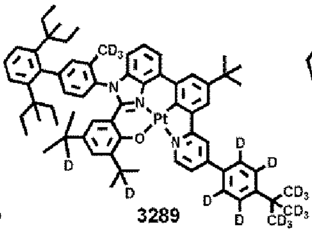
3289

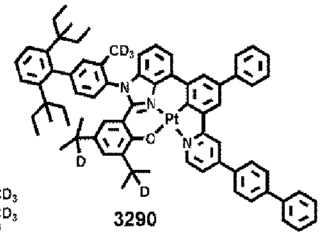
3290

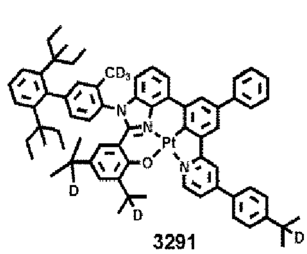
3291
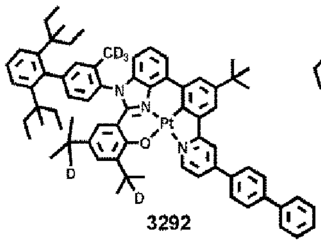
3292
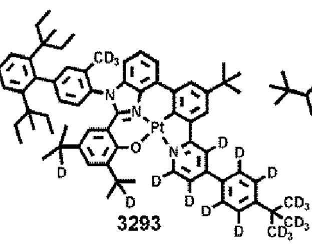
3293
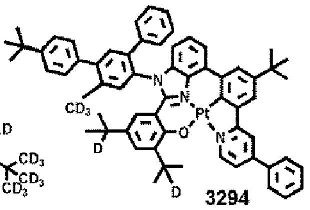
3294

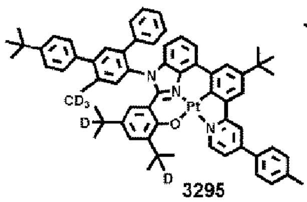
3295
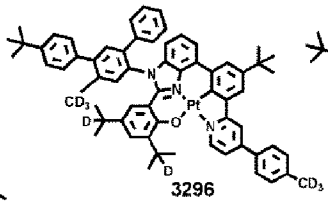
3296
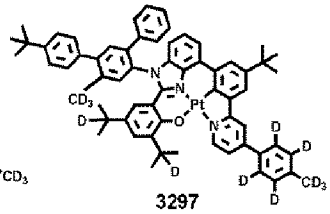
3297
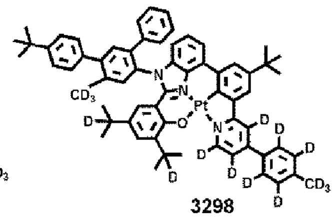
3298

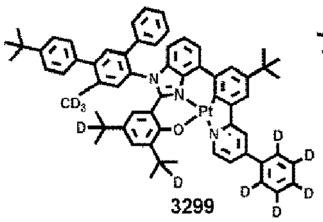
3299
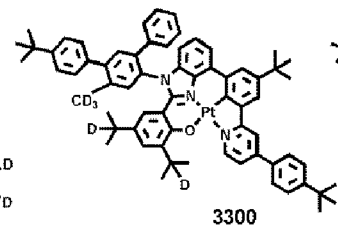
3300
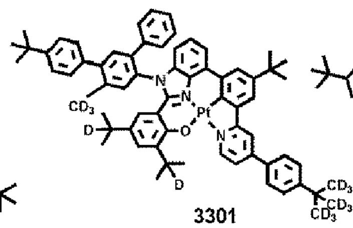
3301
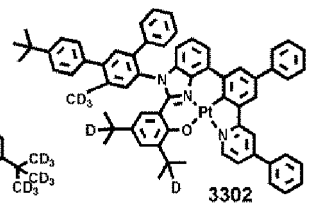
3302

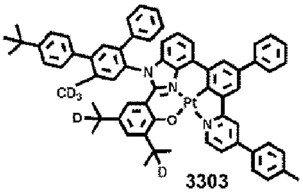
3303
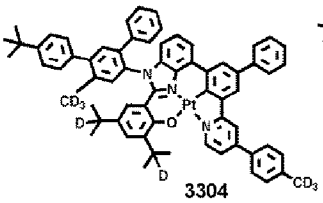
3304
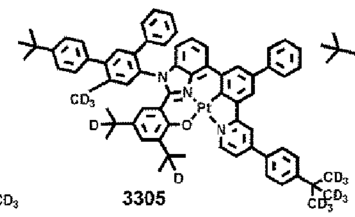
3305
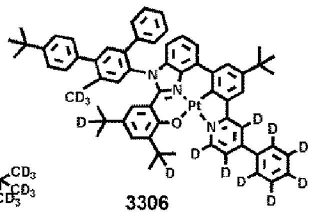
3306

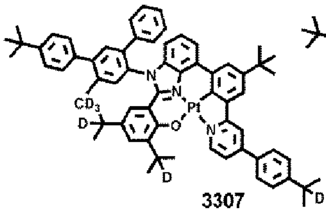
3307
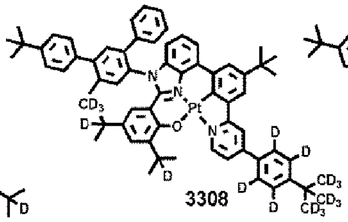
3308
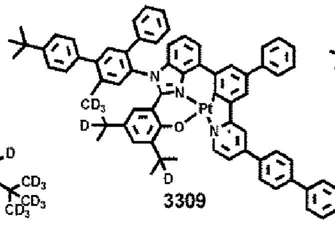
3309
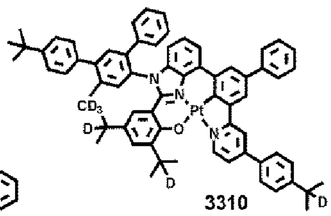
3310

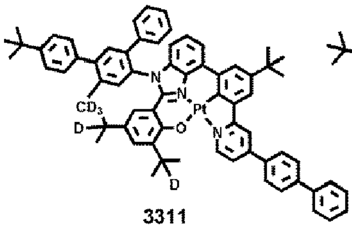
3311
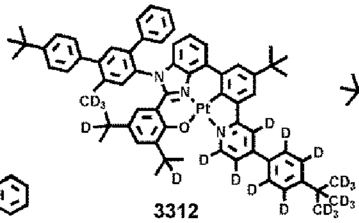
3312
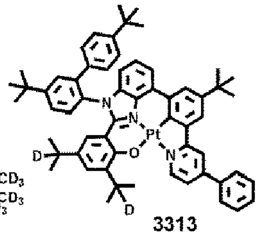
3313
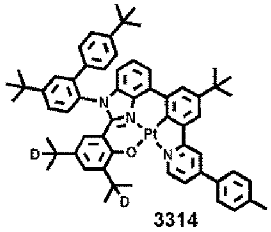
3314

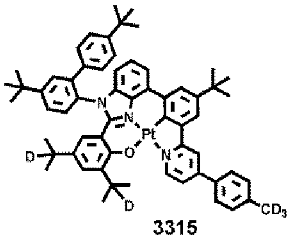
3315

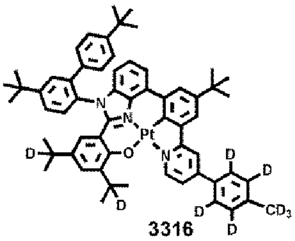
3316

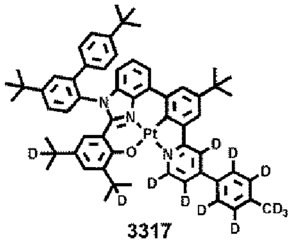
3317

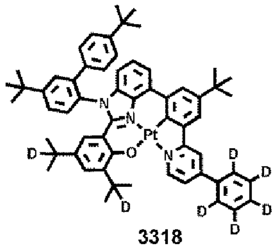
3318

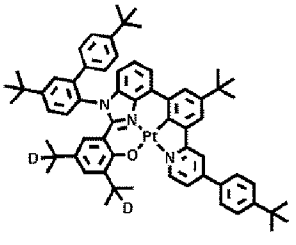
3319

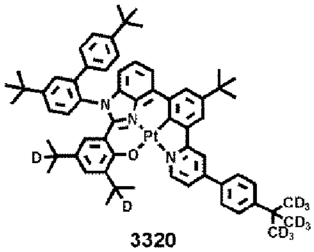
3320

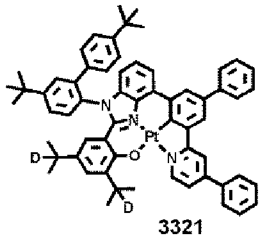
3321

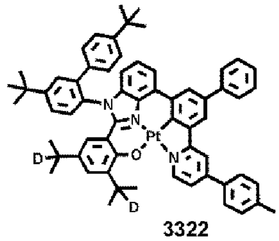
3322

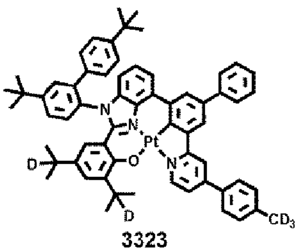
3323

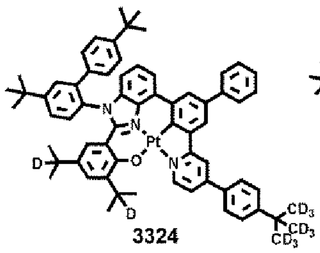
3324

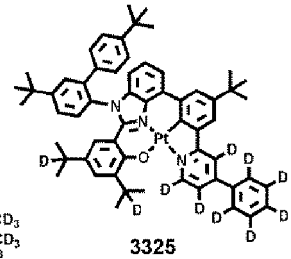
3325

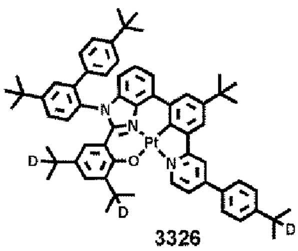
3326

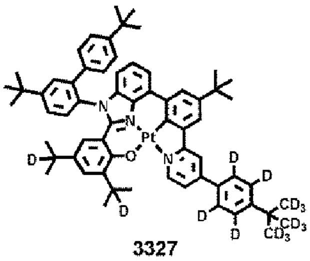
3327

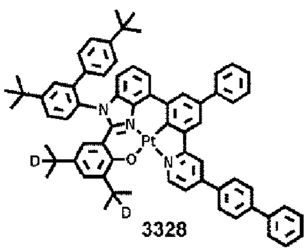
3328

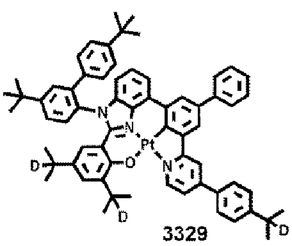
3329

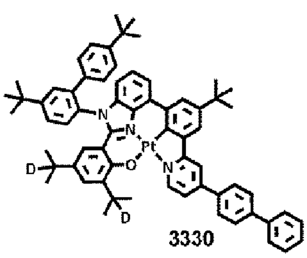
3330

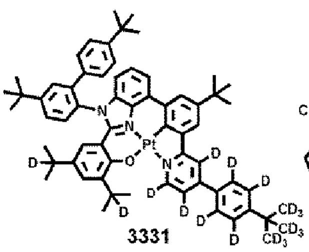
3331

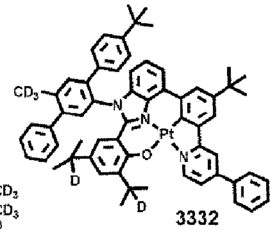
3332

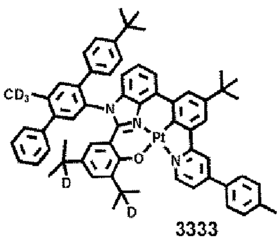
3333

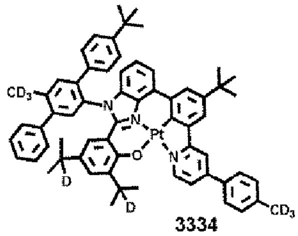
3334

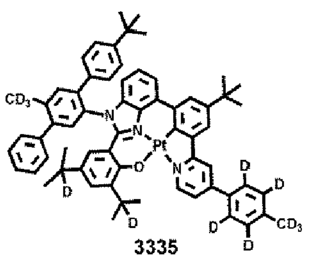
3335
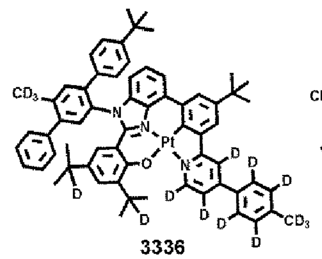
3336
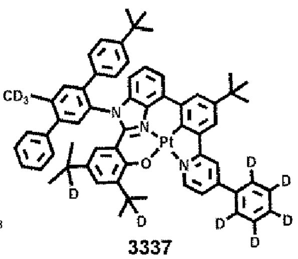
3337
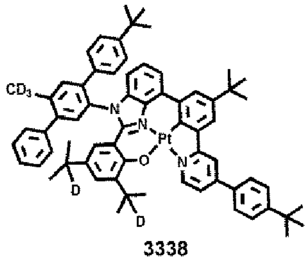
3338

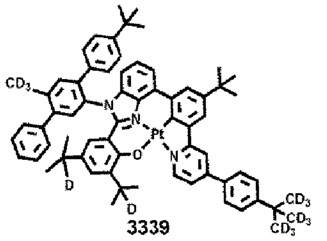
3339
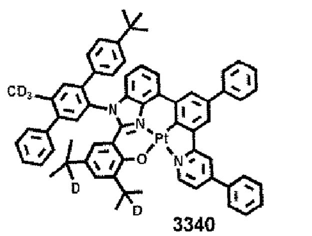
3340
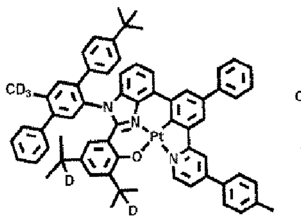
3341
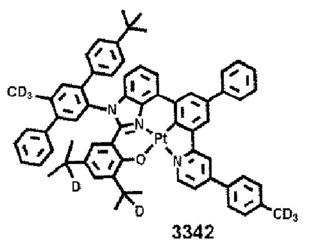
3342

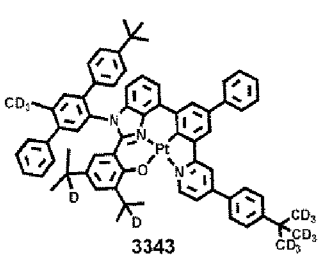
3343
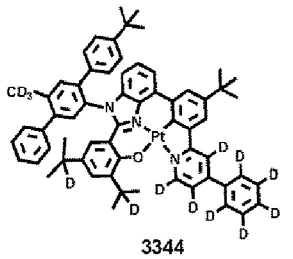
3344
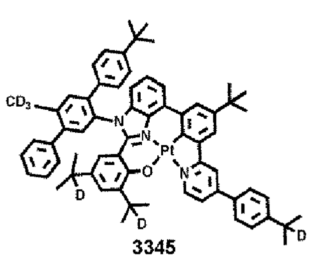
3345
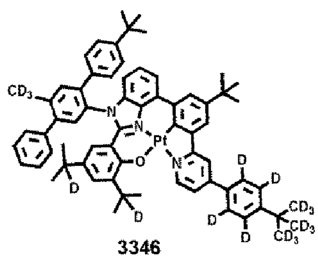
3346

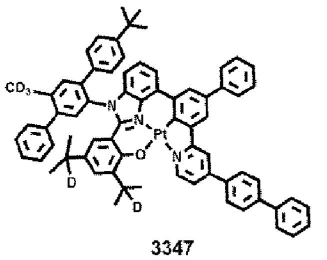
3347
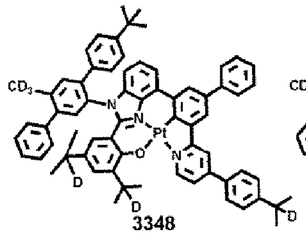
3348
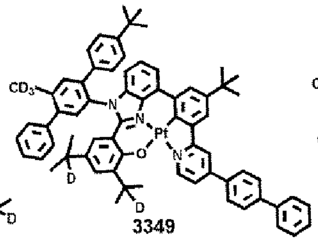
3349
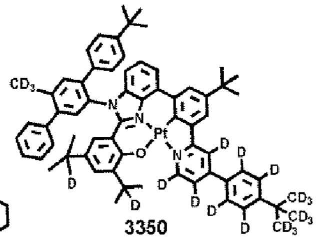
3350

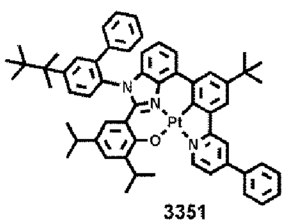
3351
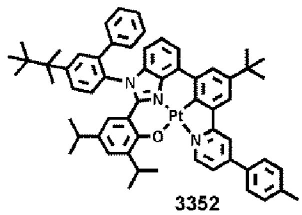
3352
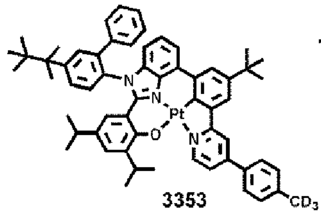
3353
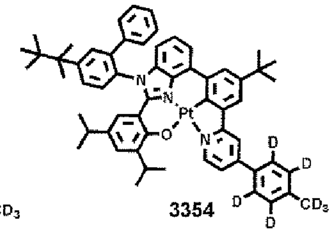
3354

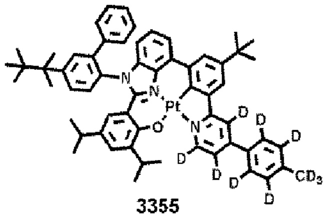
3355
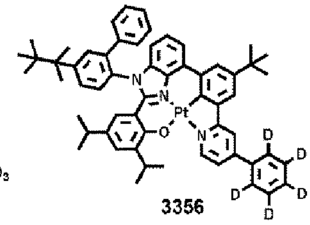
3356
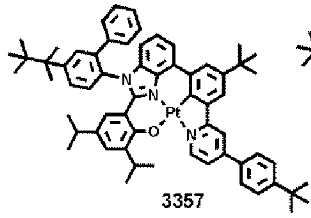
3357
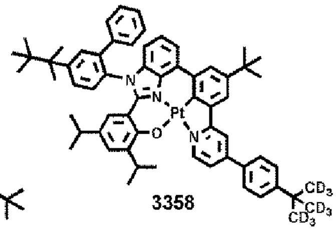
3358

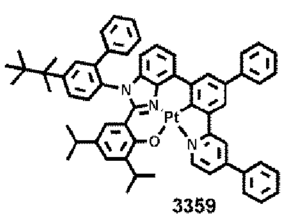
3359

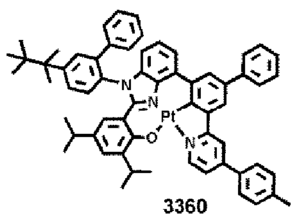
3360

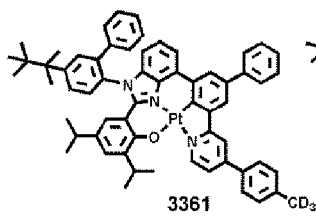
3361

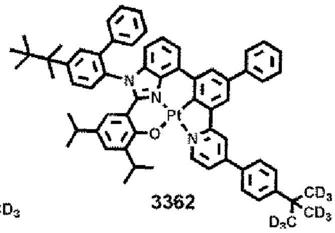
3362

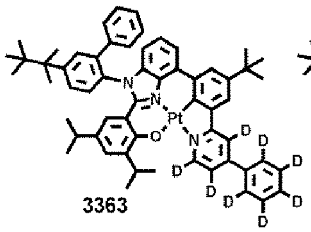
3363

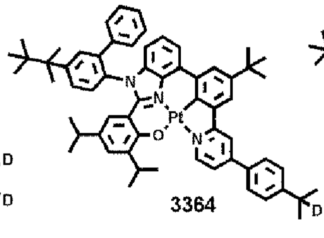
3364

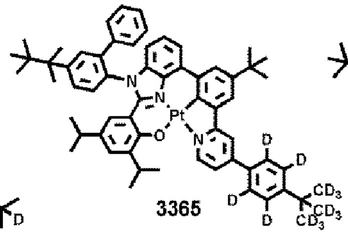
3365

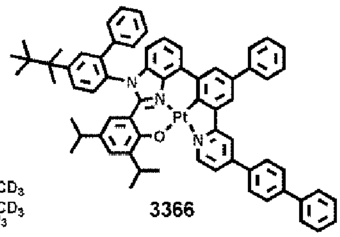
3366

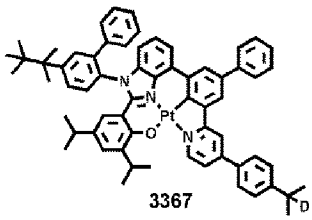
3367

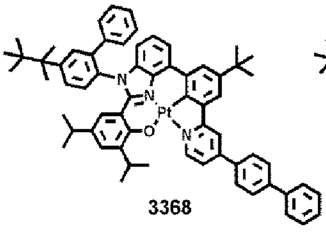
3368

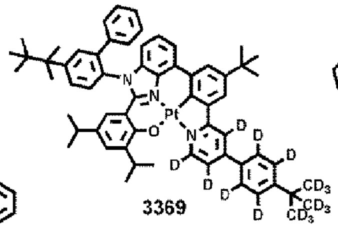
3369

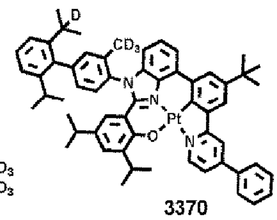
3370

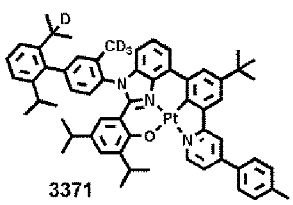
3371

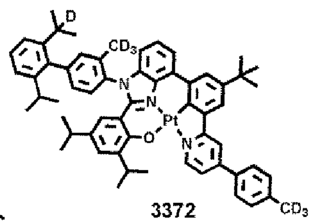
3372

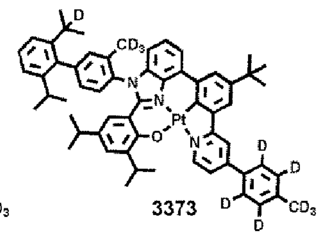
3373

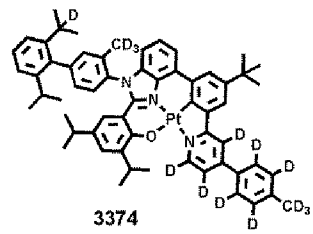
3374

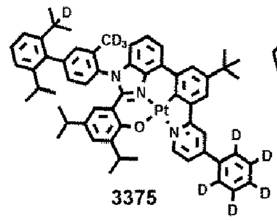
3375

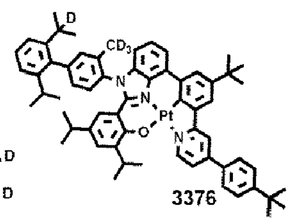
3376

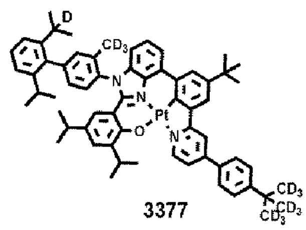
3377

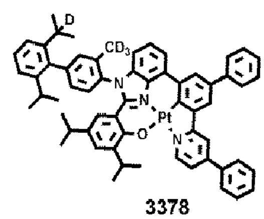
3378

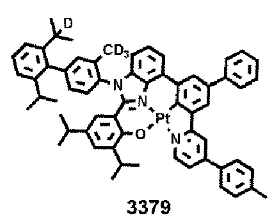
3379

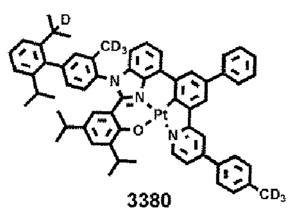
3380

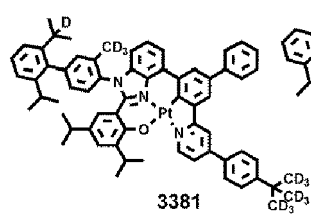
3381

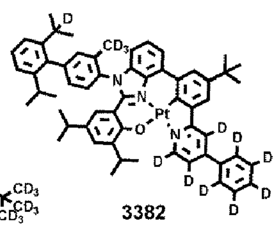
3382

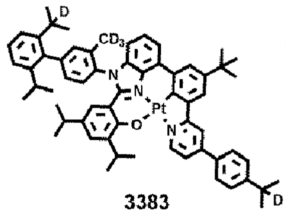
3383
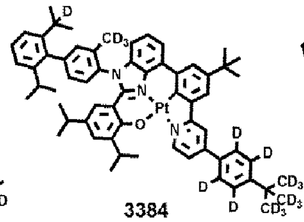
3384
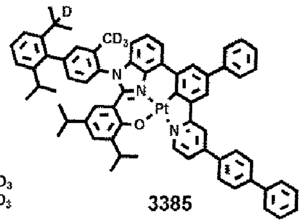
3385
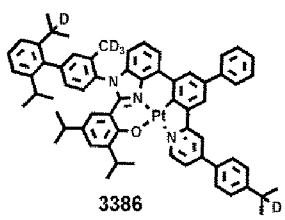
3386

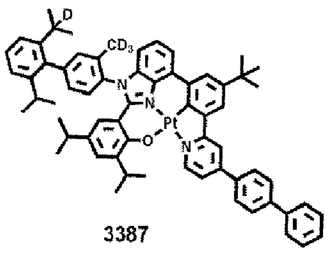
3387
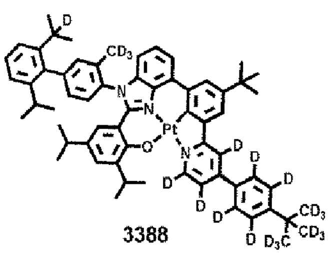
3388
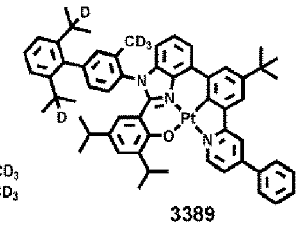
3389
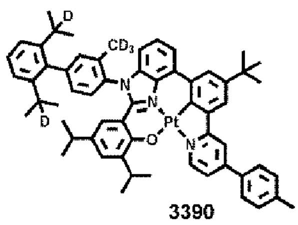
3390

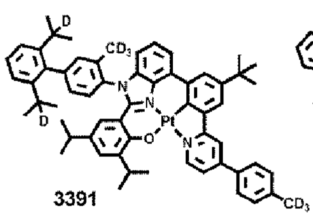
3391
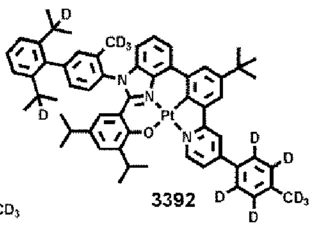
3392
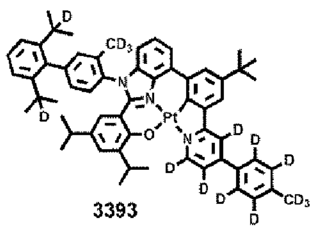
3393
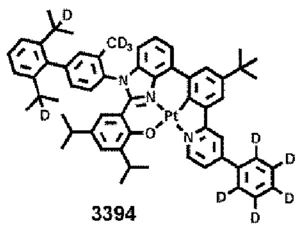
3394

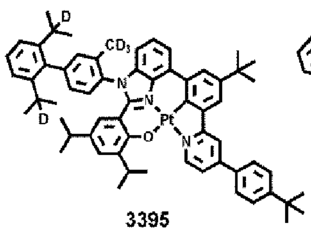
3395
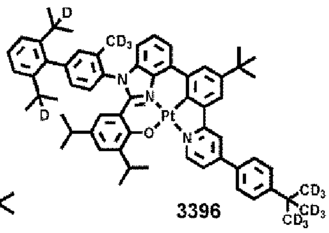
3396
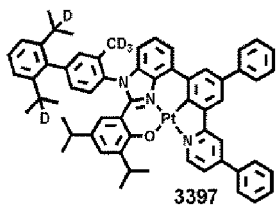
3397
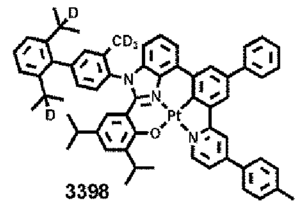
3398

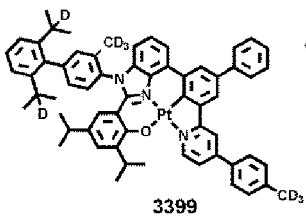
3399
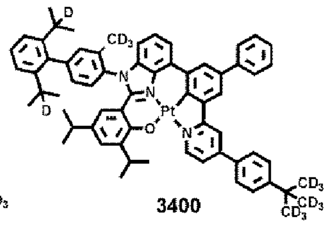
3400
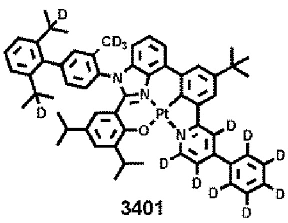
3401
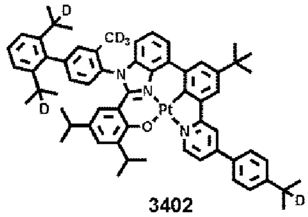
3402

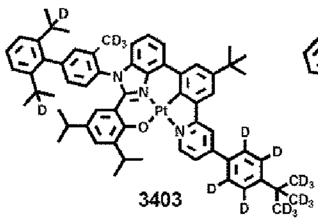
3403
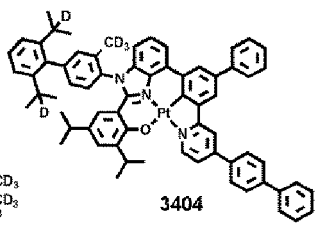
3404
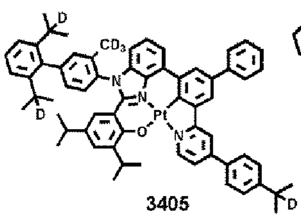
3405
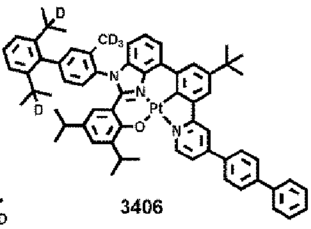
3406

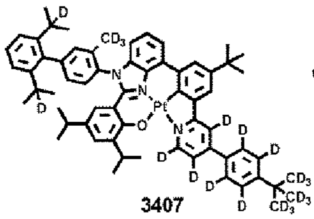
3407

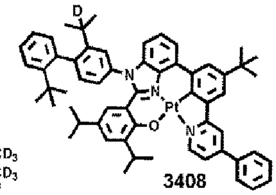
3408

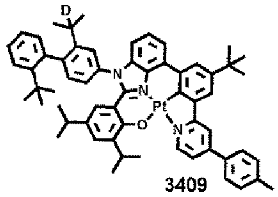
3409

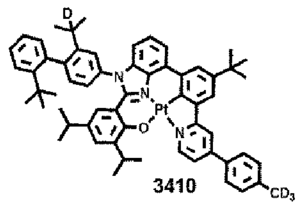
3410

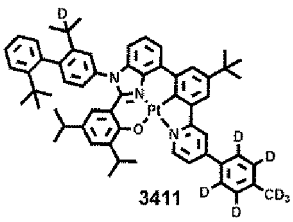
3411

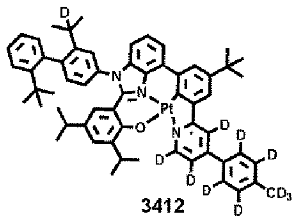
3412

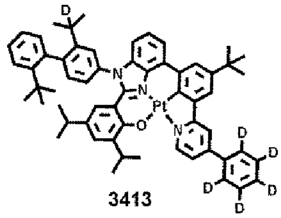
3413

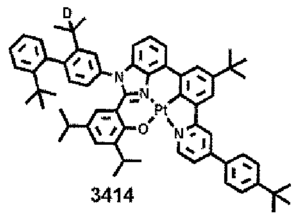
3414

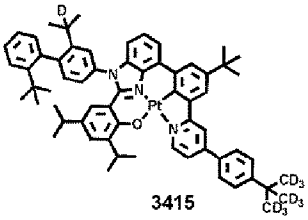
3415

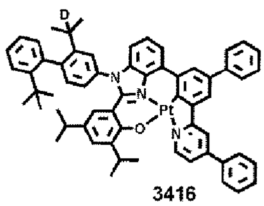
3416

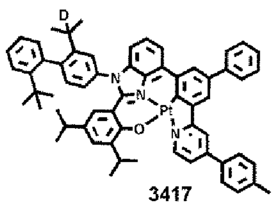
3417

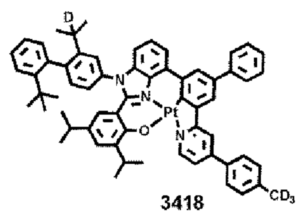
3418

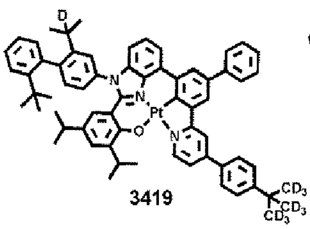
3419

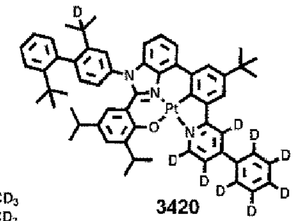
3420

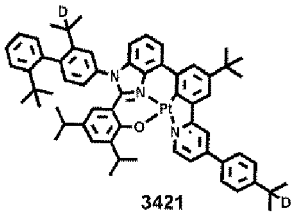
3421

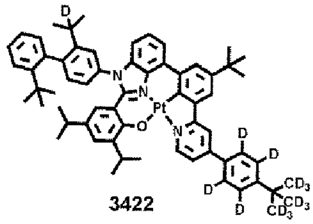
3422

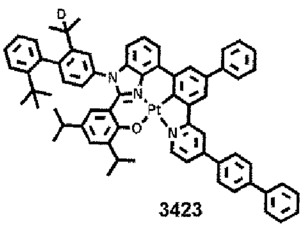
3423

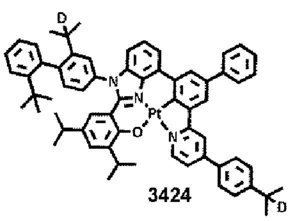
3424

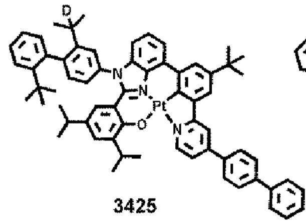
3425

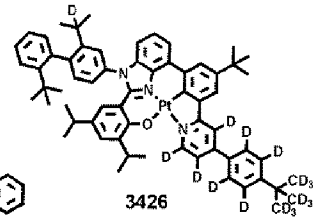
3426

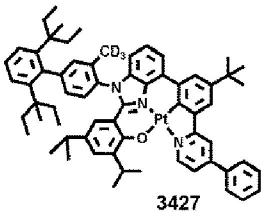
3427

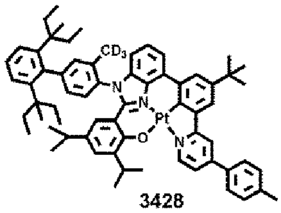
3428

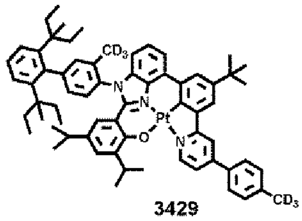
3429

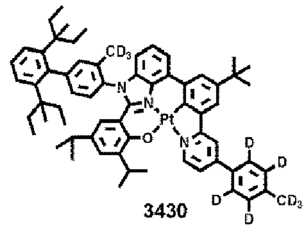
3430

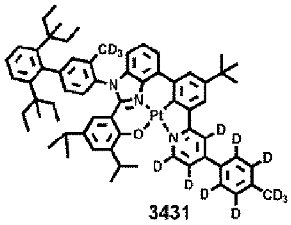
3431

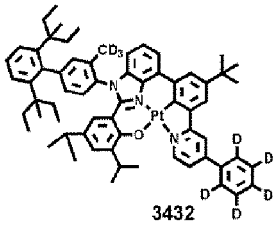
3432

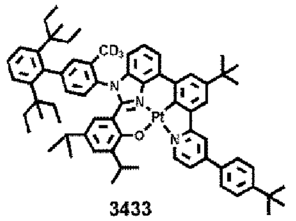
3433

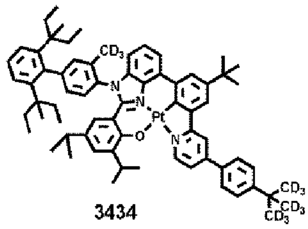
3434

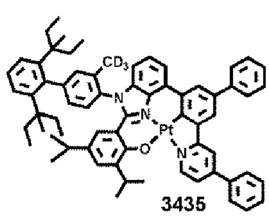
3435

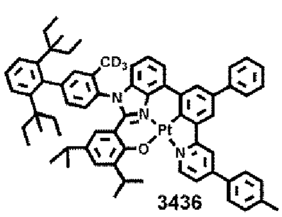
3436

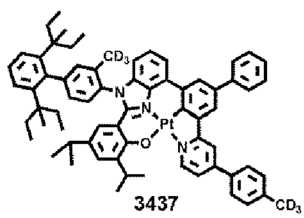
3437

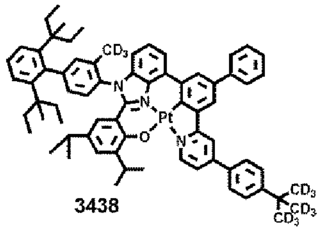
3438

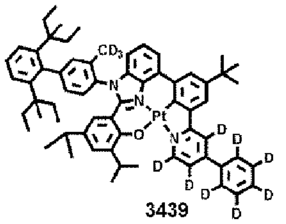
3439

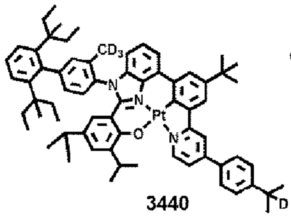
3440

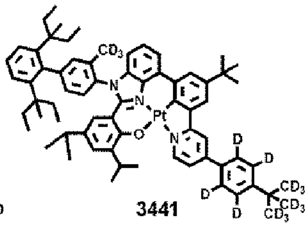
3441

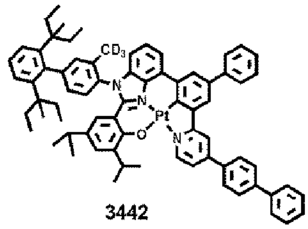
3442

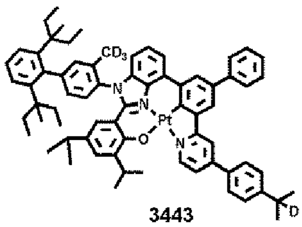
3443

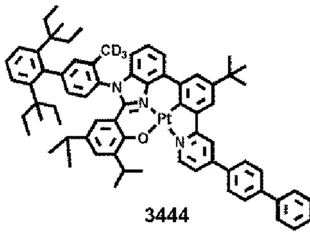
3444

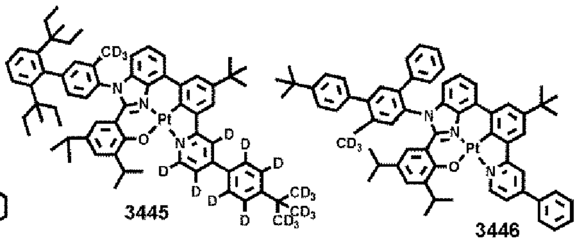
3445

3446

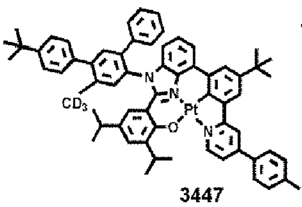
3447

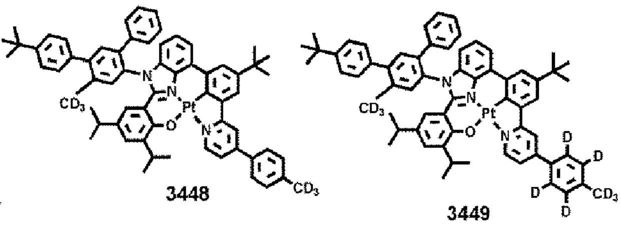
3448

3449

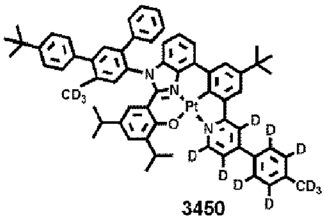
3450

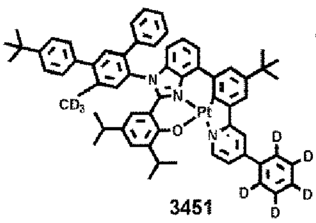
3451

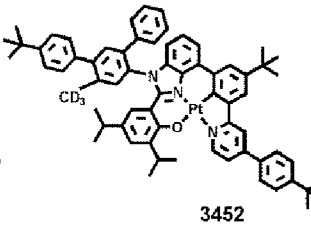
3452

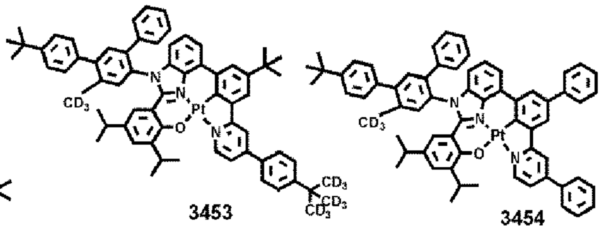
3453

3454

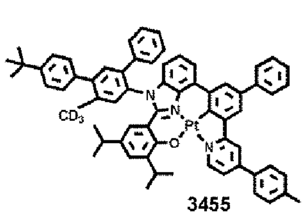
3455

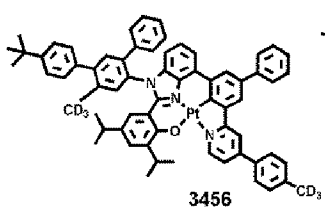
3456

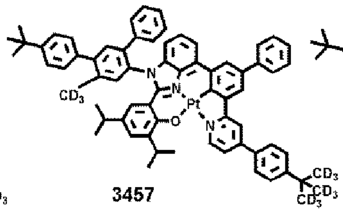
3457

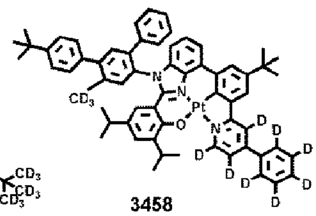
3458

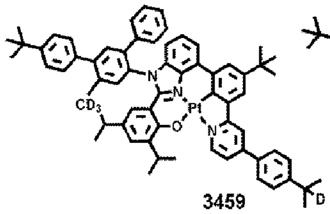
3459

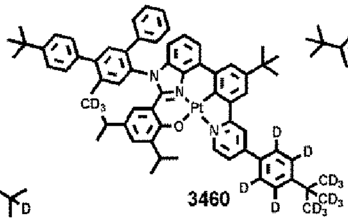
3460

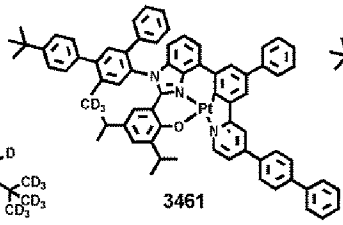
3461

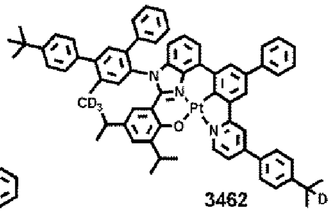
3462

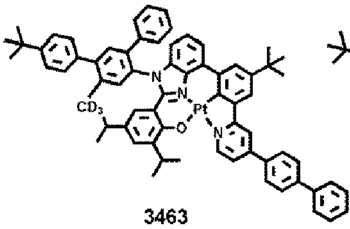
3463

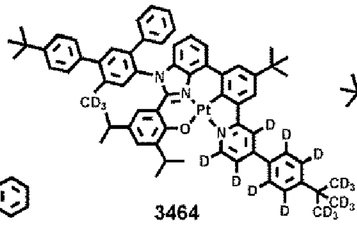
3464

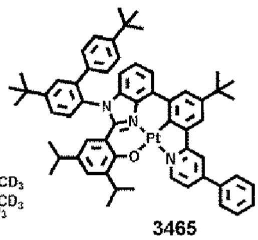
3465

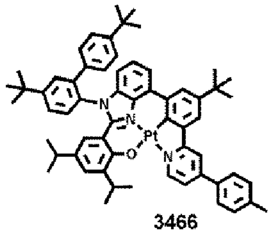
3466

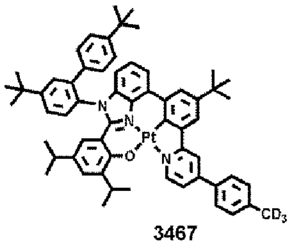
3467

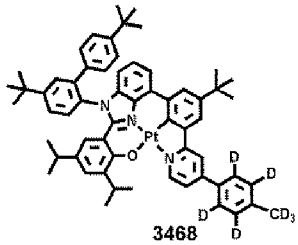
3468

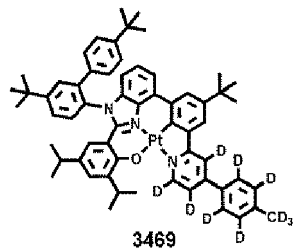
3469

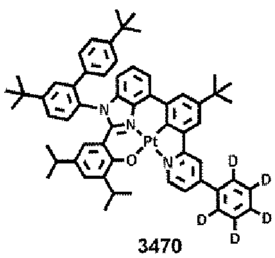
3470

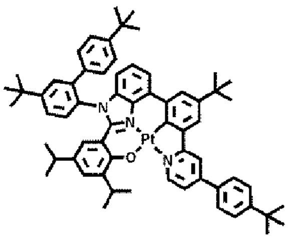
3471

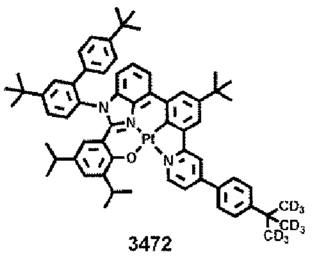
3472

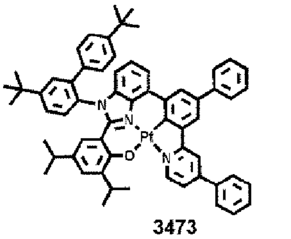
3473

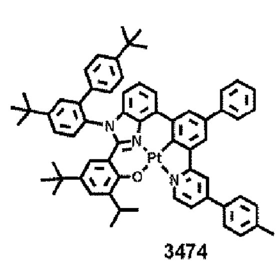
3474

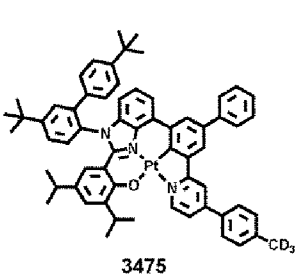
3475

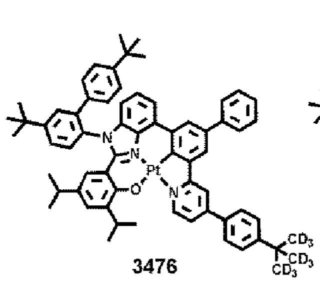
3476

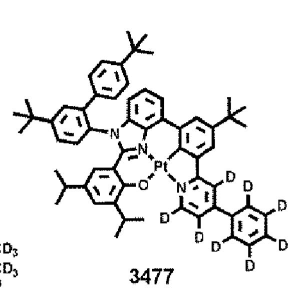
3477

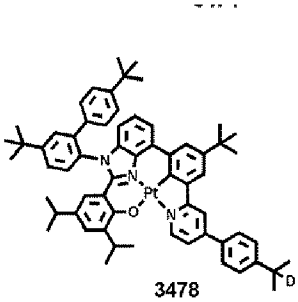
3478

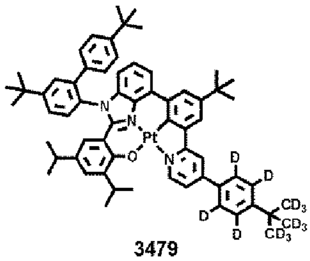
3479

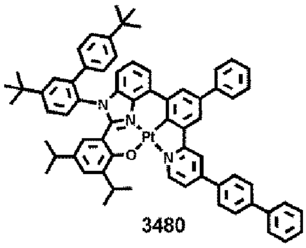
3480

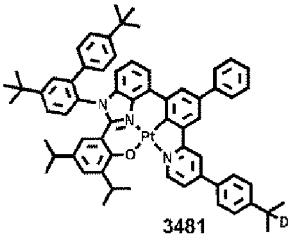
3481

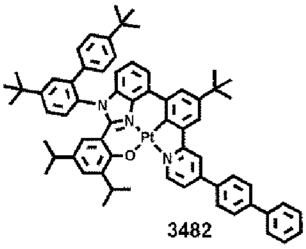
3482

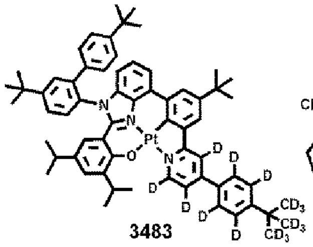
3483

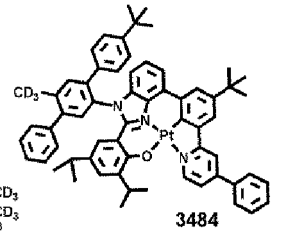
3484

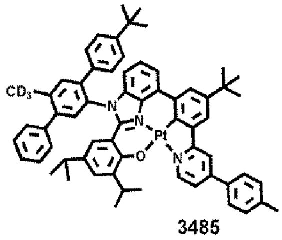
3485

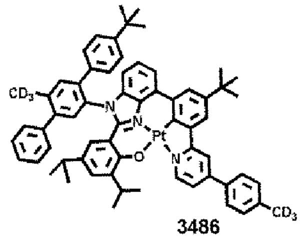
3486

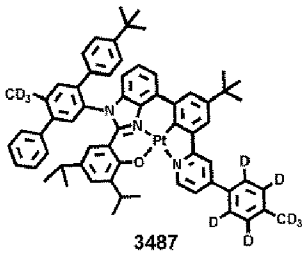
3487

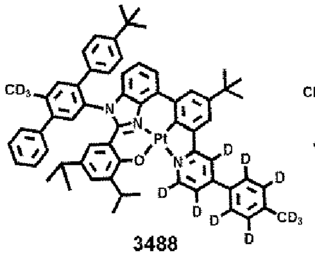
3488

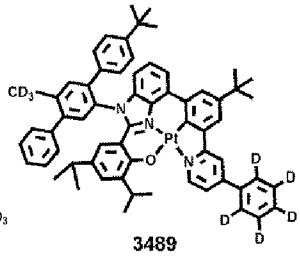
3489

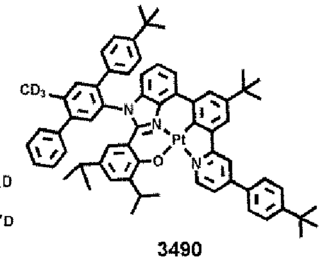
3490

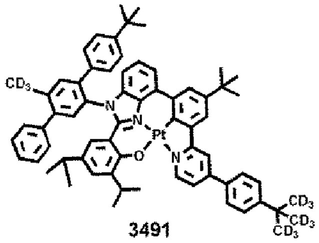
3491

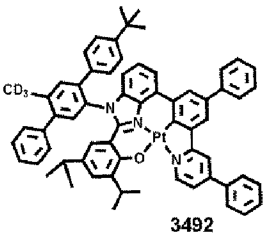
3492

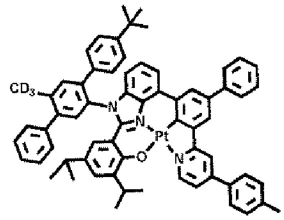
3493

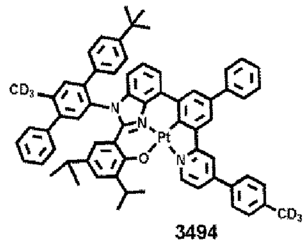
3494

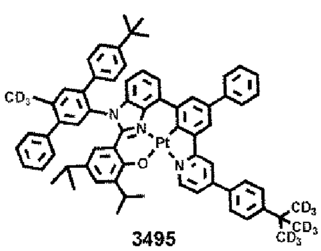
3495

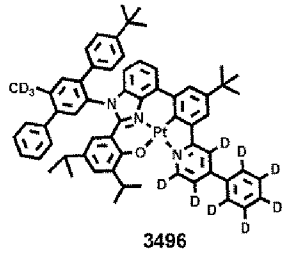
3496

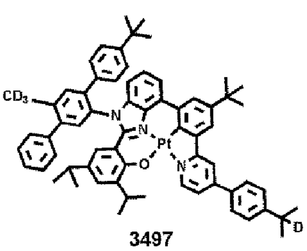
3497

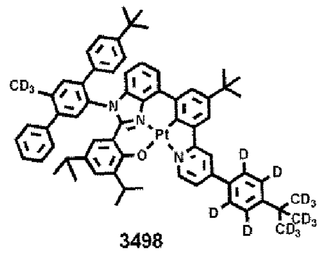
3498

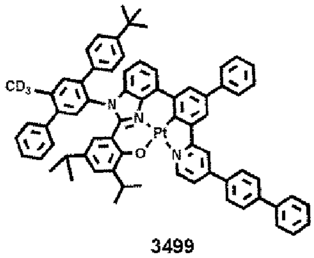
3499

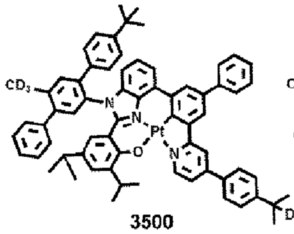
3500

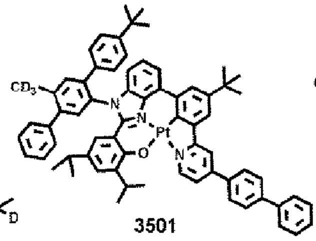
3501

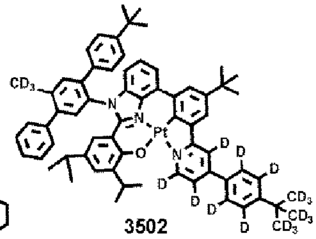
3502

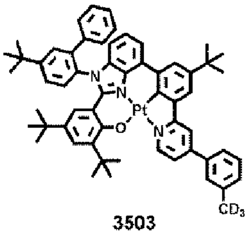
3503

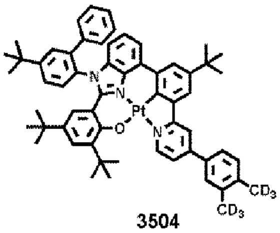
3504

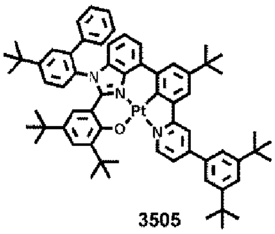
3505

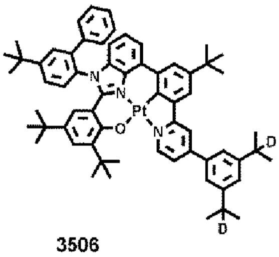
3506

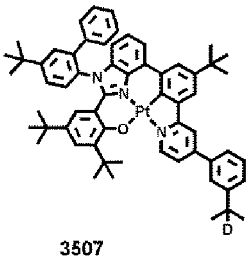
3507

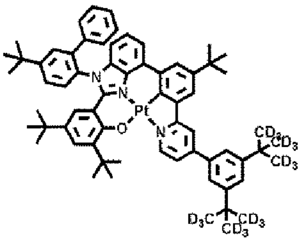
3508

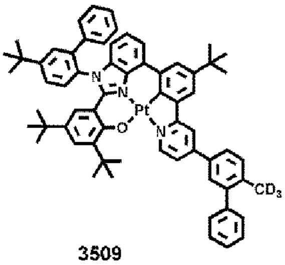
3509

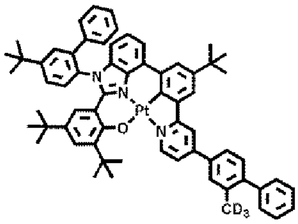
3510

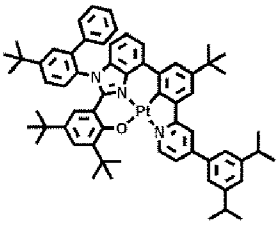
3511

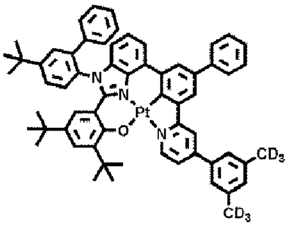
3512

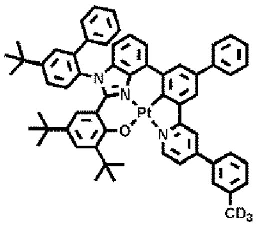
3513

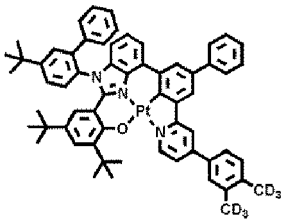
3514

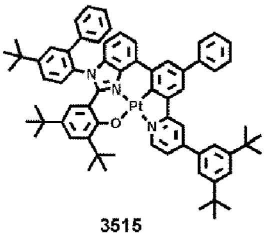
3515

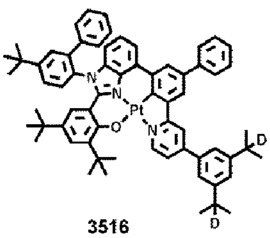
3516

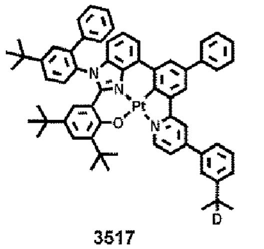
3517

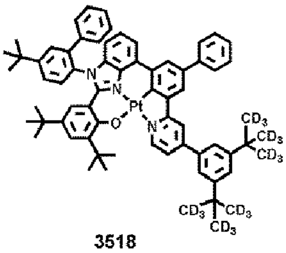
3518

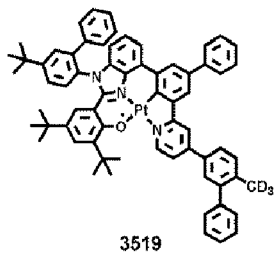
3519

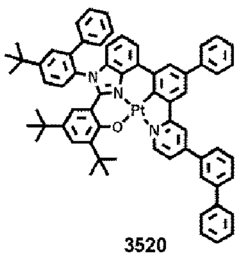
3520

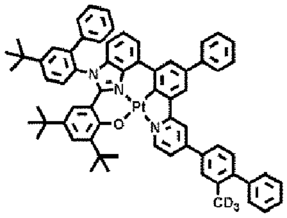
3521

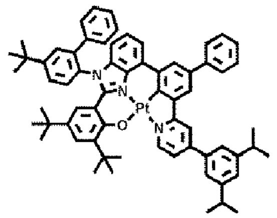
3522

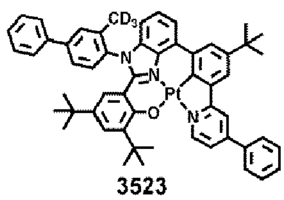
3523

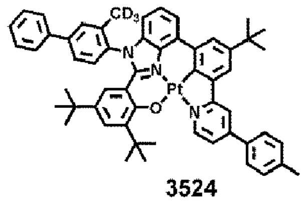
3524

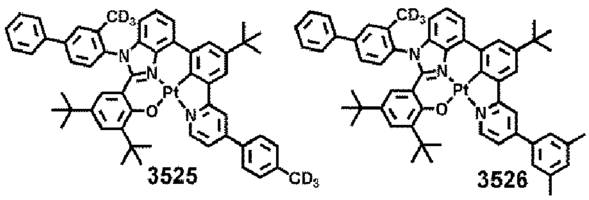
3525

3526

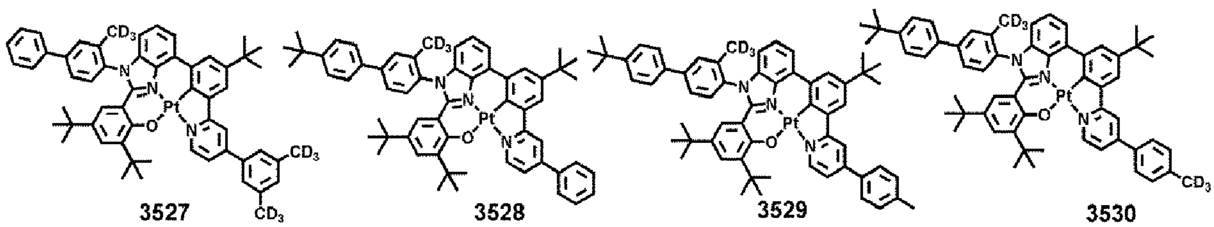
3527

3528

3529

3530

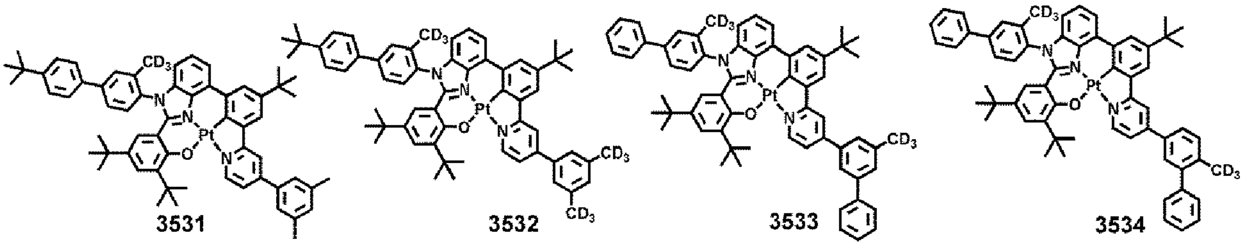
3531

3532

3533

3534

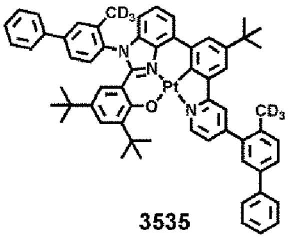
3535

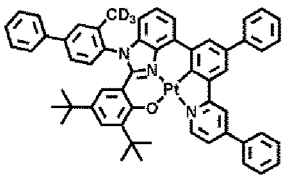
3536

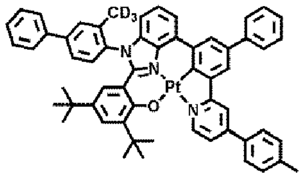
3537

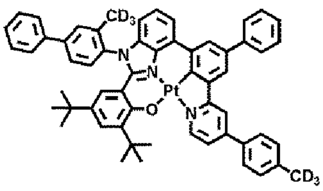
3538

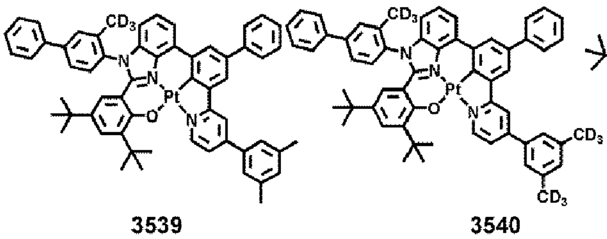
3539

3540

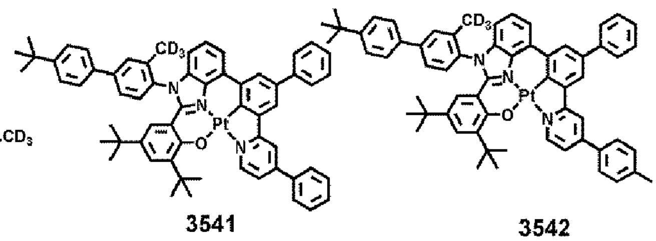
3541

3542

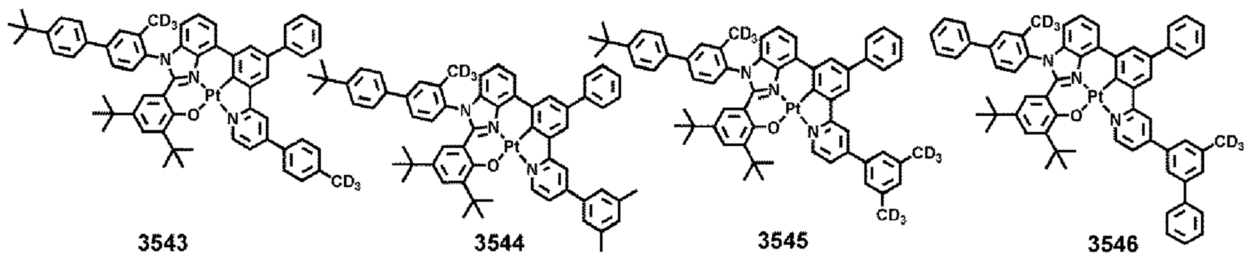

3543 3544 3545 3546

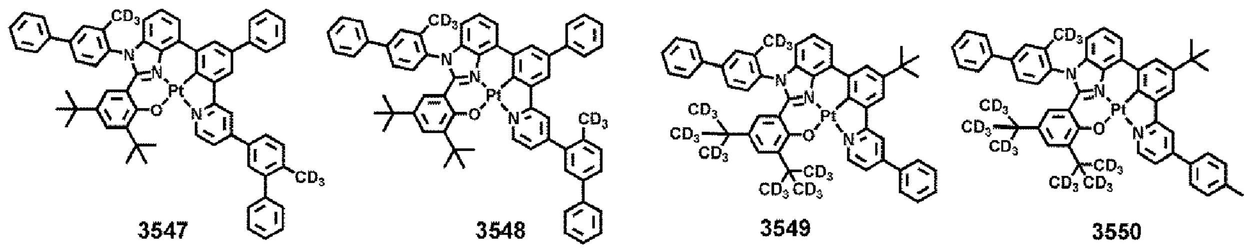

3547 3548 3549 3550

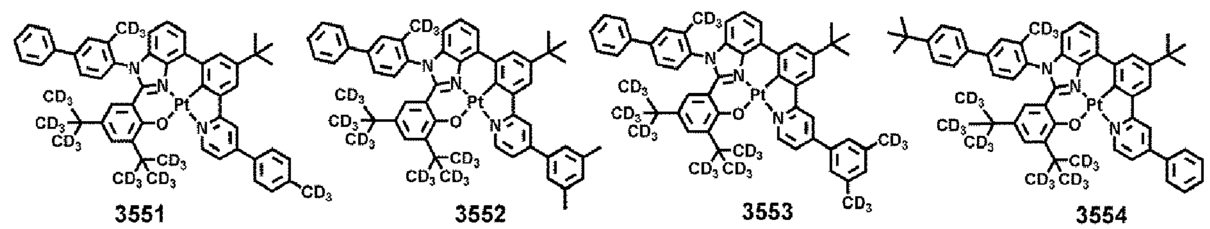

3551 3552 3553 3554

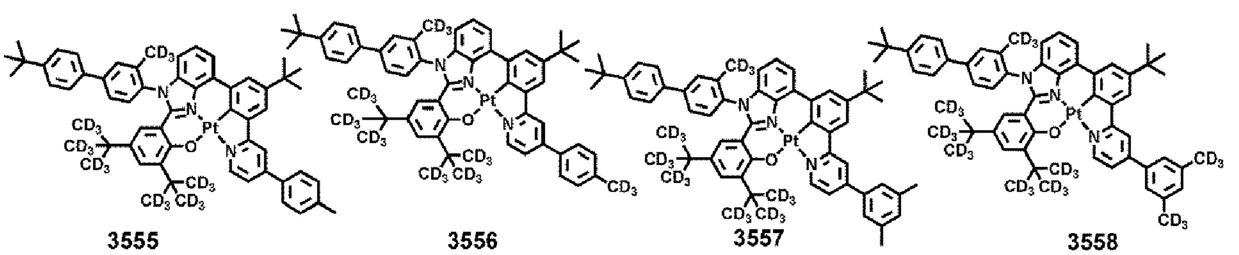

3555 3556 3557 3558

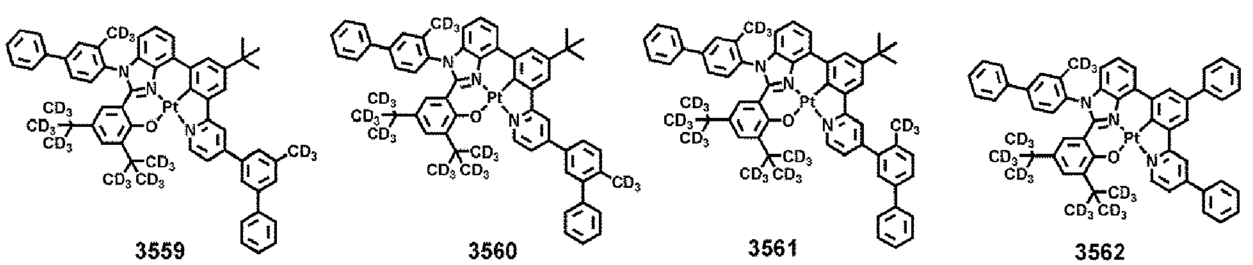

3559 3560 3561 3562

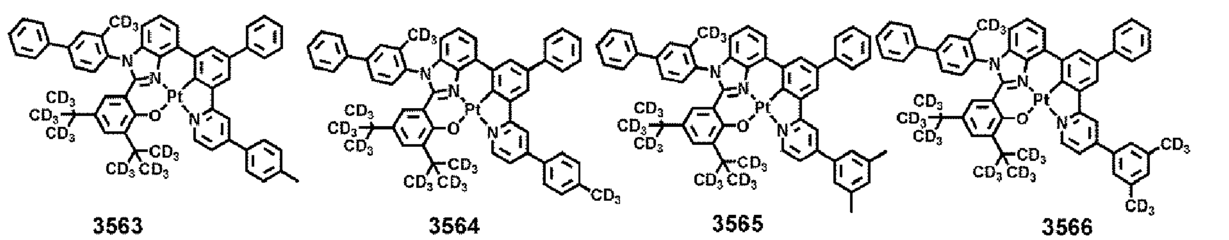

3563 3564 3565 3566

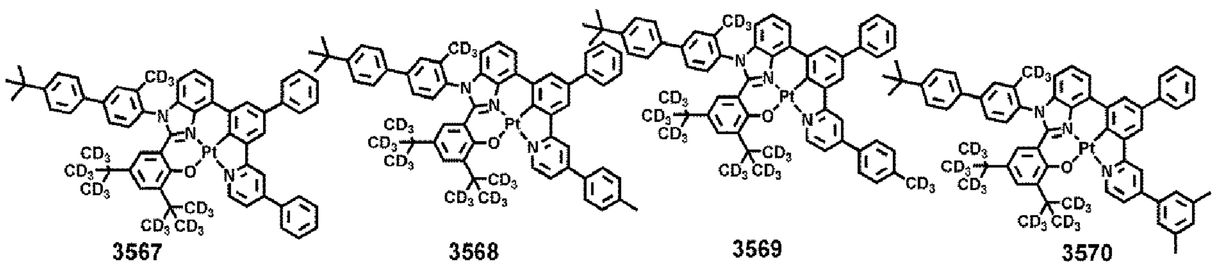

3567 3568 3569 3570

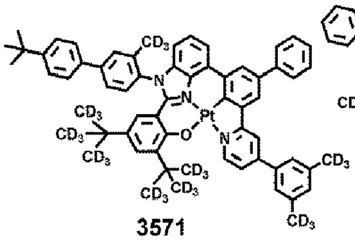

3571

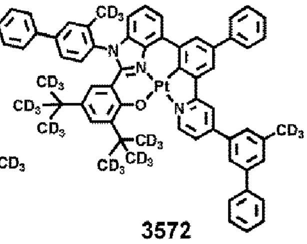

3572

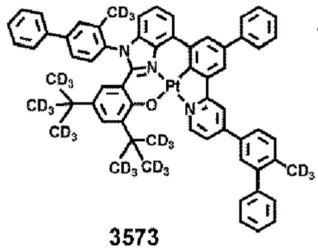

3573

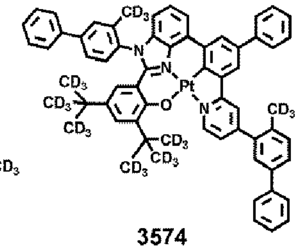

3574

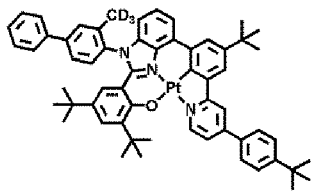

3575

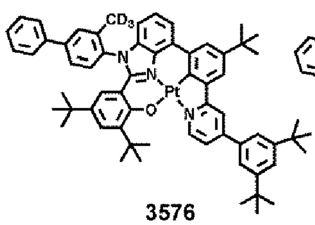

3576

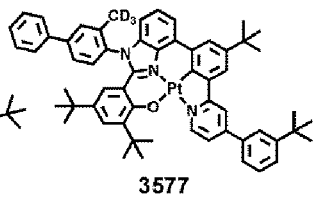

3577

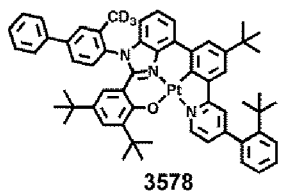

3578

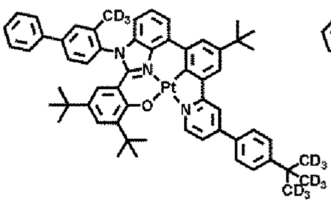

3579

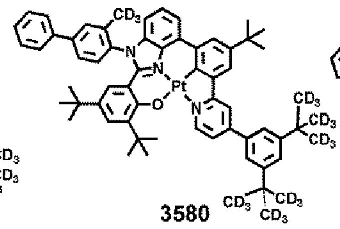

3580

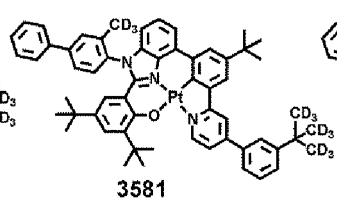

3581

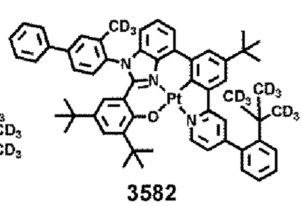

3582

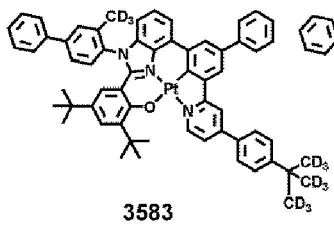

3583

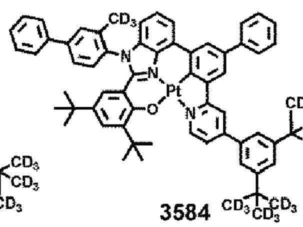

3584

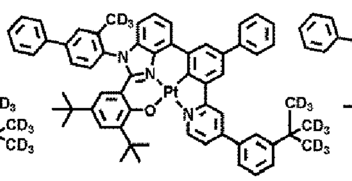

3585

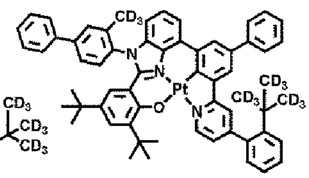

3586

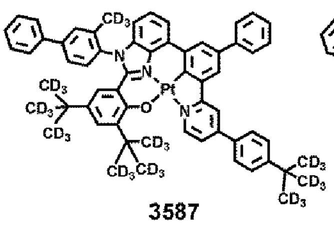

3587

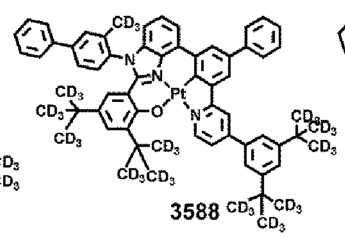

3588

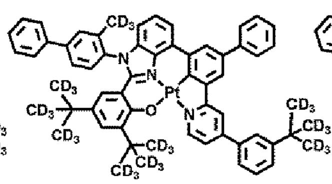

3589

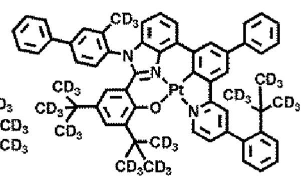

3590

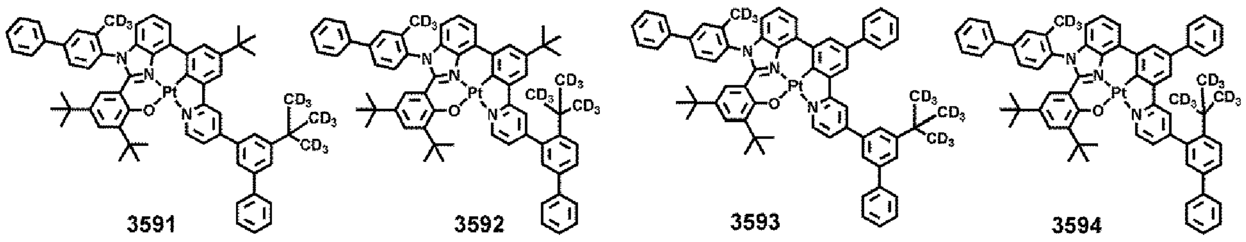

3591 3592 3593 3594

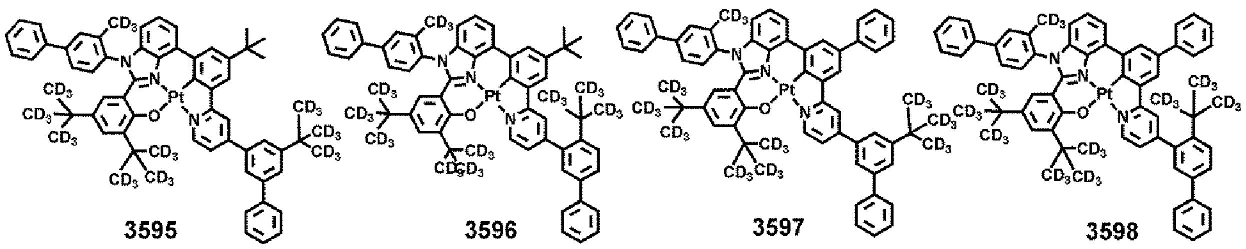

3595 3596 3597 3598

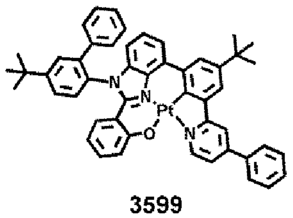

3599

.

In Formula 1, $A_{51}$ may be a $C_4$-$C_{60}$ alkyl group, $A_{52}$ may be deuterium or a deuterium-containing $C_1$-$C_{60}$ alkyl group unsubstituted or substituted with at least one $C_3$-$C_{10}$ cycloalkyl group, a51 (the number of $A_{51}$) and a52 (the number of $A_{52}$) may each independently be an integer from 0 to 10, provided that the sum of a51 and a52 may be 1 or more. That is, in Formula 1, ring $CY_{51}$ is essentially substituted with at least one selected from a group represented by $A_{51}$ and a group represented by $A_{52}$. Since ring $CY_{51}$ is essentially substituted with at least one electron donating group, an electronic device, for example, an organic light-emitting device, which includes the organometallic compound represented by Formula 1, may have improved luminescent efficiency and lifespan.

In some embodiments, $A_{51}$ in Formula 1 may be a group having the formula-$C(R_{511})(R_{512})(R_{513})$, wherein $R_{511}$, $R_{512}$, and $R_{513}$ are each independently a $C_1$-$C_{60}$ alkyl group, for example, a $C_1$-$C_{50}$ alkyl group, a $C_1$-$C_{40}$ alkyl group, a $C_1$-$C_{30}$ alkyl group, a $C_1$-$C_{20}$ alkyl group, a $C_1$-$C_{10}$ alkyl group, or a $C_1$-$C_5$ alkyl group. While not wishing to be bound by theory, it is understood that when $A_{51}$ has the formula-$C(R_{511})(R_{512})(R_{513})$, the organometallic compound represented by Formula 1, may have improved luminescent efficiency and lifespan compared to the compounds in which at least one of $R_{511}$, $R_{512}$, and $R_{513}$ is hydrogen.

In some embodiments, $A_{52}$ in Formula 1 may be a group having the formula-$CD(R_{521})(R_{522})$, wherein $R_{521}$ and $R_{522}$ are each independently a $C_1$-$C_{60}$ alkyl group, a $C_1$-$C_{60}$ alkyl group substituted with at least one deuterium, or a $C_3$-$C_{10}$ cycloalkyl group. In some other embodiments, $A_{52}$ in Formula 1 may be a group having the formula -$CD_2(R_{521})$, wherein $R_{521}$ is the same as defined above. In still some other embodiments, $A_{52}$ may be a fully deuterated group, such as $CD_3$, $C_2D_5$, $C_3D_7$, $C_4D_9$, but is not limited thereto. While not wishing to be bound by theory, it is understood that when $A_{52}$ has the formula -$CD(R_{521})(R_{522})$, the formula -$CD_2(R_{521})$, or wherein $A_{52}$ is a fully deuterated group, the organometallic compound represented by Formula 1, may have improved luminescent efficiency and lifespan compared to the compounds in which at least one of $R_{521}$ and $R_{522}$ is hydrogen.

$A_{52}$ may be a partially or fully deuterated $C_1$-$C_{60}$ alkyl group unsubstituted or substituted with at least one $C_3$-$C_{10}$ cycloalkyl group. When the degree of deuteration is 100%, the group $A_{52}$ is fully deuterated. When the degree of deuteration is lower than 100%, the group $A_{52}$ is partially deuterated. The degree of deuteration of the group $A_{52}$ may be calculated by using Equation 10:

Equation 10

$$\text{degree of deuteration (\%)} = n_{D2}/(n_{H2} + n_{D2}) \times 100.$$

In Equation 10, $n_{H2}$ represents the total number of hydrogens included in the group $A_{52}$, and $n_{D2}$ represents the total number of deuterium atoms included in the group $A_{52}$.

In an embodiment, the degree of deuteration of the group $A_{52}$ may be about 5% or more, 10% or more, 20% or more, 30% or more, 40% or more, 50% or more, 60% or more, 70% or more, about 90% or more, about 95% or more, about 96% or more, about 97% or more, about 98% or more, or about 99% or more, but embodiments of the present disclosure are not limited thereto.

In addition, a53 that is the number of groups represented by

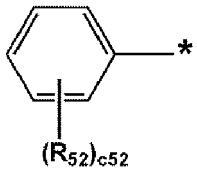

in Formula 1 may be an integer from 1 to 10. That is, since a53 in Formula 1 is not 0, ring $CY_{51}$ in Formula 1 is essentially substituted with at least one group represented by

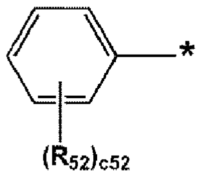

.

Therefore, due to the resonance effect caused by the group represented by

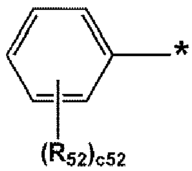

,

an electronic device, for example, an organic light-emitting device, which includes the organometallic compound represented by Formula 1, may have improved luminescent efficiency and lifespan. In addition, although not limited by a specific theory, a group represented by

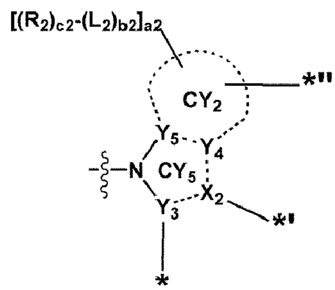

in Formula 1 is protected from electrons, heat, or the like by the group represented by

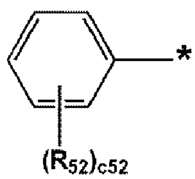

.

Therefore, an electronic device, for example, an organic light-emitting device, which includes the organometallic compound represented by Formula 1, may have improved luminescent efficiency and lifespan.

In Formula 1, the sum of a51 and a52 may be 1 or more and a53 may be an integer from 1 to 10. Thus, in some embodiments, the organometallic compound may simultaneously include

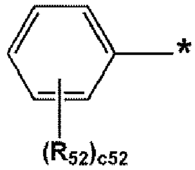

and $A_{51}$. In some other embodiments, the organometallic compound may simultaneously include

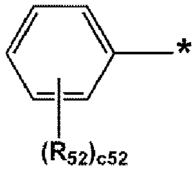

and $A_{52}$.

For example, highest occupied molecular orbital (HOMO), lowest unoccupied molecular orbital (LUMO), energy band gap, singlet ($S_1$), and triplet ($T_1$) energy levels of Compounds 1 to 8 were evaluated by using a DFT method of Gaussian program (structurally optimized at a level of B3LYP, 6-31G(d,p)). Evaluation results are shown in Table 1 below.

Table 1

| Compound No. | HOMO (eV) | LUMO (eV) | Energy band gap (eV) | $S_1$ (eV) | $T_1$ (eV) |
|---|---|---|---|---|---|
| 1 | -4.647 | -1.681 | 2.965 | 2.489 | 2.333 |
| 2 | -4.660 | -1.694 | 2.966 | 2.489 | 2.334 |
| 3 | -4.584 | -1.642 | 2.942 | 2.486 | 2.336 |
| 4 | -4.584 | -1.642 | 2.942 | 2.486 | 2.336 |
| 5 | -4.647 | -1.681 | 2.965 | 2.489 | 2.333 |
| 6 | -4.647 | -1.681 | 2.965 | 2.489 | 2.333 |
| 7 | -4.598 | -1.624 | 2.974 | 2.488 | 2.344 |
| 8 | -4.621 | -1.695 | 2.926 | 2.486 | 2.311 |

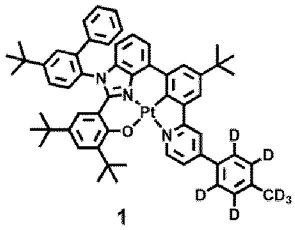
1

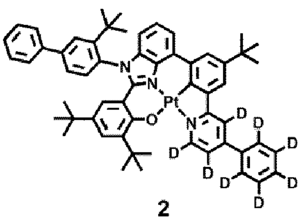
2

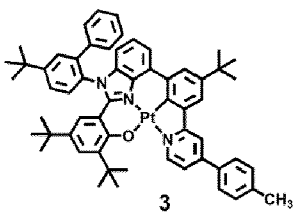
3

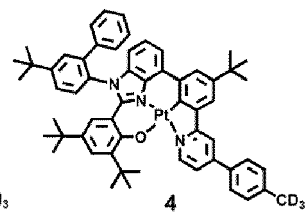
4

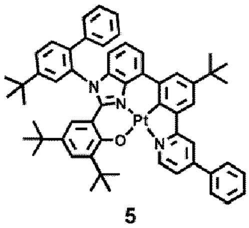
5

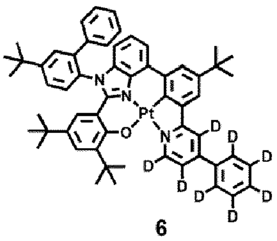
6

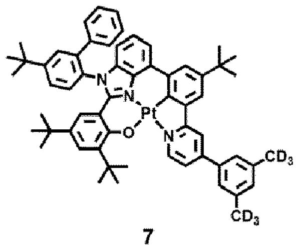
7

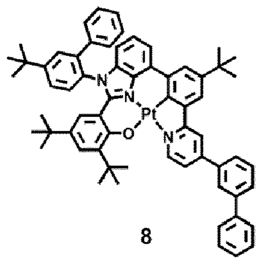
8

From Table 1, it is confirmed that the organometallic compound represented by Formula 1 has such electric characteristics that are suitable for use in an electronic device, for example, for use as a dopant for an organic light-emitting device.

Synthesis methods of the organometallic compound represented by Formula 1 may be recognizable by one of ordinary skill in the art by referring to Synthesis Examples provided below.

The organometallic compound represented by Formula 1 is suitable for use in an organic layer of an organic light-emitting device, for example, for use as a dopant in an emission layer of the organic layer. Thus, another aspect provides an organic light-emitting device that includes: a first electrode; a second electrode; and an organic layer that is disposed between the first electrode and the second electrode, wherein the organic layer includes an emission layer and at least one organometallic compound represented by Formula 1.

The organic light-emitting device may have, due to the inclusion of an organic layer including the organometallic compound represented by Formula 1, a low driving voltage, high quantum efficiency, a low roll-off ratio, and a long lifespan,.

The organometallic compound of Formula 1 may be used between a pair of electrodes of an organic light-emitting device. For example, the organometallic compound represented by Formula 1 may be included in the emission layer. In this embodiment, the organometallic compound may act as a dopant, and the emission layer may further include a host (that is, an amount of the organometallic compound represented by Formula 1 is smaller than an amount of the host).

In one or more embodiment, the emission layer may include a host and a dopant, and the dopant may include the organometallic compound represented by Formula 1. The organometallic compound represented by Formula 1 may be a red phosphorescent dopant.

The expression "(an organic layer) includes at least one of organometallic compounds" as used herein may include an embodiment in which "(an organic layer) includes identical organometallic compounds represented by Formula 1" and an embodiment in which "(an organic layer) includes two or more different organometallic compounds represented by Formula 1."

For example, the organic layer may include, as the organometallic compound, only Compound 1. In this embodiment, Compound 1 may be included in an emission layer of the organic light-emitting device. In one or more embodiments, the organic layer may include, as the organometallic compound, Compound 1 and Compound 2. In this embodiment, Compound 1 and Compound 2 may be included in an identical layer (for example, Compound 1 and Compound 2 may both be included in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In an embodiment, in the organic light-emitting device, the first electrode may be an anode, and the second electrode may be a cathode, and the organic layer may further include a hole transport region disposed between the first electrode and the emission layer and an electron transport region disposed between the emission layer and the second electrode, wherein the hole transport region may include a hole injection layer, a hole transport layer, an electron blocking layer, or any combination thereof, and the electron transport region may include a hole blocking layer, an electron transport layer, an electron injection layer, or any combination thereof.

The term "organic layer" as used herein refers to a single layer and/or a plurality of layers disposed between the first electrode and the second electrode of the organic light-emitting device. The "organic layer" may include, in addition to an organic compound, an organometallic complex including metal.

The FIGURE is a schematic view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate

may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may be a material(s) with a high work function to facilitate hole injection. The first electrode 11 may be a reflective electrode, a semi-transmissive electrode, or a transmissive electrode. The material for forming the first electrode may be, for example, indium tin oxide (ITO), indium zinc oxide (IZO), tin oxide ($SnO_2$), and zinc oxide (ZnO). In one or more embodiments, magnesium (Mg), aluminum (Al), aluminum-lithium (Al-Li), calcium (Ca), magnesium-indium (Mg-In), or magnesium-silver (Mg-Ag) may be used as the material for forming the first electrode.

The first electrode 11 may have a single-layered structure or a multi-layered structure including two or more layers. For example, the first electrode 11 may have a three-layered structure of ITO/Ag/ITO, but the structure of the first electrode 110 is not limited thereto.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

A hole injection layer may be formed on the first electrode 11 by using one or more suitable methods for example, vacuum deposition, spin coating, casting, Langmuir-Blodgett (LB) deposition, or any combination thereof.

When a hole injection layer is formed by vacuum deposition, the deposition conditions may vary according to a compound that is used to form the hole injection layer, and the structure and thermal characteristics of the hole injection layer. For example, the deposition conditions may include a deposition temperature of about 100 °C to about 500 °C, a vacuum pressure of about $10^{-8}$ torr to about $10^{-3}$ torr, and a deposition rate of about 0.01 Angstroms per second (Å/sec) to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 revolutions per minute (rpm) to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzene sulfonic acid (PANI/DBSA), poly(3,4-ethylenedioxythiophene)/poly(4-styrene sulfonate) (PEDOT/PSS), polyaniline/camphor sulfonic acid (PANI/CSA), polyaniline/poly(4-styrene sulfonate) (PANI/PSS), a compound represented by Formula 201, a compound represented by Formula 202, or any combination thereof:

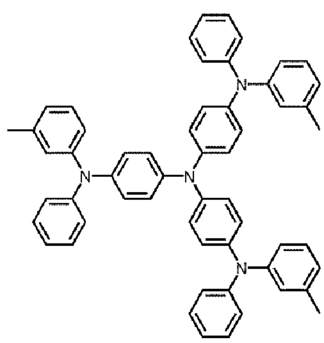

m-MTDATA

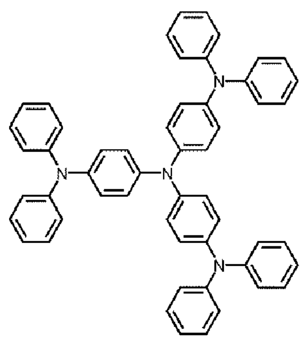

TDATA

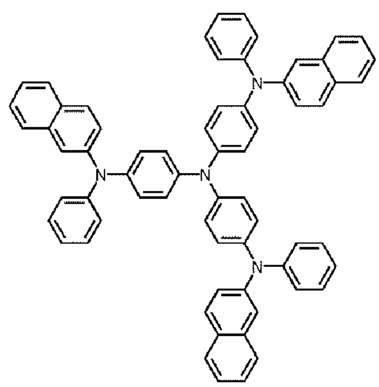

2-TNATA

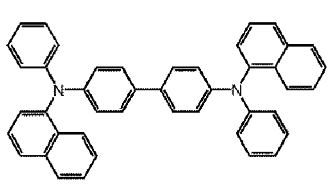

NPB

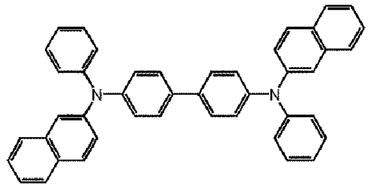

β-NPB

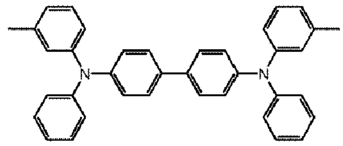

TPD

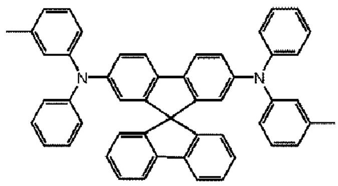

Spiro-TPD

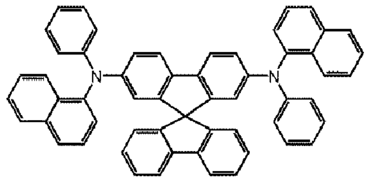

Spiro-NPB

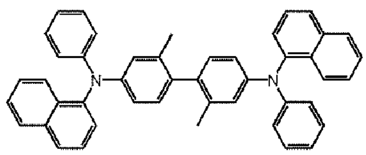

methylated NPB

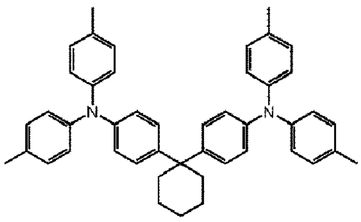

TAPC

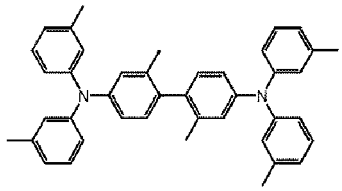

HMTPD

Formula 201

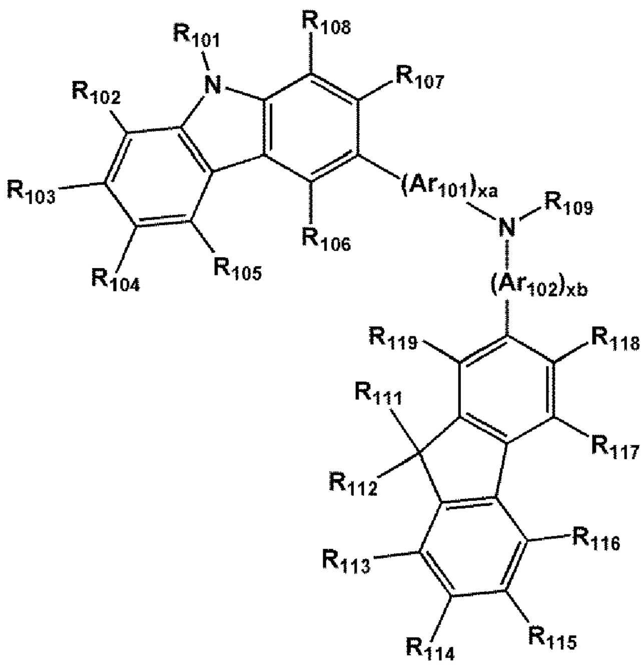

Formula 202

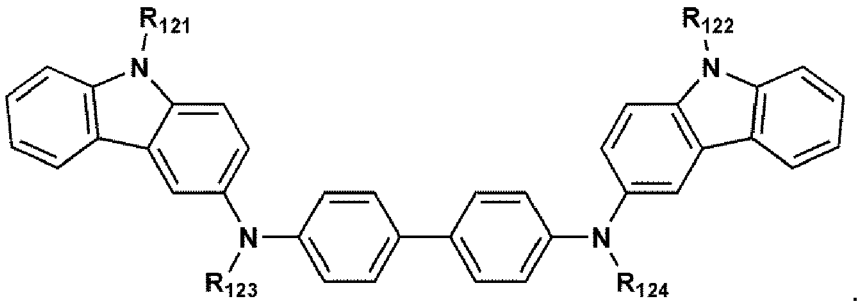

In Formula 201, $Ar_{101}$ and $Ar_{102}$ may each independently be a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, a $C_1$-$C_{60}$ alkoxy group, a $C_3$-$C_{10}$ cycloalkyl group, a $C_3$-$C_{10}$ cycloalkenyl group, a $C_1$-$C_{10}$ heterocycloalkyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, or any combination thereof.

In Formula 201, xa and xb may each independently be an integer from 0 to 5, or may be 0, 1, or 2. For example, xa is 1 and xb is 0, but xa and xb are not limited thereto.

In Formulae 201 and 202, $R_{101}$ to $R_{108}$, $R_{111}$ to $R_{119}$, and $R_{121}$ to $R_{124}$ may each independently be:

hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group (for example, a methyl group, an ethyl group, a propyl group, a butyl group, a pentyl group, a hexyl group, and so on), or a $C_1$-$C_{10}$ alkoxy group (for example, a methoxy group, an ethoxy group, a propoxy group, a butoxy group, a pentoxy group, and so on);

a $C_1$-$C_{10}$ alkyl group or a $C_1$-$C_{10}$ alkoxy group, each substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, or any combination thereof; or

a phenyl group, a naphthyl group, an anthracenyl group, a fluorenyl group, or a pyrenyl group, each unubstituted or substituted with deuterium,-F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{10}$ alkyl group, a $C_1$-$C_{10}$ alkoxy group or any combination thereof,

but embodiments of the present disclosure are not limited thereto.

In Formula 201, $R_{109}$ may be a phenyl group, a naphthyl group, an anthracenyl group, or a pyridinyl group, each unsubstituted or substituted with deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a $C_1$-$C_{20}$ alkyl group, a $C_1$-$C_{20}$ alkoxy group, a phenyl group, a naphthyl group, an anthracenyl group, a pyridinyl group, or any combination thereof.

In an embodiment, the compound represented by Formula 201 may be represented by Formula 201A, but embodiments of the present disclosure are not limited thereto:

Formula 201A

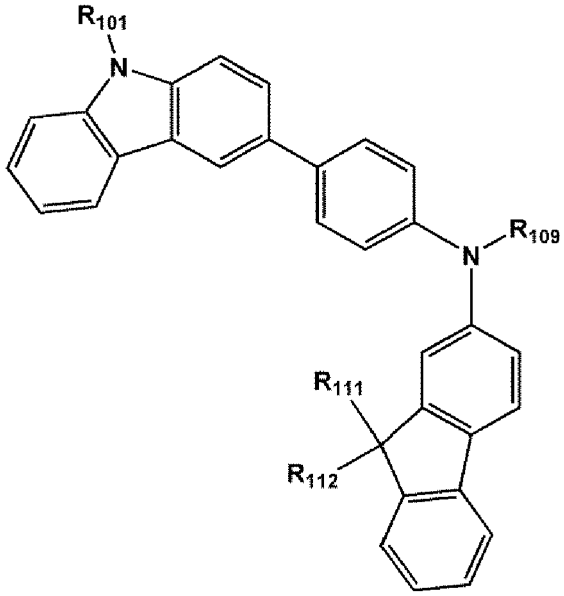

In Formula 201A, $R_{101}$, $R_{111}$, $R_{112}$, and $R_{109}$ are each independently the same as described above.

For example, the compound represented by Formula 201 and the compound represented by Formula 202 may each independently include Compounds HT1 to HT20, but are not limited thereto:

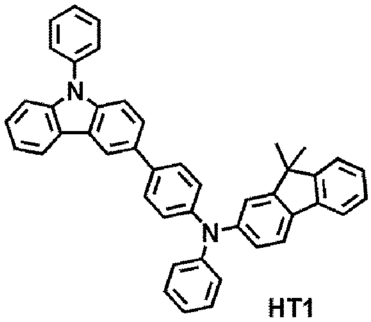

HT1

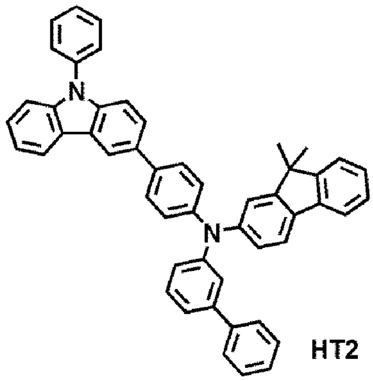

HT2

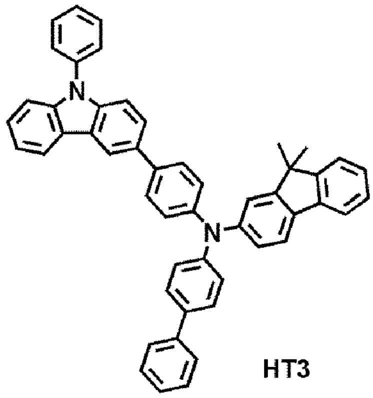

HT3

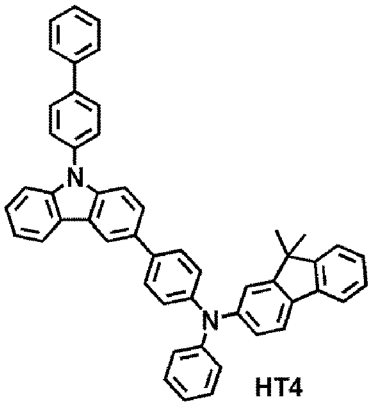

HT4

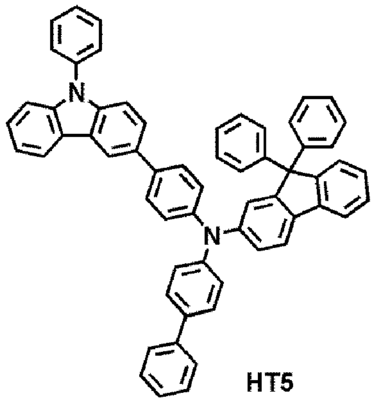

HT5

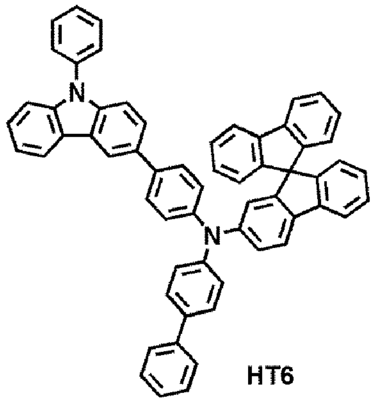

HT6

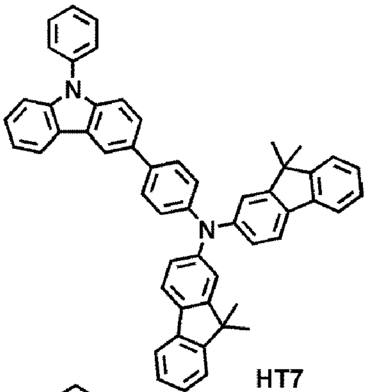

HT7

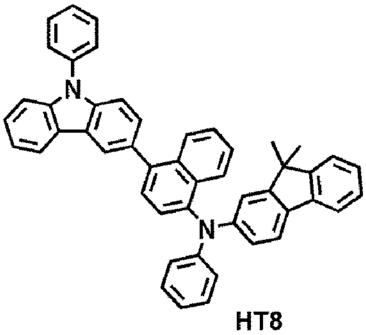

HT8

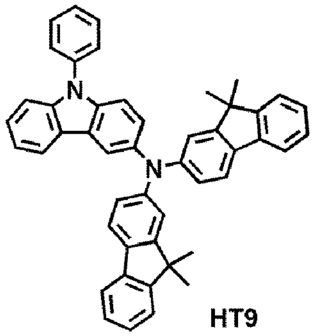

HT9

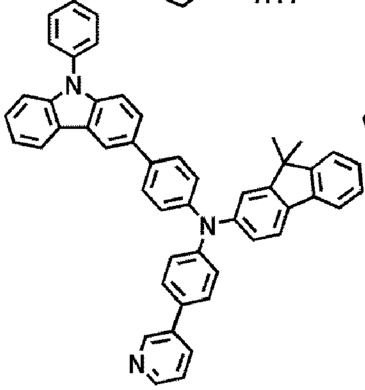

HT10

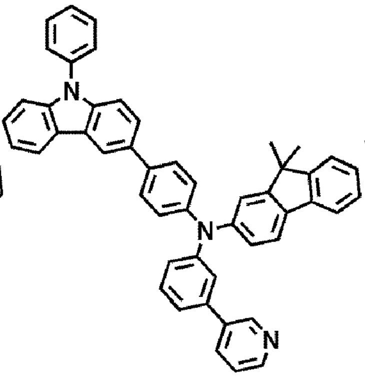

HT11

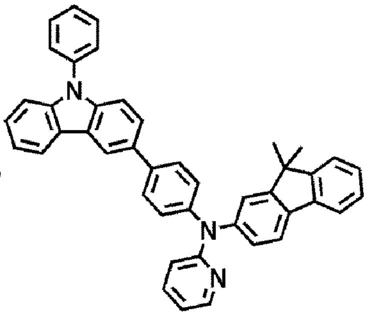

HT12

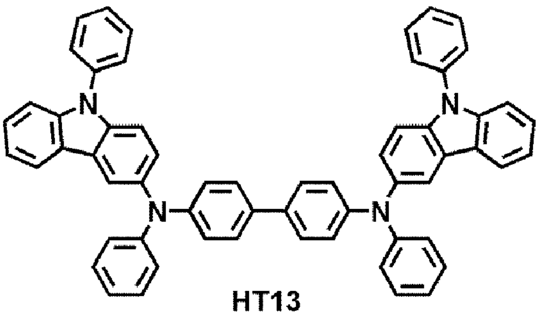

HT13

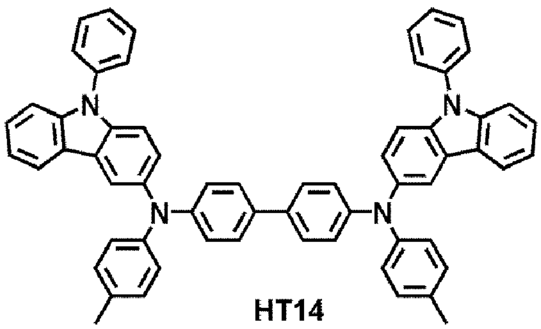

HT14

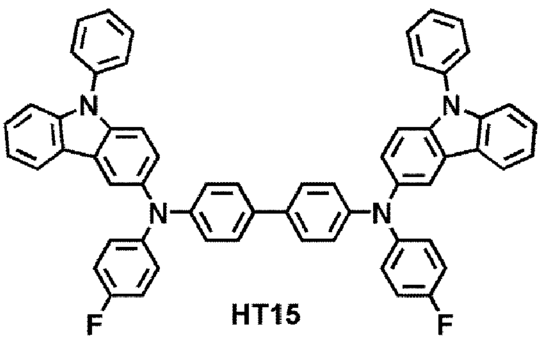

HT15

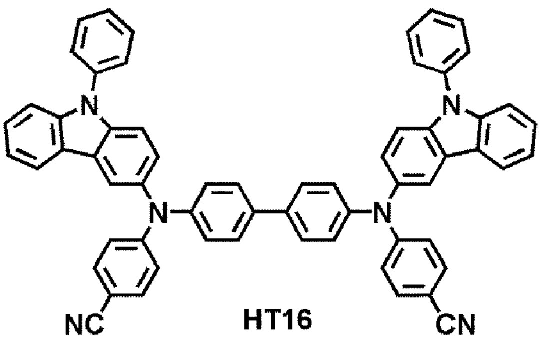

HT16

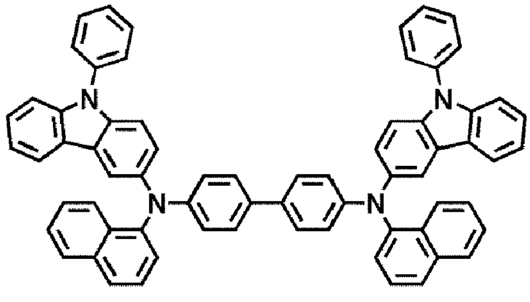

HT17

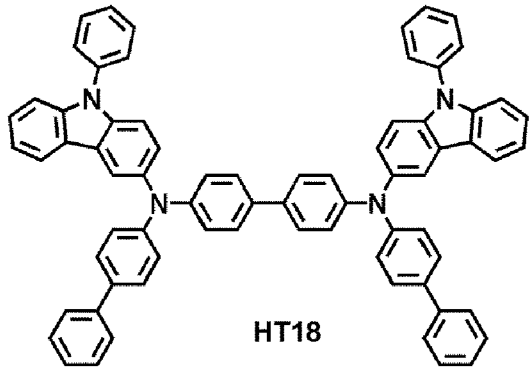

HT18

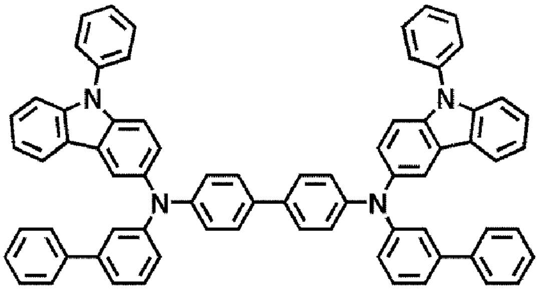

HT19

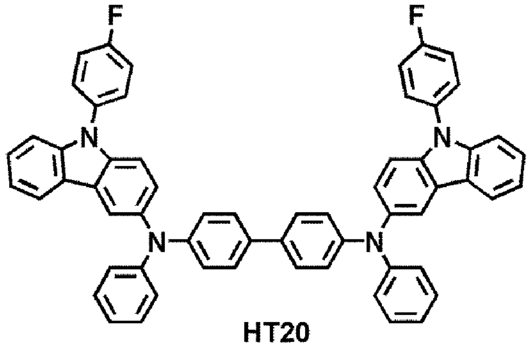

HT20

A thickness of the hole transport region may be in a range of about 100 Angstroms (Å) to about 10,000 Å, for example, about 100 Å to about 1,000 Å. When the hole transport region includes a hole injection layer, a hole transport layer, or any combination thereof, the thickness of the hole injection layer may be in a range of about 100 Å to about 10,000 Å, and for example, about 100 Å to about 1,000 Å, and the thickness of the hole transport layer may be in a range of about 50 Å to about 2,000 Å, and for example, about 100 Å to about 1,500 Å. While not wishing to be bound by theory, it is understood that when the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be a quinone derivative, a metal oxide, a cyano group-containing compound, or any combination thereof, but embodiments of the present disclosure are not limited thereto. Non-limiting examples of the p-dopant are a quinone derivative, such as tetracyanoquinonedimethane (TCNQ) or 2,3,5,6-tetrafluorotetracyano-1,4-benzoquinonedimethane (F4-TCNQ); a metal oxide, such as a tungsten oxide or a molybdenium oxide; and a cyano group-containing compound, such as Compound HT-D1, but are not limited thereto.

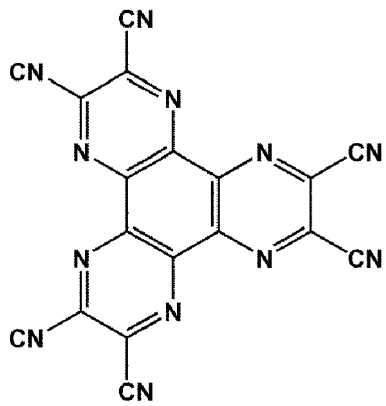

HT-D1

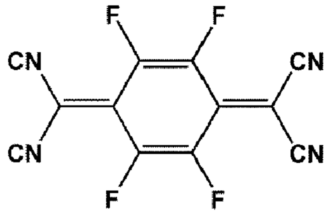

F4-TCNQ .

The hole transport region may include a buffer layer.

Also, the buffer layer may compensate for an optical resonance distance according to a wavelength of light emitted from the emission layer, and thus, efficiency of a formed organic light-emitting device may be improved.

Meanwhile, when the hole transport region includes an electron blocking layer, a material for the electron blocking layer may be a material(s) for the hole transport region described above and materials for a host to be explained later. However, the material for the electron blocking layer is not limited thereto. For example, when the hole transport region includes an electron blocking layer, a material for the electron blocking layer may be mCP, which will be explained later.

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

The emission layer may include a host and a dopant, and the dopant may include the organometallic compound represented by Formula 1.

The host may include TPBi, TBADN, ADN (also referred to as "DNA"), CBP, CDBP, TCP, mCP, Compound H50, Compound H51, or any combination thereof:

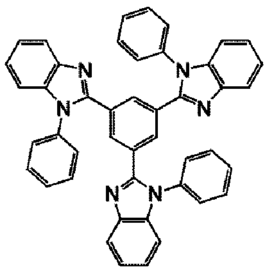

TPBi

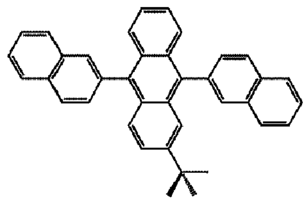

TBADN

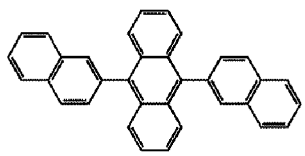

ADN

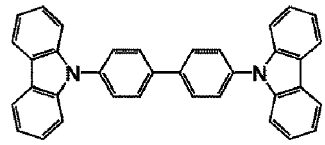

CBP

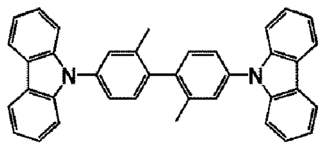

CDBP

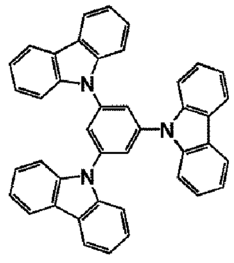

TCP

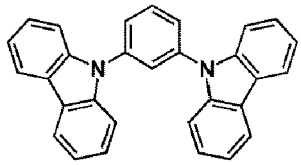

mCP

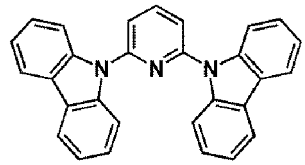

H50

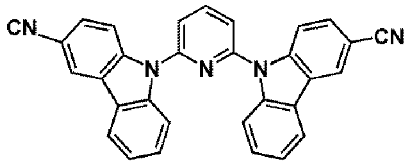

H51

.

When the organic light-emitting device is a full-color organic light-emitting device, the emission layer may be patterned into a red emission layer, a green emission layer, and/or a blue emission layer. In one or more embodiments, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host, but embodiments of the present disclosure are not limited thereto.

The dopant may include the organometallic compound represented by Formula 1. For example, the dopant may be a red phosphorescent dopant.

A thickness of the emission layer may be in a range of about 100 Å to about 1,000 Å, for example, about 200 Å to about 600 Å. While not wishing to be bound by theory, it is understood that when the thickness of the emission layer is within this range, excellent light-emission characteristics may be obtained without a substantial increase in driving voltage.

Then, an electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, BCP, Bphen, BAlq, or any combination thereof, but embodiments of the present disclosure are not limited thereto:

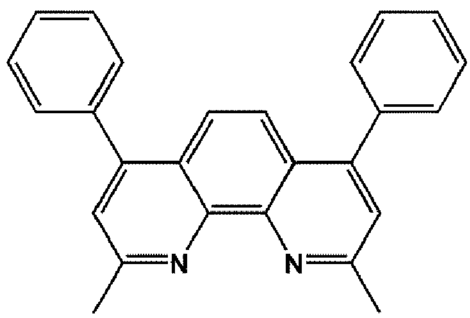

BCP

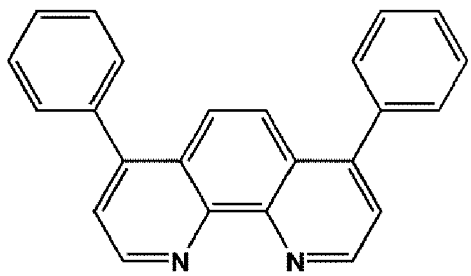

Bphen

.

A thickness of the hole blocking layer may be in a range of about 20 Å to about 1,000 Å, for example, about

30 Å to about 300 Å. While not wishing to be bound by theory, it is understood that when the thickness of the hole blocking layer is within these ranges, the hole blocking layer may have improved hole blocking ability without a substantial increase in driving voltage.

The electron transport layer may include BCP, Bphen, $Alq_3$, BAlq, TAZ, NTAZ, or any combination thereof:

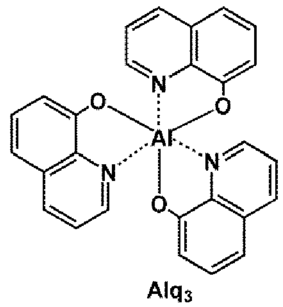

$Alq_3$

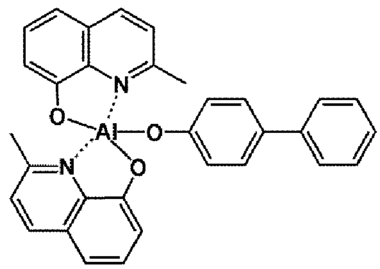

BAlq

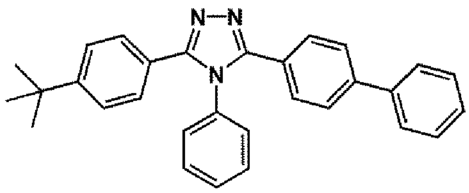

TAZ

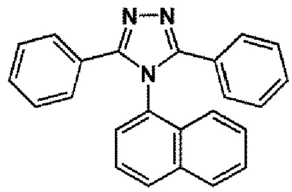

NTAZ .

In one or more embodiments, the electron transport layer may include at least one of ET1 to ET25, but are not limited thereto:

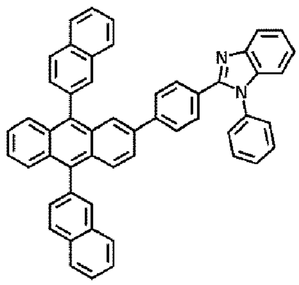

ET1

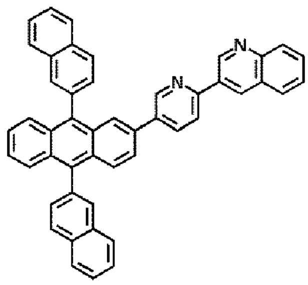

ET2

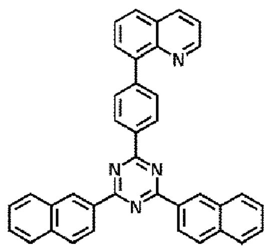

ET3

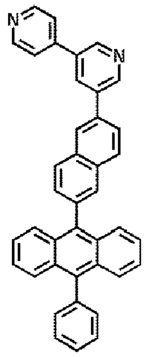

ET4

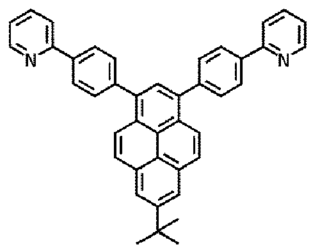

ET5

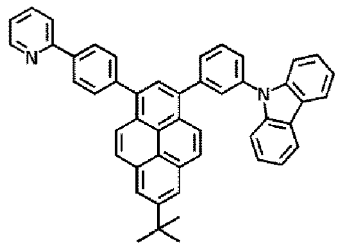

ET6

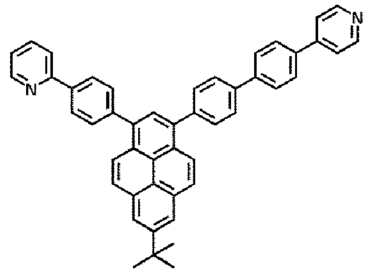

**ET7**

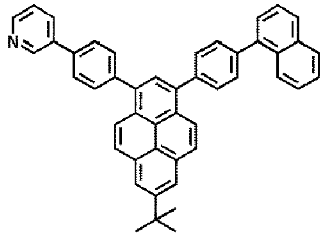

**ET8**

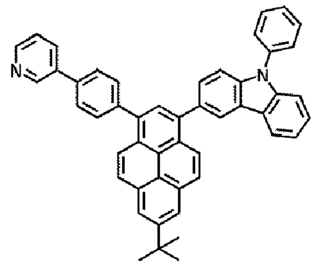

**ET9**

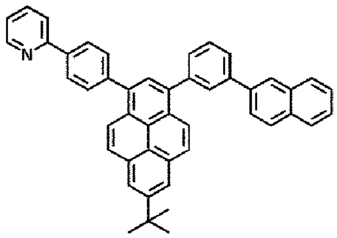

**ET10**

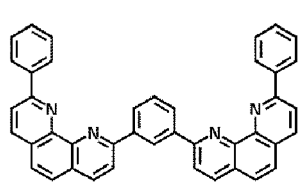

**ET11**

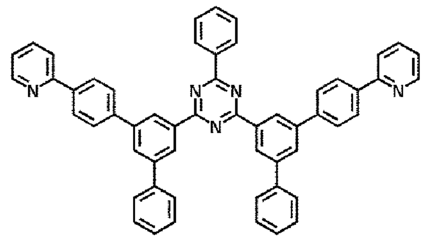

**ET12**

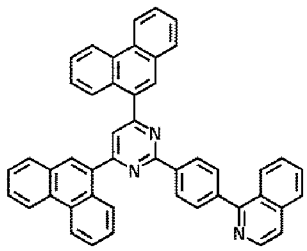

**ET13**

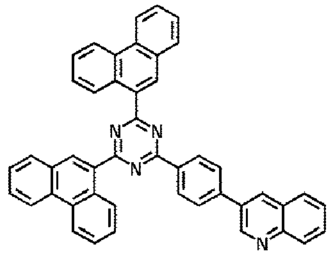

**ET14**

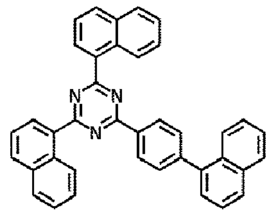

**ET15**

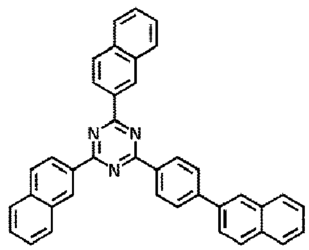

**ET16**

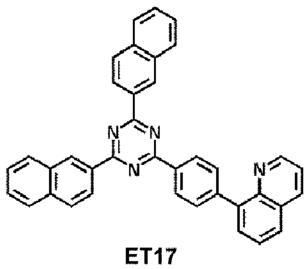

**ET17**

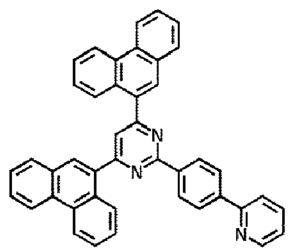

**ET18**

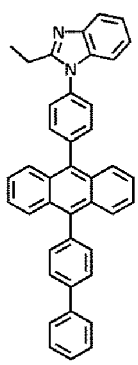

**ET19**

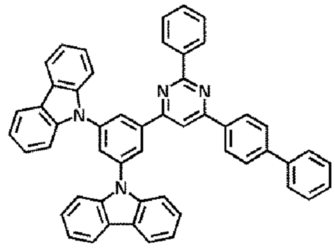

**ET20**

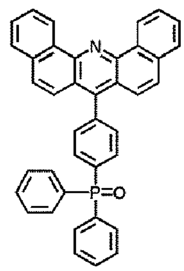

**ET21**

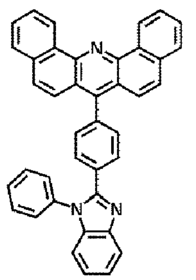
ET22

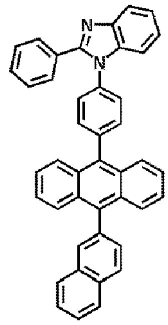
ET23

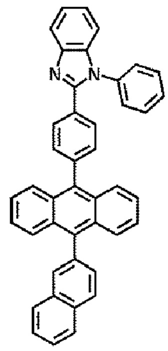
ET24

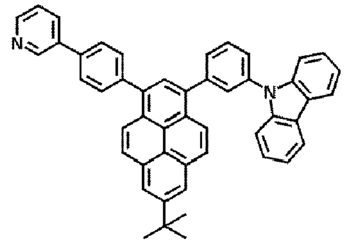
ET25

.

A thickness of the electron transport layer may be in a range of about 100 Å to about 1,000 Å, for example, about 150 Å to about 500 Å. While not wishing to be bound by theory, it is understood that when the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium 8-hydroxyquinolate, LiQ) or ET-D2:

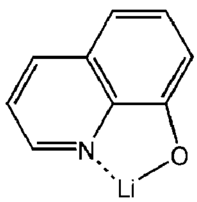
ET-D1

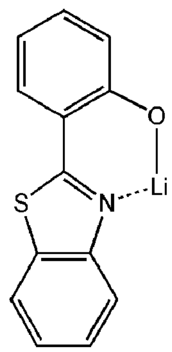
ET-D2

.

The electron transport region may include an electron injection layer that promotes flow of electrons from the second electrode 19 thereinto.

The electron injection layer may include LiF, NaCl, CsF, $Li_2O$, BaO, or any combination thereof.

A thickness of the electron injection layer may be in a range of about 1 Å to about 100 Å, for example, about 3 Å to about 90 Å. While not wishing to be bound by theory, it is understood that when the thickness of the electron injection layer is within the range described above, the electron injection layer may have satisfactory electron injection characteristics without a substantial increase in driving voltage.

The second electrode 19 is disposed on the organic layer 15. The second electrode 19 may be a cathode. A material for forming the second electrode 19 may be metal, an alloy, an electrically conductive compound, or a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be used as a material for forming the second electrode 19. In one or more embodiments, to manufacture a top-emission type light-emitting device, a transmissive electrode formed using ITO or IZO may be used as the second electrode 19.

Hereinbefore, the organic light-emitting device has been described with reference to the FIGURE, but embodiments of the present disclosure are not limited thereto.

Another aspect of the present disclosure provides a diagnostic composition including at least one organometallic compound represented by Formula 1.

The organometallic compound represented by Formula 1 provides high luminescent efficiency. Accordingly, a diagnostic composition including the organometallic compound may have high diagnostic efficiency.

The diagnostic composition may be used in various applications including a diagnosis kit, a diagnosis reagent, a biosensor, and a biomarker.

The term "$C_1$-$C_{60}$ alkyl group" as used herein refers to a linear or branched saturated aliphatic hydrocarbon monovalent group having 1 to 60 carbon atoms, and non-limiting examples thereof include a methyl group, an ethyl group, a propyl group, an iso-butyl group, a sec-butyl group, a tert-butyl group, a pentyl group, an iso-amyl group, and a hexyl group. The term "$C_1$-$C_{60}$ alkylene group" as used herein refers to a divalent group having the same structure as the $C_1$-$C_{60}$ alkyl group.

The term "$C_1$-$C_{60}$ alkoxy group" as used herein refers to a monovalent group represented by -$OA_{101}$ (wherein $A_{101}$ is the $C_1$-$C_{60}$ alkyl group), and non-limiting examples thereof include a methoxy group, an ethoxy group, and an iso-propyloxy group.

The term "$C_2$-$C_{60}$ alkenyl group" as used herein refers to a hydrocarbon group formed by including at least one carbon-carbon double bond in the middle or at the terminus of the $C_2$-$C_{60}$ alkyl group, and examples thereof include an ethenyl group, a propenyl group, and a butenyl group. The term "$C_2$-$C_{60}$ alkenylene group" as used herein refers to a divalent group having the same structure as the $C_2$-$C_{60}$ alkenyl group.

The term "$C_2$-$C_{60}$ alkynyl group" as used herein refers to a hydrocarbon group formed by including at least one carbon-carbon triple bond in the middle or at the terminus of the $C_2$-$C_{60}$ alkyl group, and examples thereof include an ethynyl group, and a propynyl group. The term "$C_2$-$C_{60}$ alkynylene group" as used herein refers to a divalent group having the same structure as the $C_2$-$C_{60}$ alkynyl group.

The term "$C_3$-$C_{10}$ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "$C_3$-$C_{10}$ cycloalkylene group" as used herein refers to a divalent group having the same structure as the $C_3$-$C_{10}$ cycloalkyl group.

The term "$C_1$-$C_{10}$ heterocycloalkyl group" as used herein refers to a monovalent saturated monocyclic group having at least one heteroatom selected from N, O, P, Si, and S as a ring-forming atom and 1 to 10 carbon atoms, and non-limiting examples thereof include a tetrahydrofuranyl group, and a tetrahydrothiophenyl group. The term "$C_1$-$C_{10}$ heterocycloalkylene group" as used herein refers to a divalent group having the same structure as the $C_1$-$C_{10}$ heterocycloalkyl group.

The term "$C_3$-$C_{10}$ cycloalkenyl group" as used herein refers to a monovalent monocyclic group that has 3 to 10 carbon atoms and at least one carbon-carbon double bond in the ring thereof and that has no aromaticity, and non-limiting examples thereof include a cyclopentenyl group, a cyclohexenyl group, and a cycloheptenyl group. The term "$C_3$-$C_{10}$ cycloalkenylene group" as used herein refers to a divalent group having the same structure as the $C_3$-$C_{10}$ cycloalkenyl group.

The term "$C_1$-$C_{10}$ heterocycloalkenyl group" as used herein refers to a monovalent monocyclic group that has at least one heteroatom selected from N, O, P, Si, and S as a ring-forming atom, 1 to 10 carbon atoms, and at least one carbon-carbon double bond in its ring. Examples of the $C_1$-$C_{10}$ heterocycloalkenyl group are a 2,3-dihydrofuranyl group, and a 2,3-dihydrothiophenyl group. The term "$C_1$-$C_{10}$ heterocycloalkenylene group" as used herein refers to a divalent group having the same structure as the $C_1$-$C_{10}$ heterocycloalkenyl group.

The term "$C_6$-$C_{60}$ aryl group" as used herein refers to a monovalent group having a carbocyclic aromatic system having 6 to 60 carbon atoms, and the term "$C_6$-$C_{60}$ arylene group" as used herein refers to a divalent group having a carbocyclic aromatic system having 6 to 60 carbon atoms. Non-limiting examples of the $C_6$-$C_{60}$ aryl group include a phenyl group, a naphthyl group, an anthracenyl group, a phenanthrenyl group, a pyrenyl group, and a chrysenyl group. When the $C_6$-$C_{60}$ aryl group and the $C_6$-$C_{60}$ arylene group each include two or more rings, the rings may be fused to each other.

The term "$C_7$-$C_{60}$ alkylaryl group" as used herein refers to a $C_1$-$C_{60}$ aryl group substituted with at least one $C_6$-$C_{60}$ alkyl group.

The term "$C_1$-$C_{60}$ heteroaryl group" as used herein refers to a monovalent group having an aromatic system that has at least one heteroatom selected from N, O, P, Si, and S as a ring-forming atom, and 1 to 60 carbon atoms. The term "$C_1$-$C_{60}$ heteroarylene group" as used herein refers to a divalent group having an aromatic system that has at least one heteroatom selected from N, O, P, and S as a ring-forming atom, and 1 to 60 carbon atoms. Non-limiting examples of the $C_1$-$C_{60}$ heteroaryl group include a pyridinyl group, a pyrimidinyl group, a pyrazinyl group, a pyridazinyl group, a triazinyl group, a quinolinyl group, and an isoquinolinyl group. When the $C_1$-$C_{60}$ heteroaryl group and the $C_1$-$C_{60}$ heteroarylene group each include two or more rings, the rings may be fused to each other.

The term "$C_2$-$C_{60}$ alkylheteroaryl group" as used herein refers to a $C_1$-$C_{60}$ heteroaryl group substituted with at least one $C_1$-$C_{60}$ alkyl group.

The term "$C_6$-$C_{60}$ aryloxy group" as used herein indicates -$OA_{102}$ (wherein $A_{102}$ is the $C_6$-$C_{60}$ aryl group), a $C_6$-$C_{60}$ arylthio group as used herein indicates -$SA_{103}$ (wherein $A_{103}$ is the $C_6$-$C_{60}$ aryl group), and the term "$C_7$-$C_{60}$ arylalkyl group" as used herein indicates -$A_{104}A_{105}$ (wherein $A_{105}$ is the $C_6$-$C_{59}$ aryl group and $A_{104}$ is the $C_1$-C53 alkylene group).

The term "$C_1$-$C_{60}$ heteroaryloxy group" as used herein refers to -$OA_{106}$ (wherein $A_{106}$ is the $C_2$-$C_{60}$ heteroaryl group), the term "$C_1$-$C_{60}$ heteroarylthio group" as used herein indicates -$SA_{107}$ (wherein $A_{107}$ is the $C_1$-$C_{60}$ heteroaryl

group), and the term "$C_2$-$C_{60}$ heteroarylalkyl group" as used herein refers to -$A_{108}A_{109}$ ($A_{109}$ is a $C_1$-$C_{59}$ heteroaryl group, and $A_{108}$ is a $C_1$-$C_{59}$ alkylene group).

The term "monovalent non-aromatic condensed polycyclic group" as used herein refers to a monovalent group (for example, having 8 to 60 carbon atoms) having two or more rings condensed to each other, only carbon atoms as ring-forming atoms, and having no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, a heteroatom selected from N, O, P, Si, and S, other than carbon atoms, as a ring-forming atom, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "$C_5$-$C_{30}$ carbocyclic group" as used herein refers to a saturated or unsaturated cyclic group having, as a ring-forming atom, 5 to 30 carbon atoms only. The $C_5$-$C_{30}$ carbocyclic group may be a monocyclic group or a polycyclic group.

The term "$C_1$-$C_{30}$ heterocyclic group" as used herein refers to a saturated or unsaturated cyclic group having, as a ring-forming atom, at least one heteroatom selected from N, O, Si, P, and S other than 1 to 30 carbon atoms. The $C_1$-$C_{30}$ heterocyclic group may be a monocyclic group or a polycyclic group.

A substituent(s) of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_7$-$C_{60}$ arylalkyl group, the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted monovalent non-aromatic condensed polycyclic group, and the substituted monovalent non-aromatic condensed heteropolycyclic group may be:

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$, $-CD_2H$,$-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{11})(Q_{12})$,$-Si(Q_{13})(Q_{14})(Q_{15})$, $-Ge(Q_{13})(Q_{14})(Q_{15})$, $-B(Q_{16})(Q_{17})$, $-P(=O)(Q_{18})(Q_{19})$, $-P(Q_{18})(Q_{19})$, or any combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-C60 alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{21})(Q_{22})$, $-Si(Q_{23})(Q_{24})(Q_{25})$,$-Ge(Q_{23})(Q_{24})(Q_{25})$, $-B(Q_{26})(Q_{27})$, $-P(=O)(Q_{28})(Q_{29})$, $-P(Q_{28})(Q_{29})$, or any combination thereof;

$-N(Q_{31})(Q_{32})$, $-Si(Q_{33})(Q_{34})(Q_{35})$, $-Ge(Q_{33})(Q_{34})(Q_{35})$, $-B(Q_{36})(Q_{37})$,$-P(=O)(Q_{38})(Q_{39})$, or $-P(Q_{38})(Q_{39})$; or any combination thereof.

$Q_1$ to $Q_9$, $Q_{11}$ to $Q_{19}$, $Q_{21}$ to $Q_{29}$, and $Q_{31}$ to $Q_{39}$ in this disclosure may each independently be hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a $C_1$-$C_{60}$ alkyl group unsubstituted or substituted with deuterium, a $C_1$-$C_{60}$ alkyl group, a $C_6$-$C_{60}$ aryl group, or any combination thereof; a $C_2$-$C_{60}$ alkenyl group; a $C_2$-$C_{60}$ alkynyl group; a $C_1$-$C_{60}$ alkoxy group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group unsubstituted or substituted with deuterium, a $C_1$-$C_{60}$ alkyl group, a $C_6$-$C_{60}$ aryl group, or any combination thereof; a $C_6$-$C_{60}$ aryloxy group; a $C_6$-$C_{60}$ arylthio group; a $C_7$-$C_{60}$ arylalkyl group; a $C_1$-$C_{60}$ heteroaryl group; a $C_1$-$C_{60}$ heteroaryloxy group; a $C_1$-$C_{60}$ heteroarylthio group; a $C_2$-$C_{60}$ heteroarylalkyl group; a monovalent non-aromatic condensed polycyclic group; or a monovalent non-aromatic condensed heteropolycyclic group.

Hereinafter, a compound and an organic light-emitting device according to embodiments are described in detail with reference to Synthesis Example and Examples. However, the organic light-emitting device is not limited thereto. The expression "'A' is used instead of 'B'" used in describing Synthesis Examples means that a molar equivalent of "A" was identical to a molar equivalent of "B".

EXAMPLES

**Synthesis Example 1 (Compound 6)**

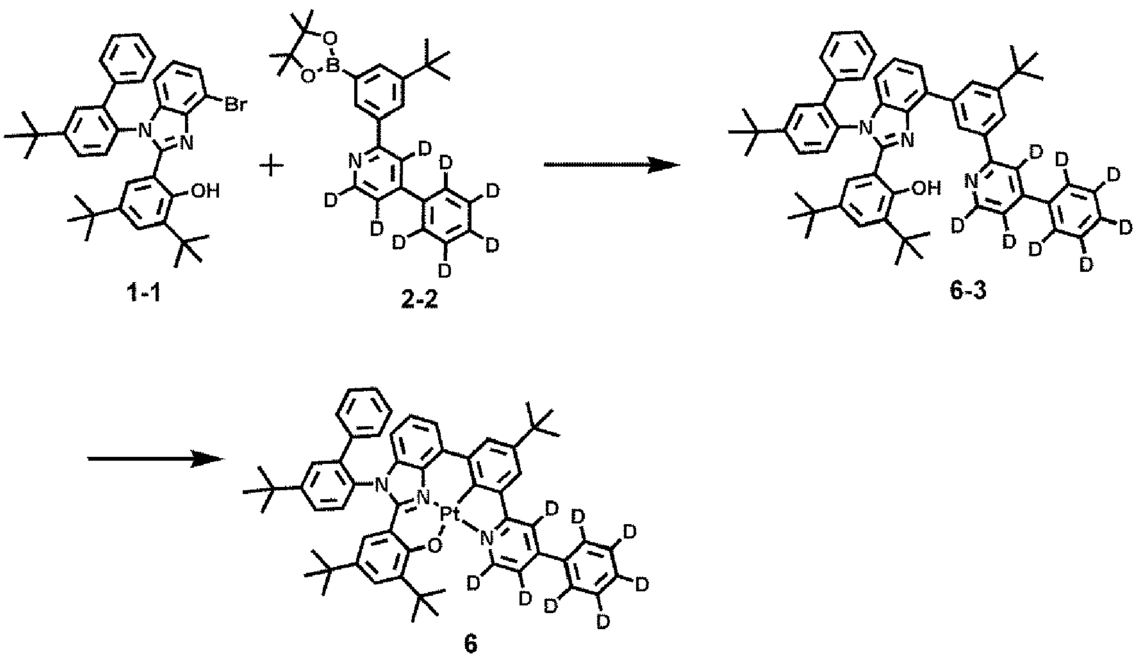

1-1 2-2 6-3

6

Synthesis of Intermediate 6-3

2.4 grams (g) (0.006 millimoles (mmol), 1.2 equivalents, equiv.) of Intermediate 2-2, 1.4 g (0.005 mmol, 1 equiv.) of Intermediate 1-1, 0.40 g (0.001 mmol, 0.07 equiv.) of tetrakis(triphenylphosphine)palladium(0), and 2.0 g (0.015 mmol, 3 equiv.) of potassium carbonate were dissolved in 20 milliliters (mL) of a solvent in which tetrahydrofuran (THF) and distilled water ($H_2O$) were mixed at a ratio of 3:1, and the reaction mixture was refluxed for 12 hours. The resultant obtained therefrom was cooled to room temperature, and the precipitate was filtered to obtain a filtrate. The filtrate was washed by using ethyl acetate (EA)/$H_2O$ and the crude product was purified by column chromatography (while increasing a rate of EA/hexane (Hex) to between 20 % to 35 %) to obtain 2.2 g (yield of 68 %) of Intermediate 6-3. The obtained compound was identified by Mass spectrum and HPLC analysis.
HRMS(MALDI) calcd for $C_{58}H_{53}D_8N_3O$ : m/z 823.5317 , Found: 823.5319.

Synthesis of Compound 6

2.2 g (2.26 mmol) of Intermediate 6-3 and 1.1 g (2.26 mmol, 1.0 equiv.) of $K_2PtCl_4$ were dissolved in 40 mL of

a solvent in which 30 mL of AcOH and 10 mL of $H_2O$ were mixed, and the reaction mixture was refluxed for 16 hours. The resultant obtained therefrom was cooled to room temperature, and the precipitate was filtered. The precipitate was dissolved again in MC and washed by using $H_2O$. The crude product was purified by column chromatography (methylene chloride (MC) 40 %, EA 1 %, Hex 59 %) to obtain 1.6 g (yield of 60 %) of Compound 6. The obtained compound was identified by Mass spectrum and HPLC analysis.
HRMS (MALDI) calcd for $C_{58}H_{51}D_8N_3OPt$: m/z 1016.4808, Found: 1016.4805.

**Synthesis Example 2 (Compound 1)**

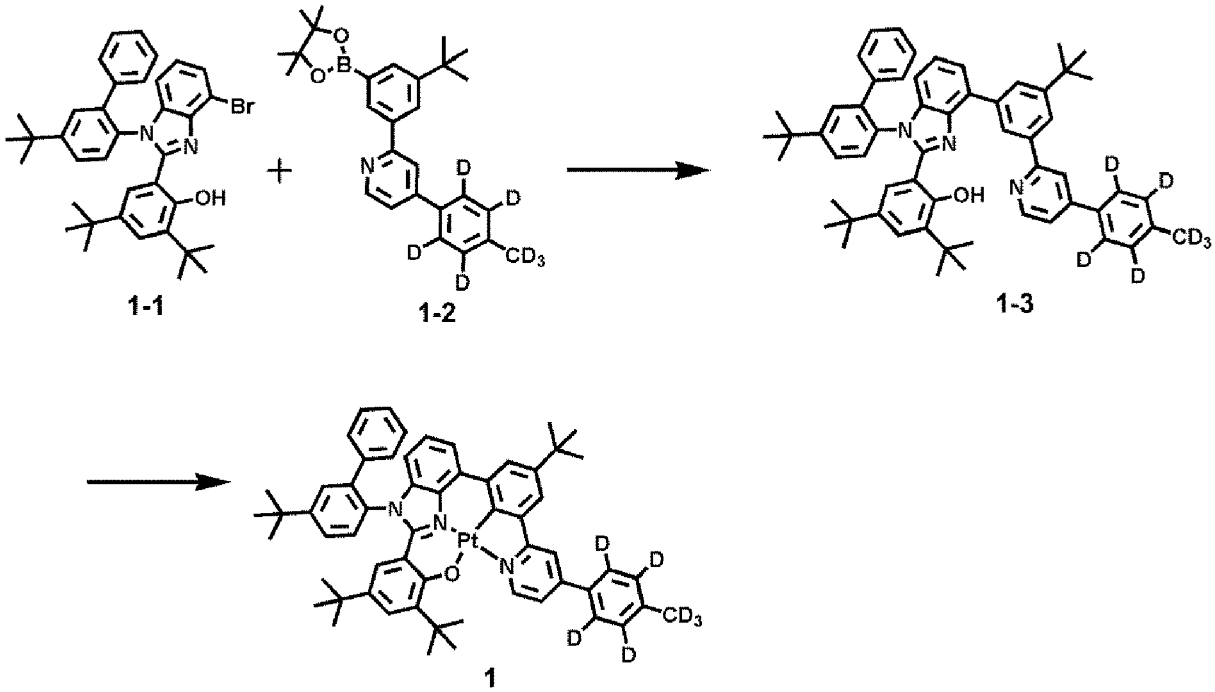

1-1 1-2 1-3

1

Synthesis of Intermediate 1-3

Intermeidate 1-3 1.2g (yield of 65%) was obtained in the same manner as in the synthesis of Intermediate 6-3 of Synthesis Example 1, except that Intermediate 1-2 was used instead of Intermediate 2-2. The obtained compound was identified by Mass spectrum and HPLC analysis.
HRMS(MALDI) calcd for $C_{59}H_{56}D_7N_3O$ : m/z 836.5411 , Found: 836.5414.

Synthesis of Compound 1

Compound 11.0g (yield of 68%) was obtained in the same manner as in the synthesis of Compound 6 of Synthesis Example 1, except that Intermediate 1-3 was used instead of Intermediate 6-3. The obtained compound was identified by Mass spectrum and HPLC analysis.
HRMS(MALDI) calcd for $C_{59}H_{54}D_7N_3OPt$ : m/z 1029.4902 , Found: 1029.4901.

**Synthesis Example 3 (Compound 2)**

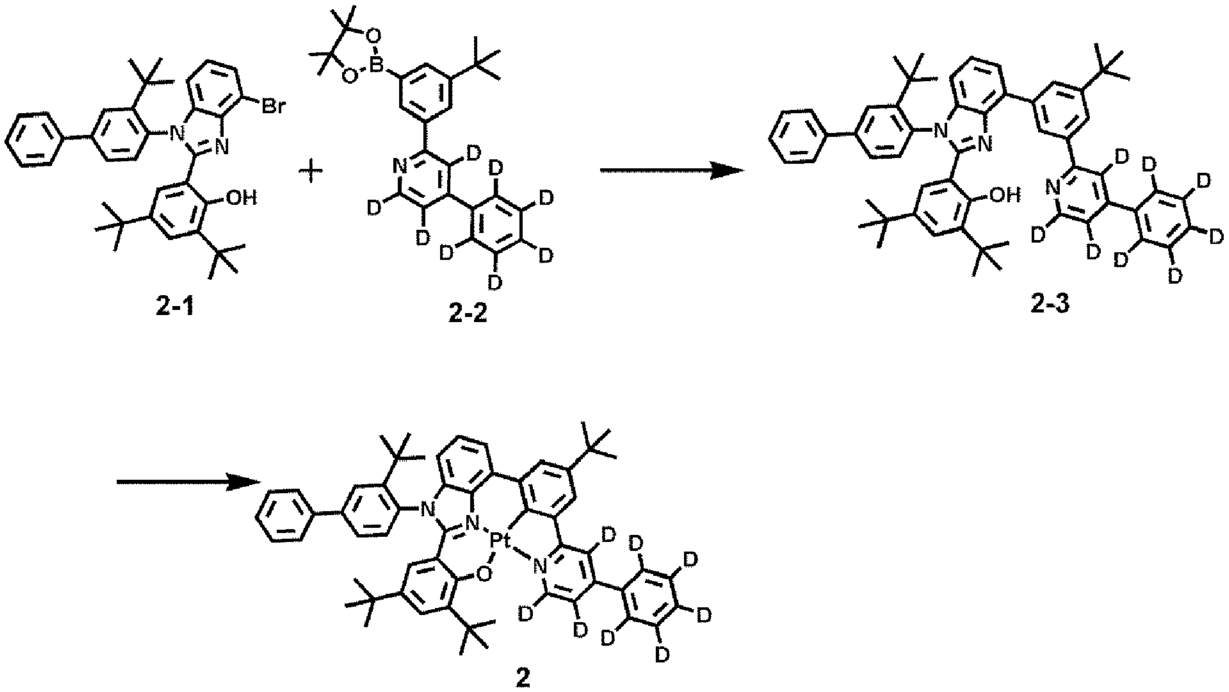

2-1 2-2 2-3

2

Synthesis of Intermediate 2-3

Intermeidate 2-3 2.1 g (yield of 70%) was obtained in the same manner as in the synthesis of Intermediate 6-3 of Synthesis Example 1, except that Intermediate 2-1 was used instead of Intermediate 1-1. The obtained compound was identified by Mass spectrum and HPLC analysis.
HRMS(MALDI) calcd for $C_{58}H_{53}D_8N_3O$ : m/z 823.5317 , Found: 823.5319.

Synthesis of Compound 2

Compound 2 1.84g (yield of 71%) was obtained in the same manner as in the synthesis of Compound 6 of Synthesis Example 1, except that Intermediate 2-3 was used instead of Intermediate 6-3. The obtained compound was identified by Mass spectrum and HPLC analysis.
HRMS(MALDI) calcd for $C_{58}H_{51}D_8N_3OPt$ : m/z 1016.4808 , Found: 1016.4809.

**Synthesis Example 4 (Compound 3)**

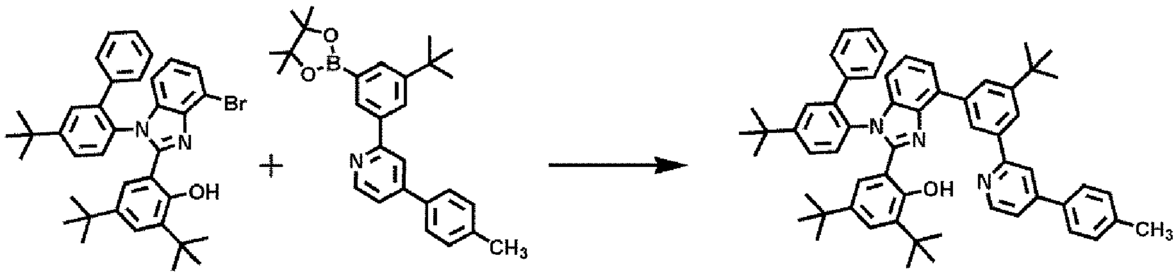

1-1 3-2 3-3

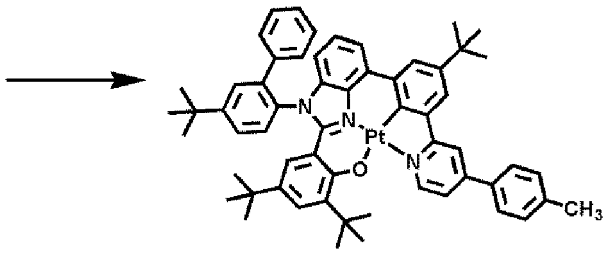

3

Synthesis of Intermediate 3-3

Intermeidate 3-3 1.7g (yield of 72%) was obtained in the same manner as in the synthesis of Intermediate 6-3 of Synthesis Example 1, except that Intermediate 3-2 was used instead of Intermediate 2-2. The obtained compound was identified by Mass and HPLC analysis.
HRMS(MALDI) calcd for $C_{59}H_{63}N_3O$ : m/z 829.4971 , Found : 829.4973.

Synthesis of Compound 3

Compound 3 1.53g (yield of 73%) was obtained in the same manner as in the synthesis of Compound 6 of Synthesis Example 1, except that Intermediate 3-3 was used instead of Intermediate 6-3. The obtained compound was identified by Mass spectrum and HPLC analysis.
HRMS(MALDI) calcd for $C_{59}H_{61}N_3OPt$ : m/z 1022.4462 , Found : 1022.4461.

**Synthesis Example 5 (Compound 4)**

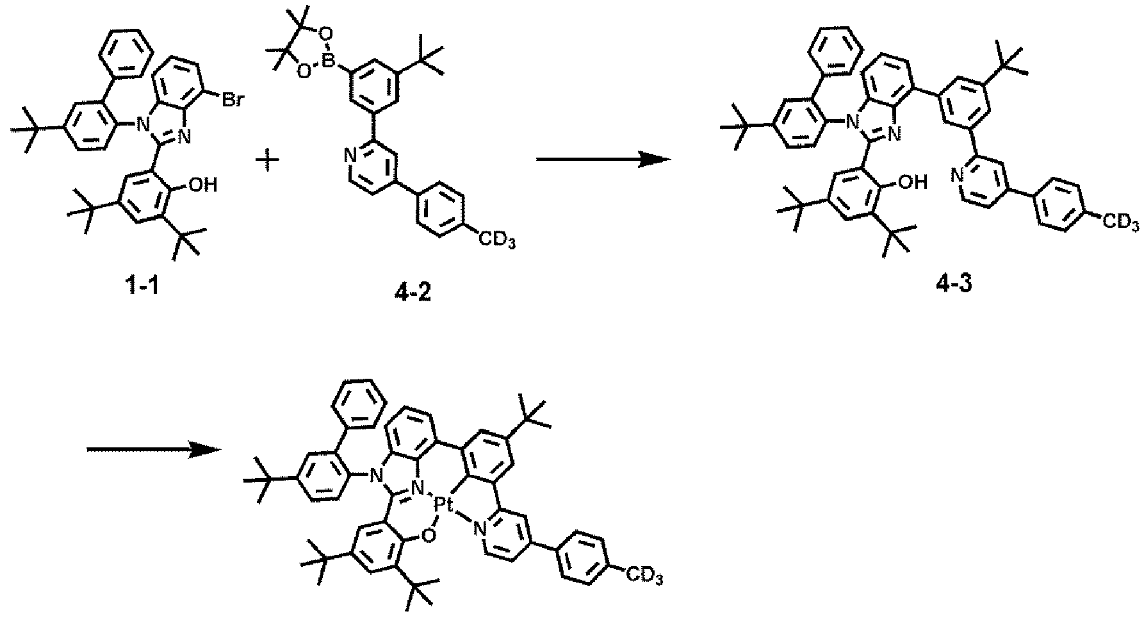

1-1 4-2 4-3

4

Synthesis of Intermediate 4-3

Intermeidate 4-3 1.85g (yield of 73%) was obtained in the same manner as in the synthesis of Intermediate 6-3 of Synthesis Example 1, except that Intermediate 4-2 was used instead of Intermediate 2-2. The obtained compound was identified by Mass spectrum and HPLC analysis.
HRMS(MALDI) calcd for $C_{59}H_{60}D_3N_3O$ : m/z 832.5159 , Found : 832.52.

Synthesis of Compound 4

Compound 4 1.64g (yield of 72%) was obtained in the same manner as in the synthesis of Compound 6 of Synthesis Example 1, except that Intermediate 4-3 was used instead of Intermediate 6-3. The obtained compound was identified by Mass spectrum and HPLC analysis.
HRMS(MALDI) calcd for $C_{59}H_{58}D_3N_3OPt$ : m/z 1025.4651 , Found : 1025.4652.

**Synthesis Example 6 (Compound 5)**

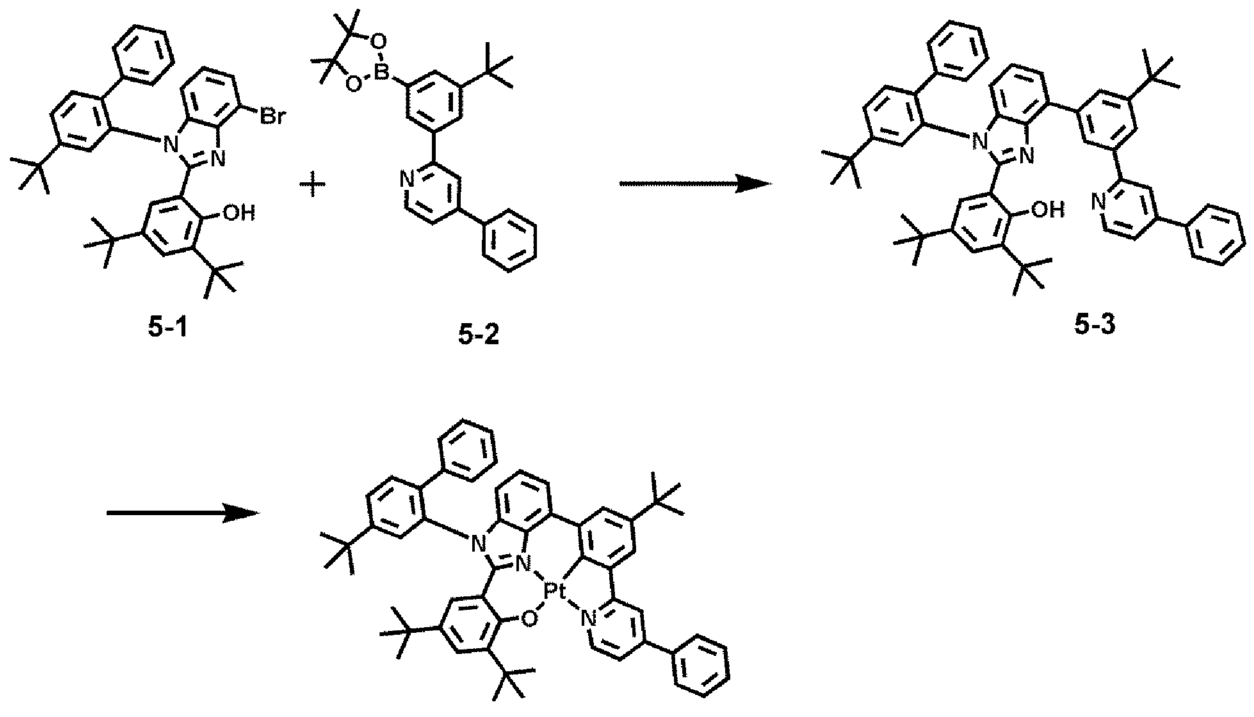

5-1 5-2 5-3

5

Synthesis of Intermediate 5-3

Intermeidate 5-3 2.0g (yield of 68%) was obtained in the same manner as in the synthesis of Intermediate 6-3 of Synthesis Example 1, except that Intermediate 5-1 and Intermediate 5-2 were used instead of Intermediate 1-1 and Intermediate 2-2. The obtained compound was identified by Mass and HPLC analysis.
HRMS(MALDI) calcd for $C_{58}H_{61}N_3O$ : m/z 815.4815 , Found : 815.4813.

Synthesis of Compound 5

Compound 5 1.73g (yield of 70%) was obtained in the same manner as in the synthesis of Compound 6 of Synthesis Example 1, except that Intermediate 5-3 was used instead of Intermediate 6-3. The obtained compound was identified by Mass spectrum and HPLC analysis.
HRMS(MALDI) calcd for $C_{58}H_{59}N_3OPt$ : m/z 1008.4306 , Found : 1008.4308.

**Synthesis Example 7 (Compound 7)**

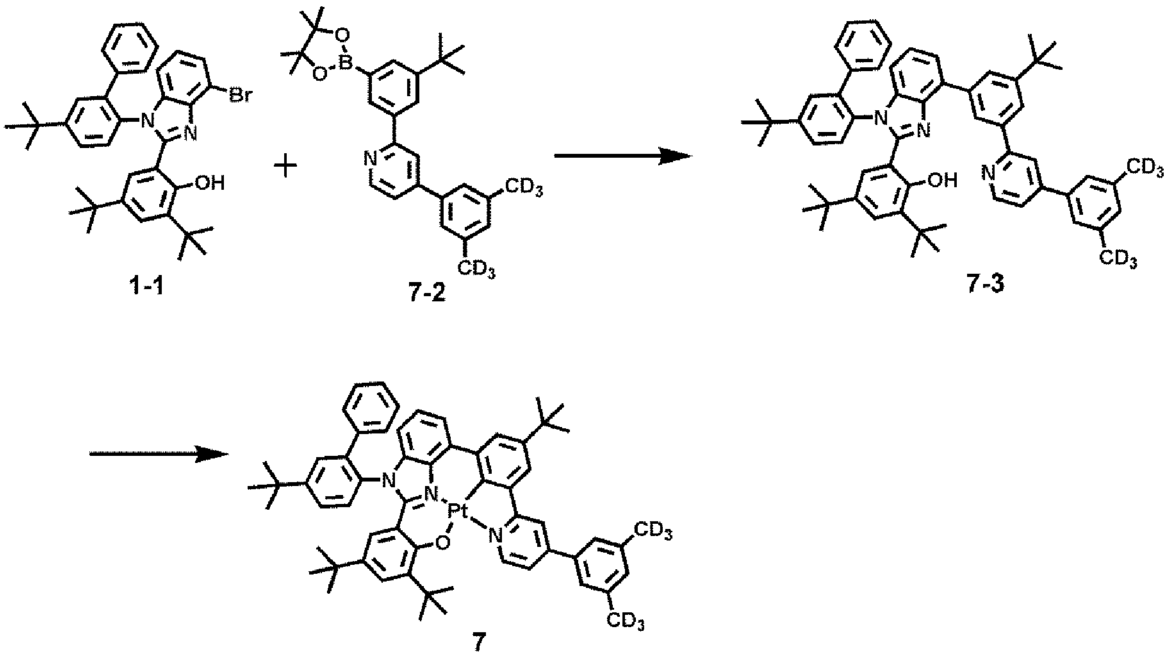

1-1 7-2 7-3

7

Synthesis of Intermediate 7-3

Intermeidate 7-3 1.8g (yield of 69%) was obtained in the same manner as in the synthesis of Intermediate 6-3 of Synthesis Example 1, except that Intermediate 7-2 was used instead of Intermediate 2-2. The obtained compound was identified by Mass spectrum and HPLC analysis.
HRMS(MALDI) calcd for $C_{60}H_{59}D_6N_3O$ : m/z 849.5504 , Found : 849.5502.

Synthesis of Compound 7

Compound 7 1.65g (yield of 75%) was obtained in the same manner as in the synthesis of Compound 6 of Synthesis Example 1, except that Intermediate 7-3 was used instead of Intermediate 6-3. The obtained compound was identified by Mass spectrum and HPLC analysis.
HRMS(MALDI) calcd for $C_{60}H_{57}D_6N_3OPt$ : m/z 1042.4995 , Found : 1042.4996.

**Synthesis Example 8 (Compound 8)**

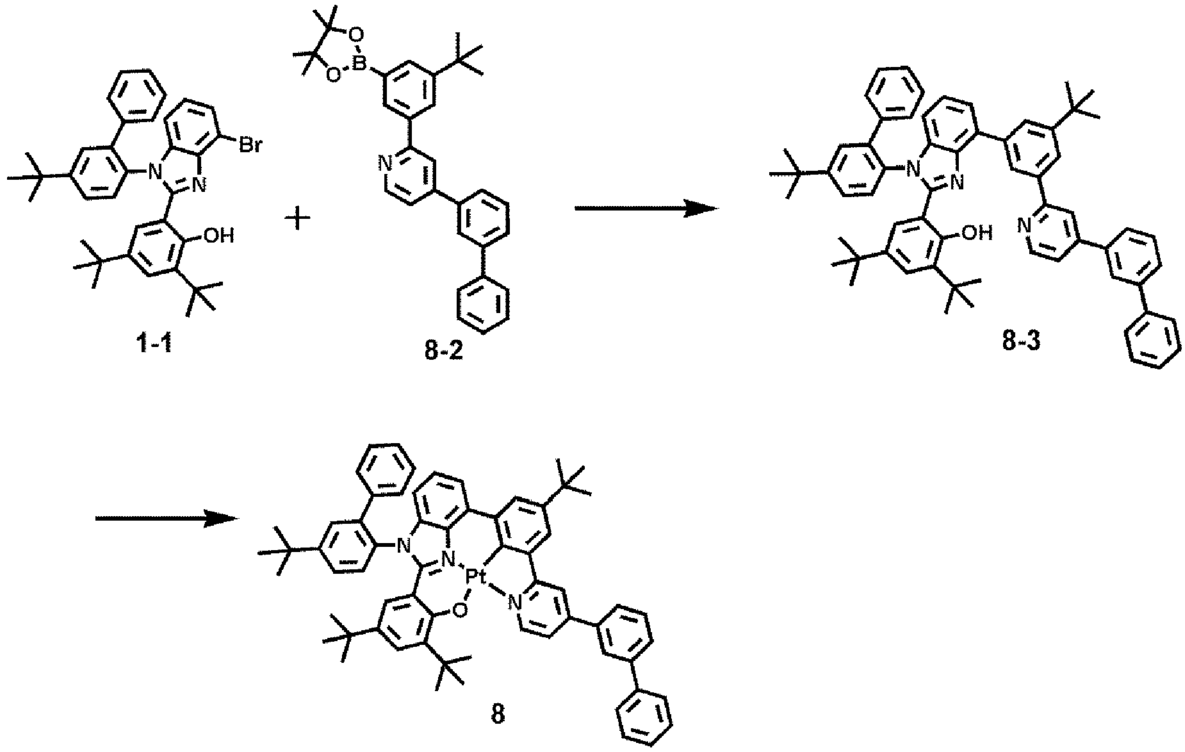

1-1 8-2 8-3

8

Synthesis of Intermediate 8-3

Intermeidate 8-3 1.7g (yield of 65%) was obtained in the same manner as in the synthesis of Intermediate 6-3 of Synthesis Example 1, except that Intermediate 8-2 was used instead of Intermediate 2-2. The obtained compound was identified by Mass spectrum and HPLC analysis.
HRMS(MALDI) calcd for $C_{64}H_{65}N_3O$ : m/z 891.5128 , Found : 891.5127.

Synthesis of Compound 8

Compound 8 1.41g (yield of 68%) was obtained in the same manner as in the synthesis of Compound 6 of Synthesis Example 1, except that Intermediate 8-3 was used instead of Intermediate 6-3. The obtained compound was identified by Mass spectrum and HPLC analysis.
HRMS(MALDI) calcd for $C_{64}H_{63}N_3OPt$ : m/z 1084.4619 , Found : 1084.4617.

**Synthesis Example 9 (Compound 9)**

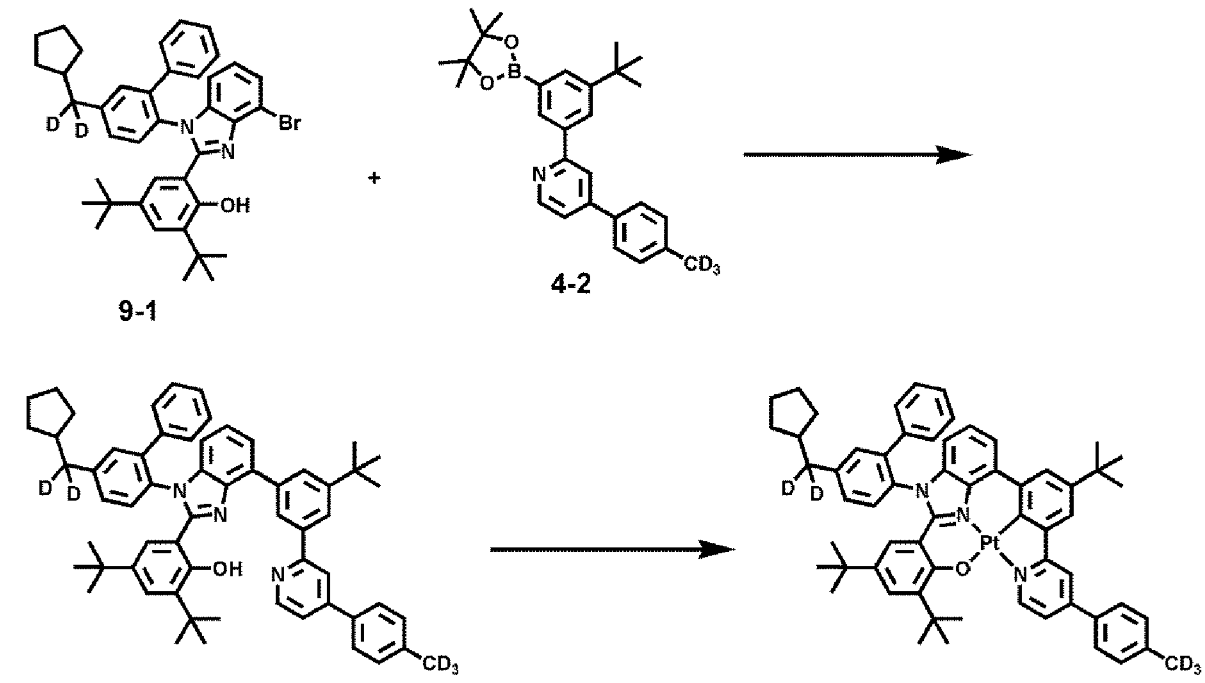

9-1 4-2 9-3 9

Synthesis of Intermediate 9-3

Intermeidate 9-3 2.1g (yield of 63%) was obtained in the same manner as in the synthesis of Intermediate 4-3 of Synthesis Example 5, except that Intermediate 9-1 was used instead of Intermediate 1-1. The obtained compound was identified by Mass spectrum and HPLC analysis.
HRMS(MALDI) calcd for $C_{61}H_{60}D_5N_3O$ : m/z 860.5441 , Found : 860.5443.

Synthesis of Compound 9

Compound 9 1.77g (yield of 69%) was obtained in the same manner as in the synthesis of Compound 4 of Synthesis Example 5, except that Intermediate 9-3 was used instead of Intermediate 4-3. The obtained compound was identified by Mass spectrum and HPLC analysis.
HRMS(MALDI) calcd for $C_{61}H_{58}D_5N_3OPt$ : m/z 1053.4933 , Found : 1053.4932.

Evaluation Example 1 : Evaluation of photoluminescence quantum yield (PLQY) and radiative decay rate

CBP and Compound 1 were co-deposited at a weight ratio of 9:1 at a vacuum degree of $10^{-7}$ torr to manufacture a film having a thickness of 40 nanometers (nm).

A PLQY of the film was evaluated by using a Hamamatsu Photonics absolute PL quantum yield measurement system including a xenon light source, a monochromator, a photonic multichannel analyzer, an integrating sphere and employing a PLQY measurement software (Hamamatsu Photonics, Ltd., Shizuoka, Japan), and a PLQY of Compound 1 was confirmed. The results of this study are shown in Table 2.

A PL spectrum of the film was evaluated at room temperature by using a PicoQuant TRPL measurement system FluoTime 300 and a PicoQuant pumping source PLS340 (excitation wavelength = 340 nm, spectral width = 20 nm), a wavelength of a main peak of the spectrum was determined, PLS340 repeatedly measured the number of photons emitted from the film at the wavelength of the main peak due to a photon pulse (pulse width = 500 picoseconds, ps) applied to the film according to time based on time-correlated single photon counting (TCSPC), thereby obtaining a sufficiently fittable TRPL curve. $T_{decay}$(Ex) (decay time) of the film was obtained by fitting one or more exponential decay functions to the result obtained therefrom, and the radiative decay rate that is a reciprocal of $T_{decay}$(Ex) was calculated. The results are shown in Table 2. The function used for fitting is expressed by Equation 20, and the greatest value of $T_{decay}$ obtained from each exponential decay function used for fitting was taken as $T_{decay}$(Ex). At this time, a baseline or background signal curve was obtained by repeating the same measurement once more for the same measurement time as the measurement time for obtaining the TRPL curve in a dark state (a state in which a pumping signal applied to the predetermined film was blocked), and the baseline or background signal curve was used for fitting as a baseline.

Equation 20

$$f(t) = \sum_{i=1}^{n} A_i \, exp\left(-t/T_{decay,i}\right)$$

The measurement of the PLQY and the radiative decay time was performed on Compounds 2 to 9, and results thereof are shown in Table 2.

Table 2

| Compound No. | PLQY (%) | Radiative decay rate ($s^{-1}$) |
|---|---|---|
| 1 | 0.999 | $4.15 \times 10^5$ |
| 2 | 0.992 | $4.09 \times 10^5$ |
| 3 | 0.999 | $4.81 \times 10^5$ |
| 4 | 0.999 | $4.85 \times 10^5$ |
| 5 | 0.999 | $4.99 \times 10^5$ |
| 6 | 0.999 | $4.93 \times 10^5$ |
| 7 | 0.998 | $4.90 \times 10^5$ |
| 8 | 0.999 | $4.83 \times 10^5$ |
| 9 | 0.999 | $4.16 \times 10^5$ |
| *$s^{-1}$ = reverse seconds | | |

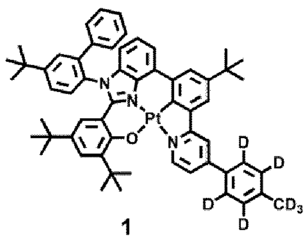
1

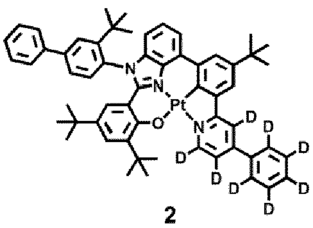
2

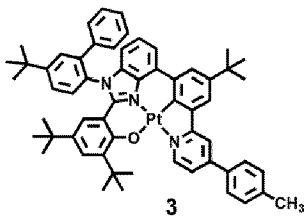
3

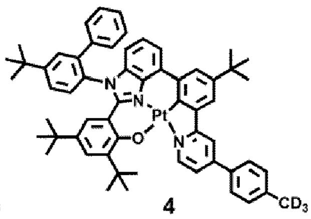
4

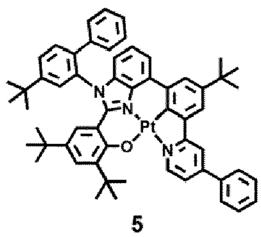
5

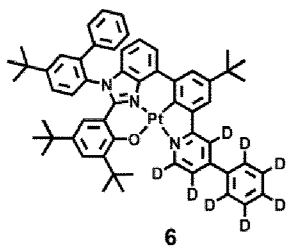
6

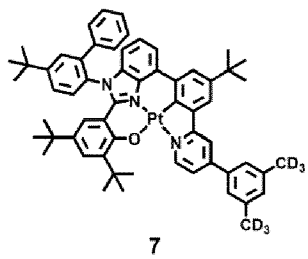
7

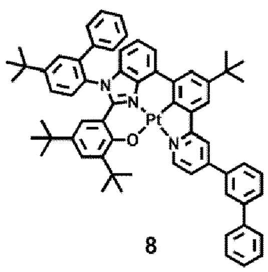
8

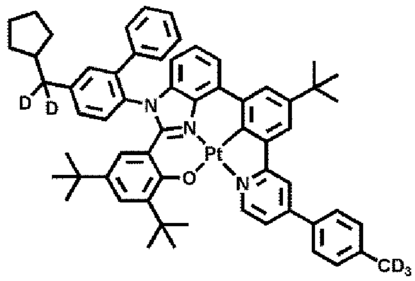

9

Referring to Table 2, it is confirmed that Compounds 1 to 9 have a high PLQY and a high radiative decay rate.

Evaluation Example 2: Evaluation of horizontal orientation ratio

mCP and Compound 1 were co-deposited on a fused silica base layer (thickness of 1 mm) at a weight ratio of 92:8 in a vacuum deposition apparatus having a vacuum degree of $1\times10^{-7}$ torr to form Sample 1 having a thickness of 30 nm (8 weight%), and Sample 1 was sealed with glass and glue in a nitrogen atmosphere. This procedure was repeated on Compounds shown in Table 3 to manufacture Samples 2 to 9.

Meanwhile, an angle-dependent PL measurement apparatus having a structure illustrated in FIG. 3 of Korean Patent Application No. 10-2013-0150834 was prepared. Detailed specifications are as follows:

- Excited light wavelength: 325 nm
- Excited light supply source: He-Cd laser, Melles Griot
- Excited light irradiation means: Optical fiber, diameter of 1 millimeter (mm), Thorlabs
- Semi-cylindrical prism: Fused silica, diameter of 100 mm, length of 30 mm
- Emitted light detection means: Photomultiplier tube, Acton
- Polarizer mounted on emitted light detection means: Linear polarizer, Thorlabs
- Recording apparatus: SpectraSense, Acton
- Excited light incidence angle: $\theta P$ = 45°, $\theta H$ = 0°
- Distance from sample to emitted light detection means (or radius of moving path of emitted light detection means): 900 mm

Then, each of Samples 1 to 8 was fixed on a semi-cylindrical lens, and a 325-nm laser was irradiated to emit light. The emitted light passed through a polarization film, and p-polarization light emission strength was measured with respect to 530-nm light of 90° to 0° while turning by 1° with respect to the axis of the semi-cylindrical lens, to which the sample was fixed, by using a charge-coupled device (CCD).

p-Polarization light emission strength (first p-polarization light emission strength) appearing when each Compound has a vertical orientation and p-polarization light emission strength (second p-polarization light emission strength) appearing when each Compound had a horizontal orientation were calculated with respect to 0° to 90°. The weights at which p-polarization light emission strength obtained by multiplying each weight by the first and second p-polarization light emission strengths coincide with the measured p-polarization light emission strength were calculated, and the horizontal orientation ratios of Compounds shown in Table 3 were measured. The results of this study are shown in Table 3. The angle-dependent PL spectrum was analyzed by using a classical dipole moment regarded as dissipated power from a dipole oscillating light emission from excitons.

Table 3

| Sample No. | Co-deposition material | Horizontal orientation ratio (%) |
|---|---|---|
| 1 | mCP : Compound 1(8 weight%) | 90 % |
| 2 | mCP : Compound 2(8 weight%) | 90 % |
| 3 | mCP : Compound 3(8 weight%) | 88 % |
| 4 | mCP : Compound 4(8 weight%) | 88 % |
| 5 | mCP : Compound 5(8 weight%) | 90 % |

(continued)

| Sample No. | Co-deposition material | Horizontal orientation ratio (%) |
|---|---|---|
| 6 | mCP : Compound 6(8 weight%) | 90 % |
| 7 | mCP : Compound 7(8 weight%) | 90% |
| 8 | mCP : Compound 8(8 weight%) | 90% |
| 9 | mCP : Compound 9(8 weight%) | 90% |
| * weight% = percent by weight | | |

Referring to Table 3, it is confirmed that Compound 1 to 9 have excellent horizontal orientation ratios, that is, excellent horizontal optical orientation.

Example 1

As an anode, a glass substrate, on which ITO/Ag/ITO (70 Å / 1,000 Å / 70 Å) were deposited, was cut to a size of 50 mm x 50 mm x 0.5 mm, sonicated with iso-propyl alcohol and pure water each for 5 minutes, and cleaned by exposure to ultraviolet rays and ozone for 30 minutes. Then, the glass substrate was provided to a vacuum deposition apparatus.
2-TNATA was vacuum-deposited on the anode of the glass substrate to form a hole injection layer having a thickness of 600 Å, and 4,4'-bis[N-(1-naphthyl)-N-phenylamino]biphenyl (NPB) was vacuum-deposited on the hole injection layer to form a hole transport layer having a thickness of 1,350 Å.
CBP (host) and Compound 1 (dopant) were co-deposited on the hole transport layer at a weight ratio of 94:6 to form an emission layer having a thickness of 400 Å.
Then, BCP was vacuum-deposited on the emission layer to form a hole blocking layer having a thickness of 50 Å, $Alq_3$ was vacuum-deposited on the hole blocking layer to form an electron transport layer having a thickness of 350 Å, LiF was deposited on the electron transport layer to form an electron injection layer having a thickness of 10 Å, and MgAg was deposited on the electron injection layer at a weight ratio of 90:10 to form a cathode having a thickness of 120 Å, thereby completing an organic light-emitting device having a structure of anode / 2-TNATA (600 Å) / NPB (1,350 Å) / CBP + Compound 1 (6 weight%) (400 Å) / BCP (50 Å) / $Alq_3$ (350 Å) / LiF (10 Å) / MgAg (120 Å).

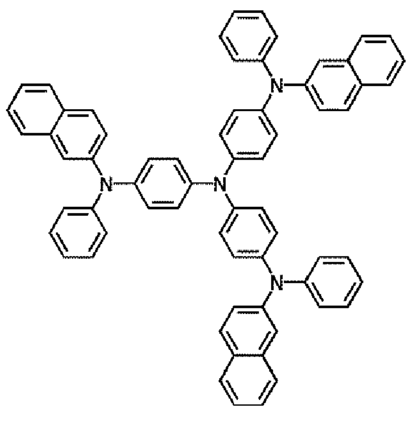

**2-TNATA**

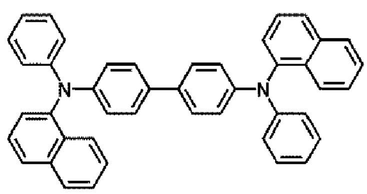

**NPB**

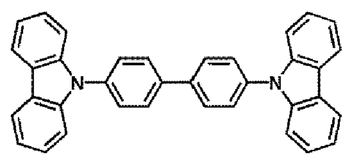

**CBP**

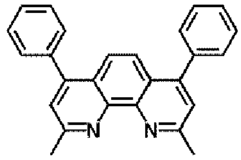

**BCP**

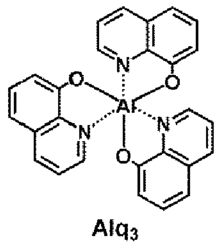

**$Alq_3$**

Examples 2 to 9 and Comparative Examples A to E

Organic light-emitting devices were manufactured in the same manner as in Example 1, except that Compound shown in Table 4 were each used instead of Compound 1 as a dopant in forming an emission layer.

Evaluation Example 3: Evaluation of characteristics of organic light-emitting devices

The driving voltage, current density, maximum quantum efficiency, roll-off ratio, full width at half maximum (FWHM), peak emission wavelength, and lifespan of the organic light-emitting devices manufactured according to Examples 1 to 9 and Comparative Example A to E were evaluated by using a current-voltage meter (Keithley 2400) and a luminance meter (Minolta Cs-1000A), and results thereof are shown in Tables 4 and 5. The roll-off ratio was calculated by Equation 30. The lifespan ($LT_{99}$, at 3,500 nit) indicates an amount of time that elapsed when luminance was 99 % of initial luminance (100 %) and is expressed by a relative value (%).

Equation 30

roll off ratio = {1- (efficiency (at 3,500 nit) / maximum luminescent efficiency)} X 100%

Table 4

| | Dopant compound No. | Driving voltage (V) | Current density ($mA/cm^2$) | Maximum quantum efficiency (%) | Roll-off ratio (%) | FWHM (nm) |
|---|---|---|---|---|---|---|
| Example 1 | 1 | 3.69 | 10 | 112 | 9 | 68 |
| Example 2 | 2 | 3.72 | 10 | 102 | 6 | 68 |
| Example 3 | 3 | 3.79 | 10 | 107 | 8 | 68 |
| Example 4 | 4 | 3.79 | 10 | 109 | 9 | 67 |
| Example 5 | 5 | 3.83 | 10 | 99 | 7 | 69 |
| Example 6 | 6 | 4.60 | 10 | 108 | 10 | 70 |
| Example 7 | 7 | 3.84 | 10 | 111 | 8 | 67 |
| Example 8 | 8 | 3.80 | 10 | 114 | 7 | 67 |
| Example 9 | 9 | 3.96 | 10 | 110 | 7 | 67 |
| Comparative Example A | A | 3.94 | 10 | 94 | 10 | 68 |
| Comparative Example B | B | 4.08 | 10 | 93 | 8 | 68 |
| Comparative Example C | C | 3.85 | 10 | 98 | 4 | 68 |
| Comparative Example D | D | 3.90 | 10 | 88 | 8 | 67 |
| Comparative Example E | E | 4.39 | 10 | 88 | 17 | 64 |

Table 5

| | Dopant compound No. | Peak emission wavelength (nm) | Lifespan ($LT_{99}$) (at 3,500 nit) a relative value (%) |
|---|---|---|---|
| Example 1 | 1 | 521 | 115 % |
| Example 2 | 2 | 523 | 110 % |
| Example 3 | 3 | 521 | 113 % |
| Example 4 | 4 | 521 | 115 % |
| Example 5 | 5 | 526 | 110 % |
| Example 6 | 6 | 525 | 130 % |
| Example 7 | 7 | 524 | 110% |
| Example 8 | 8 | 527 | 180% |
| Example 9 | 9 | 523 | 105% |
| Comparative Example A | A | 523 | 29 % |
| Comparative Example B | B | 526 | 50 % |
| Comparative Example C | C | 523 | 60% |
| Comparative Example D | D | 520 | 3 % |
| Comparative Example E | E | 502 | 1 % |

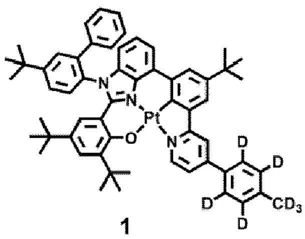
1

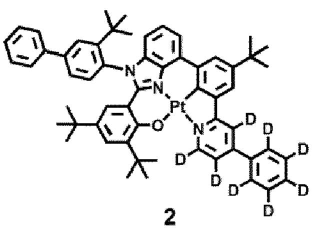
2

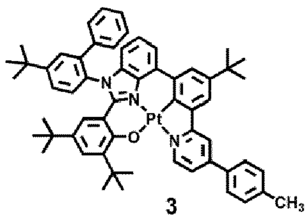
3

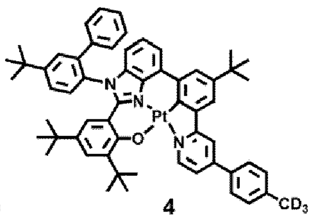
4

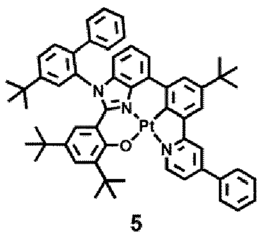
5

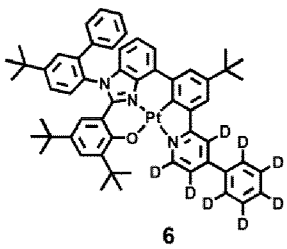
6

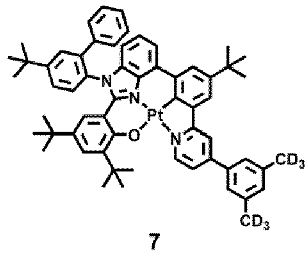
7

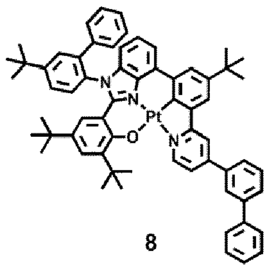
8

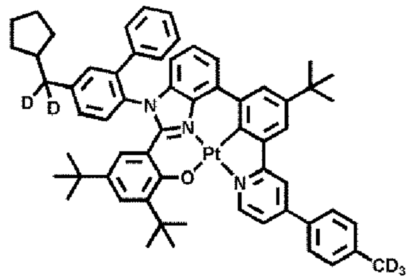

9

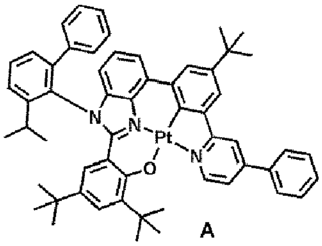

A

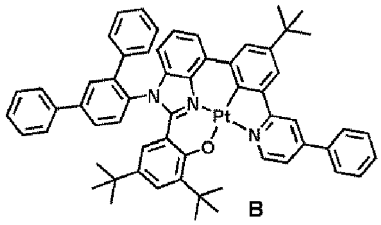

B

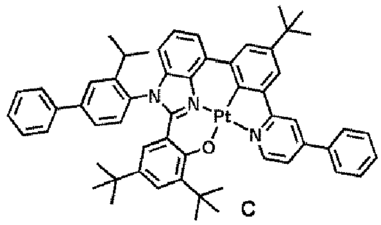

C

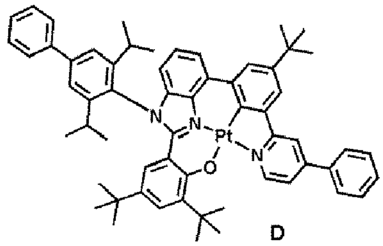

D

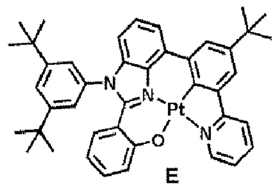

E

Referring to Tables 4 and 5, it is confirmed that the organic light-emitting devices of Examples 1 to 9 have improved driving voltage, improved maximum quantum efficiency, and/or improved roll-off ratio, and also have improved lifespan characteristics, as compared with those of the organic light-emitting devices of Comparative Examples A to E.

Since the organometallic compound may emit light having a relatively small FWHM and have excellent PLQY, excellent radiative decay rate, and excellent horizontal orientation ratio, the organic light-emitting device including the organometallic compound may have improved driving voltage, external quantum efficiency, roll-off ratio, and lifespan characteristics. In addition, since the organometallic compound has excellent phosphorescence characteristics, a diagnostic composition including the organometallic compound may have high diagnostic efficiency.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope of the present description as defined by the following claims.

## Claims

**1.** An organometallic compound represented by Formula 1:

Formula 1

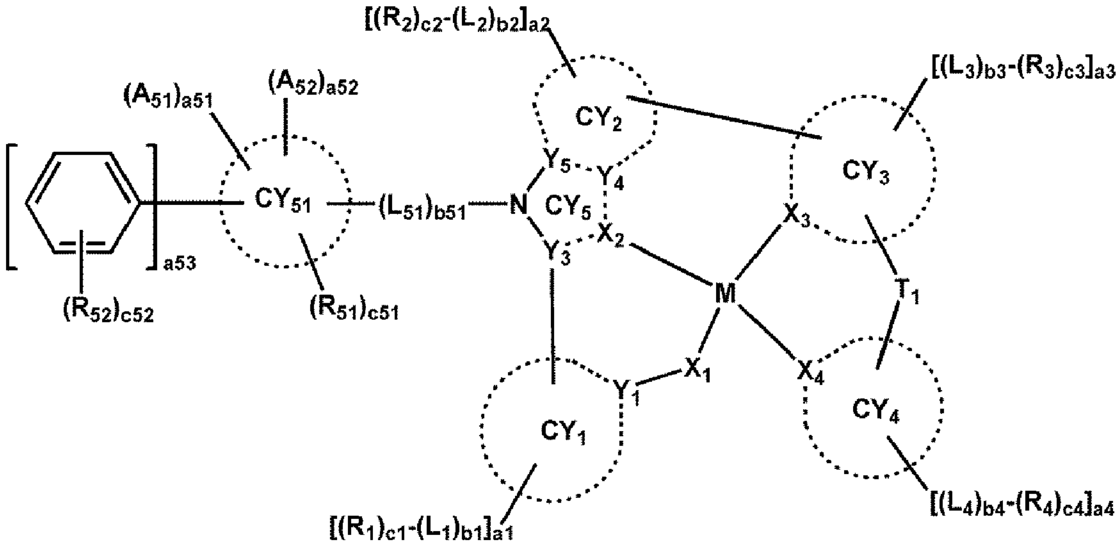

,

wherein, in Formula 1,

M is a transition metal,
$X_1$ is O or S, wherein a bond between $X_1$ and M is a covalent bond,
$X_2$ to $X_4$ are each independently C or N,
one bond selected from a bond between $X_2$ and M, a bond between $X_3$ and M, and a bond between $X_4$ and M is a covalent bond, and the other bonds selected from a bond between $X_2$ and M, a bond between $X_3$ and M, and a bond between $X_4$ and M are coordinate bonds,
$Y_1$ and $Y_3$ to $Y_5$ are each independently C or N,
a bond between $X_2$ and $Y_3$, a bond between $X_2$ and $Y_4$, and a bond between $Y_4$ and $Y_5$ is a chemical bond,
ring $CY_1$ to ring $CY_4$ and ring $CY_{51}$ are each independently a $C_5$-$C_{30}$ carbocyclic group or a $C_1$-$C_{30}$ heterocyclic group,
a cyclometalated ring formed by ring $CY_5$, ring $CY_2$, ring $CY_3$, and M is a 6-membered ring,
$T_1$ is a single bond, a double bond, *-N($R_5$)-*', *-B($R_5$)-*', *-P($R_5$)-*', *-C($R_5$)($R_6$)-*', *-Si($R_5$)($R_6$)-*', *-Ge($R_5$)($R_6$)-*', *-S-*', *-Se-*', *-O-*', *-C(=O)-*', *-S(=O)-*', *-S(=O)$_2$-*', *-C($R_5$)=*', *=C($R_5$)-*', *-C($R_5$)=C($R_6$)-*', *-C(=S)-*', or *-C≡C-*', wherein * and *' each indicate a binding site to a neighboring atom,
$L_1$ to $L_4$ and $L_{51}$ are each independently a single bond, a $C_5$-$C_{30}$ carbocyclic group that is unsubstituted or substituted with at least one $R_{10a}$, or a $C_1$-$C_{30}$ heterocyclic group that is unsubstituted or substituted with at least one $R_{10a}$,
b1 to b4 and b51 are each independently an integer from 1 to 5,
$R_1$ to $R_6$, $R_{51}$, and $R_{52}$ are each independently hydrogen, deuterium, -F, -Cl,-Br, -I, -$SF_5$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted $C_1$-$C_{60}$ alkyl group, a substituted or unsubstituted $C_2$-$C_{60}$ alkenyl group, a substituted or unsubstituted $C_2$-$C_{60}$ alkynyl group, a substituted or unsubstituted $C_1$-$C_{60}$ alkoxy group, a substituted or unsubstituted $C_3$-$C_{10}$ cycloalkyl group, a substituted or unsubstituted $C_1$-$C_{10}$ heterocycloalkyl group, a substituted or unsubstituted $C_3$-$C_{10}$ cycloalkenyl group, a substituted or unsubstituted $C_1$-$C_{10}$ heterocycloalkenyl group, a substituted or unsubstituted $C_6$-$C_{60}$ aryl group, a substituted or unsubstituted $C_6$-$C_{60}$ aryloxy group, a substituted or unsubstituted $C_6$-$C_{60}$ arylthio group, a substituted or unsubstituted $C_7$-$C_{60}$ arylalkyl group, a substituted or unsubstituted $C_1$-$C_{60}$ heteroaryl group, a substituted or unsubstituted $C_1$-$C_{60}$ heteroaryloxy group, a substituted or unsubstituted $C_1$-$C_{60}$ heteroarylthio group, a substituted or unsubstituted $C_2$-$C_{60}$ heteroarylalkyl group, a substituted or unsubstituted monovalent non-aromatic condensed polycyclic group, a substituted or unsubstituted monovalent non-aromatic condensed heteropolycyclic group,-N($Q_1$)($Q_2$), -Si($Q_3$)($Q_4$)($Q_5$), -Ge($Q_3$)($Q_4$)($Q_5$), -B($Q_6$)($Q_7$), -P(=O)($Q_8$)($Q_9$), or-P($Q_8$)($Q_9$),
c1 to c4, c51 and c52 are each independently an integer from 1 to 5,
$A_{51}$ is a $C_4$-$C_{60}$ alkyl group,
$A_{52}$ is deuterium or a deuterium-containing $C_1$-$C_{60}$ alkyl group unsubstituted or substituted with at least one

$C_3$-$C_{10}$ cycloalkyl group,

a1 to a4, a51, and a52 are each independently an integer from 0 to 10, provided that the sum of a51 and a52 is 1 or more,

a53 is an integer from 1 to 10,

two or more groups selected from a plurality of groups $R_1$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

two or more groups selected from a plurality of groups $R_2$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

two or more groups selected from a plurality of groups $R_3$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

two or more groups selected from a plurality of groups $R_4$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

two or more groups selected from $R_1$ to $R_6$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

$R_{10a}$ is the same as described in connection with $R_1$,

a substituent(s) of the substituted $C_1$-$C_{60}$ alkyl group, the substituted $C_2$-$C_{60}$ alkenyl group, the substituted $C_2$-$C_{60}$ alkynyl group, the substituted $C_1$-$C_{60}$ alkoxy group, the substituted $C_3$-$C_{10}$ cycloalkyl group, the substituted $C_1$-$C_{10}$ heterocycloalkyl group, the substituted $C_3$-$C_{10}$ cycloalkenyl group, the substituted $C_1$-$C_{10}$ heterocycloalkenyl group, the substituted $C_6$-$C_{60}$ aryl group, the substituted $C_6$-$C_{60}$ aryloxy group, the substituted $C_6$-$C_{60}$ arylthio group, the substituted $C_7$-$C_{60}$ arylalkyl group, the substituted $C_1$-$C_{60}$ heteroaryl group, the substituted $C_7$-$C_{60}$ arylalkyl group, the substituted $C_1$-$C_{60}$ heteroaryl group, the substituted $C_1$-$C_{60}$ heteroaryloxy group, the substituted $C_1$-$C_{60}$ heteroarylthio group, the substituted $C_2$-$C_{60}$ heteroarylalkyl group, the substituted monovalent non-aromatic condensed polycyclic group, and the substituted monovalent non-aromatic condensed heteropolycyclic group is(are):

deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-$C_{60}$ alkenyl group, a $C_2$-$C_{60}$ alkynyl group, or a $C_1$-$C_{60}$ alkoxy group;

a $C_1$-$C_{60}$ alkyl group, a $C_2$-$C_{60}$ alkenyl group, a $C_2$-$C_{60}$ alkynyl group, or a $C_1$-$C_{60}$ alkoxy group, each substituted with deuterium, -F, -Cl, -Br, -I, $-CD_3$, $-CD_2H$,$-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{11})(Q_{12})$,$-Si(Q_{13})(Q_{14})(Q_{15})$, $-Ge(Q_{13})(Q_{14})(Q_{15})$, $-B(Q_{16})(Q_{17})$, $-P(=O)(Q_{18})(Q_{19})$, $-P(Q_{18})(Q_{19})$, or any combination thereof;

a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, and a monovalent non-aromatic condensed heteropolycyclic group, each unsubstituted or substituted with deuterium, -F, -Cl, -Br, -I, $-CD_3$, $-CD_2H$, $-CDH_2$, $-CF_3$, $-CF_2H$, $-CFH_2$, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a $C_1$-$C_{60}$ alkyl group, a $C_2$-C60 alkenyl group, a $C_2$-$C_{60}$ alkynyl group, a $C_1$-$C_{60}$ alkoxy group, a $C_3$-$C_{10}$ cycloalkyl group, a $C_1$-$C_{10}$ heterocycloalkyl group, a $C_3$-$C_{10}$ cycloalkenyl group, a $C_1$-$C_{10}$ heterocycloalkenyl group, a $C_6$-$C_{60}$ aryl group, a $C_6$-$C_{60}$ aryloxy group, a $C_6$-$C_{60}$ arylthio group, a $C_7$-$C_{60}$ arylalkyl group, a $C_1$-$C_{60}$ heteroaryl group, a $C_1$-$C_{60}$ heteroaryloxy group, a $C_1$-$C_{60}$ heteroarylthio group, a $C_2$-$C_{60}$ heteroarylalkyl group, a monovalent non-aromatic condensed polycyclic group, a monovalent non-aromatic condensed heteropolycyclic group, $-N(Q_{21})(Q_{22})$, $-Si(Q_{23})(Q_{24})(Q_{25})$,$-Ge(Q_{23})(Q_{24})(Q_{25})$, $-B(Q_{26})(Q_{27})$, $-P(=O)(Q_{28})(Q_{29})$, $-P(Q_{28})(Q_{29})$, or any combination

thereof;

$-N(Q_{31})(Q_{32})$, $-Si(Q_{33})(Q_{34})(Q_{35})$, $-Ge(Q_{33})(Q_{34})(Q_{35})$, $-B(Q_{36})(Q_{37})$,$-P(=O)(Q_{38})(Q_{39})$, or $-P(Q_{38})(Q_{39})$; or any combination thereof; and

$Q_1$ to $Q_9$, $Q_{11}$ to $Q_{19}$, $Q_{21}$ to $Q_{29}$, and $Q_{31}$ to $Q_{39}$ are each independently hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a $C_1$-$C_{60}$ alkyl group unsubstituted or substituted with deuterium, a $C_1$-$C_{60}$ alkyl group, a $C_6$-$C_{60}$ aryl group, or any combination thereof; a $C_2$-$C_{60}$ alkenyl group; a $C_2$-$C_{60}$ alkynyl group; a $C_1$-$C_{60}$ alkoxy group; a $C_3$-$C_{10}$ cycloalkyl group; a $C_1$-$C_{10}$ heterocycloalkyl group; a $C_3$-$C_{10}$ cycloalkenyl group; a $C_1$-$C_{10}$ heterocycloalkenyl group; a $C_6$-$C_{60}$ aryl group unsubstituted or substituted with deuterium, a $C_1$-$C_{60}$ alkyl group, a $C_6$-$C_{60}$ aryl group, or any combination thereof; a $C_6$-$C_{60}$ aryloxy group; a $C_6$-$C_{60}$ arylthio group; a $C_7$-$C_{60}$ arylalkyl group; a $C_1$-$C_{60}$ heteroaryl group; a $C_1$-$C_{60}$ heteroaryloxy group; a $C_1$-$C_{60}$ heteroarylthio group; a $C_2$-$C_{60}$ heteroarylalkyl group; a monovalent non-aromatic condensed polycyclic group; or a monovalent non-aromatic condensed heteropolycyclic group.

**2.** The organometallic compound of claim 1, wherein

M is Pt, Pd, or Au; and/or

wherein

$A_{51}$ is an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, or a sec-iso-pentyl group, unsubstituted or substituted with a methyl group, an ethyl group, an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, a sec-iso-pentyl group, or any combination thereof, and

$A_{52}$ is a deuterium-containing linear or branched $C_1$-$C_{20}$ alkyl group unsubstituted or substituted with at least one $C_3$-$C_{10}$ cycloalkyl group, in which the linear or branced $C_1$-$C_{20}$ alkyl group is a methyl group, an ethyl group, an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, or a sec-iso-pentyl group, unsubstituted or substituted with a methyl group, an ethyl group, an n-propyl group, an iso-propyl group, an n-butyl group, a sec-butyl group, an iso-butyl group, a tert-butyl group, an n-pentyl group, a tert-pentyl group, a neo-pentyl group, an iso-pentyl group, a sec-pentyl group, a 3-pentyl group, a sec-iso-pentyl group, or any combination thereof.

**3.** The organometallic compound of claims 1 or 2, wherein

a3 and a4 are not 0, and a group represented by $*-(L_3)_{b3}-(R_3)_{c3}$ and a group represented by $*-(L_4)_{b4}-(R_4)_{c4}$ are not hydrogen; or

wherein

a3 is not 0, and

at least one group represented by $*-(L_3)_{b3}-(R_3)_{c3}$ in a number of a3 satisfies <Condition A> and <Condition B>:

<Condition A>

$L_3$ is a single bond,

<Condition B>

$R_3$ is

hydrogen, deuterium, -F, a cyano group, $C_1$-$C_{20}$ alkyl group, or a $C_1$-$C_{20}$ alkoxy group;

a $C_1$-$C_{20}$ alkyl group or a $C_1$-$C_{20}$ alkoxy group, each substituted with deuterium, -F, $-CD_3$, $-CD_2H$, $-CDH_2$, $-CF_3$, $-CF_2H$, $-CFH_2$, a cyano group, a $C_1$-$C_{10}$ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof; or

a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F,-$CD_3$, -$CD_2H$, -$CDH_2$, -$CF_3$, -$CF_2H$, -$CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

**4.** The organometallic compound of claims 1 or 2, wherein

a4 is not 0, and
at least one group represented by *-$(L_4)_{b4}$-$(R_4)_{c4}$ in a number of a4 satisfies <Condition 1>, <Condition 2>, or combination thereof:

<Condition 1>
At least one $R_4$ in a number of c4 is a substituted or unsubstituted $C_6$-$C_{60}$ aryl group, a substituted or unsubstituted $C_1$-$C_{60}$ heteroaryl group, a substituted or unsubstituted monovalent non-aromatic condensed polycyclic group, or a substituted or unsubstituted monovalent non-aromatic condensed heteropolycyclic group,
<Condition 2>
$L_4$ is not a single bond.

**5.** The organometallic compound of claims 1 or 2, wherein

a4 is not 0, and
at least one group represented by *-$(L_4)_{b4}$-$(R_4)_{c4}$ in a number of a4 satisfies <Condition 1(1)>, <Condition 2(1)>, or combination thereof:

<Condition 1(1)>
At least one $R_4$ in a number of c4 is a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a ($C_1$-$C_{20}$ alkyl)phenyl group, a biphenyl group, or a terphenyl group, each unsubstituted or substituted with deuterium, -F, -$CD_3$, -$CD_2H$, -$CDH_2$, -$CF_3$, -$CF_2H$, -$CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a (C1-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof,
<Condition 2(1)>
$L_4$ is a benzene group unsubstituted or substituted with deuterium, -F, -$CD_3$,-$CD_2H$, -$CDH_2$, -$CF_3$, -$CF_2H$, -$CFH_2$, a cyano group, a $C_1$-$C_{20}$ alkyl group, a deuterium-containing $C_2$-$C_{20}$ alkyl group, a $C_1$-$C_{20}$ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a ($C_1$-$C_{20}$ alkyl)cyclopentyl group, a ($C_1$-$C_{20}$ alkyl)cyclohexyl group, a ($C_1$-$C_{20}$ alkyl)cycloheptyl group, a ($C_1$-$C_{20}$ alkyl)cyclooctyl group, a ($C_1$-$C_{20}$ alkyl)adamantanyl group, a ($C_1$-$C_{20}$ alkyl)norbornanyl group, a ($C_1$-$C_{20}$ alkyl)norbornenyl group, a ($C_1$-$C_{20}$ alkyl)cyclopentenyl group, a ($C_1$-$C_{20}$ alkyl)cyclohexenyl group, a ($C_1$-$C_{20}$ alkyl)cycloheptenyl group, a ($C_1$-$C_{20}$ alkyl)bicyclo[1.1.1]pentyl group, a ($C_1$-$C_{20}$ alkyl)bicyclo[2.1.1]hexyl group, a ($C_1$-$C_{20}$ alkyl)bicyclo[2.2.1]heptyl group, a ($C_1$-$C_{20}$ alkyl)bicyclo[2.2.2]octyl group, a phenyl group, a ($C_1$-$C_{20}$ alkyl)phenyl group, a biphenyl group, a terphenyl group, or any combination thereof.

**6.** The organometallic compound of claims 1 or 2, wherein

a4 is not 0, and
at least one group represented by *-$(L_4)_{b4}$-$(R_4)_{c4}$ in a number of a4 satisfies <Condition 3>, <Condition 4>, <Condition 5>, or combination thereof:

<Condition 3>
At least one $R_4$ in a number of c4 is a substituted $C_6$-$C_{60}$ aryl group,
<Condition 4>
$L_4$ is a $C_5$-$C_{30}$ carbocyclic group substituted with at least one $R_{10a}$,
<Condition 5>
$L_4$ is a $C_5$-$C_{30}$ carbocyclic group that is unsubstituted or substituted with at least one $R_{10a}$ and $R_4$ is not hydrogen.

**7.** The organometallic compound of any of claims 1-6, wherein

a51 and a52 are each independently 0, 1, or 2,
the sum of a51 and a52 is 1 or 2, and
a53 is 1 or 2.

**8.** The organometallic compound of any of claims 1-7, wherein, in Formula 1,

a group represented by

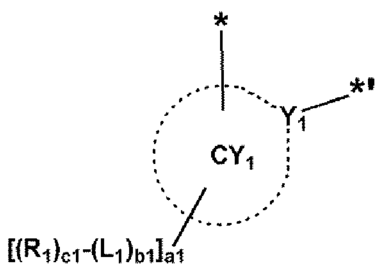

is a group represented by one of Formulae CY1(1) to CY1(8),
a group represented by

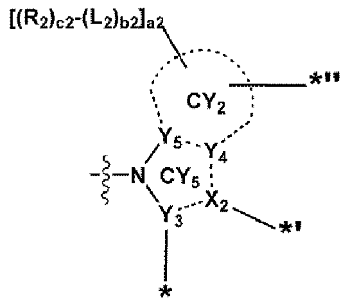

is a group represented by one of Formulae CY2(1) to CY2(4),
a group represented by

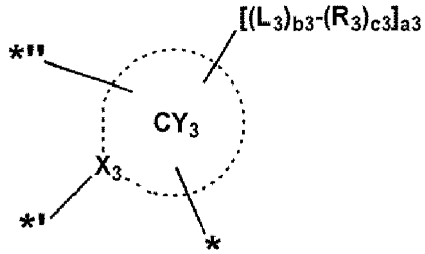

is a group represented by one of Formulae CY3(1) to CY3(24), and
a group represented by

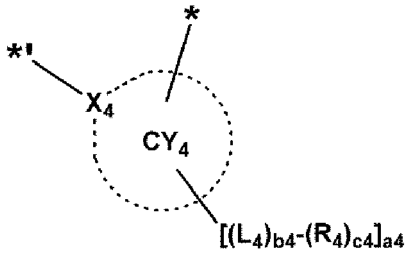

is a group represented by one of Formulae CY4(1) to CY4(74):

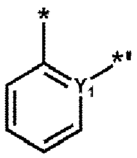

CY1(1)

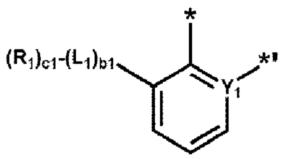

CY1(2)

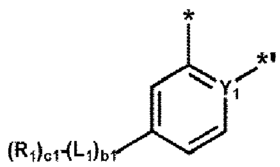

CY1(3)

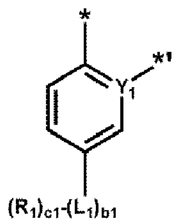

CY1(4)

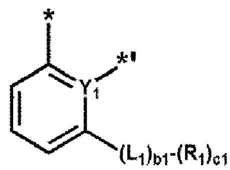

CY1(5)

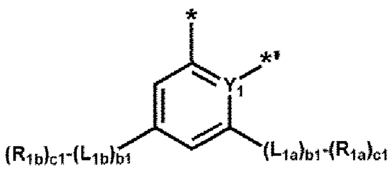

CY1(6)

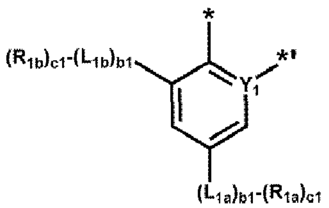

CY1(7)

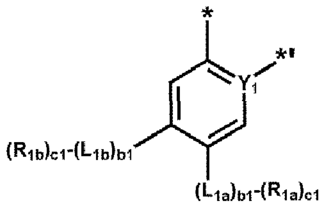

CY1(8)

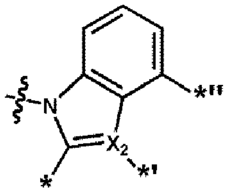
CY2(1)

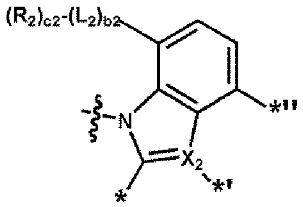
CY2(2)

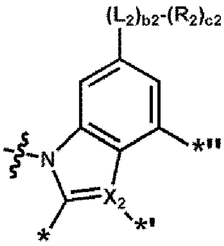
CY2(3)

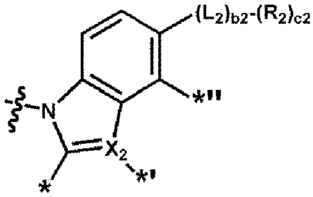
CY2(4)

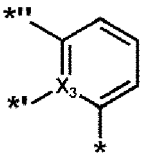
CY3(1)

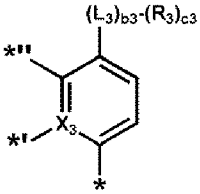
CY3(2)

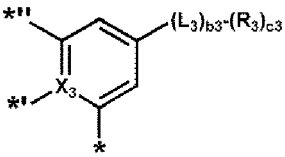
CY3(3)

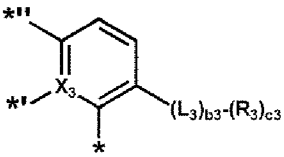
CY3(4)

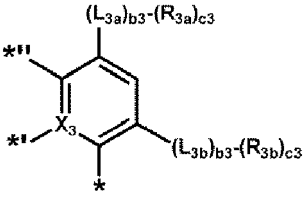
CY3(5)

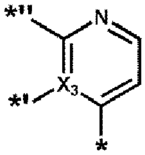
CY3(6)

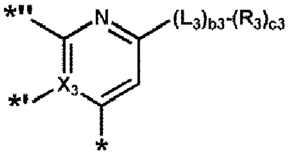
CY3(7)

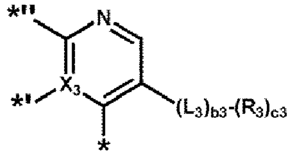
CY3(8)

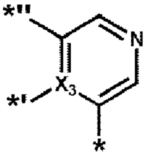
CY3(9)

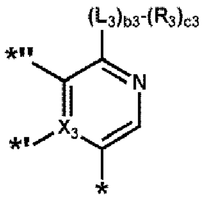
CY3(10)

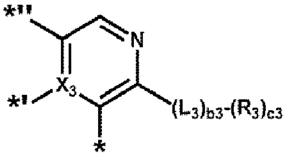
CY3(11)

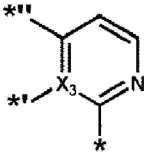
CY3(12)

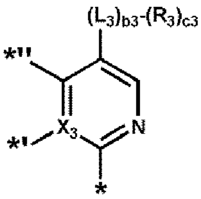
CY3(13)

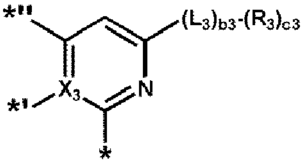
CY3(14)

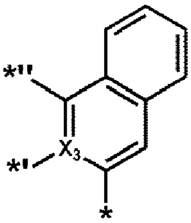
CY3(15)

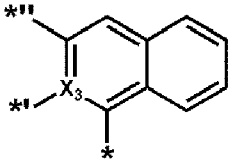
CY3(16)

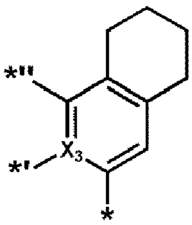
CY3(17)

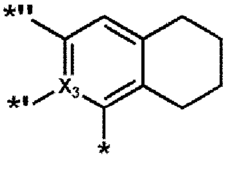
CY3(18)

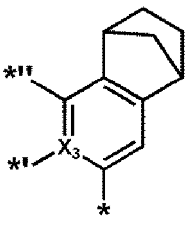
CY3(19)

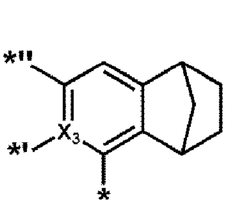
CY3(20)

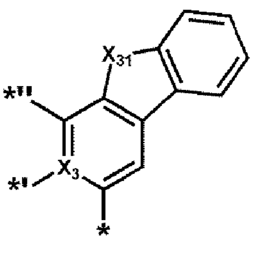
CY3(21)

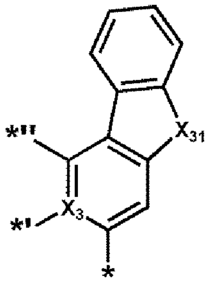

CY3(22)

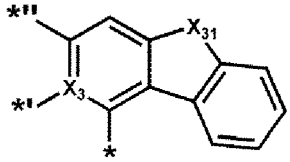

CY3(23)

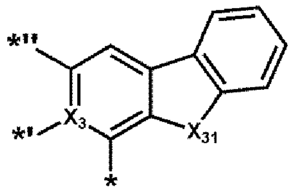

CY3(24)

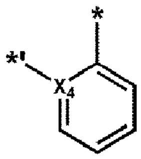

CY4(1)

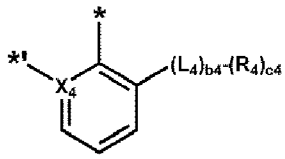

CY4(2)

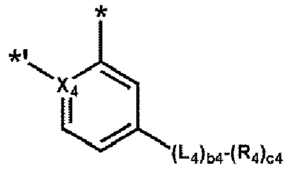

CY4(3)

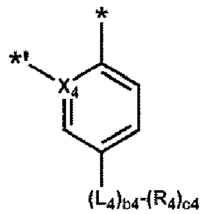

CY4(4)

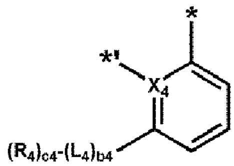

CY4(5)

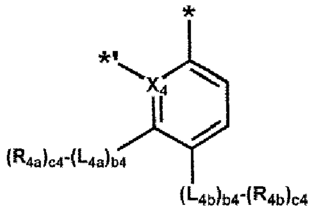

CY4(6)

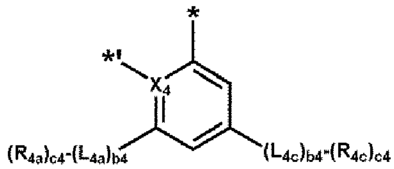

CY4(7)

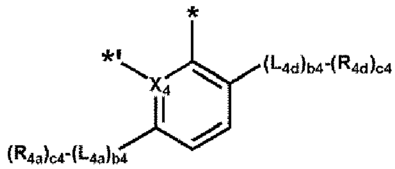

CY4(8)

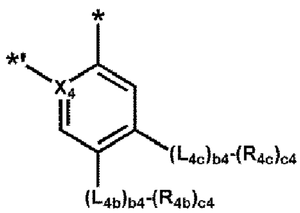

CY4(9)

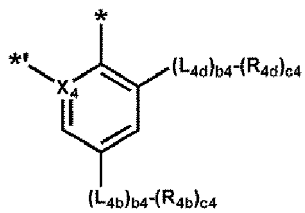

CY4(10)

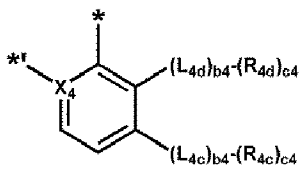

CY4(11)

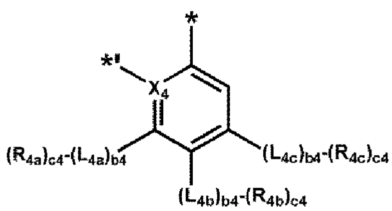

CY4(12)

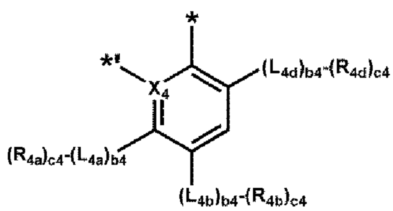

CY4(13)

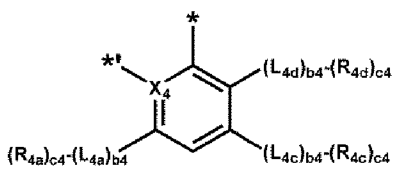

CY4(14)

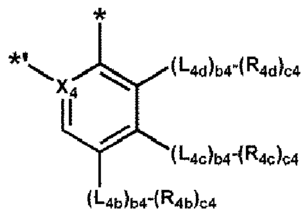
CY4(15)

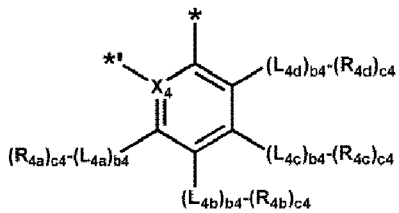
CY4(16)

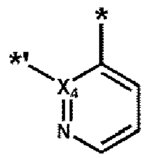
CY4(17)

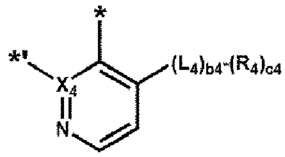
CY4(18)

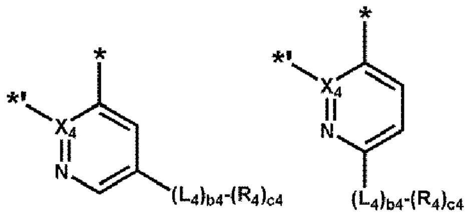
CY4(19)

CY4(20)

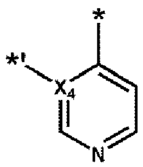
CY4(21)

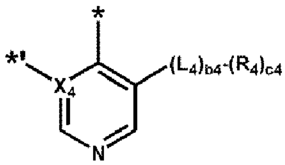
CY4(22)

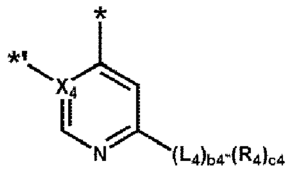
CY4(23)

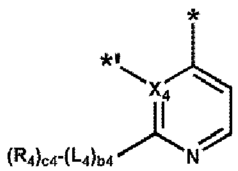
CY4(24)

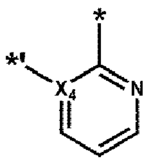
CY4(25)

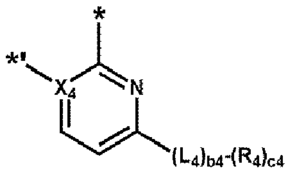
CY4(26)

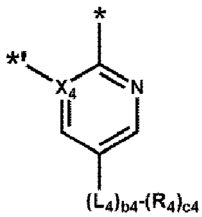
CY4(27)

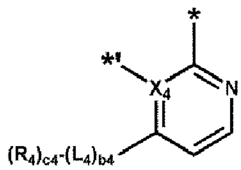
CY4(28)

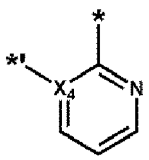
CY4(29)

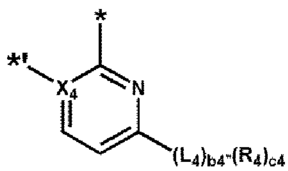
CY4(30)

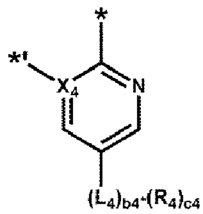
CY4(31)

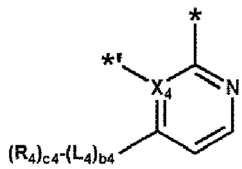
CY4(32)

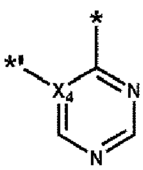
CY4(33)

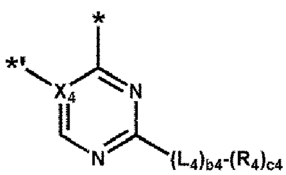
CY4(34)

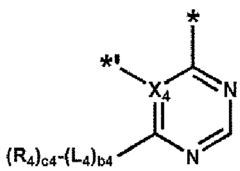
CY4(35)

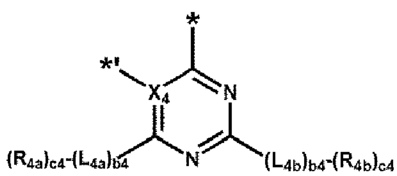
CY4(36)

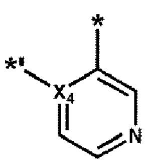
CY4(37)

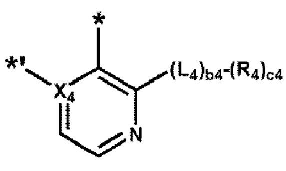
CY4(38)

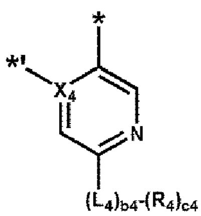
CY4(39)

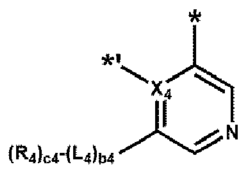
CY4(40)

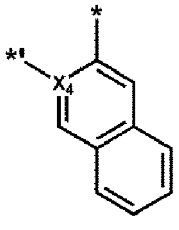
CY4(41)
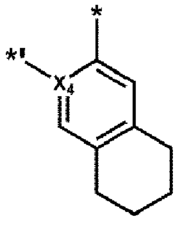
CY4(42)
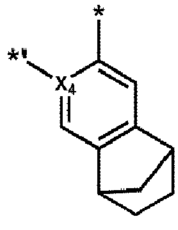
CY4(43)
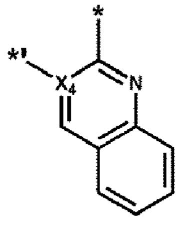
CY4(44)
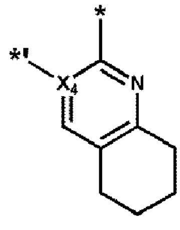
CY4(45)
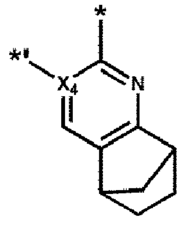
CY4(46)

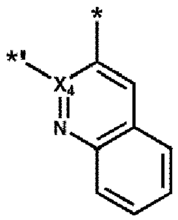
CY4(47)
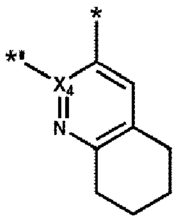
CY4(48)
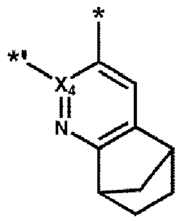
CY4(49)
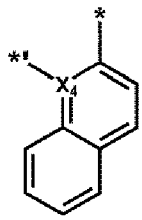
CY4(50)
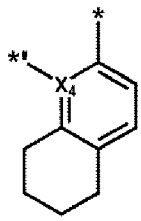
CY4(51)
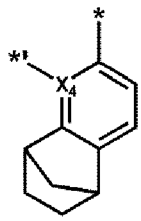
CY4(52)

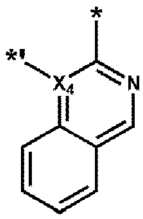
CY4(53)
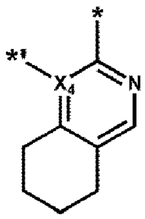
CY4(54)
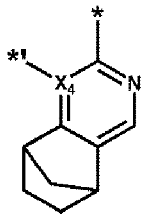
CY4(55)
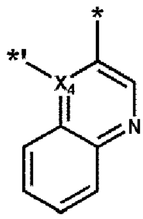
CY4(56)
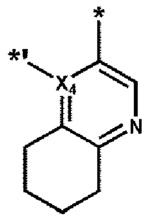
CY4(57)
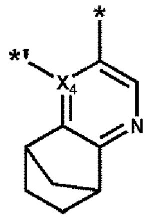
CY4(58)

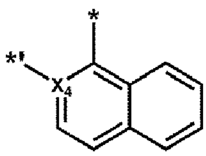
CY4(59)
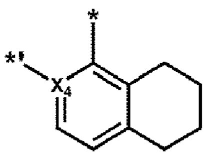
CY4(60)
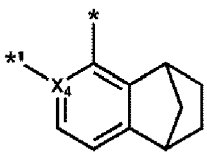
CY4(61)
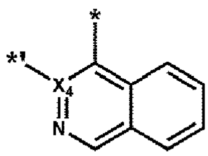
CY4(62)
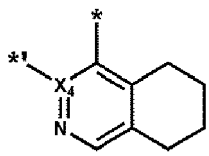
CY4(63)
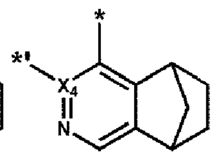
CY4(64)

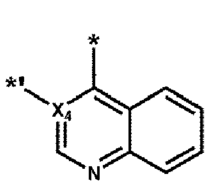
CY4(65)
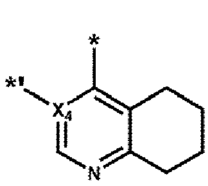
CY4(66)
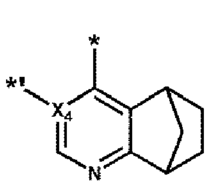
CY4(67)
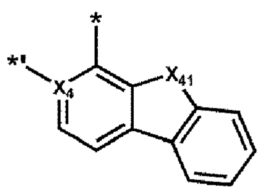
CY4(68)
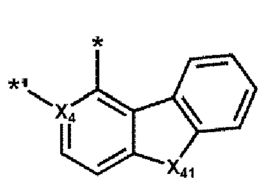
CY4(69)

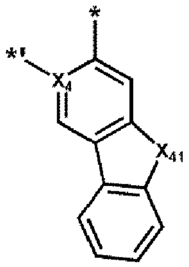
CY4(70)

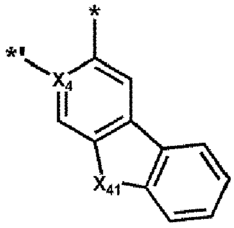
CY4(71)

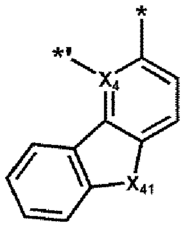
CY4(72)

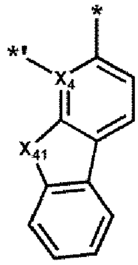
CY4(73)

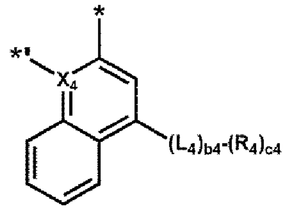
CY4(74)

wherein, in Formulae CY1(1) to CY1(8), CY2(1) to CY2(4), CY3(1) to CY3(24), and CY4(1) to CY4(74),
$X_2$ to $X_4$, $Y_1$, $L_1$ to $L_4$, b1 to b4, $R_1$ to $R_4$, and c1 to c4 are each independently the same as described in claim 1,
$X_{31}$ is O, S, $N(R_{31})$, $C(R_{31})(R_{32})$, or $Si(R_{31})(R_{32})$,
$X_{41}$ is O, S, $N(R_{41})$, $C(R_{41})(R_{42})$, or $Si(R_{41})(R_{42})$,
$L_{1a}$ and $L_{1b}$ are each independently the same as described in connection with $L_1$ in claim 1,
$R_{1a}$ and $R_{1b}$ are each independently the same as described in connection with $R_1$ in claim 1,
$L_{3a}$ and $L_{3b}$ are each independently the same as described in connection with $L_3$ in claim 1,
$R_{3a}$, $R_{3b}$, $R_{31}$, and $R_{32}$ are each independently the same as described in connection with $R_3$ in claim 1,
$L_{4a}$ to $L_{4d}$ are each independently the same as described in connection with $L_4$ in claim 1,
$R_{4a}$ to $R_{4d}$, $R_{41}$, and $R_{42}$ are each independently the same as described in connection with $R_4$ in claim 1,
$*-(L_1)_{b1}-(R_1)_{c1}$, $*-(L_{1a})_{b1}-(R_{1a})_{c1}$, $*-(L_{1b})_{b1}-(R_{1b})_{c1}$, $*-(L_2)_{b2}-(R_2)_{c2}$, $*-(L_3)_{b3}-(R_3)_{c3}$, $*-(L_{3a})_{b3}-(R_{3a})_{c3}$, $*-(L_{3b})_{b3}-(R_{3b})_{c3}$, $*-(L_4)_{b4}-(R_4)_{c4}$, $*-(L_{4a})_{b4}-(R_{4a})_{c4}$, $*-(L_{4b})_{b4}-(R_{4b})_{c4}$, $*-(L_{4c})_{b4}-(R_{4c})_{c4}$, and $*-(L_{4d})_{b4}-(R_{4d})_{c4}$ are not hydrogen, wherein * indicates a binding site to a neighboring atom,
in Formulae CY1(1) to CY1(8), *' indicates a binding site to $X_1$ in Formula 1,
in Formulae CY2(1) to CY2(4), CY3(1) to CY3(24), and CY4(1) to CY4(74), *' indicates a binding site to M in Formula 1,
in Formulae CY1(1) to CY1(8), * indicates a binding site to $Y_3$ in Formula 1,
in Formulae CY2(1) to CY2(4), * indicates a binding site to ring $CY_1$ in Formula 1, and *" indicates a binding site to ring $CY_3$ in Formula 1,
in Formulae CY3(1) to CY3(24), *" indicates a binding site to ring $CY_2$ in Formula 1, and * indicates a binding site to $T_1$ in Formula 1, and
in Formulae CY4(1) to CY4(74), * indicates a binding site to $T_1$ in Formula 1.

**9.** The organometallic compound of any of claims 1-8, wherein,
in Formula 1,

a group represented by

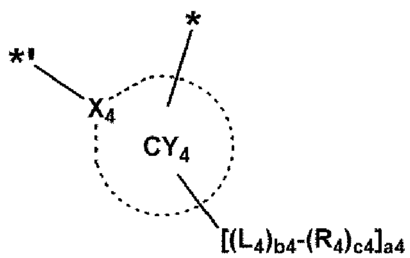

is a group represented by Formula CY4-1 or CY4-2:

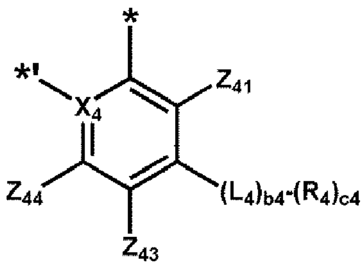

CY4-1

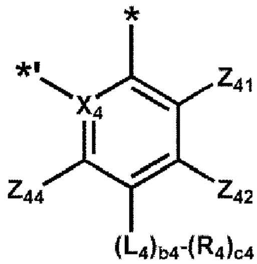

CY4-2

wherein, in Formulae CY4-1 and CY4-2,
$X_4$, $L_4$, b4, $R_4$, and c4 are each independently the same as described in claim 1,
$Z_{41}$ to $Z_{44}$ are each independently the same as described in connection with $R_4$ in claim 1,
*' indicates a binding site to M in Formula 1, and
* indicates a binding site to $T_1$ in Formula 1.

**10.** The organometallic compound of claims 8 or 9, wherein, in Formula 1,

a group represented by

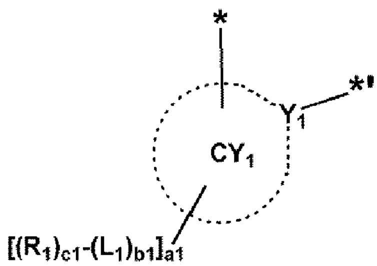

is a group represented by Formula CY1(1) or CY1(6),
a group represented by

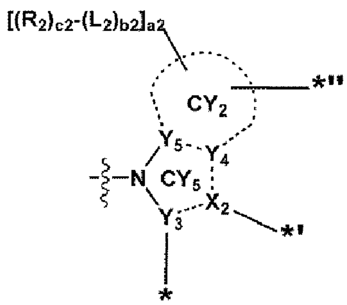

is a group represented by Formula CY2(1),
a group represented by

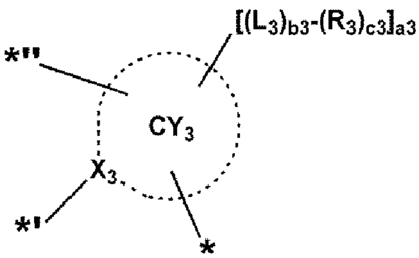

is a group represented by Formula CY3(3), and
a group represented by

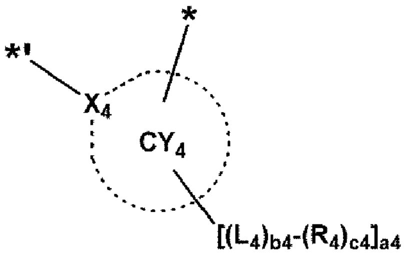

is a group represented by Formula CY4(3), CY4(4) or CY4(16).

**11.** The organometallic compound of any of claims 1-10, wherein

a group represented by

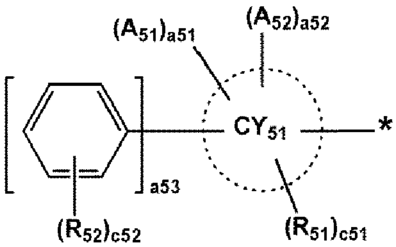

in Formula 1 is a group represented by one of Formulae 51-1 to 51-32:

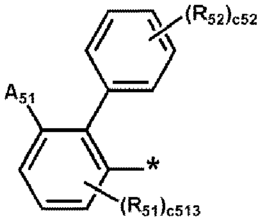

51-1

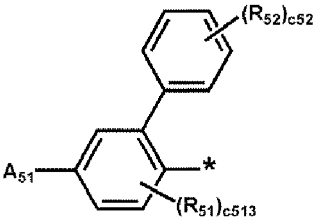

51-2

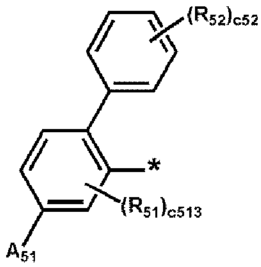

51-3

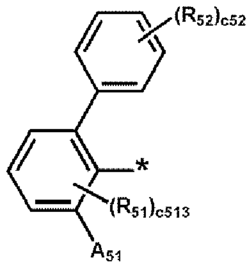

51-4

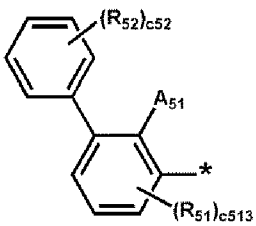

51-5

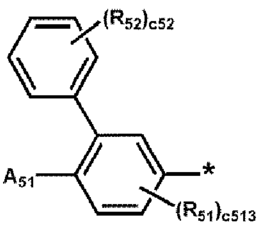

51-6

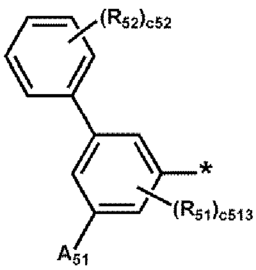

51-7

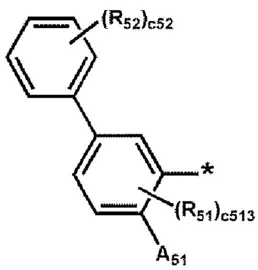

51-8

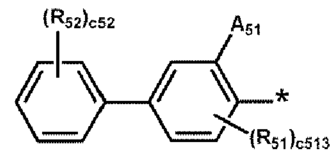
51-9

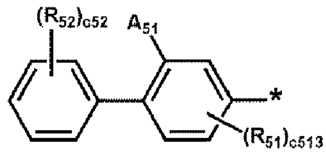
51-10

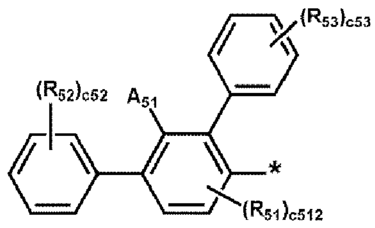
51-11

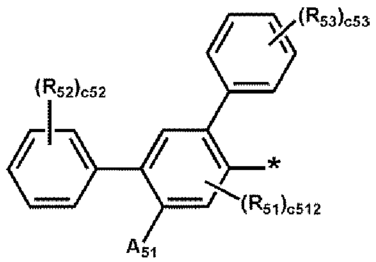
51-12

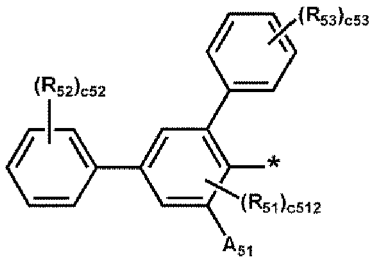
51-13

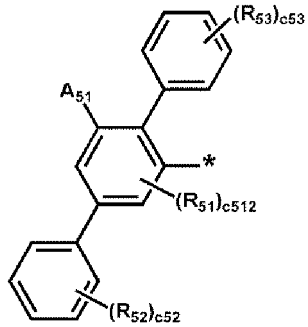
51-14

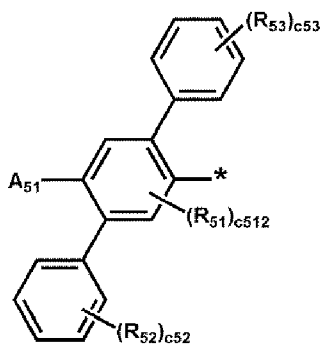
51-15

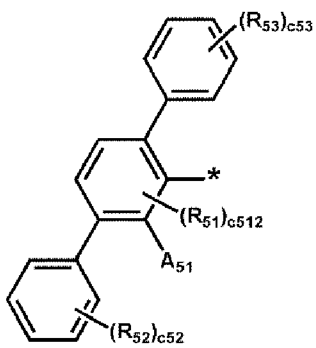
51-16

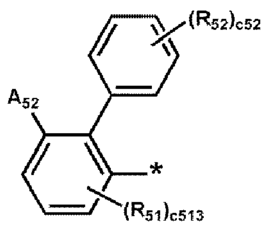
51-17

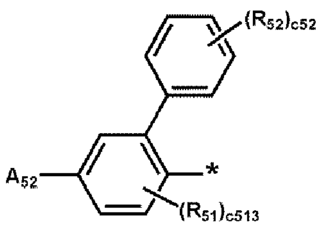
51-18

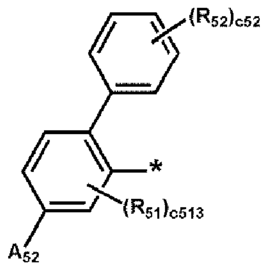
51-19

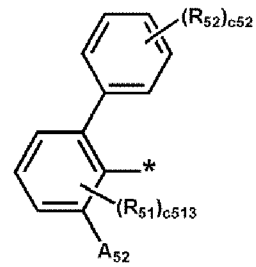
51-20

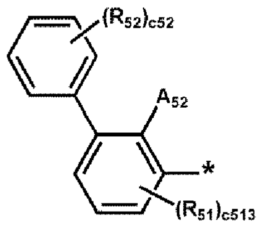
51-21

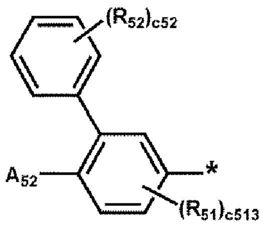
51-22

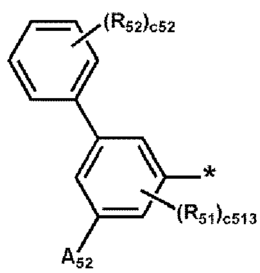
51-23

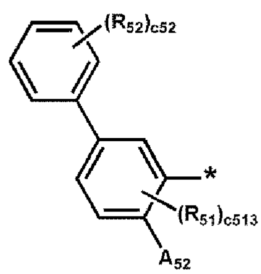
51-24

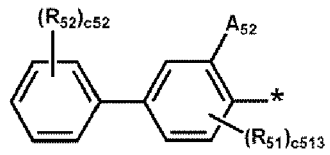

51-25

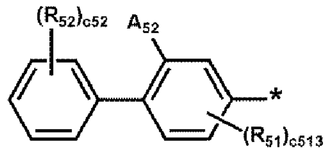

51-26

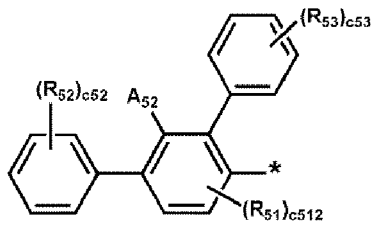

51-27

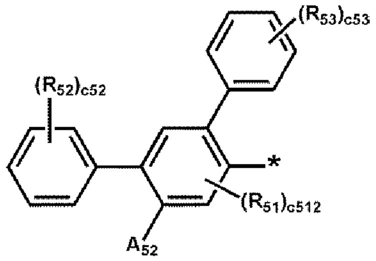

51-28

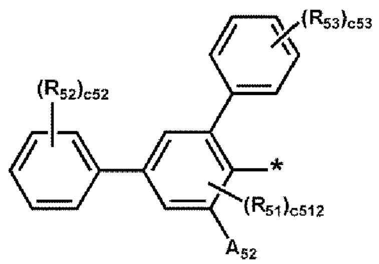

51-29

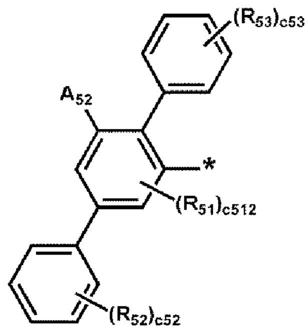

51-30

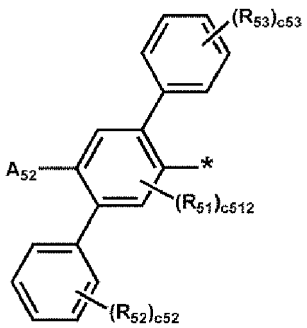

51-31

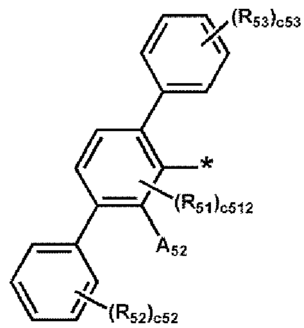

51-32

,

wherein, in Formulae 51-1 to 51-32,
$R_{51}$, $R_{52}$, c52, $A_{51}$, and $A_{52}$ are each independently the same as described in claim 1,
$R_{53}$ and c53 are each independently the same as described in connection with $R_{52}$ and c52 in claim 1,
c512 is an integer of 0 to 2,
c513 is an integer of 0 to 3, and
* indicates a binding site to $L_{51}$.

**12.** The organometallic compound of any of claims 1-11, wherein

the organometallic compound is represented by Formula 1-1 or 1-2:

<Formula 1-1>

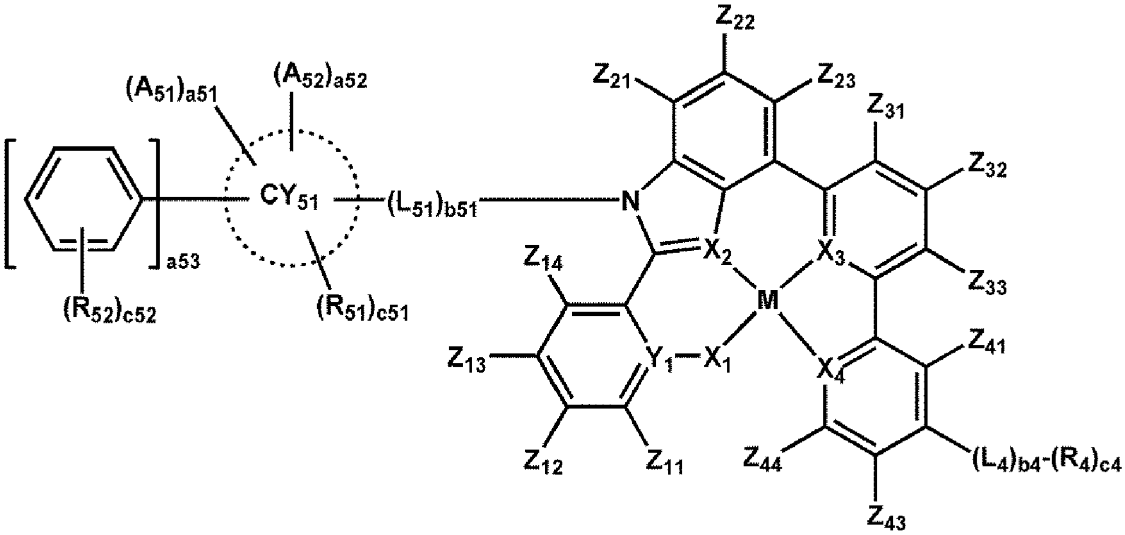

<Formula 1-2>

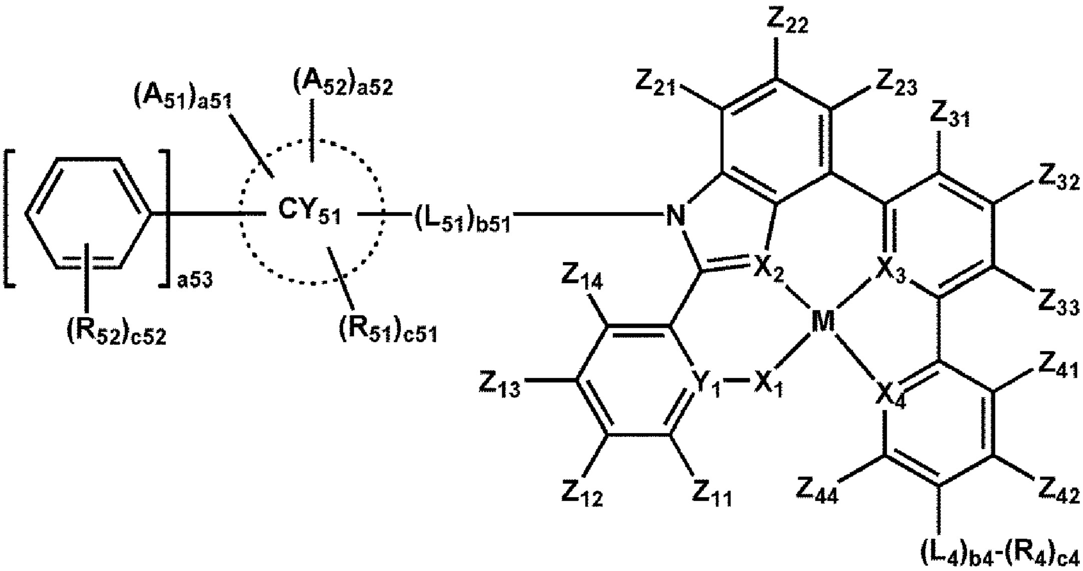

wherein, in Formulae 1-1 and 1-2,

M, $X_1$ to $X_4$, $Y_1$, $Y_3$ to $Y_5$, $L_4$, $L_{51}$, b4, b51, $R_{51}$, $R_{52}$, c51, c52, $A_{51}$, $A_{52}$, a51, a52 and a53 are each independently the same as described in claim 1,

$Z_{11}$ to $Z_{14}$ are each independently the same as described in connection with $R_1$ in claim 1,

$Z_{21}$ to $Z_{23}$ are each independently the same as described in connection with $R_2$ in claim 1,

$Z_{31}$ to $Z_{33}$ are each independently the same as described in connection with $R_3$ in claim 1,

$Z_{41}$ to $Z_{44}$ are each independently the same as described in connection with $R_4$ in claim 1,

two or more groups selected from $Z_{11}$ to $Z_{14}$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

two or more groups selected from $Z_{21}$ to $Z_{23}$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$,

two or more groups selected from $Z_{31}$ to $Z_{33}$ are optionally linked to form a $C_5$-$C_{30}$ carbocyclic group that is

unsubstituted or substituted with at least one $R_{10a}$ or a $C_1$-$C_{30}$ heterocyclic group that is unsubstituted or substituted with at least one $R_{10a}$,
two or more groups selected from $Z_{41}$ to $Z_{44}$ are optionally linked to form a $C_5$-$C_{30}$ carbocyclic group that is unsubstituted or substituted with at least one $R_{10a}$ or a $C_1$-$C_{30}$ heterocyclic group that is unsubstituted or substituted with at least one $R_{10a}$, and
$R_{10a}$ is the same as described in connection with $R_1$;
preferably wherein
$Z_{12}$, $Z_{14}$, $Z_{21}$ to $Z_{23}$, $Z_{31}$, $Z_{33}$ and $Z_{41}$ to $Z_{44}$ are each independently hydrogen, deuterium, $-CH_3$, or $-CD_3$.

**13.** An organic light-emitting device comprising:

a first electrode;
a second electrode; and
an organic layer disposed between the first electrode and the second electrode, wherein the organic layer comprises an emission layer, and at least one organometallic compound of any of claims 1-12;
preferably wherein
the first electrode is an anode,
the second electrode is a cathode,
the organic layer further comprises a hole transport region disposed between the first electrode and the emission layer and an electron transport region disposed between the emission layer and the second electrode,
wherein the hole transport region comprises a hole injection layer, a hole transport layer, an electron blocking layer, or any combination thereof, and
wherein the electron transport region comprises a hole blocking layer, an electron transport layer, an electron injection layer, or any combination thereof.

**14.** The organic light-emitting device of claim 13, wherein
the emission layer comprises the organometallic compound; .
preferably wherein the emission layer further comprises a host, and an amount of the host is larger than an amount of the organometallic compound.

**15.** A diagnostic composition comprising at least one organometallic compound of any of claims 1-12.

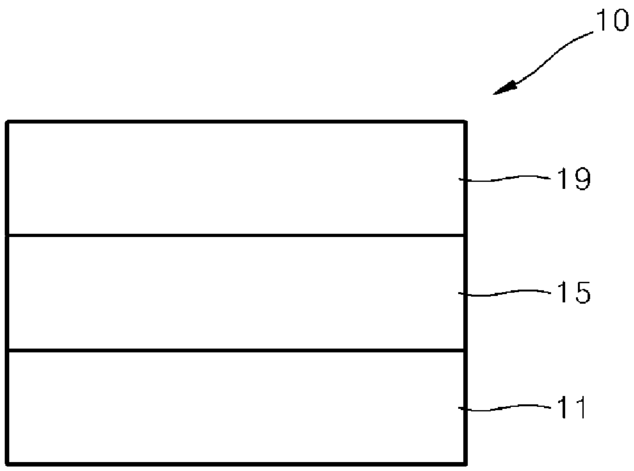
10
19
15
11

Europäisches Patentamt
European Patent Office
Office européen des brevets

# EUROPEAN SEARCH REPORT

Application Number
EP 19 19 3722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 266 790 A1 (SAMSUNG ELECTRONICS CO LTD [KR]) 10 January 2018 (2018-01-10)<br>* page 156 - page 162; claims 1,12-15 * | 1-15 | INV.<br>C07F15/00<br>C09K11/06<br>H01L51/00 |
| X | EP 3 366 690 A1 (SAMSUNG ELECTRONICS CO LTD [KR]) 29 August 2018 (2018-08-29)<br>* page 78 - page 79; claims 1,11-15; examples 75,76,78,79,81,84 * | 1-15 | |
| X,P | EP 3 508 491 A1 (SAMSUNG ELECTRONICS CO LTD [KR]) 10 July 2019 (2019-07-10)<br>* page 137 - page 145; claims 1,12-15 * | 1-15 | |

TECHNICAL FIELDS SEARCHED (IPC)

C07F
H05B
C09K
H01L

The present search report has been drawn up for all claims

1

| Place of search | Date of completion of the search | Examiner |
|---|---|---|
| The Hague | 13 December 2019 | Voyiazoglou, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 19 3722

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2019

| Patent document cited in search report | Publication date | Patent family member(s) | Publication date |
|---|---|---|---|
| EP 3266790 A1 | 10-01-2018 | CN 107573383 A | 12-01-2018 |
| | | EP 3266790 A1 | 10-01-2018 |
| | | JP 2018002722 A | 11-01-2018 |
| | | KR 20180005128 A | 15-01-2018 |
| | | KR 20190084015 A | 15-07-2019 |
| | | TW 201808977 A | 16-03-2018 |
| | | US 2018013078 A1 | 11-01-2018 |
| EP 3366690 A1 | 29-08-2018 | EP 3366690 A1 | 29-08-2018 |
| | | KR 20180098011 A | 03-09-2018 |
| | | US 2018244706 A1 | 30-08-2018 |
| EP 3508491 A1 | 10-07-2019 | CN 110003281 A | 12-07-2019 |
| | | EP 3508491 A1 | 10-07-2019 |
| | | US 2019211042 A1 | 11-07-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020130150834